# EUROPEAN PATENT APPLICATION

(11) **EP 4 421 093 A1**
(43) Date of publication of application: **28.08.2024**
(21) Application number: 22884040.1
(22) Date of filing: 22.10.2022
(51) Int. Cl.: C07K 16/28, C07K 16/32, C07K 16/30, A61P 35/00

(54) **ANTIGEN-BINDING PROTEIN COMPRISING TWO FC DOMAINS AND USE THEREOF**

(30) Priority: 22.10.2021 KR 20210142135
(71) Applicant: Centenaire Biosciences, Inc., Seongnam-si, Gyeonggi-do 13453 (KR)
(72) Inventor: CHOI, Eun Shik, Yongin-si, Gyeonggi-do 16938 (KR); PARK, Hyunkyu, Yongin-si, Gyeonggi-do 16836 (KR); BAE, Ji Seon, Suwon-si, Gyeonggi-do 16528 (KR); YANG, Gi-Hyeok, Seoul 06544 (KR)
(74) Representative: HGF
(86) International application number: PCT/KR2022/015981
(87) International publication number: WO 2023/068818

(57) **Abstract**

The present invention provides a fusion protein comprising one antigen-binding site and two Fc domains and having a novel antibody structure. Although such a novel antibody has a molecular weight similar to human IgG, the antibody structure enables Fc domains to be present on a cell surface antigen a maximum of four times compared to a natural human antibody. Thus, the fusion protein has increased affinity for Fcγ receptors, and has increased effector functions. Therefore, the fusion protein having a novel antibody format can be used as a novel antibody platform.

## Description

### Technical Field

The present invention relates to a novel antibody format having an antigen-binding site that specifically binds to a cancer surface antigen, and two Fc domains.

### Background Art

Antibody-based therapeutic agents and Fc fusion proteins are a group of clinically important drugs for patients with cancer, immune diseases, infectious diseases, and inflammatory diseases. ADCC (antibody-dependent cell-mediated cytotoxicity), ADCP (antibody-dependent cellular phagocytosis), and CDC (complement-dependent cytotoxicity), which are induced by the interaction between the antibody Fc domain and the innate immune system, play an important role in alleviating or treating symptoms of the disease.

Attempts are being made to maintain the bivalency of the antibody and to improve the effector function by increasing the number of Fc domains (Claudio Sustmann et al., MAbs. 2019; Dennis R Goulet et al., Proteins, 2020). Although these platforms confirmed improvements in binding ability to Fcγ receptors and ADCC, it is difficult to obtain homogeneous antibodies due to the complexity of production and purification. Attempts to improve the effector function by connecting the Fc domains of antibodies in tandem or constituting a large number of Fc domains are also currently underway (U.S. Patent Publication US 2020/0040084 A1). In this case, there is a disadvantage that permeability to the tissue may be significantly lowered due to the increase in the size or molecular weight of the antibody.

### Detailed Description of Invention

### Technical Problem

Accordingly, the present inventors studied to improve the function of the antibody and at the same time to solve the problems of the existing antibody format designed to comprise multiple Fc domains in tandem as described above. As a result, the present inventors developed a novel improved antibody format that enables Fc domains to be present on a cell surface antigen a maximum of four times compared to a natural human antibody, even though it has a molecular weight (approximately 150 kDa) similar to that of natural human immunoglobulin G (IgG).

### Solution to Problem

In one aspect of the present invention, there is provided a fusion protein comprising an antigen-binding site, a first Fc domain or a variant thereof linked to a first linking position of the antigen-binding site, and a second Fc domain or a variant thereof linked to a second linking position of the antigen-binding site.

In another aspect of the present invention, there is provided a fusion protein comprising two antigen-binding sites linked in tandem (tandem two antigen-binding regions), a first Fc domain or a variant thereof linked to a first linking position of the tandem 2 antigen-binding sites, and a second Fc domain or a variant thereof linked to a second linking position of the tandem 2 antigen-binding sites. According to one embodiment, each of the two antigen-binding sites constituting the tandem 2 antigen-binding sites may be a sequence comprising a CDR sequence or a variable region that is each capable of binding to different epitopes of the same antigen or to different antigens, or a sequence consisting of a variable region.

In the fusion proteins described in the present disclosure, according to one embodiment, the antigen-binding site may be a sequence comprising a CDR sequence or a variable region of an antibody or a sequence consisting of a variable region. Therefore, the antigen-binding site may comprise a first peptide consisting of or comprising a light chain CDR sequence or a light chain variable region of an antibody, and a second peptide consisting of or comprising a heavy chain CDR sequence or a heavy chain variable region of an antibody. The first Fc domain and the second Fc domain may each be a dimer consisting of two peptide sequences. The first peptide of the antigen-binding site binds to a first Fc domain or a variant thereof, and the second peptide of the antigen-binding site binds to a second Fc domain or a variant thereof.

In the fusion proteins described in the present disclosure, according to another embodiment, the first Fc domain and the second Fc domain may be linked to each other through a covalent bond, non-covalent bond, or linker, or may not be linked to each other. In a preferred embodiment, the first Fc domain and the second Fc domain are not linked to each other.

According to embodiments of the present disclosure, the Fc domain may be an Fc domain of a wild-type immunoglobulin, and may comprise modifications for modulating the reactivity of the Fc domain to Fcγ receptors (FcγRs), ADCC or minimizing the formation of undesirable multimers of the Fc domain, for example amino acid substitutions. The Fc domain comprises CH2 and CH3 regions, and may comprise a CH4 region and/or a hinge region, and it should be interpreted that the Fc domain comprises Fc domain fragments that exhibit the function of the Fc domain.

According to embodiments of the present disclosure, an antigen-binding site and an Fc domain or a variant thereof may be joined either directly or through a linker. For example, an antigen-binding site and an Fc domain or a variant thereof may be connected with or without a linker between the N-terminus and the C-terminus, between the N-terminus and the N-terminus, or between the C-terminus and the C-terminus of each peptide molecule.

According to embodiments of the present disclosure, when an antigen-binding site and an Fc domain or a variant thereof is joined through a linker, the linker may be a commonly used pepetide linker. For example, the linker may be a peptide consisting of 1-70 amino acid residues, 2-60 amino acid residues, 2-50 amino acid residues, 2-40 amino acid residues, 2-30 amino acid residues, 3-50 amino acid residues, 3-40 amino acid residues, 3-30 amino acid residues, 2-28 amino acid residues, 2-26 amino acid residues, 2-24 amino acid residues, 2-22 amino acid residues, 2-20 amino acid residues, 2-18 amino acid residues, 2-16 amino acid residues, 2-14 amino acid residues, 2-12 amino acid residues, or 2-10 amino acid residues. The connection between a first Fc domain and an antigen-binding site, the connection between a second Fc domain and an antigen-binding site, or both may be achieved through a linker.

In another aspect of the present invention, there is provided a pharmaceutical composition for preventing or treating cancer, comprising the fusion protein as an active ingredient.

In another aspect of the present invention, there is provided nucleotides encoding the fusion protein, and a vector comprising the nucleotides, and a transformed cell into which the vector has been introduced.

In another aspect of the present invention, there is provided a method for treating or preventing cancer, comprising administering the fusion protein to a subject.

In another aspect of the present invention, there is provided a use of the fusion protein for the treatment of cancer.

### Effects of Invention

Unlike wild type antibodies, the fusion protein having the novel antibody format of the present invention comprises one or two antigen-binding sites and two Fc domains. The two Fc domains are not directly linked to each other, but the two Fc domains are each independently linked to two different polypeptide chains constituting the antigen-binding site. Even though this novel antibody format has a size and molecular weight similar to that of human IgG, it can enable Fc domains to be present on a cell surface antigen a maximum of four times compared to a natural human antibody. Due to these properties, the fusion protein having the novel antibody format has improved affinity (avidity) for Fcγ receptors and can induce improved effector functions. Therefore, the fusion protein having the novel antibody format can be utilized for various purposes by replacing conventional antibodies.

### Brief Description of Drawings

Figure 1a is a schematic diagram of natural human immunoglobulin (IgG).
Figure 1b is a schematic diagram of the novel engineered antibody format.
Figure 1c is a schematic diagram showing a cancer cell with its tumor antigens bound to the antigen-specific human immunoglobulins (IgG) in a monovalent manner.
Figure 1d is a schematic diagram showing a cancer cell with its tumor antigens bound to the antigen-specific human immunoglobulin (IgG) in a monovalent or bivalent manner.
Figure 1e is a schematic diagram showing a cancer cell with its tumor antigens bound to the antigen-specific human immunoglobulins (IgG) in a bivalent manner.
Figure 1f is a schematic diagram showing a cancer cell with its tumor antigens bound to the antigen-specific novel engineered antibody.
Figure 2a is a schematic diagram of the novel monovalent antibody format (WT) with two Fc domains.
Figure 2b is a schematic diagram of an antibody (M1) in which VH Q105C and VL A43C amino acid substitutions are introduced in the novel monovalent antibody format with two Fc domains.
Figure 2c is a schematic diagram of an antibody (M2) in which CH1 F122C and CL S121C amino acid substitutions are introduced in the novel monovalent antibody format with two Fc domains.
Figure 2d is a schematic diagram of an antibody (M3) in which VH G44C and VL Q100C amino acid substitutions are introduced in the novel monovalent antibody format with two Fc domains.
Figure 3a illustrates sequence information indicating the position of Cys substitution in the VH-CH1 domain of trastuzumab. The WT sequence is SEQ ID NO: 8, the sequence of Mutant 1 is SEQ ID NO: 10, the sequence of Mutant 2 is SEQ ID NO: 12, and the sequence of Mutant 3 is SEQ ID NO: 14.
Figure 3b illustrates sequence information indicating the position of Cys substitution in the VL-CL domain of trastuzumab. The WT sequence is SEQ ID NO: 9, the sequence of Mutant 1 is SEQ ID NO: 11, the sequence of Mutant 2 is SEQ ID NO: 13, and the sequence of Mutant 3 is SEQ ID NO: 15.
Figure 4 illustrates a result obtained by SDS-PAGE analysis of WT, M1, M2, and M3.
Figure 5a illustrates a result obtained by size exclusion chromatography analysis of WT.
Figure 5b illustrates a result obtained by size exclusion chromatography analysis of M1.
Figure 5c illustrates a result obtained by size exclusion chromatography analysis of M2.
Figure 5d illustrates a result obtained by size exclusion chromatography analysis of M3.
Figure 6a is a schematic diagram of an antibody (M3) in which the CL domain and the hinge region are linked with a 15-mer peptide.
Figure 6b is a schematic diagram of an antibody (V1) in which the CL domain and the hinge region are linked with a 10-mer peptide.
Figure 6c is a schematic diagram of an antibody (V2) in which the CL domain and the hinge region are linked with a 5-mer peptide.
Figure 6d is a schematic diagram of an antibody (V3) in which the CL domain and the hinge region are directly linked without a linker.
Figure 7 illustrates a result obtained by SDS-PAGE analysis of M3, V1, V2, and V3.
Figure 8a is a schematic diagram of fragments generated when H01 and P01 are cleaved with papain.
Figure 8b is a schematic diagram of fragments generated by papain cleavage of H01 and P01 when a disulfide bond in the hinge region is abnormally formed.
Figure 8c illustrates a result obtained by SDS-PAGE analysis of the H01 papain cleavage product.
Figure 8d illustrates a result obtained by SDS-PAGE analysis of the P01 papain cleavage product.
Figure 9 illustrates a result obtained by SDS-PAGE analysis of H01wt and H01.
Figure 10a is a schematic diagram of H01Fv1, which has an Fv-(Fc)₂ structure.
Figure 10b is a schematic diagram of H01Fv2, which has an Fv-(Fc)₂ structure.
Figure 10c is a schematic diagram of H01Fv3, which has an Fv-(Fc)₂ structure.
Figure 10d is a schematic diagram of H01Fv4, which has an Fv-(Fc)₂ structure.
Figure 10e is a schematic diagram of H01Fv5, which has an Fv-(Fc)₂ structure.
Figure 10f is a schematic diagram of H01Fv6, which has an Fv-(Fc)₂ structure.
Figure 10g is a schematic diagram of H01Fv7, which has an Fv-(Fc)₂ structure.
Figure 10h is a table showing the mutated positions of the Fv-(Fc)₂ structure.
Figure 11 illustrates a result obtained by SDS-PAGE analysis of the Fv-(Fc)₂ structure after Protein A purification.
Figures 12a to 12g illustrate results obtained by SEC analysis of the Fv-(Fc)₂ structure after Protein A purification.
Figure 13a illustrates an analysis of the sensorgram data for the binding of H01Fv1, a purified Fv-(Fc)₂ structure, to human HER2.
Figure 13b illustrates an analysis of the sensorgram data for the binding of H01Fv2, a purified Fv-(Fc)₂ structure, to human HER2.
Figure 13c illustrates an analysis of the sensorgram data for the binding of H01Fv4, a purified Fv-(Fc)₂ structure, to human HER2.
Figure 13d illustrates an analysis of the sensorgram data for the binding of H01Fv5, a purified Fv-(Fc)₂ structure, to human HER2.
Figure 13e illustrates an analysis of the sensorgram data for the binding of H01Fv6, a purified Fv-(Fc)₂ structure, to human HER2.
Figure 13f illustrates an analysis of the sensorgram data for the binding of H01Fv7, a purified Fv-(Fc)₂ structure, to human HER2.
Figure 14 illustrates a differential scanning fluorimetry analysis of the melting temperatures of H01, P01, trastuzumab, and pertuzumab.
Figure 15 is a bio-layer interferometry analysis of the sensorgram data showing competitive binding of H01 and P01.
Figure 16a is a schematic diagram showing the binding mode of H01 in combination with P01 to HER2 on HER2-positive cancer cells.
Figure 16b is a schematic diagram showing the binding mode of trastuzumab in combination with pertuzumab to HER2 on HER2-positive cancer cells.
Figure 17a illustrates a flow cytometry analysis of the amount of Fc domain present on the surface of NCI-N87 gastric cancer cell line upon treatment with 50 nM anti-HER2 antibody.
Figure 17b illustrates a flow cytometry analysis of the amount of Fc domain present on the surface of BT474 breast cancer cell line upon treatment with 50 nM anti-HER2 antibody.
Figure 17c illustrates a flow cytometry analysis of the amount of Fc domain present on the surface of SK-OV3 ovarian cancer cell line upon treatment with 50 nM anti-HER2 antibody.
Figure 17d illustrates a flow cytometry analysis of the amount of Fc domain present on the surface of SNU-1 gastric cancer cell line upon treatment with 50 nM anti-HER2 antibody.
Figure 17e illustrates a flow cytometry analysis of the amount of Fc domain present on the surface of SNU-5 gastric cancer cell line upon treatment with 50 nM anti-HER2 antibody.
Figure 18a illustrates a flow cytometry analysis of the amount of Fc domain present on the surface of NCI-N87 gastric cancer cell line upon treatment with anti-HER2 antibody at indicated concentrations.
Figure 18b illustrates a flow cytometry analysis of the amount of Fc domain present on the surface of BT474 breast cancer cell line upon treatment with anti-HER2 antibody at indicated concentrations.
Figure 18c illustrates a flow cytometry analysis of the amount of Fc domain present on the surface of SK-OV3 ovarian cancer cell line upon treatment with anti-HER2 antibody at indicated concentrations.
Figure 18d illustrates a flow cytometry analysis of the amount of Fc domain present on the surface of SNU-1 gastric cancer cell line upon treatment with anti-HER2 antibody at indicated concentrations.
Figure 18e illustrates a flow cytometry analysis of the amount of Fc domain present on the surface of SNU-5 gastric cancer cell line upon treatment with anti-HER2 antibody at indicated concentrations.
Figure 19a is a schematic diagram of H01DE4 in which S239D and I332E mutations were introduced in H01.
Figure 19b is a schematic diagram of P01DE4 in which S239D and I332E mutations were introduced in P01.
Figures 20a to 20d are sensorgrams binding profiles of H01, H01DE4, P01, and P01DE4 for human HER2.
Figures 21a to 21h are sensorgrams binding profiles of H01, P01, H01DE4, P01DE4, human IgG1, trastuzumab, pertuzumab, and margetuximab for Fcγ receptor 1.
Figures 22a to 22h are sensorgrams binding profiles of H01, P01, H01DE4, P01DE4, human IgG1, trastuzumab, pertuzumab, and margetuximab for Fcγ receptor 2A (131R isoform).
Figures 23a to 23h are sensorgrams binding profiles of H01, P01, H01DE4, P01DE4, human IgG1, trastuzumab, pertuzumab, and margetuximab for Fcγ receptor 3A (176V isoform).
Figure 24a is a graph showing an antibody concentration in blood over time when H01, P01, trastuzumab, and pertuzumab were administered intravenously at 10 mg/kg to Sprague-Dawley rats.
Figure 24b illustrates the PK parameters calculated from Figure 24a when H01, P01, trastuzumab, and pertuzumab were administered intravenously at 10 mg/kg to Sprague-Dawley rats.
Figure 25 is a schematic diagram of HP501, which is a HER2 biparatopic engineered antibody.
Figure 26 is a schematic diagram of HP501 to HP516, which are HER2 biparatopic engineered antibodies.
Figure 27 illustrates a size exclusion chromatography analysis of HP501 to HP516, which are HER2 biparatopic engineered antibodies.
Figure 28a is a bio-layer interferometry analysis of the sensorgram data for the binding of HP503 to HER2.
Figure 28b is a bio-layer interferometry analysis of the sensorgram data for the binding of HP507 to HER2.
Figure 28c is a bio-layer interferometry analysis of the sensorgram data for the binding of HP511 for HER2.
Figure 28d is a bio-layer interferometry analysis of the sensorgram data for the binding of HP515 for HER2 protein using bio-layer interferometry analysis.
Figure 29a is a bio-layer interferometry analysis of the sensorgram data for the binding of HP503 for Fcγ receptor 1, Fcγ receptor 2A (131R isoform), and Fcγ receptor 3A (176V isoform).
Figure 29b is a bio-layer interferometry analysis of the sensorgram data for the binding of HP507 for Fcγ receptor 1, Fcγ receptor 2A (131R isoform), and Fcγ receptor 3A (176V isoform).
Figure 29c is a bio-layer interferometry analysis of the sensorgram data for the binding of HP511 for Fcγ receptor 1, Fcγ receptor 2A (131R isoform), and Fcγ receptor 3A (176V isoform).
Figure 29d is a bio-layer interferometry analysis of the sensorgram data for the binding of HP515 for Fcγ receptor 1, Fcγ receptor 2A (131R isoform), and Fcγ receptor 3A (176V isoform).
Figure 30a is a bio-layer interferometry analysis of the sensorgram data for the binding of HP503, HP507, HP511, and HP515 for the neonatal Fc receptor (FcRn).
Figure 30b is a bio-layer interferometry analysis of the sensorgram data for the binding of human IgG1, trastuzumab, pertuzumab, and margetuximab for the neonatal Fc receptor (FcRn).
Figure 31a illustrates the CDC activity of anti-HER2 antibodies in the BT474 breast cancer cell line.
Figure 31b illustrates the CDC activity of anti-HER2 antibodies in the NCI-N87 gastric cancer cell line.
Figure 32a illustrates the ADCC activity of anti-HER2 antibodies in the NCI-N87 gastric cancer cell line.
Figure 32b illustrates the ADCC activity of anti-HER2 antibodies in the MDA-MB-453 breast cancer cell line.
Figure 32c illustrates the ADCC activity of anti-HER2 antibodies in the SNU-601 gastric cancer cell line.
Figure 32d illustrates the ADCC activity of anti-HER2 antibodies in the SNU-5 gastric cancer cell line.
Figure 33a illustrates the antitumor activity of anti-HER2 antibodies in a C.B-17 SCID mouse model of tumor xenograft of SNU-5 gastric cancer cell line.
Figure 33b illustrates the antitumor activity of anti-HER2 antibodies in a Balb/c-nude mouse model of tumor xenograft of SNU-5 gastric cancer cell line.
Figure 34 illustrates the antitumor activity of anti-HER2 antibodies in a mouse model of tumor xenograft of SNU-601 gastric cancer cell line.
Figure 35 illustrates the antitumor activity of anti-HER2 antibodies in a mouse model of tumor xenograft of NCI-N87 gastric cancer cell line.
Figure 36 illustrates a vector to express human HER2 protein in mammalian cells.
Figure 37 illustrates the flow cytometry quantification of HER2 expression in a CT26 mouse large intestine cancer cell line clone expressing human HER2(CT26-HER2).
Figure 38 illustrates the stability of human HER2 expression in the CT26-HER2 clones.
Figure 39 illustrates the relative expression of human HER2 in CT26-HER2 cell line (Clone name: #2-60) compared to human cancer cell lines, and shows that H01 allows a greater amount of Fc domain to bind to the surface of CT26-HER2 cells compared to trastuzumab.
Figure 40 illustrates antitumor activity of anti-HER2 antibodies in a syngeneic CT26-HER2 mouse tumor model.
Figure 41a is a schematic diagram of a monovalent engineered mAb according to one embodiment.
Figure 41b is a schematic diagram of a biparatopic engineered mAb according to one embodiment.

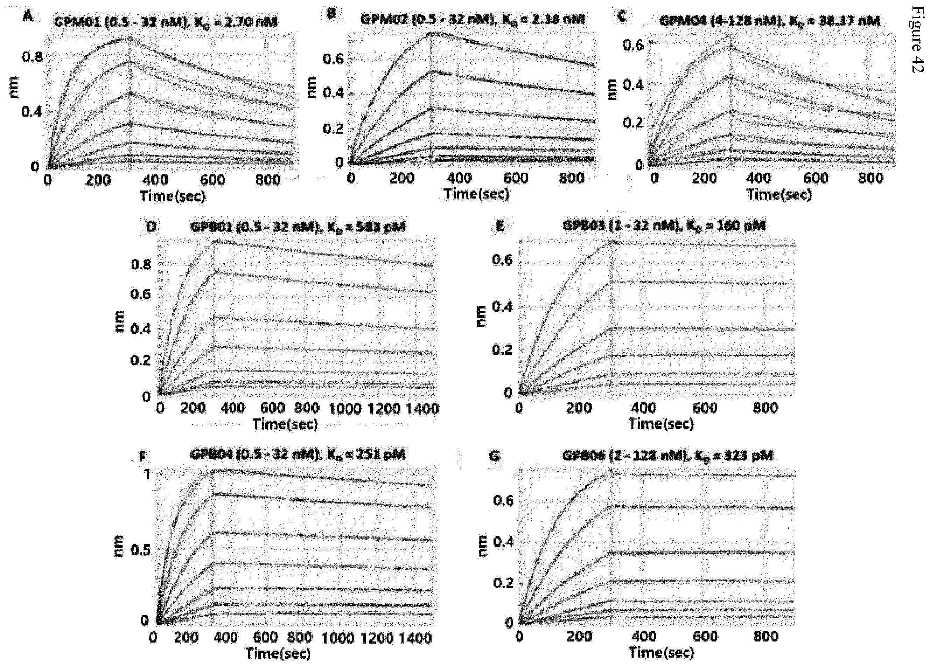

Figure 42 illustrates the bio-layer interferometry analysis of the sensorgram data for the binding of a fusion protein to the target according to one embodiment. Specifically, figure 42a illustrates the sensorgram data for the binding of GPM01, a monovalent engineered mAb targeting GPC-3, to human GPC-3. Figure 42b illustrates the sensorgram data for the binding of GPM02, a monovalent engineered mAb targeting GPC-3, to human GPC-3. Figure 42c illustrates the sensorgram data for the binding of GPM04, a monovalent engineered mAb targeting GPC-3, to human GPC-3. Figure 42d illustrates the sensorgram data for the binding of GPB01, a biparatopic engineered mAb targeting GPC-3, to human GPC-3. Figure 42e illustrates the sensorgram data for the binding of GPB03, a biparatopic engineered mAb targeting GPC-3, to human GPC-3. Figure 42f illustrates the sensorgram data for the binding of GPB04, a biparatopic engineered mAb targeting GPC-3, to human GPC-3. Figure 42g illustrates the sensorgram data for the binding of GPB06, a biparatopic engineered mAb targeting GPC-3, to human GPC-3.
Figure 43 illustrates the flow cytometry analysis of the amount of Fc domain present on the surface of HepG2 liver cancer cell line upon treatment with 100 nM GPC-3 antibody.
Figure 44 illustrates the SDS-PAGE analysis showing the inhibition of AKT phosphorylation in the PC-3 prostate cancer cell line upon treatment with 50 nM EphA2 antibody.
Figure 45 illustrates the flow cytometry analysis of the amount of Fc domain present on the surface of PC-3 prostate cancer cell line upon treatment with 100 nM EphA2 antibody.
Figure 46 illustrates the bio-layer interferometry analysis of the sensorgram data for the binding of MEM01 and MEM06, which are monovalent engineered mAbs targeting MET, to human MET.
Figure 47 illustrates the flow cytometry analysis of the amount of Fc domain present on the surface of MKN45 gastric cancer cell line upon treatment with MET antibody at indicated concentrations.
Figure 48 illustrates the flow cytometry analysis of the amount of Fc domain present on the surface of SNU5 gastric cancer cell line upon treatment with an antibody at indicated concentrations.
Figure 49 illustrates the bio-layer interferometry analysis of the sensorgram data for the binding of monovalent engineered mAbs targeting EGFR to human EGFR.
Figure 50 illustrates the bio-layer interferometry analysis of the sensorgram data for the binding of 33-1, 33-2, and 33-3, which are monovalent engineered mAbs targeting CD33, and 33-4, 33-5, 33-6, and 33-7, which are biparatopic engineered mAbs targeting CD33, to human CD33.
Figure 51 illustrates the bio-layer interferometry analysis of the sensorgram data for the binding of monovalent engineered mAbs targeting CEACAM5 to human CEACAM5.
Figure 52 illustrates the bio-layer interferometry analysis of the sensorgram data for the binding of a fusion protein according to one embodiment for the target. Specifically, figure 52a illustrates the sensorgram data for the binding of T01, a monovalent engineered mAb targeting TROP2, to human TROP2. Figure 52b illustrates the sensorgram data for the binding of MSM01, a monovalent engineered mAb targeting mesothelin, to human mesothelin. Figure 52c illustrates the sensorgram data for the binding of LIM01, a monovalent engineered mAb targeting LIV-1, to human LIV-1. A:T01, B:MSM01, C:LIM01.

### Best Mode for Carrying out the Invention

### Definition of Terms

As used herein, the term "fusion protein with two Fcs" or "antibody with two Fcs" refers to a fusion protein in which two Fc domains are independently joined to two polypeptide chains constituting the antigen-binding site. The two polypeptide chains constituting the antigen-binding site may be different from each other. For example, one of the two polypeptide chains constituting the antigen-binding site may be a sequence comprising or consisting of the light chain CDR sequence or light chain variable region of the antibody, or may be an scFv, and the other may be a sequence comprising or consisting of the heavy chain CDR sequence or heavy chain variable region of the antibody, or may be an scFv. In one embodiment, the fusion protein having the two Fc regions may comprise the sequence of a "humanized" form of a non-human antibody, which is a chimeric antibody comprising a human immunoglobulin comprising native CDRs. In addition, the fusion protein may comprise a "fully human antibody" or a portion of a "human antibody." In addition, in one embodiment, the multispecific fusion protein or antigen binding domain may be a "monoclonal antibody" or a portion thereof.

As used herein, the term "antibody" refers to a substance that specifically binds to an antigen and causes an antigen-antibody reaction. In addition, the antibody is also referred to as immunoglobulin. The antibody may refer to any one selected from IgG, IgE, IgM, IgD, and IgA, and may be a subclass of IgG, such as IgG1, IgG2, IgG3, IgG4, IgA1, or IgA2. In addition, the antibody may be an agonistic antibody or an antagonistic antibody.

As used herein, the term "Fab" or "Fab region" refers to a region of an antibody that binds to an antigen. Conventional IgG generally comprises two Fab regions. Each Fab region typically consists of one variable region and one constant region of each heavy chain and light chain. Specifically, the variable region and the constant region of the heavy chain in the Fab region are the VH and CH1 regions, and the variable region and the constant region of the light chain in the Fab region are the VL and CL regions. VH, CH1, VL, and CL of the Fab region may be arranged in various ways to impart the antigen binding ability according to the present disclosure, including the CrossMab Fab technology in which VH and VL have an arrangement substituted for each other.

As used herein, the term "heavy chain" refers to a polypeptide chain of about 50 kDa to about 70 kDa. Here, the N-terminal portion comprises a variable region of at least about 120 to 130 amino acids, and the C-terminal portion comprises a constant region. The constant region may be one of five types: alpha (α), delta (δ), epsilon (ε), gamma (γ), and mu (µ). Here, α, δ, and γ comprise about 450 amino acids, and µ and ε comprise about 550 amino acids.

As used herein, the term "light chain" refers to a polypeptide chain of about 25 kDa. Here, the N-terminal portion comprises a variable region of at least about 100 to about 110 amino acids, and the C-terminal portion comprises a constant region. There are two types of light chain constant domains: kappa (κ) or lambda (λ). In addition, the constant region of the light chain is referred to as "CL". The heavy chain C domains (CH domains) are numbered from N-terminus to C-terminus (e.g., CH1, CH2, CH3, etc.). The CL and a CH1 regions of any of these antibody classes may be used in the present disclosure. In certain embodiments, the CL and CH1 regions provided herein are of the IgG type (for example, IgG1).

As used herein, the term "Fc" or "Fc region" refers to the C-terminal region of an immunoglobulin heavy chain, including a native Fc region, a recombinant Fc region, and a variant Fc region. Therefore, Fc refers to the last two constant region immunoglobulin domains of IgA, IgD, and IgG, the last three constant region immunoglobulin domains of IgE and IgM, and the hinge present in the N-terminus of these domains. For IgA and IgM, the Fc may comprise a J chain. For IgG, the Fc comprises the immunoglobulin domains Cy2 (CH2) and Cy3 (CH3) and the hinge between Cy1 and Cy2. Although the interface of the Fc region may vary, the human IgG heavy chain Fc region is generally defined as comprising residues C226, P230, or A231 at the C-terminus, where numbering is according to the EU index. As used herein, "Fc polypeptide" or "Fc-derived polypeptide" refers to a polypeptide comprising all or part of an Fc region. In one embodiment, a variant Fc region may be in a form in which at least one amino acid, for example, about 1 to about 10 amino acids, or about 1 to about 5 amino acids, are substituted compared to the native sequence Fc region. In addition, the variant Fc region may have at least about 80% homology, at least about 90% homology, or at least about 95% homology to the native sequence Fc region.

As used herein, the term "Fv" or "Fv fragment" or "Fv region" is a polypeptide comprising the VL and VH domains of a single antibody.

As used herein, the term "single chain Fv" or "scFv" refers to an antibody fragment comprising the VH and VL domains of an antibody within a single polypeptide chain.

As used herein, the term "variable region" refers to an antibody region comprising one or more immunoglobulin domains encoded by any one of the VL (including Vkappa (VK) and Vlambda (VL)) and/or VH genes that constitute the light chain (including kappa and lambda) and heavy chain immunoglobulin loci, respectively. The light or heavy chain variable region (VL or VH) consists of a "framework" or "FR" region that includes three hypervariable regions referred to as "complementarity determining regions" or "CDRs". As used herein, the term "antigen" refers to a structure capable of selectively binding to an antibody. A target antigen may be a polypeptide, carbohydrate, nucleic acid, lipid, hapten, or other naturally occurring compound or synthetic compound. Specifically, an antigen is a polypeptide and may be a protein present on or within a cell.

As used herein, the term "epitope" refers to an antigenic determinant and is a part on an antigen to which an antibody or polypeptide binds. A protein epitope may comprise amino acid residues that are directly involved in binding as well as amino acid residues that are effectively blocked by specific antigen-binding antibodies or peptides. It is the simplest form or smallest structural region of a complex antigen molecule that may bind to an antibody or receptor. The epitope may be linear or structural/conformational.

As used herein, the term "vector" refers to a material for transporting or expressing a nucleic acid sequence including a nucleic acid sequence encoding a multispecific fusion protein (for example, an antibody) described herein. Specifically, vectors include expression vectors, plasmids, phage vectors, viral vectors, episomes, and artificial chromosomes.

As used herein, the term "polynucleotide," also referred to as "nucleic acid," refers to a polymer of nucleotides of any length. Specifically, the polynucleotide may be DNA or RNA.

### Antibody comprising two Fcs

In one aspect of the present invention, there is provided an antibody comprising a plurality of Fc domains, characterized in that the ratio of the antigen-binding site and the Fc domain is 1:2 or 2:2. Specifically, the antibody may be a fusion protein comprising an antigen-binding site, a first Fc domain or a variant thereof, and a second Fc domain or a variant thereof.

Here, the antigen-binding site may consist of two different polypeptide chains. In addition, each polypeptide may be linked to a first Fc domain or a variant thereof, and a second Fc domain or a variant thereof.

Here, in one embodiment of the fusion protein, when the antigen-binding site comprises Fab, it may be a fusion protein in which the two Fc domains are bound to the C-terminus of the CH1 region of the heavy chain and the C-terminus of the constant region of the light chain, respectively. In addition, the Fc domain and Fab may be linked through a peptide linker.

In addition, in one embodiment of the fusion protein, when the antigen-binding site is Fv, it may be a fusion protein in which the two Fc domains are bound to the C-terminus of the variable region of the heavy chain and the C-terminus of the variable region of the light chain, respectively. In addition, the Fc domain and Fv may be linked through a peptide linker.

This novel antibody format or sturcture has a molecular weight similar to human IgG. In addition, the fusion protein may have an antigen binding affinity equivalent to that of a human IgG-based antibody. However, the antibody format enables Fc domains to be present on a cell surface antigen a maximum of four times compared to a natural human antibody. Due to these characteristics, the fusion protein may have increased affinity for Fcγ receptors, and may have increased effector functions compared to the wild type antibody. Each Fc domain bound to the fusion protein may have a similar level of Fc receptor (Fcy receptor and FcRn) binding affinity as the Fc domain of an IgG-based antibody, but due to the avidity effect, the apparent binding affinity (apparent affinity) of the fusion protein to the Fc receptor (Fcy receptor and FcRn) may be significantly increased compared to a human IgG antibody. In addition, the fusion protein has a similar level of thermal stability as that of an IgG-based antibody.

Specifically, the fusion protein may be a fusion protein comprising (a) an antigen-binding site consisting of a first polypeptide comprising at least one complementarity-determining region (CDR) sequence and a second polypeptide comprising at least one complementarity-determining region (CDR) sequence, wherein the first polypeptide and the second polypeptide form a dimer, and the antigen-binding site is capable of specifically binding to a target antigen, (b) a first Fc domain or a variant thereof that is a dimer consisting of two polypeptide sequences, one of which is joined to the first polypeptide of the antigen-binding site, and (c) a second Fc domain or a variant thereof that is a dimer consisting of two polypeptide sequences, one of which is joined to the second polypeptide of the antigen-binding site.

Here, the first polypeptide of the antigen-binding site may comprise CDR1, CDR2, and CDR3 of an antibody heavy chain, and the second polypeptide of the antigen-binding site may comprise CDR1, CDR2, and CDR3 of an antibody light chain. In addition, the first polypeptide of the antigen-binding site may further comprise a CH1 region of an antibody heavy chain, and/or the second polypeptide of the antigen-binding site may further comprise a constant region of an antibody light chain.

The specific structure of the fusion protein is described in more detail below.

### Antigen-binding site

Here, the antigen-binding site is capable of specifically binding to a protein expressed on the cell surface. Specifically, the antigen-binding site is capable of specifically binding to a cancer antigen.

In one embodiment, the antigen-binding site is capable of specifically binding to any one selected from the group consisting of PD-L1, EGFR, EGFRvIII, BCMA, CD22, CD25, CD30, CD33, CD37, CD38, CD52, CD56, CD123, c-Met (MET), DLL3, DR4, DR5, GD2, nectin-4, RANKL, SLAMF7, Trop-2, LIV-1, claudin 18.2, IL13α2, CD3, HER2, HER3, FGFR2, FGFR3, GPC3, ROR1, Folα, CD20, CD19, CTLA-4, VEGFR, NCAM1, ICAM-1, ICAM-2, CEACAM5, CEACAM6, carcinoembryonic antigen (CEA), CA-125, alphafetoprotein (AFP), MUC-1, MUC-16, PSMA, PSCA, epithelial tumor antigen (ETA), melanoma-associated antigen (MAGE), immature laminin receptor, TAG-72, HPV E6/E7, BING-4, calcium-activated chloride channel 2, cyclin-B1, 9D7, Ep-CAM, EphA2, EphA3, mesothelin, SAP-1, survivin, and virus-derived antigens.

A second antigen-binding site is also capable of specifically binding to any one antigen selected from the above group. According to one embodiment, the antigen to which a first antigen-binding site binds may be different from the antigen to which a second antigen-binding site binds. For example, the first antigen-binding site may comprise a sequence that specifically binds to HER2, and the second antigen-binding site may comprise a sequence that specifically binds to EGFR. In another embodiment, the first antigen-binding site may comprise a sequence that specifically binds to one epitope of an antigen, and the second antigen-binding site may comprise a sequence that specifically binds to a different epitope of the same antigen.

### Specific examples of antigen-binding sites

Here, the antigen-binding site may comprise a variable region that specifically binds to the antigen. Specifically, the variable region may include the heavy chain variable region and light chain variable region of any one antibody selected from the group consisting of cetuximab, panitumumab, necitumumab, imgatuzumab, depatuxizumab, losatuxizumab, etevritamab, AMG-595, atezolizumab, avelumab, durvalumab, trastuzumab, pertuzumab, onartuzumab, emibetuzumab, telisotuzumab, datopotamab, sacituzumab, rovalpituzumab, tarlatamab, belantamab, ladiratuzumab, codrituzumab, aprutumab, bemarituzumab, vofatamab, ramucirumab, rituximab, obinutuzumab, daratumumab, and 1C1(Clone name), but is not limited thereto.

As a specific example of the present invention, it may include an antigen-binding site that specifically binds to EGFR. For example, it may comprise a heavy chain variable region comprising H-CDR1 represented by SEQ ID NO: 175, H-CDR2 represented by SEQ ID NO: 176, and H-CDR3 represented by SEQ ID NO: 177 of cetuximab, and may comprise a light chain variable region comprising L-CDR1 represented by SEQ ID NO: 178, L-CDR2 represented by SEQ ID NO: 179, and L-CDR3 represented by SEQ ID NO: 180. As another example, it may comprise a heavy chain variable region comprising H-CDR1 represented by SEQ ID NO: 181, H-CDR2 represented by SEQ ID NO: 182, and H-CDR3 represented by SEQ ID NO: 183 of panitumumab, and may comprise a light chain variable region comprising L-CDR1 represented by SEQ ID NO: 184, L-CDR2 represented by SEQ ID NO: 185, and L-CDR3 represented by SEQ ID NO: 186. As another example, it may comprise a heavy chain variable region comprising H-CDR1 represented by SEQ ID NO: 187, H-CDR2 represented by SEQ ID NO: 188, and H-CDR3 represented by SEQ ID NO: 189 of necitumumab, and may comprise a light chain variable region comprising L-CDR1 represented by SEQ ID NO: 190, L-CDR2 represented by SEQ ID NO: 191, and L-CDR3 represented by SEQ ID NO: 192. In addition, it may comprise a heavy chain variable region comprising H-CDR1 represented by SEQ ID NO: 193, H-CDR2 represented by SEQ ID NO: 194, and H-CDR3 represented by SEQ ID NO: 195 of imgatuzumab, and may comprise a light chain variable region comprising L-CDR1 represented by SEQ ID NO: 196, L-CDR2 represented by SEQ ID NO: 197, and L-CDR3 represented by SEQ ID NO: 198. In addition, it may comprise a heavy chain variable region comprising H-CDR1 represented by SEQ ID NO: 199, H-CDR2 represented by SEQ ID NO: 200, and H-CDR3 represented by SEQ ID NO: 201 of depatuxizumab, and may comprise a light chain variable region comprising L-CDR1 represented by SEQ ID NO: 202, L-CDR2 represented by SEQ ID NO: 203, and L-CDR3 represented by SEQ ID NO: 204. In addition, it may comprise a heavy chain variable region comprising H-CDR1 represented by SEQ ID NO: 199, H-CDR2 represented by SEQ ID NO: 205, and H-CDR3 represented by SEQ ID NO: 206 of losatuxizumab, and may comprise a light chain variable region comprising L-CDR1 represented by SEQ ID NO: 202, L-CDR2 represented by SEQ ID NO: 203, and L-CDR3 represented by SEQ ID NO: 204.

As a specific example of the present invention, it may include an antigen-binding site that specifically binds to EGFRvIII. For example, it may comprise a heavy chain variable region comprising H-CDR1 represented by SEQ ID NO: 207, H-CDR2 represented by SEQ ID NO: 208, and H-CDR3 represented by SEQ ID NO: 209 of etevritamab, and may comprise a light chain variable region comprising L-CDR1 represented by SEQ ID NO: 210, L-CDR2 represented by SEQ ID NO: 211, and L-CDR3 represented by SEQ ID NO: 212. In addition, it may comprise a heavy chain variable region comprising H-CDR1 represented by SEQ ID NO: 213, H-CDR2 represented by SEQ ID NO: 214, and H-CDR3 represented by SEQ ID NO: 215 of AMG-595, and may comprise a light chain variable region comprising L-CDR1 represented by SEQ ID NO: 210, L-CDR2 represented by SEQ ID NO: 216, and L-CDR3 represented by SEQ ID NO: 217.

As a specific example of the present invention, it may include an antigen-binding site that specifically binds to PD-L1. For example, it may comprise a heavy chain variable region comprising H-CDR1 represented by SEQ ID NO: 218, H-CDR2 represented by SEQ ID NO: 219, and H-CDR3 represented by SEQ ID NO: 220 of atezolizumab, and may comprise a light chain variable region comprising L-CDR1 represented by SEQ ID NO: 221, L-CDR2 represented by SEQ ID NO: 222, and L-CDR3 represented by SEQ ID NO: 223. In addition, it may comprise a heavy chain variable region comprising H-CDR1 represented by SEQ ID NO: 224, H-CDR2 represented by SEQ ID NO: 225, and H-CDR3 represented by SEQ ID NO: 226 of avelumab, and may comprise a light chain variable region comprising L-CDR1 represented by SEQ ID NO: 227, L-CDR2 represented by SEQ ID NO: 228, and L-CDR3 represented by SEQ ID NO: 229. In addition, it may comprise a heavy chain variable region comprising H-CDR1 represented by SEQ ID NO: 230, H-CDR2 represented by SEQ ID NO: 231, and H-CDR3 represented by SEQ ID NO: 232 of durvalumab, and may comprise a light chain variable region comprising L-CDR1 represented by SEQ ID NO: 233, L-CDR2 represented by SEQ ID NO: 234, and L-CDR3 represented by SEQ ID NO: 235.

As a specific example of the present invention, it may include an antigen-binding site that specifically binds to HER2. For example, it may comprise a heavy chain variable region comprising H-CDR1 represented by SEQ ID NO: 21, H-CDR2 represented by SEQ ID NO: 22, and H-CDR3 represented by SEQ ID NO: 23 of trastuzumab, and may comprise a light chain variable region comprising L-CDR1 represented by SEQ ID NO: 24, L-CDR2 represented by SEQ ID NO: 25, and L-CDR3 represented by SEQ ID NO: 26. In addition, it may comprise a heavy chain variable region comprising H-CDR1 represented by SEQ ID NO: 33, H-CDR2 represented by SEQ ID NO: 34, and H-CDR3 represented by SEQ ID NO: 35 of pertuzumab, and may comprise a light chain variable region comprising L-CDR1 represented by SEQ ID NO: 36, L-CDR2 represented by SEQ ID NO: 37, and L-CDR3 represented by SEQ ID NO: 38.

As a specific example of the present invention, it may include an antigen-binding site that specifically binds to c-Met. For example, it may comprise a heavy chain variable region comprising H-CDR1 represented by SEQ ID NO: 236, H-CDR2 represented by SEQ ID NO: 237, and H-CDR3 represented by SEQ ID NO: 238 of onartuzumab, and may comprise a light chain variable region comprising L-CDR1 represented by SEQ ID NO: 239, L-CDR2 represented by SEQ ID NO: 240, and L-CDR3 represented by SEQ ID NO: 241. In addition, it may comprise a heavy chain variable region comprising H-CDR1 represented by SEQ ID NO: 242, H-CDR2 represented by SEQ ID NO: 243, and H-CDR3 represented by SEQ ID NO: 244 of emibetuzumab, and may comprise a light chain variable region comprising L-CDR1 represented by SEQ ID NO: 245, L-CDR2 represented by SEQ ID NO: 246, and L-CDR3 represented by SEQ ID NO: 247. In addition, it may comprise a heavy chain variable region comprising H-CDR1 represented by SEQ ID NO: 248, H-CDR2 represented by SEQ ID NO: 249, and H-CDR3 represented by SEQ ID NO: 250 of telisotuzumab, and may comprise a light chain variable region comprising L-CDR1 represented by SEQ ID NO: 251, L-CDR2 represented by SEQ ID NO: 252, and L-CDR3 represented by SEQ ID NO: 253.

As a specific example of the present invention, it may include an antigen-binding site that specifically binds to Trop-2. For example, it may comprise a heavy chain variable region comprising H-CDR1 represented by SEQ ID NO: 254, H-CDR2 represented by SEQ ID NO: 255, and H-CDR3 represented by SEQ ID NO: 256 of datopotamab, and may comprise a light chain variable region comprising L-CDR1 represented by SEQ ID NO: 257, L-CDR2 represented by SEQ ID NO: 258, and L-CDR3 represented by SEQ ID NO: 259. In addition, it may comprise a heavy chain variable region comprising H-CDR1 represented by SEQ ID NO: 260, H-CDR2 represented by SEQ ID NO: 261, and H-CDR3 represented by SEQ ID NO: 262 of sacituzumab, and may comprise a light chain variable region comprising L-CDR1 represented by SEQ ID NO: 263, L-CDR2 represented by SEQ ID NO: 264, and L-CDR3 represented by SEQ ID NO: 265.

As a specific example of the present invention, it may include an antigen-binding site that specifically binds to DLL3. For example, it may comprise a heavy chain variable region comprising H-CDR1 represented by SEQ ID NO: 266, H-CDR2 represented by SEQ ID NO: 267, and H-CDR3 represented by SEQ ID NO: 268 of rovalpituzumab, and may comprise a light chain variable region comprising L-CDR1 represented by SEQ ID NO: 269, L-CDR2 represented by SEQ ID NO: 270, and L-CDR3 represented by SEQ ID NO: 271. In addition, it may comprise a heavy chain variable region comprising H-CDR1 represented by SEQ ID NO: 272, H-CDR2 represented by SEQ ID NO: 273, and H-CDR3 represented by SEQ ID NO: 274 of tarlatamab, and may comprise a light chain variable region comprising L-CDR1 represented by SEQ ID NO: 275, L-CDR2 represented by SEQ ID NO: 276, and L-CDR3 represented by SEQ ID NO: 277.

As a specific example of the present invention, it may include an antigen-binding site that specifically binds to BCMA. For example, it may comprise a heavy chain variable region comprising H-CDR1 represented by SEQ ID NO: 278, H-CDR2 represented by SEQ ID NO: 279, and H-CDR3 represented by SEQ ID NO: 280 of belantamab, and may comprise a light chain variable region comprising L-CDR1 represented by SEQ ID NO: 281, L-CDR2 represented by SEQ ID NO: 282, and L-CDR3 represented by SEQ ID NO: 283.

As a specific example of the present invention, it may include an antigen-binding site that specifically binds to LIV-1. For example, it may comprise a heavy chain variable region comprising H-CDR1 represented by SEQ ID NO: 284, H-CDR2 represented by SEQ ID NO: 285, and H-CDR3 represented by SEQ ID NO: 286 of ladiratuzumab, and may comprise a light chain variable region comprising L-CDR1 represented by SEQ ID NO: 287, L-CDR2 represented by SEQ ID NO: 288, and L-CDR3 represented by SEQ ID NO: 289.

As a specific example of the present invention, it may include an antigen-binding site that specifically binds to GPC-3. For example, it may comprise a heavy chain variable region comprising H-CDR1 represented by SEQ ID NO: 99, H-CDR2 represented by SEQ ID NO: 100, and H-CDR3 represented by SEQ ID NO: 101 of codrituzumab, and may comprise a light chain variable region comprising L-CDR1 represented by SEQ ID NO: 102, L-CDR2 represented by SEQ ID NO: 103, and L-CDR3 represented by SEQ ID NO: 104.

As a specific example of the present invention, it may include an antigen-binding site that specifically binds to FGFR2. For example, it may comprise a heavy chain variable region comprising H-CDR1 represented by SEQ ID NO: 290, H-CDR2 represented by SEQ ID NO: 291, and H-CDR3 represented by SEQ ID NO: 292 of aprutumab, and may comprise a light chain variable region comprising L-CDR1 represented by SEQ ID NO: 293, L-CDR2 represented by SEQ ID NO: 294, and L-CDR3 represented by SEQ ID NO: 295. In addition, it may comprise a heavy chain variable region comprising H-CDR1 represented by SEQ ID NO: 296, H-CDR2 represented by SEQ ID NO: 297, and H-CDR3 represented by SEQ ID NO: 298 of bemarituzumab, and may comprise a light chain variable region comprising L-CDR1 represented by SEQ ID NO: 299, L-CDR2 represented by SEQ ID NO: 300, and L-CDR3 represented by SEQ ID NO: 301.

As a specific example of the present invention, it may include an antigen-binding site that specifically binds to FGFR3. For example, it may comprise a heavy chain variable region comprising H-CDR1 represented by SEQ ID NO: 302, H-CDR2 represented by SEQ ID NO: 303, and H-CDR3 represented by SEQ ID NO: 304 of vofatamab, and may comprise a light chain variable region comprising L-CDR1 represented by SEQ ID NO: 305, L-CDR2 represented by SEQ ID NO: 306, and L-CDR3 represented by SEQ ID NO: 307.

As a specific example of the present invention, it may include an antigen-binding site that specifically binds to VEGFR2. For example, it may comprise a heavy chain variable region comprising H-CDR1 represented by SEQ ID NO: 308, H-CDR2 represented by SEQ ID NO: 309, and H-CDR3 represented by SEQ ID NO: 310 of ramucirumab, and may comprise a light chain variable region comprising L-CDR1 represented by SEQ ID NO: 311, L-CDR2 represented by SEQ ID NO: 312, and L-CDR3 represented by SEQ ID NO: 313.

As a specific example of the present invention, it may include an antigen-binding site that specifically binds to CD20. For example, it may comprise a heavy chain variable region comprising H-CDR1 represented by SEQ ID NO: 314, H-CDR2 represented by SEQ ID NO: 315, and H-CDR3 represented by SEQ ID NO: 316 of rituximab, and may comprise a light chain variable region comprising L-CDR1 represented by SEQ ID NO: 317, L-CDR2 represented by SEQ ID NO: 318, and L-CDR3 represented by SEQ ID NO: 319. For example, it may comprise a heavy chain variable region comprising H-CDR1 represented by SEQ ID NO: 320, H-CDR2 represented by SEQ ID NO: 321, and H-CDR3 represented by SEQ ID NO: 322 of obinutuzumab, and may comprise a light chain variable region comprising L-CDR1 represented by SEQ ID NO: 323, L-CDR2 represented by SEQ ID NO: 324, and L-CDR3 represented by SEQ ID NO: 325.

As a specific example of the present invention, it may include an antigen-binding site that specifically binds to CD38. For example, it may comprise a heavy chain variable region comprising H-CDR1 represented by SEQ ID NO: 326, H-CDR2 represented by SEQ ID NO: 327, and H-CDR3 represented by SEQ ID NO: 328 of daratumumab, and may comprise a light chain variable region comprising L-CDR1 represented by SEQ ID NO: 329, L-CDR2 represented by SEQ ID NO: 330, and L-CDR3 represented by SEQ ID NO: 331.

As a specific example of the present invention, it may include an antigen-binding site that specifically binds to EphA2. For example, it may comprise a heavy chain variable region comprising H-CDR1 represented by SEQ ID NO: 157, H-CDR2 represented by SEQ ID NO: 158, and H-CDR3 represented by SEQ ID NO: 159 of 1C1, and may comprise a light chain variable region comprising L-CDR1 represented by SEQ ID NO: 160, L-CDR2 represented by SEQ ID NO: 161, and L-CDR3 represented by SEQ ID NO: 162.

Table 1 below shows the CDR sequences of non-limiting exemplary antibodies with anticancer efficacy that may be used in specific examples of the present invention.

**[Table 1]**

| Antibody | Heavy chain | | | Light chain | | |
|---|---|---|---|---|---|---|
| | CDR | Sequence | SEQ ID NO | CDR | Sequence | SEQ ID NO |
| Cetuximab | VH-CDR1 | NYGVH | 175 | VL-CDR1 | RASQSIGTNIH | 178 |
| | VH-CDR2 | VIWSGGNTDYNTPF TS | 176 | VL-CDR2 | YASESIS | 179 |
| | VH-CDR3 | ALTYYDYEFAY | 177 | VL-CDR3 | QQNNNWPTT | 180 |
| Panitumumab | VH-CDR1 | DYYWT | 181 | VL-CDR1 | QASQDISNYLN | 184 |
| | VH-CDR2 | HIYYSGNTNYNPSL KS | 182 | VL-CDR2 | DASNLET | 185 |
| | VH-CDR3 | DRVTGAFDI | 183 | VL-CDR3 | QHFDHLPLA | 186 |
| Necitumumab | VH-CDR1 | DYYWS | 187 | VL-CDR1 | RASQSVSSYLA | 190 |
| | VH-CDR2 | YIYYSGSTDYNPSL KS | 188 | VL-CDR2 | DASNRAT | 191 |
| | VH-CDR3 | VSIFGVGTFDY | 189 | VL-CDR3 | HQYGSTPLT | 192 |
| Imgatuzumab | VH-CDR1 | DYKIH | 193 | VL-CDR1 | RASQGINNYLN | 196 |
| | VH-CDR2 | YFNPNSGYSTYAQK FQG | 194 | VL-CDR2 | NTNNLQT | 197 |
| | VH-CDR3 | LSPGGYYVMDA | 195 | VL-CDR3 | LQHNSFPT | 198 |
| Depatuxizumab | VH-CDR1 | DFAWN | 199 | VL-CDR1 | HSSQDINSNIG | 202 |
| | VH-CDR2 | YISYSGNTRYQPSL | 200 | VL-CDR2 | HGTNLDD | 203 |
| | | KS | | | | |
| | VH-CDR3 | AGRGFPY | 201 | VL-CDR3 | VQYAQFPWT | 204 |
| Losatuxizumab | VH-CDR1 | DFAWN | 199 | VL-CDR1 | HSSQDINSNIG | 202 |
| | VH-CDR2 | YISYNGNTRYQPSL KS | 205 | VL-CDR2 | HGTNLDD | 203 |
| | VH-CDR3 | ASRGFPY | 206 | VL-CDR3 | VQYAQFPWT | 204 |
| Etevritamab | VH-CDR1 | NYGMH | 207 | VL-CDR1 | RSSQSLVHSDG NTYLS | 210 |
| | VH-CDR2 | VIWYDGSDKYYAD SVRG | 208 | VL-CDR2 | RISRRFS | 211 |
| | VH-CDR3 | DGYDILTGNPRDFD Y | 209 | VL-CDR3 | MQSTHVPRT | 212 |
| AMG-595 | VH-CDR1 | SYGMH | 213 | VL-CDR1 | RSSQSLVHSDG NTYLS | 210 |
| | VH-CDR2 | VIWYDGSNKYYVD SVKG | 214 | VL-CDR2 | KISNRFS | 216 |
| | VH-CDR3 | DGWQQLAPFDY | 215 | VL-CDR3 | MQATQLPRT | 217 |
| Atezolizumab | VH-CDR1 | DSWIH | 218 | VL-CDR1 | RASQDVSTAVA | 221 |
| | VH-CDR2 | WISPYGGSTYYADS VKG | 219 | VL-CDR2 | SASFLYS | 222 |
| | VH-CDR3 | RHWPGGFDY | 220 | VL-CDR3 | QQYLYHPAT | 223 |
| Avelumab | VH-CDR1 | SSYIM | 224 | VL-CDR1 | TGTSSDVGGYN YVS | 227 |
| | VH-CDR2 | SIYPSGGITFYADTV KG | 225 | VL-CDR2 | DVSNRPS | 228 |
| | VH-CDR3 | IKLGTVTTVDY | 226 | VL-CDR3 | SSYTSSSTRV | 229 |
| Durvalumab | VH-CDR1 | RYWMS | 230 | VL-CDR1 | RASQRVSSSYL A | 233 |
| | VH-CDR2 | NIKQDGSEKYYVDS VKG | 231 | VL-CDR2 | DASSRAT | 234 |
| | VH-CDR3 | EGGWFGELAFDY | 232 | VL-CDR3 | QQYGSLPWT | 235 |
| Trastuzumab | VH-CDR1 | DTYIH | 21 | VL-CDR1 | RASQDVNTAV A | 24 |
| | VH-CDR2 | RIYPTNGYTRYADS VKG | 22 | VL-CDR2 | SASFLYS | 25 |
| | VH-CDR3 | WGGDGFYAMDY | 23 | VL-CDR3 | QQHYTTPPT | 26 |
| Pertuzumab | VH-CDR1 | DYTMD | 33 | VL-CDR1 | KASQDVSIGVA | 36 |
| | VH-CDR2 | DVNPNSGGSIYNQR FKG | 34 | VL-CDR2 | SASYRYT | 37 |
| | VH-CDR3 | NLGPSFYFDYW | 35 | VL-CDR3 | QQYYIYPYT | 38 |
| Onartuzumab | VH-CDR1 | SYWLH | 236 | VL-CDR1 | KSSQSLLYTSS QKNYLA | 239 |
| | VH-CDR2 | MIDPSNSDTRFNPN FKD | 237 | VL-CDR2 | WASTRES | 240 |
| | VH-CDR3 | YRSYVTPLDY | 238 | VL-CDR3 | QQYYAYPWT | 241 |
| Emibetuzumab | VH-CDR1 | DYYMH | 242 | VL-CDR1 | SVSSSVSSIYLH | 245 |
| | VH-CDR2 | RVNPNRRGTTYNQ KFEG | 243 | VL-CDR2 | STSNLAS | 246 |
| | VH-CDR3 | ANWLDY | 244 | VL-CDR3 | QVYSGYPLT | 247 |
| Telisotuzumab | VH-CDR1 | AYTMH | 248 | VL-CDR1 | KSSESVDSYAN SFLH | 251 |
| | VH-CDR2 | WIKPNNGLANYAQ KFQG | 249 | VL-CDR2 | RASTRES | 252 |
| | VH-CDR3 | SEITTEFDY | 250 | VL-CDR3 | QQSKEDPLT | 253 |
| Datopotamab | VH-CDR1 | TAGMQ | 254 | VL-CDR1 | KASQDVSTAV A | 257 |
| | VH-CDR2 | WINTHSGVPKYAE DFKG | 255 | VL-CDR2 | SASYRYT | 258 |
| | VH-CDR3 | SGFGSSYWYFDV | 256 | VL-CDR3 | QQHYITPLT | 259 |
| Sacituzumab | VH-CDR1 | NYGMN | 260 | VL-CDR1 | KASQDVSIAVA | 263 |
| | VH-CDR2 | WINTYTGEPTYTDD FKG | 261 | VL-CDR2 | SASYRYT | 264 |
| | VH-CDR3 | GGFGSSYWYFDV | 262 | VL-CDR3 | QQHYITPLT | 265 |
| Rovalpituzuma b | VH-CDR1 | NYGMN | 266 | VL-CDR1 | KASQSVSNDW | 269 |
| | VH-CDR2 | WINTYTGEPTYADD FKG | 267 | VL-CDR2 | YASNRYT | 270 |
| | VH-CDR3 | IGDSSPSDY | 268 | VL-CDR3 | QQDYTSPWT | 271 |
| Tarlatamab | VH-CDR1 | SYYWS | 272 | VL-CDR1 | RASQRVNNNY LA | 275 |
| | VH-CDR2 | YVYYSGTTNYNPSL KS | 273 | VL-CDR2 | GASSRAT | 276 |
| | VH-CDR3 | IAVTGFYFDY | 274 | VL-CDR3 | QQYDRSPLT | 277 |
| Belantamab | VH-CDR1 | NYWMH | 278 | VL-CDR1 | SASQDISNYLN | 281 |
| | VH-CDR2 | ATYRGHSDTYYNQ KFKG | 279 | VL-CDR2 | YTSNLHS | 282 |
| | VH-CDR3 | GAIYDGYDVLDN | 280 | VL-CDR3 | QQYRKLPWT | 283 |
| Ladiratuzumab | VH-CDR1 | DYYMH | 284 | VL-CDR1 | RSSQSLLHSSG NTYLE | 287 |
| | VH-CDR2 | WIDPENGDTEYGPK FQG | 285 | VL-CDR2 | KISTRFS | 288 |
| | VH-CDR3 | HNAHYGTWFAY | 286 | VL-CDR3 | FQGSHVPYT | 289 |
| Codrituzumab | VH-CDR1 | DYEMH | 99 | VL-CDR1 | RSSQSLVHSNR NTYLH | 102 |
| | VH-CDR2 | ALDPKTGDTAYSQ KFKG | 100 | VL-CDR2 | KVSNRFS | 103 |
| | VH-CDR3 | FYSYTY | 101 | VL-CDR3 | SQNTHVPPT | 104 |
| Aprutumab | VH-CDR1 | SYAMS | 290 | VL-CDR1 | SGSSSNIGNNY VS | 293 |
| | VH-CDR2 | AISGSGTSTYYADS VKG | 291 | VL-CDR2 | ENYNRPA | 294 |
| | VH-CDR3 | VRYNWNHGDWFD P | 292 | VL-CDR3 | SSWDDSLNYW V | 295 |
| Bemarituzumab | VH-CDR1 | TYNVH | 296 | VL-CDR1 | KASQGVSNDV A | 299 |
| | VH-CDR2 | SIYPDNGDTSYNQN FKG | 297 | VL-CDR2 | SASYRYT | 300 |
| | VH-CDR3 | GDFAY | 298 | VL-CDR3 | QQHSTTPYT | 301 |
| Vofatamab | VH-CDR1 | STGIS | 302 | VL-CDR1 | RASQDVDTSLA | 305 |
| | VH-CDR2 | RIYPTSGSTNYADS VKG | 303 | VL-CDR2 | SASFLYS | 306 |
| | VH-CDR3 | TYGIYDLYVDYTEY VMDY | 304 | VL-CDR3 | QQSTGHPQT | 307 |
| Ramucirumab | VH-CDR1 | SYSMN | 308 | VL-CDR1 | RASQGIDNWLG | 311 |
| | VH-CDR2 | SISSSSSYIYYADSV KG | 309 | VL-CDR2 | DASNLDT | 312 |
| | VH-CDR3 | VTDAFDI | 310 | VL-CDR3 | QQAKAFPPT | 313 |
| Rituximab | VH-CDR1 | SYNMH | 314 | VL-CDR1 | RSSKSLLHSNGI TYLY | 317 |
| | VH-CDR2 | AIYPGNGDTSYNQK FKG | 315 | VL-CDR2 | QMSNL VS | 318 |
| | VH-CDR3 | STYYGGDWYFNV | 316 | VL-CDR3 | AQNLELPYT | 319 |
| Obinutuzumab | VH-CDR1 | YSWIN | 320 | VL-CDR1 | RASQSVSSYLA | 323 |
| | VH-CDR2 | RIFPGDGDTDYNGK FKG | 321 | VL-CDR2 | DASNRAT | 324 |
| | VH-CDR3 | NVFDGYWLVY | 322 | VL-CDR3 | QQRSNWPPT | 325 |
| Daratumumab | VH-CDR1 | SFAMS | 326 | VL-CDR1 | RASQSVSSYLA | 329 |
| | VH-CDR2 | AISGSGGGTYYADS VKG | 327 | VL-CDR2 | DASNRAT | 330 |
| | VH-CDR3 | DKILWFGEPVFDY | 328 | VL-CDR3 | QQRSNWPPT | 331 |
| 1C1 (Clone name) | VH-CDR1 | HYMMA | 157 | VL-CDR1 | RASQSISTWLA | 160 |
| | VH-CDR2 | RIGPSGGPTHYADS VKG | 158 | VL-CDR2 | KASNLHT | 161 |
| | VH-CDR3 | YDSGYDYVAVAGP AEYFQH | 159 | VL-CDR3 | QQYNSYSRT | 162 |

### Antigen

As antigens to which the antigen-binding site described herein may specifically bind, the following non-limiting substances may be exemplified.

"Epidermal growth factor receptor (EGFR)": It is a cell membrane receptor that regulates cell growth, division, survival, and death. In various cancers, the expression of EGFR is increased in tumor tissues. It is known that tumor tissues with the increased EGFR are invasive, metastatic, and highly resistant to anticancer agents. In one embodiment, the substance that inhibits EGFR may be cetuximab, panitumumab, necitumumab, imgatuzumab, depatuxizumab, or losatuxizumab, but is not limited thereto.

"Epidermal growth factor receptor variant 3 (EGFRvIII)": It is a mutation in which exons 2 to 7 of EGFR are deleted. It is mainly reported in glioblastoma multiforme, and most patients with EGFRvIII-positive mutation have a poor prognosis. In one embodiment, the substance that inhibits EGFRvIII may be cetuximab, panitumumab, necitumumab, imgatuzumab, depatuxizumab, losatuxizumab, etevritamab, or AMG-595, but is not limited thereto.

Programmed death-ligand 1 (PD-L1)": It is a protein overexpressed on the surface of cancer cells. It is a major cancer-specific antigen that plays an important role in inducing exhaustion and apoptosis of T cells and acquiring immune tolerance in cancer cells. In one embodiment, PD-L1 targeting anticancer antibody may be atezolizumab, avelumab, and durvalumab, but is not limited thereto.

"HER-2/neu (human epidermal growth factor receptor 2): It regulates cell proliferation through activation of PI3K/AkT. It is known that it is overexpressed in metastatic breast cancer and ovarian cancer, etc., and induces resistance against anticancer agents. The HER2/neu targeting anticancer agent may be trastuzumab or pertuzumab, but is not limited thereto.

"c-Met (mesenchymal-epithelial transition factor)": It is a hepatocyte growth factor receptor. Its amplification or mutation is frequently reported in cancer cells, and it is known to promote tumor growth, metastasis, and malignancy. Specifically, the inhibitor of the protein may be onartuzumab, emibetuzumab, or telisotuzumab, but is not limited thereto.

"Trop-2 (tumor-associated calcium signal transducer 2)": It is a cellular glycoprotein related to cancer cell growth and proliferation. It is known to be specifically overexpressed in non-small cell lung cancer and breast cancer. Specifically, the antibody targeting the protein may be datopotamab or sacituzumab, but is not limited thereto.

"DLL3 (delta-like ligand 3)": It is a major cancer target antigen known to be expressed in approximately 85% of small cell lung cancer patients. Specifically, the antibody targeting the protein may be rovalpituzumab or tarlatamab, but is not limited thereto.

"BCMA (B-cell maturation antigen)": It is an important factor in the survival and proliferation of myeloma cells and is a clinically proven target for treatment of multiple myeloma. Specifically, the antibody targeting the protein may be belantamab, but is not limited thereto.

"LIV-1 (zinc transporter ZIP6)": It is a highly cancer-specific antigen overexpressed in breast cancer. Specifically, the antibody targeting the protein may be ladiratuzumab, but is not limited thereto.

"GPC-3 (glypican-3)": It is a highly cancer-specific antigen that is specifically overexpressed in liver cancer. Specifically, the antibody targeting the protein may be codrituzumab, but is not limited thereto.

"FGFR (fibroblast growth factor receptor)": It is a receptor for fibroblast growth factor (FGF), which regulates various biological processes including cell growth, differentiation, and migration. The FGFR gene is easily mutated, and these variants are commonly observed in breast cancer, uterine cancer, ovarian cancer, cervical cancer, and the like. The four FGFR genes are made of seven signaling receptors, of which FGFR2 and FGFR3 are highly cancer-specific antigens. The antibody targeting FGFR2 or FGFR3 may be aprutumab, bemarituzumab, or vofatamab, but is not limited thereto.

"Vascular endothelial growth factor receptor (VEGFR)": It is a cell membrane receptor for a vascular endothelial growth factor that induces angiogenesis. The VEGFR inhibitor inhibits tumor growth and metastasis by inhibiting angiogenesis. In one embodiment, the VEGFR inhibitor may be ramucirumab, but is not limited thereto.

"CD20 (B lymphocyte antigen CD20)": It is a protein expressed on the surface of B cells, which is used as a target protein for the treatment of B cell lymphoma. The CD20 inhibitor may be rituximab or obinutuzumab, but is not limited thereto.

"CD38 (cluster of differentiation 38)": It is a protein that acts as a signal transduction receptor in immune cells and regulates cell proliferation and death. The inhibitor targeting the protein may be daratumumab, but is not limited thereto.

"EphA2 (EPH receptor A2)": It is overexpressed in cancer cells and has a significant impact on the growth and metastasis of cancer cells. The antibody targeting the protein may be 1C1, but is not limited thereto.

The antigen-binding site that specifically binds to the antigens exemplified above may include CDR sequences as exemplified below.

### EGFR:

1) VH region (SEQ ID NO: 334) comprising the amino acid sequences of SEQ ID NO: 175 (VH-CDR1), SEQ ID NO: 176 (VH-CDR2) and SEQ ID NO: 177 (VH-CDR3), and VL region (SEQ ID NO: 335) comprising the amino acid sequences of SEQ ID NO: 178 (VL-CDR1), SEQ ID NO: 179 (VL-CDR2) and SEQ ID NO: 180 (VL-CDR3);
2) VH region (SEQ ID NO: 336) comprising the amino acid sequences of SEQ ID NO: 181 (VH-CDR1), SEQ ID NO: 182 (VH-CDR2) and SEQ ID NO: 183 (VH-CDR3), and VL region (SEQ ID NO: 337) comprising the amino acid sequences of SEQ ID NO: 184 (VL-CDR1), SEQ ID NO: 185 (VL-CDR2) and SEQ ID NO: 186 (VL-CDR3);
3) VH region (SEQ ID NO: 338) comprising the amino acid sequences of SEQ ID NO: 187 (VH-CDR1), SEQ ID NO: 188 (VH-CDR2) and SEQ ID NO: 189 (VH-CDR3), and VL region (SEQ ID NO: 339) comprising the amino acid sequences of SEQ ID NO: 190 (VL-CDR1), SEQ ID NO: 191 (VL-CDR2) and SEQ ID NO: 192 (VL-CDR3);
4) VH region (SEQ ID NO: 340) comprising the amino acid sequences of SEQ ID NO: 193 (VH-CDR1), SEQ ID NO: 194 (VH-CDR2) and SEQ ID NO: 195 (VH-CDR3), and VL region (SEQ ID NO: 341) comprising the amino acid sequences of SEQ ID NO: 196 (VL-CDR1), SEQ ID NO: 197 (VL-CDR2) and SEQ ID NO: 198 (VL-CDR3);
5) VH region (SEQ ID NO: 342) comprising the amino acid sequences of SEQ ID NO: 199 (VH-CDR1), SEQ ID NO: 200 (VH-CDR2) and SEQ ID NO: 201 (VH-CDR3), and VL region (SEQ ID NO: 343) comprising the amino acid sequences of SEQ ID NO: 202 (VL-CDR1), SEQ ID NO: 203 (VL-CDR2) and SEQ ID NO: 204 (VL-CDR3);
6) VH region (SEQ ID NO: 344) comprising the amino acid sequences of SEQ ID NO: 199 (VH-CDR1), SEQ ID NO: 205 (VH-CDR2) and SEQ ID NO: 206 (VH-CDR3), and VL region (SEQ ID NO: 345) comprising the amino acid sequences of SEQ ID NO: 202 (VL-CDR1), SEQ ID NO: 203 (VL-CDR2) and SEQ ID NO: 204 (VL-CDR3);

### EGFRvIII:

7) VH region (SEQ ID NO: 346) comprising the amino acid sequences of SEQ ID NO: 207 (VH-CDR1), SEQ ID NO: 208 (VH-CDR2) and SEQ ID NO: 209 (VH-CDR3), and VL region (SEQ ID NO: 347) comprising the amino acid sequences of SEQ ID NO: 210 (VL-CDR1), SEQ ID NO: 211 (VL-CDR2) and SEQ ID NO: 212 (VL-CDR3);
8) VH region (SEQ ID NO: 348) comprising the amino acid sequences of SEQ ID NO: 213 (VH-CDR1), SEQ ID NO: 214 (VH-CDR2) and SEQ ID NO: 215 (VH-CDR3), and VL region (SEQ ID NO: 349) comprising the amino acid sequences of SEQ ID NO: 210 (VL-CDR1), SEQ ID NO: 216 (VL-CDR2) and SEQ ID NO: 217 (VL-CDR3);

### PD-L1:

9) VH region (SEQ ID NO: 350) comprising the amino acid sequences of SEQ ID NO: 218 (VH-CDR1), SEQ ID NO: 219 (VH-CDR2) and SEQ ID NO: 220 (VH-CDR3), and VL region (SEQ ID NO: 351) comprising the amino acid sequences of SEQ ID NO: 221 (VL-CDR1), SEQ ID NO: 222 (VL-CDR2) and SEQ ID NO: 223 (VL-CDR3);
10) VH region (SEQ ID NO: 352) comprising the amino acid sequences of SEQ ID NO: 224 (VH-CDR1), SEQ ID NO: 225 (VH-CDR2) and SEQ ID NO: 226 (VH-CDR3), and VL region (SEQ ID NO: 353) comprising the amino acid sequences of SEQ ID NO: 227 (VL-CDR1), SEQ ID NO: 228 (VL-CDR2) and SEQ ID NO: 229 (VL-CDR3);
11) VH region (SEQ ID NO: 354) comprising the amino acid sequences of SEQ ID NO: 230 (VH-CDR1), SEQ ID NO: 231 (VH-CDR2) and SEQ ID NO: 232 (VH-CDR3), and VL region (SEQ ID NO: 355) comprising the amino acid sequences of SEQ ID NO: 233 (VL-CDR1), SEQ ID NO: 234 (VL-CDR2) and SEQ ID NO: 235 (VL-CDR3);

### HER2:

12) VH region (SEQ ID NO: 356) comprising the amino acid sequences of SEQ ID NO: 21 (VH-CDR1), SEQ ID NO: 22 (VH-CDR2) and SEQ ID NO: 23 (VH-CDR3), and VL region (SEQ ID NO: 357) comprising the amino acid sequences of SEQ ID NO: 24 (VL-CDR1), SEQ ID NO: 25 (VL-CDR2) and SEQ ID NO: 26 (VL-CDR3);
13) VH region (SEQ ID NO: 27) comprising the amino acid sequences of SEQ ID NO: 33 (VH-CDR1), SEQ ID NO: 34 (VH-CDR2) and SEQ ID NO: 35 (VH-CDR3), and VL region (SEQ ID NO: 28) comprising the amino acid sequences of SEQ ID NO: 36 (VL-CDR1), SEQ ID NO: 37 (VL-CDR2) and SEQ ID NO: 38 (VL-CDR3);

### c-Met:

14) VH region (SEQ ID NO: 358) comprising the amino acid sequences of SEQ ID NO: 236 (VH-CDR1), SEQ ID NO: 237 (VH-CDR2) and SEQ ID NO: 238 (VH-CDR3), and VL region (SEQ ID NO: 359) comprising the amino acid sequences of SEQ ID NO: 239 (VL-CDR1), SEQ ID NO: 240 (VL-CDR2) and SEQ ID NO: 241 (VL-CDR3);
15) VH region (SEQ ID NO: 360) comprising the amino acid sequences of SEQ ID NO: 242 (VH-CDR1), SEQ ID NO: 243 (VH-CDR2) and SEQ ID NO: 244 (VH-CDR3), and VL region (SEQ ID NO: 361) comprising the amino acid sequences of SEQ ID NO: 245 (VL-CDR1), SEQ ID NO: 246 (VL-CDR2) and SEQ ID NO: 247 (VL-CDR3);
16) VH region (SEQ ID NO: 362) comprising the amino acid sequences of SEQ ID NO: 248 (VH-CDR1), SEQ ID NO: 249 (VH-CDR2) and SEQ ID NO: 250 (VH-CDR3), and VL region (SEQ ID NO: 363) comprising the amino acid sequences of SEQ ID NO: 251 (VL-CDR1), SEQ ID NO: 252 (VL-CDR2) and SEQ ID NO: 253 (VL-CDR3);

### Trop-2:

17) VH region (SEQ ID NO: 364) comprising the amino acid sequences of SEQ ID NO: 254 (VH-CDR1), SEQ ID NO: 255 (VH-CDR2) and SEQ ID NO: 256 (VH-CDR3), and VL region (SEQ ID NO: 365) comprising the amino acid sequences of SEQ ID NO: 257 (VL-CDR1), SEQ ID NO: 258 (VL-CDR2) and SEQ ID NO: 259 (VL-CDR3);
18) VH region (SEQ ID NO: 366) comprising the amino acid sequences of SEQ ID NO: 260 (VH-CDR1), SEQ ID NO: 261 (VH-CDR2) and SEQ ID NO: 262 (VH-CDR3), and VL region (SEQ ID NO: 367) comprising the amino acid sequences of SEQ ID NO: 263 (VL-CDR1), SEQ ID NO: 264 (VL-CDR2) and SEQ ID NO: 265 (VL-CDR3);

### DLL3:

19) VH region (SEQ ID NO: 368) comprising the amino acid sequences of SEQ ID NO: 266 (VH-CDR1), SEQ ID NO: 267 (VH-CDR2) and SEQ ID NO: 268 (VH-CDR3), and VL region (SEQ ID NO: 369) comprising the amino acid sequences of SEQ ID NO: 269 (VL-CDR1), SEQ ID NO: 270 (VL-CDR2) and SEQ ID NO: 271 (VL-CDR3);
20) VH region (SEQ ID NO: 370) comprising the amino acid sequences of SEQ ID NO: 272 (VH-CDR1), SEQ ID NO: 273 (VH-CDR2) and SEQ ID NO: 274 (VH-CDR3), and VL region (SEQ ID NO: 371) comprising the amino acid sequences of SEQ ID NO: 275 (VL-CDR1), SEQ ID NO: 276 (VL-CDR2) and SEQ ID NO: 277 (VL-CDR3);

### BCMA:

21) VH region (SEQ ID NO: 372) comprising the amino acid sequences of SEQ ID NO: 278 (VH-CDR1), SEQ ID NO: 279 (VH-CDR2) and SEQ ID NO: 280 (VH-CDR3), and VL region (SEQ ID NO: 373) comprising the amino acid sequences of SEQ ID NO: 281 (VL-CDR1), SEQ ID NO: 282 (VL-CDR2) and SEQ ID NO: 283 (VL-CDR3);

### LIV-1:

22) VH region (SEQ ID NO: 374) comprising the amino acid sequences of SEQ ID NO: 284 (VH-CDR1), SEQ ID NO: 285 (VH-CDR2) and SEQ ID NO: 286 (VH-CDR3), and VL region (SEQ ID NO: 375) comprising the amino acid sequences of SEQ ID NO: 287 (VL-CDR1), SEQ ID NO: 288 (VL-CDR2) and SEQ ID NO: 289 (VL-CDR3);

### GPC-3:

23) VH region (SEQ ID NO: 87) comprising the amino acid sequences of SEQ ID NO: 99 (VH-CDR1), SEQ ID NO: 100 (VH-CDR2) and SEQ ID NO: 101 (VH-CDR3), and VL region (SEQ ID NO: 88) comprising the amino acid sequences of SEQ ID NO: 102 (VL-CDR1), SEQ ID NO: 103 (VL-CDR2) and SEQ ID NO: 104 (VL-CDR3);

### FGFR2:

24) VH region (SEQ ID NO: 376) comprising the amino acid sequences of SEQ ID NO: 290 (VH-CDR1), SEQ ID NO: 291 (VH-CDR2) and SEQ ID NO: 292 (VH-CDR3), and VL region (SEQ ID NO: 377) comprising the amino acid sequences of SEQ ID NO: 293 (VL-CDR1), SEQ ID NO: 294 (VL-CDR2) and SEQ ID NO: 295 (VL-CDR3);
25) VH region (SEQ ID NO: 378) comprising the amino acid sequences of SEQ ID NO: 296 (VH-CDR1), SEQ ID NO: 297 (VH-CDR2) and SEQ ID NO: 298 (VH-CDR3), and VL region (SEQ ID NO: 379) comprising the amino acid sequences of SEQ ID NO: 299 (VL-CDR1), SEQ ID NO: 300 (VL-CDR2) and SEQ ID NO: 301 (VL-CDR3);

### FGFR3:

26) VH region (SEQ ID NO: 380) comprising the amino acid sequences of SEQ ID NO: 302 (VH-CDR1), SEQ ID NO: 303 (VH-CDR2) and SEQ ID NO: 304 (VH-CDR3), and VL region (SEQ ID NO: 381) comprising the amino acid sequences of SEQ ID NO: 305 (VL-CDR1), SEQ ID NO: 306 (VL-CDR2) and SEQ ID NO: 307 (VL-CDR3);

### VEGFR2:

27) VH region (SEQ ID NO: 382) comprising the amino acid sequences of SEQ ID NO: 308 (VH-CDR1), SEQ ID NO: 309 (VH-CDR2) and SEQ ID NO: 310 (VH-CDR3), and VL region (SEQ ID NO: 383) comprising the amino acid sequences of SEQ ID NO: 311 (VL-CDR1), SEQ ID NO: 312 (VL-CDR2) and SEQ ID NO: 313 (VL-CDR3);

### CD20:

28) VH region (SEQ ID NO: 384) comprising the amino acid sequences of SEQ ID NO: 314 (VH-CDR1), SEQ ID NO: 315 (VH-CDR2) and SEQ ID NO: 316 (VH-CDR3), and VL region (SEQ ID NO: 385) comprising the amino acid sequences of SEQ ID NO: 317 (VL-CDR1), SEQ ID NO: 318 (VL-CDR2) and SEQ ID NO: 319 (VL-CDR3);
29) VH region (SEQ ID NO: 386) comprising the amino acid sequences of SEQ ID NO: 320 (VH-CDR1), SEQ ID NO: 321 (VH-CDR2) and SEQ ID NO: 322 (VH-CDR3), and VL region (SEQ ID NO: 387) comprising the amino acid sequences of SEQ ID NO: 323 (VL-CDR1), SEQ ID NO: 324 (VL-CDR2) and SEQ ID NO: 325 (VL-CDR3);

### CD38:

30) VH region (SEQ ID NO: 388) comprising the amino acid sequences of SEQ ID NO: 326 (VH-CDR1), SEQ ID NO: 327 (VH-CDR2) and SEQ ID NO: 328 (VH-CDR3), and VL region (SEQ ID NO: 389) comprising the amino acid sequences of SEQ ID NO: 329 (VL-CDR1), SEQ ID NO: 330 (VL-CDR2) and SEQ ID NO: 331 (VL-CDR3);

### EphA2:

31) VH region (SEQ ID NO: 143) comprising the amino acid sequences of SEQ ID NO: 157 (VH-CDR1), SEQ ID NO: 158 (VH-CDR2) and SEQ ID NO: 159 (VH-CDR3), and VL region (SEQ ID NO: 145) comprising the amino acid sequences of SEQ ID NO: 160 (VL-CDR1), SEQ ID NO: 161 (VL-CDR2) and SEQ ID NO: 162 (VL-CDR3).

Table 2 below shows the nucleotide sequence and the polypeptide sequence of an exemplary signal sequence for efficient expression of the fusion proteins according to various embodiments. When the above antibodies are expressed in mammalian cells, SEQ ID NO: 333 may be used as the signal sequence, but is not limited thereto.

**[Table 2]**

| Name | Sequence | SEQ ID NO |
|---|---|---|
| Nucleotide sequence | ATGACACGCCTCACAGTTCTTGCCTTGCTGGCTGGACTTCTTG CCTCATCCAGGGCA | 332 |
| Polypeptide sequence | MTRLTVLALLAGLLASSRA | 333 |

Table 3 below shows the variable region polypeptide sequences of anticancer antibodies, which are described as antigen-binding sites of various fusion proteins described herein. The fusion proteins according to exemplary embodiments may comprise or consist of these variable region polypeptides.

**[Table 3]**

| **Antigen** | **Antibody** | **Variable Region** | **Polypeptide sequence of variable region** | **SEQ ID NO** |
|---|---|---|---|---|
| EGFR | Cetuximab | VH | QVQLKQSGPGLVQPSQSLSITCTVSGFSLTNYGVHWVRQSPGKGLEWLGVI WSGGNTDYNTPFTSRLSINKDNSKSQVFFKMNSLQSNDTAIYYCARALTYY DYEFAYWGQGTLVTVSA | 334 |
| | | VL | DILLTQSPVILSVSPGERVSFSCRASQSIGTNIHWYQQRTNGSPRLLIKYA SESISGIPSRFSGSGSGTDFTLSINSVESEDIADYYCQQNNNWPTTFGAGT KLELK | 335 |
| | Panitumumab | VH | | 336 |
| | | VL | | 337 |
| | Necitumumab | VH | | 338 |
| | | VL | | 330 |
| | Imgatuzumab | VH | | 340 |
| | | VL | | 341 |
| | Depatuxizum ab | VH | QVQLQESGPGLVKPSQTLSLTCTVSGYSISSDFAWNWIRQPPGKGLEWMGY ISYSGNTRYQPSLKSRITISRDTSKNQFFLKLNSVTAADTATYYCVTAGRG FPYWGQGTLVTVSS | 342 |
| | | VL | DIQMTQSPSSMSVSVGDRVTITCHSSQDINSNIGWLQQKPGKSFKGLIYHG TNLDDGVPSRFSGSGSGTDYTLTISSLQPEDFATYYCVQYAQFPWTFGGGT KLEIK | 343 |
| | Losatuxizum ab | VH | EVQLQESGPGLVKPSQTLSLTCTVSGYSISRDFAWNWIRQPPGKGLEWMGY ISYNGNTRYQPSLKSRITISRDTSKNQFFLKLNSVTAADTATYYCVTASRG FPYWGQGTLVTVSS | 344 |
| | | VL | DIQMTQSPSSMSVSVGDRVTITCHSSQDINSNIGWLQQKPGKSFKGLIYHG TNLDDGVPSRFSGSGSGTDYTLTISSLQPEDFATYYCVQYAQFPWTFGGGT KLEIK | 345 |
| EGFRv III | Etevritamab | VH | QVQLVESGGGVVQSGRSLRLSCAASGFTFRNYGMHWVRQAPGKCLEWVAVI WYDGSDKYYADSVRGRFTISRDNSKNTLYLQMNSLRAEDTAVYYCARDGYD ILTGNPRDFDYWGQGTLVTVSS | 346 |
| | | VI. | DTVMTQTPLSSHVTLGQPASISCRSSQSLVHSDGNTYLSWLQQRPGQPPRL LIYRISRRFSGVPDRFSGSGAGTDFTLEISRVEAEDVGVYYCMQSTHVPRT FGGGTKVEIK | 347 |
| | AMG-595 | VH | QVQLVESGGGVVQPGRSLRLSCAASGFTFSSYGMHWVRQAPGKGLEWVAVI WYDGSNKYYVDSVKGRFTISRDNSKNTLYLQMNSLRAEDTAVYYCARDGWQ QLAPFDYWGQGTLVTVSS | 348 |
| | | VL | DIVMTQTPLSSPVTLGQPASISCRSSQSLVHSDGNTYLSWLHQRPGQPPRL LIYKISNRFSGVPDRFSGSGAGTAFTLKISRVEAEDVGVYYCMQATQLPRT FGQGTKVEIK | 349 |
| PD-L1 | Atezolizuma b | VH | | 350 |
| | | VL | | 351 |
| | Avelumab | VH | EVQLLESGGGLVQPGGSLRLSCAASGFTFSSYIMMWVRQAPGKGLEWVSSI YPSGGITFYADTVKGRFTISRDNSKNTLYLQMNSLRAEDTAVYYCARIKLG TVTTVDYWGQGTLVTVSS | 352 |
| | | VL | QSALTQPASVSGSPGQSITISCTGTSSDVGGYNYVSWYQQHPGKAPKLMIY DVSNRPSGVSNRFSGSKSGNTASLTISGLQAEDEADYYCSSYTSSSTRVFG TGTKVTVL | 353 |
| | Durvalumab | VH | EVQLVESGGGLVQPGGSLRLSCAASGFTFSRYWMSWVRQAPGKGLEWVANI KQDGSEKYYVDSVKGRFTISRDNAKNSLYLQMNSLRAEDTAVYYCAREGGW FGELAFDYWGQGTLVTVSS | 354 |
| | | VL | EIVLTQSPGTLSLSPGERATLSCRASQRVSSSYLAWYQQKPGQAPRLLIYD ASSRATGIPDRFSGSGSGTDFTLTISRLEPEDFAVYYCQQYGSLPWTFGQG TKVEIK | 355 |
| HER2 | Trastuzumab | VH | | 356 |
| | | VL | | 357 |
| | Pertuzumab | VH | | 27 |
| | | VL | | 28 |
| | | | | |
| c-Met | Onartuzumab | VH | | 358 |
| | | VL | | 359 |
| | Emibetuzuma b | VH | | 360 |
| | | VL | | 361 |
| | Telisotuzum ab | VH | QVQLVQSGAEVKKPGASVKVSCKASGYIFTAYTMHWVRQAPGQGLEWMGWI KPNNGLANYAQKFQGRVTMTRDTSISTAYMELSRLRSDDTAVYYCARSEIT TEFDYWGQGTLVTVSS | 362 |
| | | VL | DIVMTQSPDSLAVSLGERATINCKSSESVDSYANSFLHWYQQKPGQPPKLL IYRASTRESGVPDRFSGSGSGTDFTLTISSLQAEDVAVYYCQQSKEDPLTF GGGTKVEIK | 363 |
| TROP-2 | Datopotamab | VH | QVQLVQSGAEVKKPGASVKVSCKASGYTFTTAGMQWVRQAPGQGLEWMGWI NTHSGVPKYAEDFKGRVTISADTSTSTAYLQLSSLKSEDTAVYYCARSGFG SSYWYFDVWGQGTLVTVSS | 364 |
| | | VI. | DIQMTQSPSSLSASVGDRVTITCKASQDVSTAVAWYQQKPGKAPKLLIYSA SYRYTGVPSRFSGSGSGTDFTLTISSLQPEDFAVYYCQQHYITPLTFGQGT KLEIK | 365 |
| | Sacituzumab | VH | QVQLQQSGSELKKPGASVKVSCKASGYTFTNYGMNWVKQAPGQGLKWMGWI NTYTGEPTYTDDFKGRFAFSLDTSVSTAYLQISSLKADDTAVYFCARGGFG SSYWYFDVWGQGSLVTVSS | 366 |
| | | VL | DIQLTQSPSSLSASVGDRVSITCKASQDVSIAVAWYQQKPGKAPKLLIYSA SYRYTGVPDRFSGSGSGTDFTLTISSLQPEDFAVYYCQQHYITPLTFGAGT KVEIK | 36 7 |
| DLL3 | Rovalpituzu mab | VH | QVQLVQSGAEVKKPGASVKVSCKASGYTFTNYGMNWVRQAPGQGLEWMGWI NTYTGEPTYADDFKGRVTMTTDTSTSTAYMELRSLRSDDTAVYYCARIGDS SPSDYWGQGTLVTVSS | 368 |
| | | VL | EIVMTQSPATLSVSPGERATLSCKASQSVSNDVVWYQQKPGQAPRLLIYYA SNRYTGIPARFSGSGSGTEFTLTISSLQSEDFAVYYCQQDYTSPWTFGQGT KLEIK | 369 |
| | Tarlatamab | VH | QVQLQESGPGLVKPSETLSLTCTVSGGSISSYYWSWIRQPPGKCLEWIGYV YYSGTTNYNPSLKSRVTISVDTSKNQFSLKLSSVTAADTAVYYCASIAVTG | 370 |
| | | | FYFDYWGQGILVTVSS | |
| | | VL | EIVLTQSPGTLSLSPGERVTLSCRASQRVNNNYLAWYQQRPGQAPRLLIYG ASSRATGIPDRFSGSGSGTDFTLTISRLEPEDFAVYYCQQYDRSPLTFGGG TKLEIK | 371 |
| BCMA | Belantamab | VH | QVQLVQSGAEVKKPGSSVKVSCKASGGTFSNYWMHWVRQAPGQGLEWMGAT YRGHSDTYYNQKFKGRVTITADKSTSTAYMELSSLRSEDTAVYYCARGAIY DGYDVLDNWGQGTLVTVSS | 372 |
| | | VL | DIQMTQSPSSLSASVGDRVTITCSASQDISNYLNWYQQKPGKAPKLLIYYT SNLHSGVPSRFSGSGSGTDFTLTISSLQPEDFATYYCQQYRKLPWTFGQGT KLEIK | 373 |
| LIV-1 | Ladiratuzum ab | VH | QVQLVQSGAEVKKPGASVKVSCKASGLTIEDYYMHWVRQAPGQGLEWMGWI DPENGDTEYGPKFQGRVTMTRDTSINTAYMELSRLRSDDTAVYYCAVHNAH YGTWFAYWGQGTLVTVSS | 374 |
| | | VL | DVVMTQSPLSLPVTLGQPASISCRSSQSLLHSSGNTYLEWYQQRPGQSPRP LIYKISTRFSGVPDRFSGSGSGTDFTLKISRVEAEDVGVYYCFQGSHVPYT FGGGTKVEIK | 375 |
| GPC-3 | Codrituzuma b | VH | QVQLVQSGAEVKKPGASVKVSCKASGYTFTDYEMHWVRQAPGQGLEWMGAL DPKTGDTAYSQKFKGRVTLTADKSTSTAYMELSSLTSEDTAVYYCTRFYSY TYWGQGTLVTVSS | 87 |
| | | VL | DVVMTQSPLSLPVTPGEPASISCRSSQSLVHSNRNTYLHWYLQKPGQSPQL LIYKVSNRFSGVPDRFSGSGSGTDFTLKISRVEAEDVGVYYCSQNTHVPPT FGQGTKLEIK | 88 |
| FGFR2 | Aprutumab | VH | EVQLLESGGGLVQPGGSLRLSCAASGFTFSSYAMSWVRQAPGKGLEWVSAI SGSGTSTYYADSVKGRFTISRDNSKNTLYLQMNSLRAEDTAVYYCARVRYN WNHGDWFDPWGQGTLVTVSS | 376 |
| | | VL | QSVLTQPPSASGTPGQRVTISCSGSSSNIGNNYVSWYQQLPGTAPKLLIYE NYNRPAGVPDRFSGSKSGTSASLAISGLRSEDEADYYCSSWDDSLNYWVFG GGTKLTVL | 37 7 |
| | Bemarituzum ab | VH | | 378 |
| | | VL | | 379 |
| FGFR3 | Vofatamab | VH | | 380 |
| | | VL | | 381 |
| VEGFR 2 | Ramucirumab | VH | | 382 |
| | | VL | | 383 |
| CD20 | Rituximab | VH | | 384 |
| | | VL | | 385 |
| | Obinutuzuma b | VH | QVQLVQSGAEVKKPGSSVKVSCKASGYAFSYSWINWVRQAPGQGLEWMGRI FPGDGDTDYNGKFKGRVTITADKSTSTAYMELSSLRSEDTAVYYCARNVFD GYWLVYWGQGTLVTVSS | 386 |
| | | VL | DIVMTQTPLSLPVTPGEPASISCRSSKSLLHSNGITYLYWYLQKPGQSPQL LIYQMSNLVSGVPDRFSGSGSGTDFTLKISRVEAEDVGVYYCAQNLELPYT FGGGTKVEIK | 387 |
| CD38 | Daratumumab | VH | | 388 |
| | | VL | | 380 |
| EphA2 | 1C1 | VH | EVQLLESGGGLVQPGGSLRLSCAASGFTFSHYMMAWVRQAPGKGLEWVSRI GPSGGPTHYADSVKGRFTISRDNSKNTLYLQMNSLRAEDTAVYYCAGYDSG YDYVAVAGPAEYFQHWGQGTLVTVSS | 143 |
| | | VL | DIQMTQSPSSLSASVGDRVTITCRASQSISTWLAWYQQKPGKAPKLLIYKA SNLHTGVPSRFSGSGSGTEFSLTISGLQPDDFATYYCQQYNSYSRTFGQGT KVEIK | 145 |

Table 4 below shows the nucleotide sequences encoding variable regions of anticancer antibodies, which are described as antigen-binding sites of various fusion proteins described herein.

**[Table 4]**

| **Antigen** | **Antibody** | **Variable Region** | **Nucleotide sequence of variable region** | **SEQ ID NO** |
|---|---|---|---|---|
| EGFR | Cetuximab | VH | CAAGTGCAGCTTAAACAGAGTGGACCAGGGCTCGTTCAGCCTTCTCAAAGTTTG AGCATAACATGTACCGTAAGTGGTTTTTCACTGACTAACTACGGAGTGCATTGG GTTCGACAGTCCCCTGGAAAGGGTCTCGAATGGCTTGGAGTTATTTGGAGTGGC GGTAACACTGACTACAACACACCATTCACCTCTCGGCTGTCTATAAACAAGGAT AACTCTAAGTCACAGGTCTTTTTCAAGATGAACAGCCTCCAAAGTAACGACACC GCCATCTATTACTGTGCAAGAGCCTTGACCTACTACGATTACGAGTTTGCCTAT TGGGGTCAGGGAACTCTGGTAACAGTCAGTGCT | 390 |
| | | VL | GACATCCTTCTCACTCAGTCCCCCGTAATTCTGAGCGTGTCTCCCGGCGAGCGC GTCAGTTTCTCATGCAGGGCTAGCCAGAGTATAGGTACTAACATTCATTGGTAT CAACAGCGAACCAACGGCTCACCCCGCTTGCTTATCAAATATGCCTCTGAGAGT ATCTCTGGCATTCCATCCCGTTTTAGCGGTAGTGGTTCAGGAACAGATTTCACT CTTTCAATTAACTCTGTAGAATCAGAAGACATAGCCGATTATTATTGTCAGCAA AACAACAATTGGCCTACTACATTTGGAGCTGGTACAAAACTGGAACTCAAG | 391 |
| | Panitumum ab | VH | CAGGTACAACTGCAAGAGAGCGGACCTGGGTTGGTAAAGCCAAGTGAGACCCTG TCACTTACTTGCACAGTATCAGGAGGAAGTGTCTCAAGTGGCGATTACTATTGG ACTTGGATTCGACAAAGCCCCGGAAAAGGATTGGAATGGATCGGCCACATATAT TATTCAGGAAATACCAACTATAATCCCAGTCTTAAATCTCGGCTTACCATTAGC ATTGACACTTCCAAAACCCAATTTTCACTTAAACTGAGTTCTGTGACTGCTGCC GACACTGCTATATACTACTGCGTCCGCGACCGGGTCACTGGGGCTTTTGATATA TGGGGACAAGGAACTATGGTTACTGTATCATCT | 392 |
| | | VL | GATATTCAAATGACCCAATCTCCTTCATCACTCTCAGCATCAGTGGGGGATAGA GTGACAATAACTTGCCAAGCAAGCCAGGATATATCTAATTATCTCAACTGGTAT CAACAAAAGCCAGGTAAAGCTCCAAAGTTGTTGATTTACGATGCCTCTAATCTC GAGACCGGCGTACCAAGTAGGTTCAGTGGTTCAGGAAGTGGGACTGATTTCACT TTTACCATTTCTTCTCTCCAGCCCGAAGACATAGCAACTTATTTCTGTCAACAC TTCGATCATCTTCCTTTGGCATTTGGTGGCGGCACTAAAGTTGAAATCAAG | 393 |
| | Necitumum ab | VH | CAGGTACAACTCCAGGAATCCGGTCCTGGTCTCGTAAAGCCAAGCCAAACATTG AGTTTGACCTGCACAGTCAGTGGTGGCAGTATTTCCTCAGGCGATTATTACTGG AGTTGGATACGCCAGCCACCCGGTAAGGGATTGGAATGGATCGGTTATATATAC TACTCTGGATCTACAGACTATAATCCTTCCCTGAAGTCCAGGGTTACCATGAGT GTTGACACATCCAAAAACCAGTTCTCCTTGAAGGTCAACTCTGTGACCGCTGCC GATACTGCCGTTTATTATTGTGCACGTGTATCTATCTTCGGAGTAGGCACCTTT GACTATTGGGGTCAAGGCACCCTGGTCACCGTGTCATCT | 394 |
| | | VL | GAGATAGTAATGACACAGTCACCTGCTACCCTTTCCCTCAGCCCTGGCGAACGC GCCACCTTGTCTTGCAGGGCTAGCCAAAGTGTATCCTCTTATTTGGCATGGTAT CAGCAGAAACCAGGGCAAGCCCCACGGCTCCTGATTTATGATGCCTCCAATCGA GCCACCGGGATTCCTGCCCGATTCAGCGGATCAGGCAGTGGGACTGATTTTACA TTGACCATTAGCTCTCTGGAGCCTGAGGACTTTGCCGTGTATTATTGTCATCAA TATGGGAGTACTCCCCTCACATTTGGCGGCGGGACAAAAGCAGAAATTAAA | 395 |
| | Imgatuzum ab | VH | CAAGTACAGTTGGTACAAAGTGGGGCCGAGGTCAAGAAACCTGGTTCTAGCGTA AAGGTTAGCTGCAAGGCAAGCGGCTTCACCTTCACCGATTACAAAATTCATTGG GTCCGTCAAGCCCCCGGTCAGGGGCTTGAATGGATGGGTTATTTCAACCCCAAC TCTGGTTATTCTACCTATGCACAGAAGTTCCAGGGCCGCGTCACTATAACTGCA GACAAGTCCACAAGTACAGCCTATATGGAACTGTCCAGTTTGCGTAGCGAGGAT ACTGCCGTATATTACTGTGCTAGACTCTCACCCGGCGGATATTACGTTATGGAT GCATGGGGCCAGGGCACCACCGTGACCGTGAGCAGC | 396 |
| | | VL | GACATCCAAATGACACAGTCTCCTTCCTCACTCTCCGCTTCTGTAGGAGATAGA GTGACAATAACATGCCGTGCTTCACAAGGTATCAATAACTACCTCAATTGGTAT CAACAGAAACCTGGTAAAGCTCCAAAGCGCCTTATCTATAATACCAACAATCTC CAGACTGGTGTTCCAAGTAGGTTCTCCGGGAGCGGTAGTGGGACTGAGTTTACA TTGACAATTTCATCATTGCAACCCGAAGATTTTGCCACCTACTATTGCTTGCAG CATAATAGTTTTCCCACTTTCGGTCAGGGAACTAAGCTCGAAATAAAA | 307 |
| | Depatuxiz umab | VH | CAGGTACAACTCCAAGAATCTGGACCTGGGCTGGTAAAACCATCTCAGACCTTG AGTCTCACTTGCACTGTGTCCGGTTACTCTATATCTAGCGATTTTGCATGGAAT TGGATTCGCCAGCCACCCGGTAAGGGACTGGAGTGGATGGGTTACATTTCATAC AGTGGAAACACACGTTACCAACCCAGTCTCAAGAGCCGGATAACCATTAGTAGG GACACAAGCAAAAACCAATTTTTCCTCAAACTGAATTCAGTGACTGCTGCCGAC ACAGCCACTTACTATTGTGTCACAGCGGGTCGGGGGTTCCCTTACTGGGGACAG GGTACTCTCGTTACTGTAAGTTCT | 398 |
| | | VL | GACATACAAATGACTCAATCACCTAGCTCCATGTCTGTTTCCGTTGGCGACCGC GTAACCATCACCTGTCATTCCTCACAGGATATAAATTCCAATATTGGGTGGCTT CAGCAGAAACCAGGTAAATCATTCAAGGGCCTTATTTATCACGGGACCAACCTT GATGACGGTGTCCCCTCTCGCTTTAGCGGTTCTGGATCAGGTACAGATTATACC CTGACCATCTCTTCTCTCCAGCCCGAGGATTTCGCTACTTACTACTGTGTGCAA TACGCACAGTTCCCCTGGACATTCGGCGGTGGAACTAAACTGGAGATAAAG | 390 |
| | Losatuxiz | VH | GAAGTTCAGTTGCAAGAAAGTGGCCCCGGACTTGTTAAACCATCCCAGACCTTG | 400 |
| | umab | | AGCCTGACTTGCACCGTTTCTGGCTATTCTATTTCCCGGGACTTCGCATGGAAC TGGATACGCCAACCTCCCGGCAAGGGCTTGGAGTGGATGGGGTATATCTCCTAT AACGGCAATACACGCTATCAACCTAGTTTGAAGAGCCGCATAACCATCAGTAGA GACACAAGTAAAAACCAATTCTTCTTGAAACTGAATAGTGTGACCGCTGCAGAC ACTGCAACCTATTACTGCGTCACAGCCAGTCGGGGGTTTCCATATTGGGGTCAA GGTACACTTGTAACCGTGTCTTCA | |
| | | VL | GATATACAAATGACCCAGTCCCCTTCCTCCATGTCTGTCAGCGTCGGTGATAGG GTTACCATAACTTGTCATAGCAGCCAAGACATAAACAGCAATATAGGTTGGCTG CAACAAAAGCCCGGTAAATCTTTCAAGGGGTTGATTTACCACGGAACTAACCTC GATGATGGTGTGCCCAGTCGCTTTTCAGGTAGTGGGTCTGGAACAGACTACACC CTGACAATCTCCAGTTTGCAACCAGAAGACTTTGCTACTTATTATTGTGTACAG TACGCTCAATTTCCTTGGACTTTCGGGGGAGGAACCAAACTTGAGATCAAG | 401 |
| EGFRvIII | Etevritam ab | VH | CAAGTCCAACTCGTGGAGTCTGGAGGCGGTGTGGTCCAAAGCGGGAGATCCTTG CGGCTGTCTTGCGCAGCATCAGGGTTTACATTTAGGAACTACGGCATGCACTGG GTTAGACAGGCACCCGGTAAGTGTCTTGAATGGGTAGCAGTAATCTGGTATGAT GGTAGCGATAAATACTATGCTGATAGCGTTCGTGGTCGGTTCACCATTAGTCGG GACAACTCAAAGAACACTTTGTACCTGCAAATGAACTCTCTGAGGGCCGAGGAT ACAGCTGTATATTACTGTGCTAGGGATGGCTATGACATACTCACCGGCAACCCA CGGGATTTCGATTACTGGGGGCAAGGGACATTGGTAACAGTTTCATCT | 402 |
| | | VL | GATACAGTAATGACCCAGACTCCTCTGAGTAGTCATGTTACACTGGGCCAGCCT GCCTCTATCTCCTGTCGTTCAAGTCAGAGTCTGGTGCACTCAGACGGAAACACC TACCTTTCATGGTTGCAACAGCGTCCTGGTCAACCTCCTCGATTGTTGATATAC AGAATTAGTCGGCGTTTTTCTGGCGTCCCAGATCGCTTCTCCGGCTCAGGTGCC GGAACAGACTTCACCCTTGAGATCAGTCGAGTGGAGGCTGAGGATGTGGGCGTA TATTACTGTATGCAAAGCACTCACGTGCCAAGGACATTCGGGTGTGGAACCAAG GTAGAGATCAAA | 403 |
| | AMG-595 | VH | CAGGTGCAGTTGGTTGAGAGCGGCGGAGGTGTGGTACAGCCTGGAAGGAGTCTG CGACTCTCATGTGCCGCCTCCGGTTTTACATTTTCTTCCTACGGCATGCATTGG GTTCGACAAGCACCAGGCAAGGGATTGGAATGGGTGGCAGTAATTTGGTACGAC GGGTCAAATAAATACTATGTTGACAGCGTGAAAGGGCGATTCACTATCAGTCGT GACAATAGTAAAAACACTTTGTATCTGCAAATGAATAGTCTGAGGGCAGAGGAC ACAGCAGTCTATTACTGTGCTCGTGATGGTTGGCAACAGCTTGCCCCTTTTGAC TATTGGGGACAAGGAACTTTGGTAACAGTAAGCAGC | 404 |
| | | VL | GACATTGTTATGACACAAACACCTTTGTCCTCACCCGTAACACTGGGCCAACCT GCCTCCATATCCTGCCGAAGCAGTCAATCACTTGTCCACTCTGATGGGAATACC TACTTGTCATGGCTGCATCAGCGACCAGGTCAGCCTCCAAGGCTTCTCATATAT AAAATATCCAATCGTTTTTCAGGTGTTCCCGACCGTTTCTCCGGGTCAGGGGCC GGAACCGCCTTCACTCTCAAAATATCCAGAGTGGAGGCTGAAGACGTAGGTGTA TATTATTGTATGCAAGCCACCCAGTTGCCACGCACCTTTGGGCAAGGGACTAAA GTAGAAATAAAG | 405 |
| PD-L1 | Atezolizu mab | VH | GAAGTTCAACTGGTGGAGTCTGGAGGGGGTCTCGTCCAGCCCGGCGGGAGCTTG AGACTGTCTTGTGCTGCATCCGGCTTTACCTTTAGCGATTCCTGGATACACTGG GTAAGACAAGCACCTGGTAAAGGGTTGGAATGGGTGGCATGGATTTCCCCATAC GGGGGATCAACCTATTATGCTGATAGCGTGAAGGGCCGGTTTACTATTTCAGCC GACACAAGCAAGAACACCGCCTACCTCCAAATGAATTCTCTGAGGGCCGAGGAC ACAGCAGTCTATTACTGTGCTAGGAGGCATTGGCCCGGCGGATTCGACTACTGG GGGCAGGGCACTCTGGTCACAGTAAGCTCA | 406 |
| | | VL | GACATCCAAATGACACAGTCTCCAAGCTCTCTCTCCGCAAGCGTGGGCGACCGG GTCACAATAACATGCCGCGCCTCACAAGACGTGTCTACAGCCGTTGCTTGGTAT CAGCAAAAGCCCGGAAAAGCTCCCAAACTGCTCATTTATTCAGCCAGCTTTCTC TACAGCGGGGTGCCAAGCCGGTTCAGTGGGAGCGGAAGTGGCACCGACTTTACT CTTACCATCTCCTCTCTGCAACCCGAAGACTTCGCCACCTATTACTGCCAGCAA TACCTGTATCATCCAGCTACTTTCGGGCAAGGGACCAAGGTTGAAATCAAA | 407 |
| | Avelumab | VH | GAGGTTCAGCTTCTGGAGTCAGGAGGTGGTCTTGTTCAACCCGGAGGGTCTCTC CGTTTGTCCTGCGCCGCAAGTGGGTTCACATTTTCCAGTTATATCATGATGTGG GTGAGGCAAGCCCCTGGGAAAGGATTGGAGTGGGTCTCATCTATTTATCCATCT GGAGGTATTACATTCTATGCCGACACAGTTAAGGGCAGGTTTACTATAAGCCGC GATAATTCCAAAAACACCCTGTACCTGCAAATGAATTCACTGCGAGCCGAGGAC ACTGCTGTCTACTATTGCGCCAGAATCAAACTGGGGACAGTAACAACTGTAGAC TATTGGGGGCAGGGGACTTTGGTCACTGTATCATCA | 408 |
| | | VL | CAGTCAGCTCTGACACAGCCCGCATCTGTCTCTGGGAGTCCCGGACAGTCAATA ACTATATCATGCACAGGGACCTCCTCTGATGTTGGGGGATACAATTACGTGTCC TGGTATCAGCAGCACCCCGGCAAGGCCCCCAAACTCATGATATACGACGTTTCA AATCGTCCTTCAGGCGTGTCTAACAGATTTTCCGGTTCTAAATCAGGCAATACC GCTTCCTTGACTATCTCAGGACTTCAAGCAGAGGATGAAGCAGACTATTACTGC TCAAGCTATACCTCCAGTTCTACTAGAGTGTTCGGTACAGGAACTAAGGTAACA GTGCTG | 409 |
| | Durvaluma b | VH | GAGGTTCAGCTCGTGGAGAGTGGGGGCGGACTTGTTCAGCCAGGCGGTTCATTG CGGTTGTCTTGTGCCGCCTCAGGATTTACATTCTCAAGATACTGGATGAGCTGG GTGAGGCAAGCACCCGGTAAAGGTCTCGAGTGGGTAGCTAATATCAAACAAGAT GGGAGCGAGAAGTATTATGTTGACAGCGTGAAGGGTCGCTTTACCATATCAAGG GATAACGCTAAGAACTCCCTTTATCTTCAGATGAATAGTCTCCGCGCTGAGGAC ACCGCAGTATATTACTGTGCTAGGGAAGGAGGGTGGTTTGGGGAATTGGCATTC GATTACTGGGGTCAGGGTACTCTCGTTACAGTCAGTTCC | 410 |
| | | VL | GAAATCGTTTTGACACAGAGCCCTGGGACACTGTCCTTGAGCCCAGGAGAACGC GCAACCCTCTCCTGCCGTGCAAGTCAGCGTGTTTCCTCATCTTACCTTGCTTGG TATCAACAAAAGCCAGGGCAGGCTCCTAGACTGCTTATCTATGACGCTTCTAGC AGAGCTACTGGGATACCAGATAGGTTTTCCGGGTCTGGTTCAGGCACAGACTTC ACCCTCACCATATCTCGACTCGAACCTGAGGATTTTGCAGTGTACTATTGTCAA CAATACGGTAGTTTGCCTTGGACCTTTGGACAGGGCACTAAGGTCGAGATTAAA | 411 |
| HER2 | Trastuzum ab | VH | GAAGTGCAGCTGGTCGAAAGTGGCGGTGGACTTGTGCAACCTGGCGGTAGCCTC CGTCTCAGCTGCGCTGCAAGTGGGTTCAACATCAAGGACACTTATATTCATTGG GTCCGACAGGCACCTGGGAAAGGTTTGGAGTGGGTCGCACGGATCTATCCCACT AATGGTTACACAAGATATGCCGATTCAGTAAAAGGCCGGTTTACAATCAGCGCA GATACTTCAAAAAACACTGCCTATCTTCAAATGAACTCACTTCGAGCAGAAGAC ACAGCCGTCTATTATTGTAGTCGTTGGGGAGGCGACGGCTTTTATGCTATGGAC TACTGGGGACAAGGAACTCTGGTCACAGTTTCATCA | 412 |
| | | VL | GATATTCAGATGACTCAGAGTCCTAGTTCCCTCAGCGCCTCCGTAGGCGACAGA GTTACAATAACTTGCCGAGCAAGCCAAGACGTAAACACTGCAGTCGCCTGGTAC CAACAGAAACCAGGCAAAGCTCCAAAACTCTTGATTTACAGTGCTTCCTTCCTT TATAGTGGCGTTCCAAGCCGCTTCAGCGGCAGCCGCTCTGGCACCGACTTCACT CTCACTATTTCTTCCTTGCAACCTGAAGACTTCGCCACTTATTATTGCCAGCAA CACTACACAACACCCCCAACATTCGGACAGGGCACAAAGGTAGAAATAAAA | 413 |
| | Pertuzuma b | VH | GAAGTGCAACTGGTGGAGTCTGGTGGTGGATTGGTGCAGCCAGGCGGTTCTCTG CGACTTAGTTGTGCAGCCTCCGGCTTTACCTTCACTGATTATACAATGGACTGG GTTCGGCAGGCACCCGGTAAGGGGCTTGAGTGGGTCGCCGACGTCAATCCTAAT TCAGGGGGAAGTATTTATAACCAAAGGTTCAAGGGTCGATTTACATTGTCCGTA GATCGTAGTAAAAATACCCTCTACCTTCAAATGAACTCCCTGAGGGCAGAGGAT ACCGCAGTCTACTACTGCGCTCGTAACCTGGGGCCTAGTTTTTATTTCGATTAT TGGGGCCAAGGCACATTGGTAACTGTGTCTTCA | 414 |
| | | VL | GATATACAAATGACACAATCTCCTAGTTCATTGAGTGCCTCAGTCGGCGACCGA GTCACTATAACTTGTAAAGCAAGCCAAGATGTTAGCATTGGCGTAGCTTGGTAT CAGCAGAAACCTGGAAAAGCACCAAAACTGCTTATCTACTCCGCTAGTTACCGT TACACCGGAGTTCCCTCAAGGTTTTCTGGCAGCGGAAGTGGGACTGACTTCACT CTGACTATTTCTTCACTTCAGCCAGAAGACTTCGCTACTTATTACTGTCAGCAG TACTATATCTATCCCTATACATTTGGACAAGGAACCAAAGTTGAGATTAAA | 415 |
| c-Met | Onartuzum ab | VH | GAAGTCCAACTGGTAGAATCTGGAGGGGGTCTTGTTCAACCTGGGGGCAGTCTC AGGCTGTCATGTGCAGCAAGTGGATATACATTTACTTCCTATTGGCTCCATTGG GTACGACAAGCACCTGGGAAAGGGCTGGAATGGGTTGGTATGATCGACCCATCA AACTCTGACACCCGCTTTAATCCAAATTTTAAAGACCGCTTTACAATATCCGCA GATACAAGTAAGAACACCGCATATCTCCAGATGAACAGCCTGCGTGCAGAGGAT ACCGCAGTGTACTACTGTGCAACCTACCGGTCCTATGTAACCCCTCTCGACTAT TGGGGTCAAGGCACACTTGTCACCGTGAGTAGC | 416 |
| | | VL | GACATACAAATGACCCAATCACCAAGTTCCCTTTCTGCTTCAGTCGGTGATCGC GTGACAATAACATGCAAATCATCTCAAAGTCTCTTGTACACAAGCAGCCAAAAA AATTATCTTGCTTGGTACCAGCAGAAGCCAGGGAAAGCACCAAAACTGCTGATC TACTGGGCTTCAACAAGAGAATCCGGGGTGCCCAGCCGCTTTTCCGGTTCCGGC AGTGGAACTGATTTCACCCTCACTATTTCCTCATTGCAACCCGAGGACTTCGCA ACCTATTACTGTCAACAGTATTACGCCTACCCTTGGACATTTGGACAAGGAACT AAGGTTGAAATTAAA | 417 |
| | Emibetuzu mab | VH | CAAGTACAGCTCGTCCAATCCGGCGCTGAAGTCAAGAAGCCCGGAGCTTCCGTT AAAGTTTCCTGCAAAGCCAGCGGCTACACTTTCACTGATTACTATATGCACTGG GTTAGACAAGCACCCGGGCAGGGTCTCGAATGGATGGGTAGGGTTAATCCAAAT CGCAGGGGAACTACTTACAACCAGAAATTTGAGGGCAGGGTTACCATGACTACC GATACCAGCACATCTACTGCATATATGGAGCTGCGTTCTCTGAGGAGTGATGAT ACAGCAGTGTACTATTGTGCCCGCGCTAACTGGTTGGACTACTGGGGGCAAGGT ACAACTGTCACAGTATCTAGC | 418 |
| | | VL | GACATTCAAATGACACAAAGTCCATCCTCTCTCAGTGCTTCAGTGGGGGACCGA GTAACCATAACATGCAGTGTCTCAAGTAGCGTGAGTAGCATCTACCTCCATTGG TATCAGCAGAAACCTGGCAAGGCACCCAAACTCCTCATTTATTCCACAAGTAAT CTTGCTTCCGGCGTACCTTCTAGGTTTAGCGGGTCCGGCTCTGGCACCGATTTC ACCCTCACTATTAGCTCCTTGCAACCTGAGGACTTTGCTACTTACTATTGTCAG GTTTATTCCGGTTACCCCCTCACATTCGGTGGAGGAACCAAGGTAGAGATTAAG | 419 |
| | Telisotuz umab | VH | CAGGTCCAGTTGGTACAGTCAGGGGCAGAAGTTAAGAAACCAGGCGCTTCTGTA AAGGTTAGTTGCAAGGCAAGCGGGTATATATTCACAGCCTATACCATGCATTGG GTGCGTCAAGCTCCTGGGCAAGGATTGGAGTGGATGGGCTGGATCAAGCCCAAC AATGGTCTGGCCAACTACGCACAGAAGTTCCAAGGTCGTGTAACCATGACCAGG GACACTTCAATAAGCACCGCCTACATGGAATTGAGCAGACTTCGATCAGATGAT ACAGCAGTTTACTATTGCGCTAGGAGTGAAATTACCACAGAGTTTGATTACTGG GGCCAAGGAACTCTGGTGACTGTTTCCAGT | 420 |
| | | VL | GATATCGTTATGACACAGTCCCCCGACAGCCTGGCTGTCAGTCTCGGGGAGAGA GCAACTATAAACTGTAAAAGCAGTGAATCCGTCGATTCATACGCAAACAGTTTT CTGCATTGGTATCAGCAAAAACCCGGCCAGCCACCCAAACTGCTCATATATCGG GCTAGTACACGTGAGTCAGGCGTACCAGACCGCTTTAGCGGATCAGGAAGTGGG ACAGACTTTACCTTGACCATTAGCTCACTTCAGGCTGAGGACGTTGCAGTTTAC TACTGCCAACAAAGTAAGGAAGACCCACTCACATTCGGCGGAGGAACTAAGGTC GAGATTAAG | 421 |
| TROP-2 | Datopotam ab | VH | CAGGTGCAGTTGGTCCAGTCCGGTGCAGAGGTAAAGAAACCAGGCGCTTCCGTT AAAGTATCCTGTAAAGCTAGCGGTTATACTTTCACTACTGCCGGAATGCAGTGG GTGCGACAGGCTCCTGGTCAGGGTCTCGAATGGATGGGATGGATCAATACTCAC TCAGGAGTGCCAAAGTATGCTGAAGATTTCAAGGGGCGTGTTACTATCTCCGCT GACACATCCACATCTACTGCTTATCTTCAGCTCTCTAGCCTGAAGTCAGAAGAT ACTGCTGTTTATTATTGTGCAAGGAGCGGGTTTGGAAGTTCCTATTGGTACTTC GACGTTTGGGGACAAGGAACACTGGTCACTGTTTCCAGC | 422 |
| | | VL | GATATACAGATGACCCAATCACCCTCTTCTTTGAGTGCATCAGTAGGTGATCGT GTTACAATTACCTGTAAAGCTAGTCAAGACGTGTCTACTGCTGTCGCCTGGTAT CAACAGAAACCAGGCAAAGCACCAAAACTTTTGATTTATTCTGCTTCTTATAGA TATACAGGCGTCCCCAGCCGGTTTTCAGGTTCCGGTTCCGGGACCGACTTTACC CTCACTATCAGCTCTCTTCAGCCTGAGGACTTCGCCGTTTATTATTGCCAGCAG CACTATATCACCCCACTGACTTTTGGGCAAGGGACTAAGCTGGAGATCAAA | 423 |
| | Sacituzum ab | VH | CAAGTACAGCTTCAGCAATCAGGCTCTGAACTCAAGAAACCTGGTGCCAGCGTG AAAGTATCCTGTAAGGCATCCGGGTACACTTTTACAAACTATGGAATGAATTGG GTAAAACAAGCCCCCGGACAAGGGCTTAAATGGATGGGTTGGATAAATACTTAC ACCGGAGAACCTACATATACAGACGACTTTAAAGGGCGATTCGCTTTCTCCCTT GACACAAGTGTAAGCACAGCCTACCTGCAAATCAGCAGTCTCAAAGCCGACGAT ACAGCCGTGTACTTTTGCGCCAGGGGTGGCTTCGGCTCCAGCTATTGGTACTTC GATGTCTGGGGACAGGGCAGCCTTGTAACTGTATCTAGT | 424 |
| | | VL | GATATTCAGTTGACACAGAGCCCTAGCTCACTTTCAGCCTCTGTGGGTGATAGG GTTAGTATTACCTGTAAGGCATCCCAAGACGTTAGCATTGCCGTCGCCTGGTAC CAGCAGAAACCCGGAAAAGCTCCAAAACTGCTCATCTACTCAGCATCCTATCGT TATACAGGTGTACCTGACAGGTTCTCTGGCTCCGGGAGCGGTACCGATTTTACT TTGACCATTTCAAGTTTGCAACCAGAAGACTTCGCTGTTTACTACTGCCAGCAG CATTACATTACTCCACTCACATTCGGAGCAGGGACAAAGGTCGAAATCAAA | 425 |
| DLL3 | Rovalpitu zumab | VH | CAAGTCCAGCTTGTTCAAAGTGGGGCTGAAGTGAAGAAACCAGGGGCTAGTGTT AAAGTGAGCTGTAAGGCATCAGGATACACTTTCACAAACTACGGAATGAATTGG GTTCGCCAAGCACCTGGTCAAGGCTTGGAATGGATGGGTTGGATTAACACATAT ACAGGTGAGCCAACTTACGCCGATGATTTCAAGGGGCGAGTTACCATGACTACC GACACCTCAACATCCACTGCATACATGGAGCTTCGCTCACTCCGAAGCGATGAT ACTGCAGTTTACTATTGCGCTCGCATCGGTGACTCATCACCTAGCGACTACTGG GGCCAAGGTACATTGGTAACAGTTTCTTCA | 426 |
| | | VL | GAGATCGTGATGACCCAGAGTCCCGCTACTCTCTCAGTGAGTCCTGGTGAACGT GCTACACTGTCTTGTAAGGCCAGTCAGTCCGTCTCAAACGATGTCGTTTGGTAT CAGCAAAAGCCAGGACAAGCCCCCAGACTCCTGATATACTACGCCAGTAATCGC TATACTGGAATCCCCGCTAGATTCAGTGGGAGTGGAAGCGGAACTGAATTTACC TTGACTATATCCTCATTGCAAAGCGAAGACTTTGCCGTTTACTATTGTCAACAA GACTACACCTCTCCTTGGACCTTCGGACAAGGTACAAAACTTGAAATCAAG | 427 |
| | Tarlatama b | VH | CAAGTACAGCTCCAAGAAAGTGGCCCCGGATTGGTGAAGCCATCCGAAACACTT TCCCTTACCTGCACTGTCTCCGGGGGCTCCATCAGTAGTTATTACTGGAGTTGG ATACGCCAACCACCCGGTAAGTGTCTGGAGTGGATAGGTTATGTGTATTACTCA GGCAGAACCAATTATAATCCATCCTTGAAAAGCCGGGTAACCATCTCAGTAGAT ACCAGCAAAAACCAGTTCTCCCTGAAACTGTCCAGTGTTACTGCTGCTGATACC GCCGTATATTATTGTGCATCCATTGCAGTGACAGGGTTTTATTTTGACTATTGG GGCCAGGGTACTTTGGTAACCGTATCTTCA | 428 |
| | | VL | GAGATCGTCCTGACCCAAAGCCCAGGTACTCTTTCCCTCAGCCCAGGCGAAAGG GTCACTCTGTCATGCAGGGCTAGTCAAAGAGTCAACAATAATTACCTCGCATGG TATCAACAAAGACCCGGACAGGCTCCACGCCTGCTCATATATGGAGCAAGTAGC CGAGCTACTGGCATTCCCGATAGATTCAGTGGATCTGGATCTGGGACCGATTTT ACTCTGACAATAAGTCGTCTTGAACCTGAAGATTTTGCAGTATACTATTGTCAG CAATATGACAGGAGCCCCCTGACATTCGGGTGCGGTACTAAGCTGGAAATCAAA | 429 |
| BCMA | Belantama | VH | CAAGTCCAACTGGTCCAGTCAGGCGCAGAAGTTAAAAAGCCTGGCAGCAGTGTG | 430 |
| | b | | AAGGTGTCTTGTAAGGCAAGCGGCGGTACATTTAGTAATTATTGGATGCACTGG GTACGGCAGGCTCCCGGCCAAGGGCTTGAATGGATGGGCGCCACATACCGAGGT CATTCAGACACCTATTACAACCAGAAATTCAAGGGGCGCGTGACCATTACAGCA GATAAATCAACTTCTACAGCCTACATGGAACTCAGCTCCCTCCGGTCAGAGGAT ACAGCAGTCTACTACTGTGCTCGGGGAGCCATTTACGATGGGTATGATGTGCTG GATAATTGGGGGCAGGGCACACTCGTGACCGTAAGTAGT | |
| | | VL | GATATACAGATGACCCAGTCACCATCCAGCCTTAGTGCATCCGTCGGGGATCGG GTGACTATTACTTGCTCCGCTTCTCAAGATATTTCAAACTATCTGAATTGGTAT CAGCAAAAGCCTGGGAAGGCCCCAAAATTGCTGATCTATTACACTTCAAATTTG CACTCAGGGGTTCCCTCTCGCTTCAGCGGAAGCGGAAGCGGTACTGATTTTACC TTGACTATCTCTAGCCTCCAGCCAGAGGACTTTGCTACCTACTACTGCCAACAG TACAGGAAACTCCCATGGACTTTTGGACAAGGCACCAAGCTCGAAATTAAG | 431 |
| LIV-1 | Ladiratuz umab | VH | CAAGTTCAGTTGGTTCAATCTGGGGCCGAAGTCAAAAAACCTGGCGCTTCAGTT AAAGTTAGCTGCAAAGCAAGCGGTCTCACTATAGAAGACTATTATATGCACTGG GTCAGACAGGCTCCAGGGCAAGGGCTTGAGTGGATGGGGTGGATAGATCCAGAG AATGGGGACACCGAGTATGGACCCAAATTCCAGGGGCGTGTAACCATGACCCGA GACACTTCAATAAATACTGCATACATGGAACTCTCCCGGCTCCGGAGCGACGAT ACAGCCGTGTATTACTGTGCTGTCCACAACGCCCACTACGGCACATGGTTTGCA TATTGGGGGCAGGGAACTCTTGTTACTGTTTCTTCA | 432 |
| | | VL | GACGTAGTGATGACTCAGTCTCCACTGTCCCTGCCAGTGACATTGGGCCAACCT GCAAGTATTTCATGCAGATCAAGTCAATCTCTCCTGCACAGTAGCGGCAACACA TACTTGGAGTGGTATCAACAACGCCCAGGTCAATCACCCAGGCCACTGATATAT AAAATCTCAACTCGATTCAGCGGTGTTCCCGACAGGTTCTCAGGATCTGGCTCC GGCACTGATTTTACCTTGAAGATCTCACGAGTGGAAGCTGAGGATGTGGGAGTA TATTACTGTTTCCAAGGTTCACATGTCCCTTATACTTTTGGTGGAGGAACTAAG GTAGAGATCAAG | 433 |
| GPC-3 | Codrituzu mab | VH | CAGGTGCAACTCGTTCAAAGCGGGGCCGAGGTGAAGAAACCAGGGGCCTCAGTT AAGGTGAGTTGCAAGGCAAGTGGATACACTTTCACCGATTATGAAATGCATTGG GTGCGTCAGGCCCCAGGACAAGGACTGGAGTGGATGGGCGCTCTCGATCCTAAG ACTGGTGATACTGCTTACTCTCAAAAGTTCAAAGGCCGAGTCACCTTGACCGCC GACAAGTCCACATCCACTGCATATATGGAATTGTCAAGTCTGACAAGCGAAGAT ACAGCCGTCTACTACTGCACCCGCTTTTATAGCTATACATATTGGGGACAGGGG ACCTTGGTTACTGTGTCATCT | 434 |
| | | VL | GACGTGGTAATGACACAATCACCTTTGTCTCTTCCCGTAACCCCCGGTGAACCA GCCAGCATCTCATGCAGAAGCAGTCAGTCACTGGTACATTCCAACCGTAATACT TATCTTCACTGGTACTTGCAGAAGCCTGGGCAGTCTCCTCAACTTTTGATATAT AAAGTGAGCAATCGGTTTAGCGGTGTCCCAGACCGCTTTTCTGGATCTGGAAGT GGAACAGACTTTACTCTGAAAATAAGCAGAGTCGAGGCAGAAGATGTCGGAGTT TACTACTGTAGCCAGAACACACACGTACCCCCAACCTTTGGACAGGGCACAAAG TTGGAAATCAAG | 435 |
| FGFR2 | Aprutumab | VH | GAGGTACAACTGCTTGAATCTGGAGGAGGGTTGGTACAACCTGGTGGTTCACTG CGATTGTCCTGTGCAGCCTCAGGCTTTACTTTCTCATCATATGCCATGTCCTGG GTAAGGCAGGCACCTGGAAAAGGACTCGAATGGGTCTCAGCCATCTCCGGTTCA GGCACATCAACTTACTATGCAGACTCTGTCAAAGGGCGCTTTACAATATCTAGG GATAATTCAAAAAATACATTGTACTTGCAGATGAACAGTTTGCGTGCCGAAGAT ACCGCAGTGTACTATTGCGCTAGGGTTCGATATAACTGGAACCATGGTGACTGG TTTGACCCTTGGGGCCAAGGCACACTGGTGACAGTGAGTTCC | 436 |
| | | VL | CAGTCCGTCCTCACACAACCACCTAGTGCCTCTGGTACACCAGGACAACGTGTC ACAATTTCCTGCAGCGGGTCAAGTTCAAACATAGGGAATAACTATGTGTCATGG TATCAACAACTTCCTGGTACTGCTCCAAAGCTCCTCATTTATGAAAACTATAAC AGGCCCGCAGGTGTCCCAGATCGATTTTCAGGATCAAAGTCCGGTACCTCAGCC AGTTTGGCAATTAGTGGCCTTCGATCCGAAGATGAAGCAGATTACTACTGTTCA TCCTGGGACGATTCTCTTAACTATTGGGTATTCGGCGGAGGCACTAAACTCACC GTCCTT | 437 |
| | Bemarituz umab | VH | CAAGTGCAGCTTGTTCAGAGTGGGGCTGAAGTCAAAAAGCCAGGCTCAAGCGTG AAAGTCAGCTGCAAGGCCAGCGGTTACATCTTCACAACTTACAATGTTCACTGG GTCAGACAAGCCCCTGGTCAGGGGCTTGAGTGGATAGGATCAATCTACCCCGAT AATGGGGACACCAGCTATAATCAAAACTTCAAAGGACGTGCAACAATCACAGCC GACAAGTCAACTTCAACAGCCTACATGGAGCTTTCCAGCTTGCGATCCGAAGAT ACTGCCGTATATTACTGTGCTAGAGGCGACTTCGCTTATTGGGGACAAGGTACT TTGGTGACTGTTTCTTCT | 439 |
| | | VL | GACATTCAAATGACCCAATCCCCCAGTTCCTTGAGTGCCTCCGTCGGAGATCGA GTTACTATTACATGTAAAGCTAGTCAAGGGGTCAGTAACGACGTTGCTTGGTAC CAGCAGAAGCCAGGTAAGGCTCCCAAGCTCCTGATATATAGCGCCTCATACCGC TAGACAGGTGTGCCTTCCCGGTTTAGTGGCTCAGGATCAGGGACAGATTTTACA TTCACTATAAGCTCTTTGCAGCCCGAAGACATAGCCACATATTATTGCCAGCAG CATTCCACTACTCCATACACATTTGGACAGGGAACAAAGCTGGAGATTAAG | 439 |
| FGFR3 | Vofatamab | VH | GAAGTACAGCTTGTCGAGAGCGGTGGCGGGCTTGTACAACCTGGTGGAAGCTTG CGGTTGAGTTGTGCAGCCAGCGGTTTCACATTTACTAGCACTGGAATATCATGG GTCCGTCAAGCTCCAGGGAAAGGGCTGGAATGGGTTGGCCGAATATATCCCACC AGTGGCTCTACCAACTACGCAGACTCAGTCAAGGGTCGCTTTACAATTTCTGCT GACACAAGTAAGAACACCGCATATTTGCAGATGAACTCACTGCGAGCCGAGGAT ACCGCCGTTTACTACTGTGCAAGGACATACGGAATTTACGATCTTTACGTTGAT TATACAGAGTATGTGATGGATTATTGGGGGCAGGGCACCCTCGTCACTGTCAGT TCT | 440 |
| | | VL | GACATCCAGATGACCCAGAGCCCCTCTTCTTTGTCAGCAAGCGTCGGAGACCGC GTTACCATTACTTGCCGTGCCTCTCAGGACGTGGACACCAGCCTTGCTTGGTAC AAGCAGAAACCAGGAAAAGCCCCCAAGCTGCTCATCTATTCCGCTTCATTTCTC TACAGCGGAGTGCCATCCCGTTTCTCCGGTTCAGGCTCTGGAACAGACTTCACT CTGACTATAAGCAGTCTTCAACCCGAAGACTTCGCTACATACTATTGTCAGCAA | 441 |
| | | | TCAACCGGACACCCACAGACATTCGGCCAGGGCACTAAAGTAGAGATTAAA | |
| VEGFR2 | Ramucirum ab | VH | GAAGTTCAGCTTGTGCAGAGTGGCGGAGGGCTTGTGAAACCAGGAGGATCACTG AGGCTCTCCTGTGCAGCATCCGGTTTCACATTCAGCAGCTATAGTATGAACTGG GTGCGTCAGGCTCCAGGGAAGGGACTGGAATGGGTCTCAAGCATTTCCTCCTCC TCTTCATATATATATTACGCCGACAGTGTAAAAGGCCGCTTTACAATATCTCGG GATAACGCTAAAAATAGCTTGTACCTTCAGATGAATTCACTGAGGGCTGAGGAC ACTGCTGTGTACTACTGTGCAAGAGTCACCGACGCTTTTGATATTTGGGGTCAG GGGACAATGGTGACCGTCTCCTCA | 442 |
| | | VL | GATATACAAATGACCCAGTCTCCCAGTTCAGTATCCGCCAGCATAGGTGACCGC GTGACCATAACATGCCGGGCCAGCCAGGGAATTGATAATTGGTTGGGGTGGTAT CAACAAAAGCCCGGAAAAGCTCCCAAGCTCCTTATCTATGATGCTTCTAACTTG GATACAGGTGTACCCAGTCGATTTAGTGGCTCCGGGAGTGGGACTTATTTCACC CTCACTATATCTTCTCTGCAAGCAGAAGACTTTGCAGTATATTTCTGTCAACAG GCCAAAGCATTCCCTCCAACCTTCGGTGGGGGGACAAAGGTAGACATTAAA | 443 |
| CD20 | Rituximab | VH | CAGGTCCAGCTCCAACAACCTGGAGCAGAATTGGTCAAGCCAGGGGCAAGCGTC AAGATGAGCTGCAAGGCAAGCGGCTATACTTTCACCTCCTACAATATGCATTGG GTCAAACAAACTCCAGGTCGTGGGCTTGAGTGGATCGGGGCCATTTACCCAGGC AACGGCGACACCTCATATAACCAAAAGTTTAAGGGAAAAGCCACTTTGACAGCA GATAAAAGTAGTAGCACTGCATACATGCAACTGTCAAGTCTGACTAGCGAAGAT AGTGCCGTATATTATTGCGCTAGGTCCACATATTACGGCGGTGATTGGTACTTC AATGTTTGGGGAGCCGGGACTACAGTCACCGTATCCGCT | 444 |
| | | VL | CAAATCGTCCTGTCTCAATCACCAGCAATTCTGAGTGCTAGTCCCGGAGAAAAA GTCACTATGACCTGTAGAGCCTCATCATCTGTTTCCTATATACATTGGTTTCAG CAGAAACCTGGATCTTCTCCCAAGCCCTGGATTTATGCAACCTCTAACCTCGCA AGTGGAGTCCCCGTGCGGTTTTCAGGCAGCGGTTCCGGTACAAGTTATTCCCTG ACCATCAGCCGTGTGGAAGCAGAAGACGCCGCCACATACTACTGCCAACAGTGG ACCTCAAATCCTCCCACCTTTGGGGGAGGTACTAAACTTGAAATAAAA | 445 |
| | Obinutuzu mab | VH | CAAGTGCAACTGGTTCAAAGTGGAGCCGAGGTCAAAAAACCTGGTTCCTCCGTC AAAGTGTCTTGTAAAGCTTCAGGGTACGCATTCTCCTACTCCTGGATAAACTGG GTGCGTCAGGCTCCTGGGCAAGGTCTGGAATGGATGGGCCGGATTTTCCCAGGA GATGGCGACACAGACTACAATGGGAAGTTTAAGGGTCGGGTAACCATCACCGCT GACAAGAGTACATCTACCGCCTATATGGAGCTTTCTTCACTTAGGAGTGAGGAC ACAGCAGTCTACTATTGTGCTCGAAATGTGTTTGACGGGTATTGGCTGGTGTAT TGGGGCCAGGGTACCCTCGTAACAGTATCATCA | 446 |
| | | VL | GATATTGTTATGACACAAACACCACTGTCCCTCCCTGTTACACCCGGAGAGCCT GCTTCCATAAGTTGTCGATCCTCCAAATCACTTCTCCACTCAAATGGAATCACT TATCTTTATTGGTATCTTCAGAAGCCAGGACAGTCCCCTCAACTGTTGATTTAT CAGATGTCAAATTTGGTGAGTGGGGTGCCAGATAGGTTTTCTGGATCCGGTTCC GGTACTGACTTTACATTGAAAATATCCCGAGTCGAAGCCGAAGACGTGGGCGTG TACTATTGCGCTCAGAACCTTGAGCTGCCTTACACCTTTGGCGGTGGGACTAAA | 447 |
| | | | GTGGAAATTAAG | |
| CD38 | Daratumum ab | VH | GAGGTTCAGTTGCTGGAGAGCGGAGGGGGGCTTGTCCAACCAGGCGGTTCTCTG CGACTTTCTTGTGCAGTGTCTGGGTTTACCTTCAACAGCTTTGCCATGTCCTGG GTGCGCCAAGCACCCGGAAAGGGACTGGAGTGGGTTAGCGCAATCTCTGGGTCA GGGGGAGGGACTTATTATGCTGACTCTGTTAAGGGTAGATTTACAATCAGTCGC GATAATAGTAAAAATACACTGTATCTTCAGATGAACTCTCTCAGAGCCGAGGAT ACAGCCGTGTATTTCTGTGCCAAAGACAAGATCCTTTGGTTCGGAGAGCCTGTT TTCGACTATTGGGGTCAAGGGACATTGGTGACAGTAAGCTCT | 448 |
| | | VL | GAAATTGTTCTCACCCAGAGTCCAGCTACCCTGTCCCTGAGCCCCGGCGAGAGA GCAACCTTGAGTTGCCGAGCCTCTCAATCCGTCTCCTCCTATCTGGCCTGGTAC CAACAAAAACCAGGCCAAGCCCCCCGTTTGCTGATATACGACGCCAGTAACCGA GCTACCGGCATACCCGCCCGCTTTAGCGGCTCTGGATCTGGTACAGATTTCACA CTCACTATATCAAGTCTGGAACCTGAAGATTTCGCAGTCTATTATTGCCAACAA CGGTCAAATTGGCCCCCTACATTTGGACAAGGGACCAAAGTGGAGATTAAG | 449 |
| EphA2 | 1C1 | VH | GAGGTACAGTTGCTGGAGTCAGGAGGTGGATTGGTCCAACCCGGAGGATCTCTT CGTCTGTCCTGCGCCGCCTCAGGATTTACCTTCTCTCATTATATGATGGCATGG GTACGTCAGGCTCCAGGCAAAGGTCTGGAATGGGTTAGTCGGATTGGTCCCTCA GGGGGTCCTACCCATTATGCCGATTCTGTAAAGGGCCGTTTTACCATAAGCAGA GACAACTCTAAGAACACCCTTTACCTTCAGATGAATAGCCTGAGGGCTGAGGAT ACCGCAGTGTATTACTGCGCAGGCTATGACTCTGGGTACGACTATGTCGCCGTA GCAGGACCTGCCGAGTATTTTCAACACTGGGGACAGGGGACCCTTGTCACAGTT TCTAGT | 450 |
| | | VL | GATATTCAAATGACACAAAGCCCAAGTTCCTTGTCCGCCTCAGTTGGTGATCGT GTGACAATAACCTGTCGGGCTTCACAATCCATATCTACATGGCTGGCTTGGTAC CAGCAAAAGCCAGGTAAAGCCCCAAAACTCCTGATTTACAAGGCAAGTAACTTG CATACTGGGGTACCCAGCCGTTTCTCTGGGTCAGGCTCTGGGACAGAGTTTAGT CTTACAATTTCTGGTCTGCAACCCGATGACTTCGCTACCTATTACTGTCAACAA TATAATAGTTATTCTCGAACATTTGGTCAGGGAACAAAAGTGGAAATCAAA | 451 |

### Fc region or fragment thereof

Here, the above-described first Fc domain and the second Fc domain may each be an Fc region of an immunoglobulin. The Fc region of an immunoglobulin may be an Fc domain variant as well as a wild type Fc domain. Here, the Fc region may be an Fc region of IgG, IgA, IgE, IgD, or IgM.

As used herein, the term "Fc domain variant" may refer to a form which is different from the wild type Fc domain in terms of glycosylation pattern, has a high level of specific glycan species as compared with the wild type Fc domain, a low level of specific glycan species as compared with the wild type Fc domain, or a deglycosylated form. In addition, an aglycosylated Fc domain is included therein. The Fc domain or a variant thereof may be adapted to have an adjusted number of sialic acids, fucosylations, or other types of glycosylations, through modulation of culture conditions or genetic manipulation of a host cell.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulin IgG, IgA, IgE, IgD, or IgM. In addition, the Fc domain variant may be a form in which some amino acids of the Fc domain are substituted with other amino acids.

An "amino acid" introduced by the substitution and/or addition may be any one selected from the group consisting of lysine (K), alanine (A), arginine (R), asparagine (N), aspartic acid (D), cysteine (C), glutamine (Q), glutamic acid (E), glycine (G), histidine (H), isoleucine (I), leucine (L), methionine (M), phenylalanine (F), proline (P), serine (S), threonine (T), tryptophan (W), tyrosine (Y), and valine (V).

In one embodiment, the variant of the Fc region may be a form in which amino acids 239 and/or 332 of the CH2 region are substituted with other amino acids (see Kabat numbering system). Specifically, S239 may be substituted with an amino acid other than S, and specifically, S239 may be substituted with S239D. In addition, I332 may be substituted with an amino acid other than I, and specifically, I332 may be substituted with I332E.

In addition, the Fc region may include a variant or structure of a knob, or a variant or structure of a hole.

As used herein, the term "knob-into-hole" refers to an Fc heterodimerization strategy for producing antibodies that specifically bind to different regions, such as bispecific antibodies, multispecific antibodies, or heterodimeric antibodies. Generally, this technique involves introducing a knob mutation at the interface of a first polypeptide (e.g., the first CH3 domain of a first antibody heavy chain) and a corresponding hole mutation at the interface of a second polypeptide (e.g., the second CH3 domain of a second antibody heavy chain), such that a knob may be placed within the hole to promote heterodimer formation and prevent homodimer formation.

The 'knob' variant is constructed by replacing small amino acid side chains from the interface of the first polypeptide (e.g., the first CH3 domain of the first antibody heavy chain) with larger side chains (e.g., arginine, phenylalanine, tyrosine, or tryptophan). The complementary 'hole' variant of the same or similar size to the knob is created by replacing large amino acid side chains at the interface of the second polypeptide (e.g., the second CH3 domain of the second antibody heavy chain) with smaller side chains (e.g., alanine, serine, valine, or threonine). The knob and hole may be created by altering the nucleic acid encoding the polypeptide, for example, by site-directed mutagenesis, or by peptide synthesis.

Examples of variants of the Fc region that promote the formation of a heterodimer may include those described in WO2014084607A1 and WO2018059502A1, etc. The disclosures of WO2014084607A1 and WO2018059502A1 are incorporated herein by reference. WO2014084607A1 describes, for example, mutations in the CH3 domain that may comprise (a-1) tryptophan (W) substituted at Lys409 of one CH3 domain that interacts with valine (V) substituted at Asp399 and threonine (T) substituted at Phe405 of another CH3 domain; and (a-2) serine (S) substituted at Tyr349 of one CH3 domain that interacts with tryptophan (W) substituted at Glu357 of another CH3 domain, and in addition, may further comprise (b-1) glutamic acid (E) substituted at Lys360 of one CH3 domain that interacts with arginine (R) substituted at Gln347 of another CH3 domain; and (b-2) glutamic acid (E) substituted at Gln347 and glutamic acid substituted at Lys360 of one CH3 domain that interact with arginine (R) substituted at Gln347 of another CH3 domain. Here, the position of the amino acid residue follows the EU index. WO2018059502A1, for example, describes mutations in the Fc domain including one or more mutations selected from a) - e), respectively: a) L351G, L351Y, L351V, L351P, L351D, L351E, L351K, or L351W; b) T366L, T366P, T366W, or T366V; c) D399C, D399N, D399I, D399G, D399R, D399T, or D399A; d) Y407L, Y407A, Y407P, Y407F, Y407T, or Y407H; and e) K409C, K409P, K409S, K409F, K409V, K409Q, or K409R. Here, the position of the amino acid residue follows the EU index.

### Structure of fusion protein

The fusion protein may comprise polypeptide chains represented by the following structural formulas (I), (II), (III), and (IV), respectively:

N'-X-(L1)n-A-C' (I);

N'-Y-(L2)m-B-C' (II);

N'-C-C' (III);

and

N'-D-C' (IV)

wherein, in the structural formulas (I), (II), (III), and (IV),
N' is the N-terminus of each polypeptide,
C' is the C-terminus of each polypeptide,
- refers to a linkage,
A, B, C, and D are monomeric polypeptide sequences of an Fc domain each comprising the CH2 and CH3 regions of an immunoglobulin, and optionally further comprising CH4 and/or a hinge sequence, wherein
   A forms a dimer with one of C or D to form the first Fc domain (b), and
   B forms a dimer with the remaining one of C or D to form the second Fc domain (c);
   L1 and L2 are each peptide linker,
   n and m are each independently 0 or 1,
   X comprises a heavy chain variable region or a light chain variable region of an antibody that specifically binds to an antigen;
   Y comprises a light chain variable region or a heavy chain variable region of an antibody that specifically binds to an antigen; and
   X and Y pair with each other to form the antigen-binding site (a) that specifically binds to an antigen, and
   the polypeptides of (I), (II), (III), and (IV) may be assembled into a fusion protein comprising one antigen-binding site and two Fc domains.

Specifically, X is a first polypeptide sequence of the antigen-binding site, which comprises heavy chain CDR1, CDR2, and CDR3 sequences of an antibody that specifically binds to a first antigen, or a heavy chain variable region of an antibody that specifically binds to a first antigen; Y is a second polypeptide sequence of the antigen-binding site, which comprises light chain CDR1, CDR2, and CDR3 sequences of an antibody that specifically binds to a first antigen, or a light chain variable region of an antibody that specifically binds to a first antigen; and X and Y pair with each other to form the antigen-binding site (a) that specifically binds to an antigen.

According to one embodiment, the CH3 region may be mutated to minimize the interaction between A and B, and between C and D and promote the formation of a heterodimeric Fc between A and C, and between B and D. Specifically, the Fc domain monomer comprises a knob variant or a hole variant that promotes the formation of an Fc heterodimer (heterodimeric Fc); or the Fc domain monomer may comprise a variant that promotes the formation of a heterodimer by electrostatic steering mechanism.

According to one embodiment, X in the structural formula (I) may further comprise a heavy chain CH1 region, and/or Y in the structural formula (II) may further comprise a light chain constant region.

In addition, the fusion protein may comprise polypeptide chains represented by the following structural formulas (I'), (II'), (III), and (IV):

N'-VD1-(L3)p-X-(L1)n-A-C' (I');

N'-VD2-(L4)q-Y-(L2)m-B-C' (II');

N'-C-C' (III);

and

N'-D-C' (IV)

wherein, in the structural formulas (I'), (II'), (III), and (IV),
N' is the N-terminus of the polypeptide chain,
C' is the C-terminus of the polypeptide chain,
- refers to a linkage,
A, B, C, and D are monomeric polypeptide sequences of an Fc domain each comprising the CH2 and CH3 regions of an immunoglobulin, and optionally further comprising CH4 and/or a hinge sequence, wherein A forms a dimer with one of C or D to form the first Fc domain (b), and B forms a dimer with the remaining one of C or D to form the second Fc domain (c);
L1, L2, L3, and L4 are each peptide linker,
n, m, p, and q are each independently 0 or 1,
VD1 consists of a heavy chain or light chain variable region of an antibody that specifically binds to an antigen, or CDR1, CDR2, and CDR3 of an antibody heavy chain or light chain;
VD2 consists of a light chain or heavy chain variable region of an antibody that specifically binds to an antigen, or CDR1, CDR2, and CDR3 of an antibody heavy chain or light chain;
VD1 and VD2 pair with each other to form a second antibody variable region that specifically binds to a second antigen,
X comprises a heavy chain or light chain variable region of an antibody that specifically binds to an antigen, or CDR1, CDR2, and CDR3 of an antibody heavy chain or light chain;
Y comprises a light chain or heavy chain variable region of an antibody that specifically binds to an antigen, or CDR1, CDR2, and CDR3 of an antibody heavy chain or light chain; and
X and Y pair with each other to form a first antibody variable region that specifically binds to a first antigen, and
VD 1-(L3)p-X forms a first polypeptide sequence of the antigen-binding site (a), and VD2-(L4)q-Y forms a second polypeptide sequence of the antigen-binding site (a).

According to one embodiment, the CH3 region may be mutated to minimize the interaction between A and B, and between C and D and promote the formation of a heterodimeric Fc between A and C, and between B and D. Specifically, the Fc domain monomer comprises a knob variant or a hole variant that promotes the formation of an Fc heterodimer (heterodimeric Fc); or the Fc domain monomer may comprise a variant that promotes the formation of a heterodimer by electrostatic steering mechanism.

According to one embodiment, the heavy chain variable region may further comprise a heavy chain CH1 region. In addition, the light chain variable region may further comprise a light chain constant region.

In the structures of the fusion proteins described herein, the binding between X and Y may be achieved i) through a disulfide bond formed by Cys present in CH1 and a light chain constant region, ii) through a disulfide bond formed by Cys present in a heavy chain variable region and a light chain variable region, or iii) through a disulfide bond formed by Cys present in CH1 and a light chain constant region, and a disulfide bond formed by Cys present in a heavy chain variable region and a light chain variable region.

Specifically, the binding between X and Y may be formed by a disulfide bond present between CH₁233 and CL214 based on Kabat numbering system. In addition, X and Y may further comprise Cys through amino acid substitution. Examples of such variants may include mutations in the variable region, and specifically may include mutations at 105C of VH and 43C of VL, or mutations at 44C of VH and 100C of VL based on Kabat numbering system. In one embodiment, the mutation may be Q105C of VH and A43C of VL. In addition, in one embodiment, the mutation may be G44C of VH and Q100C of VL. In addition, examples of variants in the constant region may include mutations at 122C of CH1 and 121C of CL based on Kabat numbering system. In one embodiment, the mutation may be F122C of CH1 and S121C of CL.

### Linker and hinge

The hinge is a hinge region derived from immunoglobulins. In one embodiment, the antibody hinge region is an IgG hinge region. The IgG hinge region provided herein may be selected, for example, from antibody hinge regions of various IgG subtypes. The table below lists exemplary IgG subtypes with core hinge sequences that may be included in the flexible peptide regions provided herein. In addition, at least one Cys may exist within the hinge. Specifically, 1, 2, or 3 Cys may exist within the hinge.

**[Table 5]**

| IgG subtype | Sequence of core hinge | SEQ ID NO |
|---|---|---|
| IgG1 | EPKSCDKTHTCPPCP | 828 |
| IgG2 | ERKCCVECPPCP | 829 |
| IgG3 | ELKTPLDTTHTCPRCP(EPKSCDTPPPCPRCP)3 | 830 |
| IgG4 | ESKYGPPCPSCP | 831 |

The hinge may be modified to delete disulfide bonds or introduce additional disulfide bonds.

In addition, the linkers L1 and L2 may each comprise 1 to about 70 amino acids. According to one exemplary embodiment, L1 and L2 may each comprise about 5 to about 60 amino acids, about 10 to about 50 amino acids, about 15 to about 40 amino acids, or about 20 to about 30 amino acids. According to another exemplary embodiment, for example, L1 and L2 may each be a peptide consisting of 1-70 amino acid residues, 2-60 amino acid residues, 2-50 amino acid residues, 2-40 amino acid residues, 2-30 amino acid residues, 3-50 amino acid residues, 3-40 amino acid residues, 3-30 amino acid residues, 2-28 amino acid residues, 2-26 amino acid residues, 2-24 amino acid residues, 2-22 amino acid residues, 2-20 amino acid residues, 2-18 amino acid residues, 2-16 amino acid residues, 2-14 amino acid residues, 2-12 amino acid residues, or 2-10 amino acid residues. Specifically, L1 and L2 may include the amino acid sequence of (G4S)o (where o is an integer of 1 to 5) in Table 6 below, but are not limited thereto. In addition, L1 and L2 may have different amino acid sequences. In addition, here, L1 and L2 may comprise at least one Cys. In addition, a disulfide bond may be formed through Cys present in L1 and L2.

**[Table 6]**

| Sequence of linker | SEQ ID NO |
|---|---|
| GGGGS | 832 |
| GGGGSGGGGS | 833 |
| GGGGSGGGGSGGGGS | 6 |

In addition, the linkers L3 and L4 may each comprise 1 to about 30 amino acids. According to one exemplary embodiment, L3 and L4 may each comprise about 5 to about 25 amino acids, about 10 to about 20 amino acids, or about 15 amino acids. According to another exemplary embodiment, L3 and L4 may each be a peptide consisting of 2-30 amino acid residues, 2-25 amino acid residues, 2-20 amino acid residues, 2-15 amino acid residues, 3-30 amino acid residues, 2-28 amino acid residues, 2-26 amino acid residues, 2-24 amino acid residues, 2-22 amino acid residues, 2-20 amino acid residues, 2-18 amino acid residues, 2-16 amino acid residues, 2-14 amino acid residues, 2-12 amino acid residues, or 2-10 amino acid residues. Specifically, L3 and L4 may include the amino acid sequence of (G4S)o (where o is an integer of 1 to 5) in Table 6 above, but are not limited thereto. In addition, L3 and L4 may have different amino acid sequences.

### Specific examples of fusion proteins

### Fusion protein comprising one antigen-binding site and two Fcs

### i) Fusion protein in which antigen-binding site is Fab

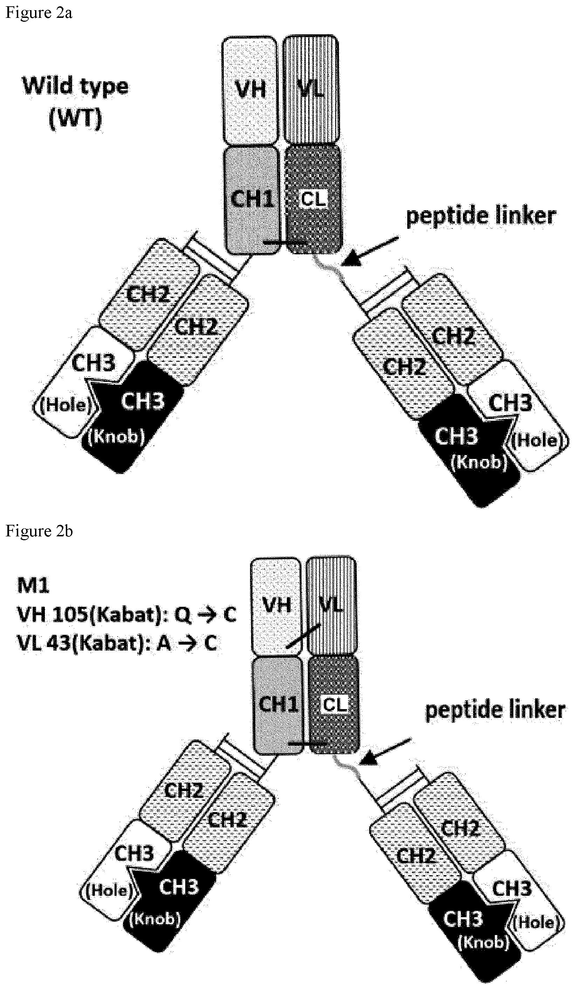

As shown in Figure 2a, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, the antigen-binding site, antigen, hinge, linker, and Fc are as described above.

As shown in Figure 2b, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, X comprises the mutation of 105C, and Y comprises the mutation of 43C, and a disulfide bond between Cys is formed. In addition, the antigen-binding site, antigen, hinge, linker, and Fc are as described above.

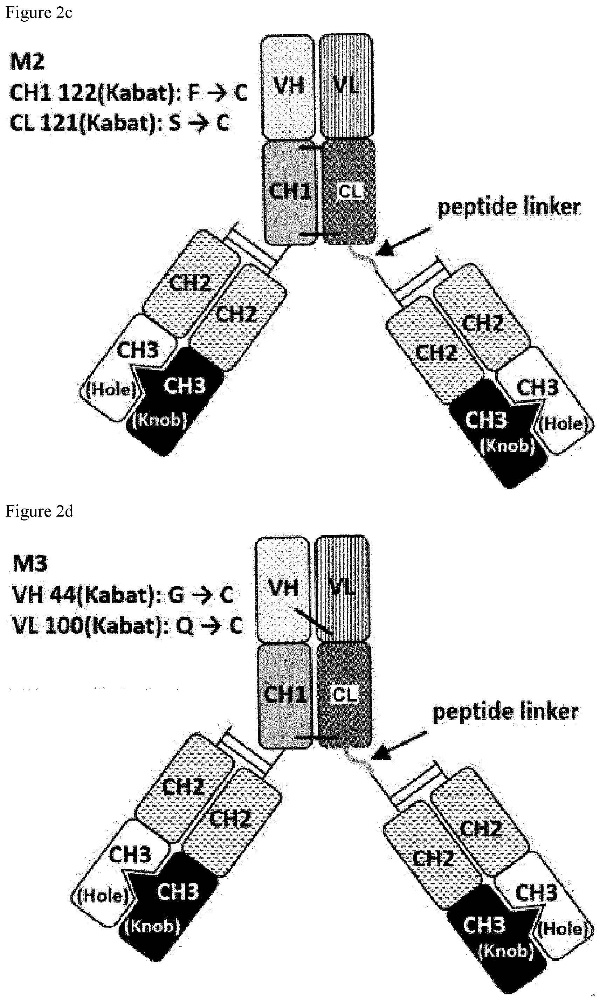

As shown in Figure 2c, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, CH1 comprises the mutation of 122C, and the light chain constant region comprises the mutation of 121C, and a disulfide bond between Cys is formed. In addition, the antigen-binding site, antigen, hinge, linker, and Fc are as described above.

As shown in Figure 2d, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, X comprises the mutation of 44C, and Y comprises the mutation of 100C, and a disulfide bond between Cys is formed. In addition, the antigen-binding site, antigen, hinge, linker, and Fc are as described above.

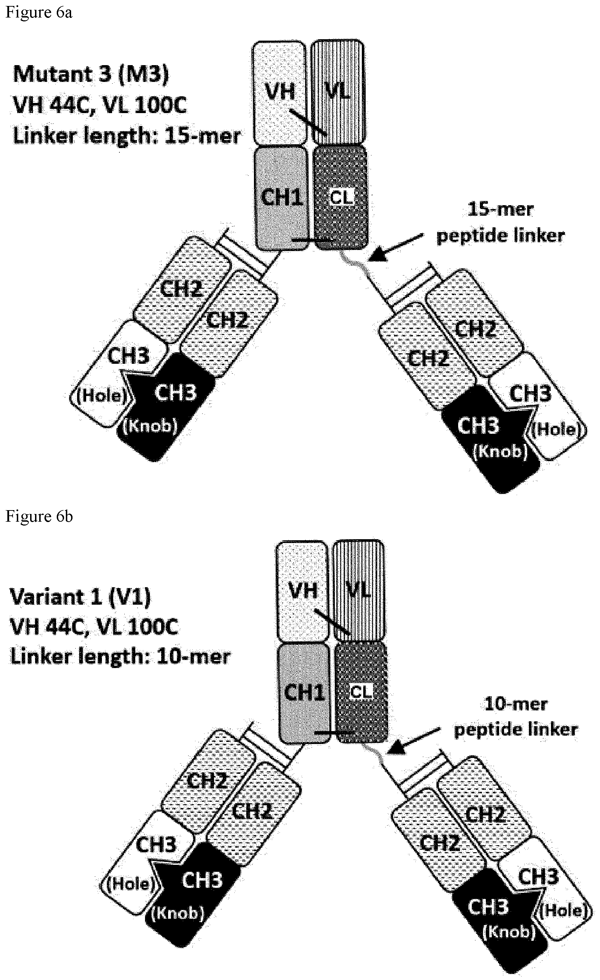

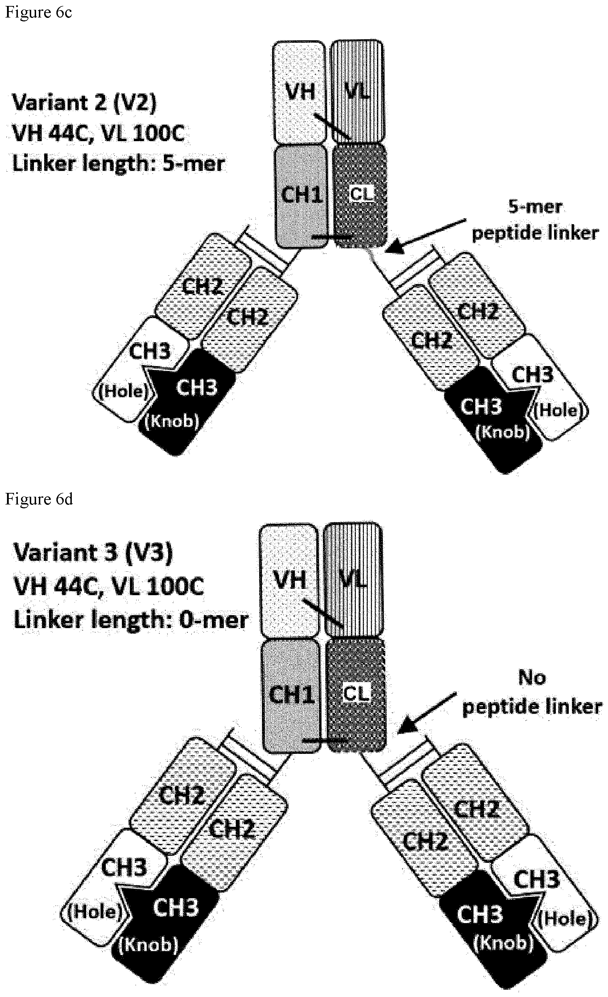

As shown in Figures 6a to 6d, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, and CH1 is directly linked to the hinge. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, X comprises the mutation of 44C, and Y comprises the mutation of 100C, and a disulfide bond between Cys is formed. Here, m is 0, and the light chain variable region may be directly linked to the hinge (Figure 6d). In addition, m is 1, and L2 may include a 15-mer peptide linker (Figure 6a), a 10-mer peptide linker (Figure 6b), or a 5-mer peptide linker (Figure 6c). In addition, the antigen-binding site, antigen, hinge, linker, and Fc are as described above.

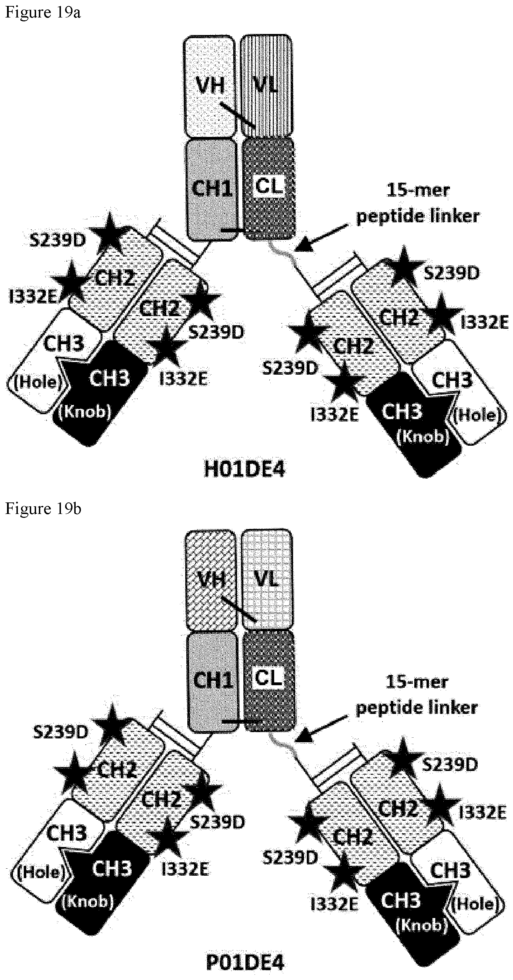

As shown in Figure 19a, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, X comprises the mutation of 44C, and Y comprises the mutation of 100C, and a disulfide bond between Cys is formed. Here, all CH2s of A, B, C, and D comprise the 239D and 332E mutations. In addition, the antigen-binding site, antigen, hinge, linker, and Fc are as described above.

### ii) Fusion protein in which antigen-binding site is Fv

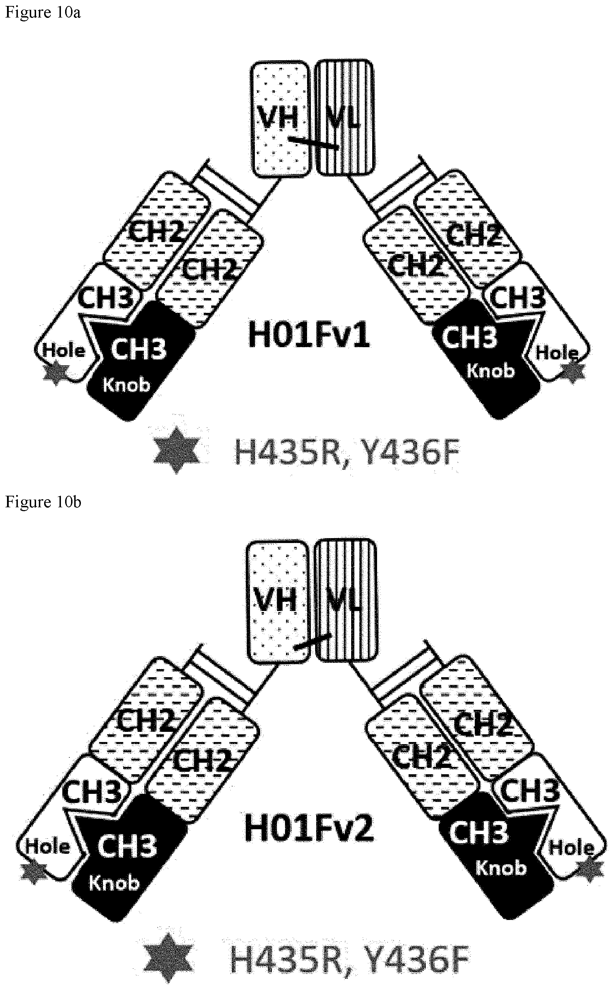

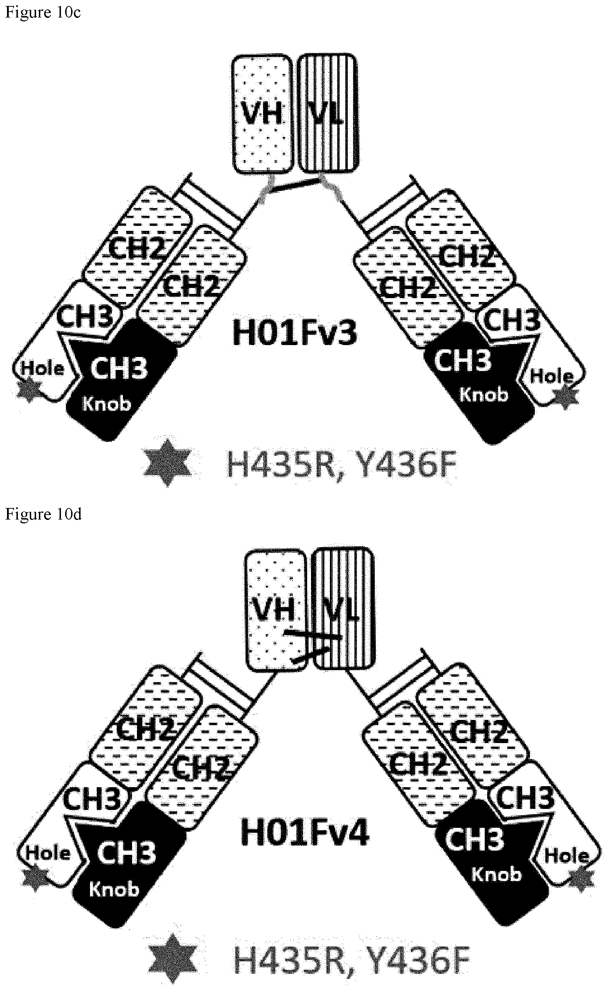

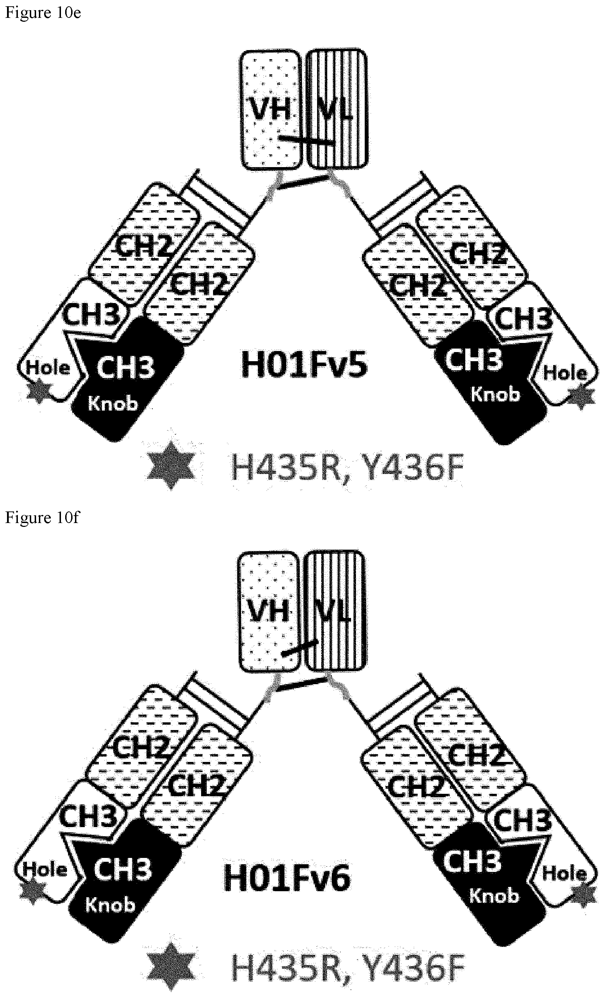

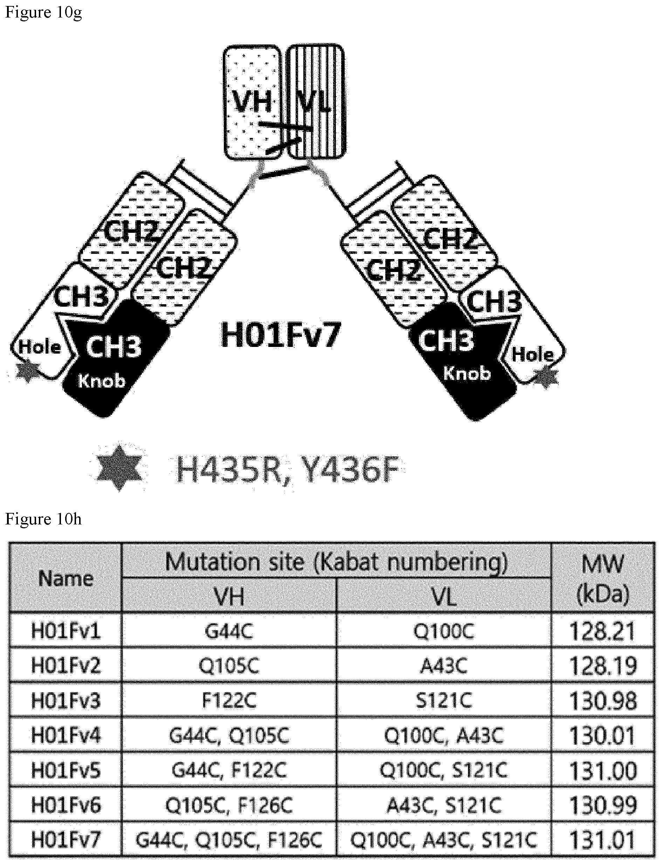

As shown in Figure 10, the fusion protein comprises polypeptides of the structural formulas (I), (II), (III), and (IV), where X and Y are attached to each other by at least one Cys present therein to form a Fv structure. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. X may comprise the mutations of 44C, 105C, 122C, 44C/105C, 44C/122C, 105C/126C or 44C/105C/126C, and Y may comprise the mutations of 100C, 43C, 121C, 100C/43C, 100C/121C, 43C/121C, or 100C/43C/121C. In addition, a disulfide bond may be formed by Cys present in L1 and L2. In addition, the antigen-binding site, antigen, hinge, linker, and Fc are as described above.

### Fusion protein comprising two antigen-binding sites and two Fcs

### iii) Fusion protein in which antigen-binding site is Fab

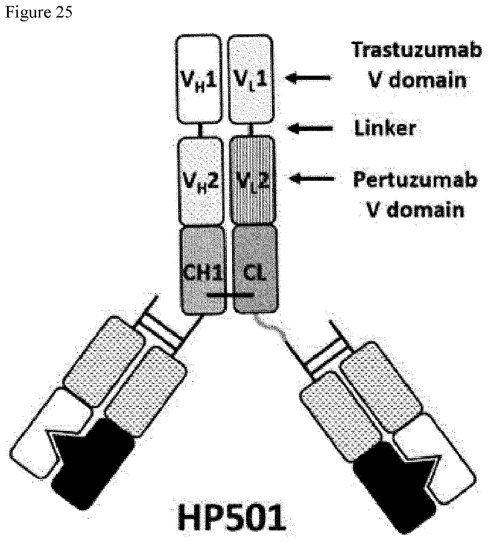

As shown in Figure 25, the fusion protein comprises polypeptides of the structural formulas (I'), (II'), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, VD1 in the structural formula (I') is a heavy chain variable region, and VD2 in the structural formula (II') is a light chain variable region, and VD1 and VD2 pair with each other to form Fv. In addition, p and q are each 1, and L3 and L4 are peptide linkers. As a non-limiting example, X and Y may pair with each other to form the variable region of pertuzumab, and VD1 and VD2 may pair with each other to form the variable region of trastuzumab. In addition, the antigen-binding site, antigen, hinge, linker, and Fc are as described above.

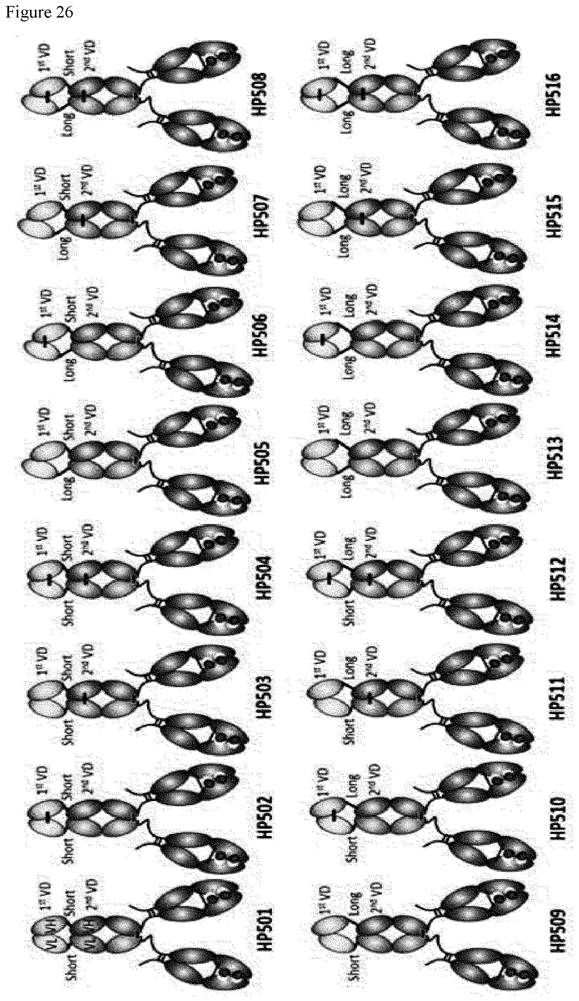

As shown in Figure 26, the peptide linkers of L3 and L4 in the structural formulas (I') and (II') may be of various lengths. In addition, the first antigen-binding site formed by pairing between X and Y, and the second antigen-binding site formed by pairing between VD1 and VD2 may be the same or different. In addition, L1 and L2 may also comprise various peptide linkers.

### Use of fusion protein

In another aspect of the present invention, there is provided a pharmaceutical composition for preventing or treating cancer, comprising the fusion protein as an active ingredient.

Here, cancer may be any one selected from the group consisting of gastric cancer, liver cancer, lung cancer, large intestine cancer, breast cancer, prostate cancer, skin cancer, bone cancer, multiple myeloma, glioma, ovarian cancer, pancreatic cancer, cervical cancer, thyroid cancer, laryngeal cancer, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer, and lymphoma.

### Polynucleotide encoding fusion protein

In another aspect of the present invention, there is provided a polynucleotide encoding a polypeptide of the structural formula (I), (II), (III), and/or (IV).

In another aspect of the present invention, there is provided a polynucleotide encoding a polypeptide of the structural formula (I'), (II'), (III), and/or (IV).

The polynucleotide may further comprise a nucleic acid encoding a signal sequence or a leader sequence. As used herein, the term "signal sequence" refers to a signal peptide that directs secretion of a target protein. The signal peptide is translated and then cleaved in a host cell. Specifically, the signal sequence is an amino acid sequence that initiates migration of a protein across the endoplasmic reticulum (ER) membrane.

The signal sequences are well known in the art for their characteristics. Such signal sequences typically comprise 16 to 30 amino acid residues, and may comprise more or fewer amino acid residues than such amino acid residues. Atypical signal peptide consists of three regions, that is, a N-terminal region, a central hydrophobic region, and a more polar C-terminal region. The central hydrophobic region comprises 4 to 12 hydrophobic residues that cause the signal sequence to be immobilized during migration of an immature polypeptide through the membrane lipid bilayer.

After initiation, signal sequences are cleaved in the lumen of ER by cellular enzymes, commonly known as signal peptidases. Here, the signal sequence may be a secretory signal sequence of tPa (tissue plasminogen activator), HSV gDs (signal sequence of Herpes simplex virus glycoprotein D), or a growth hormone. Preferably, a secretory signal sequence used in higher eukaryotic cells including mammals and the like may be used. In addition, as the signal sequence, a wild type signal sequence may be used, or a signal sequence that has been substituted with a codon having high expression frequency in a host cell may be used.

### Vector loaded with polynucleotide

In another aspect of the present invention, there is provided a vector comprising the polynucleotide. The vector may comprise a polynucleotide encoding a polypeptide of the structural formula (I), (II), (III), and/or (IV). In addition, the vector may comprise a polynucleotide encoding a polypeptide of the structural formula (I'), (II'), (III), and/or (IV).

The vector may be introduced into a host cell to be recombined with and inserted into the genome of the host cell. Alternatively, the vector is understood as nucleic acid means comprising a polynucleotide sequence which is autonomously replicable as an episome. The vectors include linear nucleic acids, plasmids, phagemids, cosmids, RNA vectors, viral vectors, and analogs thereof. Examples of the viral vector include, but are not limited to, retroviruses, adenoviruses, and adeno-associated viruses.

Specifically, the vector may include plasmid DNA, phage DNA, and the like; and commercially developed plasmids (pUC18, pBAD, pIDTSAMRT-AMP, and the like), *E. coli-*derived plasmids (pYG601BR322, pBR325, pUC118, pUC119, and the like), *Bacillus subtilis-derived* plasmids (pUB110, pTP5, and the like), yeast-derived plasmids (YEp13, YEp24, YCp50, and the like), phage DNA (Charon4A, Charon21A, EMBL3, EMBL4, λgt10, λgt11, λZAP, and the like), animal viral vectors (retroviruses, adenoviruses, vaccinia viruses, and the like), insect viral vectors (baculoviruses and the like). Since the vector exhibits different expression levels and modification of a protein depending on a host cell, it is preferred to select and use a host cell which is most suitable for the purpose.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. The expression vector may comprise human cytomegalovirus (CMV) promoter for promoting continuous transcription of a target gene in mammalian cells, and a bovine growth hormone polyadenylation signal sequence for increasing the stability level of RNA after transcription.

### Transformed cell expressing fusion protein

In another aspect of the present invention, there is provided a transformed cell expressing. Specifically, the transformed cell may be one into which the vector has been introduced.

Host cells for the transformed cell may include, but are not limited to, prokaryotic cells, eukaryotic cells, and cells of mammalian, plant, insect, fungal, or cellular origin. As an example of the prokaryotic cells, *E. coli* may be used. In addition, as an example of the eukaryotic cells, yeast may be used. In addition, for the mammalian cells, CHO cells, F2N cells, CSO cells, BHK cells, Bowes melanoma cells, HeLa cells, 911 cells, AT1080 cells, A549 cells, HEK 293 cells, HEK293T cells, or the like may be used. However, the mammalian cells are not limited thereto, and any cells which are known to those of ordinary skill in the art to be usable as mammalian host cells may be used.

In addition, for the introduction of an expression vector into the host cell, CaCl₂ precipitation, Hanahan method whose efficiency has been increased efficiency by using a reducing agent such as dimethyl sulfoxide (DMSO) in CaCl₂ precipitation, electroporation, calcium phosphate precipitation, protoplast fusion, agitation using silicon carbide fiber, Agrobacteria-mediated transformation, transformation using PEG, dextran sulfate-, Lipofectamine-, and dry/inhibition-mediated transformation, and the like may be used.

As described above, for optimization of properties of a fusion protein as a therapeutic agent or for any other purpose, glycosylation pattern of the fusion protein (for example, sialic acids, fucosylations, glycosylations) may be adjusted by manipulating, through methods known to those of ordinary skill in the art, glycosylation-related genes possessed by host cells.

### Method for producing a fusion protein

In another aspect of the present invention, there is provided a method for producing a fusion protein comprising an antigen-binding site, a first Fc domain or a variant thereof, and a second Fc domain or a variant thereof, the method comprising the steps of: i) culturing the transformed cells; and ii) collecting the produced fusion proteins.

The step of culturing the transformed cells may be carried out using methods well known in the art. Specifically, the culture may be carried out in a batch process, or carried out continuously in a fed batch or repeated fed batch process.

### Composition or preparation containing fusion protein

In another aspect of the present invention, there is provided a pharmaceutical composition comprising the fusion protein as an active ingredient.

The pharmaceutical composition may be used for the prevention or treatment of cancer, such as any one cancer selected from the group consisting of gastric cancer, liver cancer, lung cancer, large intestine cancer, breast cancer, prostate cancer, gallbladder cancer, bladder cancer, kidney cancer, esophageal cancer, skin cancer, rectal cancer, osteosarcoma, multiple myeloma, glioma, ovarian cancer, pancreatic cancer, cervical cancer, endometrial cancer, thyroid cancer, laryngeal cancer, testicular cancer, mesothelioma, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer, and lymphoma.

A preferred dosage of the pharmaceutical composition varies depending on the patient's condition and body weight, severity of disease, form of drug, route and duration of administration and may be appropriately selected by those of ordinary skill in the art. In the pharmaceutical composition for treating or preventing tumor of the present invention, the active ingredient may be contained in any amount (effective amount) depending on application, dosage form, blending purpose, and the like, as long as the active ingredient may exhibit therapeutic activity against tumor or, in particular, may exhibit a therapeutic effect on cancer. A conventional effective amount thereof will be determined within a range of 0.001% to 20.0% by weight, based on the total weight of the composition. Here, the term "effective amount" refers to an amount of an active ingredient that may induce an effect of improving or treating the condition of a disease, especially an effect of improving or treating the condition of cancer. Such an effective amount may be experimentally determined within the scope of common knowledge of those of ordinary skill in the art.

As used herein, the term "treatment" may be used to mean both therapeutic and prophylactic treatment. Here, prophylaxis may be used to mean that a pathological condition or disease of a subject is alleviated or mitigated. In one embodiment, the term "treatment" includes both application or any form of administration for treating a disease in a mammal, including a human. In addition, the term includes inhibiting or slowing down the progression of a disease; and includes meanings of restoring or repairing impaired or lost function so that a disease is partially or completely alleviated; stimulating inefficient processes; or alleviating a serious disease.

Pharmacokinetic parameters such as bioavailability and underlying parameters such as clearance rate may also affect efficacy. Therefore, "improved efficacy" (for example, improvement in efficacy) may be due to improved pharmacokinetic parameters and improved efficacy, which may be measured by comparing clearance rate in test animals or human subjects, and parameters such as tumor treatment or improvement.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, type and severity of disease, activity of drug, the patient's sensitivity to drug, mode of administration, time of administration, route of administration and excretion rate, duration of treatment, combined or simultaneously used drugs, and other factors well known in the medical field. In one embodiment, the therapeutically effective amount means an amount of drug effective to treat cancer.

Here, the pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffer, dispersant) may also be contained in the pharmaceutical composition.

Specifically, by including a pharmaceutically acceptable carrier in addition to the active ingredient, the pharmaceutical composition may be prepared into a parenteral formulation depending on its route of administration using conventional methods known in the art. Here, the term "pharmaceutically acceptable" means that the carrier does not have more toxicity than the subject to be applied (prescribed) may adapt while not inhibiting activity of the active ingredient.

When the pharmaceutical composition is prepared into a parenteral formulation, it may be made into preparations in the form of injections, transdermal patches, nasal inhalants, or suppositories with suitable carriers according to methods known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) containing triethanol amine or sterile water for injection, and 5% dextrose, or the like may preferably be used. Formulation of pharmaceutical compositions is known in the art, and reference may specifically be made to Remington's Pharmaceutical Sciences (19th ed., 1995) and the like. This document is considered part of the present specification.

A preferred dosage of the pharmaceutical composition may range from 0.01 µg/kg to 10 g/kg, or 0.01 mg/kg to 1 g/kg, per day, depending on the patient's condition, body weight, sex, age, severity of the patient, and route of administration. The dosage may be administered once a day or may be divided into several times a day. Such a dosage should not be construed as limiting the scope of the present invention in any aspect.

Subjects to which the pharmaceutical composition may be applied (prescribed) are mammals including dogs, cats, humans, etc., with humans being particularly preferred. In addition to the active ingredient, the pharmaceutical composition of the present invention may further comprise any compound or natural extract, which is known to have a therapeutic effect on tumor.

### Treatment method using fusion protein

In another aspect of the present invention, there is provided a method for treating or preventing cancer, comprising administering a fusion protein comprising an antigen-binding site, a first Fc domain or a variant thereof, and a second Fc domain or a variant thereof administering to a subject.

In another aspect of the present invention, there is provided a use of a fusion protein comprising an antigen-binding site, a first Fc domain or a variant thereof, and a second Fc domain or a variant thereof for the treatment of cancer.

Here, the subject may be an individual suffering from cancer. In addition, the subject may be a mammal, preferably a human.

Route of administration, dosage, and frequency of administration of the fusion protein may vary depending on the patient's condition and the presence or absence of side effects, and thus the fusion protein may be administered to a subject in various ways and amounts. The optimal administration method, dosage, and frequency of administration may be selected in an appropriate range by those of ordinary skill in the art. In addition, the fusion protein may be administered in combination with other drugs or physiologically active substances whose therapeutic effect is known with respect to a disease to be treated, or may be formulated in the form of combination preparations with other drugs.

### Mode for Carrying out the Invention

Hereinafter, the present invention will be described in more detail by way of the following examples. However, the following examples are only for illustrating the present invention, and the scope of the present invention is not limited only to these examples.

### Example 1. Design, preparation and analysis of a novel antibody with two Fc domains

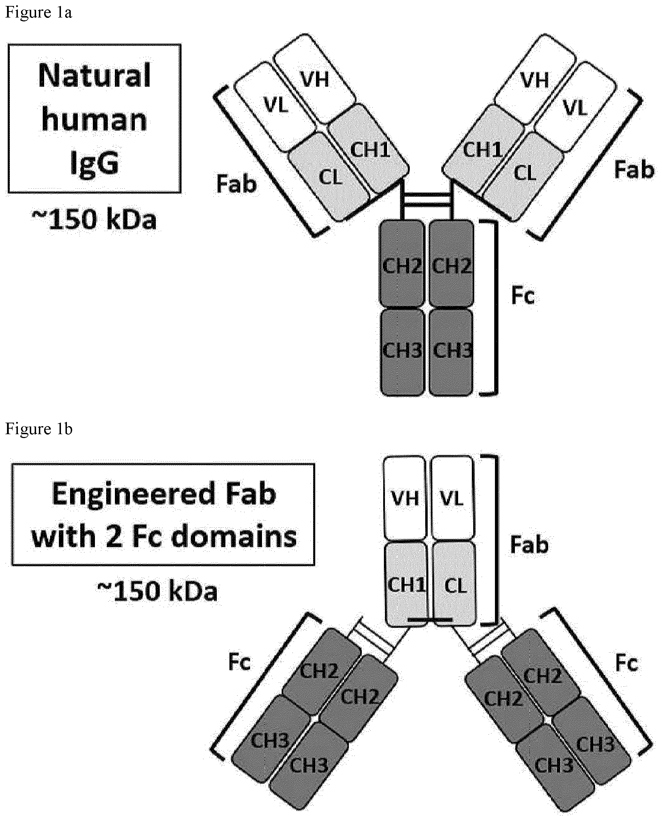

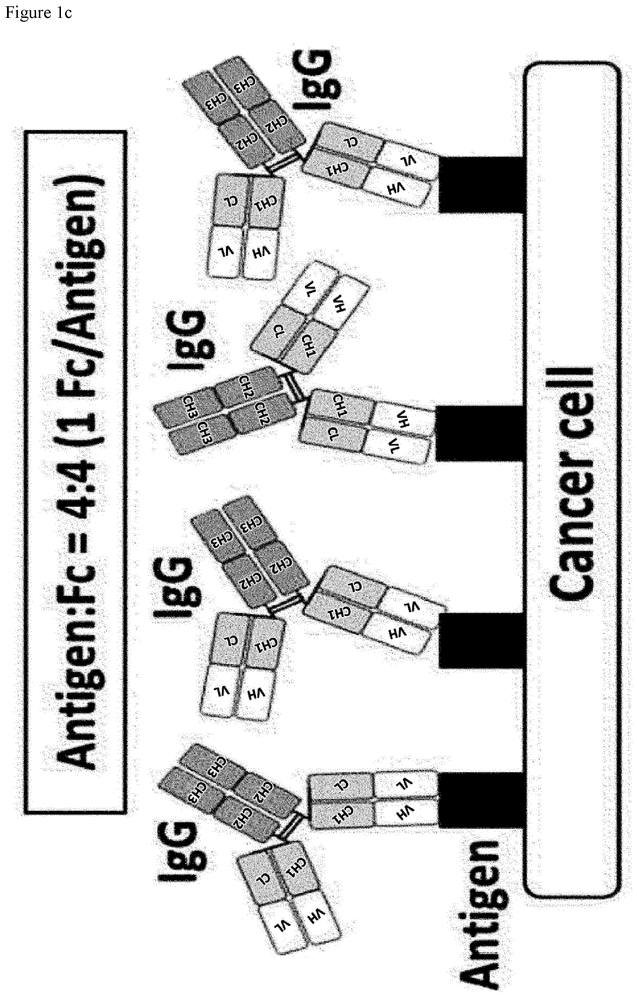

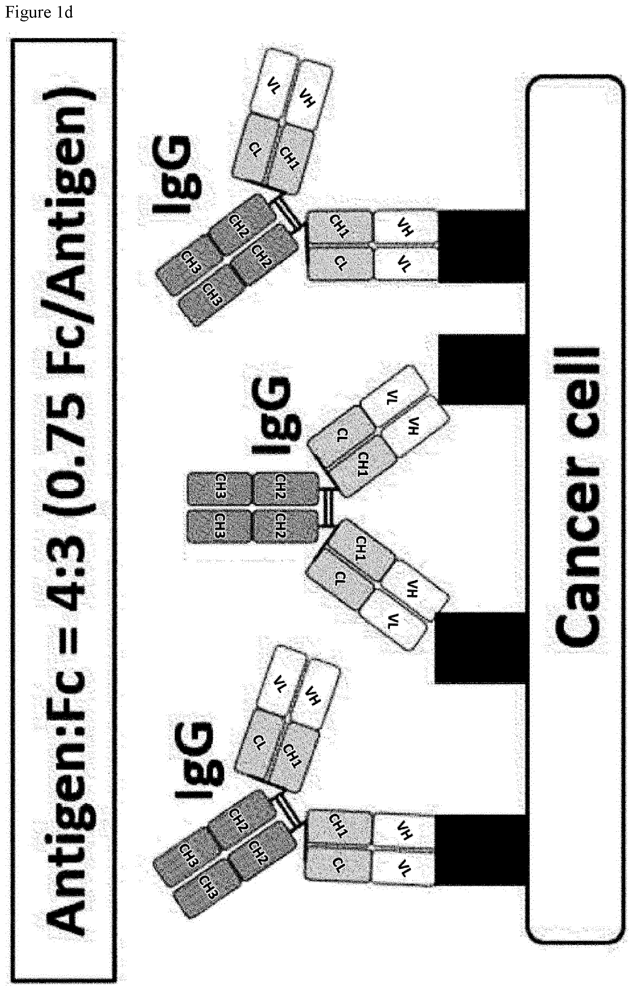

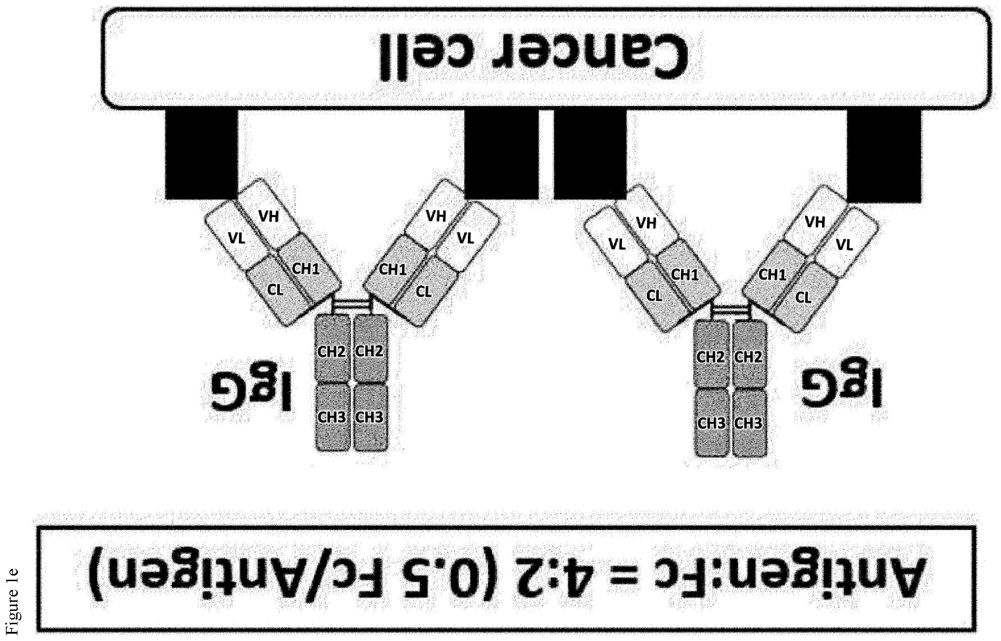

Naturally occurring human immunoglobulin G (IgG) consists of two fragment antigen-binding (Fab) regions and one fragment crystallizable (Fc) region (Figure 1a). Human IgG binds to the target antigen in a monovalent or bivalent manner, and in some cases has 0.5 to 1 Fc region per target antigen (Figures 1c, 1d, and 1e).

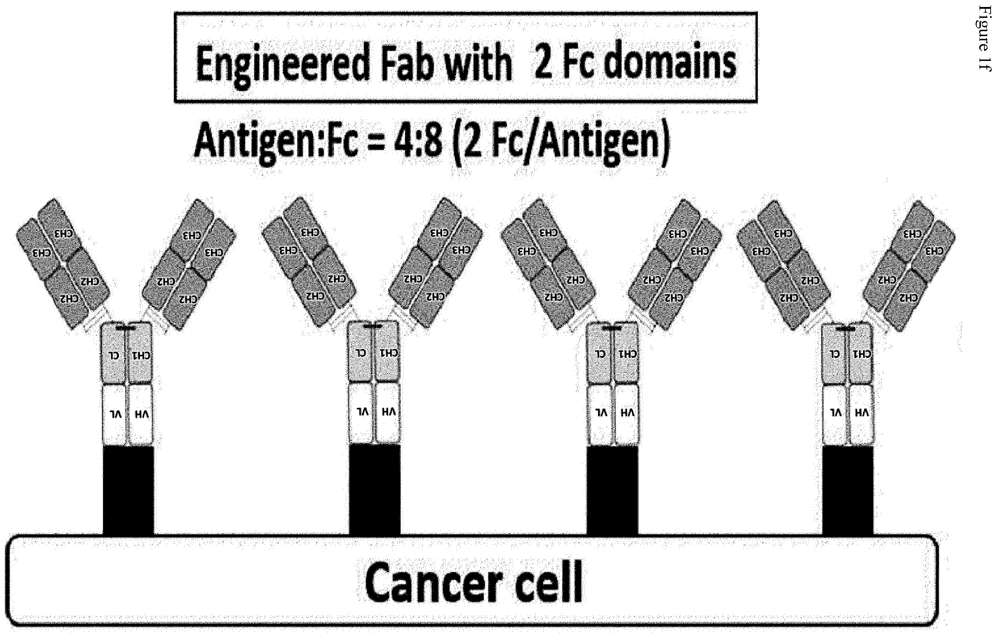

The object of the present invention is to improve effector function by increasing the amount of Fc region present per antigen while having a molecular weight similar to that of an antibody and having a homogeneous composition. Therefore, we designed a novel antibody format with two Fc regions that has a molecular weight similar to that of a natural human IgG antibody (approximately 150 kDa) (Figure 1b). This form binds to a cancer cell surface antigen and enables Fc regions to be present on a cancer cell surface a maximum of four times compared to conventional antibodies (Figures 1b and 1f).

In order to implement the novel antibody format mentioned above, trastuzumab was used as a template (Figure 2a). In order to improve pairing between the VH-CH1 region and the VL-CL region of the trastuzumab Fab region, a specific amino acid was substituted with cysteine to introduce an artificial disulfide bond (Figures 2b, 2c, and 2d).

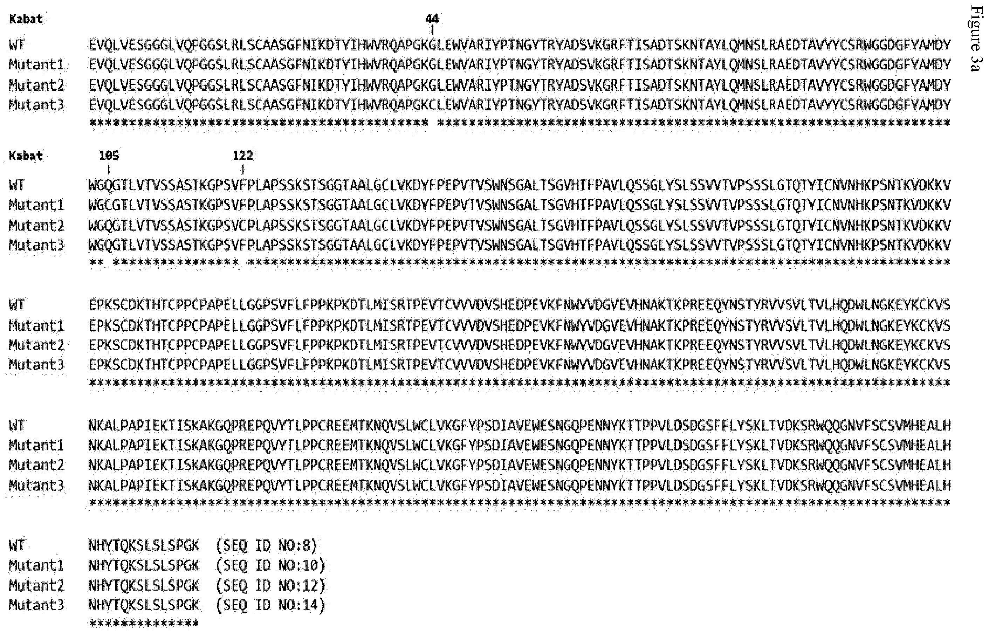

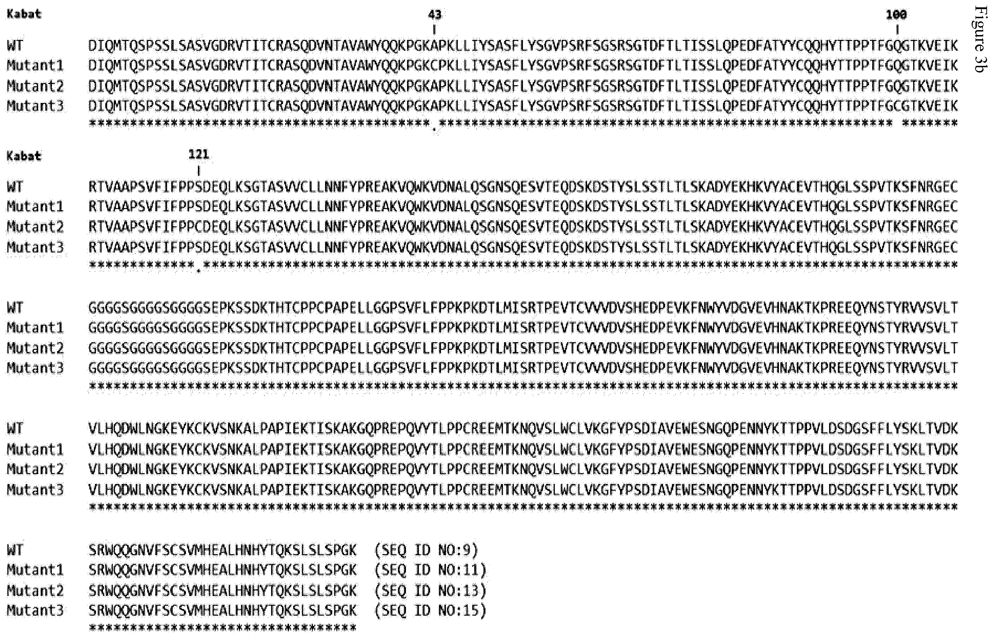

Fab in which glutamine (Q) at number 105 of the heavy chain and alanine (A) at number 43 of the light chain are substituted with cysteines, Fab in which phenylalanine (F) at number 122 of the heavy chain and serine (S) at number 121 of the light chain are substituted with cysteines, and Fab in which glycine (G) at number 44 of the heavy chain and glutamine (Q) at number 100 of the light chain are substituted with cysteines were designed, and they were referred to as Mutant 1, Mutant 2, and Mutant 3, respectively (Figures 2b, 2c, 2d, 3a, and 3b). Based on the above, the Fab-(Fc)₂ structure with trastuzumab as a template was referred to as wild type (WT) (Figure 2a), and the Fab-(Fc)₂ structures with Fab corresponding to Mutants 1 to 3 were referred to as respective M1, M2, and M3 (Figures 2b, 2c, 2d, 3a, and 3b).

The notation of the positions in amino acids constituting an antibody follows the Kabat numbering system. In order to minimize unwanted Fc-related byproducts, knob-into-hole mutation technology (Merchant et al., Nat. Biotechnol. 1998) was applied to the Fc domain (SEQ ID NO: 3) of human immunoglobulin G1 (IgG1) to design polypeptides of Fc with knob mutation (S354C and T366W; SEQ ID NO: 4) and Fc with hole mutation (Y349C, T366S, L368A, and Y407V; SEQ ID NO: 5). In order to provide additional flexibility between the CL domain and hinge region, a (G₄S)₃ linker was introduced (SEQ ID NO: 6; Figure 2). The expression of WT was performed by co-transfection of vectors capable of expressing polypeptides corresponding to Fc-Hole (SEQ ID NO: 7), TraH-WT-Knob (SEQ ID NO: 8), and TraL-WT-Knob (SEQ ID NO: 9) into the EXPICHO-S^{™} (Gibco, A29127) cell line.

M1 consists of Fc-Hole (SEQ ID NO: 7), TraH-Q105C-Knob (SEQ ID NO: 10), and TraL-A43C-Knob (SEQ ID NO: 11), M2 consists of Fc-Hole (SEQ ID NO: 7), TraH-F122C-Knob (SEQ ID NO: 12), and TraL-S121C-Knob (SEQ ID NO: 13), and M3 consists of Fc-Hole (SEQ ID NO: 7), TraH-G44C-Knob (SEQ ID NO: 14), and TraL-Q100C-Knob (SEQ ID NO: 15). All of M1, M2, and M3 were expressed in the EXPICHO-S^{™} (Gibco, A29127) cell line. They were purified using an AKTA pure 25 (Cytiva) or AKTA avant 150 (Cytiva) protein isolation and purification system equipped with a CAPTURESELECT^{™} CH1-XI, Pre-packed Column (Thermo Scientific, 494346205) purification column, and the purified product was further subjected to affinity chromatography using KappaSelect resin (Cytiva, 17545801), and the sample was concentrated using an Amicon Ultra-15 Centrifugal Filter Unit (Merck millipore). For the final purified product, the absorbance of the sample at 280 nm was measured using a NanoDrop One trace spectrophotometer (Thermo Fisher Scientific), and the concentration was quantified based on the sample's intrinsic extinction coefficient and molecular weight.

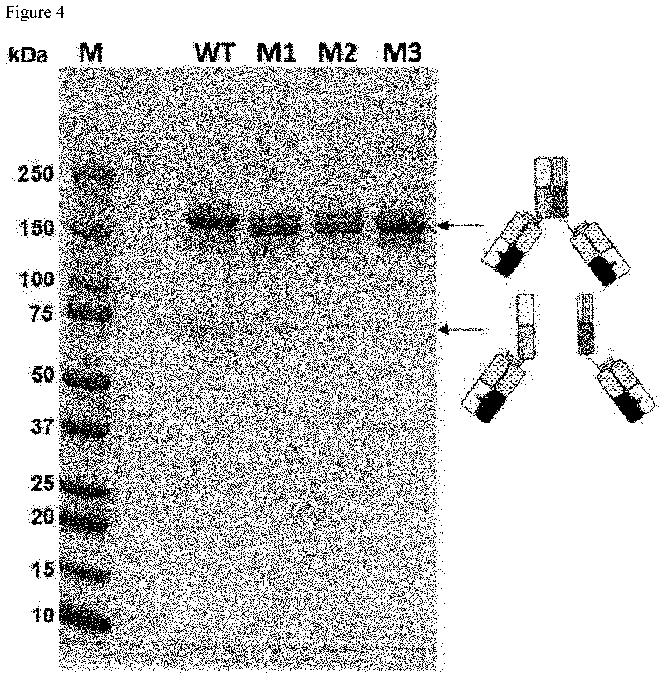

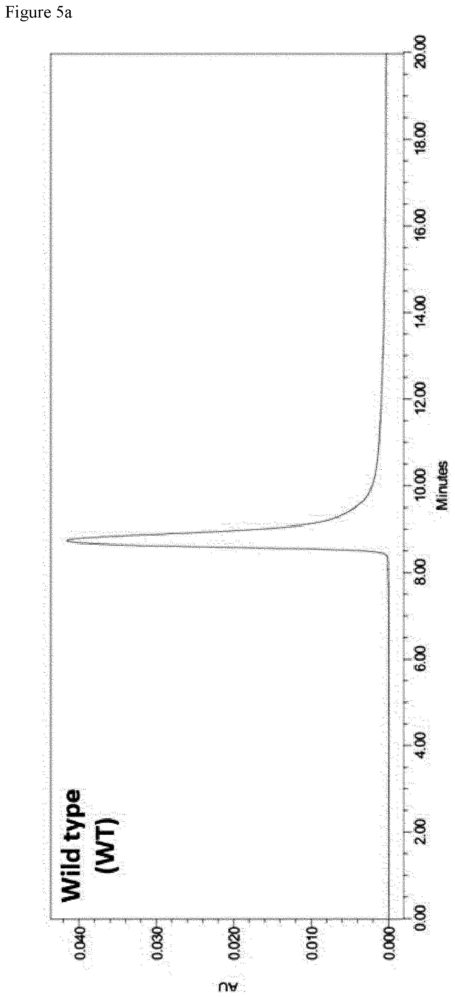

The purified product was analyzed using sodium dodecyl sulfate polyacrylamide gel electrophoresis (SDS-PAGE) and size exclusion chromatography (SEC) (Figures 4 and 5a to 5d). Bio-rad's electrophoresis gel and system were used for SDS-PAGE analysis, and samples were analyzed under non-reducing conditions, and size of each band were identified using Coomassie Brilliant Blue staining (Figure 4). WT, M1, M2, and M3 were identified at about 150 kDa, and monomers were identified at about 75 kDa in WT which has no additional disulfide bond introduced into the Fab interface (Figure 4). Similarly, for M1 and M2, a trace amount of monomer was identified at 75 kDa. For M3, almost no monomers were identified probably because most of the monomers were easily paired through the formation of disulfide bonds (Figure 4).

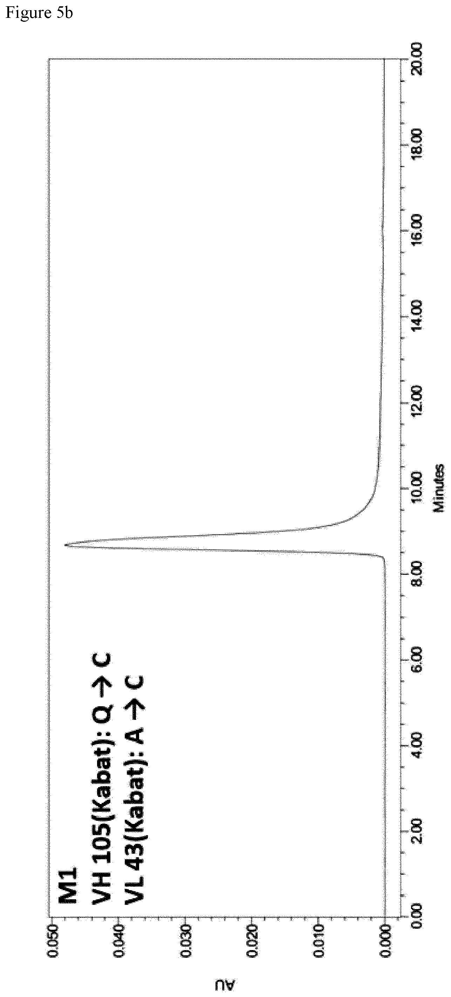

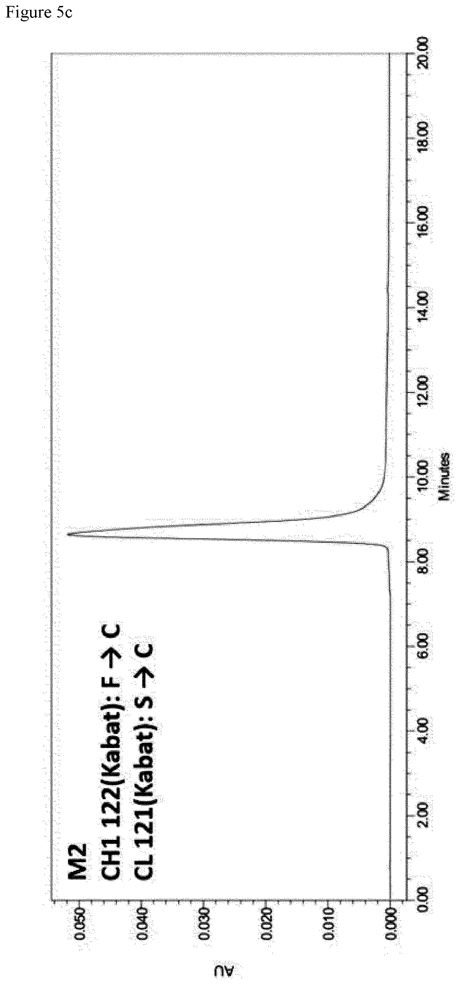

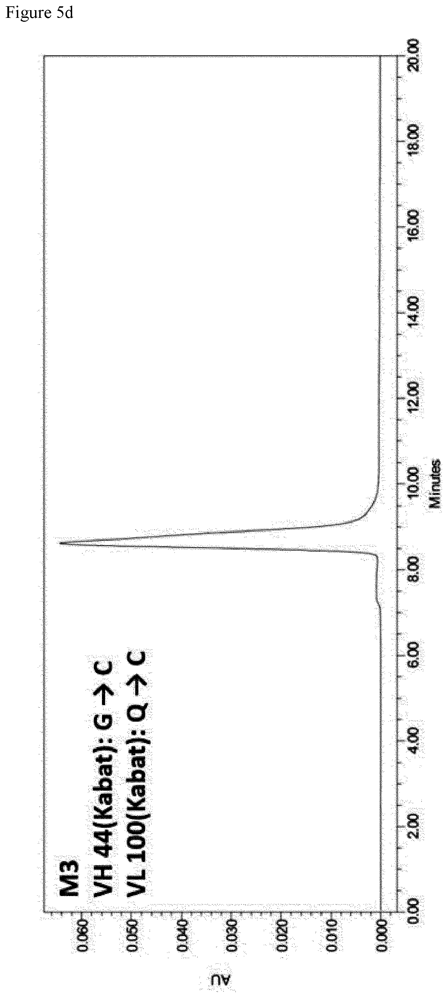

For size exclusion chromatography analysis, an ALLIANCE^{®} HPLC - e2695 Separations Module (Waters, 2695) equipped with an Agilent Bio SEC-3 HPLC column (Agilent, 5190-2511) was used. The analysis showed that the main product was identified at a retention time of 8.6 to 8.8 minutes (Figures 5a, 5b, 5c, and 5d).

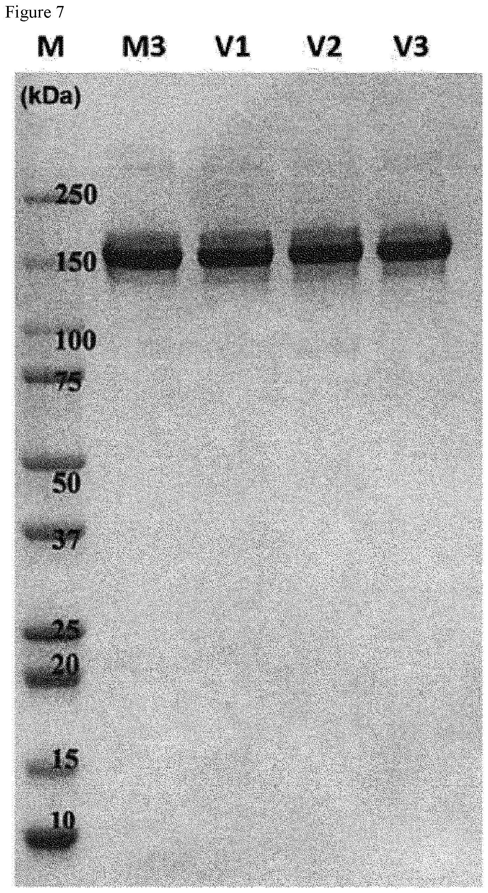

Based on the M3 structure, the impact of the linker that connects the CL domain with the hinge region on the structural integrity of the antibody were analyzed. M3 has a 15-mer polypeptide linker consisting of (G₄S)₃, and V1 (SEQ ID NO: 7, 14, and 16) and V2 (SEQ ID NO: 7, 14, and 17) have polypeptide linkers of (G₄S)₂ and G₄S, respectively, and V3 (SEQ ID NO: 7, 14, and 18) directly linked the CL domain and the hinge region without a linker (Figure 6). By SDS-PAGE analysis, the main product was identified at about 150 kDa, and the byproduct was not identified (Figure 7). It was found that the presence or absence of a linker between the CL domain and the hinge region had no significant effect on the formation of the byproduct.

Based on these results, it was found that the Fab-(Fc)₂ structure was stably formed when the VH 44 and VL 100 positions of Fab were substituted with respective cysteines.

**[Table 7]**

| Name | Polypeptide sequence | SEQ ID NO |
|---|---|---|
| Trastuzumab VH-CH1 domain | EVQLVESGGGLVQPGGSLRLSCAASGFNIKDTYIHWVRQAPGKGLEW VARIYPTNGYTRYADSVKGRFTISADTSKNTAYLQMNSLRAEDTAVY YCSRWGGDGFYAMDYWGQGTLVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKV | 1 |
| Trastuzumab VL-CL domain | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGQGTKVEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGEC | 2 |
| IgG1 Fc(CH2-CH3) | APELLGGPSVFLFPPKPKDTLMISRTPEVTCVVVDVSHEDPEVKFNWY VDGVEVHNAKTKPREEQYNSTYRVVSVLTVLHQDWLNGKEYKCKV SNKALPAPIEKTISKAKGQPREPQVYTLPPSREEMTKNQVSLTCLVKG FYPSDIAVEWESNGQPENNYKTTPPVLDSDGSFFLYSKLTVDKSRWQ QGNVFSCSVMHEALHNHYTQKSLSLSPGK | 3 |
| IgG1 Fc(Knob; S354C, T366W) | APELLGGPSVFLFPPKPKDTLMISRTPEVTCVVVDVSHEDPEVKFNWY VDGVEVHNAKTKPREEQYNSTYRVVSVLTVLHQDWLNGKEYKCKV SNKALPAPIEKTISKAKGQPREPQVYTLPPCREEMTKNQVSLWCLVKG FYPSDIAVEWESNGQPENNYKTTPPVLDSDGSFFLYSKLTVDKSRWQ QGNVFSCSVMHEALHNHYTQKSLSLSPGK | 4 |
| IgG1 Fc(Hole; Y349C, T366S, | APELLGGPSVFLFPPKPKDTLMISRTPEVTCVVVDVSHEDPEVKFNWY VDGVEVHNAKTKPREEQYNSTYRVVSVLTVLHQDWLNGKEYKCKV | 5 |
| L368A, Y407V) | SNKALPAPIEKTISKAKGQPREPQVCTLPPSREEMTKNQVSLSCAVKG FYPSDIAVEWESNGQPENNYKTTPPVLDSDGSFFLVSKLTVDKSRWQ QGNVFSCSVMHEALHNHYTQKSLSLSPGK | |
| 15-mer Linker | GGGGSGGGGSGGGGS | 6 |
| Fc-Hole | DKTHTCPPCPAPELLGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVCTLPPSREEMTKNQ VSLSCAVKGFYPSDIAVEWESNGQPENNYKTTPPVLDSDGSFFLVSKL TVDKSRWQQGNVFSCSVMHEALHNHYTQKSLSLSPGK | 7 |
| TraH-WT-Knob | EVQLVESGGGLVQPGGSLRLSCAASGFNIKDTYIHWVRQAPGKGLEW VARIYPTNGYTRYADSVKGRFTISADTSKNTAYLQMNSLRAEDTAVY YCSRWGGDGFYAMDYWGQGTLVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVD GVEVHNAKTKPREEQYNSTYRVVSVLTVLHQDWLNGKEYKCKVSN KALPAPIEKTISKAKGQPREPQVYTLPPCREEMTKNQVSLWCLVKGFY PSDIAVEWESNGQPENNYKTTPPVLDSDGSFFLYSKLTVDKSRWQQG NVFSCSVMHEALHNHYTQKSLSLSPGK | 8 |
| TraL-WT-Knob | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGQGTKVEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGECGGGGSGGGGSGGGGSEPKSSDKTH TCPPCPAPELLGGPSVFLFPPKPKDTLMISRTPEVTCVVVDVSHEDPEV KFNWYVDGVEVHNAKTKPREEQYNSTYRVVSVLTVLHQDWLNGKE YKCKVSNKALPAPIEKTISKAKGQPREPQVYTLPPCREEMTKNQVSL WCLVKGFYPSDIAVEWESNGQPENNYKTTPPVLDSDGSFFLYSKLTV DKSRWQQGNVFSCSVMHEALHNHYTQKSLSLSPGK | 9 |
| TraH-Q105C-Knob | EVQLVESGGGLVQPGGSLRLSCAASGFNIKDTYIHWVRQAPGKGLEW VARIYPTNGYTRYADSVKGRFTISADTSKNTAYLQMNSLRAEDTAVY YCSRWGGDGFYAMDYWGCGTLVTVSSASTKGPSVFPLAPSSKSTSGG TAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPE LLGGPSVFLFPPKPKDTLMISRTPEVTCVVVDVSHEDPEVKFNWYVD GVEVHNAKTKPREEQYNSTYRVVSVLTVLHQDWLNGKEYKCKVSN KALPAPIEKTISKAKGQPREPQVYTLPPCREEMTKNQVSLWCLVKGFY PSDIAVEWESNGQPENNYKTTPPVLDSDGSFFLYSKLTVDKSRWQQG NVFSCSVMHEALHNHYTQKSLSLSPGK | 10 |
| TraL-A43C-Knob | DIQMTQSPSSLSASVGDRVTITCRASQDVNTAVAWYQQKPGKCPKLLI YSASFLYSGVPSRFSGSRSGTDFTLTISSLQPEDFATYYCQQHYTTPPTF GQGTKVEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGECGGGGSGGGGSGGGGSEPKSSDKTHT CPPCPAPELLGGPSVFLFPPKPKDTLMISRTPEVTCVVVDVSHEDPEVK | 11 |
| | FNWYVDGVEVHNAKTKPREEQYNSTYRVVSVLTVLHQDWLNGKEY KCKVSNKALPAPIEKTISKAKGQPREPQVYTLPPCREEMTKNQVSLWC LVKGFYPSDIAVEWESNGQPENNYKTTPPVLDSDGSFFLYSKLTVDKS RWQQGNVFSCSVMHEALHNHYTQKSLSLSPGK | |
| TraH-F122C-Knob | EVQLVESGGGLVQPGGSLRLSCAASGFNIKDTYIHWVRQAPGKGLEW VARIYPTNGYTRYADSVKGRFTISADTSKNTAYLQMNSLRAEDTAVY YCSRWGGDGFYAMDYWGQGTLVTVSSASTKGPSVCPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVD GVEVHNAKTKPREEQYNSTYRVVSVLTVLHQDWLNGKEYKCKVSN KALPAPIEKTISKAKGQPREPQVYTLPPCREEMTKNQVSLWCLVKGFY PSDIAVEWESNGQPENNYKTTPPVLDSDGSFFLYSKLTVDKSRWQQG NVFSCSVMHEALHNHYTQKSLSLSPGK | 12 |
| TraL-S121C-Knob | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGQGTKVEIKRTVAAPSVFIFPPCDEQLKSGTASVVCLLNNFYPREAK VQWKVDNALQSGNSQESVTEQDSKDSTYSLSSTLTLSKADYEKHKV YACEVTHQGLSSPVTKSFNRGECGGGGSGGGGSGGGGSEPKSSDKTH TCPPCPAPELLGGPSVFLFPPKPKDTLMISRTPEVTCVVVDVSHEDPEV KFNWYVDGVEVHNAKTKPREEQYNSTYRVVSVLTVLHQDWLNGKE YKCKVSNKALPAPIEKTISKAKGQPREPQVYTLPPCREEMTKNQVSL WCLVKGFYPSDIAVEWESNGQPENNYKTTPPVLDSDGSFFLYSKLTV DKSRWQQGNVFSCSVMHEALHNRFTQKSLSLSPGK | 13 |
| TraH-G44C-Knob | EVQLVESGGGLVQPGGSLRLSCAASGFNIKDTYIHWVRQAPGKCLEW VARIYPTNGYTRYADSVKGRFTISADTSKNTAYLQMNSLRAEDTAVY YCSRWGGDGFYAMDYWGQGTLVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVD GVEVHNAKTKPREEQYNSTYRVVSVLTVLHQDWLNGKEYKCKVSN KALPAPIEKTISKAKGQPREPQVYTLPPCREEMTKNQVSLWCLVKGFY PSDIAVEWESNGQPENNYKTTPPVLDSDGSFFLYSKLTVDKSRWQQG NVFSCSVMHEALHNHYTQKSLSLSPGK | 14 |
| TraL-Q100C-Knob | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGCGTKVEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGECGGGGSGGGGSGGGGSEPKSSDKTHT CPPCPAPELLGGPSVFLFPPKPKDTLMISRTPEVTCVVVDVSHEDPEVK FNWYVDGVEVHNAKTKPREEQYNSTYRVVSVLTVLHQDWLNGKEY KCKVSNKALPAPIEKTISKAKGQPREPQVYTLPPCREEMTKNQVSLWC LVKGFYPSDIAVEWESNGQPENNYKTTPPVLDSDGSFFLYSKLTVDKS RWQQGNVFSCSVMHEALHNHYTQKSLSLSPGK | 15 |
| TraL-Q100C- | DIQMTQSPSSLSASVGDRVTITCRASQDVNTAVAWYQQKPGKAPKLL | 16 |
| Knob1 | IYSASFLYSGVPSRFSGSRSGTDFTLTISSLQPEDFATYYCQQHYTTPPT FGCGTKVEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGECGGGGSGGGGSEPKSSDKTHTCPPCPA PELLGGPSVFLFPPKPKDTLMISRTPEVTCVVVDVSHEDPEVKFNWYV DGVEVHNAKTKPREEQYNSTYRVVSVLTVLHQDWLNGKEYKCKVS NKALPAPIEKTISKAKGQPREPQVYTLPPCREEMTKNQVSLWCLVKGF YPSDIAVEWESNGQPENNYKTTPPVLDSDGSFFLYSKLTVDKSRWQQ GNVFSCSVMHEALHNHYTQKSLSLSPGK | |
| TraL-Q100C-Knob2 | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGCGTKVEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGECGGGGSEPKSSDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPCREEMTKNQVSLWCLVKGFYPSDI AVEWESNGQPENNYKTTPPVLDSDGSFFLYSKLTVDKSRWQQGNVFS CSVMHEALHNHYTQKSLSLSPGK | 17 |
| TraL-Q100C-Knob3 | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGCGTKVEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGECEPKSSDKTHTCPPCPAPELLGGPSVFL FPPKPKDTLMISRTPEVTCVVVDVSHEDPEVKFNWYVDGVEVHNAKT KPREEQYNSTYRVVSVLTVLHQDWLNGKEYKCKVSNKALPAPIEKTI SKAKGQPREPQVYTLPPCREEMTKNQVSLWCLVKGFYPSDIAVEWES NGQPENNYKTTPPVLDSDGSFFLYSKLTVDKSRWQQGNVFSCSVMHE ALHNHYTQKSLSLSPGK | 18 |

**[Table 8]**

| Name | Nucleotide sequence | SEQ ID NO |
|---|---|---|
| Trastuzumab VH-CH1 domain | GAAGTGCAGCTGGTCGAAAGTGGCGGTGGACTTGTGCAACCTGGCGG TAGCCTCCGTCTCAGCTGCGCTGCAAGTGGGTTCAACATCAAGGACA CTTATATTCATTGGGTCCGACAGGCACCTGGGAAAGGTTTGGAGTGG GTCGCACGGATCTATCCCACTAATGGTTACACAAGATATGCCGATTC AGTAAAAGGCCGGTTTACAATCAGCGCAGATACTTCAAAAAACACTG CCTATCTTCAAATGAACTCACTTCGAGCAGAAGACACAGCCGTCTAT TATTGTAGTCGTTGGGGAGGCGACGGCTTTTATGCTATGGACTACTGG GGACAAGGAACTCTGGTCACAGTTTCATCAGCTAGCACCAAAGGACC TAGTGTTTTTCCTCTTGCCCCTTCCTCAAAGTCTACCTCTGGGGGGAC AGCCGCTCTGGGCTGCCTGGTCAAGGATTATTTCCCAGAGCCTGTCAC TGTCAGTTGGAACTCTGGAGCCTTGACTTCTGGTGTTCATACATTTCC TGCTGTCCTTCAAAGCAGCGGCTTGTACTCATTGTCTTCTGTTGTGAC AGTACCCTCAAGCAGCCTCGGCACTCAGACATACATCTGCAATGTCA | 452 |
| | ACCACAAACCCTCAAATACAAAGGTAGATAAAAAAGTC | |
| Trastuzumab VL-CL domain | GATATTCAGATGACTCAGAGTCCTAGTTCCCTCAGCGCCTCCGTAGGC GACAGAGTTACAATAACTTGCCGAGCAAGCCAAGACGTAAACACTGC AGTCGCCTGGTACCAACAGAAACCAGGCAAAGCTCCAAAACTCTTGA TTTACAGTGCTTCCTTCCTTTATAGTGGCGTTCCAAGCCGCTTCAGCG GCAGCCGCTCTGGCACCGACTTCACTCTCACTATTTCTTCCTTGCAAC CTGAAGACTTCGCCACTTATTATTGCCAGCAACACTACACAACACCC CCAACATTCGGACAGGGCACAAAGGTAGAAATAAAACGTACGGTGG CAGCTCCCAGCGTTTTTATCTTTCCCCCATCCGACGAGCAGCTCAAGA GTGGCACTGCCTCTGTAGTTTGTTTGCTGAATAACTTCTATCCACGTG AAGCAAAAGTACAGTGGAAGGTCGATAATGCCCTTCAGAGCGGTAA CAGCCAAGAAAGTGTTACCGAGCAAGATTCCAAAGATTCCACTTACA GTCTGTCCAGCACATTGACACTGAGTAAGGCTGATTACGAAAAACAC AAGGTGTACGCATGCGAGGTGACACACCAAGGTCTTTCATCTCCTGT AACTAAGAGCTTTAACCGGGGAGAATGT | 453 |
| IgG1 Fc(CH2-CH3) | GCACCTGAACTCCTGGGGGGACCGTCAGTCTTCCTCTTCCCCCCAAAA CCCAAGGACACCCTCATGATCTCCCGGACCCCTGAGGTCACATGCGT GGTGGTGGACGTGAGCCACGAAGACCCTGAGGTCAAGTTCAACTGGT ACGTGGACGGCGTGGAGGTGCATAATGCCAAGACAAAGCCGCGGGA GGAGCAGTACAACAGCACGTACCGTGTGGTCAGCGTCCTCACCGTCC TGCACCAGGACTGGCTGAATGGCAAGGAGTACAAGTGCAAGGTCTCC AACAAAGCCCTCCCAGCCCCCATCGAGAAAACCATCTCCAAAGCCAA AGGGCAGCCCCGAGAACCACAGGTGTACACCCTGCCCCCAAGCCGG GAGGAGATGACCAAGAACCAGGTCAGCCTGACGTGCCTGGTCAAAG GCTTCTATCCCAGCGACATCGCCGTGGAGTGGGAGAGCAATGGGCAG CCGGAGAACAACTACAAGACCACGCCTCCCGTGCTGGACTCCGACGG CTCCTTCTTCCTCTACAGCAAGCTCACCGTGGACAAGAGCAGGTGGC AGCAGGGGAACGTCTTCTCATGCTCCGTGATGCATGAGGCTCTGCAC AACCACTACACGCAGAAGAGCCTCTCCCTGTCTCCGGGTAAA | 454 |
| IgG1 Fc(Knob; S354C, T366W) | GCACCTGAACTCCTGGGGGGACCGTCAGTCTTCCTCTTCCCCCCAAAA CCCAAGGACACCCTCATGATCTCCCGGACCCCTGAGGTCACATGCGT GGTGGTGGACGTGAGCCACGAAGACCCTGAGGTCAAGTTCAACTGGT ACGTGGACGGCGTGGAGGTGCATAATGCCAAGACAAAGCCGCGGGA GGAGCAGTACAACAGCACGTACCGTGTGGTCAGCGTCCTCACCGTCC TGCACCAGGACTGGCTGAATGGCAAGGAGTACAAGTGCAAGGTCTCC AACAAAGCCCTCCCAGCCCCCATCGAGAAAACCATCTCCAAAGCCAA AGGGCAGCCCCGAGAACCACAGGTGTACACCCTGCCCCCATGCCGGG AGGAGATGACCAAGAACCAGGTCAGCCTGTGGTGCCTGGTCAAAGG CTTCTATCCCAGCGACATCGCCGTGGAGTGGGAGAGCAATGGGCAGC CGGAGAACAACTACAAGACCACGCCTCCCGTGCTGGACTCCGACGGC TCCTTCTTCCTCTACAGCAAGCTCACCGTGGACAAGAGCAGGTGGCA GCAGGGGAACGTCTTCTCATGCTCCGTGATGCATGAGGCTCTGCACA ACCACTACACGCAGAAGAGCCTCTCCCTGTCTCCGGGTAAA | 455 |
| IgG1 Fc(Hole; Y349C, T366S, | GCACCTGAACTCCTGGGGGGACCGTCAGTCTTCCTCTTCCCCCCAAAA CCCAAGGACACCCTCATGATCTCCCGGACCCCTGAGGTCACATGCGT | 456 |
| L368A, Y407V) | GGTGGTGGACGTGAGCCACGAAGACCCTGAGGTCAAGTTCAACTGGT ACGTGGACGGCGTGGAGGTGCATAATGCCAAGACAAAGCCGCGGGA GGAGCAGTACAACAGCACGTACCGTGTGGTCAGCGTCCTCACCGTCC TGCACCAGGACTGGCTGAATGGCAAGGAGTACAAGTGCAAGGTCTCC AACAAAGCCCTCCCAGCCCCCATCGAGAAAACCATCTCCAAAGCCAA AGGGCAGCCCCGAGAACCACAGGTGTGCACCCTGCCCCCATCCCGGG AGGAGATGACCAAGAACCAGGTCAGCCTGAGCTGCGCGGTCAAAGG CTTCTATCCCAGCGACATCGCCGTGGAGTGGGAGAGCAATGGGCAGC CGGAGAACAACTACAAGACCACGCCTCCCGTGCTGGACTCCGACGGC TCCTTCTTCCTCGTGAGCAAGCTCACCGTGGACAAGAGCAGGTGGCA GCAGGGGAACGTCTTCTCATGCTCCGTGATGCATGAGGCTCTGCACA ACCACTACACGCAGAAGAGCCTCTCCCTGTCTCCGGGTAAA | |
| 15-mer Linker | GGTGGTGGGGGCAGCGGGGGCGGAGGTAGTGGAGGCGGCGGTAGT | 457 |
| Fc-Hole | GACAAAACTCACACATGCCCACCGTGCCCAGCACCTGAACTCCTGGGG GGACCGTCAGTCTTCCTCTTCCCCCCAAAACCCAAGGACACCCTCAT GATCTCCCGGACCCCTGAGGTCACATGCGTGGTGGTGGACGTGAGCC ACGAAGACCCTGAGGTCAAGTTCAACTGGTACGTGGACGGCGTGGAG GTGCATAATGCCAAGACAAAGCCGCGGGAGGAGCAGTACAACAGCA CGTACCGTGTGGTCAGCGTCCTCACCGTCCTGCACCAGGACTGGCTG AATGGCAAGGAGTACAAGTGCAAGGTCTCCAACAAAGCCCTCCCAGC CCCCATCGAGAAAACCATCTCCAAAGCCAAAGGGCAGCCCCGAGAA CCACAGGTGTGCACCCTGCCCCCATCCCGGGAGGAGATGACCAAGAA CCAGGTCAGCCTGAGCTGCGCGGTCAAAGGCTTCTATCCCAGCGACA TCGCCGTGGAGTGGGAGAGCAATGGGCAGCCGGAGAACAACTACAA GACCACGCCTCCCGTGCTGGACTCCGACGGCTCCTTCTTCCTCGTGAG CAAGCTCACCGTGGACAAGAGCAGGTGGCAGCAGGGGAACGTCTTCT CATGCTCCGTGATGCATGAGGCTCTGCACAACCACTACACGCAGAAG AGCCTCTCCCTGTCTCCGGGTAAA | 458 |
| TraH-WT-Knob | GAAGTTCAACTTGTGGAGTCTGGAGGCGGTTTGGTACAACCTGGAGG TTCACTTCGTTTGTCATGTGCTGCCTCTGGGTTTAATATCAAGGATAC TTATATTCATTGGGTTCGCCAGGCACCCGGAAAAGGGCTGGAATGGG TGGCCCGTATATATCCCACTAACGGTTACACTCGATATGCTGACTCTG TAAAGGGTCGATTTACTATATCCGCTGATACTAGCAAAAACACTGCC TACCTCCAGATGAACAGTCTGCGCGCCGAGGATACAGCAGTTTATTA TTGTTCCCGATGGGGAGGTGACGGGTTCTATGCCATGGACTACTGGG GTCAAGGGACACTGGTAACCGTTTCTTCTGCTAGCACCAAAGGACCT AGTGTTTTTCCTCTTGCCCCTTCCTCAAAGTCTACCTCTGGGGGGACA GCCGCTCTGGGCTGCCTGGTCAAGGATTATTTCCCAGAGCCTGTCACT GTCAGTTGGAACTCTGGAGCCTTGACTTCTGGTGTTCATACATTTCCT GCTGTCCTTCAAAGCAGCGGCTTGTACTCATTGTCTTCTGTTGTGACA GTACCCTCAAGCAGCCTCGGCACTCAGACATACATCTGCAATGTCAA CCACAAACCCTCAAATACAAAGGTAGATAAAAAAGTCGAACCAAAG TCTTGTGACAAAACTCACACGTGCCCACCGTGCCCAGCACCTGAACT CCTGGGGGGACCGTCAGTCTTCCTCTTCCCCCCAAAACCCAAGGACA CCCTCATGATCTCCCGGACCCCTGAGGTCACATGCGTGGTGGTGGAC | 459 |
| | GTGAGCCACGAAGACCCTGAGGTCAAGTTCAACTGGTACGTGGACGG CGTGGAGGTGCATAATGCCAAGACAAAGCCGCGGGAGGAGCAGTAC AACAGCACGTACCGTGTGGTCAGCGTCCTCACCGTCCTGCACCAGGA CTGGCTGAATGGCAAGGAGTACAAGTGCAAGGTCTCCAACAAAGCCC TCCCAGCCCCCATCGAGAAAACCATCTCCAAAGCCAAAGGGCAGCCC CGAGAACCACAGGTGTACACCCTGCCCCCATGCCGGGAGGAGATGAC CAAGAACCAGGTCAGCCTGTGGTGCCTGGTCAAAGGCTTCTATCCCA GCGACATCGCCGTGGAGTGGGAGAGCAATGGGCAGCCGGAGAACAA CTACAAGACCACGCCTCCCGTGCTGGACTCCGACGGCTCCTTCTTCCT CTACAGCAAGCTCACCGTGGACAAGAGCAGGTGGCAGCAGGGGAAC GTCTTCTCATGCTCCGTGATGCATGAGGCTCTGCACAACCACTACACG CAGAAGAGCCTCTCCCTGTCTCCGGGTAAA | |
| TraL-WT-Knob | GACATTCAGATGACCCAATCCCCCTCTAGCCTTTCAGCCTCCGTAGGG GACAGAGTTACAATAACCTGTAGGGCTAGTCAGGACGTTAATACAGC AGTCGCATGGTACCAGCAGAAGCCCGGTAAGGCTCCTAAGCTTTTGA TCTACAGTGCTTCTTTCCTGTATTCTGGTGTACCTAGCCGATTCTCAG GCTCTCGGAGTGGCACAGACTTCACTCTGACAATTTCAAGTCTCCAGC CAGAAGATTTTGCAACCTACTACTGCCAACAACACTACACTACTCCTC CAACCTTCGGACAGGGCACAAAAGTAGAGATTAAGCGTACGGTGGC AGCTCCCAGCGTTTTTATCTTTCCCCCATCCGACGAGCAGCTCAAGAG TGGCACTGCCTCTGTAGTTTGTTTGCTGAATAACTTCTATCCACGTGA AGCAAAAGTACAGTGGAAGGTCGATAATGCCCTTCAGAGCGGTAAC AGCCAAGAAAGTGTTACCGAGCAAGATTCCAAAGATTCCACTTACAG TCTGTCCAGCACATTGACACTGAGTAAGGCTGATTACGAAAAACACA AGGTGTACGCATGCGAGGTGACACACCAAGGTCTTTCATCTCCTGTA ACTAAGAGCTTTAACCGGGGAGAATGTGGTGGTGGGGGCAGCGGGG GCGGAGGTAGTGGAGGCGGCGGTAGTGAACCAAAGAGTAGTGACAA AACTCACACGTGCCCACCGTGCCCAGCACCTGAACTCCTGGGGGGAC CGTCAGTCTTCCTCTTCCCCCCAAAACCCAAGGACACCCTCATGATCT CCCGGACCCCTGAGGTCACATGCGTGGTGGTGGACGTGAGCCACGAA GACCCTGAGGTCAAGTTCAACTGGTACGTGGACGGCGTGGAGGTGCA TAATGCCAAGACAAAGCCGCGGGAGGAGCAGTACAACAGCACGTAC CGTGTGGTCAGCGTCCTCACCGTCCTGCACCAGGACTGGCTGAATGG CAAGGAGTACAAGTGCAAGGTCTCCAACAAAGCCCTCCCAGCCCCCA TCGAGAAAACCATCTCCAAAGCCAAAGGGCAGCCCCGAGAACCACA GGTGTACACCCTGCCCCCATGCCGGGAGGAGATGACCAAGAACCAG GTCAGCCTGTGGTGCCTGGTCAAAGGCTTCTATCCCAGCGACATCGC CGTGGAGTGGGAGAGCAATGGGCAGCCGGAGAACAACTACAAGACC ACGCCTCCCGTGCTGGACTCCGACGGCTCCTTCTTCCTCTACAGCAAG CTCACCGTGGACAAGAGCAGGTGGCAGCAGGGGAACGTCTTCTCATG CTCCGTGATGCATGAGGCTCTGCACAACCACTACACGCAGAAGAGCC TCTCCCTGTCTCCGGGTAAA | 460 |
| TraH-Q105C-Knob | GAAGTTCAACTTGTGGAGTCTGGAGGCGGTTTGGTACAACCTGGAGG TTCACTTCGTTTGTCATGTGCTGCCTCTGGGTTTAATATCAAGGATAC TTATATTCATTGGGTTCGCCAGGCACCCGGAAAAGGGCTGGAATGGG | 461 |
| | TGGCCCGTATATATCCCACTAACGGTTACACTCGATATGCTGACTCTG TAAAGGGTCGATTTACTATATCCGCTGATACTAGCAAAAACACTGCC TACCTCCAGATGAACAGTCTGCGCGCCGAGGATACAGCAGTTTATTA TTGTTCCCGATGGGGAGGTGACGGGTTCTATGCCATGGACTACTGGG GTTGTGGGACACTGGTAACCGTTTCTTCTGCTAGCACCAAAGGACCT AGTGTTTTTCCTCTTGCCCCTTCCTCAAAGTCTACCTCTGGGGGGACA GCCGCTCTGGGCTGCCTGGTCAAGGATTATTTCCCAGAGCCTGTCACT GTCAGTTGGAACTCTGGAGCCTTGACTTCTGGTGTTCATACATTTCCT GCTGTCCTTCAAAGCAGCGGCTTGTACTCATTGTCTTCTGTTGTGACA GTACCCTCAAGCAGCCTCGGCACTCAGACATACATCTGCAATGTCAA CCACAAACCCTCAAATACAAAGGTAGATAAAAAAGTCGAACCAAAG TCTTGTGACAAAACTCACACGTGCCCACCGTGCCCAGCACCTGAACT CCTGGGGGGACCGTCAGTCTTCCTCTTCCCCCCAAAACCCAAGGACA CCCTCATGATCTCCCGGACCCCTGAGGTCACATGCGTGGTGGTGGAC GTGAGCCACGAAGACCCTGAGGTCAAGTTCAACTGGTACGTGGACGG CGTGGAGGTGCATAATGCCAAGACAAAGCCGCGGGAGGAGCAGTAC AACAGCACGTACCGTGTGGTCAGCGTCCTCACCGTCCTGCACCAGGA CTGGCTGAATGGCAAGGAGTACAAGTGCAAGGTCTCCAACAAAGCCC TCCCAGCCCCCATCGAGAAAACCATCTCCAAAGCCAAAGGGCAGCCC CGAGAACCACAGGTGTACACCCTGCCCCCATGCCGGGAGGAGATGAC CAAGAACCAGGTCAGCCTGTGGTGCCTGGTCAAAGGCTTCTATCCCA GCGACATCGCCGTGGAGTGGGAGAGCAATGGGCAGCCGGAGAACAA CTACAAGACCACGCCTCCCGTGCTGGACTCCGACGGCTCCTTCTTCCT CTACAGCAAGCTCACCGTGGACAAGAGCAGGTGGCAGCAGGGGAAC GTCTTCTCATGCTCCGTGATGCATGAGGCTCTGCACAACCACTACACG CAGAAGAGCCTCTCCCTGTCTCCGGGTAAA | |
| TraL-A43C-Knob | GACATTCAGATGACCCAATCCCCCTCTAGCCTTTCAGCCTCCGTAGGG GACAGAGTTACAATAACCTGTAGGGCTAGTCAGGACGTTAATACAGC AGTCGCATGGTACCAGCAGAAGCCCGGTAAGTGTCCTAAGCTTTTGA TCTACAGTGCTTCTTTCCTGTATTCTGGTGTACCTAGCCGATTCTCAG GCTCTCGGAGTGGCACAGACTTCACTCTGACAATTTCAAGTCTCCAGC CAGAAGATTTTGCAACCTACTACTGCCAACAACACTACACTACTCCTC CAACCTTCGGACAGGGCACAAAAGTAGAGATTAAGCGTACGGTGGC AGCTCCCAGCGTTTTTATCTTTCCCCCATCCGACGAGCAGCTCAAGAG TGGCACTGCCTCTGTAGTTTGTTTGCTGAATAACTTCTATCCACGTGA AGCAAAAGTACAGTGGAAGGTCGATAATGCCCTTCAGAGCGGTAAC AGCCAAGAAAGTGTTACCGAGCAAGATTCCAAAGATTCCACTTACAG TCTGTCCAGCACATTGACACTGAGTAAGGCTGATTACGAAAAACACA AGGTGTACGCATGCGAGGTGACACACCAAGGTCTTTCATCTCCTGTA ACTAAGAGCTTTAACCGGGGAGAATGTGGTGGTGGGGGCAGCGGGG GCGGAGGTAGTGGAGGCGGCGGTAGTGAACCAAAGAGTAGTGACAA AACTCACACGTGCCCACCGTGCCCAGCACCTGAACTCCTGGGGGGAC CGTCAGTCTTCCTCTTCCCCCCAAAACCCAAGGACACCCTCATGATCT CCCGGACCCCTGAGGTCACATGCGTGGTGGTGGACGTGAGCCACGAA GACCCTGAGGTCAAGTTCAACTGGTACGTGGACGGCGTGGAGGTGCA | 462 |
| | TAATGCCAAGACAAAGCCGCGGGAGGAGCAGTACAACAGCACGTAC CGTGTGGTCAGCGTCCTCACCGTCCTGCACCAGGACTGGCTGAATGG CAAGGAGTACAAGTGCAAGGTCTCCAACAAAGCCCTCCCAGCCCCCA TCGAGAAAACCATCTCCAAAGCCAAAGGGCAGCCCCGAGAACCACA GGTGTACACCCTGCCCCCATGCCGGGAGGAGATGACCAAGAACCAG GTCAGCCTGTGGTGCCTGGTCAAAGGCTTCTATCCCAGCGACATCGC CGTGGAGTGGGAGAGCAATGGGCAGCCGGAGAACAACTACAAGACC ACGCCTCCCGTGCTGGACTCCGACGGCTCCTTCTTCCTCTACAGCAAG CTCACCGTGGACAAGAGCAGGTGGCAGCAGGGGAACGTCTTCTCATG CTCCGTGATGCATGAGGCTCTGCACAACCACTACACGCAGAAGAGCC TCTCCCTGTCTCCGGGTAAA | |
| TraH-F122C-Knob | GAAGTTCAACTTGTGGAGTCTGGAGGCGGTTTGGTACAACCTGGAGG TTCACTTCGTTTGTCATGTGCTGCCTCTGGGTTTAATATCAAGGATAC TTATATTCATTGGGTTCGCCAGGCACCCGGAAAAGGGCTGGAATGGG TGGCCCGTATATATCCCACTAACGGTTACACTCGATATGCTGACTCTG TAAAGGGTCGATTTACTATATCCGCTGATACTAGCAAAAACACTGCC TACCTCCAGATGAACAGTCTGCGCGCCGAGGATACAGCAGTTTATTA TTGTTCCCGATGGGGAGGTGACGGGTTCTATGCCATGGACTACTGGG GTCAAGGGACACTGGTAACCGTTTCTTCTGCTAGCACCAAAGGACCT AGTGTTTGTCCTCTTGCCCCTTCCTCAAAGTCTACCTCTGGGGGGACA GCCGCTCTGGGCTGCCTGGTCAAGGATTATTTCCCAGAGCCTGTCACT GTCAGTTGGAACTCTGGAGCCTTGACTTCTGGTGTTCATACATTTCCT GCTGTCCTTCAAAGCAGCGGCTTGTACTCATTGTCTTCTGTTGTGACA GTACCCTCAAGCAGCCTCGGCACTCAGACATACATCTGCAATGTCAA CCACAAACCCTCAAATACAAAGGTAGATAAAAAAGTCGAACCAAAG TCTTGTGACAAAACTCACACGTGCCCACCGTGCCCAGCACCTGAACT CCTGGGGGGACCGTCAGTCTTCCTCTTCCCCCCAAAACCCAAGGACA CCCTCATGATCTCCCGGACCCCTGAGGTCACATGCGTGGTGGTGGAC GTGAGCCACGAAGACCCTGAGGTCAAGTTCAACTGGTACGTGGACGG CGTGGAGGTGCATAATGCCAAGACAAAGCCGCGGGAGGAGCAGTAC AACAGCACGTACCGTGTGGTCAGCGTCCTCACCGTCCTGCACCAGGA CTGGCTGAATGGCAAGGAGTACAAGTGCAAGGTCTCCAACAAAGCCC TCCCAGCCCCCATCGAGAAAACCATCTCCAAAGCCAAAGGGCAGCCC CGAGAACCACAGGTGTACACCCTGCCCCCATGCCGGGAGGAGATGAC CAAGAACCAGGTCAGCCTGTGGTGCCTGGTCAAAGGCTTCTATCCCA GCGACATCGCCGTGGAGTGGGAGAGCAATGGGCAGCCGGAGAACAA CTACAAGACCACGCCTCCCGTGCTGGACTCCGACGGCTCCTTCTTCCT CTACAGCAAGCTCACCGTGGACAAGAGCAGGTGGCAGCAGGGGAAC GTCTTCTCATGCTCCGTGATGCATGAGGCTCTGCACAACCACTACACG CAGAAGAGCCTCTCCCTGTCTCCGGGTAAA | 463 |
| TraL-S121C-Knob | GACATTCAGATGACCCAATCCCCCTCTAGCCTTTCAGCCTCCGTAGGG GACAGAGTTACAATAACCTGTAGGGCTAGTCAGGACGTTAATACAGC AGTCGCATGGTACCAGCAGAAGCCCGGTAAGGCTCCTAAGCTTTTGA TCTACAGTGCTTCTTTCCTGTATTCTGGTGTACCTAGCCGATTCTCAG GCTCTCGGAGTGGCACAGACTTCACTCTGACAATTTCAAGTCTCCAGC | 464 |
| | CAGAAGATTTTGCAACCTACTACTGCCAACAACACTACACTACTCCTC CAACCTTCGGACAGGGCACAAAAGTAGAGATTAAGCGTACGGTGGC AGCTCCCAGCGTTTTTATCTTTCCCCCATGTGACGAGCAGCTCAAGAG TGGCACTGCCTCTGTAGTTTGTTTGCTGAATAACTTCTATCCACGTGA AGCAAAAGTACAGTGGAAGGTCGATAATGCCCTTCAGAGCGGTAAC AGCCAAGAAAGTGTTACCGAGCAAGATTCCAAAGATTCCACTTACAG TCTGTCCAGCACATTGACACTGAGTAAGGCTGATTACGAAAAACACA AGGTGTACGCATGCGAGGTGACACACCAAGGTCTTTCATCTCCTGTA ACTAAGAGCTTTAACCGGGGAGAATGTGGTGGTGGGGGCAGCGGGG GCGGAGGTAGTGGAGGCGGCGGTAGTGAACCAAAGAGTAGTGACAA AACTCACACGTGCCCACCGTGCCCAGCACCTGAACTCCTGGGGGGAC CGTCAGTCTTCCTCTTCCCCCCAAAACCCAAGGACACCCTCATGATCT CCCGGACCCCTGAGGTCACATGCGTGGTGGTGGACGTGAGCCACGAA GACCCTGAGGTCAAGTTCAACTGGTACGTGGACGGCGTGGAGGTGCA TAATGCCAAGACAAAGCCGCGGGAGGAGCAGTACAACAGCACGTAC CGTGTGGTCAGCGTCCTCACCGTCCTGCACCAGGACTGGCTGAATGG CAAGGAGTACAAGTGCAAGGTCTCCAACAAAGCCCTCCCAGCCCCCA TCGAGAAAACCATCTCCAAAGCCAAAGGGCAGCCCCGAGAACCACA GGTGTACACCCTGCCCCCATGCCGGGAGGAGATGACCAAGAACCAG GTCAGCCTGTGGTGCCTGGTCAAAGGCTTCTATCCCAGCGACATCGC CGTGGAGTGGGAGAGCAATGGGCAGCCGGAGAACAACTACAAGACC ACGCCTCCCGTGCTGGACTCCGACGGCTCCTTCTTCCTCTACAGCAAG CTCACCGTGGACAAGAGCAGGTGGCAGCAGGGGAACGTCTTCTCATG CTCCGTGATGCATGAGGCTCTGCACAACAGATTTACGCAGAAGAGCC TCTCCCTGTCTCCGGGTAAA | |
| TraH-G44C-Knob | GAAGTTCAACTTGTGGAGTCTGGAGGCGGTTTGGTACAACCTGGAGG TTCACTTCGTTTGTCATGTGCTGCCTCTGGGTTTAATATCAAGGATAC TTATATTCATTGGGTTCGCCAGGCACCCGGAAAATGCCTGGAATGGG TGGCCCGTATATATCCCACTAACGGTTACACTCGATATGCTGACTCTG TAAAGGGTCGATTTACTATATCCGCTGATACTAGCAAAAACACTGCC TACCTCCAGATGAACAGTCTGCGCGCCGAGGATACAGCAGTTTATTA TTGTTCCCGATGGGGAGGTGACGGGTTCTATGCCATGGACTACTGGG GTCAAGGGACACTGGTAACCGTTTCTTCTGCTAGCACCAAAGGACCT AGTGTTTTTCCTCTTGCCCCTTCCTCAAAGTCTACCTCTGGGGGGACA GCCGCTCTGGGCTGCCTGGTCAAGGATTATTTCCCAGAGCCTGTCACT GTCAGTTGGAACTCTGGAGCCTTGACTTCTGGTGTTCATACATTTCCT GCTGTCCTTCAAAGCAGCGGCTTGTACTCATTGTCTTCTGTTGTGACA GTACCCTCAAGCAGCCTCGGCACTCAGACATACATCTGCAATGTCAA CCACAAACCCTCAAATACAAAGGTAGATAAAAAAGTCGAACCAAAG TCTTGTGACAAAACTCACACGTGCCCACCGTGCCCAGCACCTGAACT CCTGGGGGGACCGTCAGTCTTCCTCTTCCCCCCAAAACCCAAGGACA CCCTCATGATCTCCCGGACCCCTGAGGTCACATGCGTGGTGGTGGAC GTGAGCCACGAAGACCCTGAGGTCAAGTTCAACTGGTACGTGGACGG CGTGGAGGTGCATAATGCCAAGACAAAGCCGCGGGAGGAGCAGTAC AACAGCACGTACCGTGTGGTCAGCGTCCTCACCGTCCTGCACCAGGA | 465 |
| | CTGGCTGAATGGCAAGGAGTACAAGTGCAAGGTCTCCAACAAAGCCC TCCCAGCCCCCATCGAGAAAACCATCTCCAAAGCCAAAGGGCAGCCC CGAGAACCACAGGTGTACACCCTGCCCCCATGCCGGGAGGAGATGAC CAAGAACCAGGTCAGCCTGTGGTGCCTGGTCAAAGGCTTCTATCCCA GCGACATCGCCGTGGAGTGGGAGAGCAATGGGCAGCCGGAGAACAA CTACAAGACCACGCCTCCCGTGCTGGACTCCGACGGCTCCTTCTTCCT CTACAGCAAGCTCACCGTGGACAAGAGCAGGTGGCAGCAGGGGAAC GTCTTCTCATGCTCCGTGATGCATGAGGCTCTGCACAACCACTACACG CAGAAGAGCCTCTCCCTGTCTCCGGGTAAA | |
| TraL-Q100C-Knob | GACATTCAGATGACCCAATCCCCCTCTAGCCTTTCAGCCTCCGTAGGG GACAGAGTTACAATAACCTGTAGGGCTAGTCAGGACGTTAATACAGC AGTCGCATGGTACCAGCAGAAGCCCGGTAAGGCTCCTAAGCTTTTGA TCTACAGTGCTTCTTTCCTGTATTCTGGTGTACCTAGCCGATTCTCAG GCTCTCGGAGTGGCACAGACTTCACTCTGACAATTTCAAGTCTCCAGC CAGAAGATTTTGCAACCTACTACTGCCAACAACACTACACTACTCCTC CAACCTTCGGATGTGGCACAAAAGTAGAGATTAAGCGTACGGTGGCA GCTCCCAGCGTTTTTATCTTTCCCCCATCCGACGAGCAGCTCAAGAGT GGCACTGCCTCTGTAGTTTGTTTGCTGAATAACTTCTATCCACGTGAA GCAAAAGTACAGTGGAAGGTCGATAATGCCCTTCAGAGCGGTAACA GCCAAGAAAGTGTTACCGAGCAAGATTCCAAAGATTCCACTTACAGT CTGTCCAGCACATTGACACTGAGTAAGGCTGATTACGAAAAACACAA GGTGTACGCATGCGAGGTGACACACCAAGGTCTTTCATCTCCTGTAA CTAAGAGCTTTAACCGGGGAGAATGTGGTGGTGGGGGCAGCGGGGG CGGAGGTAGTGGAGGCGGCGGTAGTGAACCAAAGAGTAGTGACAAA ACTCACACGTGCCCACCGTGCCCAGCACCTGAACTCCTGGGGGGACC GTCAGTCTTCCTCTTCCCCCCAAAACCCAAGGACACCCTCATGATCTC CCGGACCCCTGAGGTCACATGCGTGGTGGTGGACGTGAGCCACGAAG ACCCTGAGGTCAAGTTCAACTGGTACGTGGACGGCGTGGAGGTGCAT AATGCCAAGACAAAGCCGCGGGAGGAGCAGTACAACAGCACGTACC GTGTGGTCAGCGTCCTCACCGTCCTGCACCAGGACTGGCTGAATGGC AAGGAGTACAAGTGCAAGGTCTCCAACAAAGCCCTCCCAGCCCCCAT CGAGAAAACCATCTCCAAAGCCAAAGGGCAGCCCCGAGAACCACAG GTGTACACCCTGCCCCCATGCCGGGAGGAGATGACCAAGAACCAGGT CAGCCTGTGGTGCCTGGTCAAAGGCTTCTATCCCAGCGACATCGCCG TGGAGTGGGAGAGCAATGGGCAGCCGGAGAACAACTACAAGACCAC GCCTCCCGTGCTGGACTCCGACGGCTCCTTCTTCCTCTACAGCAAGCT CACCGTGGACAAGAGCAGGTGGCAGCAGGGGAACGTCTTCTCATGCT CCGTGATGCATGAGGCTCTGCACAACCACTACACGCAGAAGAGCCTC TCCCTGTCTCCGGGTAAA | 466 |
| TraL-Q100C-Knob1 | GACATTCAGATGACCCAATCCCCCTCTAGCCTTTCAGCCTCCGTAGGG GACAGAGTTACAATAACCTGTAGGGCTAGTCAGGACGTTAATACAGC AGTCGCATGGTACCAGCAGAAGCCCGGTAAGGCTCCTAAGCTTTTGA TCTACAGTGCTTCTTTCCTGTATTCTGGTGTACCTAGCCGATTCTCAG GCTCTCGGAGTGGCACAGACTTCACTCTGACAATTTCAAGTCTCCAGC CAGAAGATTTTGCAACCTACTACTGCCAACAACACTACACTACTCCTC | 467 |
| | CAACCTTCGGATGTGGCACAAAAGTAGAGATTAAGCGTACGGTGGCA GCTCCCAGCGTTTTTATCTTTCCCCCATCCGACGAGCAGCTCAAGAGT GGCACTGCCTCTGTAGTTTGTTTGCTGAATAACTTCTATCCACGTGAA GCAAAAGTACAGTGGAAGGTCGATAATGCCCTTCAGAGCGGTAACA GCCAAGAAAGTGTTACCGAGCAAGATTCCAAAGATTCCACTTACAGT CTGTCCAGCACATTGACACTGAGTAAGGCTGATTACGAAAAACACAA GGTGTACGCATGCGAGGTGACACACCAAGGTCTTTCATCTCCTGTAA CTAAGAGCTTTAACCGGGGAGAATGTGGTGGTGGGGGCAGCGGGGG CGGAGGTAGTGAACCAAAGAGTAGTGACAAAACTCACACGTGCCCA CCGTGCCCAGCACCTGAACTCCTGGGGGGACCGTCAGTCTTCCTCTTC CCCCCAAAACCCAAGGACACCCTCATGATCTCCCGGACCCCTGAGGT CACATGCGTGGTGGTGGACGTGAGCCACGAAGACCCTGAGGTCAAGT TCAACTGGTACGTGGACGGCGTGGAGGTGCATAATGCCAAGACAAA GCCGCGGGAGGAGCAGTACAACAGCACGTACCGTGTGGTCAGCGTCC TCACCGTCCTGCACCAGGACTGGCTGAATGGCAAGGAGTACAAGTGC AAGGTCTCCAACAAAGCCCTCCCAGCCCCCATCGAGAAAACCATCTC CAAAGCCAAAGGGCAGCCCCGAGAACCACAGGTGTACACCCTGCCC CCATGCCGGGAGGAGATGACCAAGAACCAGGTCAGCCTGTGGTGCCT GGTCAAAGGCTTCTATCCCAGCGACATCGCCGTGGAGTGGGAGAGCA ATGGGCAGCCGGAGAACAACTACAAGACCACGCCTCCCGTGCTGGAC TCCGACGGCTCCTTCTTCCTCTACAGCAAGCTCACCGTGGACAAGAG CAGGTGGCAGCAGGGGAACGTCTTCTCATGCTCCGTGATGCATGAGG CTCTGCACAACCACTACACGCAGAAGAGCCTCTCCCTGTCTCCGGGT AAA | |
| TraL-Q100C-Knob2 | GACATTCAGATGACCCAATCCCCCTCTAGCCTTTCAGCCTCCGTAGGG GACAGAGTTACAATAACCTGTAGGGCTAGTCAGGACGTTAATACAGC AGTCGCATGGTACCAGCAGAAGCCCGGTAAGGCTCCTAAGCTTTTGA TCTACAGTGCTTCTTTCCTGTATTCTGGTGTACCTAGCCGATTCTCAG GCTCTCGGAGTGGCACAGACTTCACTCTGACAATTTCAAGTCTCCAGC CAGAAGATTTTGCAACCTACTACTGCCAACAACACTACACTACTCCTC CAACCTTCGGATGTGGCACAAAAGTAGAGATTAAGCGTACGGTGGCA GCTCCCAGCGTTTTTATCTTTCCCCCATCCGACGAGCAGCTCAAGAGT GGCACTGCCTCTGTAGTTTGTTTGCTGAATAACTTCTATCCACGTGAA GCAAAAGTACAGTGGAAGGTCGATAATGCCCTTCAGAGCGGTAACA GCCAAGAAAGTGTTACCGAGCAAGATTCCAAAGATTCCACTTACAGT CTGTCCAGCACATTGACACTGAGTAAGGCTGATTACGAAAAACACAA GGTGTACGCATGCGAGGTGACACACCAAGGTCTTTCATCTCCTGTAA CTAAGAGCTTTAACCGGGGAGAATGTGGTGGTGGGGGCAGCGAACC AAAGAGTAGTGACAAAACTCACACGTGCCCACCGTGCCCAGCACCTG AACTCCTGGGGGGACCGTCAGTCTTCCTCTTCCCCCCAAAACCCAAG GACACCCTCATGATCTCCCGGACCCCTGAGGTCACATGCGTGGTGGT GGACGTGAGCCACGAAGACCCTGAGGTCAAGTTCAACTGGTACGTGG ACGGCGTGGAGGTGCATAATGCCAAGACAAAGCCGCGGGAGGAGCA GTACAACAGCACGTACCGTGTGGTCAGCGTCCTCACCGTCCTGCACC AGGACTGGCTGAATGGCAAGGAGTACAAGTGCAAGGTCTCCAACAA | 468 |
| | AGCCCTCCCAGCCCCCATCGAGAAAACCATCTCCAAAGCCAAAGGGC AGCCCCGAGAACCACAGGTGTACACCCTGCCCCCATGCCGGGAGGAG ATGACCAAGAACCAGGTCAGCCTGTGGTGCCTGGTCAAAGGCTTCTA TCCCAGCGACATCGCCGTGGAGTGGGAGAGCAATGGGCAGCCGGAG AACAACTACAAGACCACGCCTCCCGTGCTGGACTCCGACGGCTCCTT CTTCCTCTACAGCAAGCTCACCGTGGACAAGAGCAGGTGGCAGCAGG GGAACGTCTTCTCATGCTCCGTGATGCATGAGGCTCTGCACAACCACT ACACGCAGAAGAGCCTCTCCCTGTCTCCGGGTAAA | |
| TraL-Q100C-Knob3 | GACATTCAGATGACCCAATCCCCCTCTAGCCTTTCAGCCTCCGTAGGG GACAGAGTTACAATAACCTGTAGGGCTAGTCAGGACGTTAATACAGC AGTCGCATGGTACCAGCAGAAGCCCGGTAAGGCTCCTAAGCTTTTGA TCTACAGTGCTTCTTTCCTGTATTCTGGTGTACCTAGCCGATTCTCAG GCTCTCGGAGTGGCACAGACTTCACTCTGACAATTTCAAGTCTCCAGC CAGAAGATTTTGCAACCTACTACTGCCAACAACACTACACTACTCCTC CAACCTTCGGATGTGGCACAAAAGTAGAGATTAAGCGTACGGTGGCA GCTCCCAGCGTTTTTATCTTTCCCCCATCCGACGAGCAGCTCAAGAGT GGCACTGCCTCTGTAGTTTGTTTGCTGAATAACTTCTATCCACGTGAA GCAAAAGTACAGTGGAAGGTCGATAATGCCCTTCAGAGCGGTAACA GCCAAGAAAGTGTTACCGAGCAAGATTCCAAAGATTCCACTTACAGT CTGTCCAGCACATTGACACTGAGTAAGGCTGATTACGAAAAACACAA GGTGTACGCATGCGAGGTGACACACCAAGGTCTTTCATCTCCTGTAA CTAAGAGCTTTAACCGGGGAGAATGTGAACCAAAGAGTAGTGACAA AACTCACACGTGCCCACCGTGCCCAGCACCTGAACTCCTGGGGGGAC CGTCAGTCTTCCTCTTCCCCCCAAAACCCAAGGACACCCTCATGATCT CCCGGACCCCTGAGGTCACATGCGTGGTGGTGGACGTGAGCCACGAA GACCCTGAGGTCAAGTTCAACTGGTACGTGGACGGCGTGGAGGTGCA TAATGCCAAGACAAAGCCGCGGGAGGAGCAGTACAACAGCACGTAC CGTGTGGTCAGCGTCCTCACCGTCCTGCACCAGGACTGGCTGAATGG CAAGGAGTACAAGTGCAAGGTCTCCAACAAAGCCCTCCCAGCCCCCA TCGAGAAAACCATCTCCAAAGCCAAAGGGCAGCCCCGAGAACCACA GGTGTACACCCTGCCCCCATGCCGGGAGGAGATGACCAAGAACCAG GTCAGCCTGTGGTGCCTGGTCAAAGGCTTCTATCCCAGCGACATCGC CGTGGAGTGGGAGAGCAATGGGCAGCCGGAGAACAACTACAAGACC ACGCCTCCCGTGCTGGACTCCGACGGCTCCTTCTTCCTCTACAGCAAG CTCACCGTGGACAAGAGCAGGTGGCAGCAGGGGAACGTCTTCTCATG CTCCGTGATGCATGAGGCTCTGCACAACCACTACACGCAGAAGAGCC TCTCCCTGTCTCCGGGTAAA | 469 |

Table 9 below shows the polypeptide sequences of the heavy chain variable region (VH) and light chain variable region (VL) of H01. Table 10 below shows the nucleotide sequences of the heavy chain variable region (VH) and light chain variable region (VL) of H01.

**[Table 9]**

| Name | Sequence | SEQ ID NO |
|---|---|---|
| H01 VH | EVQLVESGGGLVQPGGSLRLSCAASGFNIKDTYIHWVRQAPGKCLEW | 19 |
| | VARIYPTNGYTRYADSVKGRFTISADTSKNTAYLQMNSLRAEDTAVY YCSRWGGDGFYAMDYWGQGTLVTVSS | |
| H01 VL | DIQMTQSPSSLSASVGDRVTITCRASQDVNTAVAWYQQKPGKAPKLLI YSASFLYSGVPSRFSGSRSGTDFTLTISSLQPEDFATYYCQQHYTTPPTF GCGTKVEIK | 20 |

**[Table 10]**

| Name | Sequence | SEQ ID NO |
|---|---|---|
| H01 VH | GAAGTTCAACTTGTGGAGTCTGGAGGCGGTTTGGTACAACCTGGAG GTTCACTTCGTTTGTCATGTGCTGCCTCTGGGTTTAATATCAAGGAT ACTTATATTCATTGGGTTCGCCAGGCACCCGGAAAATGCCTGGAAT GGGTGGCCCGTATATATCCCACTAACGGTTACACTCGATATGCTGA CTCTGTAAAGGGTCGATTTACTATATCCGCTGATACTAGCAAAAAC ACTGCCTACCTCCAGATGAACAGTCTGCGCGCCGAGGATACAGCAG TTTATTATTGTTCCCGATGGGGAGGTGACGGGTTCTATGCCATGGAC TACTGGGGTCAAGGGACACTGGTAACCGTTTCTTCT | 470 |
| H01 VL | GACATTCAGATGACCCAATCCCCCTCTAGCCTTTCAGCCTCCGTAGG GGACAGAGTTACAATAACCTGTAGGGCTAGTCAGGACGTTAATACA GCAGTCGCATGGTACCAGCAGAAGCCCGGTAAGGCTCCTAAGCTTT TGATCTACAGTGCTTCTTTCCTGTATTCTGGTGTACCTAGCCGATTCT CAGGCTCTCGGAGTGGCACAGACTTCACTCTGACAATTTCAAGTCT CCAGCCAGAAGATTTTGCAACCTACTACTGCCAACAACACTACACT ACTCCTCCAACCTTCGGATGTGGCACAAAAGTAGAGATTAAG | 471 |

Table 11 below shows the H01 heavy chain and light chain CDR sequences.

**[Table 11]**

| Name | CDR region | Sequence | SEQ ID NO |
|---|---|---|---|
| H01 VH | CDR-H1 | DTYIH | 21 |
| | CDR-H2 | RIYPTNGYTRYADSVKG | 22 |
| | CDR-H3 | WGGDGFYAMDY | 23 |
| H01 VL | CDR-L1 | RASQDVNTAVA | 24 |
| | CDR-L2 | SASFLYS | 25 |
| | CDR-L3 | QQHYTTPPT | 26 |

### Example 2. Preparation of a novel antibody with two Fc domains using pertuzumab as a template

M3 is characterized by a (trastuzumab Fab)-(Fc)₂ structure with mutations of VH G44C and VL Q100C, hereinafter referred to as H01. Similarly, based on the VH and VL regions of pertuzumab (SEQ ID NOs: 27 and 28), the (pertuzumab Fab)-(Fc)₂ structure with the mutations VH G44C and VL Q100C is hereinafter referred to as P01. For P01, expression vectors containing the sequences corresponding to Fc-Hole (SEQ ID NO: 7), PerH-G44C-Knob (SEQ ID NO: 29), and PerL-Q100C-Knob (SEQ ID NO: 30) were co-transfected into EXPICHO-S^{™} (Gibco, A29127), and purification and analysis were performed in the same manner as described in Example 1.

**[Table 12]**

| Name | Sequence | SEQ ID NO |
|---|---|---|
| Pertuzumab VH domain | | 27 |
| Pertuzumab VL domain | | 28 |
| PerH-G44C-Knob | EVQLVESGGGLVQPGGSLRLSCAASGFTFTDYTMDWVRQAPGKCLEW VADVNPNSGGSIYNQRFKGRFTLSVDRSKNTLYLQMNSLRAEDTAVY YCARNLGPSFYFDYWGQGTLVTVSSASTKGPSVFPLAPSSKSTSGGTA ALGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPI EKTISKAKGQPREPQVYTLPPCREEMTKNQVSLWCLVKGFYPSDIAVE WESNGQPENNYKTTPPVLDSDGSFFLYSKLTVDKSRWQQGNVFSCSV MHEALHNHYTQKSLSLSPGK | 29 |
| PerL-Q100C-Knob | DIQMTQSPSSLSASVGDRVTITCKASQDVSIGVAWYQQKPGKAPKLLIY SASYRYTGVPSRFSGSGSGTDFTLTISSLQPEDFATYYCQQYYIYPYTFG CGTKVEIKRTVAAPSVFIFPPSDEQLKSGTASVVCLLNNFYPREAKVQW KVDNALQSGNSQESVTEQDSKDSTYSLSSTLTLSKADYEKHKVYACEV THQGLSSPVTKSFNRGECGGGGSGGGGSGGGGSEPKSSDKTHTCPPCP APELLGGPSVFLFPPKPKDTLMISRTPEVTCVVVDVSHEDPEVKFNWY VDGVEVHNAKTKPREEQYNSTYRVVSVLTVLHQDWLNGKEYKCKVS NKALPAPIEKTISKAKGQPREPQVYTLPPCREEMTKNQVSLWCLVKGF YPSDIAVEWESNGQPENNYKTTPPVLDSDGSFFLYSKLTVDKSRWQQG NVFSCSVMHEALHNHYTQKSLSLSPGK | 30 |

**[Table 13]**

| Name | Sequence | SEQ ID NO |
|---|---|---|
| Pertuzumab VH domain | GAAGTGCAACTGGTGGAGTCTGGTGGTGGATTGGTGCAGCCAGGCG GTTCTCTGCGACTTAGTTGTGCAGCCTCCGGCTTTACCTTCACTGAT TATACAATGGACTGGGTTCGGCAGGCACCCGGTAAGGGGCTTGAGT GGGTCGCCGACGTCAATCCTAATTCAGGGGGAAGTATTTATAACCA AAGGTTCAAGGGTCGATTTACATTGTCCGTAGATCGTAGTAAAAAT ACCCTCTACCTTCAAATGAACTCCCTGAGGGCAGAGGATACCGCAG TCTACTACTGCGCTCGTAACCTGGGGCCTAGTTTTTATTTCGATTAT TGGGGCCAAGGCACATTGGTAACTGTGTCTTCA | 414 |
| Pertuzumab VL domain | GATATACAAATGACACAATCTCCTAGTTCATTGAGTGCCTCAGTCG GCGACCGAGTCACTATAACTTGTAAAGCAAGCCAAGATGTTAGCAT TGGCGTAGCTTGGTATCAGCAGAAACCTGGAAAAGCACCAAAACTG CTTATCTACTCCGCTAGTTACCGTTACACCGGAGTTCCCTCAAGGTT | 415 |
| | TTCTGGCAGCGGAAGTGGGACTGACTTCACTCTGACTATTTCTTCAC TTCAGCCAGAAGACTTCGCTACTTATTACTGTCAGCAGTACTATATC TATCCCTATACATTTGGACAAGGAACCAAAGTTGAGATTAAA | |
| PerH-G44C-Knob | GAAGTACAGTTGGTGGAGTCTGGTGGAGGTCTTGTACAACCTGGCG GGAGTTTGCGGCTTTCCTGCGCTGCAAGCGGGTTTACCTTCACCGAT TATACCATGGATTGGGTACGCCAAGCCCCTGGTAAGTGCCTTGAGT GGGTTGCCGATGTAAACCCTAATTCCGGAGGAAGTATCTATAATCA ACGCTTCAAGGGCCGATTCACTCTGAGTGTGGATCGAAGCAAGAAC ACCTTGTACTTGCAGATGAATTCCTTGCGGGCTGAAGACACAGCCG TCTATTACTGCGCCCGAAATTTGGGGCCTTCATTCTATTTTGACTAT TGGGGTCAGGGAACTCTGGTAACTGTTTCAAGTGCTAGCACCAAAG GACCTAGTGTTTTTCCTCTTGCCCCTTCCTCAAAGTCTACCTCTGGG GGGACAGCCGCTCTGGGCTGCCTGGTCAAGGATTATTTCCCAGAGC CTGTCACTGTCAGTTGGAACTCTGGAGCCTTGACTTCTGGTGTTCAT ACATTTCCTGCTGTCCTTCAAAGCAGCGGCTTGTACTCATTGTCTTC TGTTGTGACAGTACCCTCAAGCAGCCTCGGCACTCAGACATACATC TGCAATGTCAACCACAAACCCTCAAATACAAAGGTAGATAAAAAA GTCGAACCAAAGTCTTGTGACAAAACTCACACGTGCCCACCGTGCC CAGCACCTGAACTCCTGGGGGGACCGTCAGTCTTCCTCTTCCCCCCA AAACCCAAGGACACCCTCATGATCTCCCGGACCCCTGAGGTCACAT GCGTGGTGGTGGACGTGAGCCACGAAGACCCTGAGGTCAAGTTCAA CTGGTACGTGGACGGCGTGGAGGTGCATAATGCCAAGACAAAGCC GCGGGAGGAGCAGTACAACAGCACGTACCGTGTGGTCAGCGTCCTC ACCGTCCTGCACCAGGACTGGCTGAATGGCAAGGAGTACAAGTGCA AGGTCTCCAACAAAGCCCTCCCAGCCCCCATCGAGAAAACCATCTC CAAAGCCAAAGGGCAGCCCCGAGAACCACAGGTGTACACCCTGCC CCCATGCCGGGAGGAGATGACCAAGAACCAGGTCAGCCTGTGGTG CCTGGTCAAAGGCTTCTATCCCAGCGACATCGCCGTGGAGTGGGAG AGCAATGGGCAGCCGGAGAACAACTACAAGACCACGCCTCCCGTG CTGGACTCCGACGGCTCCTTCTTCCTCTACAGCAAGCTCACCGTGGA CAAGAGCAGGTGGCAGCAGGGGAACGTCTTCTCATGCTCCGTGATG CATGAGGCTCTGCACAACCACTACACGCAGAAGAGCCTCTCCCTGT CTCCGGGTAAA | 472 |
| PerL-Q100C-Knob | GATATTCAAATGACCCAAAGTCCAAGTTCCCTTTCAGCATCTGTCGG TGATCGAGTCACTATAACTTGTAAGGCCAGCCAAGACGTTAGCATA GGAGTAGCATGGTACCAACAAAAGCCTGGGAAGGCTCCCAAACTTC TCATTTATTCTGCTTCCTACCGATATACTGGTGTCCCAAGTAGATTT TCTGGCAGCGGATCTGGAACTGATTTTACATTGACTATCAGCTCCCT CCAGCCTGAGGACTTCGCTACTTATTACTGCCAACAGTACTACATTT ATCCCTATACATTCGGTTGTGGGACCAAAGTAGAGATCAAACGTAC GGTGGCAGCTCCCAGCGTTTTTATCTTTCCCCCATCCGACGAGCAGC TCAAGAGTGGCACTGCCTCTGTAGTTTGTTTGCTGAATAACTTCTAT CCACGTGAAGCAAAAGTACAGTGGAAGGTCGATAATGCCCTTCAGA GCGGTAACAGCCAAGAAAGTGTTACCGAGCAAGATTCCAAAGATTC CACTTACAGTCTGTCCAGCACATTGACACTGAGTAAGGCTGATTAC | 473 |
| | GAAAAACACAAGGTGTACGCATGCGAGGTGACACACCAAGGTCTTT CATCTCCTGTAACTAAGAGCTTTAACCGGGGAGAATGTGGTGGTGG GGGCAGCGGGGGCGGAGGTAGTGGAGGCGGCGGTAGTGAACCAAA GAGTAGTGACAAAACTCACACGTGCCCACCGTGCCCAGCACCTGAA CTCCTGGGGGGACCGTCAGTCTTCCTCTTCCCCCCAAAACCCAAGG ACACCCTCATGATCTCCCGGACCCCTGAGGTCACATGCGTGGTGGT GGACGTGAGCCACGAAGACCCTGAGGTCAAGTTCAACTGGTACGTG GACGGCGTGGAGGTGCATAATGCCAAGACAAAGCCGCGGGAGGAG CAGTACAACAGCACGTACCGTGTGGTCAGCGTCCTCACCGTCCTGC ACCAGGACTGGCTGAATGGCAAGGAGTACAAGTGCAAGGTCTCCA ACAAAGCCCTCCCAGCCCCCATCGAGAAAACCATCTCCAAAGCCAA AGGGCAGCCCCGAGAACCACAGGTGTACACCCTGCCCCCATGCCGG GAGGAGATGACCAAGAACCAGGTCAGCCTGTGGTGCCTGGTCAAA GGCTTCTATCCCAGCGACATCGCCGTGGAGTGGGAGAGCAATGGGC AGCCGGAGAACAACTACAAGACCACGCCTCCCGTGCTGGACTCCGA CGGCTCCTTCTTCCTCTACAGCAAGCTCACCGTGGACAAGAGCAGG TGGCAGCAGGGGAACGTCTTCTCATGCTCCGTGATGCATGAGGCTC TGCACAACCACTACACGCAGAAGAGCCTCTCCCTGTCTCCGGGTAA A | |

Table 14 below shows the polypeptide sequences of the heavy chain variable region (VH) and light chain variable region (VL) of P01. Table 15 below shows the nucleotide sequences of the heavy chain variable region (VH) and light chain variable region (VL) of P01.

**[Table 14]**

| Name | Sequence | SEQ ID NO |
|---|---|---|
| P01 VH | EVQLVESGGGLVQPGGSLRLSCAASGFTFTDYTMDWVRQAPGKCLE WVADVNPNSGGSIYNQRFKGRFTLSVDRSKNTLYLQMNSLRAEDTA VYYCARNLGPSFYFDYWGQGTLVTVSS | 31 |
| P01 VL | DIQMTQSPSSLSASVGDRVTITCKASQDVSIGVAWYQQKPGKAPKLLI YSASYRYTGVPSRFSGSGSGTDFTLTISSLQPEDFATYYCQQYYIYPYT FGCGTKVEIK | 32 |

**[Table 15]**

| Name | Sequence | SEQ ID NO |
|---|---|---|
| P01 VH | GAAGTACAGTTGGTGGAGTCTGGTGGAGGTCTTGTACAACCTGGC GGGAGTTTGCGGCTTTCCTGCGCTGCAAGCGGGTTTACCTTCACCG ATTATACCATGGATTGGGTACGCCAAGCCCCTGGTAAGTGCCTTGA GTGGGTTGCCGATGTAAACCCTAATTCCGGAGGAAGTATCTATAAT CAACGCTTCAAGGGCCGATTCACTCTGAGTGTGGATCGAAGCAAG AACACCTTGTACTTGCAGATGAATTCCTTGCGGGCTGAAGACACAG CCGTCTATTACTGCGCCCGAAATTTGGGGCCTTCATTCTATTTTGAC TATTGGGGTCAGGGAACTCTGGTAACTGTTTCAAGT | 474 |
| P01 VL | GATATTCAAATGACCCAAAGTCCAAGTTCCCTTTCAGCATCTGTCG GTGATCGAGTCACTATAACTTGTAAGGCCAGCCAAGACGTTAGCA TAGGAGTAGCATGGTACCAACAAAAGCCTGGGAAGGCTCCCAAAC | 475 |
| | TTCTCATTTATTCTGCTTCCTACCGATATACTGGTGTCCCAAGTAGA TTTTCTGGCAGCGGATCTGGAACTGATTTTACATTGACTATCAGCT CCCTCCAGCCTGAGGACTTCGCTACTTATTACTGCCAACAGTACTA CATTTATCCCTATACATTCGGTTGTGGGACCAAAGTAGAGATCAAA | |

Table 16 below shows CDR sequences in heavy and light chains of the P01.

**[Table 16]**

| Name | | Sequence | SEQ ID NO |
|---|---|---|---|
| P01 VH | CDR-H1 | DYTMD | 33 |
| | CDR-H2 | DVNPNSGGSIYNQRFKG | 34 |
| | CDR-H3 | NLGPSFYFDY | 35 |
| P01 VL | CDR-L1 | KASQDVSIGVA | 36 |
| | CDR-L2 | SASYRYT | 37 |
| | CDR-L3 | QQYYIYPYT | 38 |

### Example 3. Analysis of H01 and P01 byproducts through papain digestion

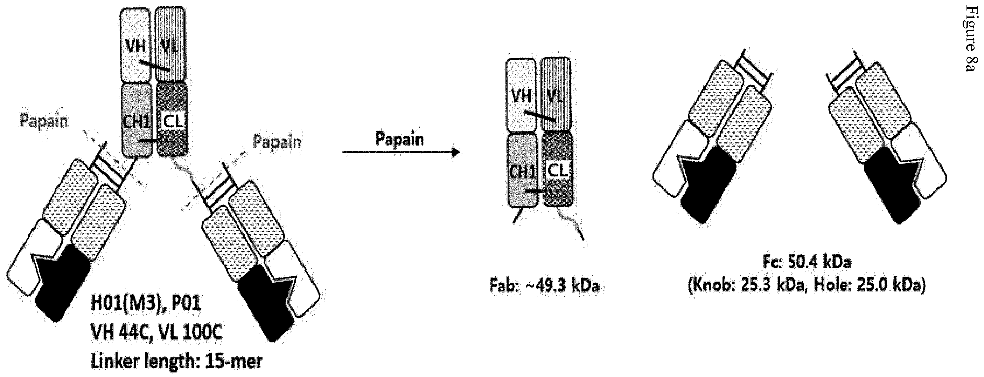

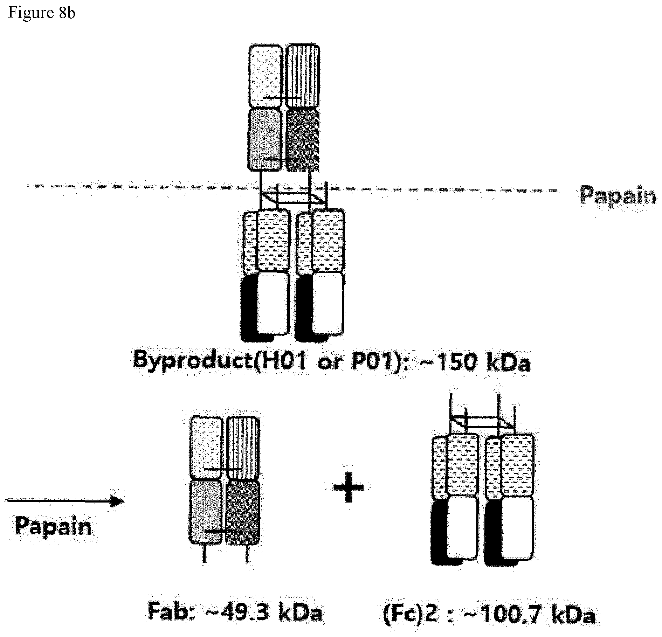

Papain recognizes specific sequences in the hinge region and induces antibody digestion. In the case of the Fab-(Fc)₂ structure, when papain digestion is performed, it is cleaved into a Fab portion of approximately 49.3 kDa and two Fc domains of approximately 50.4 kDa (Figure 8a). However, if abnormal disulfide bonds are formed in the hinge region, unwanted inter-chain disulfide bond byproducts could be observed (Figure 8b). In this case, a Fab fragment of approximately 49.3 kDa and an abnormal (Fc)₂ product of approximately 100.7 kDa could be observed (Figure 8b).

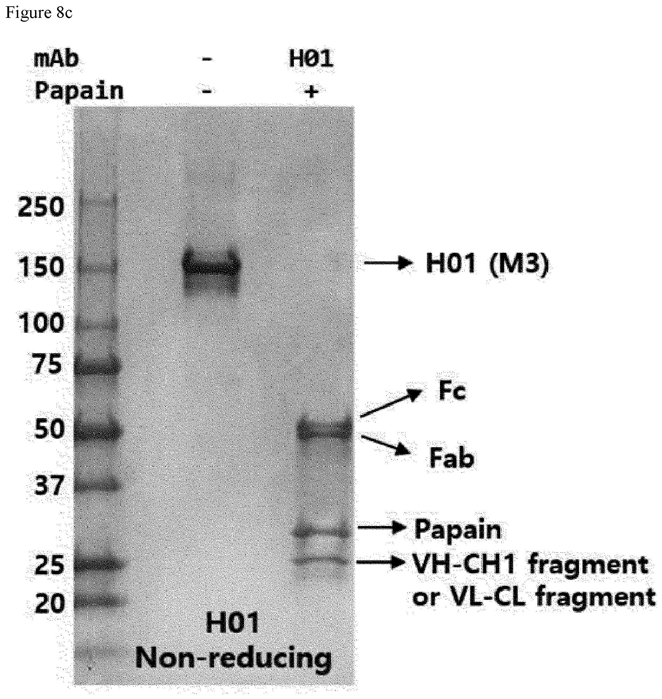

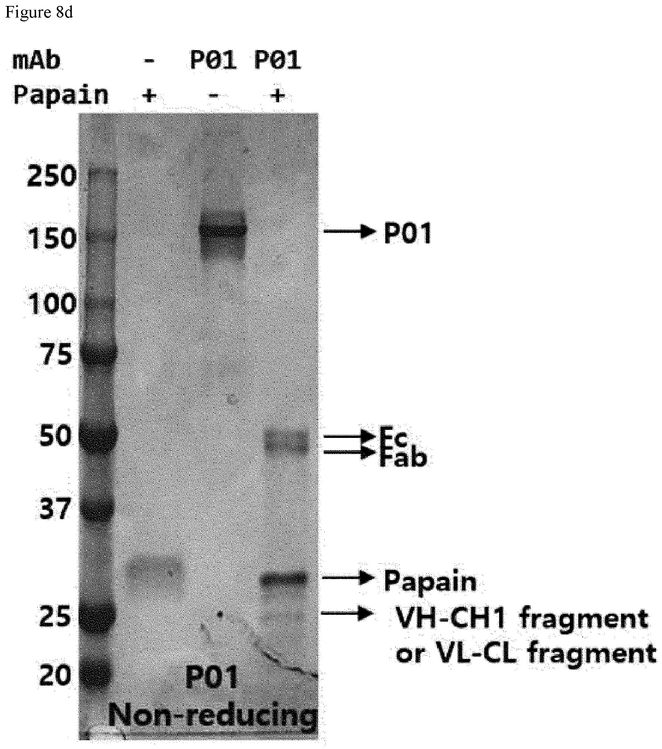

To verify this, papain digestion of H01 and P01 was performed. Papain (Sigma, P3125) was used by diluting it to 0.1 mg/mL in digestion buffer (20 mM EDTA + 10 mM Cys-HCl in PBS pH 7.4). 200 µg of H01 and P01 were digested at 37°C for 2 hours, and then SDS-PAGE was performed. As a result of SDS-PAGE performed under non-reducing conditions, abnormal (Fc)₂ at about 100 kDa was not identified (Figures 8c and 8d).

### Example 4. Analysis of physical properties of H01wt

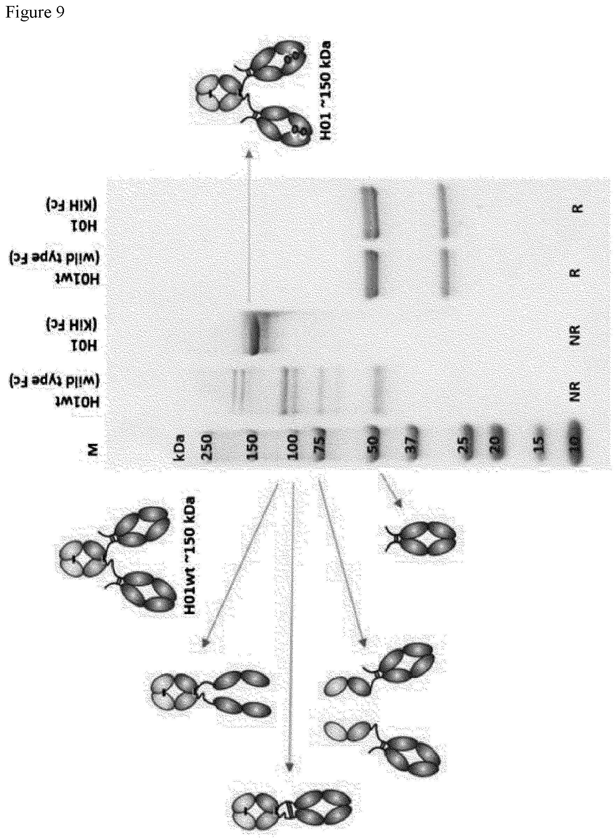

In H01, four Fc monomers are assembled into two Fc dimers due to knob-into-hole mutations, resulting in a structure as shown in Figure 6a. To analyze the effect of the knob-into-hole mutations on the formation of H01 structure, the Fc hole monomer polypeptide (SEQ ID NO: 7) was substituted with a polypeptide (SEQ ID NO: 39) corresponding to the wild type IgG1 Fc monomer (Table 17). The two knob polypeptides (SEQ ID NOs: 14 and 15) constituting H01 were also substituted with polypeptides (SEQ ID NOs: 40 and 41) corresponding to the wild type IgG1 Fc monomer (Table 17). This novel antibody format consisting of two wtFc polypeptides (SEQ ID NO: 39), one TraH-G44C-wtFc polypeptide (SEQ ID NO: 40), and one TraL-Q100C-wtFc polypeptide (SEQ ID NO: 41) is referred to as H01wt (Figure 9).

Expression vectors containing sequences corresponding to wtFc (SEQ ID NO: 39), TraH-G44C-wtFc (SEQ ID NO: 40), and TraL-Q100C-wtFc (SEQ ID NO: 41) were co-transfected into EXPICHO-S^{™} (Gibco, A29127), and purification and analysis were performed in the same manner as described in Example 1. SDS-PAGE analysis under non-reducing conditions (NR) identified a small amount of H01wt at about 150 kDa, and most of H01wt were expressed as abnormally structured byproducts (Figure 9). In the case of H01, which has knob-into-hole mutations in the Fc region, each polypeptide was efficiently assembled into a product that is identified at about 150 kDa (Figure 9).

**[Table 17]**

| Name | Sequence | SEQ ID NO |
|---|---|---|
| wtFc | DKTHTCPPCPAPELLGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVYTLPPSREEMTKNQ VSLTCLVKGFYPSDIAVEWESNGQPENNYKTTPPVLDSDGSFFLYSKL TVDKSRWQQGNVFSCSVMHEALHNHYTQKSLSLSPGK | 39 |
| TraH-G44C-wtFc | EVQLVESGGGLVQPGGSLRLSCAASGFNIKDTYIHWVRQAPGKCLEW VARIYPTNGYTRYADSVKGRFTISADTSKNTAYLQMNSLRAEDTAVY YCSRWGGDGFYAMDYWGQGTLVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVD GVEVHNAKTKPREEQYNSTYRVVSVLTVLHQDWLNGKEYKCKVSN KALPAPIEKTISKAKGQPREPQVYTLPPSREEMTKNQVSLTCLVKGFY PSDIAVEWESNGQPENNYKTTPPVLDSDGSFFLYSKLTVDKSRWQQG NVFSCSVMHEALHNHYTQKSLSLSPGK | 40 |
| TraL-Q100C-wtFc | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGCGTKVEIKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGECGGGGSGGGGSGGGGSEPKSSDKTHT CPPCPAPELLGGPSVFLFPPKPKDTLMISRTPEVTCVVVDVSHEDPEVK FNWYVDGVEVHNAKTKPREEQYNSTYRVVSVLTVLHQDWLNGKEY KCKVSNKALPAPIEKTISKAKGQPREPQVYTLPPSREEMTKNQVSLTC LVKGFYPSDIAVEWESNGQPENNYKTTPPVLDSDGSFFLYSKLTVDKS RWQQGNVFSCSVMHEALHNHYTQKSLSLSPGK | 41 |

Table 18 below shows the nucleotide sequences encoding wtFc, TraH-G44C-wtFc, and TraL-Q100C-wtFc of HO1wt.

**[Table 18]**

| Name | Sequence | SEQ ID NO |
|---|---|---|
| wtFc | GACAAAACTCACACATGCCCACCGTGCCCAGCACCTGAACTCCTG GGGGGACCGTCAGTCTTCCTCTTCCCCCCAAAACCCAAGGACACCC TCATGATCTCCCGGACCCCTGAGGTCACATGCGTGGTGGTGGACGT GAGCCACGAAGACCCTGAGGTCAAGTTCAACTGGTACGTGGACGG | 476 |
| | CGTGGAGGTGCATAATGCCAAGACAAAGCCGCGGGAGGAGCAGT ACAACAGCACGTACCGTGTGGTCAGCGTCCTCACCGTCCTGCACCA GGACTGGCTGAATGGCAAGGAGTACAAGTGCAAGGTCTCCAACAA AGCCCTCCCAGCCCCCATCGAGAAAACCATCTCCAAAGCCAAAGG GCAGCCCCGAGAACCACAGGTGTACACCCTGCCCCCATCCCGGGA GGAGATGACCAAGAACCAGGTCAGCCTGACGTGCCTGGTCAAAGG CTTCTATCCCAGCGACATCGCCGTGGAGTGGGAGAGCAATGGGCA GCCGGAGAACAACTACAAGACCACGCCTCCCGTGCTGGACTCCGA CGGCTCCTTCTTCCTCTACAGCAAGCTCACCGTGGACAAGAGCAGG TGGCAGCAGGGGAACGTCTTCTCATGCTCCGTGATGCATGAGGCTC TGCACAACCACTACACGCAGAAGAGCCTCTCCCTGTCTCCGGGTAA A | |
| TraH-G44C-wtFc | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGGA ATGGGTGGCCCGTATATATCCCACTAACGGTTACACTCGATATGCT GACTCTGTAAAGGGTCGATTTACTATATCCGCTGATACTAGCAAAA ACACTGCCTACCTCCAGATGAACAGTCTGCGCGCCGAGGATACAG CAGTTTATTATTGTTCCCGATGGGGAGGTGACGGGTTCTATGCCAT GGACTACTGGGGTCAAGGGACACTGGTAACCGTTTCTTCTGCTAGC ACCAAAGGACCTAGTGTTTTTCCTCTTGCCCCTTCCTCAAAGTCTAC CTCTGGGGGGACAGCCGCTCTGGGCTGCCTGGTCAAGGATTATTTC CCAGAGCCTGTCACTGTCAGTTGGAACTCTGGAGCCTTGACTTCTG GTGTTCATACATTTCCTGCTGTCCTTCAAAGCAGCGGCTTGTACTC ATTGTCTTCTGTTGTGACAGTACCCTCAAGCAGCCTCGGCACTCAG ACATACATCTGCAATGTCAACCACAAACCCTCAAATACAAAGGTA GATAAAAAAGTCGAACCAAAGTCTTGTGACAAAACTCACACGTGC CCACCGTGCCCAGCACCTGAACTCCTGGGGGGACCGTCAGTCTTCC TCTTCCCCCCAAAACCCAAGGACACCCTCATGATCTCCCGGACCCC TGAGGTCACATGCGTGGTGGTGGACGTGAGCCACGAAGACCCTGA GGTCAAGTTCAACTGGTACGTGGACGGCGTGGAGGTGCATAATGC CAAGACAAAGCCGCGGGAGGAGCAGTACAACAGCACGTACCGTGT GGTCAGCGTCCTCACCGTCCTGCACCAGGACTGGCTGAATGGCAA GGAGTACAAGTGCAAGGTCTCCAACAAAGCCCTCCCAGCCCCCAT CGAGAAAACCATCTCCAAAGCCAAAGGGCAGCCCCGAGAACCACA GGTGTACACCCTGCCCCCAAGCCGGGAGGAGATGACCAAGAACCA GGTCAGCCTGACGTGCCTGGTCAAAGGCTTCTATCCCAGCGACATC GCCGTGGAGTGGGAGAGCAATGGGCAGCCGGAGAACAACTACAA GACCACGCCTCCCGTGCTGGACTCCGACGGCTCCTTCTTCCTCTAC AGCAAGCTCACCGTGGACAAGAGCAGGTGGCAGCAGGGGAACGT CTTCTCATGCTCCGTGATGCATGAGGCTCTGCACAACCACTACACG CAGAAGAGCCTCTCCCTGTCTCCGGGTAAA | 477 |
| TraL-Q100C-wtFc | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC | 478 |
| | TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACTA CACTACTCCTCCAACCTTCGGATGTGGCACAAAAGTAGAGATTAA GCGTACGGTGGCAGCTCCCAGCGTTTTTATCTTTCCCCCATCCGAC GAGCAGCTCAAGAGTGGCACTGCCTCTGTAGTTTGTTTGCTGAATA ACTTCTATCCACGTGAAGCAAAAGTACAGTGGAAGGTCGATAATG CCCTTCAGAGCGGTAACAGCCAAGAAAGTGTTACCGAGCAAGATT CCAAAGATTCCACTTACAGTCTGTCCAGCACATTGACACTGAGTAA GGCTGATTACGAAAAACACAAGGTGTACGCATGCGAGGTGACACA CCAAGGTCTTTCATCTCCTGTAACTAAGAGCTTTAACCGGGGAGAA TGTGGTGGTGGGGGCAGCGGGGGCGGAGGTAGTGGAGGCGGCGG TAGTGAACCAAAGAGTAGTGACAAAACTCACACGTGCCCACCGTG CCCAGCACCTGAACTCCTGGGGGGACCGTCAGTCTTCCTCTTCCCC CCAAAACCCAAGGACACCCTCATGATCTCCCGGACCCCTGAGGTC ACATGCGTGGTGGTGGACGTGAGCCACGAAGACCCTGAGGTCAAG TTCAACTGGTACGTGGACGGCGTGGAGGTGCATAATGCCAAGACA AAGCCGCGGGAGGAGCAGTACAACAGCACGTACCGTGTGGTCAGC GTCCTCACCGTCCTGCACCAGGACTGGCTGAATGGCAAGGAGTAC AAGTGCAAGGTCTCCAACAAAGCCCTCCCAGCCCCCATCGAGAAA ACCATCTCCAAAGCCAAAGGGCAGCCCCGAGAACCACAGGTGTAC ACCCTGCCCCCAAGCCGGGAGGAGATGACCAAGAACCAGGTCAGC CTGACGTGCCTGGTCAAAGGCTTCTATCCCAGCGACATCGCCGTGG AGTGGGAGAGCAATGGGCAGCCGGAGAACAACTACAAGACCACG CCTCCCGTGCTGGACTCCGACGGCTCCTTCTTCCTCTACAGCAAGC TCACCGTGGACAAGAGCAGGTGGCAGCAGGGGAACGTCTTCTCAT GCTCCGTGATGCATGAGGCTCTGCACAACCACTACACGCAGAAGA GCCTCTCCCTGTCTCCGGGTAAA | |

### Example 5. Characterization of H01Fv variant

The schematic diagram of Fv-(Fc)₂, in which two Fc domains are fused in parallel to an antibody Fv fragment, is shown in Figures 10a to 10g. Fv consists of a VH domain and a VL domain. In order to improve interaction of the domains at the domain interfaces, a disulfide bond was formed artificially by substituting an amino acid at a specific position with cysteine (Figures 10a to 10h, Table 19).

**[Table 19]**

| | VH Mutation site (Kabat numbering) | VL Mutation site (Kabat numbering) | MW (kDa) |
|---|---|---|---|
| H01Fv1 | G44C | Q100C | 128.21 |
| H01Fv2 | Q105C | A43C | 128.19 |
| H01Fv3 | F122C | S121C | 130.98 |
| H01Fv4 | G44C, Q105C | Q100C, A43C | 130.01 |
| H01Fv5 | G44C, F122C | Q100C, S121C | 130.00 |
| H01Fv6 | Q105C, F126C | A43C, S121C | 130.99 |
| H01Fv7 | G44C, Q105C, F126C | Q100C, A43C, S121C | 131.01 |

Table 20 shows the polypeptide sequences constituting H01Fv1 to H01Fv7.

**[Table 20]**

| Name | Sequence | SEQ ID NO |
|---|---|---|
| Fc-Hole-RF | DKTHTCPPCPAPELLGGPSVFLFPPKPKDTLMISRTPEVTCVVVDVSHE DPEVKFNWYVDGVEVHNAKTKPREEQYNSTYRVVSVLTVLHQDWL NGKEYKCKVSNKALPAPIEKTISKAKGQPREPQVCTLPPSREEMTKNQ VSLSCAVKGFYPSDIAVEWESNGQPENNYKTTPPVLDSDGSFFLVSKL TVDKSRWQQGNVFSCSVMHEALHNRFTQKSLSLSPGK | 789 |
| H01Fv1-HC | EVQLVESGGGLVQPGGSLRLSCAASGFNIKDTYIHWVRQAPGKCLEW VARIYPTNGYTRYADSVKGRFTISADTSKNTAYLQMNSLRAEDTAVY YCSRWGGDGFYAMDYWGQGTLVTVSSEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPCREEMTKNQVSLWCLVKGFYPSD IAVEWESNGQPENNYKTTPPVLDSDGSFFLYSKLTVDKSRWQQGNVF SCSVMHEALHNHYTQKSLSLSPGK | 790 |
| H01Fv1-LC | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGCGTKVEIKREPKSCDKTHTCPPCPAPELLGGPSVFLFPPKPKDTLMI SRTPEVTCVVVDVSHEDPEVKFNWYVDGVEVHNAKTKPREEQYNST YRVVSVLTVLHQDWLNGKEYKCKVSNKALPAPIEKTISKAKGQPREP QVYTLPPCREEMTKNQVSLWCLVKGFYPSDIAVEWESNGQPENNYK TTPPVLDSDGSFFLYSKLTVDKSRWQQGNVFSCSVMHEALHNHYTQK SLSLSPGK | 791 |
| H01Fv2-HC | EVQLVESGGGLVQPGGSLRLSCAASGFNIKDTYIHWVRQAPGKGLEW VARIYPTNGYTRYADSVKGRFTISADTSKNTAYLQMNSLRAEDTAVY YCSRWGGDGFYAMDYWGCGTLVTVSSEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPCREEMTKNQVSLWCLVKGFYPSD IAVEWESNGQPENNYKTTPPVLDSDGSFFLYSKLTVDKSRWQQGNVF SCSVMHEALHNHYTQKSLSLSPGK | 792 |
| H01Fv2-LC | DIQMTQSPSSLSASVGDRVTITCRASQDVNTAVAWYQQKPGKCPKLLI YSASFLYSGVPSRFSGSRSGTDFTLTISSLQPEDFATYYCQQHYTTPPTF GQGTKVEIKREPKSCDKTHTCPPCPAPELLGGPSVFLFPPKPKDTLMIS RTPEVTCVVVDVSHEDPEVKFNWYVDGVEVHNAKTKPREEQYNSTY RVVSVLTVLHQDWLNGKEYKCKVSNKALPAPIEKTISKAKGQPREPQ VYTLPPCREEMTKNQVSLWCLVKGFYPSDIAVEWESNGQPENNYKTT PPVLDSDGSFFLYSKLTVDKSRWQQGNVFSCSVMHEALHNHYTQKSL SLSPGK | 793 |
| H01Fv3-HC | EVQLVESGGGLVQPGGSLRLSCAASGFNIKDTYIHWVRQAPGKGLEW | 794 |
| | VARIYPTNGYTRYADSVKGRFTISADTSKNTAYLQMNSLRAEDTAVY YCSRWGGDGFYAMDYWGQGTLVTVSSASTKGPSVCPLAPEPKSCDK THTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQVS LWCLVKGFYPSDIAVEWESNGQPENNYKTTPPVLDSDGSFFLYSKLT VDKSRWQQGNVFSCSVMHEALHNHYTQKSLSLSPGK | |
| H01Fv3-LC | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGQGTKVEIKRTVAAPSVFIFPPCGGGSEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPCREEMTKNQVSLWCLVKGFYPSDI AVEWESNGQPENNYKTTPPVLDSDGSFFLYSKLTVDKSRWQQGNVFS CSVMHEALHNHYTQKSLSLSPGK | 795 |
| H01Fv4-HC | EVQLVESGGGLVQPGGSLRLSCAASGFNIKDTYIHWVRQAPGKCLEW VARIYPTNGYTRYADSVKGRFTISADTSKNTAYLQMNSLRAEDTAVY YCSRWGGDGFYAMDYWGCGTLVTVSSASTKGPSVCPLAPEPKSCDK THTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQVS LWCLVKGFYPSDIAVEWESNGQPENNYKTTPPVLDSDGSFFLYSKLT VDKSRWQQGNVFSCSVMHEALHNHYTQKSLSLSPGK | 796 |
| H01Fv4-LC | DIQMTQSPSSLSASVGDRVTITCRASQDVNTAVAWYQQKPGKCPKLLI YSASFLYSGVPSRFSGSRSGTDFTLTISSLQPEDFATYYCQQHYTTPPTF GCGTKVEIKREPKSCDKTHTCPPCPAPELLGGPSVFLFPPKPKDTLMIS RTPEVTCVVVDVSHEDPEVKFNWYVDGVEVHNAKTKPREEQYNSTY RVVSVLTVLHQDWLNGKEYKCKVSNKALPAPIEKTISKAKGQPREPQ VYTLPPCREEMTKNQVSLWCLVKGFYPSDIAVEWESNGQPENNYKTT PPVLDSDGSFFLYSKLTVDKSRWQQGNVFSCSVMHEALHNHYTQKSL SLSPGK | 797 |
| H01Fv5-HC | EVQLVESGGGLVQPGGSLRLSCAASGFNIKDTYIHWVRQAPGKCLEW VARIYPTNGYTRYADSVKGRFTISADTSKNTAYLQMNSLRAEDTAVY YCSRWGGDGFYAMDYWGQGTLVTVSSASTKGPSVCPLAPEPKSCDK THTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQVS LWCLVKGFYPSDIAVEWESNGQPENNYKTTPPVLDSDGSFFLYSKLT VDKSRWQQGNVFSCSVMHEALHNHYTQKSLSLSPGK | 798 |
| H01Fv5-LC | DIQMTQSPSSLSASVGDRVTITCRASQDVNTAVAWYQQKPGKAPKLL IYSASFLYSGVPSRFSGSRSGTDFTLTISSLQPEDFATYYCQQHYTTPPT FGCGTKVEIKRTVAAPSVFIFPPCGGGSEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPCREEMTKNQVSLWCLVKGFYPSDI | 799 |
| | AVEWESNGQPENNYKTTPPVLDSDGSFFLYSKLTVDKSRWQQGNVFS CSVMHEALHNHYTQKSLSLSPGK | |
| H01Fv6-HC | EVQLVESGGGLVQPGGSLRLSCAASGFNIKDTYIHWVRQAPGKGLEW VARIYPTNGYTRYADSVKGRFTISADTSKNTAYLQMNSLRAEDTAVY YCSRWGGDGFYAMDYWGCGTLVTVSSASTKGPSVCPLAPEPKSCDK THTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQVS LWCLVKGFYPSDIAVEWESNGQPENNYKTTPPVLDSDGSFFLYSKLT VDKSRWQQGNVFSCSVMHEALHNHYTQKSLSLSPGK | 800 |
| H01Fv6-LC | DIQMTQSPSSLSASVGDRVTITCRASQDVNTAVAWYQQKPGKCPKLLI YSASFLYSGVPSRFSGSRSGTDFTLTISSLQPEDFATYYCQQHYTTPPTF GQGTKVEIKRTVAAPSVFIFPPCGGGSEPKSCDKTHTCPPCPAPELLGG PSVFLFPPKPKDTLMISRTPEVTCVVVDVSHEDPEVKFNWYVDGVEV HNAKTKPREEQYNSTYRVVSVLTVLHQDWLNGKEYKCKVSNKALPA PIEKTISKAKGQPREPQVYTLPPCREEMTKNQVSLWCLVKGFYPSDIA VEWESNGQPENNYKTTPPVLDSDGSFFLYSKLTVDKSRWQQGNVFSC SVMHEALHNHYTQKSLSLSPGK | 801 |
| H01Fv7-HC | EVQLVESGGGLVQPGGSLRLSCAASGFNIKDTYIHWVRQAPGKCLEW VARIYPTNGYTRYADSVKGRFTISADTSKNTAYLQMNSLRAEDTAVY YCSRWGGDGFYAMDYWGCGTLVTVSSASTKGPSVCPLAPEPKSCDK THTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQVS LWCLVKGFYPSDIAVEWESNGQPENNYKTTPPVLDSDGSFFLYSKLT VDKSRWQQGNVFSCSVMHEALHNHYTQKSLSLSPGK | 802 |
| H01Fv7-LC | DIQMTQSPSSLSASVGDRVTITCRASQDVNTAVAWYQQKPGKCPKLLI YSASFLYSGVPSRFSGSRSGTDFTLTISSLQPEDFATYYCQQHYTTPPTF GCGTKVEIKRTVAAPSVFIFPPCGGGSEPKSCDKTHTCPPCPAPELLGG PSVFLFPPKPKDTLMISRTPEVTCVVVDVSHEDPEVKFNWYVDGVEV HNAKTKPREEQYNSTYRVVSVLTVLHQDWLNGKEYKCKVSNKALPA PIEKTISKAKGQPREPQVYTLPPCREEMTKNQVSLWCLVKGFYPSDIA VEWESNGQPENNYKTTPPVLDSDGSFFLYSKLTVDKSRWQQGNVFSC SVMHEALHNHYTQKSLSLSPGK | 803 |

Table 21 shows the nucleotide sequences encoding polypeptides constituting H01Fv1 to H01Fv7.

**[Table 21]**

| Name | Sequence | SEQ ID NO |
|---|---|---|
| Fc-Hole-RF | GACAAAACTCACACATGCCCACCGTGCCCAGCACCTGAACTCCTG GGGGGACCGTCAGTCTTCCTCTTCCCCCCAAAACCCAAGGACACCC TCATGATCTCCCGGACCCCTGAGGTCACATGCGTGGTGGTGGACGT GAGCCACGAAGACCCTGAGGTCAAGTTCAACTGGTACGTGGACGG CGTGGAGGTGCATAATGCCAAGACAAAGCCGCGGGAGGAGCAGT ACAACAGCACGTACCGTGTGGTCAGCGTCCTCACCGTCCTGCACCA | 804 |
| | GGACTGGCTGAATGGCAAGGAGTACAAGTGCAAGGTCTCCAACAA AGCCCTCCCAGCCCCCATCGAGAAAACCATCTCCAAAGCCAAAGG GCAGCCCCGAGAACCACAGGTGTGCACCCTGCCCCCATCCCGGGA GGAGATGACCAAGAACCAGGTCAGCCTGAGCTGCGCGGTCAAAGG CTTCTATCCCAGCGACATCGCCGTGGAGTGGGAGAGCAATGGGCA GCCGGAGAACAACTACAAGACCACGCCTCCCGTGCTGGACTCCGA CGGCTCCTTCTTCCTCGTGAGCAAGCTCACCGTGGACAAGAGCAGG TGGCAGCAGGGGAACGTCTTCTCATGCTCCGTGATGCATGAGGCTC TGCACAACAGATTTACGCAGAAGAGCCTCTCCCTGTCTCCGGGTAA A | |
| H01Fv1-HC | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGGA ATGGGTGGCCCGTATATATCCCACTAACGGTTACACTCGATATGCT GACTCTGTAAAGGGTCGATTTACTATATCCGCTGATACTAGCAAAA ACACTGCCTACCTCCAGATGAACAGTCTGCGCGCCGAGGATACAG CAGTTTATTATTGTTCCCGATGGGGAGGTGACGGGTTCTATGCCAT GGACTACTGGGGTCAAGGGACACTGGTAACCGTTTCTTCTGAACCA AAGTCTTGTGACAAAACTCACACGTGCCCACCGTGCCCAGCACCTG AACTCCTGGGGGGACCGTCAGTCTTCCTCTTCCCCCCAAAACCCAA GGACACCCTCATGATCTCCCGGACCCCTGAGGTCACATGCGTGGTG GTGGACGTGAGCCACGAAGACCCTGAGGTCAAGTTCAACTGGTAC GTGGACGGCGTGGAGGTGCATAATGCCAAGACAAAGCCGCGGGA GGAGCAGTACAACAGCACGTACCGTGTGGTCAGCGTCCTCACCGT CCTGCACCAGGACTGGCTGAATGGCAAGGAGTACAAGTGCAAGGT CTCCAACAAAGCCCTCCCAGCCCCCATCGAGAAAACCATCTCCAA AGCCAAAGGGCAGCCCCGAGAACCACAGGTGTACACCCTGCCCCC ATGCCGGGAGGAGATGACCAAGAACCAGGTCAGCCTGTGGTGCCT GGTCAAAGGCTTCTATCCCAGCGACATCGCCGTGGAGTGGGAGAG CAATGGGCAGCCGGAGAACAACTACAAGACCACGCCTCCCGTGCT GGACTCCGACGGCTCCTTCTTCCTCTACAGCAAGCTCACCGTGGAC AAGAGCAGGTGGCAGCAGGGGAACGTCTTCTCATGCTCCGTGATG CATGAGGCTCTGCACAACCACTACACGCAGAAGAGCCTCTCCCTGT CTCCGGGTAAA | 805 |
| H01Fv1-LC | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACTA CACTACTCCTCCAACCTTCGGATGTGGCACAAAAGTAGAGATTAA GCGTGAACCAAAGTCTTGTGACAAAACTCACACGTGCCCACCGTG CCCAGCACCTGAACTCCTGGGGGGACCGTCAGTCTTCCTCTTCCCC CCAAAACCCAAGGACACCCTCATGATCTCCCGGACCCCTGAGGTC ACATGCGTGGTGGTGGACGTGAGCCACGAAGACCCTGAGGTCAAG | 806 |
| | TTCAACTGGTACGTGGACGGCGTGGAGGTGCATAATGCCAAGACA AAGCCGCGGGAGGAGCAGTACAACAGCACGTACCGTGTGGTCAGC GTCCTCACCGTCCTGCACCAGGACTGGCTGAATGGCAAGGAGTAC AAGTGCAAGGTCTCCAACAAAGCCCTCCCAGCCCCCATCGAGAAA ACCATCTCCAAAGCCAAAGGGCAGCCCCGAGAACCACAGGTGTAC ACCCTGCCCCCATGCCGGGAGGAGATGACCAAGAACCAGGTCAGC CTGTGGTGCCTGGTCAAAGGCTTCTATCCCAGCGACATCGCCGTGG AGTGGGAGAGCAATGGGCAGCCGGAGAACAACTACAAGACCACG CCTCCCGTGCTGGACTCCGACGGCTCCTTCTTCCTCTACAGCAAGC TCACCGTGGACAAGAGCAGGTGGCAGCAGGGGAACGTCTTCTCAT GCTCCGTGATGCATGAGGCTCTGCACAACCACTACACGCAGAAGA GCCTCTCCCTGTCTCCGGGTAAA | |
| H01Fv2-HC | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAAGGGCTGG AATGGGTGGCCCGTATATATCCCACTAACGGTTACACTCGATATGC TGACTCTGTAAAGGGTCGATTTACTATATCCGCTGATACTAGCAAA AACACTGCCTACCTCCAGATGAACAGTCTGCGCGCCGAGGATACA GCAGTTTATTATTGTTCCCGATGGGGAGGTGACGGGTTCTATGCCA TGGACTACTGGGGTTGTGGGACACTGGTAACCGTTTCTTCTGAACC AAAGTCTTGTGACAAAACTCACACGTGCCCACCGTGCCCAGCACCT GAACTCCTGGGGGGACCGTCAGTCTTCCTCTTCCCCCCAAAACCCA AGGACACCCTCATGATCTCCCGGACCCCTGAGGTCACATGCGTGGT GGTGGACGTGAGCCACGAAGACCCTGAGGTCAAGTTCAACTGGTA CGTGGACGGCGTGGAGGTGCATAATGCCAAGACAAAGCCGCGGGA GGAGCAGTACAACAGCACGTACCGTGTGGTCAGCGTCCTCACCGT CCTGCACCAGGACTGGCTGAATGGCAAGGAGTACAAGTGCAAGGT CTCCAACAAAGCCCTCCCAGCCCCCATCGAGAAAACCATCTCCAA AGCCAAAGGGCAGCCCCGAGAACCACAGGTGTACACCCTGCCCCC ATGCCGGGAGGAGATGACCAAGAACCAGGTCAGCCTGTGGTGCCT GGTCAAAGGCTTCTATCCCAGCGACATCGCCGTGGAGTGGGAGAG CAATGGGCAGCCGGAGAACAACTACAAGACCACGCCTCCCGTGCT GGACTCCGACGGCTCCTTCTTCCTCTACAGCAAGCTCACCGTGGAC AAGAGCAGGTGGCAGCAGGGGAACGTCTTCTCATGCTCCGTGATG CATGAGGCTCTGCACAACCACTACACGCAGAAGAGCCTCTCCCTGT CTCCGGGTAAA | 807 |
| H01Fv2-LC | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGTGTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACTA CACTACTCCTCCAACCTTCGGACAGGGCACAAAAGTAGAGATTAA GCGTGAACCAAAGTCTTGTGACAAAACTCACACGTGCCCACCGTG CCCAGCACCTGAACTCCTGGGGGGACCGTCAGTCTTCCTCTTCCCC | 808 |
| | CCAAAACCCAAGGACACCCTCATGATCTCCCGGACCCCTGAGGTC ACATGCGTGGTGGTGGACGTGAGCCACGAAGACCCTGAGGTCAAG TTCAACTGGTACGTGGACGGCGTGGAGGTGCATAATGCCAAGACA AAGCCGCGGGAGGAGCAGTACAACAGCACGTACCGTGTGGTCAGC GTCCTCACCGTCCTGCACCAGGACTGGCTGAATGGCAAGGAGTAC AAGTGCAAGGTCTCCAACAAAGCCCTCCCAGCCCCCATCGAGAAA ACCATCTCCAAAGCCAAAGGGCAGCCCCGAGAACCACAGGTGTAC ACCCTGCCCCCATGCCGGGAGGAGATGACCAAGAACCAGGTCAGC CTGTGGTGCCTGGTCAAAGGCTTCTATCCCAGCGACATCGCCGTGG AGTGGGAGAGCAATGGGCAGCCGGAGAACAACTACAAGACCACG CCTCCCGTGCTGGACTCCGACGGCTCCTTCTTCCTCTACAGCAAGC TCACCGTGGACAAGAGCAGGTGGCAGCAGGGGAACGTCTTCTCAT GCTCCGTGATGCATGAGGCTCTGCACAACCACTACACGCAGAAGA GCCTCTCCCTGTCTCCGGGTAAA | |
| H01Fv3-HC | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAAGGGCTGG AATGGGTGGCCCGTATATATCCCACTAACGGTTACACTCGATATGC TGACTCTGTAAAGGGTCGATTTACTATATCCGCTGATACTAGCAAA AACACTGCCTACCTCCAGATGAACAGTCTGCGCGCCGAGGATACA GCAGTTTATTATTGTTCCCGATGGGGAGGTGACGGGTTCTATGCCA TGGACTACTGGGGTCAAGGGACACTGGTAACCGTTTCTTCTGCTAG CACCAAAGGACCTAGTGTTTGTCCTCTTGCCCCTGAACCAAAGTCT TGTGACAAAACTCACACGTGCCCACCGTGCCCAGCACCTGAACTCC TGGGGGGACCGTCAGTCTTCCTCTTCCCCCCAAAACCCAAGGACAC CCTCATGATCTCCCGGACCCCTGAGGTCACATGCGTGGTGGTGGAC GTGAGCCACGAAGACCCTGAGGTCAAGTTCAACTGGTACGTGGAC GGCGTGGAGGTGCATAATGCCAAGACAAAGCCGCGGGAGGAGCA GTACAACAGCACGTACCGTGTGGTCAGCGTCCTCACCGTCCTGCAC CAGGACTGGCTGAATGGCAAGGAGTACAAGTGCAAGGTCTCCAAC AAAGCCCTCCCAGCCCCCATCGAGAAAACCATCTCCAAAGCCAAA GGGCAGCCCCGAGAACCACAGGTGTACACCCTGCCCCCATGCCGG GAGGAGATGACCAAGAACCAGGTCAGCCTGTGGTGCCTGGTCAAA GGCTTCTATCCCAGCGACATCGCCGTGGAGTGGGAGAGCAATGGG CAGCCGGAGAACAACTACAAGACCACGCCTCCCGTGCTGGACTCC GACGGCTCCTTCTTCCTCTACAGCAAGCTCACCGTGGACAAGAGCA GGTGGCAGCAGGGGAACGTCTTCTCATGCTCCGTGATGCATGAGG CTCTGCACAACCACTACACGCAGAAGAGCCTCTCCCTGTCTCCGGG TAAA | 809 |
| H01Fv3-LC | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACTA | 810 |
| | CACTACTCCTCCAACCTTCGGACAGGGCACAAAAGTAGAGATTAA GCGTACGGTGGCAGCTCCCAGCGTTTTTATCTTTCCCCCATGTGGA GGTGGAAGTGAACCAAAGTCTTGTGACAAAACTCACACGTGCCCA CCGTGCCCAGCACCTGAACTCCTGGGGGGACCGTCAGTCTTCCTCT TCCCCCCAAAACCCAAGGACACCCTCATGATCTCCCGGACCCCTGA GGTCACATGCGTGGTGGTGGACGTGAGCCACGAAGACCCTGAGGT CAAGTTCAACTGGTACGTGGACGGCGTGGAGGTGCATAATGCCAA GACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGGT GTACACCCTGCCCCCATGCCGGGAGGAGATGACCAAGAACCAGGT CAGCCTGTGGTGCCTGGTCAAAGGCTTCTATCCCAGCGACATCGCC GTGGAGTGGGAGAGCAATGGGCAGCCGGAGAACAACTACAAGAC CACGCCTCCCGTGCTGGACTCCGACGGCTCCTTCTTCCTCTACAGC AAGCTCACCGTGGACAAGAGCAGGTGGCAGCAGGGGAACGTCTTC TCATGCTCCGTGATGCATGAGGCTCTGCACAACCACTACACGCAGA AGAGCCTCTCCCTGTCTCCGGGTAAA | |
| H01Fv4-HC | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGGA ATGGGTGGCCCGTATATATCCCACTAACGGTTACACTCGATATGCT GACTCTGTAAAGGGTCGATTTACTATATCCGCTGATACTAGCAAAA ACACTGCCTACCTCCAGATGAACAGTCTGCGCGCCGAGGATACAG CAGTTTATTATTGTTCCCGATGGGGAGGTGACGGGTTCTATGCCAT GGACTACTGGGGTTGTGGGACACTGGTAACCGTTTCTTCTGCTAGC ACCAAAGGACCTAGTGTTTGTCCTCTTGCCCCTGAACCAAAGTCTT GTGACAAAACTCACACGTGCCCACCGTGCCCAGCACCTGAACTCCT GGGGGGACCGTCAGTCTTCCTCTTCCCCCCAAAACCCAAGGACACC CTCATGATCTCCCGGACCCCTGAGGTCACATGCGTGGTGGTGGACG TGAGCCACGAAGACCCTGAGGTCAAGTTCAACTGGTACGTGGACG GCGTGGAGGTGCATAATGCCAAGACAAAGCCGCGGGAGGAGCAG TACAACAGCACGTACCGTGTGGTCAGCGTCCTCACCGTCCTGCACC AGGACTGGCTGAATGGCAAGGAGTACAAGTGCAAGGTCTCCAACA AAGCCCTCCCAGCCCCCATCGAGAAAACCATCTCCAAAGCCAAAG GGCAGCCCCGAGAACCACAGGTGTACACCCTGCCCCCATGCCGGG AGGAGATGACCAAGAACCAGGTCAGCCTGTGGTGCCTGGTCAAAG GCTTCTATCCCAGCGACATCGCCGTGGAGTGGGAGAGCAATGGGC AGCCGGAGAACAACTACAAGACCACGCCTCCCGTGCTGGACTCCG ACGGCTCCTTCTTCCTCTACAGCAAGCTCACCGTGGACAAGAGCAG GTGGCAGCAGGGGAACGTCTTCTCATGCTCCGTGATGCATGAGGCT CTGCACAACCACTACACGCAGAAGAGCCTCTCCCTGTCTCCGGGTA AA | 811 |
| H01Fv4-LC | GACATTCAGATGACCCAATCCCCCTCTAGCCTTTCAGCCTCCGTAG GGGACAGAGTTACAATAACCTGTAGGGCTAGTCAGGACGTTAATA | 812 |
| | CAGCAGTCGCATGGTACCAGCAGAAGCCCGGTAAGTGTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACTA CACTACTCCTCCAACCTTCGGATGTGGCACAAAAGTAGAGATTAA GCGTGAACCAAAGTCTTGTGACAAAACTCACACGTGCCCACCGTG CCCAGCACCTGAACTCCTGGGGGGACCGTCAGTCTTCCTCTTCCCC CCAAAACCCAAGGACACCCTCATGATCTCCCGGACCCCTGAGGTC ACATGCGTGGTGGTGGACGTGAGCCACGAAGACCCTGAGGTCAAG TTCAACTGGTACGTGGACGGCGTGGAGGTGCATAATGCCAAGACA AAGCCGCGGGAGGAGCAGTACAACAGCACGTACCGTGTGGTCAGC GTCCTCACCGTCCTGCACCAGGACTGGCTGAATGGCAAGGAGTAC AAGTGCAAGGTCTCCAACAAAGCCCTCCCAGCCCCCATCGAGAAA ACCATCTCCAAAGCCAAAGGGCAGCCCCGAGAACCACAGGTGTAC ACCCTGCCCCCATGCCGGGAGGAGATGACCAAGAACCAGGTCAGC CTGTGGTGCCTGGTCAAAGGCTTCTATCCCAGCGACATCGCCGTGG AGTGGGAGAGCAATGGGCAGCCGGAGAACAACTACAAGACCACG CCTCCCGTGCTGGACTCCGACGGCTCCTTCTTCCTCTACAGCAAGC TCACCGTGGACAAGAGCAGGTGGCAGCAGGGGAACGTCTTCTCAT GCTCCGTGATGCATGAGGCTCTGCACAACCACTACACGCAGAAGA GCCTCTCCCTGTCTCCGGGTAAA | |
| H01Fv5-HC | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGGA ATGGGTGGCCCGTATATATCCCACTAACGGTTACACTCGATATGCT GACTCTGTAAAGGGTCGATTTACTATATCCGCTGATACTAGCAAAA ACACTGCCTACCTCCAGATGAACAGTCTGCGCGCCGAGGATACAG CAGTTTATTATTGTTCCCGATGGGGAGGTGACGGGTTCTATGCCAT GGACTACTGGGGTCAAGGGACACTGGTAACCGTTTCTTCTGCTAGC ACCAAAGGACCTAGTGTTTGTCCTCTTGCCCCTGAACCAAAGTCTT GTGACAAAACTCACACGTGCCCACCGTGCCCAGCACCTGAACTCCT GGGGGGACCGTCAGTCTTCCTCTTCCCCCCAAAACCCAAGGACACC CTCATGATCTCCCGGACCCCTGAGGTCACATGCGTGGTGGTGGACG TGAGCCACGAAGACCCTGAGGTCAAGTTCAACTGGTACGTGGACG GCGTGGAGGTGCATAATGCCAAGACAAAGCCGCGGGAGGAGCAG TACAACAGCACGTACCGTGTGGTCAGCGTCCTCACCGTCCTGCACC AGGACTGGCTGAATGGCAAGGAGTACAAGTGCAAGGTCTCCAACA AAGCCCTCCCAGCCCCCATCGAGAAAACCATCTCCAAAGCCAAAG GGCAGCCCCGAGAACCACAGGTGTACACCCTGCCCCCATGCCGGG AGGAGATGACCAAGAACCAGGTCAGCCTGTGGTGCCTGGTCAAAG GCTTCTATCCCAGCGACATCGCCGTGGAGTGGGAGAGCAATGGGC AGCCGGAGAACAACTACAAGACCACGCCTCCCGTGCTGGACTCCG ACGGCTCCTTCTTCCTCTACAGCAAGCTCACCGTGGACAAGAGCAG GTGGCAGCAGGGGAACGTCTTCTCATGCTCCGTGATGCATGAGGCT CTGCACAACCACTACACGCAGAAGAGCCTCTCCCTGTCTCCGGGTA | 813 |
| | AA | |
| H01Fv5-LC | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACTA CACTACTCCTCCAACCTTCGGATGTGGCACAAAAGTAGAGATTAA GCGTACGGTGGCAGCTCCCAGCGTTTTTATCTTTCCCCCATGTGGA GGTGGAAGTGAACCAAAGTCTTGTGACAAAACTCACACGTGCCCA CCGTGCCCAGCACCTGAACTCCTGGGGGGACCGTCAGTCTTCCTCT TCCCCCCAAAACCCAAGGACACCCTCATGATCTCCCGGACCCCTGA GGTCACATGCGTGGTGGTGGACGTGAGCCACGAAGACCCTGAGGT CAAGTTCAACTGGTACGTGGACGGCGTGGAGGTGCATAATGCCAA GACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGGT GTACACCCTGCCCCCATGCCGGGAGGAGATGACCAAGAACCAGGT CAGCCTGTGGTGCCTGGTCAAAGGCTTCTATCCCAGCGACATCGCC GTGGAGTGGGAGAGCAATGGGCAGCCGGAGAACAACTACAAGAC CACGCCTCCCGTGCTGGACTCCGACGGCTCCTTCTTCCTCTACAGC AAGCTCACCGTGGACAAGAGCAGGTGGCAGCAGGGGAACGTCTTC TCATGCTCCGTGATGCATGAGGCTCTGCACAACCACTACACGCAGA AGAGCCTCTCCCTGTCTCCGGGTAAA | 814 |
| H01Fv6-HC | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAAGGGCTGG AATGGGTGGCCCGTATATATCCCACTAACGGTTACACTCGATATGC TGACTCTGTAAAGGGTCGATTTACTATATCCGCTGATACTAGCAAA AACACTGCCTACCTCCAGATGAACAGTCTGCGCGCCGAGGATACA GCAGTTTATTATTGTTCCCGATGGGGAGGTGACGGGTTCTATGCCA TGGACTACTGGGGTTGTGGGACACTGGTAACCGTTTCTTCTGCTAG CACCAAAGGACCTAGTGTTTGTCCTCTTGCCCCTGAACCAAAGTCT TGTGACAAAACTCACACGTGCCCACCGTGCCCAGCACCTGAACTCC TGGGGGGACCGTCAGTCTTCCTCTTCCCCCCAAAACCCAAGGACAC CCTCATGATCTCCCGGACCCCTGAGGTCACATGCGTGGTGGTGGAC GTGAGCCACGAAGACCCTGAGGTCAAGTTCAACTGGTACGTGGAC GGCGTGGAGGTGCATAATGCCAAGACAAAGCCGCGGGAGGAGCA GTACAACAGCACGTACCGTGTGGTCAGCGTCCTCACCGTCCTGCAC CAGGACTGGCTGAATGGCAAGGAGTACAAGTGCAAGGTCTCCAAC AAAGCCCTCCCAGCCCCCATCGAGAAAACCATCTCCAAAGCCAAA GGGCAGCCCCGAGAACCACAGGTGTACACCCTGCCCCCATGCCGG GAGGAGATGACCAAGAACCAGGTCAGCCTGTGGTGCCTGGTCAAA GGCTTCTATCCCAGCGACATCGCCGTGGAGTGGGAGAGCAATGGG | 815 |
| | CAGCCGGAGAACAACTACAAGACCACGCCTCCCGTGCTGGACTCC GACGGCTCCTTCTTCCTCTACAGCAAGCTCACCGTGGACAAGAGCA GGTGGCAGCAGGGGAACGTCTTCTCATGCTCCGTGATGCATGAGG CTCTGCACAACCACTACACGCAGAAGAGCCTCTCCCTGTCTCCGGG TAAA | |
| H01Fv6-LC | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGTGTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACTA CACTACTCCTCCAACCTTCGGACAGGGCACAAAAGTAGAGATTAA GCGTACGGTGGCAGCTCCCAGCGTTTTTATCTTTCCCCCATGTGGA GGTGGAAGTGAACCAAAGTCTTGTGACAAAACTCACACGTGCCCA CCGTGCCCAGCACCTGAACTCCTGGGGGGACCGTCAGTCTTCCTCT TCCCCCCAAAACCCAAGGACACCCTCATGATCTCCCGGACCCCTGA GGTCACATGCGTGGTGGTGGACGTGAGCCACGAAGACCCTGAGGT CAAGTTCAACTGGTACGTGGACGGCGTGGAGGTGCATAATGCCAA GACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGGT GTACACCCTGCCCCCATGCCGGGAGGAGATGACCAAGAACCAGGT CAGCCTGTGGTGCCTGGTCAAAGGCTTCTATCCCAGCGACATCGCC GTGGAGTGGGAGAGCAATGGGCAGCCGGAGAACAACTACAAGAC CACGCCTCCCGTGCTGGACTCCGACGGCTCCTTCTTCCTCTACAGC AAGCTCACCGTGGACAAGAGCAGGTGGCAGCAGGGGAACGTCTTC TCATGCTCCGTGATGCATGAGGCTCTGCACAACCACTACACGCAGA AGAGCCTCTCCCTGTCTCCGGGTAAA | 816 |
| H01Fv7-HC | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGGA ATGGGTGGCCCGTATATATCCCACTAACGGTTACACTCGATATGCT GACTCTGTAAAGGGTCGATTTACTATATCCGCTGATACTAGCAAAA ACACTGCCTACCTCCAGATGAACAGTCTGCGCGCCGAGGATACAG CAGTTTATTATTGTTCCCGATGGGGAGGTGACGGGTTCTATGCCAT GGACTACTGGGGTTGTGGGACACTGGTAACCGTTTCTTCTGCTAGC ACCAAAGGACCTAGTGTTTGTCCTCTTGCCCCTGAACCAAAGTCTT GTGACAAAACTCACACGTGCCCACCGTGCCCAGCACCTGAACTCCT GGGGGGACCGTCAGTCTTCCTCTTCCCCCCAAAACCCAAGGACACC CTCATGATCTCCCGGACCCCTGAGGTCACATGCGTGGTGGTGGACG TGAGCCACGAAGACCCTGAGGTCAAGTTCAACTGGTACGTGGACG GCGTGGAGGTGCATAATGCCAAGACAAAGCCGCGGGAGGAGCAG TACAACAGCACGTACCGTGTGGTCAGCGTCCTCACCGTCCTGCACC AGGACTGGCTGAATGGCAAGGAGTACAAGTGCAAGGTCTCCAACA | 817 |
| | AAGCCCTCCCAGCCCCCATCGAGAAAACCATCTCCAAAGCCAAAG GGCAGCCCCGAGAACCACAGGTGTACACCCTGCCCCCATGCCGGG AGGAGATGACCAAGAACCAGGTCAGCCTGTGGTGCCTGGTCAAAG GCTTCTATCCCAGCGACATCGCCGTGGAGTGGGAGAGCAATGGGC AGCCGGAGAACAACTACAAGACCACGCCTCCCGTGCTGGACTCCG ACGGCTCCTTCTTCCTCTACAGCAAGCTCACCGTGGACAAGAGCAG GTGGCAGCAGGGGAACGTCTTCTCATGCTCCGTGATGCATGAGGCT CTGCACAACCACTACACGCAGAAGAGCCTCTCCCTGTCTCCGGGTA AA | |
| H01Fv7-LC | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGTGTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACTA CACTACTCCTCCAACCTTCGGATGTGGCACAAAAGTAGAGATTAA GCGTACGGTGGCAGCTCCCAGCGTTTTTATCTTTCCCCCATGTGGA GGTGGAAGTGAACCAAAGTCTTGTGACAAAACTCACACGTGCCCA CCGTGCCCAGCACCTGAACTCCTGGGGGGACCGTCAGTCTTCCTCT TCCCCCCAAAACCCAAGGACACCCTCATGATCTCCCGGACCCCTGA GGTCACATGCGTGGTGGTGGACGTGAGCCACGAAGACCCTGAGGT CAAGTTCAACTGGTACGTGGACGGCGTGGAGGTGCATAATGCCAA GACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGGT GTACACCCTGCCCCCATGCCGGGAGGAGATGACCAAGAACCAGGT CAGCCTGTGGTGCCTGGTCAAAGGCTTCTATCCCAGCGACATCGCC GTGGAGTGGGAGAGCAATGGGCAGCCGGAGAACAACTACAAGAC CACGCCTCCCGTGCTGGACTCCGACGGCTCCTTCTTCCTCTACAGC AAGCTCACCGTGGACAAGAGCAGGTGGCAGCAGGGGAACGTCTTC TCATGCTCCGTGATGCATGAGGCTCTGCACAACCACTACACGCAGA AGAGCCTCTCCCTGTCTCCGGGTAAA | 818 |

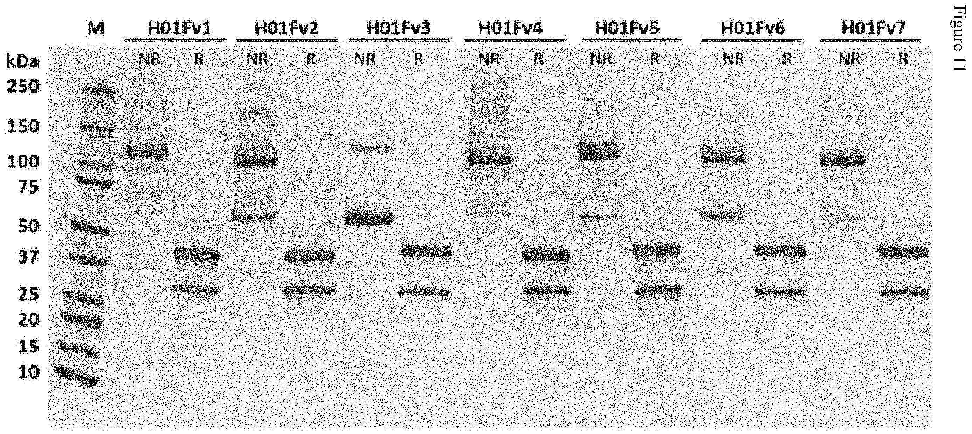

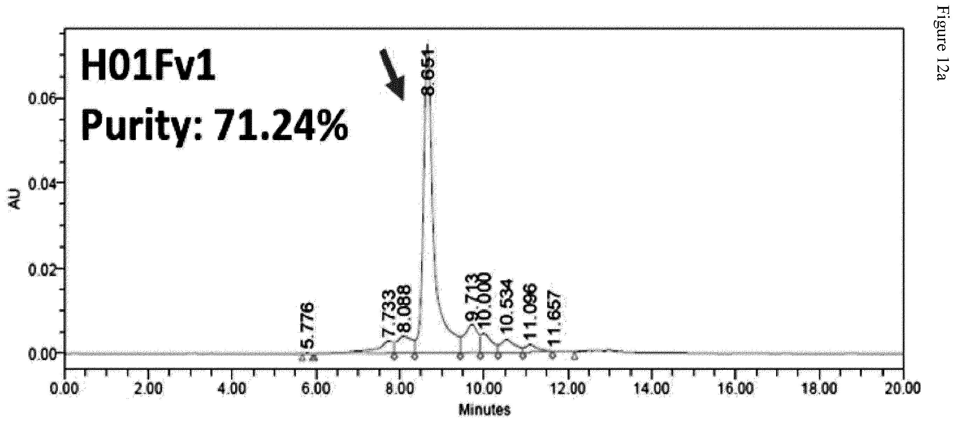

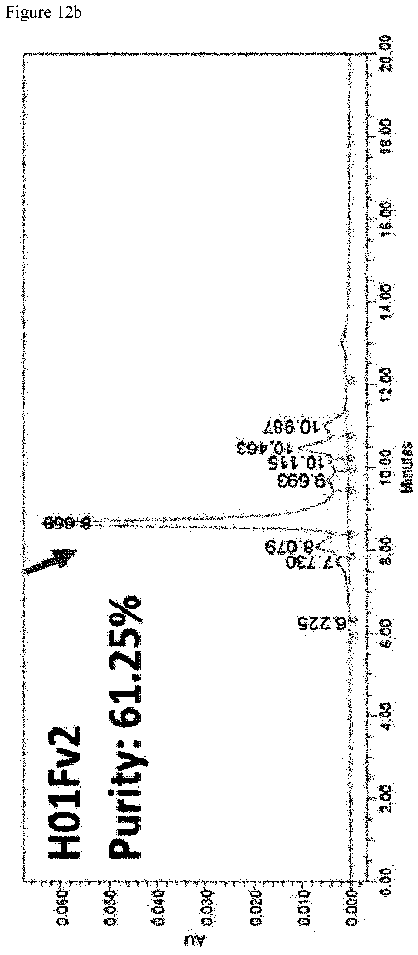

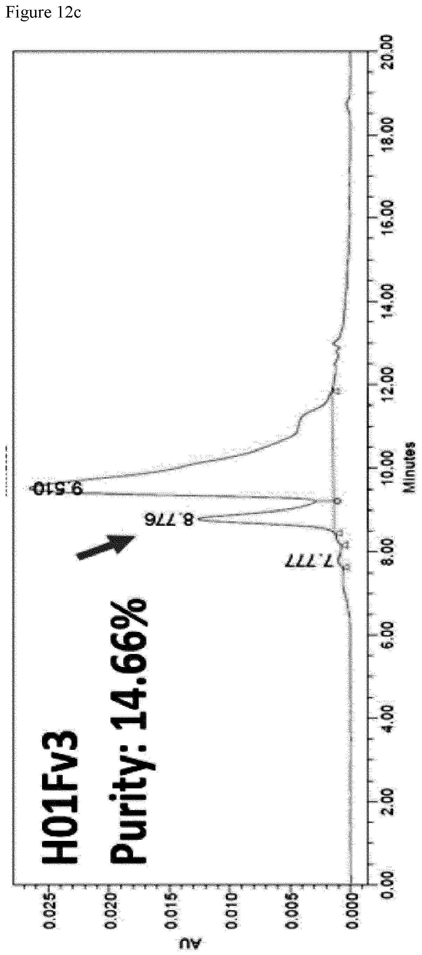

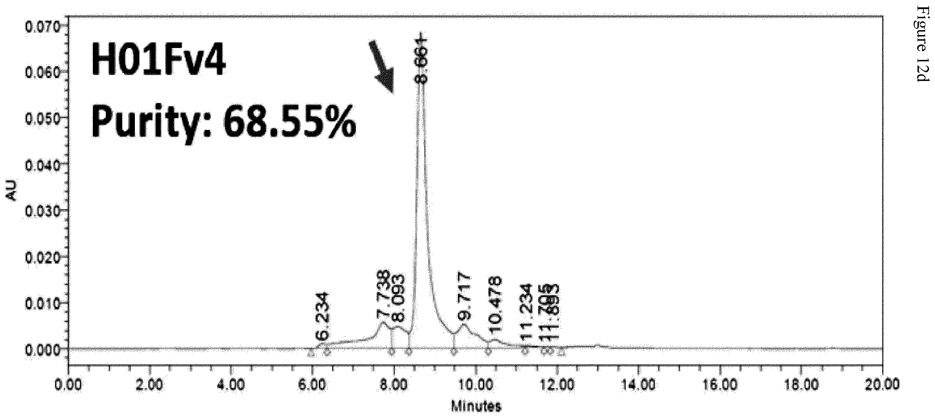

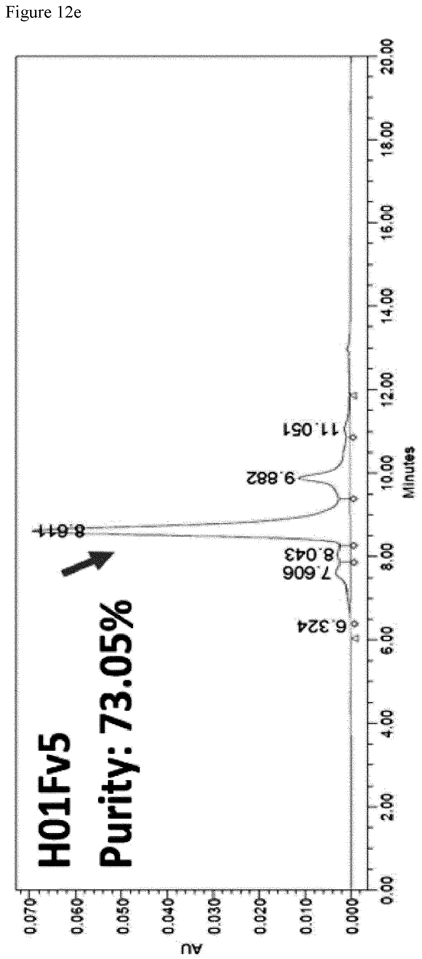

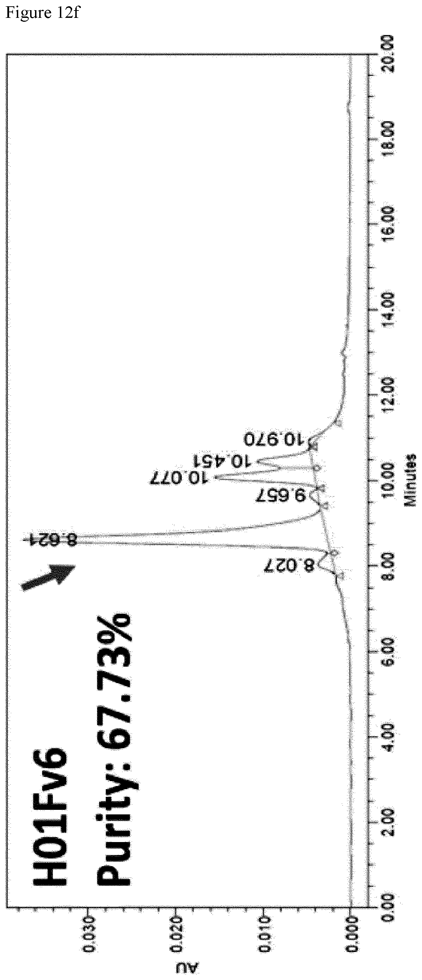

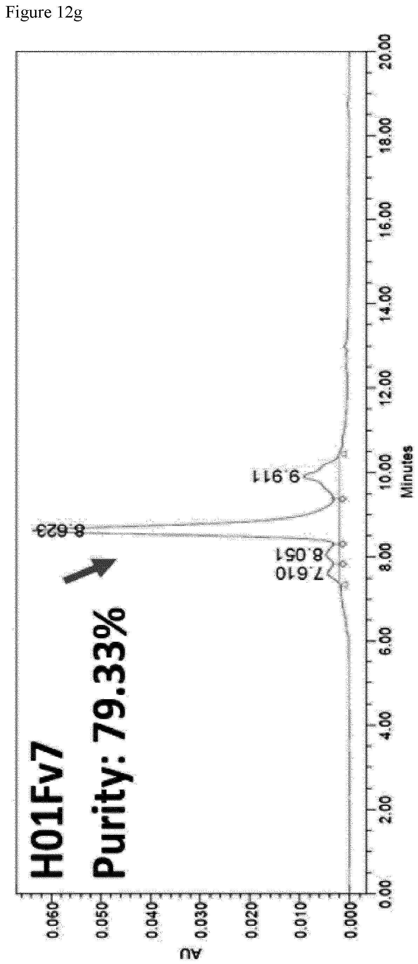

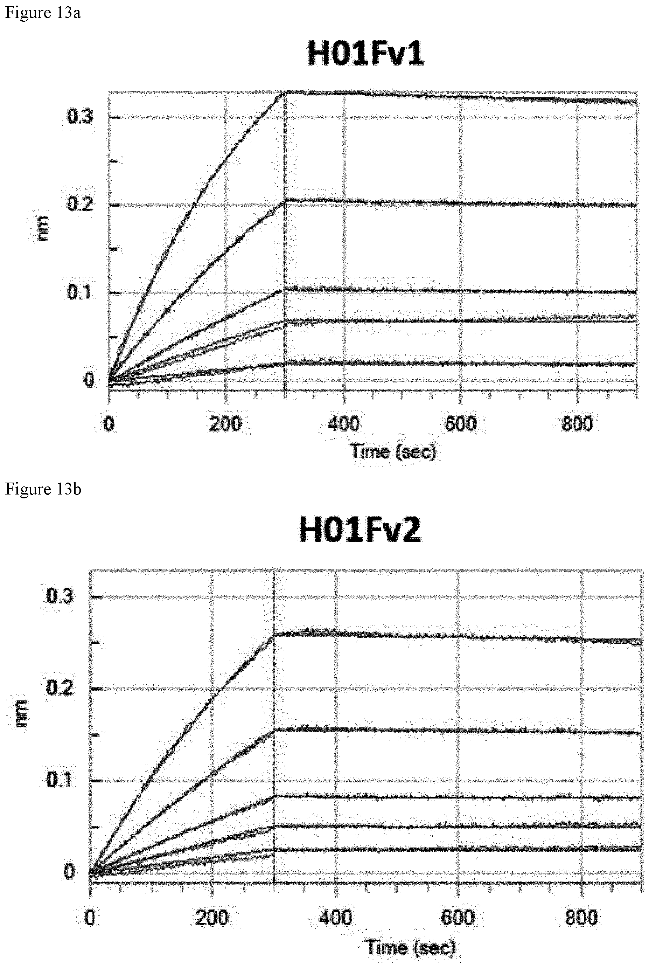

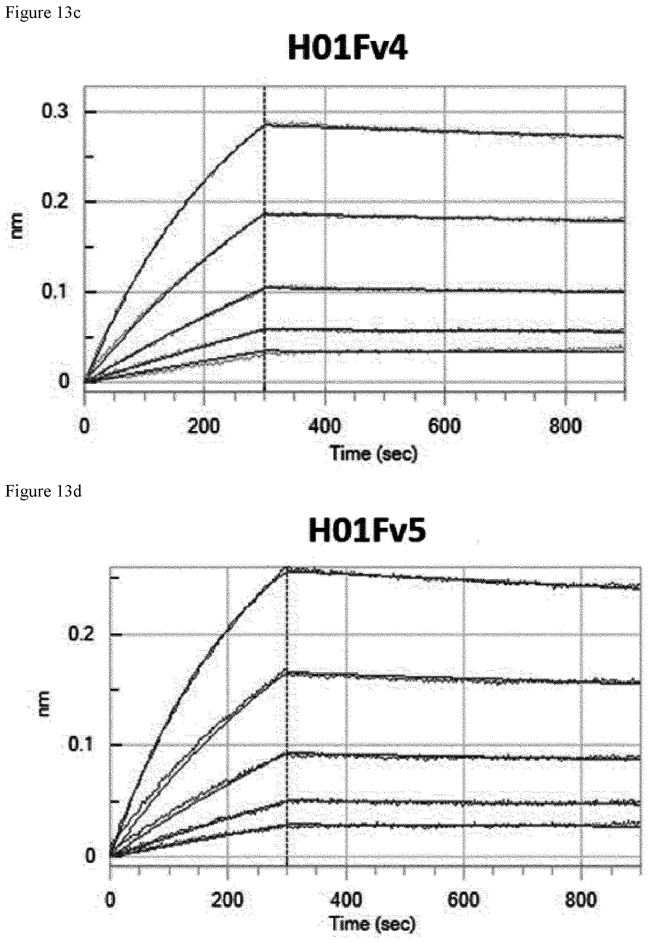

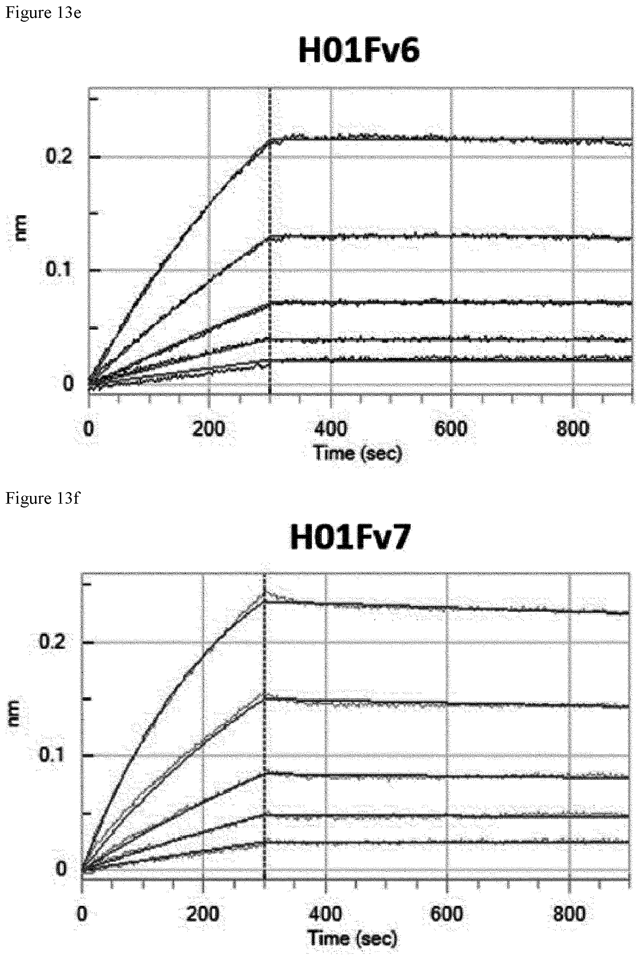

When expression vectors containing the sequences corresponding to Fc-Hole-RF (SEQ ID NO: 789), H01Fv1-HC (SEQ ID NO: 790), and H01Fv1-LC (SEQ ID NO: 791) were co-transfect into EXPICHO-S^{™} (Gibco, A29127), H01Fv1 was formed (Figure 10, Tables 20 and 21). Thereafter, it was purified through affinity chromatography using MABSELECT^{™} PrismA (Cytiva, 17549853). The Fc-Hole polypeptide (SEQ ID NO: 7) can form an Fc-Hole/Fc-Hole dimer. In order to remove this Fc-Hole/Fc-Hole dimer, H435R and Y436F mutations were introduced in the Fc-Hole polypeptide sequence (SEQ ID NO: 7) to produce the Fc-Hole-RF polypeptide (SEQ ID NO: 789) (Figure 10, Tables 20 and 21). This prevents the Fc-Hole/Fc-Hole dimer from binding to Protein A resin and removes the mispaired Fc-Hole/Fc-Hole dimer (Figures 10 and 11). SDS-PAGE analysis identified the main product at about 130 kDa under non-reducing conditions (NR) (Figure 11), and monomer purity was determined by SEC (Figure 12). H01Fv3 exists mostly in an unpaired form of about 65 kDa, and the monomer purity of fully assembled form was determined to be 14.66% (Figures 11 and 12c). It was found that H01Fv1, H01Fv2, H01Fv4, H01Fv5, H01Fv6, and H01Fv7 have monomer purities of 71.24%, 61.25%, 68.55%, 73.05%, 67.73%, and 79.33%, respectively (Figure 12). The binding characteristics of H01Fv1, H01Fv2, H01Fv4, H01Fv5, H01Fv6, and H01Fv7 were analyzed using Octet Red96e (Sartorius), a bio-layer interferometry (BLI) (Figure 13). The human HER2 recombinant protein (R&D systems, 1129-ER) was loaded onto the Anti-Penta-HIS (HIS1K) biosensor (Sartorius, 18-5120), and then the binding constants of H01Fv1, H01Fv2, H01Fv4, H01Fv5, H01Fv6, and H01Fv7 were calculated (Figure 13, Table 22).

**[Table 22]**

| Clone | KD(M) | kon(1/Ms) | kdis(1/s) |
|---|---|---|---|
| H01Fv1 | 2.44E-10 | 2.10E+05 | 5.11E-05 |
| H01Fv2 | 3.24E-10 | 1.17E+05 | 3.78E-05 |
| H01Fv3 | Not Determined | Not Determined | Not Determined |
| H01Fv4 | 3.26E-10 | 2.37E+05 | 7.71E-05 |
| H01Fv5 | 3.93E-10 | 2.73E+05 | 1.07E-04 |
| H01Fv6 | 4.87E-11 | 1.36E+05 | 6.61E-06 |
| H01Fv7 | 2.79E-10 | 2.76E+05 | 7.70E-05 |

### Example 6. Analysis of thermal stability

Analysis of thermal stability was performed using the PROTEIN THERMAL SHIFT^{™} Dye Kit (Applied biosystems, 4461146) according to the manufacturer's manual. Briefly, 5 µL of reaction buffer and 2.5 µL of dye included in the kit were mixed with 5 µg of trastuzumab, pertuzumab, H01, or P01, and the final volume was adjusted to 20 µL using PBS.

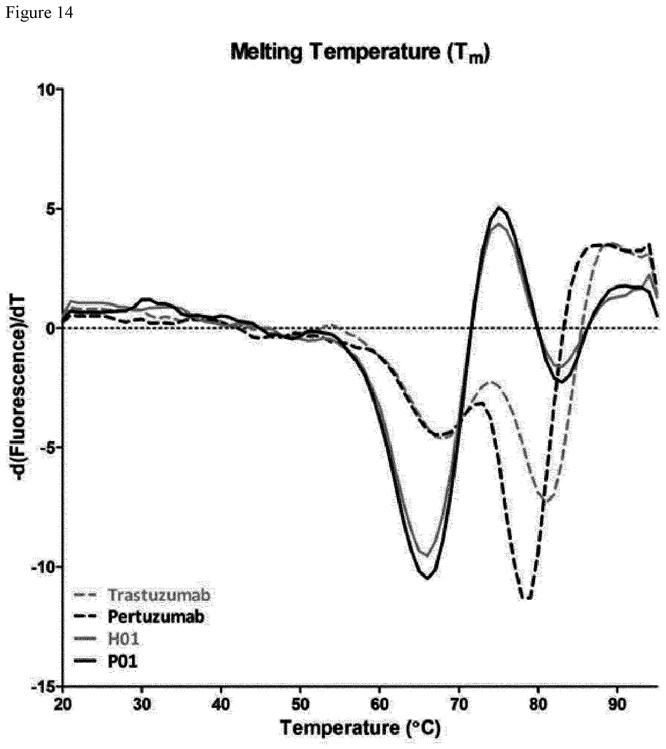

The mixture was incubated at 20°C for 30 seconds in a C1000 thermal cycler (Bio-Rad, 1841000) equipped with a CFX96 optical reaction module (Bio-Rad, 1845096), and the fluorescence intensity of the plate was measured while increasing the temperature from 20°C to 95 °C at 1 °C/min, and the reaction was stopped after incubation at 95 °C for 30 seconds. After the reaction, the median value of relative fluorescence unit (RFU) values was taken, and analysis of melting temperature (Tₘ) was performed. The Tₘ₁ values were found to be 68, 68, 66, and 66°C and the Tₘ₂ values were found to be 81, 79, 83, and 83 °C for trastuzumab, pertuzumab, H01, and P01, respectively, indicating that H01 and P01 have Tₘ values similar to those of commercialized therapeutic antibodies (Figure 14, Table 23).

**[Table 23]**

| Antibody | Tₘ₁ | Tₘ₂ |
|---|---|---|
| Trastuzumab | 68 | 81 |
| Pertuzumab | 68 | 79 |
| H01 | 66 | 83 |
| P01 | 66 | 83 |

### Example 7. Identification of competitive binding of H01 and P01

In order to identify whether H01 and P01 bind to different epitopes or compete for binding, Octet Red96e (Sartorius), a bio-layer interferometry (BLI), was used.

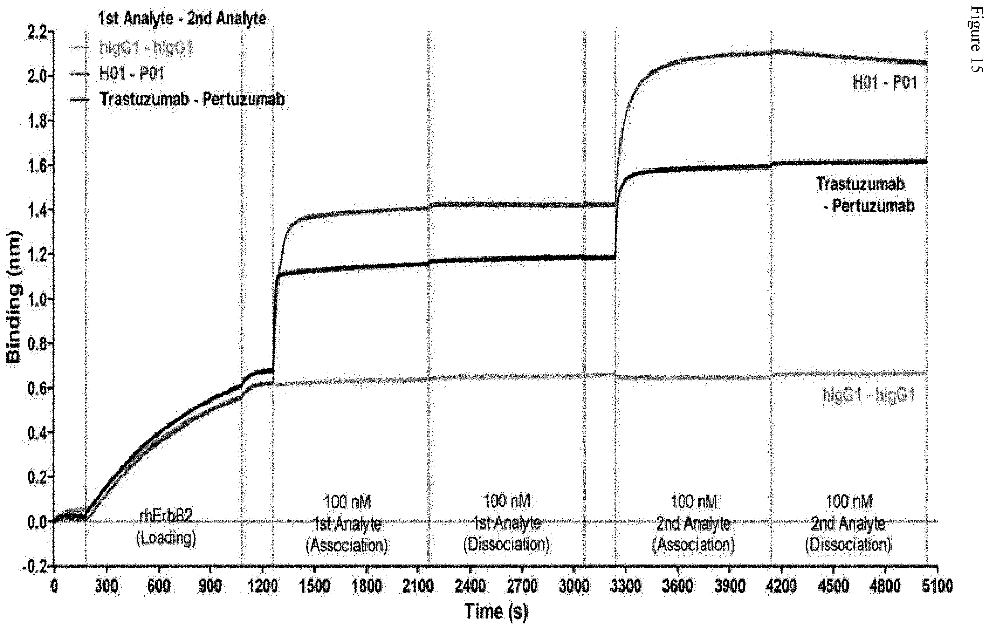

The human HER2 recombinant protein (R&D systems, 1129-ER) was loaded onto the Anti-Penta-HIS (HIS1K) biosensor (Sartorius, 18-5120). 100 nM human IgG1 (Bio X cell, BE0297) or 100 nM H01 or 100 nM trastuzumab was first bound to each biosensor loaded with HER2 antigen, followed by 100 nM human IgG1, 100 nM P01, or 100 nM pertuzumab to determine whether they bind competitively (Figure 15). The binding signals (nm shift from baseline) measured at equilibrium after completion of Her2 recombinant protein loading were 0.620, 0.625, and 0.672 nm, respectively (Figure 15, Table 24).

The first and second analytes were sequentially bound with binding time and dissociation time of 900 seconds. When the human IgG1 antibody was sequentially bound, it did not bind to HER2 (Figure 15, Table 24). Sequential binding of H01 and P01 or sequential binding of trastuzumab and pertuzumab was observed, indicating that they bind to different epitopes (Figure 15, Table 24). The binding signals (nm shift from baseline) of H01 + P01 on the HER2-loaded sensor and the binding signals (nm shift from baseline) of trastuzumab + pertuzumab were measured to be 1.477 nm (= y-axis value at 4140 s - y-axis value at 1260 s), 0.923 nm (= y-axis value at 4140 s - y-axis value at 1260 s), respectively. The binding signals (nm shift from baseline) tend to increase as more proteins bind to the surface of the biosensor. Therefore, this indicates that to the same amount of HER2, H01 and P01 cause a greater amount of antibody binding than trastuzumab and pertuzumab (Figure 15, Table 24).

**[Table 24]**

| 1^{st} analyte - 2^{nd} analyte | Baseline(0 s) | HER2 Loading (1260 s) | 1^{st} Analyte binding(2160 s) | 2^{nd} Analyte binding(4140 s) |
|---|---|---|---|---|
| hIgG1 - hIgG1 | 0 | 0.620 | 0.635 | 0.647 |
| H01- P01 | 0 | 0.625 | 1.407 | 2.102 |
| Trastuzumab - Pertuzumab | 0 | 0.679 | 1.157 | 1.595 |

### Example 8. Quantification of Fc loads

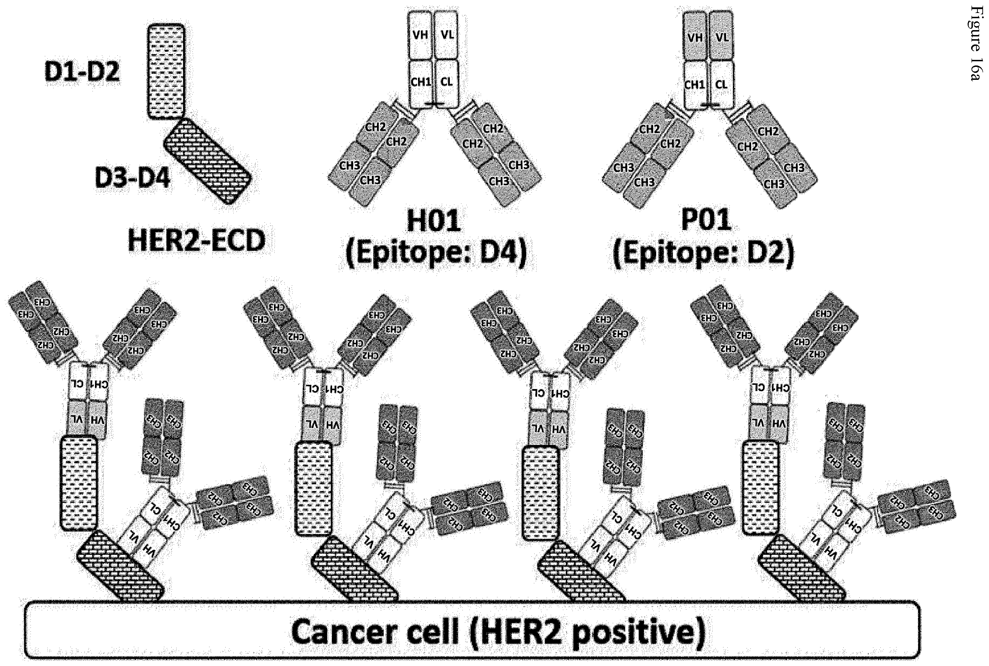

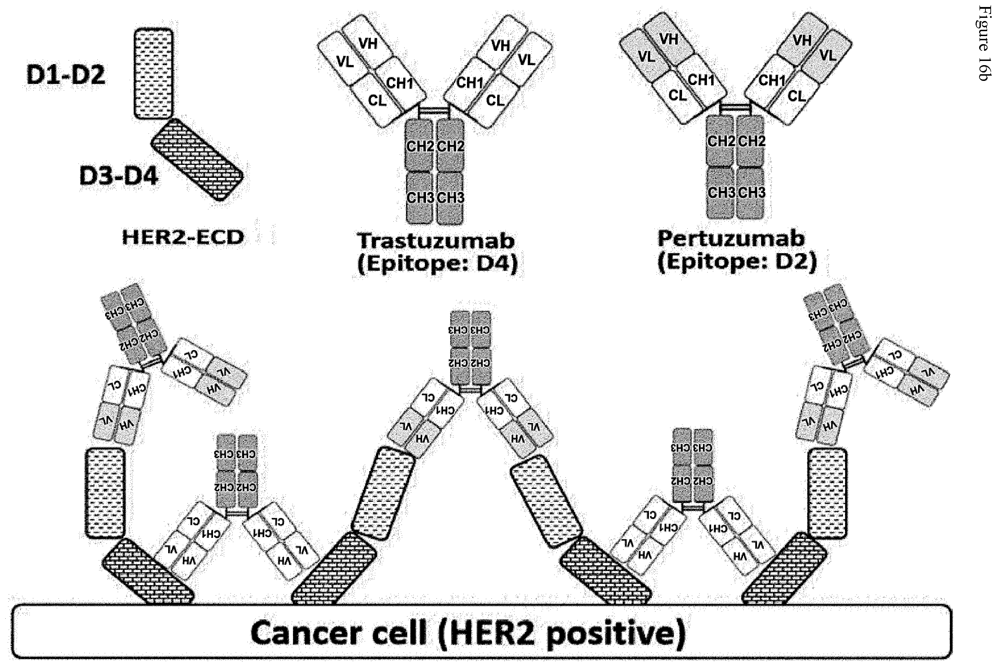

When H01 and P01 are treated in combination, a total of 16 Fc domains bind to four HER2 antigens present on the surface of cancer cells (Figure 16a). When trastuzumab and pertuzumab are treated in combination, eight Fc domains bind to four HER2 antigens present on the surface of cancer cells if the binding is in monovalent mode, and fewer Fc domains can be bound if the binding is in bivalent mode (Figure 16b). The combination of H01 and P01 should result in increased Fc loads on the surface of HER2-positive cancer cells than the combination of trastuzumab and pertuzumab and thereby should lead to a stronger effector function.

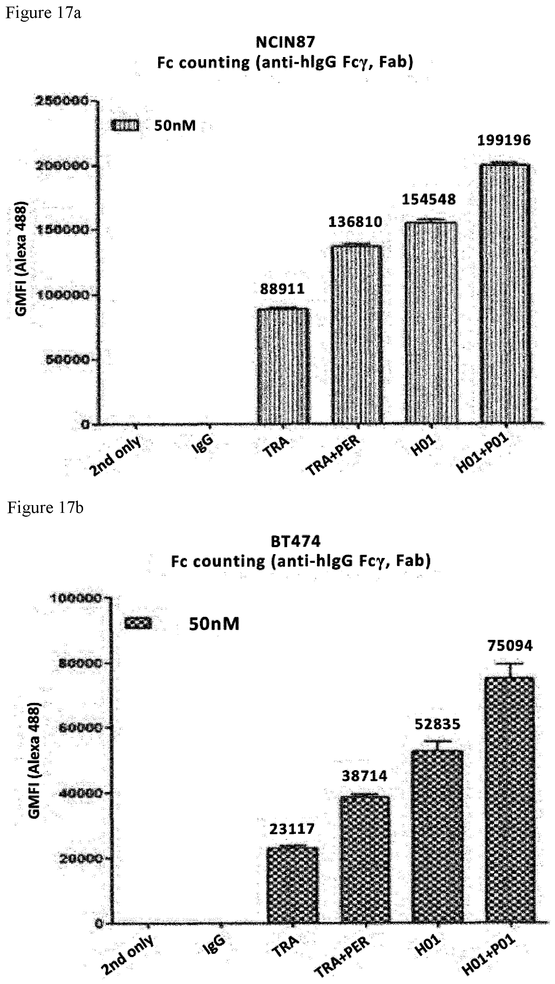

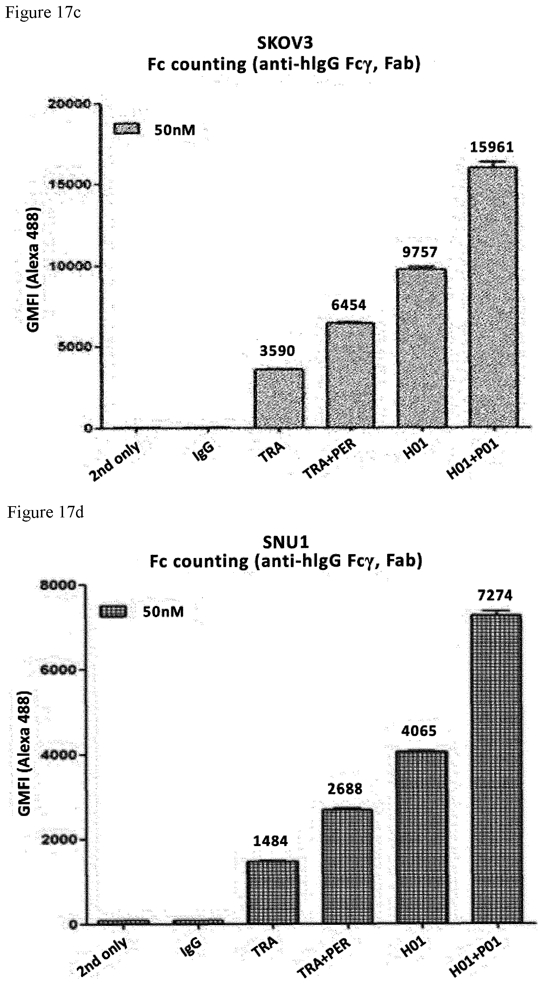

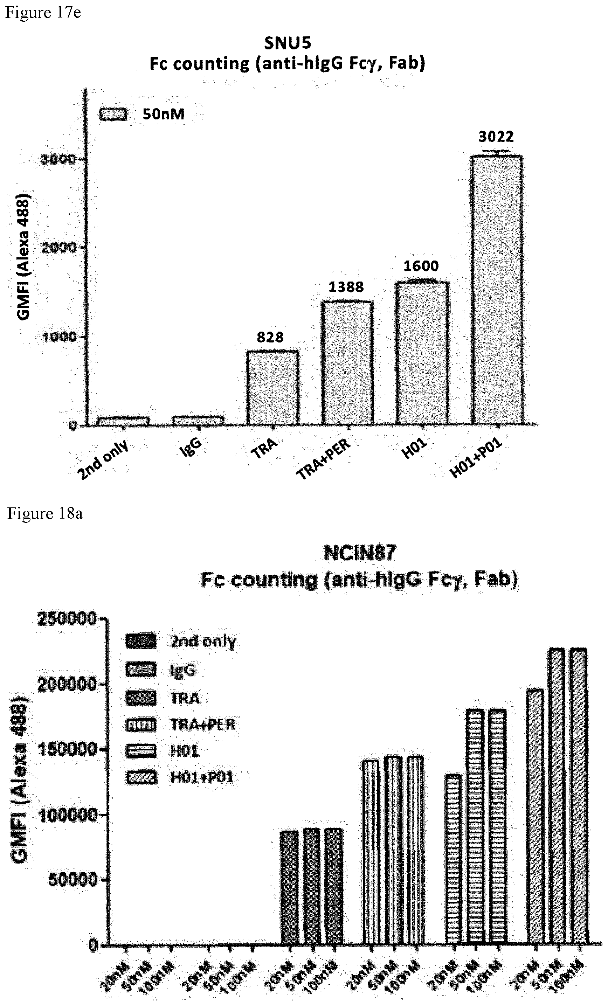

NCI-N87, BT474, SK-OV-3, SNU1, and SNU5 cancer cell lines used to quantify Fc loads of the antibodies on the surface of HER2-expressing cells were cultured in RPMI-1640 + 10% FBS medium. The cancer cell lines were treated with 50 nM human IgG1 (Bio X cell, BE0297), 50 nM trastuzumab (TRA), or 50 nM trastuzumab + 50 nM pertuzumab (TRA + PER), 50 nM H01, 50 nM H01 + 50 nM P01 antibody at 4°C for 30 minutes in a 96-well plate. Thereafter, they were treated with the Alexa 488 fluorescence-conjugated anti-human IgG Fcγ Fab antibody (Jackson ImmunoResearch, 109-547-008), and the antibody Fc bound to the cells was quantified using a flow cytometer (BD biosciences, FACSverse) (Figures 17a to 17e, Table 25). It was found that the fluorescence intensities of the 50 nM H01 alone group were higher than those of the 50 nM trastuzumab + 50 nM pertuzumab (TRA + PER) combination group in the five cancer cell lines analyzed (Figures 17a to 17e, Table 25). Compared to treatment of 50 nM H01 alone, an additional increase in the Fc loads on the cancer cell surface was observed in the NCI-N87, BT474, SK-OV-3, SNU1, and SNU5 cancer cells when 50 nM P01 was treated in combination with 50 nM H01 (Figures 17a to 17e, Table 25).

**[Table 25]**

| GMFI | NCI-N87 | BT474 | SK-OV-3 | SNU-1 | SNU-5 |
|---|---|---|---|---|---|
| 50 nM Trastuzumab | 88911 | 23117 | 3590 | 1484 | 828 |
| 50 nM Trastuzumab + 50 nM Pertuzumab | 136810 | 38714 | 6454 | 2688 | 1388 |
| 50 nM H01 | 154548 | 52835 | 9757 | 4065 | 1600 |
| 50 nM H01 + 50 nM P01 | 199196 | 75094 | 15961 | 7274 | 3022 |

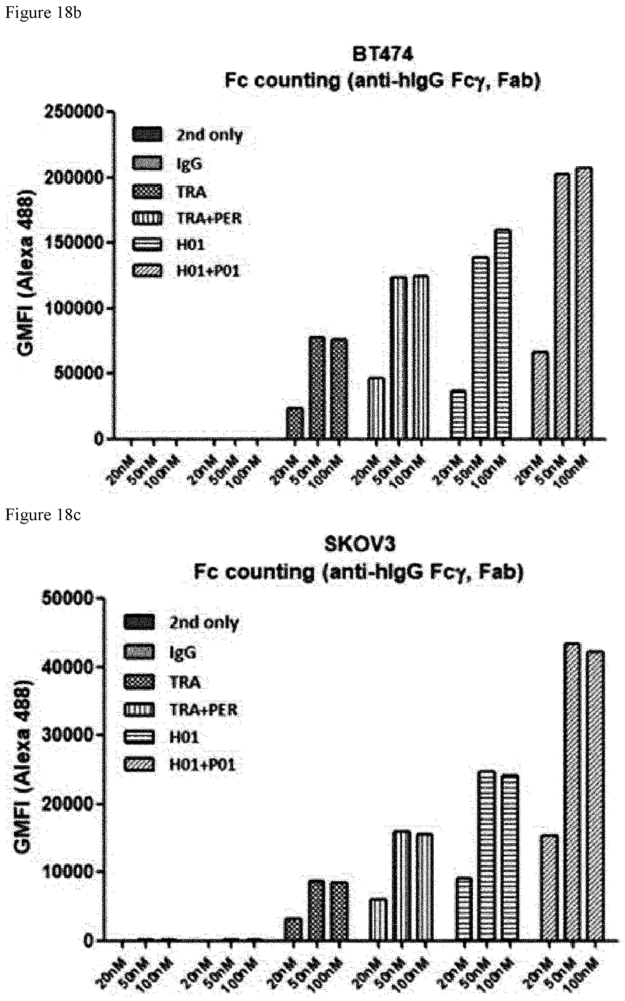

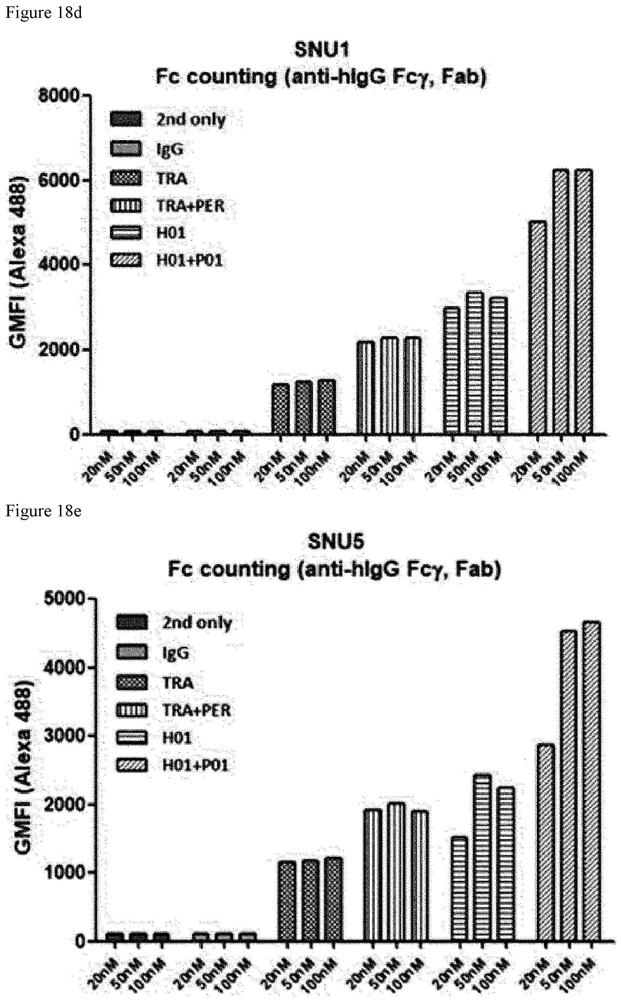

In order to determine the saturation concentration of antibodies binding to the cell surface, each test antibody was allowed to bind to a final concentration of 20, 50, and 100 nM, and the subsequent sampling process was carried out in the same manner as the above experimental conditions (Figures 18a to 18e). It was found that the saturation concentration of the antibody in the NCI-N87, BT474, SK-OV-3, SNU1, and SNU5 cancer cell lines was 50 nM (Figures 18a to 18e). It was found that treatment of 50 nM H01 alone results in more Fc loads on the surface of the five cell lines than treatment of 50 nM trastuzumab and 50 nM pertuzumab in combination (Figures 18a to 18e).

### Example 9. Analysis of antibody binding affinity to HER2

The S239D and I332E mutations in the antibody Fc domain improve the affinity of the antibody for Fcy receptors, which leads to improved effector function (Greg A. Lazar et al., PNAS, 2006). H01DE4 and P01DE4 were designed by introducing the S239D and I332E mutations in the H01 and P01 Fc domains (Figures 19a and 19b). H01DE4 was prepared by co-transfection of vectors capable of expressing the polypeptides corresponding to Fc-HoleS239D-I332E (Table 26, SEQ ID NO: 42), TraH-G44C-Knob-S239D-I332E (Table 26, SEQ ID NO: 43), and TraL-Q100C-Knob-S239D-I332E (Table 26, SEQ ID NO: 44) into EXPICHO-S^{™} (Gibco, A29127). P01DE4 was prepared by co-transfection of vectors capable of expressing the polypeptides corresponding to Fc-Hole-S239D-I332E (Table 26, Table 27, and SEQ ID NO: 42), PerH-G44C-Knob-S239D-I332E (Table 26, Table 27, and SEQ ID NO: 45), and PerL-Q100C-Knob-S239D-I332E (Table 26, Table 27, and SEQ ID NO: 46) into EXPICHO-S^{™} (Gibco, A29127).

**[Table 26]**

| Name | Sequence | SEQ ID NO |
|---|---|---|
| Fc-Hole-S239D-I332E | DKTHTCPPCPAPELLGGPDVFLFPPKPKDTLMISRTPEVTCVVVDVSH EDPEVKFNWYVDGVEVHNAKTKPREEQYNSTYRVVSVLTVLHQDW LNGKEYKCKVSNKALPAPEEKTISKAKGQPREPQVCTLPPSREEMTK NQVSLSCAVKGFYPSDIAVEWESNGQPENNYKTTPPVLDSDGSFFLV SKLTVDKSRWQQGNVFSCSVMHEALHNHYTQKSLSLSPGK | 42 |
| TraH-G44C-KnobS239D-I332E | EVQLVESGGGLVQPGGSLRLSCAASGFNIKDTYIHWVRQAPGKCLE WVARIYPTNGYTRYADSVKGRFTISADTSKNTAYLQMNSLRAEDTA VYYCSRWGGDGFYAMDYWGQGTLVTVSSASTKGPSVFPLAPSSKST SGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCP APELLGGPDVFLFPPKPKDTLMISRTPEVTCVVVDVSHEDPEVKFNW YVDGVEVHNAKTKPREEQYNSTYRVVSVLTVLHQDWLNGKEYKCK VSNKALPAPEEKTISKAKGQPREPQVYTLPPCREEMTKNQVSLWCLV KGFYPSDIAVEWESNGQPENNYKTTPPVLDSDGSFFLYSKLTVDKSR WQQGNVFSCSVMHEALHNHYTQKSLSLSPGK | 43 |
| TraL-Q100C-KnobS239D-I332E | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGCGTKVEIKRTVAAPSVFIFPPSDEQLKSGTASVVCLLNNFYPRE AKVQWKVDNALQSGNSQESVTEQDSKDSTYSLSSTLTLSKADYEKH KVYACEVTHQGLSSPVTKSFNRGECGGGGSGGGGSGGGGSEPKSSD KTHTCPPCPAPELLGGPDVFLFPPKPKDTLMISRTPEVTCVVVDVSHE DPEVKFNWYVDGVEVHNAKTKPREEQYNSTYRVVSVLTVLHQDWL NGKEYKCKVSNKALPAPEEKTISKAKGQPREPQVYTLPPCREEMTKN QVSLWCLVKGFYPSDIAVEWESNGQPENNYKTTPPVLDSDGSFFLYS KLTVDKSRWQQGNVFSCSVMHEALHNHYTQKSLSLSPGK | 44 |
| PerH-G44C-KnobS239D-I332E | EVQLVESGGGLVQPGGSLRLSCAASGFTFTDYTMDWVRQAPGKCLE WVADVNPNSGGSIYNQRFKGRFTLSVDRSKNTLYLQMNSLRAEDTA VYYCARNLGPSFYFDYWGQGTLVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDKTHTCPPCPA PELLGGPDVFLFPPKPKDTLMISRTPEVTCVVVDVSHEDPEVKFNWY | 45 |
| | VDGVEVHNAKTKPREEQYNSTYRVVSVLTVLHQDWLNGKEYKCKV SNKALPAPEEKTISKAKGQPREPQVYTLPPCREEMTKNQVSLWCLVK GFYPSDIAVEWESNGQPENNYKTTPPVLDSDGSFFLYSKLTVDKSRW QQGNVFSCSVMHEALHNHYTQKSLSLSPGK | |
| PerL-Q100C-KnobS239D-I332E | DIQMTQSPSSLSASVGDRVTITCKASQDVSIGVAWYQQKPGKAPKLLI YSASYRYTGVPSRFSGSGSGTDFTLTISSLQPEDFATYYCQQYYIYPY TFGCGTKVEIKRTVAAPSVFIFPPSDEQLKSGTASVVCLLNNFYPREA KVQWKVDNALQSGNSQESVTEQDSKDSTYSLSSTLTLSKADYEKHK VYACEVTHQGLSSPVTKSFNRGECGGGGSGGGGSGGGGSEPKSSDK THTCPPCPAPELLGGPDVFLFPPKPKDTLMISRTPEVTCVVVDVSHED PEVKFNWYVDGVEVHNAKTKPREEQYNSTYRVVSVLTVLHQDWLN GKEYKCKVSNKALPAPEEKTISKAKGQPREPQVYTLPPCREEMTKNQ VSLWCLVKGFYPSDIAVEWESNGQPENNYKTTPPVLDSDGSFFLYSK LTVDKSRWQQGNVFSCSVMHEALHNHYTQKSLSLSPGK | 46 |

**[Table 27]**

| Name | Sequence | SEQ ID NO |
|---|---|---|
| Fc-Hole-S239D-I332E | GACAAAACTCACACATGCCCACCGTGCCCAGCACCTGAACTCCTG GGGGGACCGGATGTCTTCCTCTTCCCCCCAAAACCCAAGGACACC CTCATGATCTCCCGGACCCCTGAGGTCACATGCGTGGTGGTGGAC GTGAGCCACGAAGACCCTGAGGTCAAGTTCAACTGGTACGTGGAC GGCGTGGAGGTGCATAATGCCAAGACAAAGCCGCGGGAGGAGCA GTACAACAGCACGTACCGTGTGGTCAGCGTCCTCACCGTCCTGCA CCAGGACTGGCTGAATGGCAAGGAGTACAAGTGCAAGGTCTCCA ACAAAGCCCTCCCAGCCCCCGAAGAGAAAACCATCTCCAAAGCC AAAGGGCAGCCCCGAGAACCACAGGTGTGCACCCTGCCCCCATCC CGGGAGGAGATGACCAAGAACCAGGTCAGCCTGAGCTGCGCGGT CAAAGGCTTCTATCCCAGCGACATCGCCGTGGAGTGGGAGAGCAA TGGGCAGCCGGAGAACAACTACAAGACCACGCCTCCCGTGCTGG ACTCCGACGGCTCCTTCTTCCTCGTGAGCAAGCTCACCGTGGACA AGAGCAGGTGGCAGCAGGGGAACGTCTTCTCATGCTCCGTGATGC ATGAGGCTCTGCACAACCACTACACGCAGAAGAGCCTCTCCCTGT CTCCGGGTAAA | 479 |
| TraH-G44C-KnobS239D-I332E | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGG AATGGGTGGCCCGTATATATCCCACTAACGGTTACACTCGATATG CTGACTCTGTAAAGGGTCGATTTACTATATCCGCTGATACTAGCAA AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACCAAAGGACCTAGTGTTTTTCCTCTTGCCCCTTCCTCAAAGT CTACCTCTGGGGGGACAGCCGCTCTGGGCTGCCTGGTCAAGGATT ATTTCCCAGAGCCTGTCACTGTCAGTTGGAACTCTGGAGCCTTGAC TTCTGGTGTTCATACATTTCCTGCTGTCCTTCAAAGCAGCGGCTTG TACTCATTGTCTTCTGTTGTGACAGTACCCTCAAGCAGCCTCGGCA | 480 |
| | CTCAGACATACATCTGCAATGTCAACCACAAACCCTCAAATACAA AGGTAGATAAAAAAGTCGAACCAAAGTCTTGTGACAAAACTCAC ACGTGCCCACCGTGCCCAGCACCTGAACTCCTGGGGGGACCGGAT GTCTTCCTCTTCCCCCCAAAACCCAAGGACACCCTCATGATCTCCC GGACCCCTGAGGTCACATGCGTGGTGGTGGACGTGAGCCACGAA GACCCTGAGGTCAAGTTCAACTGGTACGTGGACGGCGTGGAGGTG CATAATGCCAAGACAAAGCCGCGGGAGGAGCAGTACAACAGCAC GTACCGTGTGGTCAGCGTCCTCACCGTCCTGCACCAGGACTGGCT GAATGGCAAGGAGTACAAGTGCAAGGTCTCCAACAAAGCCCTCC CAGCCCCCGAAGAGAAAACCATCTCCAAAGCCAAAGGGCAGCCC CGAGAACCACAGGTGTACACCCTGCCCCCATGCCGGGAGGAGAT GACCAAGAACCAGGTCAGCCTGTGGTGCCTGGTCAAAGGCTTCTA TCCCAGCGACATCGCCGTGGAGTGGGAGAGCAATGGGCAGCCGG AGAACAACTACAAGACCACGCCTCCCGTGCTGGACTCCGACGGCT CCTTCTTCCTCTACAGCAAGCTCACCGTGGACAAGAGCAGGTGGC AGCAGGGGAACGTCTTCTCATGCTCCGTGATGCATGAGGCTCTGC ACAACCACTACACGCAGAAGAGCCTCTCCCTGTCTCCGGGTAAA | |
| TraL-Q100C-KnobS239D-I332E | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGATGTGGCACAAAAGTAGAGATTA AGCGTACGGTGGCAGCTCCCAGCGTTTTTATCTTTCCCCCATCCGA CGAGCAGCTCAAGAGTGGCACTGCCTCTGTAGTTTGTTTGCTGAAT AACTTCTATCCACGTGAAGCAAAAGTACAGTGGAAGGTCGATAAT GCCCTTCAGAGCGGTAACAGCCAAGAAAGTGTTACCGAGCAAGA TTCCAAAGATTCCACTTACAGTCTGTCCAGCACATTGACACTGAGT AAGGCTGATTACGAAAAACACAAGGTGTACGCATGCGAGGTGAC ACACCAAGGTCTTTCATCTCCTGTAACTAAGAGCTTTAACCGGGG AGAATGTGGTGGTGGGGGCAGCGGGGGCGGAGGTAGTGGAGGCG GCGGTAGTGAACCAAAGAGTAGTGACAAAACTCACACGTGCCCA CCGTGCCCAGCACCTGAACTCCTGGGGGGACCGGATGTCTTCCTC TTCCCCCCAAAACCCAAGGACACCCTCATGATCTCCCGGACCCCT GAGGTCACATGCGTGGTGGTGGACGTGAGCCACGAAGACCCTGA GGTCAAGTTCAACTGGTACGTGGACGGCGTGGAGGTGCATAATGC CAAGACAAAGCCGCGGGAGGAGCAGTACAACAGCACGTACCGTG TGGTCAGCGTCCTCACCGTCCTGCACCAGGACTGGCTGAATGGCA AGGAGTACAAGTGCAAGGTCTCCAACAAAGCCCTCCCAGCCCCCG AAGAGAAAACCATCTCCAAAGCCAAAGGGCAGCCCCGAGAACCA CAGGTGTACACCCTGCCCCCATGCCGGGAGGAGATGACCAAGAA CCAGGTCAGCCTGTGGTGCCTGGTCAAAGGCTTCTATCCCAGCGA CATCGCCGTGGAGTGGGAGAGCAATGGGCAGCCGGAGAACAACT ACAAGACCACGCCTCCCGTGCTGGACTCCGACGGCTCCTTCTTCCT | 481 |
| | CTACAGCAAGCTCACCGTGGACAAGAGCAGGTGGCAGCAGGGGA ACGTCTTCTCATGCTCCGTGATGCATGAGGCTCTGCACAACCACTA CACGCAGAAGAGCCTCTCCCTGTCTCCGGGTAAA | |
| PerH-G44C-KnobS239D-I332E | GAAGTACAGTTGGTGGAGTCTGGTGGAGGTCTTGTACAACCTGGC GGGAGTTTGCGGCTTTCCTGCGCTGCAAGCGGGTTTACCTTCACCG ATTATACCATGGATTGGGTACGCCAAGCCCCTGGTAAGTGCCTTG AGTGGGTTGCCGATGTAAACCCTAATTCCGGAGGAAGTATCTATA ATCAACGCTTCAAGGGCCGATTCACTCTGAGTGTGGATCGAAGCA AGAACACCTTGTACTTGCAGATGAATTCCTTGCGGGCTGAAGACA CAGCCGTCTATTACTGCGCCCGAAATTTGGGGCCTTCATTCTATTT TGACTATTGGGGTCAGGGAACTCTGGTAACTGTTTCAAGTGCTAG CACCAAAGGACCTAGTGTTTTTCCTCTTGCCCCTTCCTCAAAGTCT ACCTCTGGGGGGACAGCCGCTCTGGGCTGCCTGGTCAAGGATTAT TTCCCAGAGCCTGTCACTGTCAGTTGGAACTCTGGAGCCTTGACTT CTGGTGTTCATACATTTCCTGCTGTCCTTCAAAGCAGCGGCTTGTA CTCATTGTCTTCTGTTGTGACAGTACCCTCAAGCAGCCTCGGCACT CAGACATACATCTGCAATGTCAACCACAAACCCTCAAATACAAAG GTAGATAAAAAAGTCGAACCAAAGTCTTGTGACAAAACTCACAC GTGCCCACCGTGCCCAGCACCTGAACTCCTGGGGGGACCGGATGT CTTCCTCTTCCCCCCAAAACCCAAGGACACCCTCATGATCTCCCGG ACCCCTGAGGTCACATGCGTGGTGGTGGACGTGAGCCACGAAGAC CCTGAGGTCAAGTTCAACTGGTACGTGGACGGCGTGGAGGTGCAT AATGCCAAGACAAAGCCGCGGGAGGAGCAGTACAACAGCACGTA CCGTGTGGTCAGCGTCCTCACCGTCCTGCACCAGGACTGGCTGAA TGGCAAGGAGTACAAGTGCAAGGTCTCCAACAAAGCCCTCCCAGC CCCCGAAGAGAAAACCATCTCCAAAGCCAAAGGGCAGCCCCGAG AACCACAGGTGTACACCCTGCCCCCATGCCGGGAGGAGATGACCA AGAACCAGGTCAGCCTGTGGTGCCTGGTCAAAGGCTTCTATCCCA GCGACATCGCCGTGGAGTGGGAGAGCAATGGGCAGCCGGAGAAC AACTACAAGACCACGCCTCCCGTGCTGGACTCCGACGGCTCCTTC TTCCTCTACAGCAAGCTCACCGTGGACAAGAGCAGGTGGCAGCAG GGGAACGTCTTCTCATGCTCCGTGATGCATGAGGCTCTGCACAAC CACTACACGCAGAAGAGCCTCTCCCTGTCTCCGGGTAAA | 482 |
| PerL-Q100C-KnobS239D-I332E | GATATTCAAATGACCCAAAGTCCAAGTTCCCTTTCAGCATCTGTCG GTGATCGAGTCACTATAACTTGTAAGGCCAGCCAAGACGTTAGCA TAGGAGTAGCATGGTACCAACAAAAGCCTGGGAAGGCTCCCAAA CTTCTCATTTATTCTGCTTCCTACCGATATACTGGTGTCCCAAGTA GATTTTCTGGCAGCGGATCTGGAACTGATTTTACATTGACTATCAG CTCCCTCCAGCCTGAGGACTTCGCTACTTATTACTGCCAACAGTAC TACATTTATCCCTATACATTCGGTTGTGGGACCAAAGTAGAGATC AAACGTACGGTGGCAGCTCCCAGCGTTTTTATCTTTCCCCCATCCG ACGAGCAGCTCAAGAGTGGCACTGCCTCTGTAGTTTGTTTGCTGA ATAACTTCTATCCACGTGAAGCAAAAGTACAGTGGAAGGTCGATA ATGCCCTTCAGAGCGGTAACAGCCAAGAAAGTGTTACCGAGCAA GATTCCAAAGATTCCACTTACAGTCTGTCCAGCACATTGACACTG | 483 |
| | AGTAAGGCTGATTACGAAAAACACAAGGTGTACGCATGCGAGGT GACACACCAAGGTCTTTCATCTCCTGTAACTAAGAGCTTTAACCG GGGAGAATGTGGTGGTGGGGGCAGCGGGGGCGGAGGTAGTGGAG GCGGCGGTAGTGAACCAAAGAGTAGTGACAAAACTCACACGTGC CCACCGTGCCCAGCACCTGAACTCCTGGGGGGACCGGATGTCTTC CTCTTCCCCCCAAAACCCAAGGACACCCTCATGATCTCCCGGACC CCTGAGGTCACATGCGTGGTGGTGGACGTGAGCCACGAAGACCCT GAGGTCAAGTTCAACTGGTACGTGGACGGCGTGGAGGTGCATAAT GCCAAGACAAAGCCGCGGGAGGAGCAGTACAACAGCACGTACCG TGTGGTCAGCGTCCTCACCGTCCTGCACCAGGACTGGCTGAATGG CAAGGAGTACAAGTGCAAGGTCTCCAACAAAGCCCTCCCAGCCCC CGAAGAGAAAACCATCTCCAAAGCCAAAGGGCAGCCCCGAGAAC CACAGGTGTACACCCTGCCCCCATGCCGGGAGGAGATGACCAAG AACCAGGTCAGCCTGTGGTGCCTGGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTTC CTCTACAGCAAGCTCACCGTGGACAAGAGCAGGTGGCAGCAGGG GAACGTCTTCTCATGCTCCGTGATGCATGAGGCTCTGCACAACCA CTACACGCAGAAGAGCCTCTCCCTGTCTCCGGGTAAA | |

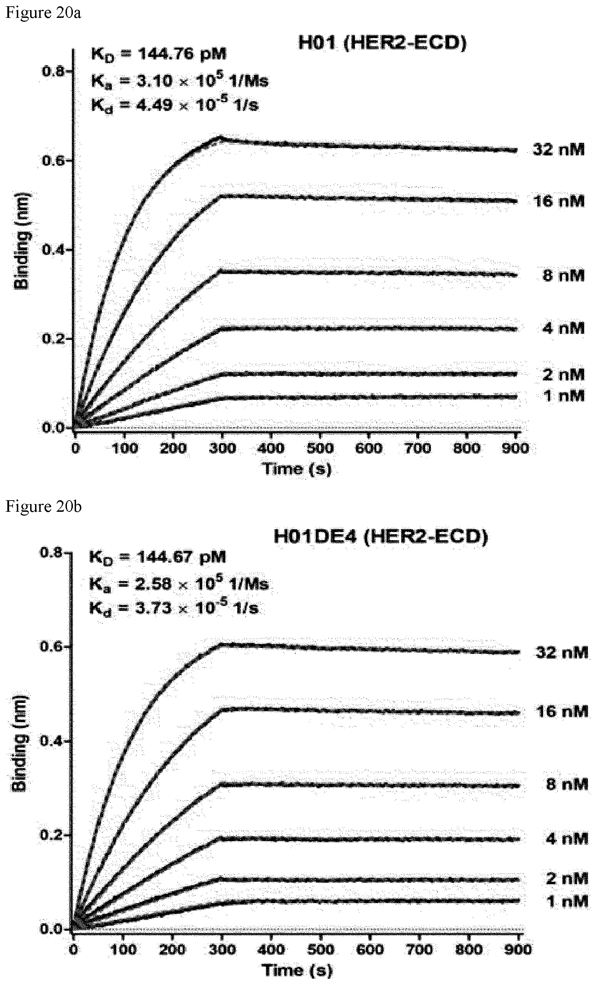

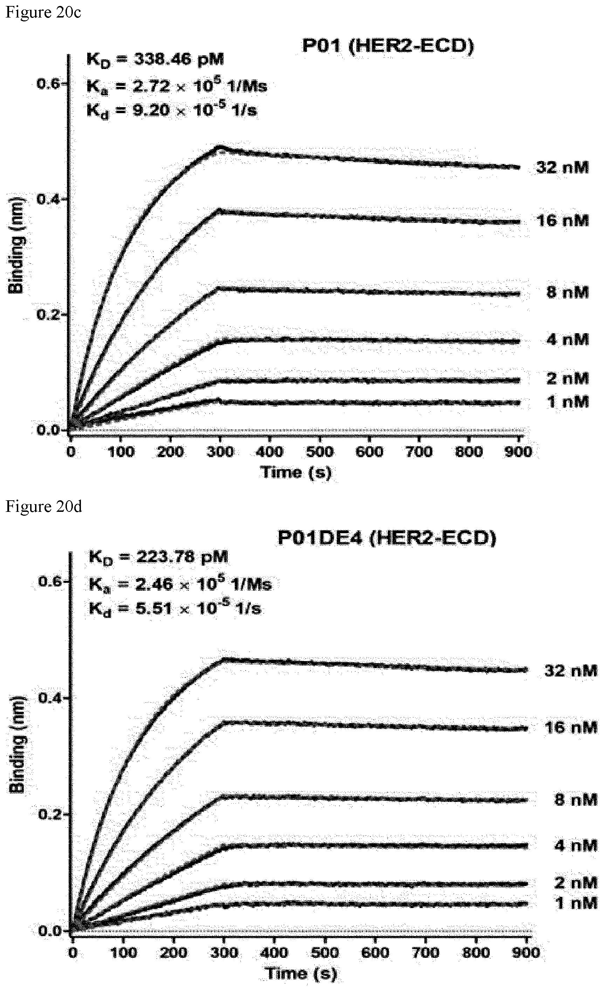

Binding characteristics were analyzed at 25 C using Octet Red96e (Sartorius), a bio-layer interferometry (BLI). The buffer used for analysis was 10X Kinetics Buffer (Sartorius, 18-1042) diluted in PBS pH 7.4 (Gibco, 10010), and analysis plate was agitated at 1,000 rpm. The human HER2 recombinant protein (R&D systems, 1129-ER) was loaded onto the Anti-Penta-HIS (HIS1K) biosensor (Sartorius, 18-5120). To measure the binding rate constant (Kₐ), 1 to 32 nM of H01, H01DE4, P01, and P01DE4 were allowed to bind to loaded antigen for 300 seconds and then the dissociation rate constant (K_{d}) was determined after 600 seconds of dissociation in Kinetics Buffer. Kₐ and K_{d} values were measured through a 1:1 binding model in Octet analysis software (Sartorius), and the equilibrium dissociation constant (K_{D}) value was determined (Figures 20a to 20d, Table 28).

**[Table 28]**

| | K_{D}(pM) | Kₐ(1/Ms) | K_{d}(1/s) |
|---|---|---|---|
| H01 | 144.76 | 3.10 x 10⁵ | 4.49 x 10⁻⁵ |
| H01DE4 | 144.67 | 2.58 x 10⁵ | 3.73 x 10⁻⁵ |
| P01 | 338.46 | 2.72 x 10⁵ | 9.20 x 10⁻⁵ |
| P01DE4 | 223.78 | 2.46 x 10⁵ | 5.51 x 10⁻⁵ |

### Example 10. Analysis of antibody binding affinity to Fcγ receptors

The binding constants of each antibody to Fcy receptors at 25°C were analyzed using Octet Red96e (Sartorius). Anti-Penta-HIS (HIS1K) biosensor (Sartorius, 18-5120) was used, and human FcyRI (R&D systems, 1257-FC) or human FcγIIA (R&D systems, 1330-CD) or human FcyRIIIA (176V isoform, R&D systems, 4325-FC) containing a His tag were loaded onto the biosensor.

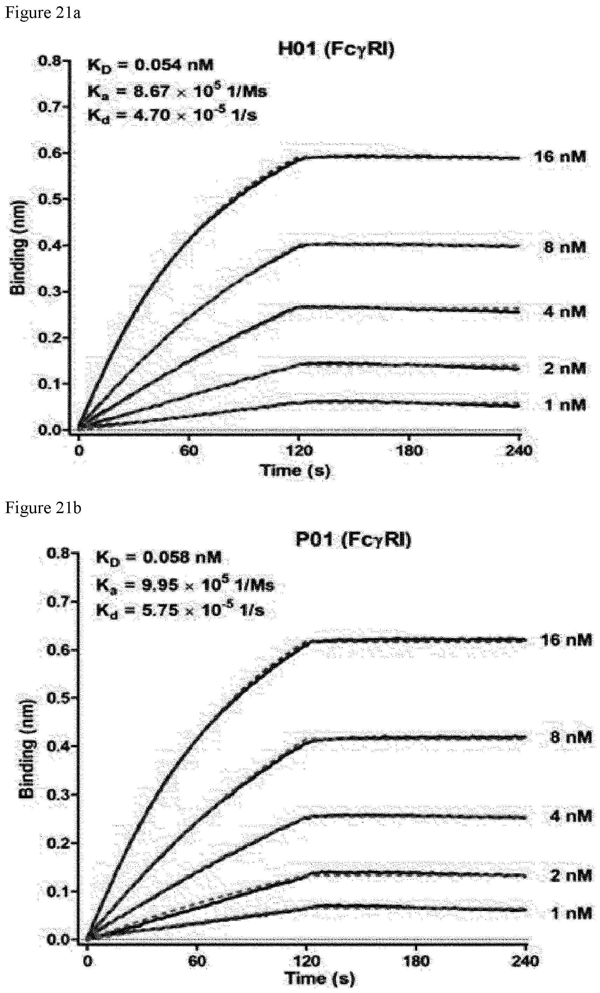

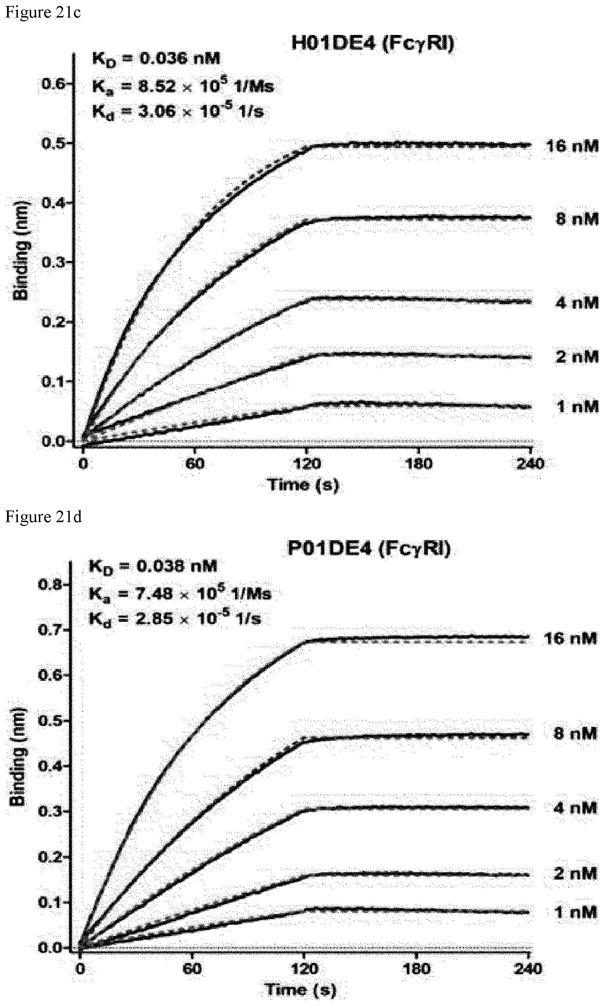

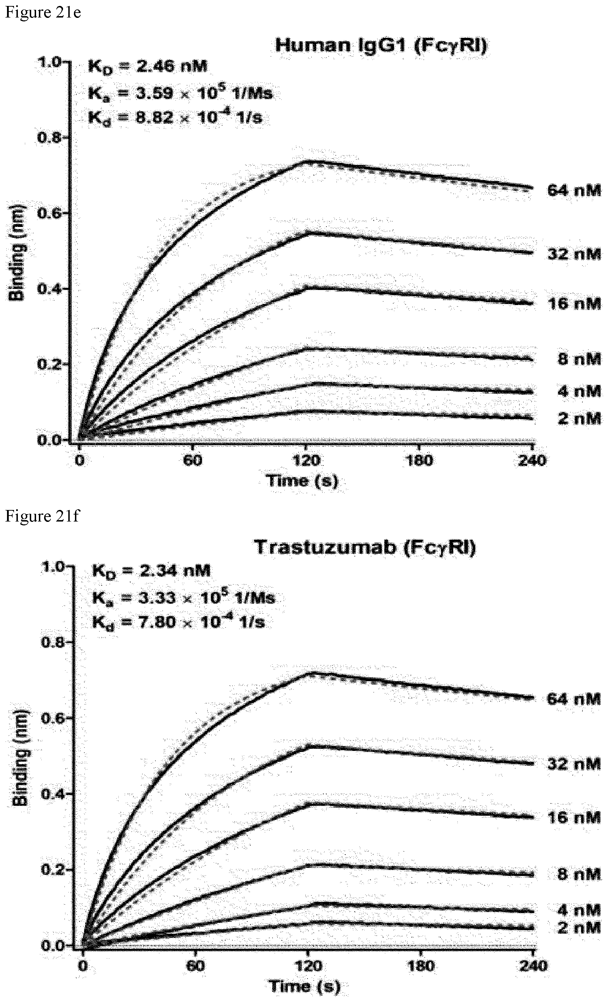

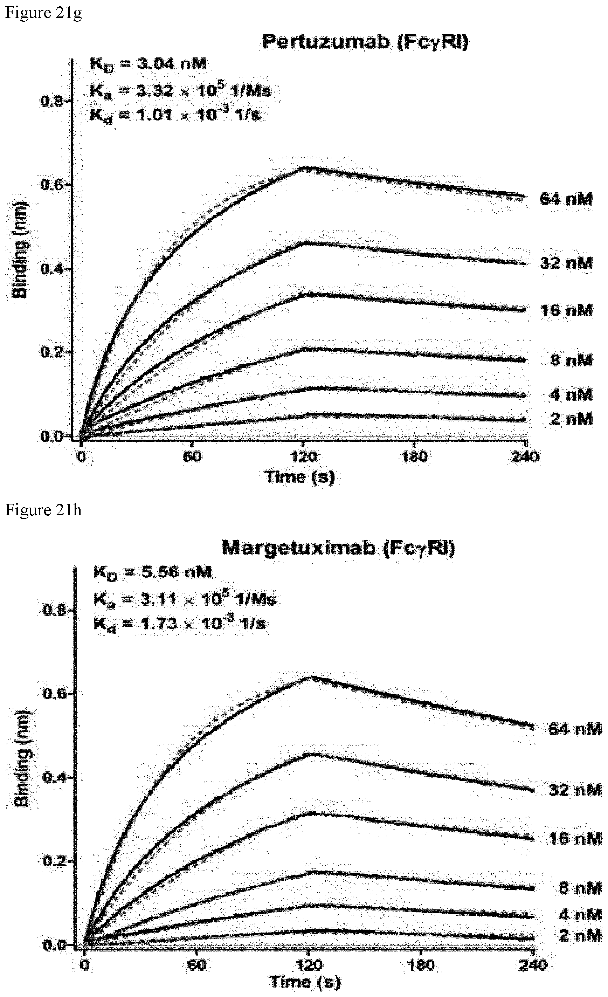

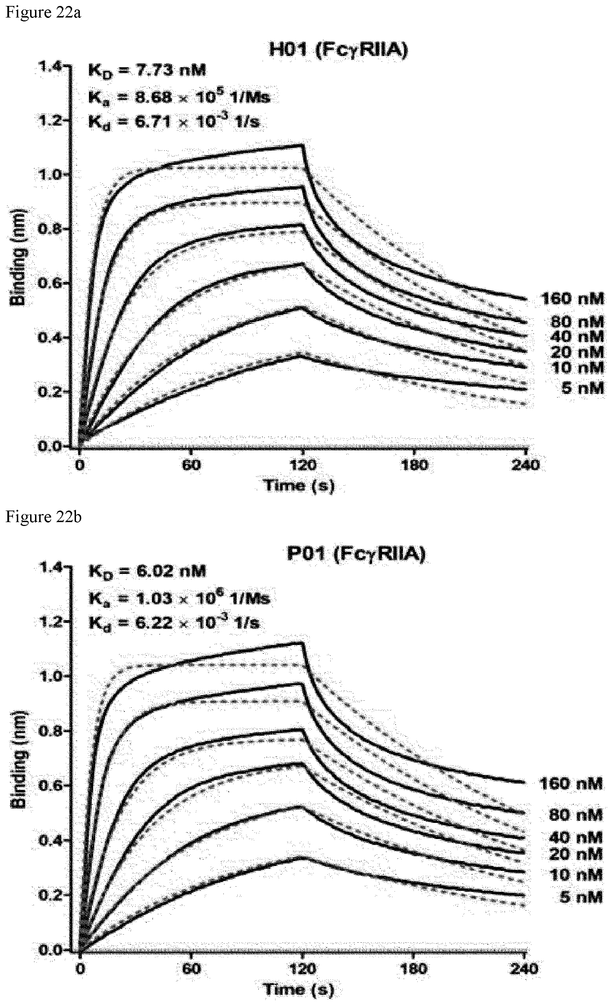

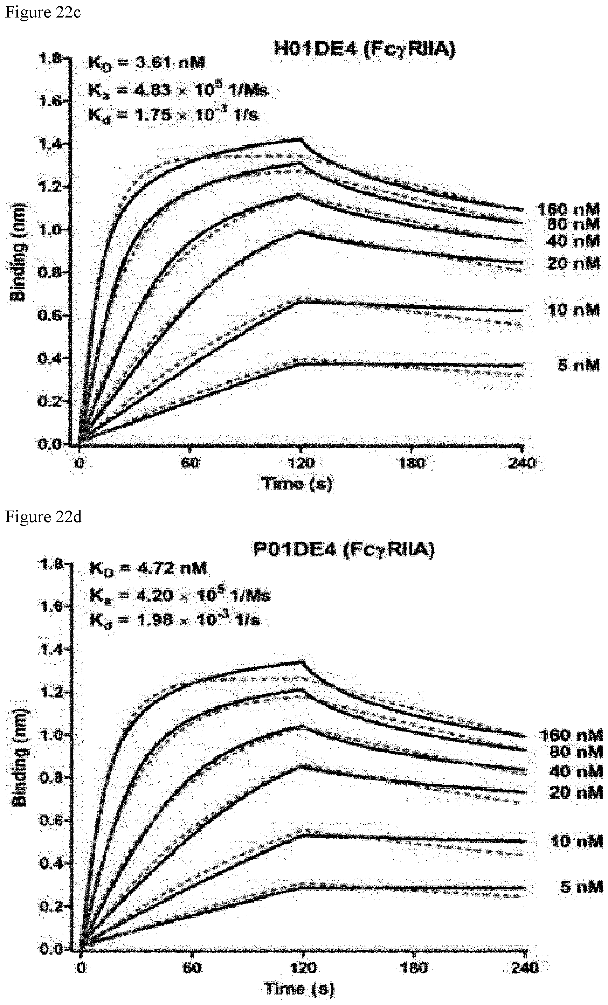

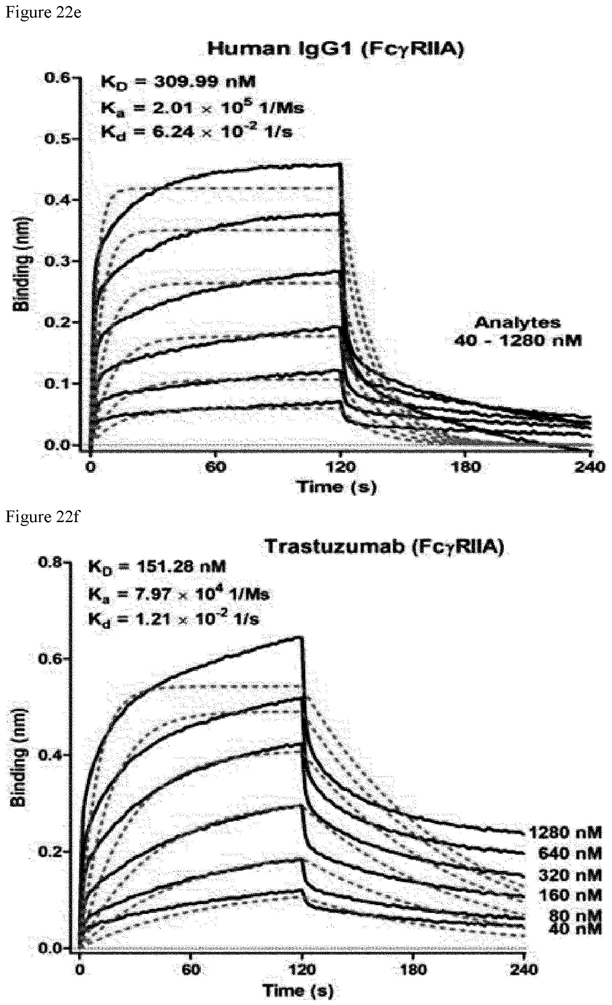

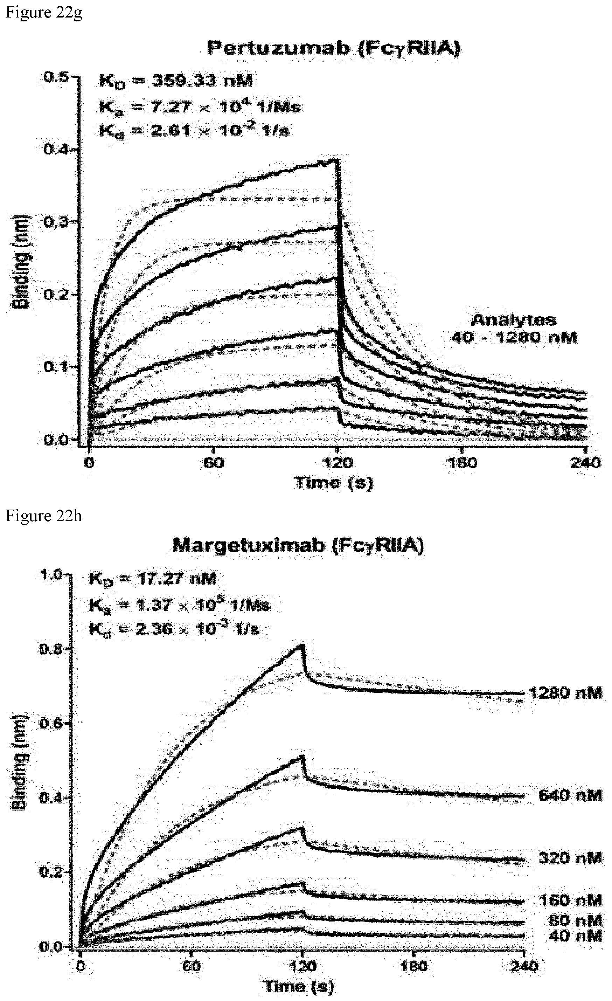

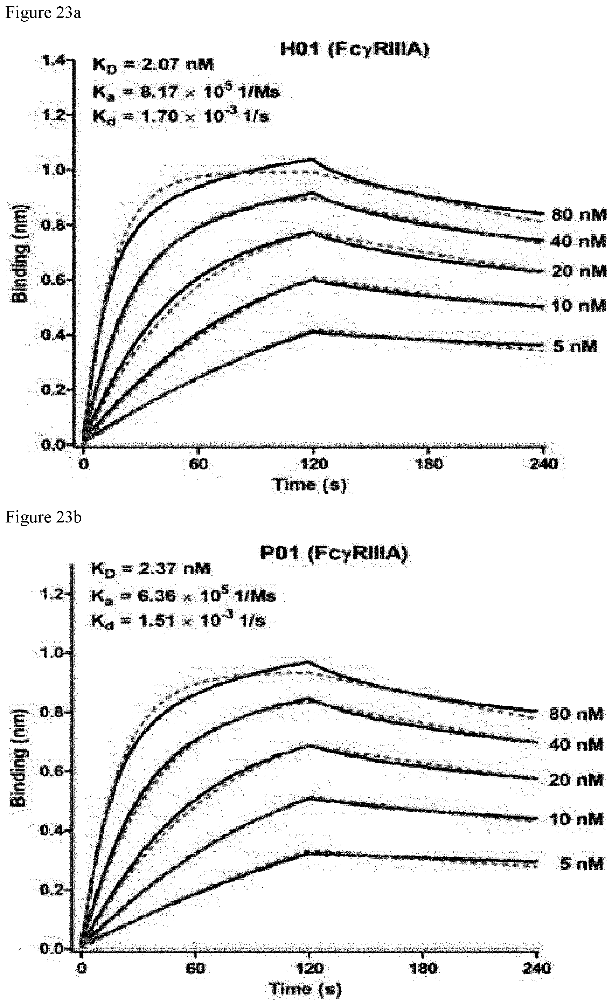

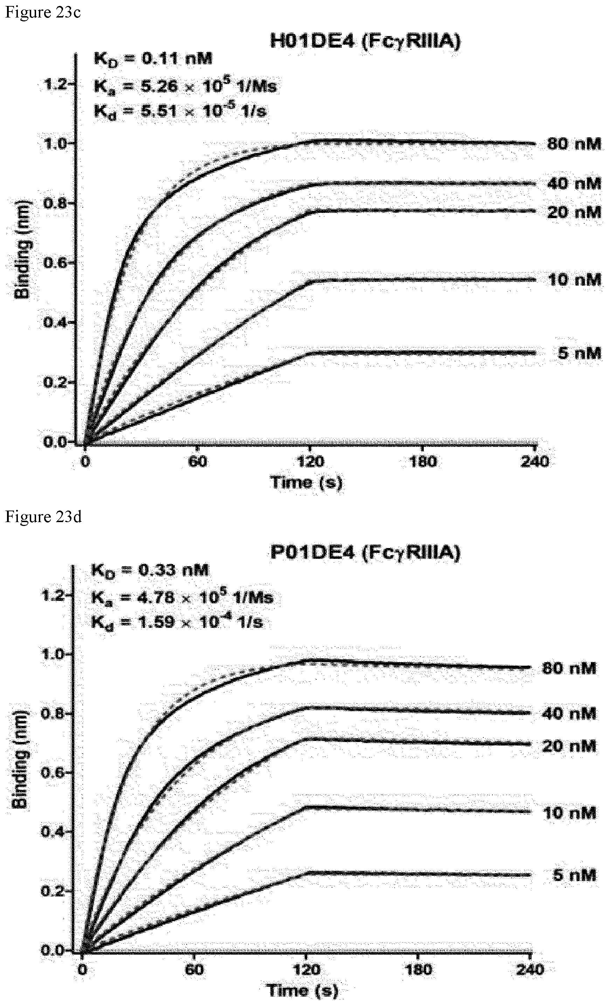

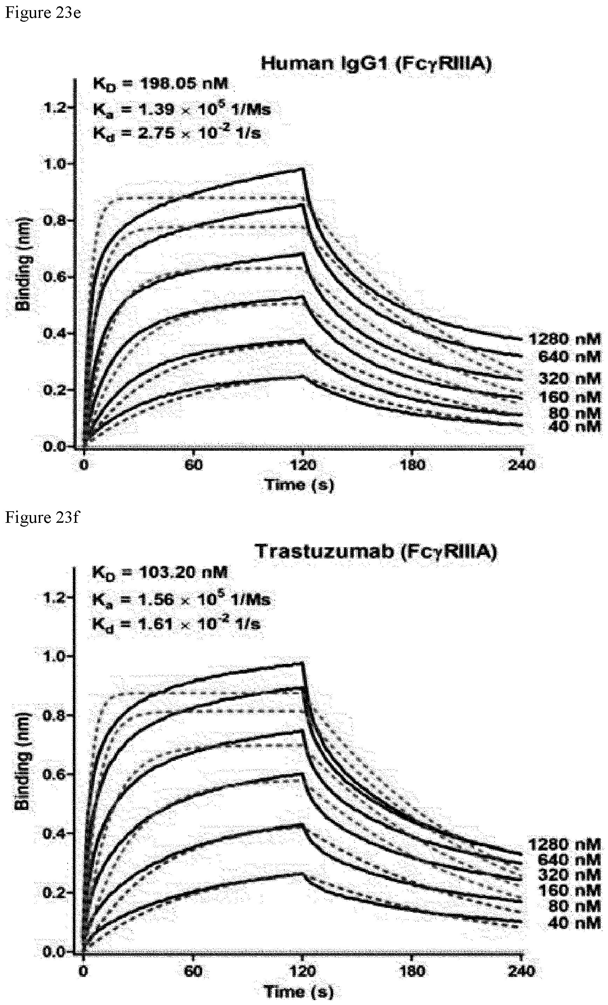

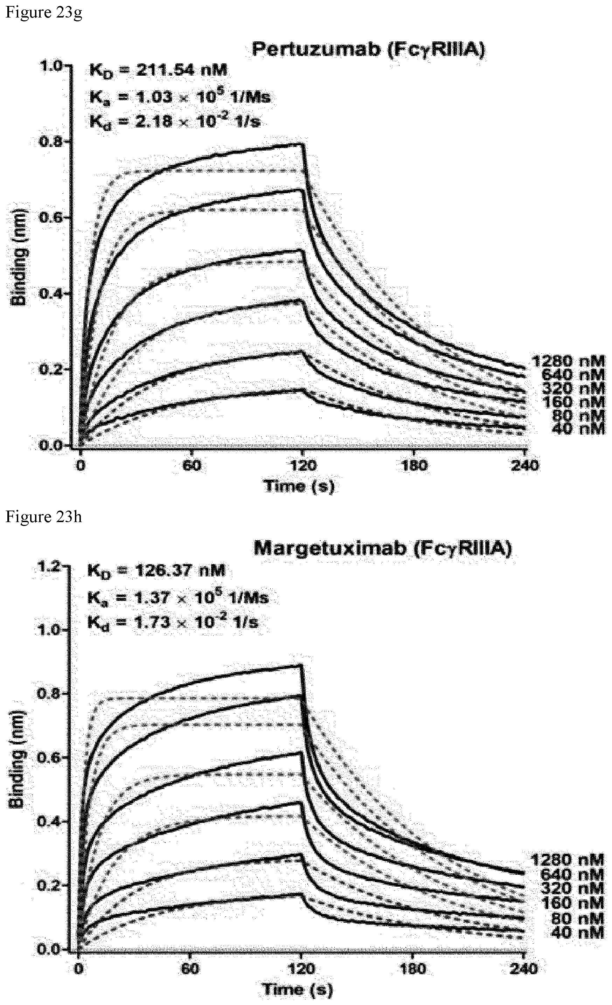

The association rate constants (Kₐ) and the dissociation rate constants (K_{d}) of H01, P01, H01DE4, P01DE4, human IgG1 (Bio X cell, BE0297), trastuzumab, pertuzumab, and margetuximab to the biosensor loaded with an antigen was determined. Kₐ and K_{d} values were calculated through a 1:1 binding model in Octet analysis software (Sartorius), and the equilibrium dissociation constant (K_{D}) value was determined (Figures 21a to 21h, Figures 22a to 22h, Figures 23a to 23h, Table 29).

**[Table 29]**

| Antigen | FcγRI(CD64) | | | FcγRIIA(CD32A, 131R) | | | FcγRIIIA(CD 16A,176V) | | |
|---|---|---|---|---|---|---|---|---|---|
| Antibody | KD (nM) | Ka (1/Ms) | Kd (1/s) | KD (nM) | Ka (1/Ms) | Kd (1/s) | KD (nM) | Ka (1/Ms) | Kd (1/s) |
| H01 | 0.054 | 8.67E+0 5 | 4.70E-05 | 7.73 | 8.68E+0 5 | 6.71E-03 | 2.08 | 8.17E+0 5 | 1.70E-03 |
| P01 | 0.058 | 9.95E+0 5 | 5.75E-05 | 6.04 | 1.03E+0 6 | 6.22E-03 | 2.37 | 6.36E+0 5 | 1.51E-03 |
| H01DE4 | 0.036 | 8.52E+0 5 | 3.06E-05 | 3.62 | 4.83E+0 5 | 1.75E-03 | 0.10 | 5.26E+0 5 | 5.51E-05 |
| P01DE4 | 0.038 | 7.48E+0 5 | 2.85E-05 | 4.71 | 4.20E+0 5 | 1.98E-03 | 0.33 | 4.78E+0 5 | 1.59E-04 |
| Human IgG1 | 2.457 | 3.59E+0 5 | 8.82E-04 | 310.45 | 2.01E+0 5 | 6.24E-02 | 197.84 | 1.39E+0 5 | 2.75E-02 |
| Trastuzumab | 2.342 | 3.33E+0 5 | 7.80E-04 | 151.82 | 7.97E+0 4 | 1.21E-02 | 103.21 | 1.56E+0 5 | 1.61E-02 |
| Pertuzumab | 3.042 | 3.32E+0 5 | 1.01E-03 | 359.01 | 7.27E+0 4 | 2.61E-02 | 211.65 | 1.03E+0 5 | 2.18E-02 |
| Margetuximab | 5.563 | 3.11E+0 5 | 1.73E-03 | 17.23 | 1.37E+0 5 | 2.36E-03 | 126.28 | 1.37E+0 5 | 1.73E-02 |

### Example 11. Pharmacokinetic (PK) analysis in rats

H01, P01 trastuzumab, and pertuzumab were administered at 10 mg/kg to 7-week-old male Sprague-Dawley rats (ORIENT BIO INC.) via the intravenous (i.v.) route. Blood samples were collected in 5 minutes, 15 minutes, 30 minutes, 1 hour, 2 hours, 4 hours, 8 hours, 1 day, 2 days, 3 days, 7 days, 10 days, 14 days, 21 days, 28 days, 35 days, and 42 days after administration. Thereafter, only the serum was separated from the blood for analysis. The antibody concentration in serum was measured by ELISA.

Briefly, a 96-well ELISA plate (Corning, 3590) was coated with human HER2 recombinant protein (R&D systems, 1129-ER) and stored overnight at 4°C, and the sera obtained at each time were appropriately diluted, and allowed to bind to the coated human HER2. Peroxidase-conjugated anti-human Fab goat antibody (Invitrogen, 31482) was used to detect H01, P01 trastuzumab, and pertuzumab.

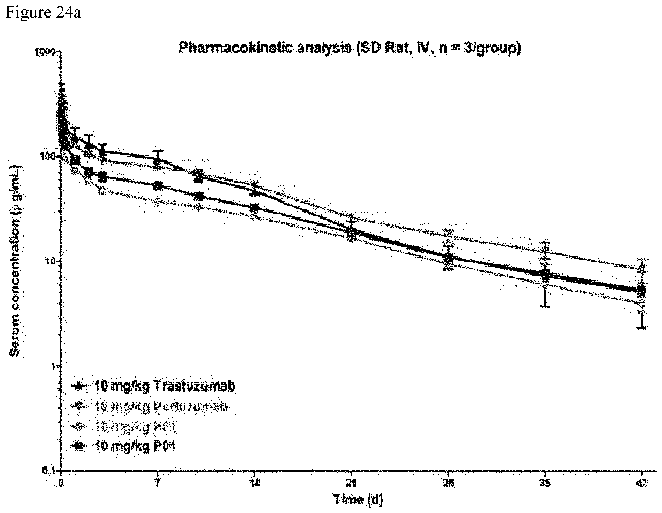

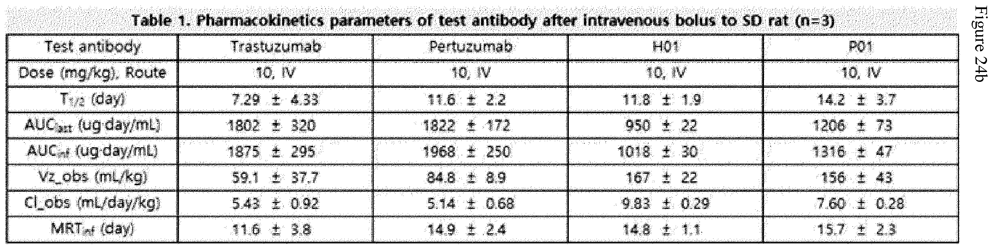

Standard samples of H01, P01, trastuzumab, and pertuzumab were prepared, and the concentrations of the analytes at each time point were quantified based on a standard curve created from naive rat serum containing different concentrations of the antibody standards. The half-lives of intravenously administered H01, P01, trastuzumab, and pertuzumab were determined to be approximately 11.8 days, 14.2 days, 7.3 days, and 11.6 days, respectively (Figures 24a and 24b, Table 30). The engineered novel antibodies, H01 and P01, were found to have similar PK parameters to the parental humanized antibodies trastuzumab and pertuzumab.

**[Table 30]**

| | Trastuzumab | Pertuzumab | H01 | P01 |
|---|---|---|---|---|
| Dose (mg/kg), Route | 10, IV | 10, IV | 10, IV | 10, IV |
| T_{1/2} (day) | 7.3 ± 4.3 | 11.6 ± 2.2 | 11.8 ± 1.9 | 14.2 ± 3.7 |
| AUCₗₐₛₜ (µg*day/mL) | 1802 ± 320 | 1822 ± 172 | 950 ± 22 | 1206 ± 73 |
| AUC_{inf} (µg*day/mL) | 1875 ± 295 | 1968 ± 250 | 1018 ± 30 | 1316 ± 47 |
| Vz_obs (mL/kg) | 59. 1 ± 37.7 | 84.8 ± 8.9 | 167 ± 22 | 156 ± 43 |
| Cl_obs (mL/day/kg) | 5.4 ± 0.9 | 5.1 ± 0.7 | 9.8 ± 0.3 | 7.6 ± 0.3 |
| MRT_{inf} (day) | 11.6 ± 3.8 | 14.9 ± 2.4 | 14.8 ± 1.1 | 15.7 ± 2.3 |

### Example 12. Design, preparation and analysis of novel antibody that recognizes two epitopes of HER2

In order to construct an antibody that recognizes two epitopes of HER2 protein, a biparatopic antibody HP501 was designed by connecting the V domains of trastuzumab and pertuzumab via a linker (Figure 25). In order to minimize the decrease in binding affinity due to interference between different V domains, linkers with variable lengths connecting the V domains were tested. At the same time, as an attempt to improve the physical integrity of the antibody, 16 variants were designed in which Cys substitution mutations capable of forming a disulfide bond in the V domain were introduced (Figure 26, Table 31). According to Kabat numbering system, mutations were introduced only at position 44 for the heavy chain and position 100 for the light chain (Table 31). In Table 31, the VH linker with 6 amino acid residues was designated as VH-S-Linker, the VH linker with 13 amino acid residues was designated as VH-L-Linker, the VL linker with 6 amino acid residues was designated as VL-S-Linker, and the VL linker with 13 amino acid residues was designated as VL-L-Linker.

**[Table 31]**

| Clone name | VH domain | | | VH domain | | |
|---|---|---|---|---|---|---|
| | VH1 44 (Upper) | Linker length | VH2 44 (Lower) | VL1 100 (Upper) | Linker length | VL2 100 (Lower) |
| HP501 | Gly (G) | 6 | Gly (G) | Gln (Q) | 6 | Gln (Q) |
| HP502 | Cys (C) | 6 | Gly (G) | Cys (C) | 6 | Gln (Q) |
| HP503 | Gly (G) | 6 | Cys (C) | Gln (Q) | 6 | Cys (C) |
| HP504 | Cys (C) | 6 | Cys (C) | Cys (C) | 6 | Cys (C) |
| HP505 | Gly (G) | 6 | Gly (G) | Gln (Q) | 13 | Gln (Q) |
| HP506 | Cys (C) | 6 | Gly (G) | Cys (C) | 13 | Gln (Q) |
| HP507 | Gly (G) | 6 | Cys (C) | Gln (Q) | 13 | Cys (C) |
| HP508 | Cys (C) | 6 | Cys (C) | Cys (C) | 13 | Cys (C) |
| HP509 | Gly (G) | 13 | Gly (G) | Gln (Q) | 6 | Gln (Q) |
| HP510 | Cys (C) | 13 | Gly (G) | Cys (C) | 6 | Gln (Q) |
| HP511 | Gly (G) | 13 | Cys (C) | Gln (Q) | 6 | Cys (C) |
| HP512 | Cys (C) | 13 | Cys (C) | Cys (C) | 6 | Cys (C) |
| HP513 | Gly (G) | 13 | Gly (G) | Gln (Q) | 13 | Gln (Q) |
| HP514 | Cys (C) | 13 | Gly (G) | Cys (C) | 13 | Gln (Q) |
| HP515 | Gly (G) | 13 | Cys (C) | Gln (Q) | 13 | Cys (C) |
| HP516 | Cys (C) | 13 | Cys (C) | Cys (C) | 13 | Cys (C) |

For HP501, expression vectors consisting of the sequences corresponding to Fc-Hole (SEQ ID NO: 7), TH-S-PH-Knob (SEQ ID NO: 51), and TL-S-PL-Knob (SEQ ID NO: 59) were co-transfected into EXPICHO-S^{™} (Gibco, A29127) (Tables 31, 32, 33, and 34), and purification and analysis were performed in the same manner as described in Example 1. Expression, purification and analysis were performed for HP502 to HP516 in the same manner as mentioned above, and the composition of the expression vector is shown in detail in Tables 32 and 34.

**[Table 32]**

| Name | Sequence | SEQ ID NO |
|---|---|---|
| VH-S-Linker | ASTKGP | 47 |
| VH-L-Linker | ASTKGPSVFPLAP | 48 |
| VL-S-Linker | RTVAAP | 49 |
| VL-L-Linker | RTVAAPSVFIFPP | 50 |
| TH-S-PH-Knob | EVQLVESGGGLVQPGGSLRLSCAASGFNIKDTYIHWVRQAPGKGLE WVARIYPTNGYTRYADSVKGRFTISADTSKNTAYLQMNSLRAEDTA VYYCSRWGGDGFYAMDYWGQGTLVTVSSASTKGPEVQLVESGGGL VQPGGSLRLSCAASGFTFTDYTMDWVRQAPGKGLEWVADVNPNSG GSIYNQRFKGRFTLSVDRSKNTLYLQMNSLRAEDTAVYYCARNLGPS FYFDYWGQGTLVTVSSASTKGPSVFPLAPSSKSTSGGTAALGCLVKD YFPEPVTVSWNSGALTSGVHTFPAVLQSSGLYSLSSVVTVPSSSLGTQ TYICNVNHKPSNTKVDKKVEPKSCDKTHTCPPCPAPELLGGPSVFLFP PKPKDTLMISRTPEVTCVVVDVSHEDPEVKFNWYVDGVEVHNAKTK PREEQYNSTYRVVSVLTVLHQDWLNGKEYKCKVSNKALPAPIEKTIS KAKGQPREPQVYTLPPCREEMTKNQVSLWCLVKGFYPSDIAVEWES NGQPENNYKTTPPVLDSDGSFFLYSKLTVDKSRWQQGNVFSCSVMH EALHNHYTQKSLSLSPGK | 51 |
| THC-S-PH-Knob | EVQLVESGGGLVQPGGSLRLSCAASGFNIKDTYIHWVRQAPGKCLE WVARIYPTNGYTRYADSVKGRFTISADTSKNTAYLQMNSLRAEDTA | 52 |
| | VYYCSRWGGDGFYAMDYWGQGTLVTVSSASTKGPEVQLVESGGGL VQPGGSLRLSCAASGFTFTDYTMDWVRQAPGKGLEWVADVNPNSG GSIYNQRFKGRFTLSVDRSKNTLYLQMNSLRAEDTAVYYCARNLGPS FYFDYWGQGTLVTVSSASTKGPSVFPLAPSSKSTSGGTAALGCLVKD YFPEPVTVSWNSGALTSGVHTFPAVLQSSGLYSLSSVVTVPSSSLGTQ TYICNVNHKPSNTKVDKKVEPKSCDKTHTCPPCPAPELLGGPSVFLFP PKPKDTLMISRTPEVTCVVVDVSHEDPEVKFNWYVDGVEVHNAKTK PREEQYNSTYRVVSVLTVLHQDWLNGKEYKCKVSNKALPAPIEKTIS KAKGQPREPQVYTLPPCREEMTKNQVSLWCLVKGFYPSDIAVEWES NGQPENNYKTTPPVLDSDGSFFLYSKLTVDKSRWQQGNVFSCSVMH EALHNHYTQKSLSLSPGK | |
| TH-S-PHC-Knob | EVQLVESGGGLVQPGGSLRLSCAASGFNIKDTYIHWVRQAPGKGLE WVARIYPTNGYTRYADSVKGRFTISADTSKNTAYLQMNSLRAEDTA VYYCSRWGGDGFYAMDYWGQGTLVTVSSASTKGPEVQLVESGGGL VQPGGSLRLSCAASGFTFTDYTMDWVRQAPGKCLEWVADVNPNSG GSIYNQRFKGRFTLSVDRSKNTLYLQMNSLRAEDTAVYYCARNLGPS FYFDYWGQGTLVTVSSASTKGPSVFPLAPSSKSTSGGTAALGCLVKD YFPEPVTVSWNSGALTSGVHTFPAVLQSSGLYSLSSVVTVPSSSLGTQ TYICNVNHKPSNTKVDKKVEPKSCDKTHTCPPCPAPELLGGPSVFLFP PKPKDTLMISRTPEVTCVVVDVSHEDPEVKFNWYVDGVEVHNAKTK PREEQYNSTYRVVSVLTVLHQDWLNGKEYKCKVSNKALPAPIEKTIS KAKGQPREPQVYTLPPCREEMTKNQVSLWCLVKGFYPSDIAVEWES NGQPENNYKTTPPVLDSDGSFFLYSKLTVDKSRWQQGNVFSCSVMH EALHNHYTQKSLSLSPGK | 53 |
| THC-S-PHC-Knob | EVQLVESGGGLVQPGGSLRLSCAASGFNIKDTYIHWVRQAPGKCLE WVARIYPTNGYTRYADSVKGRFTISADTSKNTAYLQMNSLRAEDTA VYYCSRWGGDGFYAMDYWGQGTLVTVSSASTKGPEVQLVESGGGL VQPGGSLRLSCAASGFTFTDYTMDWVRQAPGKCLEWVADVNPNSG GSIYNQRFKGRFTLSVDRSKNTLYLQMNSLRAEDTAVYYCARNLGPS FYFDYWGQGTLVTVSSASTKGPSVFPLAPSSKSTSGGTAALGCLVKD YFPEPVTVSWNSGALTSGVHTFPAVLQSSGLYSLSSVVTVPSSSLGTQ TYICNVNHKPSNTKVDKKVEPKSCDKTHTCPPCPAPELLGGPSVFLFP PKPKDTLMISRTPEVTCVVVDVSHEDPEVKFNWYVDGVEVHNAKTK PREEQYNSTYRVVSVLTVLHQDWLNGKEYKCKVSNKALPAPIEKTIS KAKGQPREPQVYTLPPCREEMTKNQVSLWCLVKGFYPSDIAVEWES NGQPENNYKTTPPVLDSDGSFFLYSKLTVDKSRWQQGNVFSCSVMH EALHNHYTQKSLSLSPGK | 54 |
| TH-L-PH-Knob | EVQLVESGGGLVQPGGSLRLSCAASGFNIKDTYIHWVRQAPGKGLE WVARIYPTNGYTRYADSVKGRFTISADTSKNTAYLQMNSLRAEDTA VYYCSRWGGDGFYAMDYWGQGTLVTVSSASTKGPSVFPLAPEVQL VESGGGLVQPGGSLRLSCAASGFTFTDYTMDWVRQAPGKGLEWVA DVNPNSGGSIYNQRFKGRFTLSVDRSKNTLYLQMNSLRAEDTAVYY CARNLGPSFYFDYWGQGTLVTVSSASTKGPSVFPLAPSSKSTSGGTA ALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL | 55 |
| | GGPSVFLFPPKPKDTLMISRTPEVTCVVVDVSHEDPEVKFNWYVDGV EVHNAKTKPREEQYNSTYRVVSVLTVLHQDWLNGKEYKCKVSNKA LPAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | |
| THC-L-PH-Knob | EVQLVESGGGLVQPGGSLRLSCAASGFNIKDTYIHWVRQAPGKCLE WVARIYPTNGYTRYADSVKGRFTISADTSKNTAYLQMNSLRAEDTA VYYCSRWGGDGFYAMDYWGQGTLVTVSSASTKGPSVFPLAPEVQL VESGGGLVQPGGSLRLSCAASGFTFTDYTMDWVRQAPGKGLEWVA DVNPNSGGSIYNQRFKGRFTLSVDRSKNTLYLQMNSLRAEDTAVYY CARNLGPSFYFDYWGQGTLVTVSSASTKGPSVFPLAPSSKSTSGGTA ALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKA LPAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | 56 |
| TH-L-PHC-Knob | EVQLVESGGGLVQPGGSLRLSCAASGFNIKDTYIHWVRQAPGKGLE WVARIYPTNGYTRYADSVKGRFTISADTSKNTAYLQMNSLRAEDTA VYYCSRWGGDGFYAMDYWGQGTLVTVSSASTKGPSVFPLAPEVQL VESGGGLVQPGGSLRLSCAASGFTFTDYTMDWVRQAPGKCLEWVA DVNPNSGGSIYNQRFKGRFTLSVDRSKNTLYLQMNSLRAEDTAVYY CARNLGPSFYFDYWGQGTLVTVSSASTKGPSVFPLAPSSKSTSGGTA ALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKA LPAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | 57 |
| THC-L-PHC-Knob | EVQLVESGGGLVQPGGSLRLSCAASGFNIKDTYIHWVRQAPGKCLE WVARIYPTNGYTRYADSVKGRFTISADTSKNTAYLQMNSLRAEDTA VYYCSRWGGDGFYAMDYWGQGTLVTVSSASTKGPSVFPLAPEVQL VESGGGLVQPGGSLRLSCAASGFTFTDYTMDWVRQAPGKCLEWVA DVNPNSGGSIYNQRFKGRFTLSVDRSKNTLYLQMNSLRAEDTAVYY CARNLGPSFYFDYWGQGTLVTVSSASTKGPSVFPLAPSSKSTSGGTA ALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKA LPAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | 58 |
| TL-S-PL-Knob | DIQMTQSPSSLSASVGDRVTITCRASQDVNTAVAWYQQKPGKAPKL | 59 |
| | LIYSASFLYSGVPSRFSGSRSGTDFTLTISSLQPEDFATYYCQQHYTTP PTFGQGTKVEIKRTVAAPDIQMTQSPSSLSASVGDRVTITCKASQDVS IGVAWYQQKPGKAPKLLIYSASYRYTGVPSRFSGSGSGTDFTLTISSL QPEDFATYYCQQYYIYPYTFGQGTKVEIKRTVAAPSVFIFPPSDEQLK SGTASVVCLLNNFYPREAKVQWKVDNALQSGNSQESVTEQDSKDST YSLSSTLTLSKADYEKHKVYACEVTHQGLSSPVTKSFNRGECGGGGS GGGGSGGGGSEPKSSDKTHTCPPCPAPELLGGPSVFLFPPKPKDTLMI SRTPEVTCVVVDVSHEDPEVKFNWYVDGVEVHNAKTKPREEQYNST YRVVSVLTVLHQDWLNGKEYKCKVSNKALPAPIEKTISKAKGQPREP QVYTLPPCREEMTKNQVSLWCLVKGFYPSDIAVEWESNGQPENNYK TTPPVLDSDGSFFLYSKLTVDKSRWQQGNVFSCSVMHEALHNHYTQ KSLSLSPGK | |
| TLC-S-PL-Knob | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGCGTKVEIKRTVAAPDIQMTQSPSSLSASVGDRVTITCKASQDVSI GVAWYQQKPGKAPKLLIYSASYRYTGVPSRFSGSGSGTDFTLTISSLQ PEDFATYYCQQYYIYPYTFGQGTKVEIKRTVAAPSVFIFPPSDEQLKS GTASVVCLLNNFYPREAKVQWKVDNALQSGNSQESVTEQDSKDSTY SLSSTLTLSKADYEKHKVYACEVTHQGLSSPVTKSFNRGECGGGGSG GGGSGGGGSEPKSSDKTHTCPPCPAPELLGGPSVFLFPPKPKDTLMIS RTPEVTCVVVDVSHEDPEVKFNWYVDGVEVHNAKTKPREEQYNST YRVVSVLTVLHQDWLNGKEYKCKVSNKALPAPIEKTISKAKGQPREP QVYTLPPCREEMTKNQVSLWCLVKGFYPSDIAVEWESNGQPENNYK TTPPVLDSDGSFFLYSKLTVDKSRWQQGNVFSCSVMHEALHNHYTQ KSLSLSPGK | 60 |
| TL-S-PLC-Knob | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGQGTKVEIKRTVAAPDIQMTQSPSSLSASVGDRVTITCKASQDVS IGVAWYQQKPGKAPKLLIYSASYRYTGVPSRFSGSGSGTDFTLTISSL QPEDFATYYCQQYYIYPYTFGCGTKVEIKRTVAAPSVFIFPPSDEQLK SGTASVVCLLNNFYPREAKVQWKVDNALQSGNSQESVTEQDSKDST YSLSSTLTLSKADYEKHKVYACEVTHQGLSSPVTKSFNRGECGGGGS GGGGSGGGGSEPKSSDKTHTCPPCPAPELLGGPSVFLFPPKPKDTLMI SRTPEVTCVVVDVSHEDPEVKFNWYVDGVEVHNAKTKPREEQYNST YRVVSVLTVLHQDWLNGKEYKCKVSNKALPAPIEKTISKAKGQPREP QVYTLPPCREEMTKNQVSLWCLVKGFYPSDIAVEWESNGQPENNYK TTPPVLDSDGSFFLYSKLTVDKSRWQQGNVFSCSVMHEALHNHYTQ KSLSLSPGK | 61 |
| TLC-S-PLC-Knob | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGCGTKVEIKRTVAAPDIQMTQSPSSLSASVGDRVTITCKASQDVSI GVAWYQQKPGKAPKLLIYSASYRYTGVPSRFSGSGSGTDFTLTISSLQ PEDFATYYCQQYYIYPYTFGCGTKVEIKRTVAAPSVFIFPPSDEQLKS GTASVVCLLNNFYPREAKVQWKVDNALQSGNSQESVTEQDSKDSTY SLSSTLTLSKADYEKHKVYACEVTHQGLSSPVTKSFNRGECGGGGSG | 62 |
| | GGGSGGGGSEPKSSDKTHTCPPCPAPELLGGPSVFLFPPKPKDTLMIS RTPEVTCVVVDVSHEDPEVKFNWYVDGVEVHNAKTKPREEQYNST YRVVSVLTVLHQDWLNGKEYKCKVSNKALPAPIEKTISKAKGQPREP QVYTLPPCREEMTKNQVSLWCLVKGFYPSDIAVEWESNGQPENNYK TTPPVLDSDGSFFLYSKLTVDKSRWQQGNVFSCSVMHEALHNHYTQ KSLSLSPGK | |
| TL-L-PL-Knob | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGQGTKVEIKRTVAAPSVFIFPPDIQMTQSPSSLSASVGDRVTITCK ASQDVSIGVAWYQQKPGKAPKLLIYSASYRYTGVPSRFSGSGSGTDF TLTISSLQPEDFATYYCQQYYIYPYTFGQGTKVEIKRTVAAPSVFIFPP SDEQLKSGTASVVCLLNNFYPREAKVQWKVDNALQSGNSQESVTEQ DSKDSTYSLSSTLTLSKADYEKHKVYACEVTHQGLSSPVTKSFNRGE CGGGGSGGGGSGGGGSEPKSSDKTHTCPPCPAPELLGGPSVFLFPPKP KDTLMISRTPEVTCVVVDVSHEDPEVKFNWYVDGVEVHNAKTKPRE EQYNSTYRVVSVLTVLHQDWLNGKEYKCKVSNKALPAPIEKTISKA KGQPREPQVYTLPPCREEMTKNQVSLWCLVKGFYPSDIAVEWESNG QPENNYKTTPPVLDSDGSFFLYSKLTVDKSRWQQGNVFSCSVMHEA LHNHYTQKSLSLSPGK | 63 |
| TLC-L-PL-Knob | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGCGTKVEIKRTVAAPSVFIFPPDIQMTQSPSSLSASVGDRVTITCK ASQDVSIGVAWYQQKPGKAPKLLIYSASYRYTGVPSRFSGSGSGTDF TLTISSLQPEDFATYYCQQYYIYPYTFGQGTKVEIKRTVAAPSVFIFPP SDEQLKSGTASVVCLLNNFYPREAKVQWKVDNALQSGNSQESVTEQ DSKDSTYSLSSTLTLSKADYEKHKVYACEVTHQGLSSPVTKSFNRGE CGGGGSGGGGSGGGGSEPKSSDKTHTCPPCPAPELLGGPSVFLFPPKP KDTLMISRTPEVTCVVVDVSHEDPEVKFNWYVDGVEVHNAKTKPRE EQYNSTYRVVSVLTVLHQDWLNGKEYKCKVSNKALPAPIEKTISKA KGQPREPQVYTLPPCREEMTKNQVSLWCLVKGFYPSDIAVEWESNG QPENNYKTTPPVLDSDGSFFLYSKLTVDKSRWQQGNVFSCSVMHEA LHNHYTQKSLSLSPGK | 64 |
| TL-L-PLC-Knob | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGCGTKVEIKRTVAAPSVFIFPPDIQMTQSPSSLSASVGDRVTITCK ASQDVSIGVAWYQQKPGKAPKLLIYSASYRYTGVPSRFSGSGSGTDF TLTISSLQPEDFATYYCQQYYIYPYTFGQGTKVEIKRTVAAPSVFIFPP SDEQLKSGTASVVCLLNNFYPREAKVQWKVDNALQSGNSQESVTEQ DSKDSTYSLSSTLTLSKADYEKHKVYACEVTHQGLSSPVTKSFNRGE CGGGGSGGGGSGGGGSEPKSSDKTHTCPPCPAPELLGGPSVFLFPPKP KDTLMISRTPEVTCVVVDVSHEDPEVKFNWYVDGVEVHNAKTKPRE EQYNSTYRVVSVLTVLHQDWLNGKEYKCKVSNKALPAPIEKTISKA KGQPREPQVYTLPPCREEMTKNQVSLWCLVKGFYPSDIAVEWESNG QPENNYKTTPPVLDSDGSFFLYSKLTVDKSRWQQGNVFSCSVMHEA LHNHYTQKSLSLSPGK | 65 |
| TLC-L-PLC-Knob | DIQMTQSPSSLSASVGDRVTITCRASQDVNTAVAWYQQKPGKAPKL LIYSASFLYSGVPSRFSGSRSGTDFTLTISSLQPEDFATYYCQQHYTTP PTFGCGTKVEIKRTVAAPSVFIFPPDIQMTQSPSSLSASVGDRVTITCK ASQDVSIGVAWYQQKPGKAPKLLIYSASYRYTGVPSRFSGSGSGTDF TLTISSLQPEDFATYYCQQYYIYPYTFGCGTKVEIKRTVAAPSVFIFPP SDEQLKSGTASVVCLLNNFYPREAKVQWKVDNALQSGNSQESVTEQ DSKDSTYSLSSTLTLSKADYEKHKVYACEVTHQGLSSPVTKSFNRGE CGGGGSGGGGSGGGGSEPKSSDKTHTCPPCPAPELLGGPSVFLFPPKP KDTLMISRTPEVTCVVVDVSHEDPEVKFNWYVDGVEVHNAKTKPRE EQYNSTYRVVSVLTVLHQDWLNGKEYKCKVSNKALPAPIEKTISKA KGQPREPQVYTLPPCREEMTKNQVSLWCLVKGFYPSDIAVEWESNG QPENNYKTTPPVLDSDGSFFLYSKLTVDKSRWQQGNVFSCSVMHEA LHNHYTQKSLSLSPGK | 66 |

**[Table 33]**

| Name | Sequence | SEQ ID NO |
|---|---|---|
| VH-S-Linker | GCTAGCACAAAAGGACCT | 484 |
| VH-L-Linker | GCTAGCACAAAAGGACCTAGTGTTTTCCCACTGGCTCCA | 485 |
| VL-S-Linker | CGTACGGTGGCTGCTCCA | 486 |
| VL-L-Linker | CGTACGGTGGCTGCTCCATCCGTTTTTATCTTTCCCCCA | 487 |
| TH-S-PH-Knob | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAAGGGCTGG AATGGGTGGCCCGTATATATCCCACTAACGGTTACACTCGATATG CTGACTCTGTAAAGGGTCGATTTACTATATCCGCTGATACTAGCAA AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACAAAAGGACCTGAAGTACAGTTGGTGGAGTCTGGTGGAGG TCTTGTACAACCTGGCGGGAGTTTGCGGCTTTCCTGCGCTGCAAGC GGGTTTACCTTCACCGATTATACCATGGATTGGGTACGCCAAGCC CCTGGTAAGGGCCTTGAGTGGGTTGCCGATGTAAACCCTAATTCC GGAGGAAGTATCTATAATCAACGCTTCAAGGGCCGATTCACTCTG AGTGTGGATCGAAGCAAGAACACCTTGTACTTGCAGATGAATTCC TTGCGGGCTGAAGACACAGCCGTCTATTACTGCGCCCGAAATTTG GGGCCTTCATTCTATTTTGACTATTGGGGTCAGGGAACTCTGGTAA CTGTTTCAAGTGCTAGCACCAAAGGACCTAGTGTTTTTCCTCTTGC CCCTTCCTCAAAGTCTACCTCTGGGGGGACAGCCGCTCTGGGCTG CCTGGTCAAGGATTATTTCCCAGAGCCTGTCACTGTCAGTTGGAA CTCTGGAGCCTTGACTTCTGGTGTTCATACATTTCCTGCTGTCCTTC AAAGCAGCGGCTTGTACTCATTGTCTTCTGTTGTGACAGTACCCTC AAGCAGCCTCGGCACTCAGACATACATCTGCAATGTCAACCACAA ACCCTCAAATACAAAGGTAGATAAAAAAGTCGAACCAAAGTCTT GTGACAAAACTCACACGTGCCCACCGTGCCCAGCACCTGAACTCC TGGGGGGACCGTCAGTCTTCCTCTTCCCCCCAAAACCCAAGGACA CCCTCATGATCTCCCGGACCCCTGAGGTCACATGCGTGGTGGTGG | 488 |
| | ACGTGAGCCACGAAGACCCTGAGGTCAAGTTCAACTGGTACGTGG ACGGCGTGGAGGTGCATAATGCCAAGACAAAGCCGCGGGAGGAG CAGTACAACAGCACGTACCGTGTGGTCAGCGTCCTCACCGTCCTG CACCAGGACTGGCTGAATGGCAAGGAGTACAAGTGCAAGGTCTC CAACAAAGCCCTCCCAGCCCCCATCGAGAAAACCATCTCCAAAGC CAAAGGGCAGCCCCGAGAACCACAGGTGTACACCCTGCCCCCATG CCGGGAGGAGATGACCAAGAACCAGGTCAGCCTGTGGTGCCTGG TCAAAGGCTTCTATCCCAGCGACATCGCCGTGGAGTGGGAGAGCA ATGGGCAGCCGGAGAACAACTACAAGACCACGCCTCCCGTGCTG GACTCCGACGGCTCCTTCTTCCTCTACAGCAAGCTCACCGTGGAC AAGAGCAGGTGGCAGCAGGGGAACGTCTTCTCATGCTCCGTGATG CATGAGGCTCTGCACAACCACTACACGCAGAAGAGCCTCTCCCTG TCTCCGGGTAAA | |
| THC-S-PH-Knob | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGG AATGGGTGGCCCGTATATATCCCACTAACGGTTACACTCGATATG CTGACTCTGTAAAGGGTCGATTTACTATATCCGCTGATACTAGCAA AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACAAAAGGACCTGAAGTACAGTTGGTGGAGTCTGGTGGAGG TCTTGTACAACCTGGCGGGAGTTTGCGGCTTTCCTGCGCTGCAAGC GGGTTTACCTTCACCGATTATACCATGGATTGGGTACGCCAAGCC CCTGGTAAGGGCCTTGAGTGGGTTGCCGATGTAAACCCTAATTCC GGAGGAAGTATCTATAATCAACGCTTCAAGGGCCGATTCACTCTG AGTGTGGATCGAAGCAAGAACACCTTGTACTTGCAGATGAATTCC TTGCGGGCTGAAGACACAGCCGTCTATTACTGCGCCCGAAATTTG GGGCCTTCATTCTATTTTGACTATTGGGGTCAGGGAACTCTGGTAA CTGTTTCAAGTGCTAGCACCAAAGGACCTAGTGTTTTTCCTCTTGC CCCTTCCTCAAAGTCTACCTCTGGGGGGACAGCCGCTCTGGGCTG CCTGGTCAAGGATTATTTCCCAGAGCCTGTCACTGTCAGTTGGAA CTCTGGAGCCTTGACTTCTGGTGTTCATACATTTCCTGCTGTCCTTC AAAGCAGCGGCTTGTACTCATTGTCTTCTGTTGTGACAGTACCCTC AAGCAGCCTCGGCACTCAGACATACATCTGCAATGTCAACCACAA ACCCTCAAATACAAAGGTAGATAAAAAAGTCGAACCAAAGTCTT GTGACAAAACTCACACGTGCCCACCGTGCCCAGCACCTGAACTCC TGGGGGGACCGTCAGTCTTCCTCTTCCCCCCAAAACCCAAGGACA CCCTCATGATCTCCCGGACCCCTGAGGTCACATGCGTGGTGGTGG ACGTGAGCCACGAAGACCCTGAGGTCAAGTTCAACTGGTACGTGG ACGGCGTGGAGGTGCATAATGCCAAGACAAAGCCGCGGGAGGAG CAGTACAACAGCACGTACCGTGTGGTCAGCGTCCTCACCGTCCTG CACCAGGACTGGCTGAATGGCAAGGAGTACAAGTGCAAGGTCTC CAACAAAGCCCTCCCAGCCCCCATCGAGAAAACCATCTCCAAAGC CAAAGGGCAGCCCCGAGAACCACAGGTGTACACCCTGCCCCCATG | 489 |
| | CCGGGAGGAGATGACCAAGAACCAGGTCAGCCTGTGGTGCCTGG TCAAAGGCTTCTATCCCAGCGACATCGCCGTGGAGTGGGAGAGCA ATGGGCAGCCGGAGAACAACTACAAGACCACGCCTCCCGTGCTG GACTCCGACGGCTCCTTCTTCCTCTACAGCAAGCTCACCGTGGAC AAGAGCAGGTGGCAGCAGGGGAACGTCTTCTCATGCTCCGTGATG CATGAGGCTCTGCACAACCACTACACGCAGAAGAGCCTCTCCCTG TCTCCGGGTAAA | |
| TH-S-PHC-Knob | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAAGGGCTGG AATGGGTGGCCCGTATATATCCCACTAACGGTTACACTCGATATG CTGACTCTGTAAAGGGTCGATTTACTATATCCGCTGATACTAGCAA AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACAAAAGGACCTGAAGTACAGTTGGTGGAGTCTGGTGGAGG TCTTGTACAACCTGGCGGGAGTTTGCGGCTTTCCTGCGCTGCAAGC GGGTTTACCTTCACCGATTATACCATGGATTGGGTACGCCAAGCC CCTGGTAAGTGCCTTGAGTGGGTTGCCGATGTAAACCCTAATTCC GGAGGAAGTATCTATAATCAACGCTTCAAGGGCCGATTCACTCTG AGTGTGGATCGAAGCAAGAACACCTTGTACTTGCAGATGAATTCC TTGCGGGCTGAAGACACAGCCGTCTATTACTGCGCCCGAAATTTG GGGCCTTCATTCTATTTTGACTATTGGGGTCAGGGAACTCTGGTAA CTGTTTCAAGTGCTAGCACCAAAGGACCTAGTGTTTTTCCTCTTGC CCCTTCCTCAAAGTCTACCTCTGGGGGGACAGCCGCTCTGGGCTG CCTGGTCAAGGATTATTTCCCAGAGCCTGTCACTGTCAGTTGGAA CTCTGGAGCCTTGACTTCTGGTGTTCATACATTTCCTGCTGTCCTTC AAAGCAGCGGCTTGTACTCATTGTCTTCTGTTGTGACAGTACCCTC AAGCAGCCTCGGCACTCAGACATACATCTGCAATGTCAACCACAA ACCCTCAAATACAAAGGTAGATAAAAAAGTCGAACCAAAGTCTT GTGACAAAACTCACACGTGCCCACCGTGCCCAGCACCTGAACTCC TGGGGGGACCGTCAGTCTTCCTCTTCCCCCCAAAACCCAAGGACA CCCTCATGATCTCCCGGACCCCTGAGGTCACATGCGTGGTGGTGG ACGTGAGCCACGAAGACCCTGAGGTCAAGTTCAACTGGTACGTGG ACGGCGTGGAGGTGCATAATGCCAAGACAAAGCCGCGGGAGGAG CAGTACAACAGCACGTACCGTGTGGTCAGCGTCCTCACCGTCCTG CACCAGGACTGGCTGAATGGCAAGGAGTACAAGTGCAAGGTCTC CAACAAAGCCCTCCCAGCCCCCATCGAGAAAACCATCTCCAAAGC CAAAGGGCAGCCCCGAGAACCACAGGTGTACACCCTGCCCCCATG CCGGGAGGAGATGACCAAGAACCAGGTCAGCCTGTGGTGCCTGG TCAAAGGCTTCTATCCCAGCGACATCGCCGTGGAGTGGGAGAGCA ATGGGCAGCCGGAGAACAACTACAAGACCACGCCTCCCGTGCTG GACTCCGACGGCTCCTTCTTCCTCTACAGCAAGCTCACCGTGGAC AAGAGCAGGTGGCAGCAGGGGAACGTCTTCTCATGCTCCGTGATG CATGAGGCTCTGCACAACCACTACACGCAGAAGAGCCTCTCCCTG | 490 |
| | TCTCCGGGTAAA | |
| THC-S-PHC-Knob | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGG AATGGGTGGCCCGTATATATCCCACTAACGGTTACACTCGATATG CTGACTCTGTAAAGGGTCGATTTACTATATCCGCTGATACTAGCAA AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACAAAAGGACCTAGTGTTTTCCCACTGGCTCCAGAAGTACAG TTGGTGGAGTCTGGTGGAGGTCTTGTACAACCTGGCGGGAGTTTG CGGCTTTCCTGCGCTGCAAGCGGGTTTACCTTCACCGATTATACCA TGGATTGGGTACGCCAAGCCCCTGGTAAGTGCCTTGAGTGGGTTG CCGATGTAAACCCTAATTCCGGAGGAAGTATCTATAATCAACGCT TCAAGGGCCGATTCACTCTGAGTGTGGATCGAAGCAAGAACACCT TGTACTTGCAGATGAATTCCTTGCGGGCTGAAGACACAGCCGTCT ATTACTGCGCCCGAAATTTGGGGCCTTCATTCTATTTTGACTATTG GGGTCAGGGAACTCTGGTAACTGTTTCAAGTGCTAGCACCAAAGG ACCTAGTGTTTTTCCTCTTGCCCCTTCCTCAAAGTCTACCTCTGGG GGGACAGCCGCTCTGGGCTGCCTGGTCAAGGATTATTTCCCAGAG CCTGTCACTGTCAGTTGGAACTCTGGAGCCTTGACTTCTGGTGTTC ATACATTTCCTGCTGTCCTTCAAAGCAGCGGCTTGTACTCATTGTC TTCTGTTGTGACAGTACCCTCAAGCAGCCTCGGCACTCAGACATA CATCTGCAATGTCAACCACAAACCCTCAAATACAAAGGTAGATAA AAAAGTCGAACCAAAGTCTTGTGACAAAACTCACACGTGCCCACC GTGCCCAGCACCTGAACTCCTGGGGGGACCGTCAGTCTTCCTCTTC CCCCCAAAACCCAAGGACACCCTCATGATCTCCCGGACCCCTGAG GTCACATGCGTGGTGGTGGACGTGAGCCACGAAGACCCTGAGGTC AAGTTCAACTGGTACGTGGACGGCGTGGAGGTGCATAATGCCAAG ACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGG TGTACACCCTGCCCCCATGCCGGGAGGAGATGACCAAGAACCAG GTCAGCCTGTGGTGCCTGGTCAAAGGCTTCTATCCCAGCGACATC GCCGTGGAGTGGGAGAGCAATGGGCAGCCGGAGAACAACTACAA GACCACGCCTCCCGTGCTGGACTCCGACGGCTCCTTCTTCCTCTAC AGCAAGCTCACCGTGGACAAGAGCAGGTGGCAGCAGGGGAACGT CTTCTCATGCTCCGTGATGCATGAGGCTCTGCACAACCACTACAC GCAGAAGAGCCTCTCCCTGTCTCCGGGTAAA | 491 |
| TH-L-PH-Knob | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAAGGGCTGG AATGGGTGGCCCGTATATATCCCACTAACGGTTACACTCGATATG CTGACTCTGTAAAGGGTCGATTTACTATATCCGCTGATACTAGCAA | 492 |
| | AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACAAAAGGACCTAGTGTTTTCCCACTGGCTCCAGAAGTACAG TTGGTGGAGTCTGGTGGAGGTCTTGTACAACCTGGCGGGAGTTTG CGGCTTTCCTGCGCTGCAAGCGGGTTTACCTTCACCGATTATACCA TGGATTGGGTACGCCAAGCCCCTGGTAAGGGCCTTGAGTGGGTTG CCGATGTAAACCCTAATTCCGGAGGAAGTATCTATAATCAACGCT TCAAGGGCCGATTCACTCTGAGTGTGGATCGAAGCAAGAACACCT TGTACTTGCAGATGAATTCCTTGCGGGCTGAAGACACAGCCGTCT ATTACTGCGCCCGAAATTTGGGGCCTTCATTCTATTTTGACTATTG GGGTCAGGGAACTCTGGTAACTGTTTCAAGTGCTAGCACCAAAGG ACCTAGTGTTTTTCCTCTTGCCCCTTCCTCAAAGTCTACCTCTGGG GGGACAGCCGCTCTGGGCTGCCTGGTCAAGGATTATTTCCCAGAG CCTGTCACTGTCAGTTGGAACTCTGGAGCCTTGACTTCTGGTGTTC ATACATTTCCTGCTGTCCTTCAAAGCAGCGGCTTGTACTCATTGTC TTCTGTTGTGACAGTACCCTCAAGCAGCCTCGGCACTCAGACATA CATCTGCAATGTCAACCACAAACCCTCAAATACAAAGGTAGATAA AAAAGTCGAACCAAAGTCTTGTGACAAAACTCACACGTGCCCACC GTGCCCAGCACCTGAACTCCTGGGGGGACCGTCAGTCTTCCTCTTC CCCCCAAAACCCAAGGACACCCTCATGATCTCCCGGACCCCTGAG GTCACATGCGTGGTGGTGGACGTGAGCCACGAAGACCCTGAGGTC AAGTTCAACTGGTACGTGGACGGCGTGGAGGTGCATAATGCCAAG ACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGG TGTACACCCTGCCCCCATGCCGGGAGGAGATGACCAAGAACCAG GTCAGCCTGTGGTGCCTGGTCAAAGGCTTCTATCCCAGCGACATC GCCGTGGAGTGGGAGAGCAATGGGCAGCCGGAGAACAACTACAA GACCACGCCTCCCGTGCTGGACTCCGACGGCTCCTTCTTCCTCTAC AGCAAGCTCACCGTGGACAAGAGCAGGTGGCAGCAGGGGAACGT CTTCTCATGCTCCGTGATGCATGAGGCTCTGCACAACCACTACAC GCAGAAGAGCCTCTCCCTGTCTCCGGGTAAA | |
| THC-L-PH-Knob | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGG AATGGGTGGCCCGTATATATCCCACTAACGGTTACACTCGATATG CTGACTCTGTAAAGGGTCGATTTACTATATCCGCTGATACTAGCAA AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACAAAAGGACCTAGTGTTTTCCCACTGGCTCCAGAAGTACAG TTGGTGGAGTCTGGTGGAGGTCTTGTACAACCTGGCGGGAGTTTG CGGCTTTCCTGCGCTGCAAGCGGGTTTACCTTCACCGATTATACCA | 493 |
| | TGGATTGGGTACGCCAAGCCCCTGGTAAGGGCCTTGAGTGGGTTG CCGATGTAAACCCTAATTCCGGAGGAAGTATCTATAATCAACGCT TCAAGGGCCGATTCACTCTGAGTGTGGATCGAAGCAAGAACACCT TGTACTTGCAGATGAATTCCTTGCGGGCTGAAGACACAGCCGTCT ATTACTGCGCCCGAAATTTGGGGCCTTCATTCTATTTTGACTATTG GGGTCAGGGAACTCTGGTAACTGTTTCAAGTGCTAGCACCAAAGG ACCTAGTGTTTTTCCTCTTGCCCCTTCCTCAAAGTCTACCTCTGGG GGGACAGCCGCTCTGGGCTGCCTGGTCAAGGATTATTTCCCAGAG CCTGTCACTGTCAGTTGGAACTCTGGAGCCTTGACTTCTGGTGTTC ATACATTTCCTGCTGTCCTTCAAAGCAGCGGCTTGTACTCATTGTC TTCTGTTGTGACAGTACCCTCAAGCAGCCTCGGCACTCAGACATA CATCTGCAATGTCAACCACAAACCCTCAAATACAAAGGTAGATAA AAAAGTCGAACCAAAGTCTTGTGACAAAACTCACACGTGCCCACC GTGCCCAGCACCTGAACTCCTGGGGGGACCGTCAGTCTTCCTCTTC CCCCCAAAACCCAAGGACACCCTCATGATCTCCCGGACCCCTGAG GTCACATGCGTGGTGGTGGACGTGAGCCACGAAGACCCTGAGGTC AAGTTCAACTGGTACGTGGACGGCGTGGAGGTGCATAATGCCAAG ACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGG TGTACACCCTGCCCCCATGCCGGGAGGAGATGACCAAGAACCAG GTCAGCCTGTGGTGCCTGGTCAAAGGCTTCTATCCCAGCGACATC GCCGTGGAGTGGGAGAGCAATGGGCAGCCGGAGAACAACTACAA GACCACGCCTCCCGTGCTGGACTCCGACGGCTCCTTCTTCCTCTAC AGCAAGCTCACCGTGGACAAGAGCAGGTGGCAGCAGGGGAACGT CTTCTCATGCTCCGTGATGCATGAGGCTCTGCACAACCACTACAC GCAGAAGAGCCTCTCCCTGTCTCCGGGTAAA | |
| TH-L-PHC-Knob | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAAGGGCTGG AATGGGTGGCCCGTATATATCCCACTAACGGTTACACTCGATATG CTGACTCTGTAAAGGGTCGATTTACTATATCCGCTGATACTAGCAA AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACAAAAGGACCTAGTGTTTTCCCACTGGCTCCAGAAGTACAG TTGGTGGAGTCTGGTGGAGGTCTTGTACAACCTGGCGGGAGTTTG CGGCTTTCCTGCGCTGCAAGCGGGTTTACCTTCACCGATTATACCA TGGATTGGGTACGCCAAGCCCCTGGTAAGTGCCTTGAGTGGGTTG CCGATGTAAACCCTAATTCCGGAGGAAGTATCTATAATCAACGCT TCAAGGGCCGATTCACTCTGAGTGTGGATCGAAGCAAGAACACCT TGTACTTGCAGATGAATTCCTTGCGGGCTGAAGACACAGCCGTCT ATTACTGCGCCCGAAATTTGGGGCCTTCATTCTATTTTGACTATTG GGGTCAGGGAACTCTGGTAACTGTTTCAAGTGCTAGCACCAAAGG | 494 |
| | ACCTAGTGTTTTTCCTCTTGCCCCTTCCTCAAAGTCTACCTCTGGG GGGACAGCCGCTCTGGGCTGCCTGGTCAAGGATTATTTCCCAGAG CCTGTCACTGTCAGTTGGAACTCTGGAGCCTTGACTTCTGGTGTTC ATACATTTCCTGCTGTCCTTCAAAGCAGCGGCTTGTACTCATTGTC TTCTGTTGTGACAGTACCCTCAAGCAGCCTCGGCACTCAGACATA CATCTGCAATGTCAACCACAAACCCTCAAATACAAAGGTAGATAA AAAAGTCGAACCAAAGTCTTGTGACAAAACTCACACGTGCCCACC GTGCCCAGCACCTGAACTCCTGGGGGGACCGTCAGTCTTCCTCTTC CCCCCAAAACCCAAGGACACCCTCATGATCTCCCGGACCCCTGAG GTCACATGCGTGGTGGTGGACGTGAGCCACGAAGACCCTGAGGTC AAGTTCAACTGGTACGTGGACGGCGTGGAGGTGCATAATGCCAAG ACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGG TGTACACCCTGCCCCCATGCCGGGAGGAGATGACCAAGAACCAG GTCAGCCTGTGGTGCCTGGTCAAAGGCTTCTATCCCAGCGACATC GCCGTGGAGTGGGAGAGCAATGGGCAGCCGGAGAACAACTACAA GACCACGCCTCCCGTGCTGGACTCCGACGGCTCCTTCTTCCTCTAC AGCAAGCTCACCGTGGACAAGAGCAGGTGGCAGCAGGGGAACGT CTTCTCATGCTCCGTGATGCATGAGGCTCTGCACAACCACTACAC GCAGAAGAGCCTCTCCCTGTCTCCGGGTAAA | |
| THC-L-PHC-Knob | GAAGTTCAACTTGTGGAGTCTGGAGGCGGTTTGGTACAACCTGGA GGTTCACTTCGTTTGTCATGTGCTGCCTCTGGGTTTAATATCAAGG ATACTTATATTCATTGGGTTCGCCAGGCACCCGGAAAATGCCTGG AATGGGTGGCCCGTATATATCCCACTAACGGTTACACTCGATATG CTGACTCTGTAAAGGGTCGATTTACTATATCCGCTGATACTAGCAA AAACACTGCCTACCTCCAGATGAACAGTCTGCGCGCCGAGGATAC AGCAGTTTATTATTGTTCCCGATGGGGAGGTGACGGGTTCTATGCC ATGGACTACTGGGGTCAAGGGACACTGGTAACCGTTTCTTCTGCT AGCACAAAAGGACCTAGTGTTTTCCCACTGGCTCCAGAAGTACAG TTGGTGGAGTCTGGTGGAGGTCTTGTACAACCTGGCGGGAGTTTG CGGCTTTCCTGCGCTGCAAGCGGGTTTACCTTCACCGATTATACCA TGGATTGGGTACGCCAAGCCCCTGGTAAGTGCCTTGAGTGGGTTG CCGATGTAAACCCTAATTCCGGAGGAAGTATCTATAATCAACGCT TCAAGGGCCGATTCACTCTGAGTGTGGATCGAAGCAAGAACACCT TGTACTTGCAGATGAATTCCTTGCGGGCTGAAGACACAGCCGTCT ATTACTGCGCCCGAAATTTGGGGCCTTCATTCTATTTTGACTATTG GGGTCAGGGAACTCTGGTAACTGTTTCAAGTGCTAGCACCAAAGG ACCTAGTGTTTTTCCTCTTGCCCCTTCCTCAAAGTCTACCTCTGGG GGGACAGCCGCTCTGGGCTGCCTGGTCAAGGATTATTTCCCAGAG CCTGTCACTGTCAGTTGGAACTCTGGAGCCTTGACTTCTGGTGTTC ATACATTTCCTGCTGTCCTTCAAAGCAGCGGCTTGTACTCATTGTC TTCTGTTGTGACAGTACCCTCAAGCAGCCTCGGCACTCAGACATA CATCTGCAATGTCAACCACAAACCCTCAAATACAAAGGTAGATAA | 495 |
| | AAAAGTCGAACCAAAGTCTTGTGACAAAACTCACACGTGCCCACC GTGCCCAGCACCTGAACTCCTGGGGGGACCGTCAGTCTTCCTCTTC CCCCCAAAACCCAAGGACACCCTCATGATCTCCCGGACCCCTGAG GTCACATGCGTGGTGGTGGACGTGAGCCACGAAGACCCTGAGGTC AAGTTCAACTGGTACGTGGACGGCGTGGAGGTGCATAATGCCAAG ACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGG TGTACACCCTGCCCCCATGCCGGGAGGAGATGACCAAGAACCAG GTCAGCCTGTGGTGCCTGGTCAAAGGCTTCTATCCCAGCGACATC GCCGTGGAGTGGGAGAGCAATGGGCAGCCGGAGAACAACTACAA GACCACGCCTCCCGTGCTGGACTCCGACGGCTCCTTCTTCCTCTAC AGCAAGCTCACCGTGGACAAGAGCAGGTGGCAGCAGGGGAACGT CTTCTCATGCTCCGTGATGCATGAGGCTCTGCACAACCACTACAC GCAGAAGAGCCTCTCCCTGTCTCCGGGTAAA | |
| TL-S-PL-Knob | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGACAGGGCACAAAAGTAGAGATTA AGCGTACGGTGGCTGCTCCAGATATTCAAATGACCCAAAGTCCAA GTTCCCTTTCAGCATCTGTCGGTGATCGAGTCACTATAACTTGTAA GGCCAGCCAAGACGTTAGCATAGGAGTAGCATGGTACCAACAAA AGCCTGGGAAGGCTCCCAAACTTCTCATTTATTCTGCTTCCTACCG ATATACTGGTGTCCCAAGTAGATTTTCTGGCAGCGGATCTGGAAC TGATTTTACATTGACTATCAGCTCCCTCCAGCCTGAGGACTTCGCT ACTTATTACTGCCAACAGTACTACATTTATCCCTATACATTCGGTC AAGGGACCAAAGTAGAGATCAAACGTACGGTGGCAGCTCCCAGC GTTTTTATCTTTCCCCCATCCGACGAGCAGCTCAAGAGTGGCACTG CCTCTGTAGTTTGTTTGCTGAATAACTTCTATCCACGTGAAGCAAA AGTACAGTGGAAGGTCGATAATGCCCTTCAGAGCGGTAACAGCCA AGAAAGTGTTACCGAGCAAGATTCCAAAGATTCCACTTACAGTCT GTCCAGCACATTGACACTGAGTAAGGCTGATTACGAAAAACACAA GGTGTACGCATGCGAGGTGACACACCAAGGTCTTTCATCTCCTGT AACTAAGAGCTTTAACCGGGGAGAATGTGGTGGTGGGGGCAGCG GGGGCGGAGGTAGTGGAGGCGGCGGTAGTGAACCAAAGAGTAGT GACAAAACTCACACGTGCCCACCGTGCCCAGCACCTGAACTCCTG GGGGGACCGTCAGTCTTCCTCTTCCCCCCAAAACCCAAGGACACC CTCATGATCTCCCGGACCCCTGAGGTCACATGCGTGGTGGTGGAC GTGAGCCACGAAGACCCTGAGGTCAAGTTCAACTGGTACGTGGAC GGCGTGGAGGTGCATAATGCCAAGACAAAGCCGCGGGAGGAGCA GTACAACAGCACGTACCGTGTGGTCAGCGTCCTCACCGTCCTGCA | 496 |
| | CCAGGACTGGCTGAATGGCAAGGAGTACAAGTGCAAGGTCTCCA ACAAAGCCCTCCCAGCCCCCATCGAGAAAACCATCTCCAAAGCCA AAGGGCAGCCCCGAGAACCACAGGTGTACACCCTGCCCCCATGCC GGGAGGAGATGACCAAGAACCAGGTCAGCCTGTGGTGCCTGGTC AAAGGCTTCTATCCCAGCGACATCGCCGTGGAGTGGGAGAGCAAT GGGCAGCCGGAGAACAACTACAAGACCACGCCTCCCGTGCTGGA CTCCGACGGCTCCTTCTTCCTCTACAGCAAGCTCACCGTGGACAA GAGCAGGTGGCAGCAGGGGAACGTCTTCTCATGCTCCGTGATGCA TGAGGCTCTGCACAACCACTACACGCAGAAGAGCCTCTCCCTGTC TCCGGGTAAA | |
| TLC-S-PL-Knob | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGATGTGGCACAAAAGTAGAGATTA AGCGTACGGTGGCTGCTCCAGATATTCAAATGACCCAAAGTCCAA GTTCCCTTTCAGCATCTGTCGGTGATCGAGTCACTATAACTTGTAA GGCCAGCCAAGACGTTAGCATAGGAGTAGCATGGTACCAACAAA AGCCTGGGAAGGCTCCCAAACTTCTCATTTATTCTGCTTCCTACCG ATATACTGGTGTCCCAAGTAGATTTTCTGGCAGCGGATCTGGAAC TGATTTTACATTGACTATCAGCTCCCTCCAGCCTGAGGACTTCGCT ACTTATTACTGCCAACAGTACTACATTTATCCCTATACATTCGGTC AAGGGACCAAAGTAGAGATCAAACGTACGGTGGCAGCTCCCAGC GTTTTTATCTTTCCCCCATCCGACGAGCAGCTCAAGAGTGGCACTG CCTCTGTAGTTTGTTTGCTGAATAACTTCTATCCACGTGAAGCAAA AGTACAGTGGAAGGTCGATAATGCCCTTCAGAGCGGTAACAGCCA AGAAAGTGTTACCGAGCAAGATTCCAAAGATTCCACTTACAGTCT GTCCAGCACATTGACACTGAGTAAGGCTGATTACGAAAAACACAA GGTGTACGCATGCGAGGTGACACACCAAGGTCTTTCATCTCCTGT AACTAAGAGCTTTAACCGGGGAGAATGTGGTGGTGGGGGCAGCG GGGGCGGAGGTAGTGGAGGCGGCGGTAGTGAACCAAAGAGTAGT GACAAAACTCACACGTGCCCACCGTGCCCAGCACCTGAACTCCTG GGGGGACCGTCAGTCTTCCTCTTCCCCCCAAAACCCAAGGACACC CTCATGATCTCCCGGACCCCTGAGGTCACATGCGTGGTGGTGGAC GTGAGCCACGAAGACCCTGAGGTCAAGTTCAACTGGTACGTGGAC GGCGTGGAGGTGCATAATGCCAAGACAAAGCCGCGGGAGGAGCA GTACAACAGCACGTACCGTGTGGTCAGCGTCCTCACCGTCCTGCA CCAGGACTGGCTGAATGGCAAGGAGTACAAGTGCAAGGTCTCCA ACAAAGCCCTCCCAGCCCCCATCGAGAAAACCATCTCCAAAGCCA AAGGGCAGCCCCGAGAACCACAGGTGTACACCCTGCCCCCATGCC GGGAGGAGATGACCAAGAACCAGGTCAGCCTGTGGTGCCTGGTC AAAGGCTTCTATCCCAGCGACATCGCCGTGGAGTGGGAGAGCAAT GGGCAGCCGGAGAACAACTACAAGACCACGCCTCCCGTGCTGGA | 497 |
| | CTCCGACGGCTCCTTCTTCCTCTACAGCAAGCTCACCGTGGACAA GAGCAGGTGGCAGCAGGGGAACGTCTTCTCATGCTCCGTGATGCA TGAGGCTCTGCACAACCACTACACGCAGAAGAGCCTCTCCCTGTC TCCGGGTAAA | |
| TL-S-PLC-Knob | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGACAGGGCACAAAAGTAGAGATTA AGCGTACGGTGGCTGCTCCAGATATTCAAATGACCCAAAGTCCAA GTTCCCTTTCAGCATCTGTCGGTGATCGAGTCACTATAACTTGTAA GGCCAGCCAAGACGTTAGCATAGGAGTAGCATGGTACCAACAAA AGCCTGGGAAGGCTCCCAAACTTCTCATTTATTCTGCTTCCTACCG ATATACTGGTGTCCCAAGTAGATTTTCTGGCAGCGGATCTGGAAC TGATTTTACATTGACTATCAGCTCCCTCCAGCCTGAGGACTTCGCT ACTTATTACTGCCAACAGTACTACATTTATCCCTATACATTCGGTT GTGGGACCAAAGTAGAGATCAAACGTACGGTGGCAGCTCCCAGC GTTTTTATCTTTCCCCCATCCGACGAGCAGCTCAAGAGTGGCACTG CCTCTGTAGTTTGTTTGCTGAATAACTTCTATCCACGTGAAGCAAA AGTACAGTGGAAGGTCGATAATGCCCTTCAGAGCGGTAACAGCCA AGAAAGTGTTACCGAGCAAGATTCCAAAGATTCCACTTACAGTCT GTCCAGCACATTGACACTGAGTAAGGCTGATTACGAAAAACACAA GGTGTACGCATGCGAGGTGACACACCAAGGTCTTTCATCTCCTGT AACTAAGAGCTTTAACCGGGGAGAATGTGGTGGTGGGGGCAGCG GGGGCGGAGGTAGTGGAGGCGGCGGTAGTGAACCAAAGAGTAGT GACAAAACTCACACGTGCCCACCGTGCCCAGCACCTGAACTCCTG GGGGGACCGTCAGTCTTCCTCTTCCCCCCAAAACCCAAGGACACC CTCATGATCTCCCGGACCCCTGAGGTCACATGCGTGGTGGTGGAC GTGAGCCACGAAGACCCTGAGGTCAAGTTCAACTGGTACGTGGAC GGCGTGGAGGTGCATAATGCCAAGACAAAGCCGCGGGAGGAGCA GTACAACAGCACGTACCGTGTGGTCAGCGTCCTCACCGTCCTGCA CCAGGACTGGCTGAATGGCAAGGAGTACAAGTGCAAGGTCTCCA ACAAAGCCCTCCCAGCCCCCATCGAGAAAACCATCTCCAAAGCCA AAGGGCAGCCCCGAGAACCACAGGTGTACACCCTGCCCCCATGCC GGGAGGAGATGACCAAGAACCAGGTCAGCCTGTGGTGCCTGGTC AAAGGCTTCTATCCCAGCGACATCGCCGTGGAGTGGGAGAGCAAT GGGCAGCCGGAGAACAACTACAAGACCACGCCTCCCGTGCTGGA CTCCGACGGCTCCTTCTTCCTCTACAGCAAGCTCACCGTGGACAA GAGCAGGTGGCAGCAGGGGAACGTCTTCTCATGCTCCGTGATGCA TGAGGCTCTGCACAACcactacACGCAGAAGAGCCTCTCCCTGTCTCC GGGTAAA | 498 |
| TLC-S-PLC-Knob | GACATTCAGATGACCCAATCCCCCTCTAGCCTTTCAGCCTCCGTAG GGGACAGAGTTACAATAACCTGTAGGGCTAGTCAGGACGTTAATA | 499 |
| | CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGATGTGGCACAAAAGTAGAGATTA AGCGTACGGTGGCTGCTCCAGATATTCAAATGACCCAAAGTCCAA GTTCCCTTTCAGCATCTGTCGGTGATCGAGTCACTATAACTTGTAA GGCCAGCCAAGACGTTAGCATAGGAGTAGCATGGTACCAACAAA AGCCTGGGAAGGCTCCCAAACTTCTCATTTATTCTGCTTCCTACCG ATATACTGGTGTCCCAAGTAGATTTTCTGGCAGCGGATCTGGAAC TGATTTTACATTGACTATCAGCTCCCTCCAGCCTGAGGACTTCGCT ACTTATTACTGCCAACAGTACTACATTTATCCCTATACATTCGGTT GTGGGACCAAAGTAGAGATCAAACGTACGGTGGCAGCTCCCAGC GTTTTTATCTTTCCCCCATCCGACGAGCAGCTCAAGAGTGGCACTG CCTCTGTAGTTTGTTTGCTGAATAACTTCTATCCACGTGAAGCAAA AGTACAGTGGAAGGTCGATAATGCCCTTCAGAGCGGTAACAGCCA AGAAAGTGTTACCGAGCAAGATTCCAAAGATTCCACTTACAGTCT GTCCAGCACATTGACACTGAGTAAGGCTGATTACGAAAAACACAA GGTGTACGCATGCGAGGTGACACACCAAGGTCTTTCATCTCCTGT AACTAAGAGCTTTAACCGGGGAGAATGTGGTGGTGGGGGCAGCG GGGGCGGAGGTAGTGGAGGCGGCGGTAGTGAACCAAAGAGTAGT GACAAAACTCACACGTGCCCACCGTGCCCAGCACCTGAACTCCTG GGGGGACCGTCAGTCTTCCTCTTCCCCCCAAAACCCAAGGACACC CTCATGATCTCCCGGACCCCTGAGGTCACATGCGTGGTGGTGGAC GTGAGCCACGAAGACCCTGAGGTCAAGTTCAACTGGTACGTGGAC GGCGTGGAGGTGCATAATGCCAAGACAAAGCCGCGGGAGGAGCA GTACAACAGCACGTACCGTGTGGTCAGCGTCCTCACCGTCCTGCA CCAGGACTGGCTGAATGGCAAGGAGTACAAGTGCAAGGTCTCCA ACAAAGCCCTCCCAGCCCCCATCGAGAAAACCATCTCCAAAGCCA AAGGGCAGCCCCGAGAACCACAGGTGTACACCCTGCCCCCATGCC GGGAGGAGATGACCAAGAACCAGGTCAGCCTGTGGTGCCTGGTC AAAGGCTTCTATCCCAGCGACATCGCCGTGGAGTGGGAGAGCAAT GGGCAGCCGGAGAACAACTACAAGACCACGCCTCCCGTGCTGGA CTCCGACGGCTCCTTCTTCCTCTACAGCAAGCTCACCGTGGACAA GAGCAGGTGGCAGCAGGGGAACGTCTTCTCATGCTCCGTGATGCA TGAGGCTCTGCACAACCACTACACGCAGAAGAGCCTCTCCCTGTC TCCGGGTAAA | |
| TL-L-PL-Knob | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGACAGGGCACAAAAGTAGAGATTA AGCGTACGGTGGCTGCTCCATCCGTTTTTATCTTTCCCCCAGATAT | 500 |
| | TCAAATGACCCAAAGTCCAAGTTCCCTTTCAGCATCTGTCGGTGAT CGAGTCACTATAACTTGTAAGGCCAGCCAAGACGTTAGCATAGGA GTAGCATGGTACCAACAAAAGCCTGGGAAGGCTCCCAAACTTCTC ATTTATTCTGCTTCCTACCGATATACTGGTGTCCCAAGTAGATTTT CTGGCAGCGGATCTGGAACTGATTTTACATTGACTATCAGCTCCCT CCAGCCTGAGGACTTCGCTACTTATTACTGCCAACAGTACTACATT TATCCCTATACATTCGGTCAAGGGACCAAAGTAGAGATCAAACGT ACGGTGGCAGCTCCCAGCGTTTTTATCTTTCCCCCATCCGACGAGC AGCTCAAGAGTGGCACTGCCTCTGTAGTTTGTTTGCTGAATAACTT CTATCCACGTGAAGCAAAAGTACAGTGGAAGGTCGATAATGCCCT TCAGAGCGGTAACAGCCAAGAAAGTGTTACCGAGCAAGATTCCA AAGATTCCACTTACAGTCTGTCCAGCACATTGACACTGAGTAAGG CTGATTACGAAAAACACAAGGTGTACGCATGCGAGGTGACACAC CAAGGTCTTTCATCTCCTGTAACTAAGAGCTTTAACCGGGGAGAA TGTGGTGGTGGGGGCAGCGGGGGCGGAGGTAGTGGAGGCGGCGG TAGTGAACCAAAGAGTAGTGACAAAACTCACACGTGCCCACCGTG CCCAGCACCTGAACTCCTGGGGGGACCGTCAGTCTTCCTCTTCCCC CCAAAACCCAAGGACACCCTCATGATCTCCCGGACCCCTGAGGTC ACATGCGTGGTGGTGGACGTGAGCCACGAAGACCCTGAGGTCAA GTTCAACTGGTACGTGGACGGCGTGGAGGTGCATAATGCCAAGAC AAAGCCGCGGGAGGAGCAGTACAACAGCACGTACCGTGTGGTCA GCGTCCTCACCGTCCTGCACCAGGACTGGCTGAATGGCAAGGAGT ACAAGTGCAAGGTCTCCAACAAAGCCCTCCCAGCCCCCATCGAGA AAACCATCTCCAAAGCCAAAGGGCAGCCCCGAGAACCACAGGTG TACACCCTGCCCCCATGCCGGGAGGAGATGACCAAGAACCAGGTC AGCCTGTGGTGCCTGGTCAAAGGCTTCTATCCCAGCGACATCGCC GTGGAGTGGGAGAGCAATGGGCAGCCGGAGAACAACTACAAGAC CACGCCTCCCGTGCTGGACTCCGACGGCTCCTTCTTCCTCTACAGC AAGCTCACCGTGGACAAGAGCAGGTGGCAGCAGGGGAACGTCTT CTCATGCTCCGTGATGCATGAGGCTCTGCACAACCACTACACGCA GAAGAGCCTCTCCCTGTCTCCGGGTAAA | |
| TLC-L-PL-Knob | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGATGTGGCACAAAAGTAGAGATTA AGCGTACGGTGGCTGCTCCATCCGTTTTTATCTTTCCCCCAGATAT TCAAATGACCCAAAGTCCAAGTTCCCTTTCAGCATCTGTCGGTGAT CGAGTCACTATAACTTGTAAGGCCAGCCAAGACGTTAGCATAGGA GTAGCATGGTACCAACAAAAGCCTGGGAAGGCTCCCAAACTTCTC ATTTATTCTGCTTCCTACCGATATACTGGTGTCCCAAGTAGATTTT CTGGCAGCGGATCTGGAACTGATTTTACATTGACTATCAGCTCCCT CCAGCCTGAGGACTTCGCTACTTATTACTGCCAACAGTACTACATT | 501 |
| | TATCCCTATACATTCGGTCAAGGGACCAAAGTAGAGATCAAACGT ACGGTGGCAGCTCCCAGCGTTTTTATCTTTCCCCCATCCGACGAGC AGCTCAAGAGTGGCACTGCCTCTGTAGTTTGTTTGCTGAATAACTT CTATCCACGTGAAGCAAAAGTACAGTGGAAGGTCGATAATGCCCT TCAGAGCGGTAACAGCCAAGAAAGTGTTACCGAGCAAGATTCCA AAGATTCCACTTACAGTCTGTCCAGCACATTGACACTGAGTAAGG CTGATTACGAAAAACACAAGGTGTACGCATGCGAGGTGACACAC CAAGGTCTTTCATCTCCTGTAACTAAGAGCTTTAACCGGGGAGAA TGTGGTGGTGGGGGCAGCGGGGGCGGAGGTAGTGGAGGCGGCGG TAGTGAACCAAAGAGTAGTGACAAAACTCACACGTGCCCACCGTG CCCAGCACCTGAACTCCTGGGGGGACCGTCAGTCTTCCTCTTCCCC CCAAAACCCAAGGACACCCTCATGATCTCCCGGACCCCTGAGGTC ACATGCGTGGTGGTGGACGTGAGCCACGAAGACCCTGAGGTCAA GTTCAACTGGTACGTGGACGGCGTGGAGGTGCATAATGCCAAGAC AAAGCCGCGGGAGGAGCAGTACAACAGCACGTACCGTGTGGTCA GCGTCCTCACCGTCCTGCACCAGGACTGGCTGAATGGCAAGGAGT ACAAGTGCAAGGTCTCCAACAAAGCCCTCCCAGCCCCCATCGAGA AAACCATCTCCAAAGCCAAAGGGCAGCCCCGAGAACCACAGGTG TACACCCTGCCCCCATGCCGGGAGGAGATGACCAAGAACCAGGTC AGCCTGTGGTGCCTGGTCAAAGGCTTCTATCCCAGCGACATCGCC GTGGAGTGGGAGAGCAATGGGCAGCCGGAGAACAACTACAAGAC CACGCCTCCCGTGCTGGACTCCGACGGCTCCTTCTTCCTCTACAGC AAGCTCACCGTGGACAAGAGCAGGTGGCAGCAGGGGAACGTCTT CTCATGCTCCGTGATGCATGAGGCTCTGCACAACCACTACACGCA GAAGAGCCTCTCCCTGTCTCCGGGTAAA | |
| TL-L-PLC-Knob | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGACAGGGCACAAAAGTAGAGATTA AGCGTACGGTGGCTGCTCCATCCGTTTTTATCTTTCCCCCAGATAT TCAAATGACCCAAAGTCCAAGTTCCCTTTCAGCATCTGTCGGTGAT CGAGTCACTATAACTTGTAAGGCCAGCCAAGACGTTAGCATAGGA GTAGCATGGTACCAACAAAAGCCTGGGAAGGCTCCCAAACTTCTC ATTTATTCTGCTTCCTACCGATATACTGGTGTCCCAAGTAGATTTT CTGGCAGCGGATCTGGAACTGATTTTACATTGACTATCAGCTCCCT CCAGCCTGAGGACTTCGCTACTTATTACTGCCAACAGTACTACATT TATCCCTATACATTCGGTTGTGGGACCAAAGTAGAGATCAAACGT ACGGTGGCAGCTCCCAGCGTTTTTATCTTTCCCCCATCCGACGAGC AGCTCAAGAGTGGCACTGCCTCTGTAGTTTGTTTGCTGAATAACTT CTATCCACGTGAAGCAAAAGTACAGTGGAAGGTCGATAATGCCCT TCAGAGCGGTAACAGCCAAGAAAGTGTTACCGAGCAAGATTCCA AAGATTCCACTTACAGTCTGTCCAGCACATTGACACTGAGTAAGG | 502 |
| | CTGATTACGAAAAACACAAGGTGTACGCATGCGAGGTGACACAC CAAGGTCTTTCATCTCCTGTAACTAAGAGCTTTAACCGGGGAGAA TGTGGTGGTGGGGGCAGCGGGGGCGGAGGTAGTGGAGGCGGCGG TAGTGAACCAAAGAGTAGTGACAAAACTCACACGTGCCCACCGTG CCCAGCACCTGAACTCCTGGGGGGACCGTCAGTCTTCCTCTTCCCC CCAAAACCCAAGGACACCCTCATGATCTCCCGGACCCCTGAGGTC ACATGCGTGGTGGTGGACGTGAGCCACGAAGACCCTGAGGTCAA GTTCAACTGGTACGTGGACGGCGTGGAGGTGCATAATGCCAAGAC AAAGCCGCGGGAGGAGCAGTACAACAGCACGTACCGTGTGGTCA GCGTCCTCACCGTCCTGCACCAGGACTGGCTGAATGGCAAGGAGT ACAAGTGCAAGGTCTCCAACAAAGCCCTCCCAGCCCCCATCGAGA AAACCATCTCCAAAGCCAAAGGGCAGCCCCGAGAACCACAGGTG TACACCCTGCCCCCATGCCGGGAGGAGATGACCAAGAACCAGGTC AGCCTGTGGTGCCTGGTCAAAGGCTTCTATCCCAGCGACATCGCC GTGGAGTGGGAGAGCAATGGGCAGCCGGAGAACAACTACAAGAC CACGCCTCCCGTGCTGGACTCCGACGGCTCCTTCTTCCTCTACAGC AAGCTCACCGTGGACAAGAGCAGGTGGCAGCAGGGGAACGTCTT CTCATGCTCCGTGATGCATGAGGCTCTGCACAACCACTACACGCA GAAGAGCCTCTCCCTGTCTCCGGGTAAA | |
| TLC-L-PLC-Knob | GACATTCAGATGACCCAATCCCCCTCTAGCCTTTCAGCCTCCGTAG GGGACAGAGTTACAATAACCTGTAGGGCTAGTCAGGACGTTAATA CAGCAGTCGCATGGTACCAGCAGAAGCCCGGTAAGGCTCCTAAGC TTTTGATCTACAGTGCTTCTTTCCTGTATTCTGGTGTACCTAGCCGA TTCTCAGGCTCTCGGAGTGGCACAGACTTCACTCTGACAATTTCAA GTCTCCAGCCAGAAGATTTTGCAACCTACTACTGCCAACAACACT ACACTACTCCTCCAACCTTCGGATGTGGCACAAAAGTAGAGATTA AGCGTACGGTGGCTGCTCCATCCGTTTTTATCTTTCCCCCAGATAT TCAAATGACCCAAAGTCCAAGTTCCCTTTCAGCATCTGTCGGTGAT CGAGTCACTATAACTTGTAAGGCCAGCCAAGACGTTAGCATAGGA GTAGCATGGTACCAACAAAAGCCTGGGAAGGCTCCCAAACTTCTC ATTTATTCTGCTTCCTACCGATATACTGGTGTCCCAAGTAGATTTT CTGGCAGCGGATCTGGAACTGATTTTACATTGACTATCAGCTCCCT CCAGCCTGAGGACTTCGCTACTTATTACTGCCAACAGTACTACATT TATCCCTATACATTCGGTTGTGGGACCAAAGTAGAGATCAAACGT ACGGTGGCAGCTCCCAGCGTTTTTATCTTTCCCCCATCCGACGAGC AGCTCAAGAGTGGCACTGCCTCTGTAGTTTGTTTGCTGAATAACTT CTATCCACGTGAAGCAAAAGTACAGTGGAAGGTCGATAATGCCCT TCAGAGCGGTAACAGCCAAGAAAGTGTTACCGAGCAAGATTCCA AAGATTCCACTTACAGTCTGTCCAGCACATTGACACTGAGTAAGG CTGATTACGAAAAACACAAGGTGTACGCATGCGAGGTGACACAC CAAGGTCTTTCATCTCCTGTAACTAAGAGCTTTAACCGGGGAGAA TGTGGTGGTGGGGGCAGCGGGGGCGGAGGTAGTGGAGGCGGCGG TAGTGAACCAAAGAGTAGTGACAAAACTCACACGTGCCCACCGTG CCCAGCACCTGAACTCCTGGGGGGACCGTCAGTCTTCCTCTTCCCC CCAAAACCCAAGGACACCCTCATGATCTCCCGGACCCCTGAGGTC | 503 |
| | ACATGCGTGGTGGTGGACGTGAGCCACGAAGACCCTGAGGTCAA GTTCAACTGGTACGTGGACGGCGTGGAGGTGCATAATGCCAAGAC AAAGCCGCGGGAGGAGCAGTACAACAGCACGTACCGTGTGGTCA GCGTCCTCACCGTCCTGCACCAGGACTGGCTGAATGGCAAGGAGT ACAAGTGCAAGGTCTCCAACAAAGCCCTCCCAGCCCCCATCGAGA AAACCATCTCCAAAGCCAAAGGGCAGCCCCGAGAACCACAGGTG TACACCCTGCCCCCATGCCGGGAGGAGATGACCAAGAACCAGGTC AGCCTGTGGTGCCTGGTCAAAGGCTTCTATCCCAGCGACATCGCC GTGGAGTGGGAGAGCAATGGGCAGCCGGAGAACAACTACAAGAC CACGCCTCCCGTGCTGGACTCCGACGGCTCCTTCTTCCTCTACAGC AAGCTCACCGTGGACAAGAGCAGGTGGCAGCAGGGGAACGTCTT CTCATGCTCCGTGATGCATGAGGCTCTGCACAACCACTACACGCA GAAGAGCCTCTCCCTGTCTCCGGGTAAA | |

**[Table 34]**

| | VH1-linker-VH2-CH1-Fc(Knob) | VL1-linker-VL2-CL-linker-Fc(Knob) | Fc(Hole) |
|---|---|---|---|
| HP501 | SEQ ID NO: 51 | SEQ ID NO: 59 | SEQ ID NO: 7 |
| HP502 | SEQ ID NO: 52 | SEQ ID NO: 60 | SEQ ID NO: 7 |
| HP503 | SEQ ID NO: 53 | SEQ ID NO: 61 | SEQ ID NO: 7 |
| HP504 | SEQ ID NO: 54 | SEQ ID NO: 62 | SEQ ID NO: 7 |
| HP505 | SEQ ID NO: 51 | SEQ ID NO: 63 | SEQ ID NO: 7 |
| HP506 | SEQ ID NO: 52 | SEQ ID NO: 64 | SEQ ID NO: 7 |
| HP507 | SEQ ID NO: 53 | SEQ ID NO: 65 | SEQ ID NO: 7 |
| HP508 | SEQ ID NO: 54 | SEQ ID NO: 66 | SEQ ID NO: 7 |
| HP509 | SEQ ID NO: 55 | SEQ ID NO: 59 | SEQ ID NO: 7 |
| HP510 | SEQ ID NO: 56 | SEQ ID NO: 60 | SEQ ID NO: 7 |
| HP511 | SEQ ID NO: 57 | SEQ ID NO: 61 | SEQ ID NO: 7 |
| HP512 | SEQ ID NO: 58 | SEQ ID NO: 62 | SEQ ID NO: 7 |
| HP513 | SEQ ID NO: 55 | SEQ ID NO: 63 | SEQ ID NO: 7 |
| HP514 | SEQ ID NO: 56 | SEQ ID NO: 64 | SEQ ID NO: 7 |
| HP515 | SEQ ID NO: 57 | SEQ ID NO: 65 | SEQ ID NO: 7 |
| HP516 | SEQ ID NO: 58 | SEQ ID NO: 66 | SEQ ID NO: 7 |

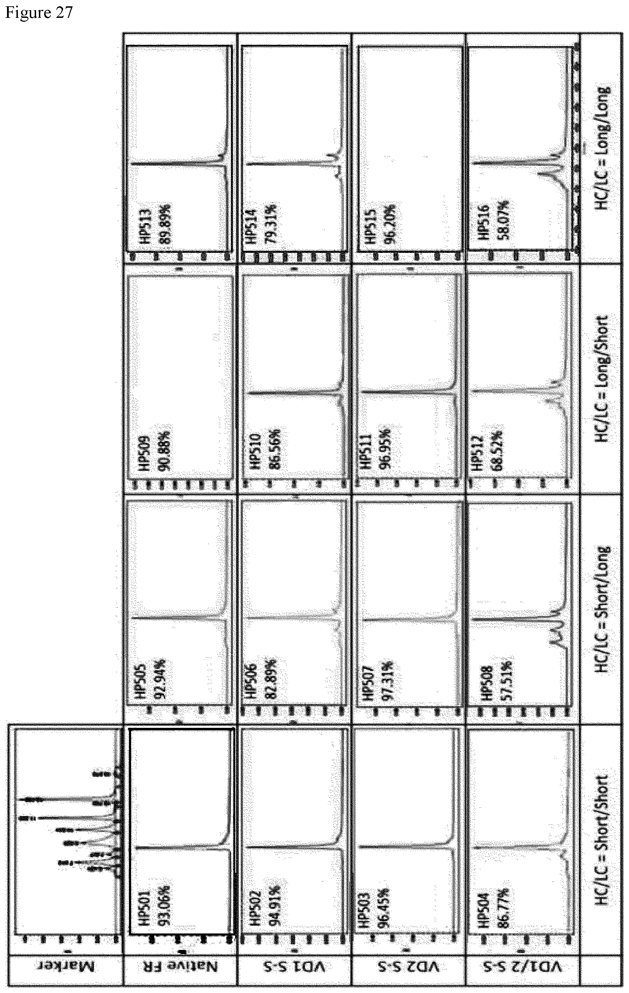

Purity was analyzed by size exclusion chromatography in the same manner as described in Example 1 (Figure 27, Table 35). The analysis showed that HP503, HP507, HP511, and HP515, in which the first V domain is wild type and the second V domain is Cys substituted variant (VH 44C, VL 100C), have an excellent purity (Figure 27, Table 35).

**[Table 35]**

| Clone name | Monomer purity (%) |
|---|---|
| HP501 | 93.06 |
| HP502 | 94.91 |
| HP503 | 96.45 |
| HP504 | 86.77 |
| HP505 | 92.94 |
| HP506 | 82.89 |
| HP507 | 97.31 |
| HP508 | 57.51 |
| HP509 | 90.88 |
| HP510 | 86.56 |
| HP511 | 96.95 |
| HP512 | 68.52 |
| HP513 | 89.89 |
| HP514 | 79.31 |
| HP515 | 96.20 |
| HP516 | 58.07 |

The binding constants of HP501, HP502, HP503, HP504, HP505, HP506, HP507, HP508, HP509, HP510, HP511, HP512, HP513, HP514, HP515, and HP516 to D2 region and D4 region in HER2 protein were determined using the Octet Red96e (Sartorius). In order to analyze the binding constants of the sixteen antibodies to the D2 region, the human HER2 recombinant protein (R&D systems, 1129-ER) was loaded onto the Anti-Penta-HIS (HIS1K) biosensor (Sartorius, 18-5120) and then saturated with 100 nM trastuzumab which targets the D4 region. Thereafter, the sixteen antibodies were added in a binding reaction (300 seconds) and a dissociation reaction (600 seconds) at a concentration of 100 nM, and their affinities for the D2 region were calculated (Table 36). In order to analyze the binding constants of the sixteen antibodies to the D4 region, the human HER2 recombinant protein (R&D systems, 1129-ER) was loaded onto the Anti-Penta-HIS (HIS 1K) biosensor (Sartorius, 18-5120), and then saturated with 100 nM pertuzumab which targets the D2 region. Thereafter, the sixteen antibodies were added in a binding reaction (300 seconds) and a dissociation reaction (600 seconds) at a concentration of 100 nM, and their affinities for the D4 region were calculated (Table 36). The binding constants of HP507, HP511, and HP515 to D2 region were 2.285, 3.267, and 2.012 nM, respectively, showing excellent binding affinities to the D2 region compared to other clones (Table 36). HP503 has a binding constant of 8.098 nM to the D2 region and shows a relatively low binding ability to the D2 region compared to HP507, HP511, and HP515. However, it was found that when measuring the binding constant to the D4 region, the binding constants of HP503, HP507, HP511, and HP515 were 0.181, 0.228, 0.162, and 0.227 nM, respectively, demonstrating strong binding affinity (Table 36).

**[Table 36]**

| 100 nM Single Kinetics | Binding kinetics of mAbs to D2 epitope | | | Binding kinetics of mAbs to D4 epitope | | |
|---|---|---|---|---|---|---|
| | K_{D} (nM) | Kₐ (1/Ms) | K_{d} (1/s) | K_{D} (nM) | Kₐ (1/Ms) | K_{d} (1/s) |
| HP501 | 13.009 | 8.05E+03 | 1.05E-04 | 0.218 | 2.00E+05 | 4.36E-05 |
| HP502 | 8.433 | 8.97E+03 | 7.56E-05 | 0.531 | 1.47E+05 | 7.82E-05 |
| HP503 | 8.098 | 4.69E+03 | 3.80E-05 | 0.181 | 2.14E+05 | 3.87E-05 |
| HP504 | 7.304 | 2.53E+04 | 1.85E-04 | 0.788 | 1.13E+05 | 8.94E-05 |
| HP505 | 5.882 | 1.41E+04 | 8.30E-05 | 0.586 | 1.50E+05 | 8.79E-05 |
| HP506 | 5.682 | 1.21E+04 | 6.89E-05 | 0.611 | 1.37E+05 | 8.37E-05 |
| HP507 | 2.285 | 1.85E+04 | 4.23E-05 | 0.228 | 1.99E+05 | 4.53E-05 |
| HP508 | 6.490 | 2.36E+04 | 1.53E-04 | 1.219 | 8.44E+04 | 1.03E-04 |
| HP509 | 7.962 | 1.33E+04 | 1.06E-04 | 0.393 | 1.72E+05 | 6.77E-05 |
| HP510 | 10.736 | 9.86E+03 | 1.06E-04 | 0.336 | 1.97E+05 | 6.60E-05 |
| HP511 | 3.267 | 1.47E+04 | 4.81E-05 | 0.162 | 2.27E+05 | 3.67E-05 |
| HP512 | 7.615 | 2.80E+04 | 2.13E-04 | 0.667 | 1.12E+05 | 7.47E-05 |
| HP513 | 4.739 | 2.17E+04 | 1.03E-04 | 0.381 | 1.95E+05 | 7.43E-05 |
| HP514 | 5.920 | 1.78E+04 | 1.06E-04 | 0.451 | 1.74E+05 | 7.82E-05 |
| HP515 | 2.012 | 2.89E+04 | 5.81E-05 | 0.227 | 2.14E+05 | 4.87E-05 |
| HP516 | 5.094 | 3.29E+04 | 1.67E-04 | 0.607 | 1.23E+05 | 7.46E-05 |

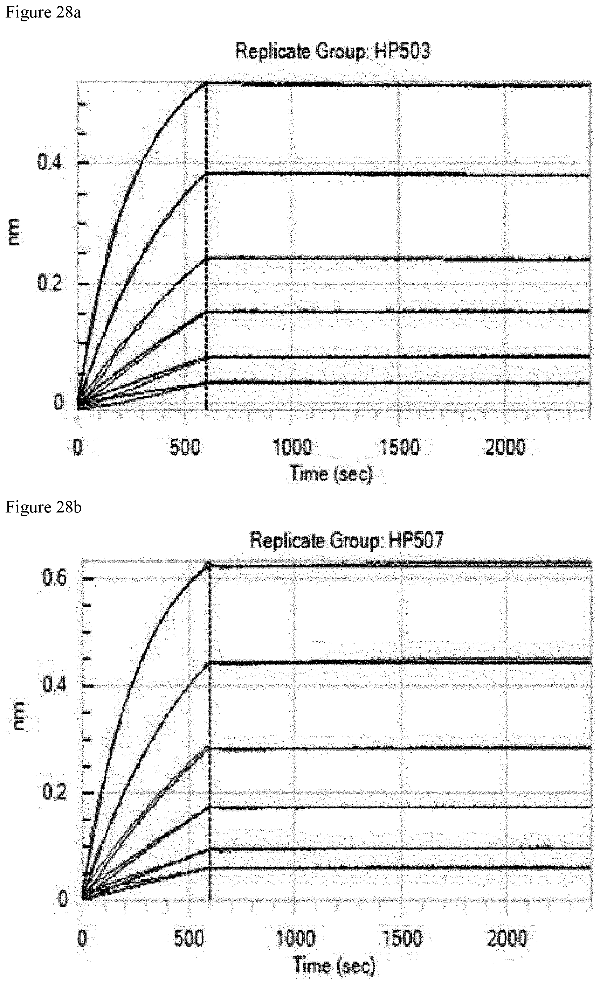

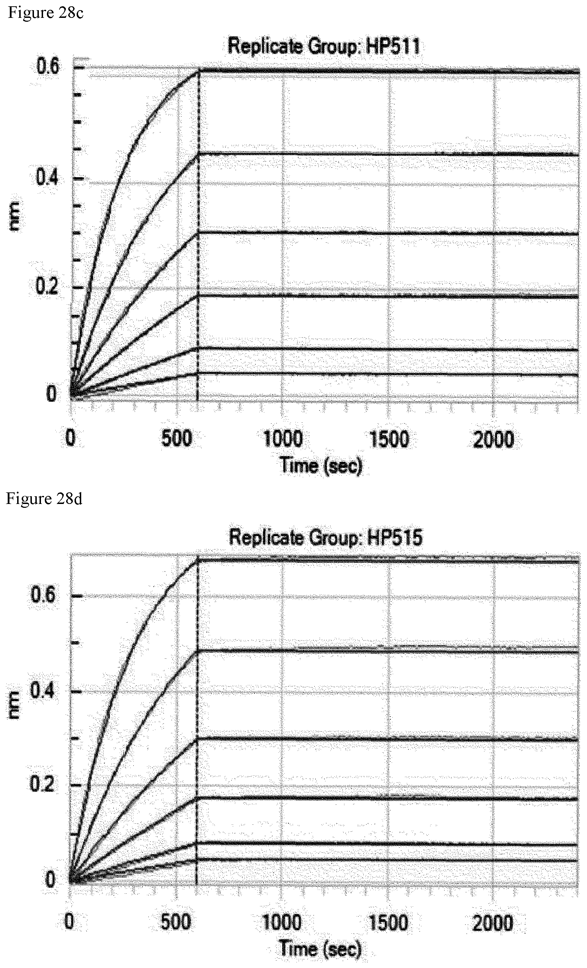

The binding constants of HP503, HP507, HP511, and HP515 to the HER2 extracellular domain (ECD) were measured using Octet Red96e (Sartorius). The human HER2 recombinant protein (R&D systems, 1129-ER) was loaded onto the Anti-Penta-HIS (HIS 1K) biosensor (Sartorius, 18-5120). HP503 or HP507 or HP511 or HP515 at a concentration of 0.25, 0.5, 1, 2, 4, or 8 nM were added in a binding reaction (600 seconds) and a dissociation reaction (1,800 seconds) in the sensor loaded with the HER2 protein, and the binding constants were calculated (Figures 28a to 28d, Table 37). It was found that HP507, HP511, and HP515 had a dissociation constant (<1.0E-07 1/s) that exceeded the measurement limit of the equipment under the following analysis conditions (Figures 28a to 28d, Table 37).

**[Table 37]**

| | K_{D} (nM) | Kₐ (1/Ms) | K_{d} (1/s) |
|---|---|---|---|
| HP503 | 6.57E-12 | 4.69E+05 | 3.08E-06 |
| HP507 | <1.0E-12 | 4.58E+05 | <1.0E-07 |
| HP511 | <1.0E-12 | 5.20E+05 | <1.0E-07 |
| HP515 | <1.0E-12 | 4.14E+05 | <1.0E-07 |

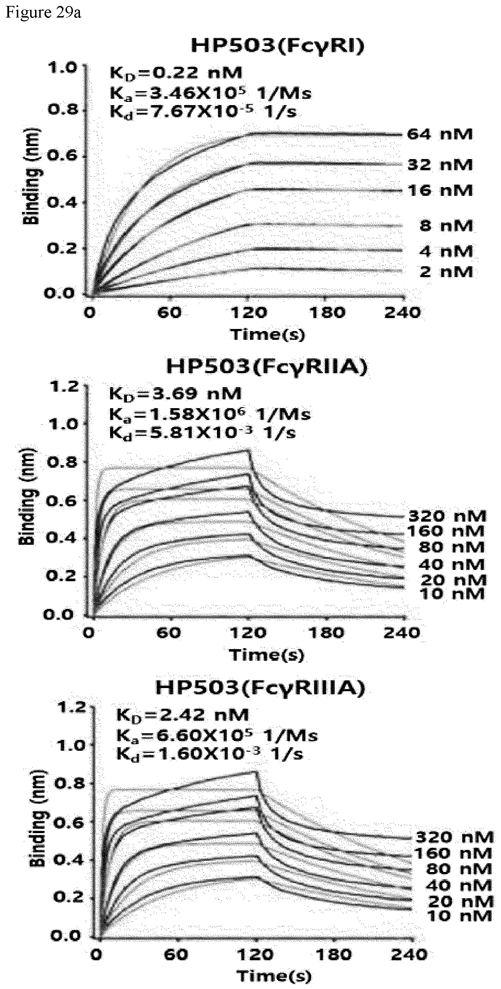

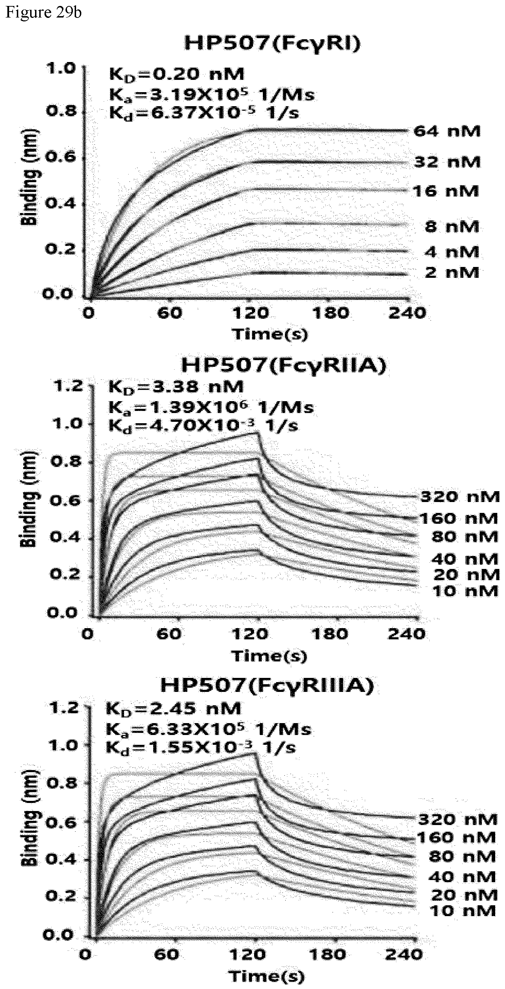

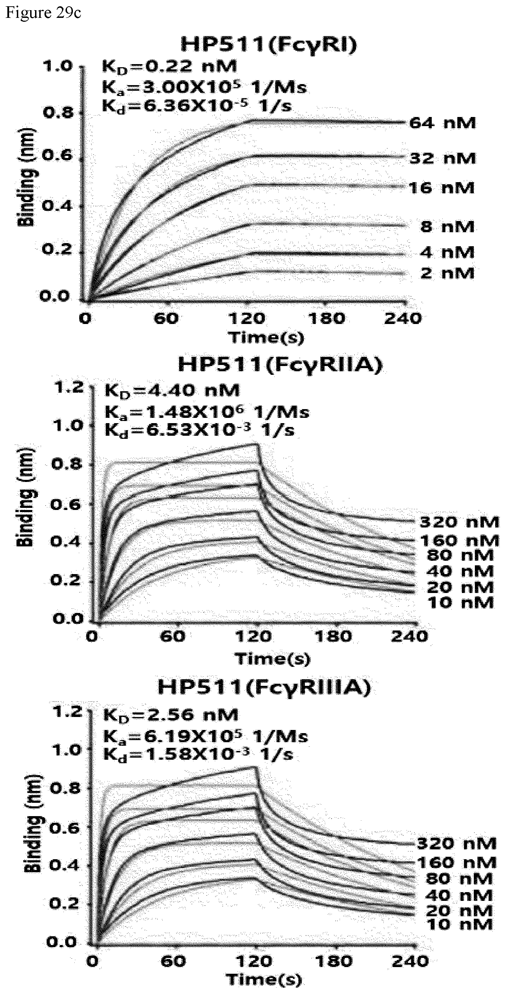

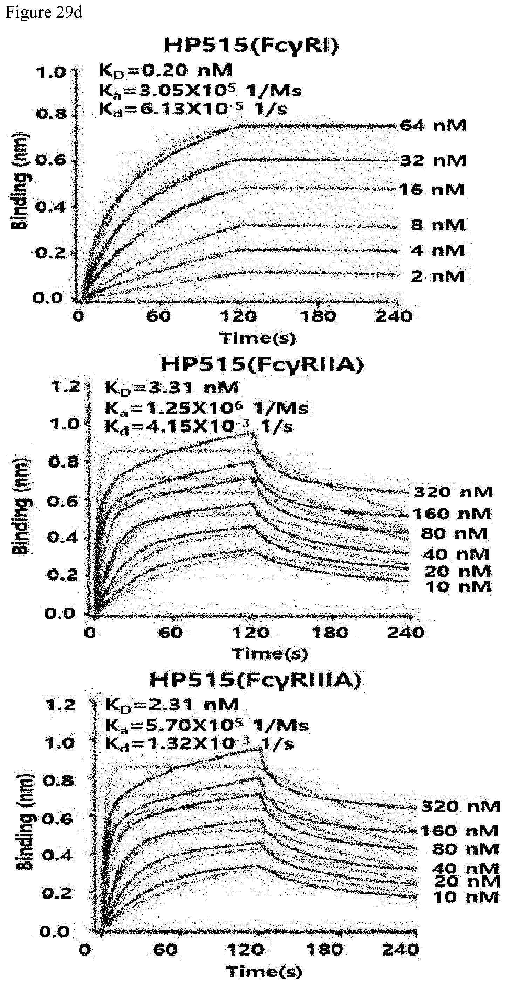

The measured binding constants of HP503, HP507, HP511, and HP515 to Fcy receptors were analyzed using Octet Red96e (Sartorius) in the same manner as described in Example 10 (Figures 29a to 29d, Table 38). The analysis showed that HP503, HP507, HP511, and HP515 have excellent binding affinities to FcyRI (CD64), FcyRIIA (CD32A, 131R), and FcγRIIIA (CD16A,176V) compared to human IgG1, trastuzumab, pertuzumab, and margetuximab (Figures 21a to 21h, Figures 22a to 22h, Figures 23a to 23h, Figures 29a to 29d, and Tables 29 and 38).

**[Table 38]**

| Antigen | FcyRI (CD64) | | | FcγRIIA (CD32A, 131R) | | | FcγRIIIA (CD16A,176V) | | |
|---|---|---|---|---|---|---|---|---|---|
| Antibody | KD (nM) | Ka (1/Ms) | Kd (1/s) | KD (nM) | Ka (1/Ms) | Kd (1/s) | KD (nM) | Ka (1/Ms) | Kd (1/s) |
| HP503 | 0.22 | 3.46E+05 | 7.67E-05 | 3.69 | 1.58E+06 | 5.81E-03 | 2.42 | 6.60E+05 | 1.60E-03 |
| HP507 | 0.20 | 3.19E+05 | 6.37E-05 | 3.38 | 1.39E+06 | 4.70E-03 | 2.45 | 6.33E+05 | 1.55E-03 |
| HP511 | 0.22 | 3.00E+05 | 6.63E-05 | 4.40 | 1.48E+06 | 6.53E-03 | 2.56 | 6.19E+05 | 1.58E-03 |
| HP515 | 0.20 | 3.05E+05 | 6.13E-05 | 3.31 | 1.25E+06 | 4.15E-03 | 2.31 | 5.70E+05 | 1.32E-03 |

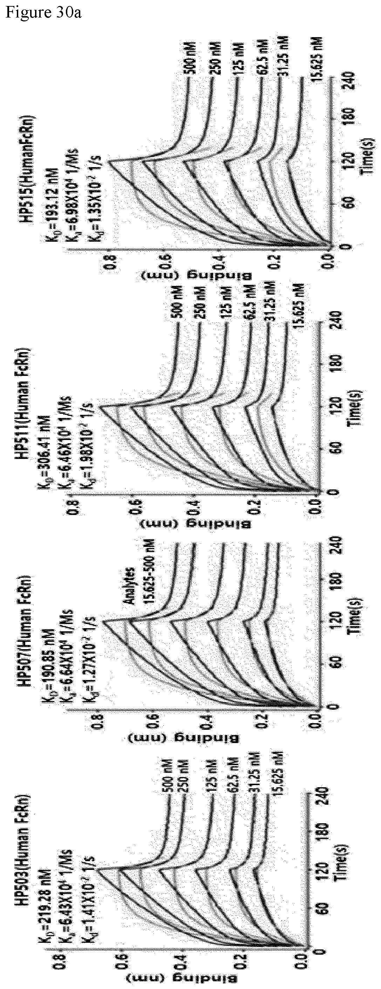

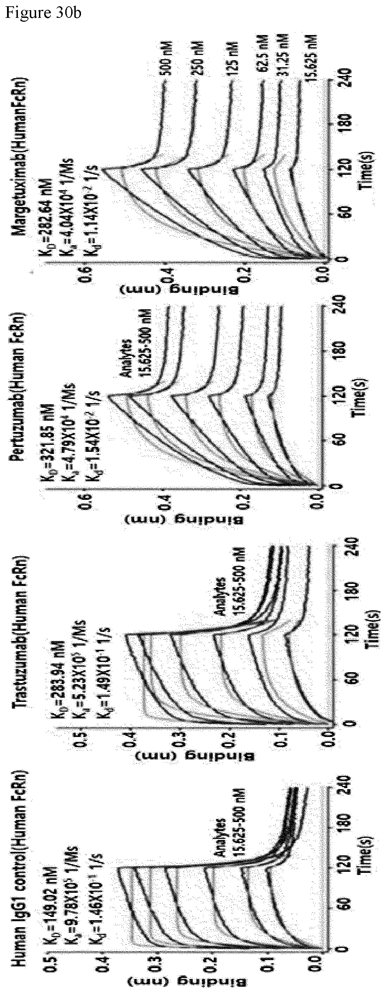

The binding constants of HP503, HP507, HP511, and HP515 to the neonatal Fc receptor (FcRn) were measured using Octet Red96e (Sartorius). HP503, HP507, HP511, HP515, human IgG1 (Bio X cell, BE0297), trastuzumab, pertuzumab, and margetuximab were loaded onto the anti-human Fab-CH1 2nd generation (FAB2G) biosensor (Sartorius, 18-5126), and a binding and a dissociation time were set to be 120 seconds, respectively (Figures 30a to 30b, Table 39). For analysis, Kinetics Buffer (Sartorius, 18-1105) was used at pH 6.0.

**[Table 39]**

| Antibodies | K_{D} (nM) | Kₐ (1/Ms) | K_{d} (1/s) |
|---|---|---|---|
| HP503 | 219.28 | 6.43E+04 | 1.41E-02 |
| HP507 | 190.85 | 6.64E+04 | 1.27E-02 |
| HP511 | 306.41 | 6.46E+04 | 1.98E-02 |
| HP515 | 193.12 | 6.98E+04 | 1.35E-02 |
| Human IgG1 | 149.02 | 9.78E+05 | 1.46E-01 |
| Trastuzumab | 283.94 | 5.23E+05 | 1.49E-01 |
| Pertuzumab | 321.85 | 4.79E+04 | 1.54E-02 |
| Margetuximab | 282.64 | 4.04E+04 | 1.14E-02 |

### Example 13. Analysis of Complement-dependent cytotoxicity

For the analysis of complement-dependent cytotoxicity, BT474 (HER2 3+; high) breast cancer cell line and NCI-N87 (HER2 3+; high) gastric cancer cell line were used. The cells were diluted in cell culture medium and dispensed in a 96-well plate at 10,000 cells per well. The cells, antibodies, and human serum (Sigma, H4522) were each reacted at a volume ratio of 1:1:1.

In order to identify a dose-response relationship, human IgG1, trastuzumab (TRA), trastuzumab + pertuzumab (TRA + PER), H01, and H01 + P01 were serially diluted by a factor of two six times from initial concentration of 1200 nM, and the reaction was carried out from 400 nM (another three-fold dilution when dispensed). The human serum was diluted and dispensed in the culture medium to a final concentration of 25%, and the mixture of the cells, antibodies, and human serum was incubated for 5 hours in a humidified incubator at 37°C and 5% (v/v) CO₂ conditions.

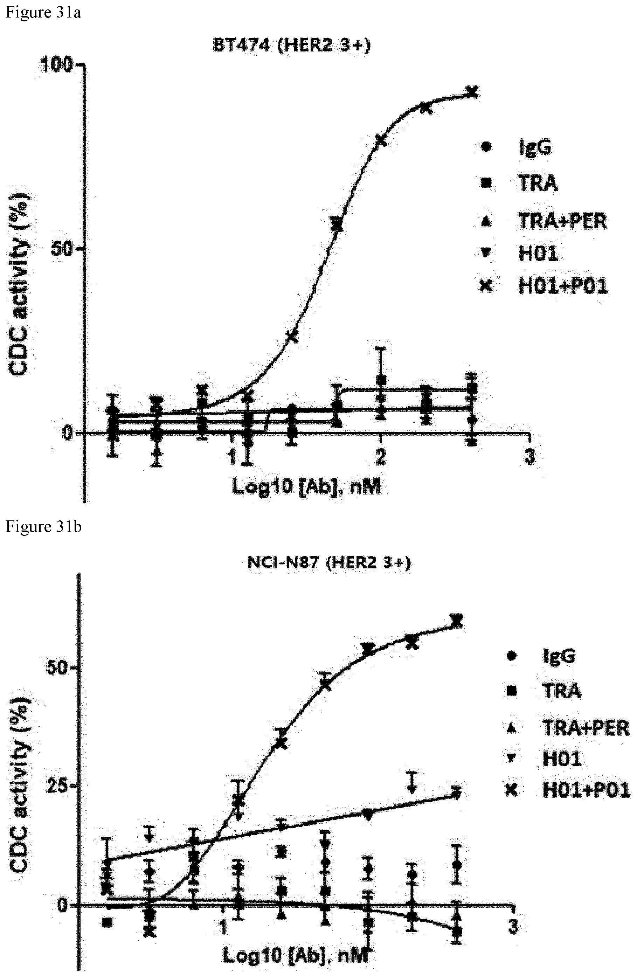

Cell Titer Glo-Reagent (Promega, G9243) previously dissolved at 4°C was dispensed into each well in an equal volume of the mixed culture medium, and then the cell lysis was induced using a plate shaker (Allsheng, MX100-4A) with agitation at 500 rpm for 2 minutes. In order to stabilize the luminescence signal, the mixture was incubated at room temperature for 10 minutes and then analyzed using a plate reader equipment (Envision; PerkinElmer, 2105-0010) (Figures 31a to 31b). The complement-dependent cytotoxicity activity (CDC activity) was calculated as follows. CDC activity (%) = 100 x [1-(luminescence with experimental antibody/luminescence without antibody)]

Human IgG1, trastuzumab (TRA), trastuzumab + pertuzumab (TRA + PER), and H01 did not induce CDC responses in BT474 and NCI-N87 cell lines (Figures 31a to 31b). It was shown that CDC is induced in both cell lines only when H01 is treated in combination with P01 (Figures 31a to 31b).

### Example 14. Analysis of antibody-dependent cytotoxicity

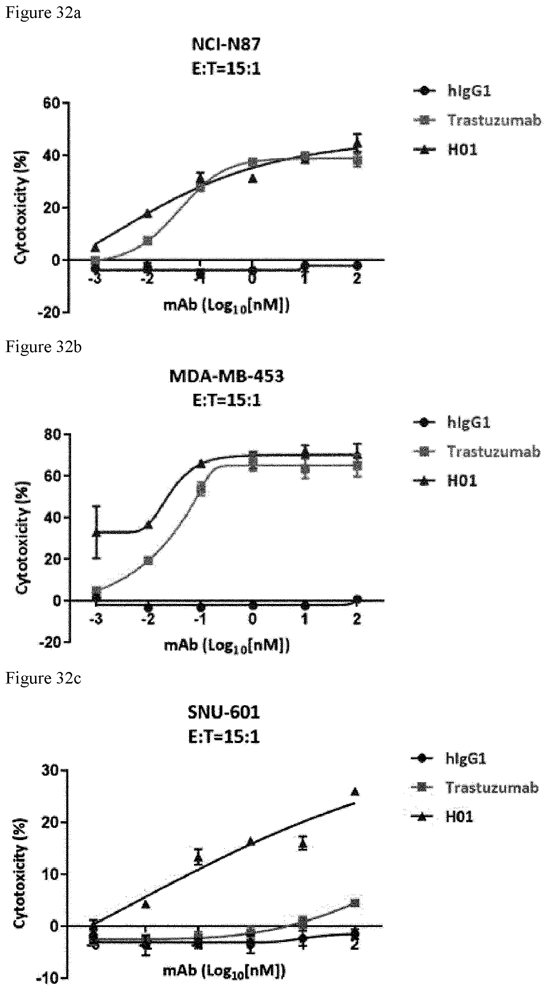

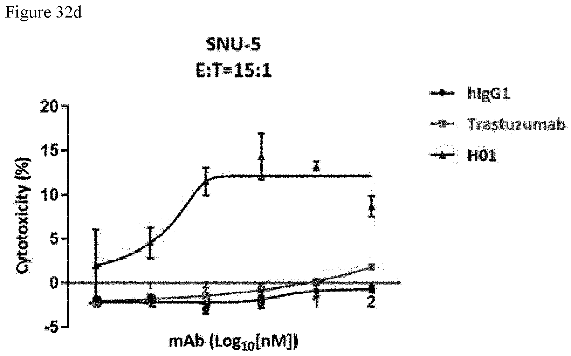

NCI-N87 (HER2 3+; high), MDA-MB-453(HER2 2+; Mid), SNU-601 (HER2 1+; low), and SNU-5 (HER2 1+; low) cancer cell lines were used for antibody-dependent cell-mediated cytotoxicity analysis (Figures 32a, 32b, 32c, and 32d). Each cancer cell line was seeded at 1.0 × 10⁴ cells/well in a 96-well plate. Thereafter, each antibody was diluted and treated in culture medium to an appropriate concentration. Peripheral blood mononuclear cells (PBMC) isolated on the same day were used as effector cells and treated at 1.5 × 10⁵ cells/well to make the number of PBMC 15 times more than the number of target cells (E:T ratio = 15:1).

After treatment, the cells were incubated for 18 hours in a humidified incubator at 37°C and 5% (v/v) CO₂ conditions, and then cytotoxicity was measured using a cytotoxicity detection kit (LDH) (Roche, 11644793001) (Figures 32a, 32b, 32c, and 32d). Cytotoxicity was calculated using the following formula for antibody-dependent cytotoxicity. Cytotoxicity (%) = [(Test release-spontaneous release)/(Maximum release- spontanous release)] x 100

H01 showed excellent cytotoxicity at a low concentration compared to trastuzumab in NCI-N87 (HER2 3+; high) and MDA-MB-453 (HER2 2+; Mid) cancer cell lines (Figures 32a and 32b). Cytotoxicity analysis in SNU-601 (HER2 1+; low) and SNU-5 (HER2 1+; low) cancer cell lines H01 showed excellent cytotoxicity of H01 compared to that of trastuzumab (Figures 32c and 32d).

### Example 15. Evaluation of efficacy in xenograft mouse models

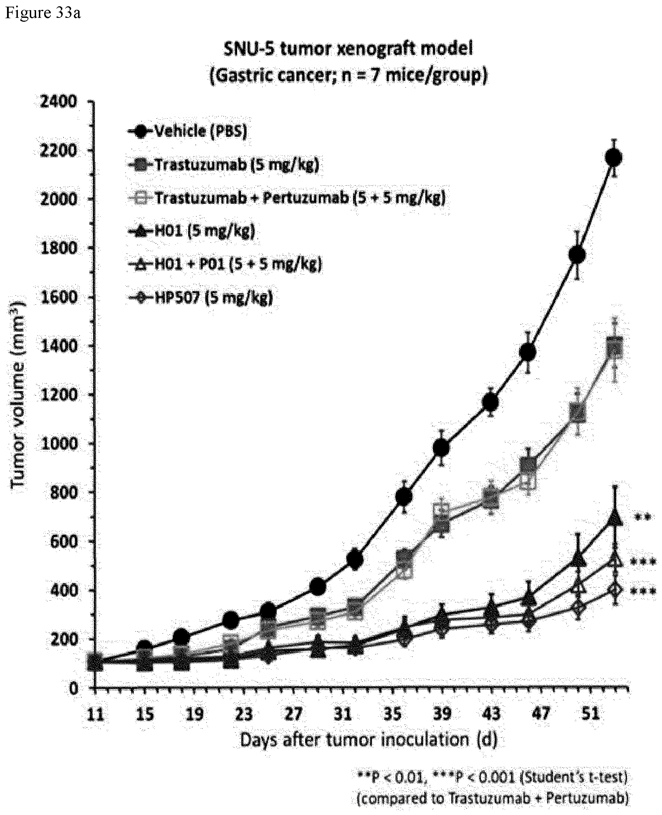

Efficacy in the SNU-5 (HER2 1+; low) gastric cancer cell line-derived xenograft model was evaluated using 6-week-old female SCID mice (C.B-17/NcrKoat-Prkdc^{scid}, Koatech) (Figure 33a). The SNU-5 cancer cell line was diluted in PBS at 1 × 10⁷ cells/100 µL and mixed with MATRIGEL^{®} Growth Factor Reduced (GFR) Basement Membrane Matrix (Corning, 354230) at a ratio of 1:1, and 100 µL of the mixture was transplanted subcutaneously into the right flank, and tumor growth was monitored. Mice were regrouped so that the average tumor volume was about 107 mm³, and PBS (vehicle), 5 mg/kg H01, 5 mg/kg H01 + 5 mg/kg P01, 5 mg/kg HP507, 5 mg/kg trastuzumab, 5 mg/kg trastuzumab + 5 mg/kg pertuzumab were administered intravenously (I.V.) once a week for a total of 6 weeks (Figure 33a). According to the analysis, H01 alone showed superior antitumor activity compared to trastuzumab and trastuzumab + pertuzumab (Figure 33a). It was shown that H01 + P01 induces improved antitumor activity compared to H01 alone, and HP507 induces the strongest antitumor activity in the SNU-5 gastric cancer xenograft model (Figure 33a).

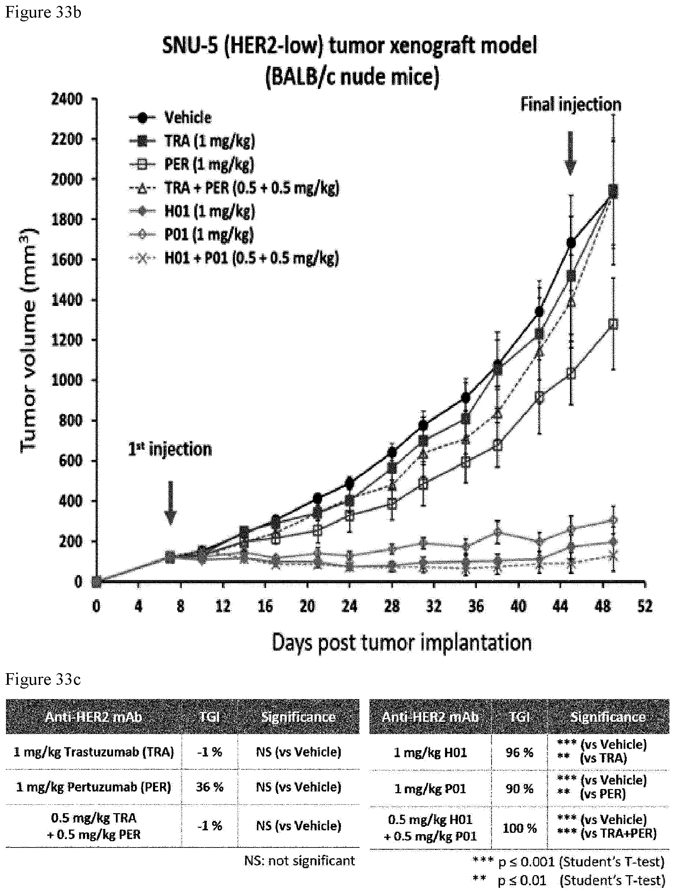

In addition, efficacy in the SNU-5 (HER2 1+; low) gastric cancer cell line-derived xenograft model was evaluated using 6-week-old female BALB/c-nu mice (ORIENT BIO INC.) (Figure 33b). The SNU-5 cancer cell line was diluted in PBS at 1 × 10⁷ cells/100 µL and mixed with MATRIGEL^{®} Growth Factor Reduced (GFR) Basement Membrane Matrix (Corning, 354230) at a ratio of 1:1, and 100 µL of the mixture was transplanted subcutaneously into the right flank, and tumor growth was observed. Mice were regrouped so that the average tumor volume was about 122 mm³, and 50 mg/kg Intravenous Immunoglobulin (IVIG; LIV-r, SK Plasma) was administered to all mice twice a week for 6 weeks (Figure 33b). PBS (vehicle), 1 mg/kg trastuzumab, 1 mg/kg pertuzumab, 0.5 mg/kg trastuzumab + 0.5 mg/kg pertuzumab, 1 mg/kg H01, 1 mg/kg P01, and 0.5 mg/kg H01 + 0.5 mg/kg P01 were administered intraperitoneally (I.P.) twice a week for a total of 6 weeks (Figure 33b). The analysis showed that H01, P01, and H01 + P01 induce superior antitumor activity compared to trastuzumab, pertuzumab, and trastuzumab + pertuzumab (Figure 33b).

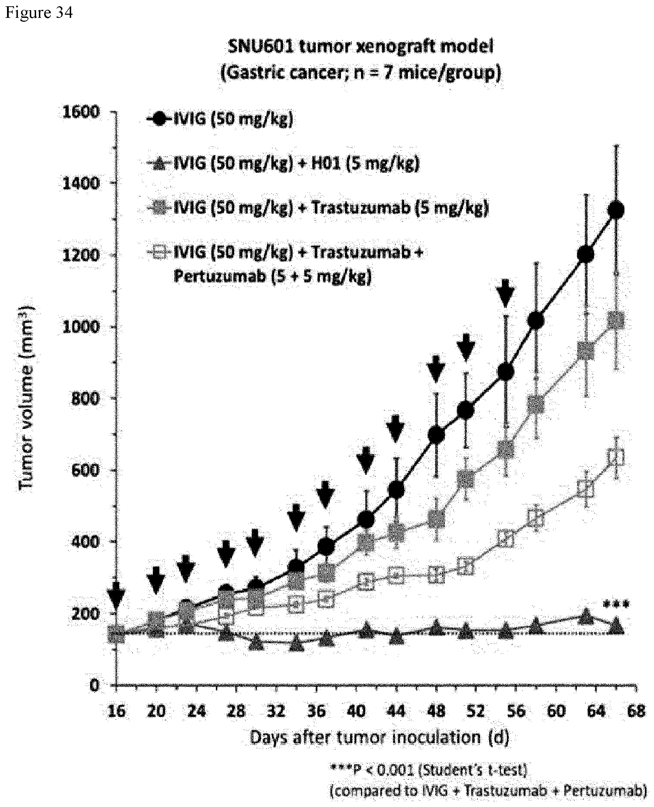

Efficacy in the SNU-601 (HER2 1+; low) gastric cancer cell line-derived xenograft model was evaluated using 6-week-old female SCID mice (C.B-17/NcrKoat-Prkdc^{scid}, Koatech). The SNU-5 cancer cell line was diluted in PBS at 1 × 10⁷ cells/100 µL and mixed with MATRIGEL^{®} Growth Factor Reduced (GFR) Basement Membrane Matrix (Corning, 354230) at a ratio of 1:1, and 100 µL of the mixture was transplanted subcutaneously into the right flank, and tumor growth was observed. Mice were regrouped so that the average tumor volume was about 142 mm³, and PBS (vehicle), 5 mg/kg H01, 5 mg/kg trastuzumab, and 5 mg/kg trastuzumab + 5 mg/kg pertuzumab were administered intraperitoneally (I.P.) twice a week for a total of 6 weeks (Figure 34). Since there are no antibodies present in the blood of the SCID mice, 50 mg/kg Intravenous Immunoglobulin (IVIG; LIV-r, SK Plasma) was administered to all mice twice a week for 6 weeks to simulate the actual human blood environment (Figure 34). In the SNU-601 gastric cancer xenograft model, H01 alone induced most superior antitumor activity (Figure 34).

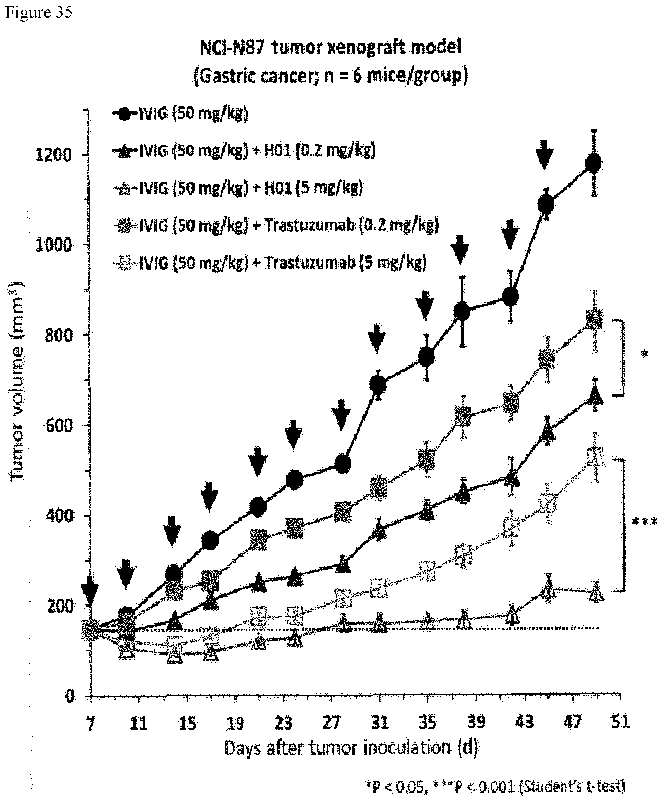

Efficacy in the NCI-N87 (HER2 3+; high) gastric cancer cell line-derived xenograft model was evaluated using 6-week-old female SCID mice (C.B-17/NcrKoat-Prkdc^{scid}, Koatech). The NCI-N87 cancer cell line was diluted in PBS at 5 × 10⁶ cells/100 µL and mixed with MATRIGEL^{®} Growth Factor Reduced (GFR) Basement Membrane Matrix (Corning, 354230) at a ratio of 1:1, and 100 µL of the mixture was transplanted subcutaneously into the right flank, and tumor growth was observed. Mice were regrouped so that the average tumor volume was about 146 mm³, and PBS (vehicle), 0.2 mg/kg H01, 5 mg/kg H01, 0.2 mg/kg trastuzumab, and 5 mg/kg trastuzumab were administered intraperitoneally (I.P.) twice a week for a total of 6 weeks (Figure 35). Since there are no antibodies present in the blood of the SCID mice, 50 mg/kg Intravenous Immunoglobulin (IVIG; LIV-r, SK Plasma) was administered to all mice twice a week for 6 weeks to simulate the actual human blood environment (Figure 35). The analysis showed that H01 induces superior antitumor activity compared to trastuzumab when the antibodies were administered at 5 mg/kg and at 0.2 mg/kg (Figure 35).

### Example 16. Evaluation of antitumor activity in CT26-HER2 syngeneic mouse model

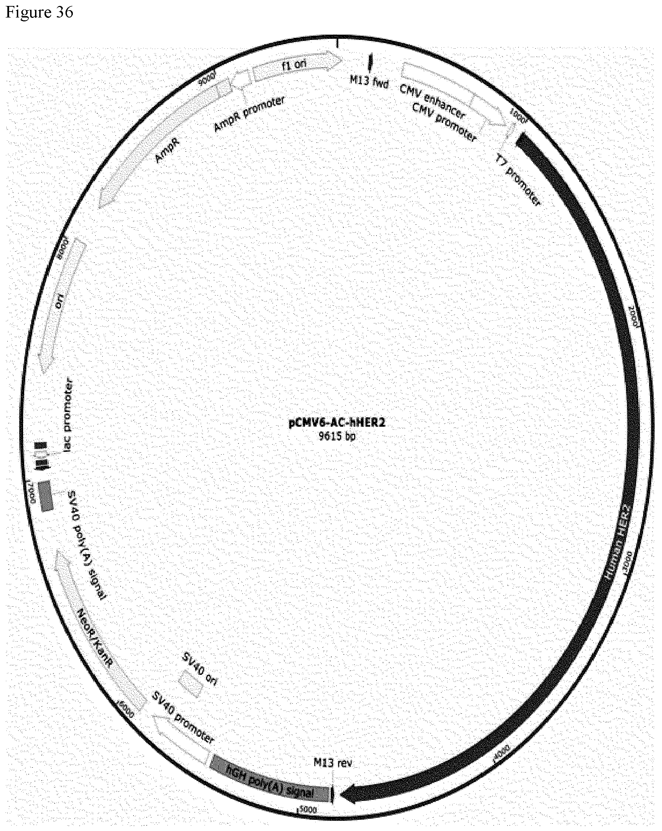

The nucleotide (SEQ ID NO: 566, Table 40) encoding the human HER2 protein (SEQ ID NO: 567, Table 40) was cloned into a protein expression vector (ORIGENE, PS100020) containing a neomycin-resistance gene to construct the human HER2 expression vector pCMV6-AC-hHER2 (Figure 36, Table 40).

**[Table 40]**

| **Name** | **Sequence** | **SEQ ID NO** |
|---|---|---|
| Human HER2 nucleotide sequence | ATGGAGCTGGCGGCCTTGTGCCGCTGGGGGCTCCTCCTCGCCCTCTTGCCCCCCGGAGC CGCGAGCACCCAAGTGTGCACCGGCACAGACATGAAGCTGCGGCTCCCTGCCAGTCCCG AGACCCACCTGGACATGCTCCGCCACCTCTACCAGGGCTGCCAGGTGGTGCAGGGAAAC CTGGAACTCACCTACCTGCCCACCAATGCCAGCCTGTCCTTCCTGCAGGATATCCAGGA GGTGCAGGGCTACGTGCTCATCGCTCACAACCAAGTGAGGCAGGTCCCACTGCAGAGGC TGCGGATTGTGCGAGGCACCCAGCTCTTTGAGGACAACTATGCCCTGGCCGTGCTAGAC AATGGAGACCCGCTGAACAATACCACCCCTGTCACAGGGGCCTCCCCAGGAGGCCTGCG GGAGCTGCAGCTTCGAAGCCTCACAGAGATCTTGAAAGGAGGGGTCTTGATCCAGCGGA ACCCCCAGCTCTGCTACCAGGACACGATTTTGTGGAAGGACATCTTCCACAAGAACAAC CAGCTGGCTCTCACACTGATAGACACCAACCGCTCTCGGGCCTGCCACCCCTGTTCTCC GATGTGTAAGGGCTCCCGCTGCTGGGGAGAGAGTTCTGAGGATTGTCAGAGCCTGACGC GCACTGTCTGTGCCGGTGGCTGTGCCCGCTGCAAGGGGCCACTGCCCACTGACTGCTGC CATGAGCAGTGTGCTGCCGGCTGCACGGGCCCCAAGCACTCTGACTGCCTGGCCTGCCT CCACTTCAACCACAGTGGCATCTGTGAGCTGCACTGCCCAGCCCTGGTCACCTACAACA CAGACACGTTTGAGTCCATGCCCAATCCCGAGGGCCGGTATACATTCGGCGCCAGCTGT GTGACTGCCTGTCCCTACAACTACCTTTCTACGGACGTGGGATCCTGCACCCTCGTCTG CCCCCTGCACAACCAAGAGGTGACAGCAGAGGATGGAACACAGCGGTGTGAGAAGTGCA GCAAGCCCTGTGCCCGAGTGTGCTATGGTCTGGGCATGGAGCACTTGCGAGAGGTGAGG GCAGTTACCAGTGCCAATATCCAGGAGTTTGCTGGCTGCAAGAAGATCTTTGGGAGCCT GGCATTTCTGCCGGAGAGCTTTGATGGGGACCCAGCCTCCAACACTGCCCCGCTCCAGC CAGAGCAGCTCCAAGTGTTTGAGACTCTGGAAGAGATCACAGGTTACCTATACATCTCA GCATGGCCGGACAGCCTGCCTGACCTCAGCGTCTTCCAGAACCTGCAAGTAATCCGGGG ACGAATTCTGCACAATGGCGCCTACTCGCTGACCCTGCAAGGGCTGGGCATCAGCTGGC TGGGGCTGCGCTCACTGAGGGAACTGGGCAGTGGACTGGCCCTCATCCACCATAACACC CACCTCTGCTTCGTGCACACGGTGCCCTGGGACCAGCTCTTTCGGAACCCGCACCAAGC TCTGCTCCACACTGCCAACCGGCCAGAGGACGAGTGTGTGGGCGAGGGCCTGGCCTGCC ACCAGCTGTGCGCCCGAGGGCACTGCTGGGGTCCAGGGCCCACCCAGTGTGTCAACTGC AGCCAGTTCCTTCGGGGCCAGGAGTGCGTGGAGGAATGCCGAGTACTGCAGGGGCTCCC CAGGGAGTATGTGAATGCCAGGCACTGTTTGCCGTGCCACCCTGAGTGTCAGCCCCAGA ATGGCTCAGTGACCTGTTTTGGACCGGAGGCTGACCAGTGTGTGGCCTGTGCCCACTAT AAGGACCCTCCCTTCTGCGTGGCCCGCTGCCCCAGCGGTGTGAAACCTGACCTCTCCTA CATGCCCATCTGGAAGTTTCCAGATGAGGAGGGCGCATGCCAGCCTTGCCCCATCAACT GCACCCACTCCTGTGTGGACCTGGATGACAAGGGCTGCCCCGCCGAGCAGAGAGCCAGC CCTCTGACGTCCATCATCTCTGCGGTGGTTGGCATTCTGCTGGTCGTGGTCTTGGGGGT GGTCTTTGGGATCCTCATCAAGCGACGGCAGCAGAAGATCCGGAAGTACACGATGCGGA GACTGCTGCAGGAAACGGAGCTGGTGGAGCCGCTGACACCTAGCGGAGCGATGCCCAAC CAGGCGCAGATGCGGATCCTGAAAGAGACGGAGCTGAGGAAGGTGAAGGTGCTTGGATC TGGCGCTTTTGGCACAGTCTACAAGGGCATCTGGATCCCTGATGGGGAGAATGTGAAAA | 566 |
| | TTCCAGTGGCCATCAAAGTGTTGAGGGAAAACACATCCCCCAAAGCCAACAAAGAAATC TTAGACGAAGCATACGTGATGGCTGGTGTGGGCTCCCCATATGTCTCCCGCCTTCTGGG CATCTGCCTGACATCCACGGTGCAGCTGGTGACACAGCTTATGCCCTATGGCTGCCTCT TAGACCATGTCCGGGAAAACCGCGGACGCCTGGGCTCCCAGGACCTGCTGAACTGGTGT ATGCAGATTGCCAAGGGGATGAGCTACCTGGAGGATGTGCGGCTCGTACACAGGGACTT GGCCGCTCGGAACGTGCTGGTCAAGAGTCCCAACCATGTCAAAATTACAGACTTCGGGC TGGCTCGGCTGCTGGACATTGACGAGACAGAGTACCATGCAGATGGGGGCAAGGTGCCC ATCAAGTGGATGGCGCTGGAGTCCATTCTCCGCCGGCGGTTCACCCACCAGAGTGATGT GTGGAGTTATGGTGTGACTGTGTGGGAGCTGATGACTTTTGGGGCCAAACCTTACGATG GGATCCCAGCCCGGGAGATCCCTGACCTGCTGGAAAAGGGGGAGCGGCTGCCCCAGCCC CCCATCTGCACCATTGATGTCTACATGATCATGGTCAAATGTTGGATGATTGACTCTGA ATGTCGGCCAAGATTCCGGGAGTTGGTGTCTGAATTCTCCCGCATGGCCAGGGACCCCC AGCGCTTTGTGGTCATCCAGAATGAGGACTTGGGCCCAGCCAGTCCCTTGGACAGCACC TTCTACCGCTCACTGCTGGAGGACGATGACATGGGGGACCTGGTGGATGCTGAGGAGTA TCTGGTACCCCAGCAGGGCTTCTTCTGTCCAGACCCTGCCCCGGGCGCTGGGGGCATGG TCCACCACAGGCACCGCAGCTCATCTACCAGGAGTGGCGGTGGGGACCTGACACTAGGG CTGGAGCCCTCTGAAGAGGAGGCCCCCAGGTCTCCACTGGCACCCTCCGAAGGGGCTGG CTCCGATGTATTTGATGGTGACCTGGGAATGGGGGCAGCCAAGGGGCTGCAAAGCCTCC CCACACATGACCCCAGCCCTCTACAGCGGTACAGTGAGGACCCCACAGTACCCCTGCCC TCTGAGACTGATGGCTACGTTGCCCCCCTGACCTGCAGCCCCCAGCCTGAATATGTGAA CCAGCCAGATGTTCGGCCCCAGCCCCCTTCGCCCCGAGAGGGCCCTCTGCCTGCTGCCC GACCTGCTGGTGCCACTCTGGAAAGGCCCAAGACTCTCTCCCCAGGGAAGAATGGGGTC GTCAAAGACGTTTTTGCCTTTGGGGGTGCCGTGGAGAACCCCGAGTACTTGACACCCCA GGGAGGAGCTGCCCCTCAGCCCCACCCTCCTCCTGCCTTCAGCCCAGCCTTCGACAACC TCTATTACTGGGACCAGGACCCACCAGAGCGGGGGGCTCCACCCAGCACCTTCAAAGGG ACACCTACGGCAGAGAACCCAGAGTACCTGGGTCTGGACGTGCCAGTG | |
| Human HER2 protein sequence | MELAALCRWGLLLALLPPGAASTQVCTGTDMKLRLPASPETHLDMLRHLYQGCQVVQGN LELTYLPTNASLSFLQDIQEVQGYVLIAHNQVRQVPLQRLRIVRGTQLFEDNYALAVLD NGDPLNNTTPVTGASPGGLRELQLRSLTEILKGGVLIQRNPQLCYQDTILWKDIFHKNN QLALTLIDTNRSRACHPCSPMCKGSRCWGESSEDCQSLTRTVCAGGCARCKGFLPTDCC HEQCAAGCTGPKHSDCLACLHFNHSGICELHCPALVTYNTDTFESMPNPEGRYTFGASC VTACPYNYLSTDVGSCTLVCPLHNQEVTAEDGTQRCEKCSKPCARVCYGLGMEHLREVR AVTSANIQEFAGCKKIFGSLAFLPESFDGDPASNTAPLQPEQLQVFETLEEITGYLYIS AWPDSLPDLSVFQNLQVIRGRILHNGAYSLTLQGLGISWLGLRSLRELGSGLALIHHNT HLCFVHTVPWDQLFRNPHQALLHTANRPEDECVGEGLACHQLCARGHCWGPGPTQCVNC SQFLRGQECVEECRVLQGLPREYVNARHCLPCHPECQPQNGSVTCFGPEADQCVACAHY KDPPFCVARCPSGVKPDLSYMPIWKFPDEEGACQPCPINCTHSCVDLDDKGCPAEQRAS PLTSIISAVVGILLVVVLGVVFGILIKRRQQKIRKYTMRRLLQETELVEPLTPSGAMPN QAQMRILKETELRKVKVLGSGAFGTVYKGIWIPDGENVKIPVAIKVLRENTSPKANKEI LDEAYVMAGVGSPYVSRLLGICLTSTVQLVTQLMPYGCLLDHVRENRGRLGSQDLLNWC | 567 |
| | | |

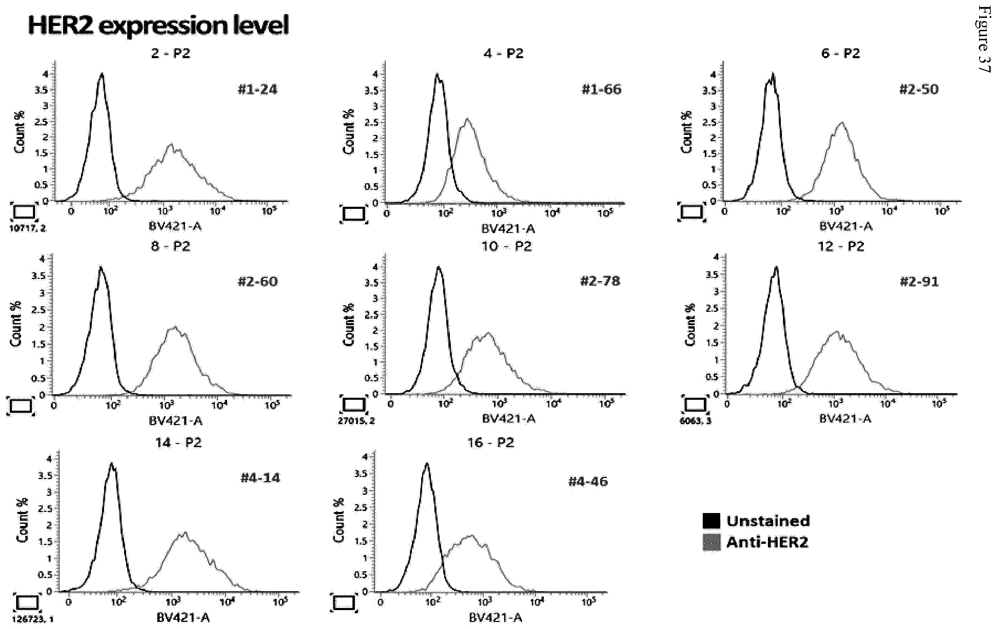

The mouse large intestine-derived CT26 cancer cells were transfected with the pCMV6-AC-hHER2 human HER2 expression vector using the lipofectamine 2000 transfection reagent (Invitrogen, 11668-019). Only cells transfected with the pCMV6-AC-hHER2 human HER2 expression vector were selected by incubating the transfected cells in culture medium containing 1 mg/mL G418 (Invivogen, ant-gn-5) for 14 days. The top 3% clones in terms of HER2 expression was sorted into a 96-well plate (ThermoFisher, 167008) with 1 cell per well using SH800S Cell Sorter (SONY). Selection was performed by incubation in G418-containing medium for 21 days, and a total of 8 CT26 mouse large intestine cancer cell line clones expressing human HER2 were obtained, and human HER2 expression in these cells was monitored by flow cytometer (BD Biosciences, FACSverse) analysis after staining with the anti-human HER2-BV421 (BD, 744811) (Figure 37, Table 41).

**[Table 41]**

| ΔGMFI (of unstained control) | |
|---|---|
| #1-24 | 27.3 |
| #1-66 | 3.7 |
| #2-50 | 19.1 |
| #2-60 | 25.9 |
| #2-78 | 7.5 |
| #2-91 | 17.5 |
| #4-14 | 26.8 |
| #4-46 | 6.5 |

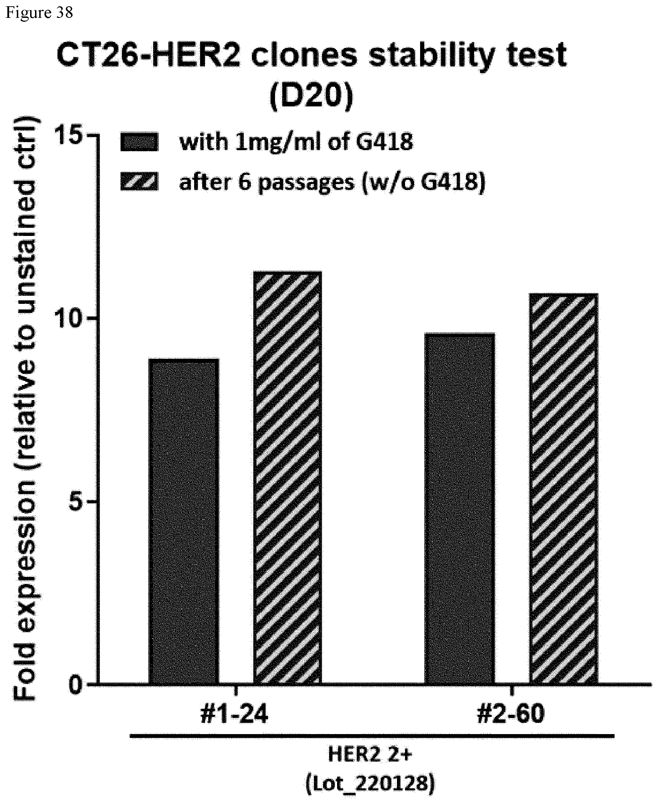

After subculturing six times for 20 days in a G418-free environment, the cells were stained with the anti-human HER2-BV421 (BD, 744811), and the level of human HER2 expression was measured. It was shown that the level of human HER2 expression was not reduced in cells grown without G418 compared to cells grown with G418 (Figure 38, Table 42).

**[Table 42]**

| ΔGMFI (of unstained control) | |
|---|---|
| #1-24 | 27.3 |
| #1-66 | 3.7 |
| #2-50 | 19.1 |
| #2-60 | 25.9 |
| #2-78 | 7.5 |
| #2-91 | 17.5 |
| #4-14 | 26.8 |
| #4-46 | 6.5 |

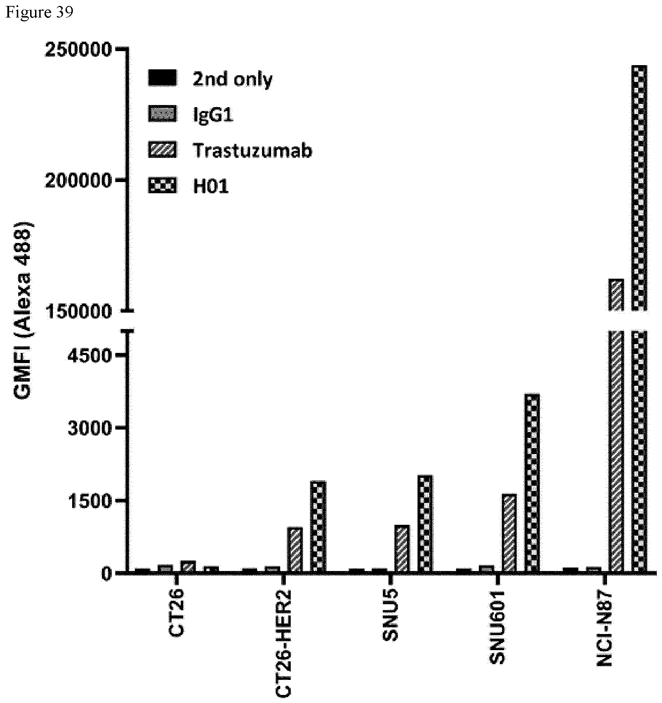

To compare the cell surface Fc loads among the parental CT26, CT26-HER2 cell line (Clone #2-60) and human cancer cell lines (SNU5, SNU601, and NCI-N87), each cell was allowed to bind to 100 nM human IgG1 (Bio X cell, BE0297), 100 nM trastuzumab (TRA), and 100 nM H01 antibody at 4°C for 30 minutes in a 96-well v-bottom plate (Corning, 3363). Thereafter, they were treated with the Alexa 488 fluorescence-conjugated anti-human IgG Fcγ Fab antibody (Jackson ImmunoResearch, 109-547-008), and the Fc loads on the cells were quantified using a flow cytometer (Figure 39, Table 43). CT26-HER2 cell line (Clone #2-60) was shown to express human HER2 at a level similar to that of SNU5 (Figure 39, Table 43). In addition, it was shown that in the CT26-HER2 cell line, treatment of 100 nM H01 results in increased Fc loads on the cell surface compared to that of 100 nM trastuzumab (TRA).

**[Table 43]**

| GMFI | CT26 | CT26-HER2 (#2-60) | SNU5 | SNU601 | NCI-N87 |
|---|---|---|---|---|---|
| 2nd only | 99 | 102 | 94 | 97 | 116 |
| 100 nM IgG1 isotype | 176 | 150 | 101 | 173 | 132 |
| 100 nM Trastuzumab | 252 | 959 | 991 | 1642 | 162433 |
| 100 nM H01 | 147 | 1907 | 2028 | 3703 | 243897 |

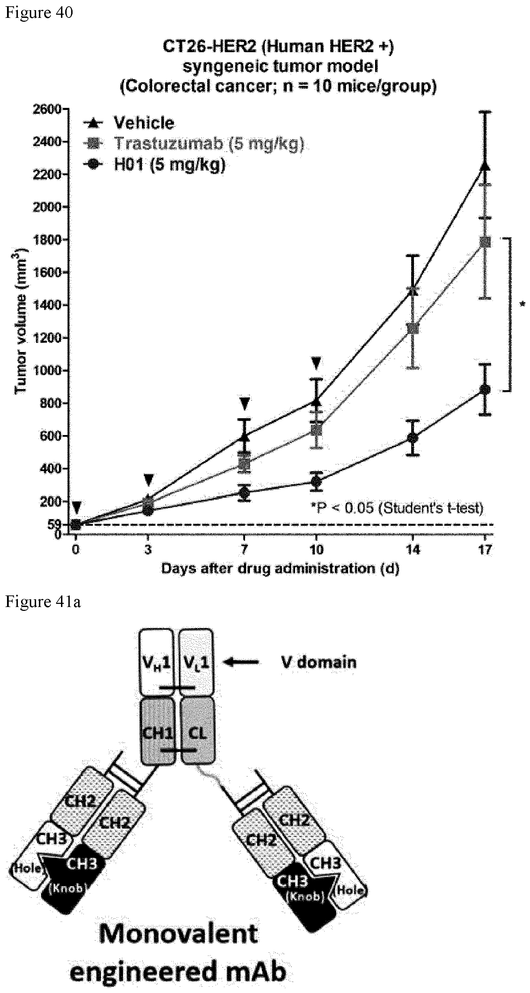

Efficacy in the CT26-HER2 (Clone #2-60) syngeneic mouse model was evaluated using 6-week-old female Balb/c mice (ORIENT BIO INC.). PBS (vehicle), 5 mg/kg trastuzumab, and 5 mg/kg H01 were administered intraperitoneally (I.P.) twice a week for a total of 2 weeks (Figure 40). The analysis showed that H01 induces superior antitumor activity compared to trastuzumab (Figure 40).

### Example 17. Design, preparation and analysis of novel antibody targeting glypican-3 (GPC3)

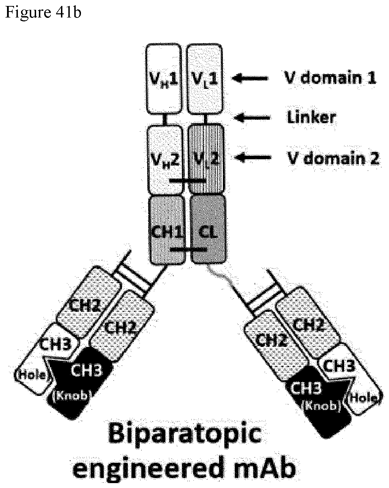

The variant light chain and heavy chain polypeptide sequences of the antibodies that specifically recognize the glypican-3 (GPC-3) protein are shown in Table 44. For GPM01, expression vectors consisting of the sequences corresponding to Fc-Hole (SEQ ID NO: 7), GPM01 HC (SEQ ID NO: 67), and GPM01 LC (SEQ ID NO: 68) were co-transfected into EXPICHO-S^{™} (Gibco, A29127) (Figure 41a, Table 44), and purification and analysis were performed in the same manner as described in Example 1. Expression, purification and analysis were performed for GPM02, GPM04, GPB01, GPB03, GPB04, and GPB06 in the same manner as mentioned above (Figures 41a to 41b, Table 44). GPM01, GPM02, and GPM04 bind monovalently to different epitopes of the antigen and have structures consisting of two Fc domains (Figure 41a). GPB01, GPB03, GPB04, and GPB06 have structures in which the variable regions of GPM01, GPM02, and GPM04 are linked with a polypeptide linker (SEQ ID NO: 48, SEQ ID NO: 50), and bind biparatopically to GPC-3, and have two Fc domains (Figure 41b).

**[Table 44]**

| Name | Sequence | SEQ ID NO |
|---|---|---|
| GPM01 HC | EVQLVESGGGLVKPGGSLKLSCAASGFTFSRYAMSWVRQIPEKCLE WVAAIDSSGGDTYYLDTVKDRFTISRDNANNTLHLQMRSLRSEDTA LYYCVRQGGAYWGQGTLVTVSSASTKGPSVFPLAPSSKSTSGGTAAL GCLVKDYFPEPVTVSWNSGALTSGVHTFPAVLQSSGLYSLSSVVTVP SSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKAL PAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | 67 |
| GPM01 LC | DVVMTQTPLTLSVTIGQPASISCKSSQSLLDSDGKTYLNWLLQRPGQS PKRLIYLVSKLDSGAPDRFTGSGSGTDFTLKISRVEAEDLGIYYCWQG THFPLTFGCGTKLEIKRTVAAPSVFIFPPSDEQLKSGTASVVCLLNNFY PREAKVQWKVDNALQSGNSQESVTEQDSKDSTYSLSSTLTLSKADY EKHKVYACEVTHQGLSSPVTKSFNRGECGGGGSGGGGSGGGGSEPK SSDKTHTCPPCPAPELLGGPSVFLFPPKPKDTLMISRTPEVTCVVVDVS HEDPEVKFNWYVDGVEVHNAKTKPREEQYNSTYRVVSVLTVLHQD WLNGKEYKCKVSNKALPAPIEKTISKAKGQPREPQVYTLPPCREEMT KNQVSLWCLVKGFYPSDIAVEWESNGQPENNYKTTPPVLDSDGSFFL YSKLTVDKSRWQQGNVFSCSVMHEALHNHYTQKSLSLSPGK | 68 |
| GPM02 HC | QVQLVQSGAEVKKPGASVKVSCKASGYTFTDYEMHWVRQAPGQCL EWMGALDPKTGDTAYSQKFKGRVTLTADKSTSTAYMELSSLTSEDT AVYYCTRFYSYTYWGQGTLVTVSSASTKGPSVFPLAPSSKSTSGGTA ALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKA LPAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | 69 |
| GPM02 LC | DVVMTQSPLSLPVTPGEPASISCRSSQSLVHSNRNTYLHWYLQKPGQ SPQLLIYKVSNRFSGVPDRFSGSGSGTDFTLKISRVEAEDVGVYYCSQ NTHVPPTFGCGTKLEIKRTVAAPSVFIFPPSDEQLKSGTASVVCLLNNF YPREAKVQWKVDNALQSGNSQESVTEQDSKDSTYSLSSTLTLSKAD YEKHKVYACEVTHQGLSSPVTKSFNRGECGGGGSGGGGSGGGGSEP KSSDKTHTCPPCPAPELLGGPSVFLFPPKPKDTLMISRTPEVTCVVVD VSHEDPEVKFNWYVDGVEVHNAKTKPREEQYNSTYRVVSVLTVLH QDWLNGKEYKCKVSNKALPAPIEKTISKAKGQPREPQVYTLPPCREE | 70 |
| | MTKNQVSLWCLVKGFYPSDIAVEWESNGQPENNYKTTPPVLDSDGS FFLYSKLTVDKSRWQQGNVFSCSVMHEALHNHYTQKSLSLSPGK | |
| GPM04 HC | EVQLVETGGGLVQPKGSLKLSCAASGFTFNASAMNWVRQAPGKCLE WVARIRSKSNNYAIYYADSVKDRFTISRDDSQSMLYLQMNNLKTED TAMYYCVRDPGYYGNPWFAYWGQGTLVTVSSASTKGPSVFPLAPSS KSTSGGTAALGCLVKDYFPEPVTVSWNSGALTSGVHTFPAVLQSSGL YSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVK FNWYVDGVEVHNAKTKPREEQYNSTYRVVSVLTVLHQDWLNGKEY KCKVSNKALPAPIEKTISKAKGQPREPQVYTLPPCREEMTKNQVSLW CLVKGFYPSDIAVEWESNGQPENNYKTTPPVLDSDGSFFLYSKLTVD KSRWQQGNVFSCSVMHEALHNHYTQKSLSLSPGK | 71 |
| GPM04 LC | QIVLTQSPAIMSAFPGEKVTMTCSASSSVSYMYWYQQKSGSSPRLLIY DTSNLASGVPVRFSGSGSGTSYSLTISRMEAEDAATYYCQQWSSYPL TFGCGTKLEIKRTVAAPSVFIFPPSDEQLKSGTASVVCLLNNFYPREA KVQWKVDNALQSGNSQESVTEQDSKDSTYSLSSTLTLSKADYEKHK VYACEVTHQGLSSPVTKSFNRGECGGGGSGGGGSGGGGSEPKSSDK THTCPPCPAPELLGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPCREEMTKNQ VSLWCLVKGFYPSDIAVEWESNGQPENNYKTTPPVLDSDGSFFLYSK LTVDKSRWQQGNVFSCSVMHEALHNHYTQKSLSLSPGK | 72 |
| GPBO1 HC | QVQLVQSGAEVKKPGASVKVSCKASGYTFTDYEMHWVRQAPGQGL EWMGALDPKTGDTAYSQKFKGRVTLTADKSTSTAYMELSSLTSEDT AVYYCTRFYSYTYWGQGTLVTVSSASTKGPSVFPLAPEVQLVESGG GLVKPGGSLKLSCAASGFTFSRYAMSWVRQIPEKCLEWVAAIDSSGG DTYYLDTVKDRFTISRDNANNTLHLQMRSLRSEDTALYYCVRQGGA YWGQGTLVTVSSASTKGPSVFPLAPSSKSTSGGTAALGCLVKDYFPE PVTVSWNSGALTSGVHTFPAVLQSSGLYSLSSVVTVPSSSLGTQTYIC NVNHKPSNTKVDKKVEPKSCDKTHTCPPCPAPELLGGPSVFLFPPKP KDTLMISRTPEVTCVVVDVSHEDPEVKFNWYVDGVEVHNAKTKPRE EQYNSTYRVVSVLTVLHQDWLNGKEYKCKVSNKALPAPIEKTISKA KGQPREPQVYTLPPCREEMTKNQVSLWCLVKGFYPSDIAVEWESNG QPENNYKTTPPVLDSDGSFFLYSKLTVDKSRWQQGNVFSCSVMHEA LHNHYTQKSLSLSPGK | 73 |
| GPBO1 LC | DVVMTQSPLSLPVTPGEPASISCRSSQSLVHSNRNTYLHWYLQKPGQ SPQLLIYKVSNRFSGVPDRFSGSGSGTDFTLKISRVEAEDVGVYYCSQ NTHVPPTFGQGTKLEIKRTVAAPSVFIFPPDVVMTQTPLTLSVTIGQPA SISCKSSQSLLDSDGKTYLNWLLQRPGQSPKRLIYLVSKLDSGAPDRF TGSGSGTDFTLKISRVEAEDLGIYYCWQGTHFPLTFGCGTKLEIKRTV AAPSVFIFPPSDEQLKSGTASVVCLLNNFYPREAKVQWKVDNALQSG NSQESVTEQDSKDSTYSLSSTLTLSKADYEKHKVYACEVTHQGLSSP VTKSFNRGECGGGGSGGGGSGGGGSEPKSSDKTHTCPPCPAPELLGG PSVFLFPPKPKDTLMISRTPEVTCVVVDVSHEDPEVKFNWYVDGVEV HNAKTKPREEQYNSTYRVVSVLTVLHQDWLNGKEYKCKVSNKALP | 74 |
| | APIEKTISKAKGQPREPQVYTLPPCREEMTKNQVSLWCLVKGFYPSDI AVEWESNGQPENNYKTTPPVLDSDGSFFLYSKLTVDKSRWQQGNVF SCSVMHEALHNHYTQKSLSLSPGK | |
| GPB03 HC | QVQLVQSGAEVKKPGASVKVSCKASGYTFTDYEMHWVRQAPGQGL EWMGALDPKTGDTAYSQKFKGRVTLTADKSTSTAYMELSSLTSEDT AVYYCTRFYSYTYWGQGTLVTVSSASTKGPSVFPLAPEVQLVETGG GLVQPKGSLKLSCAASGFTFNASAMNWVRQAPGKCLEWVARIRSKS NNYAIYYADSVKDRFTISRDDSQSMLYLQMNNLKTEDTAMYYCVR DPGYYGNPWFAYWGQGTLVTVSSASTKGPSVFPLAPSSKSTSGGTA ALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGV EVHNAKTKPREEQYNSTYRVVSVLTVLHQDWLNGKEYKCKVSNKA LPAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | 75 |
| GPB03 LC | DVVMTQSPLSLPVTPGEPASISCRSSQSLVHSNRNTYLHWYLQKPGQ SPQLLIYKVSNRFSGVPDRFSGSGSGTDFTLKISRVEAEDVGVYYCSQ NTHVPPTFGQGTKLEIKRTVAAPSVFIFPPQIVLTQSPAIMSAFPGEKV TMTCSASSSVSYMYWYQQKSGSSPRLLIYDTSNLASGVPVRFSGSGS GTSYSLTISRMEAEDAATYYCQQWSSYPLTFGCGTKLEIKRTVAAPS VFIFPPSDEQLKSGTASVVCLLNNFYPREAKVQWKVDNALQSGNSQE SVTEQDSKDSTYSLSSTLTLSKADYEKHKVYACEVTHQGLSSPVTKS FNRGECGGGGSGGGGSGGGGSEPKSSDKTHTCPPCPAPELLGGPSVF LFPPKPKDTLMISRTPEVTCVVVDVSHEDPEVKFNWYVDGVEVHNA KTKPREEQYNSTYRVVSVLTVLHQDWLNGKEYKCKVSNKALPAPIE KTISKAKGQPREPQVYTLPPCREEMTKNQVSLWCLVKGFYPSDIAVE WESNGQPENNYKTTPPVLDSDGSFFLYSKLTVDKSRWQQGNVFSCS VMHEALHNHYTQKSLSLSPGK | 76 |
| GPB04 HC | EVQLVESGGGLVKPGGSLKLSCAASGFTFSRYAMSWVRQIPEKILEW VAAIDSSGGDTYYLDTVKDRFTISRDNANNTLHLQMRSLRSEDTALY YCVRQGGAYWGQGTLVTVSSASTKGPSVFPLAPQVQLVQSGAEVKK PGASVKVSCKASGYTFTDYEMHWVRQAPGQCLEWMGALDPKTGDT AYSQKFKGRVTLTADKSTSTAYMELSSLTSEDTAVYYCTRFYSYTY WGQGTLVTVSSASTKGPSVFPLAPSSKSTSGGTAALGCLVKDYFPEP VTVSWNSGALTSGVHTFPAVLQSSGLYSLSSVVTVPSSSLGTQTYICN VNHKPSNTKVDKKVEPKSCDKTHTCPPCPAPELLGGPSVFLFPPKPK DTLMISRTPEVTCVVVDVSHEDPEVKFNWYVDGVEVHNAKTKPREE QYNSTYRVVSVLTVLHQDWLNGKEYKCKVSNKALPAPIEKTISKAK GQPREPQVYTLPPCREEMTKNQVSLWCLVKGFYPSDIAVEWESNGQ PENNYKTTPPVLDSDGSFFLYSKLTVDKSRWQQGNVFSCSVMHEAL HNHYTQKSLSLSPGK | 77 |
| GPB04 LC | DVVMTQTPLTLSVTIGQPASISCKSSQSLLDSDGKTYLNWLLQRPGQS PKRLIYLVSKLDSGAPDRFTGSGSGTDFTLKISRVEAEDLGIYYCWQG THFPLTFGAGTKLEIKRTVAAPSVFIFPPDVVMTQSPLSLPVTPGEPASI | 78 |
| | SCRSSQSLVHSNRNTYLHWYLQKPGQSPQLLIYKVSNRFSGVPDRFS GSGSGTDFTLKISRVEAEDVGVYYCSQNTHVPPTFGCGTKLEIKRTV AAPSVFIFPPSDEQLKSGTASVVCLLNNFYPREAKVQWKVDNALQSG NSQESVTEQDSKDSTYSLSSTLTLSKADYEKHKVYACEVTHQGLSSP VTKSFNRGECGGGGSGGGGSGGGGSEPKSSDKTHTCPPCPAPELLGG PSVFLFPPKPKDTLMISRTPEVTCVVVDVSHEDPEVKFNWYVDGVEV HNAKTKPREEQYNSTYRVVSVLTVLHQDWLNGKEYKCKVSNKALP APIEKTISKAKGQPREPQVYTLPPCREEMTKNQVSLWCLVKGFYPSDI AVEWESNGQPENNYKTTPPVLDSDGSFFLYSKLTVDKSRWQQGNVF SCSVMHEALHNHYTQKSLSLSPGK | |
| GPB06 HC | EVQLVETGGGLVQPKGSLKLSCAASGFTFNASAMNWVRQAPGKGLE WVARIRSKSNNYAIYYADSVKDRFTISRDDSQSMLYLQMNNLKTED TAMYYCVRDPGYYGNPWFAYWGQGTLVTVSSASTKGPSVFPLAPQ VQLVQSGAEVKKPGASVKVSCKASGYTFTDYEMHWVRQAPGQCLE WMGALDPKTGDTAYSQKFKGRVTLTADKSTSTAYMELSSLTSEDTA VYYCTRFYSYTYWGQGTLVTVSSASTKGPSVFPLAPSSKSTSGGTAA LGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKAL PAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | 79 |
| GPB06 LC | QIVLTQSPAIMSAFPGEKVTMTCSASSSVSYMYWYQQKSGSSPRLLIY DTSNLASGVPVRFSGSGSGTSYSLTISRMEAEDAATYYCQQWSSYPL TFGGGTKLEIKRTVAAPSVFIFPPDVVMTQSPLSLPVTPGEPASISCRSS QSLVHSNRNTYLHWYLQKPGQSPQLLIYKVSNRFSGVPDRFSGSGSG TDFTLKISRVEAEDVGVYYCSQNTHVPPTFGCGTKLEIKRTVAAPSVF IFPPSDEQLKSGTASVVCLLNNFYPREAKVQWKVDNALQSGNSQESV TEQDSKDSTYSLSSTLTLSKADYEKHKVYACEVTHQGLSSPVTKSFN RGECGGGGSGGGGSGGGGSEPKSSDKTHTCPPCPAPELLGGPSVFLFP PKPKDTLMISRTPEVTCVVVDVSHEDPEVKFNWYVDGVEVHNAKTK PREEQYNSTYRVVSVLTVLHQDWLNGKEYKCKVSNKALPAPIEKTIS KAKGQPREPQVYTLPPCREEMTKNQVSLWCLVKGFYPSDIAVEWES NGQPENNYKTTPPVLDSDGSFFLYSKLTVDKSRWQQGNVFSCSVMH EALHNHYTQKSLSLSPGK | 80 |

**[Table 45]**

| Name | Sequence | SEQ ID NO |
|---|---|---|
| GPM01 HC | GAAGTTCAGCTTGTCGAAAGTGGCGGAGGTCTTGTAAAGCCAGGA GGGTCCCTGAAGTTGAGTTGTGCTGCCTCTGGCTTCACCTTTTCAC GGTATGCCATGTCCTGGGTTCGACAGATACCTGAGAAGTGTCTTG AATGGGTGGCCGCAATAGACAGTAGCGGTGGTGACACCTATTACC TCGACACAGTCAAAGACCGCTTTACTATCTCACGCGATAACGCCA ACAATACCCTGCACTTGCAGATGCGATCACTTCGTTCAGAAGACA CTGCTCTTTACTATTGTGTACGCCAAGGGGGAGCATACTGGGGTC | 504 |
| | AGGGAACACTGGTTACCGTGTCTTCAGCTAGCACCAAAGGACCTA GTGTTTTTCCTCTTGCCCCTTCCTCAAAGTCTACCTCTGGGGGGAC AGCCGCTCTGGGCTGCCTGGTCAAGGATTATTTCCCAGAGCCTGT CACTGTCAGTTGGAACTCTGGAGCCTTGACTTCTGGTGTTCATACA TTTCCTGCTGTCCTTCAAAGCAGCGGCTTGTACTCATTGTCTTCTGT TGTGACAGTACCCTCAAGCAGCCTCGGCACTCAGACATACATCTG CAATGTCAACCACAAACCCTCAAATACAAAGGTAGATAAAAAAG TCGAACCAAAGTCTTGTGACAAAACTCACACGTGCCCACCGTGCC CAGCACCTGAACTCCTGGGGGGACCGTCAGTCTTCCTCTTCCCCCC AAAACCCAAGGACACCCTCATGATCTCCCGGACCCCTGAGGTCAC ATGCGTGGTGGTGGACGTGAGCCACGAAGACCCTGAGGTCAAGTT CAACTGGTACGTGGACGGCGTGGAGGTGCATAATGCCAAGACAA AGCCGCGGGAGGAGCAGTACAACAGCACGTACCGTGTGGTCAGC GTCCTCACCGTCCTGCACCAGGACTGGCTGAATGGCAAGGAGTAC AAGTGCAAGGTCTCCAACAAAGCCCTCCCAGCCCCCATCGAGAAA ACCATCTCCAAAGCCAAAGGGCAGCCCCGAGAACCACAGGTGTA CACCCTGCCCCCATGCCGGGAGGAGATGACCAAGAACCAGGTCA GCCTGTGGTGCCTGGTCAAAGGCTTCTATCCCAGCGACATCGCCG TGGAGTGGGAGAGCAATGGGCAGCCGGAGAACAACTACAAGACC ACGCCTCCCGTGCTGGACTCCGACGGCTCCTTCTTCCTCTACAGCA AGCTCACCGTGGACAAGAGCAGGTGGCAGCAGGGGAACGTCTTC TCATGCTCCGTGATGCATGAGGCTCTGCACAACCACTACACGCAG AAGAGCCTCTCCCTGTCTCCGGGTAAA | |
| GPM01 LC | GACGTGGTTATGACTCAGACTCCTCTGACTCTCTCCGTCACTATTG GTCAGCCCGCTTCCATATCATGTAAATCATCACAATCTCTTCTTGA TAGCGATGGCAAGACTTATTTGAACTGGTTGTTGCAACGCCCAGG TCAGAGCCCTAAGAGACTTATCTATTTGGTGAGCAAACTCGACAG CGGTGCACCCGATCGTTTTACCGGAAGCGGCAGCGGCACCGATTT CACACTGAAGATCAGTAGGGTCGAAGCTGAAGACCTGGGAATCT ACTACTGCTGGCAAGGTACTCACTTTCCCCTGACTTTCGGCTGCGG TACTAAACTTGAGATCAAACGTACGGTGGCAGCTCCCAGCGTTTT TATCTTTCCCCCATCCGACGAGCAGCTCAAGAGTGGCACTGCCTCT GTAGTTTGTTTGCTGAATAACTTCTATCCACGTGAAGCAAAAGTAC AGTGGAAGGTCGATAATGCCCTTCAGAGCGGTAACAGCCAAGAA AGTGTTACCGAGCAAGATTCCAAAGATTCCACTTACAGTCTGTCC AGCACATTGACACTGAGTAAGGCTGATTACGAAAAACACAAGGT GTACGCATGCGAGGTGACACACCAAGGTCTTTCATCTCCTGTAAC TAAGAGCTTTAACCGGGGAGAATGTGGTGGTGGGGGCAGCGGGG GCGGAGGTAGTGGAGGCGGCGGTAGTGAACCAAAGAGTAGTGAC AAAACTCACACGTGCCCACCGTGCCCAGCACCTGAACTCCTGGGG GGACCGTCAGTCTTCCTCTTCCCCCCAAAACCCAAGGACACCCTC ATGATCTCCCGGACCCCTGAGGTCACATGCGTGGTGGTGGACGTG AGCCACGAAGACCCTGAGGTCAAGTTCAACTGGTACGTGGACGGC GTGGAGGTGCATAATGCCAAGACAAAGCCGCGGGAGGAGCAGTA CAACAGCACGTACCGTGTGGTCAGCGTCCTCACCGTCCTGCACCA | 505 |
| | GGACTGGCTGAATGGCAAGGAGTACAAGTGCAAGGTCTCCAACA AAGCCCTCCCAGCCCCCATCGAGAAAACCATCTCCAAAGCCAAAG GGCAGCCCCGAGAACCACAGGTGTACACCCTGCCCCCATGCCGGG AGGAGATGACCAAGAACCAGGTCAGCCTGTGGTGCCTGGTCAAA GGCTTCTATCCCAGCGACATCGCCGTGGAGTGGGAGAGCAATGGG CAGCCGGAGAACAACTACAAGACCACGCCTCCCGTGCTGGACTCC GACGGCTCCTTCTTCCTCTACAGCAAGCTCACCGTGGACAAGAGC AGGTGGCAGCAGGGGAACGTCTTCTCATGCTCCGTGATGCATGAG GCTCTGCACAACCACTACACGCAGAAGAGCCTCTCCCTGTCTCCG GGTAAA | |
| GPM02 HC | CAGGTTCAGCTTGTGCAATCAGGCGCTGAAGTCAAAAAACCAGGA GCAAGCGTGAAAGTAAGCTGTAAGGCTTCCGGTTACACTTTCACC GATTACGAAATGCACTGGGTACGCCAGGCTCCTGGACAATGTCTG GAATGGATGGGCGCACTTGATCCAAAAACAGGTGACACTGCTTAC AGTCAAAAATTCAAAGGTCGTGTCACTCTTACAGCAGATAAGTCC ACCAGTACCGCTTATATGGAGCTTAGCTCCCTGACTTCCGAGGAC ACCGCCGTGTATTATTGTACAAGATTCTACTCATATACTTACTGGG GCCAAGGAACCCTGGTGACAGTGTCATCTGCTAGCACCAAAGGAC CTAGTGTTTTTCCTCTTGCCCCTTCCTCAAAGTCTACCTCTGGGGG GACAGCCGCTCTGGGCTGCCTGGTCAAGGATTATTTCCCAGAGCC TGTCACTGTCAGTTGGAACTCTGGAGCCTTGACTTCTGGTGTTCAT ACATTTCCTGCTGTCCTTCAAAGCAGCGGCTTGTACTCATTGTCTT CTGTTGTGACAGTACCCTCAAGCAGCCTCGGCACTCAGACATACA TCTGCAATGTCAACCACAAACCCTCAAATACAAAGGTAGATAAAA AAGTCGAACCAAAGTCTTGTGACAAAACTCACACGTGCCCACCGT GCCCAGCACCTGAACTCCTGGGGGGACCGTCAGTCTTCCTCTTCCC CCCAAAACCCAAGGACACCCTCATGATCTCCCGGACCCCTGAGGT CACATGCGTGGTGGTGGACGTGAGCCACGAAGACCCTGAGGTCA AGTTCAACTGGTACGTGGACGGCGTGGAGGTGCATAATGCCAAGA CAAAGCCGCGGGAGGAGCAGTACAACAGCACGTACCGTGTGGTC AGCGTCCTCACCGTCCTGCACCAGGACTGGCTGAATGGCAAGGAG TACAAGTGCAAGGTCTCCAACAAAGCCCTCCCAGCCCCCATCGAG AAAACCATCTCCAAAGCCAAAGGGCAGCCCCGAGAACCACAGGT GTACACCCTGCCCCCATGCCGGGAGGAGATGACCAAGAACCAGG TCAGCCTGTGGTGCCTGGTCAAAGGCTTCTATCCCAGCGACATCG CCGTGGAGTGGGAGAGCAATGGGCAGCCGGAGAACAACTACAAG ACCACGCCTCCCGTGCTGGACTCCGACGGCTCCTTCTTCCTCTACA GCAAGCTCACCGTGGACAAGAGCAGGTGGCAGCAGGGGAACGTC TTCTCATGCTCCGTGATGCATGAGGCTCTGCACAACCACTACACG CAGAAGAGCCTCTCCCTGTCTCCGGGTAAA | 506 |
| GPM02 LC | GATGTTGTTATGACTCAGTCACCTCTCTCACTTCCTGTAACCCCTG GTGAACCAGCCTCTATAAGCTGCCGGTCAAGTCAAAGCCTGGTTC ATAGCAACCGTAACACTTACCTTCACTGGTACTTGCAAAAACCTG GTCAGTCCCCACAACTCTTGATCTACAAAGTCTCCAATCGCTTCTC TGGAGTCCCTGACAGGTTTTCTGGTAGTGGATCAGGTACAGACTT | 507 |
| | CACTTTGAAAATCAGCCGCGTTGAGGCCGAGGACGTGGGAGTGTA TTATTGCTCTCAGAATACCCATGTACCCCCAACCTTTGGCTGTGGG ACTAAACTCGAGATTAAACGTACGGTGGCAGCTCCCAGCGTTTTT ATCTTTCCCCCATCCGACGAGCAGCTCAAGAGTGGCACTGCCTCT GTAGTTTGTTTGCTGAATAACTTCTATCCACGTGAAGCAAAAGTAC AGTGGAAGGTCGATAATGCCCTTCAGAGCGGTAACAGCCAAGAA AGTGTTACCGAGCAAGATTCCAAAGATTCCACTTACAGTCTGTCC AGCACATTGACACTGAGTAAGGCTGATTACGAAAAACACAAGGT GTACGCATGCGAGGTGACACACCAAGGTCTTTCATCTCCTGTAAC TAAGAGCTTTAACCGGGGAGAATGTGGTGGTGGGGGCAGCGGGG GCGGAGGTAGTGGAGGCGGCGGTAGTGAACCAAAGAGTAGTGAC AAAACTCACACGTGCCCACCGTGCCCAGCACCTGAACTCCTGGGG GGACCGTCAGTCTTCCTCTTCCCCCCAAAACCCAAGGACACCCTC ATGATCTCCCGGACCCCTGAGGTCACATGCGTGGTGGTGGACGTG AGCCACGAAGACCCTGAGGTCAAGTTCAACTGGTACGTGGACGGC GTGGAGGTGCATAATGCCAAGACAAAGCCGCGGGAGGAGCAGTA CAACAGCACGTACCGTGTGGTCAGCGTCCTCACCGTCCTGCACCA GGACTGGCTGAATGGCAAGGAGTACAAGTGCAAGGTCTCCAACA AAGCCCTCCCAGCCCCCATCGAGAAAACCATCTCCAAAGCCAAAG GGCAGCCCCGAGAACCACAGGTGTACACCCTGCCCCCATGCCGGG AGGAGATGACCAAGAACCAGGTCAGCCTGTGGTGCCTGGTCAAA GGCTTCTATCCCAGCGACATCGCCGTGGAGTGGGAGAGCAATGGG CAGCCGGAGAACAACTACAAGACCACGCCTCCCGTGCTGGACTCC GACGGCTCCTTCTTCCTCTACAGCAAGCTCACCGTGGACAAGAGC AGGTGGCAGCAGGGGAACGTCTTCTCATGCTCCGTGATGCATGAG GCTCTGCACAACCACTACACGCAGAAGAGCCTCTCCCTGTCTCCG GGTAAA | |
| GPM04 HC | GAAGTGCAACTGGTCGAAACAGGCGGGGGACTGGTACAGCCCAA GGGATCTTTGAAACTTAGTTGTGCTGCTAGTGGGTTTACATTCAAT GCCTCCGCAATGAACTGGGTAAGACAAGCTCCTGGCAAGTGCCTG GAATGGGTGGCCCGTATTCGCTCTAAAAGTAATAACTACGCTATT TATTACGCTGATTCTGTAAAGGATCGGTTTACAATAAGTCGGGAC GACAGCCAATCCATGCTGTATCTCCAAATGAATAACCTGAAAACA GAGGATACTGCCATGTACTATTGTGTGCGGACCCAGGCTATTAC GGGAATCCCTGGTTCGCCTATTGGGGACAGGGCACTCTGGTTACC GTATCATCAGCTAGCACCAAAGGACCTAGTGTTTTCCTCTTGCCC CTTCCTCAAAGTCTACCTCTGGGGGACAGCCGCTCTGGGCTGCC TGGTCAAGGATTATTTCCCAGAGCCTGTCACTGTCAGTTGGAACTC TGGAGCCTTGACTTCTGGTGTTCATACATTTCCTGCTGTCCTTCAA AGCAGCGGCTTGTACTCATTGTCTTCTGTTGTGACAGTACCCTCAA GCAGCCTCGGCACTCAGACATACATCTGCAATGTCAACCACAAAC CCTCAAATACAAAGGTAGATAAAAAAGTCGAACCAAAGTCTTGTG ACAAAACTCACACGTGCCCACCGTGCCCAGCACCTGAACTCCTGG GGGGACCGTCAGTCTTCCTCTTCCCCCCAAAACCCAAGGACACCC TCATGATCTCCCGGACCCCTGAGGTCACATGCGTGGTGGTGGACG | 508 |
| | TGAGCCACGAAGACCCTGAGGTCAAGTTCAACTGGTACGTGGACG GCGTGGAGGTGCATAATGCCAAGACAAAGCCGCGGGAGGAGCAG TACAACAGCACGTACCGTGTGGTCAGCGTCCTCACCGTCCTGCAC CAGGACTGGCTGAATGGCAAGGAGTACAAGTGCAAGGTCTCCAA CAAAGCCCTCCCAGCCCCCATCGAGAAAACCATCTCCAAAGCCAA AGGGCAGCCCCGAGAACCACAGGTGTACACCCTGCCCCCATGCCG GGAGGAGATGACCAAGAACCAGGTCAGCCTGTGGTGCCTGGTCA AAGGCTTCTATCCCAGCGACATCGCCGTGGAGTGGGAGAGCAATG GGCAGCCGGAGAACAACTACAAGACCACGCCTCCCGTGCTGGACT CCGACGGCTCCTTCTTCCTCTACAGCAAGCTCACCGTGGACAAGA GCAGGTGGCAGCAGGGGAACGTCTTCTCATGCTCCGTGATGCATG AGGCTCTGCACAACCACTACACGCAGAAGAGCCTCTCCCTGTCTC CGGGTAAA | |
| GPM04 LC | CAAATTGTCCTCACCCAATCACCAGCTATAATGTCTGCTTTTCCCG GCGAGAAAGTAACCATGACTTGTAGCGCCTCTAGTAGCGTGTCAT ATATGTATTGGTATCAACAAAAGAGCGGTAGTTCACCTCGACTCC TTATCTACGACACAAGTAATCTCGCTAGTGGTGTCCCAGTCCGTTT CTCCGGGAGCGGCAGCGGCACATCATACTCCCTGACCATCTCCAG AATGGAGGCCGAGGACGCTGCCACATACTATTGTCAGCAGTGGAG CTCATATCCTTTGACATTCGGTTGCGGTACTAAACTCGAAATCAAG CGTACGGTGGCAGCTCCCAGCGTTTTTATCTTTCCCCCATCCGACG AGCAGCTCAAGAGTGGCACTGCCTCTGTAGTTTGTTTGCTGAATA ACTTCTATCCACGTGAAGCAAAAGTACAGTGGAAGGTCGATAATG CCCTTCAGAGCGGTAACAGCCAAGAAAGTGTTACCGAGCAAGATT CCAAAGATTCCACTTACAGTCTGTCCAGCACATTGACACTGAGTA AGGCTGATTACGAAAAACACAAGGTGTACGCATGCGAGGTGACA CACCAAGGTCTTTCATCTCCTGTAACTAAGAGCTTTAACCGGGGA GAATGTGGTGGTGGGGGCAGCGGGGGCGGAGGTAGTGGAGGCGG CGGTAGTGAACCAAAGAGTAGTGACAAAACTCACACGTGCCCAC CGTGCCCAGCACCTGAACTCCTGGGGGGACCGTCAGTCTTCCTCTT CCCCCCAAAACCCAAGGACACCCTCATGATCTCCCGGACCCCTGA GGTCACATGCGTGGTGGTGGACGTGAGCCACGAAGACCCTGAGGT CAAGTTCAACTGGTACGTGGACGGCGTGGAGGTGCATAATGCCAA GACAAAGCCGCGGGAGGAGCAGTACAACAGCACGTACCGTGTGG TCAGCGTCCTCACCGTCCTGCACCAGGACTGGCTGAATGGCAAGG AGTACAAGTGCAAGGTCTCCAACAAAGCCCTCCCAGCCCCCATCG AGAAAACCATCTCCAAAGCCAAAGGGCAGCCCCGAGAACCACAG GTGTACACCCTGCCCCCATGCCGGGAGGAGATGACCAAGAACCA GGTCAGCCTGTGGTGCCTGGTCAAAGGCTTCTATCCCAGCGACAT CGCCGTGGAGTGGGAGAGCAATGGGCAGCCGGAGAACAACTACA AGACCACGCCTCCCGTGCTGGACTCCGACGGCTCCTTCTTCCTCTA CAGCAAGCTCACCGTGGACAAGAGCAGGTGGCAGCAGGGGAACG TCTTCTCATGCTCCGTGATGCATGAGGCTCTGCACAACCACTACAC GCAGAAGAGCCTCTCCCTGTCTCCGGGTAAA | 509 |
| GPB01 HC | CAGGTTCAGCTTGTGCAATCAGGCGCTGAAGTCAAAAAACCAGGA | 510 |
| | GCAAGCGTGAAAGTAAGCTGTAAGGCTTCCGGTTACACTTTCACC GATTACGAAATGCACTGGGTACGCCAGGCTCCTGGACAAGGCCTG GAATGGATGGGCGCACTTGATCCAAAAACAGGTGACACTGCTTAC AGTCAAAAATTCAAAGGTCGTGTCACTCTTACAGCAGATAAGTCC ACCAGTACCGCTTATATGGAGCTTAGCTCCCTGACTTCCGAGGAC ACCGCCGTGTATTATTGTACAAGATTCTACTCATATACTTACTGGG GCCAAGGAACCCTGGTGACAGTGTCATCTGCTAGCACCAAAGGAC CTAGTGTTTTTCCTCTTGCCCCTGAAGTTCAGCTTGTCGAAAGTGG CGGAGGTCTTGTAAAGCCAGGAGGGTCCCTGAAGTTGAGTTGTGC TGCCTCTGGCTTCACCTTTTCACGGTATGCCATGTCCTGGGTTCGA CAGATACCTGAGAAGTGTCTTGAATGGGTGGCCGCAATAGACAGT AGCGGTGGTGACACCTATTACCTCGACACAGTCAAAGACCGCTTT ACTATCTCACGCGATAACGCCAACAATACCCTGCACTTGCAGATG CGATCACTTCGTTCAGAAGACACTGCTCTTTACTATTGTGTACGCC AAGGGGGAGCATACTGGGGTCAGGGAACACTGGTTACCGTGTCTT CAGCTAGCACCAAAGGACCTAGTGTTTTTCCTCTTGCCCCTTCCTC AAAGTCTACCTCTGGGGGGACAGCCGCTCTGGGCTGCCTGGTCAA GGATTATTTCCCAGAGCCTGTCACTGTCAGTTGGAACTCTGGAGC CTTGACTTCTGGTGTTCATACATTTCCTGCTGTCCTTCAAAGCAGC GGCTTGTACTCATTGTCTTCTGTTGTGACAGTACCCTCAAGCAGCC TCGGCACTCAGACATACATCTGCAATGTCAACCACAAACCCTCAA ATACAAAGGTAGATAAAAAAGTCGAACCAAAGTCTTGTGACAAA ACTCACACGTGCCCACCGTGCCCAGCACCTGAACTCCTGGGGGGA CCGTCAGTCTTCCTCTTCCCCCCAAAACCCAAGGACACCCTCATGA TCTCCCGGACCCCTGAGGTCACATGCGTGGTGGTGGACGTGAGCC ACGAAGACCCTGAGGTCAAGTTCAACTGGTACGTGGACGGCGTGG AGGTGCATAATGCCAAGACAAAGCCGCGGGAGGAGCAGTACAAC AGCACGTACCGTGTGGTCAGCGTCCTCACCGTCCTGCACCAGGAC TGGCTGAATGGCAAGGAGTACAAGTGCAAGGTCTCCAACAAAGC CCTCCCAGCCCCCATCGAGAAAACCATCTCCAAAGCCAAAGGGCA GCCCCGAGAACCACAGGTGTACACCCTGCCCCCATGCCGGGAGGA GATGACCAAGAACCAGGTCAGCCTGTGGTGCCTGGTCAAAGGCTT CTATCCCAGCGACATCGCCGTGGAGTGGGAGAGCAATGGGCAGC CGGAGAACAACTACAAGACCACGCCTCCCGTGCTGGACTCCGACG GCTCCTTCTTCCTCTACAGCAAGCTCACCGTGGACAAGAGCAGGT GGCAGCAGGGGAACGTCTTCTCATGCTCCGTGATGCATGAGGCTC TGCACAACCACTACACGCAGAAGAGCCTCTCCCTGTCTCCGGGTA AA | |
| GPB01 LC | GATGTTGTTATGACTCAGTCACCTCTCTCACTTCCTGTAACCCCTG GTGAACCAGCCTCTATAAGCTGCCGGTCAAGTCAAAGCCTGGTTC ATAGCAACCGTAACACTTACCTTCACTGGTACTTGCAAAAACCTG GTCAGTCCCCACAACTCTTGATCTACAAAGTCTCCAATCGCTTCTC TGGAGTCCCTGACAGGTTTTCTGGTAGTGGATCAGGTACAGACTT CACTTTGAAAATCAGCCGCGTTGAGGCCGAGGACGTGGGAGTGTA TTATTGCTCTCAGAATACCCATGTACCCCCAACCTTTGGCCAAGGG | 511 |
| | ACTAAACTCGAGATTAAACGTACGGTGGCCGCTCCCTCCGTGTTC ATCTTCCCACCCGACGTGGTTATGACTCAGACTCCTCTGACTCTCT CCGTCACTATTGGTCAGCCCGCTTCCATATCATGTAAATCATCACA ATCTCTTCTTGATAGCGATGGCAAGACTTATTTGAACTGGTTGTTG CAACGCCCAGGTCAGAGCCCTAAGAGACTTATCTATTTGGTGAGC AAACTCGACAGCGGTGCACCCGATCGTTTTACCGGAAGCGGCAGC GGCACCGATTTCACACTGAAGATCAGTAGGGTCGAAGCTGAAGAC CTGGGAATCTACTACTGCTGGCAAGGTACTCACTTTCCCCTGACTT TCGGCTGCGGTACTAAACTTGAGATCAAACGTACGGTGGCAGCTC CCAGCGTTTTTATCTTTCCCCCATCCGACGAGCAGCTCAAGAGTGG CACTGCCTCTGTAGTTTGTTTGCTGAATAACTTCTATCCACGTGAA GCAAAAGTACAGTGGAAGGTCGATAATGCCCTTCAGAGCGGTAA CAGCCAAGAAAGTGTTACCGAGCAAGATTCCAAAGATTCCACTTA CAGTCTGTCCAGCACATTGACACTGAGTAAGGCTGATTACGAAAA ACACAAGGTGTACGCATGCGAGGTGACACACCAAGGTCTTTCATC TCCTGTAACTAAGAGCTTTAACCGGGGAGAATGTGGTGGTGGGGG CAGCGGGGGCGGAGGTAGTGGAGGCGGCGGTAGTGAACCAAAGA GTAGTGACAAAACTCACACGTGCCCACCGTGCCCAGCACCTGAAC TCCTGGGGGGACCGTCAGTCTTCCTCTTCCCCCCAAAACCCAAGG ACACCCTCATGATCTCCCGGACCCCTGAGGTCACATGCGTGGTGG TGGACGTGAGCCACGAAGACCCTGAGGTCAAGTTCAACTGGTACG TGGACGGCGTGGAGGTGCATAATGCCAAGACAAAGCCGCGGGAG GAGCAGTACAACAGCACGTACCGTGTGGTCAGCGTCCTCACCGTC CTGCACCAGGACTGGCTGAATGGCAAGGAGTACAAGTGCAAGGT CTCCAACAAAGCCCTCCCAGCCCCCATCGAGAAAACCATCTCCAA AGCCAAAGGGCAGCCCCGAGAACCACAGGTGTACACCCTGCCCC CATGCCGGGAGGAGATGACCAAGAACCAGGTCAGCCTGTGGTGC CTGGTCAAAGGCTTCTATCCCAGCGACATCGCCGTGGAGTGGGAG AGCAATGGGCAGCCGGAGAACAACTACAAGACCACGCCTCCCGT GCTGGACTCCGACGGCTCCTTCTTCCTCTACAGCAAGCTCACCGTG GACAAGAGCAGGTGGCAGCAGGGGAACGTCTTCTCATGCTCCGTG ATGCATGAGGCTCTGCACAACCACTACACGCAGAAGAGCCTCTCC CTGTCTCCGGGTAAA | |
| GPB03 HC | CAGGTTCAGCTTGTGCAATCAGGCGCTGAAGTCAAAAAACCAGGA GCAAGCGTGAAAGTAAGCTGTAAGGCTTCCGGTTACACTTTCACC GATTACGAAATGCACTGGGTACGCCAGGCTCCTGGACAAGGCCTG GAATGGATGGGCGCACTTGATCCAAAAACAGGTGACACTGCTTAC AGTCAAAAATTCAAAGGTCGTGTCACTCTTACAGCAGATAAGTCC ACCAGTACCGCTTATATGGAGCTTAGCTCCCTGACTTCCGAGGAC ACCGCCGTGTATTATTGTACAAGATTCTACTCATATACTTACTGGG GCCAAGGAACCCTGGTGACAGTGTCATCTGCTAGCACCAAAGGAC CTAGTGTTTTTCCTCTTGCCCCTGAAGTGCAACTGGTCGAAACAGG CGGGGGACTGGTACAGCCCAAGGGATCTTTGAAACTTAGTTGTGC TGCTAGTGGGTTTACATTCAATGCCTCCGCAATGAACTGGGTAAG ACAAGCTCCTGGCAAGTGCCTGGAATGGGTGGCCCGTATTCGCTC | 512 |
| | TAAAAGTAATAACTACGCTATTTATTACGCTGATTCTGTAAAGGAT CGGTTTACAATAAGTCGGGACGACAGCCAATCCATGCTGTATCTC CAAATGAATAACCTGAAAACAGAGGATACTGCCATGTACTATTGT GTGCGGGACCCAGGCTATTACGGGAATCCCTGGTTCGCCTATTGG GGACAGGGCACTCTGGTTACCGTATCATCAGCTAGCACCAAAGGA CCTAGTGTTTTTCCTCTTGCCCCTTCCTCAAAGTCTACCTCTGGGG GGACAGCCGCTCTGGGCTGCCTGGTCAAGGATTATTTCCCAGAGC CTGTCACTGTCAGTTGGAACTCTGGAGCCTTGACTTCTGGTGTTCA TACATTTCCTGCTGTCCTTCAAAGCAGCGGCTTGTACTCATTGTCT TCTGTTGTGACAGTACCCTCAAGCAGCCTCGGCACTCAGACATAC ATCTGCAATGTCAACCACAAACCCTCAAATACAAAGGTAGATAAA AAAGTCGAACCAAAGTCTTGTGACAAAACTCACACGTGCCCACCG TGCCCAGCACCTGAACTCCTGGGGGGACCGTCAGTCTTCCTCTTCC CCCCAAAACCCAAGGACACCCTCATGATCTCCCGGACCCCTGAGG TCACATGCGTGGTGGTGGACGTGAGCCACGAAGACCCTGAGGTCA AGTTCAACTGGTACGTGGACGGCGTGGAGGTGCATAATGCCAAGA CAAAGCCGCGGGAGGAGCAGTACAACAGCACGTACCGTGTGGTC AGCGTCCTCACCGTCCTGCACCAGGACTGGCTGAATGGCAAGGAG TACAAGTGCAAGGTCTCCAACAAAGCCCTCCCAGCCCCCATCGAG AAAACCATCTCCAAAGCCAAAGGGCAGCCCCGAGAACCACAGGT GTACACCCTGCCCCCATGCCGGGAGGAGATGACCAAGAACCAGG TCAGCCTGTGGTGCCTGGTCAAAGGCTTCTATCCCAGCGACATCG CCGTGGAGTGGGAGAGCAATGGGCAGCCGGAGAACAACTACAAG ACCACGCCTCCCGTGCTGGACTCCGACGGCTCCTTCTTCCTCTACA GCAAGCTCACCGTGGACAAGAGCAGGTGGCAGCAGGGGAACGTC TTCTCATGCTCCGTGATGCATGAGGCTCTGCACAACCACTACACG CAGAAGAGCCTCTCCCTGTCTCCGGGTAAA | |
| GPB03 LC | GATGTTGTTATGACTCAGTCACCTCTCTCACTTCCTGTAACCCCTG GTGAACCAGCCTCTATAAGCTGCCGGTCAAGTCAAAGCCTGGTTC ATAGCAACCGTAACACTTACCTTCACTGGTACTTGCAAAAACCTG GTCAGTCCCCACAACTCTTGATCTACAAAGTCTCCAATCGCTTCTC TGGAGTCCCTGACAGGTTTTCTGGTAGTGGATCAGGTACAGACTT CACTTTGAAAATCAGCCGCGTTGAGGCCGAGGACGTGGGAGTGTA TTATTGCTCTCAGAATACCCATGTACCCCCAACCTTTGGCCAAGGG ACTAAACTCGAGATTAAACGTACGGTGGCCGCTCCCTCCGTGTTC ATCTTCCCACCCCAAATTGTCCTCACCCAATCACCAGCTATAATGT CTGCTTTTCCCGGCGAGAAAGTAACCATGACTTGTAGCGCCTCTA GTAGCGTGTCATATATGTATTGGTATCAACAAAAGAGCGGTAGTT CACCTCGACTCCTTATCTACGACACAAGTAATCTCGCTAGTGGTGT CCCAGTCCGTTTCTCCGGGAGCGGCAGCGGCACATCATACTCCCT GACCATCTCCAGAATGGAGGCCGAGGACGCTGCCACATACTATTG TCAGCAGTGGAGCTCATATCCTTTGACATTCGGTTGCGGTACTAAA CTCGAAATCAAGCGTACGGTGGCAGCTCCCAGCGTTTTTATCTTTC CCCCATCCGACGAGCAGCTCAAGAGTGGCACTGCCTCTGTAGTTT GTTTGCTGAATAACTTCTATCCACGTGAAGCAAAAGTACAGTGGA | 513 |
| | AGGTCGATAATGCCCTTCAGAGCGGTAACAGCCAAGAAAGTGTTA CCGAGCAAGATTCCAAAGATTCCACTTACAGTCTGTCCAGCACAT TGACACTGAGTAAGGCTGATTACGAAAAACACAAGGTGTACGCAT GCGAGGTGACACACCAAGGTCTTTCATCTCCTGTAACTAAGAGCT TTAACCGGGGAGAATGTGGTGGTGGGGGCAGCGGGGGCGGAGGT AGTGGAGGCGGCGGTAGTGAACCAAAGAGTAGTGACAAAACTCA CACGTGCCCACCGTGCCCAGCACCTGAACTCCTGGGGGGACCGTC AGTCTTCCTCTTCCCCCCAAAACCCAAGGACACCCTCATGATCTCC CGGACCCCTGAGGTCACATGCGTGGTGGTGGACGTGAGCCACGAA GACCCTGAGGTCAAGTTCAACTGGTACGTGGACGGCGTGGAGGTG CATAATGCCAAGACAAAGCCGCGGGAGGAGCAGTACAACAGCAC GTACCGTGTGGTCAGCGTCCTCACCGTCCTGCACCAGGACTGGCT GAATGGCAAGGAGTACAAGTGCAAGGTCTCCAACAAAGCCCTCC CAGCCCCCATCGAGAAAACCATCTCCAAAGCCAAAGGGCAGCCC CGAGAACCACAGGTGTACACCCTGCCCCCATGCCGGGAGGAGAT GACCAAGAACCAGGTCAGCCTGTGGTGCCTGGTCAAAGGCTTCTA TCCCAGCGACATCGCCGTGGAGTGGGAGAGCAATGGGCAGCCGG AGAACAACTACAAGACCACGCCTCCCGTGCTGGACTCCGACGGCT CCTTCTTCCTCTACAGCAAGCTCACCGTGGACAAGAGCAGGTGGC AGCAGGGGAACGTCTTCTCATGCTCCGTGATGCATGAGGCTCTGC ACAACCACTACACGCAGAAGAGCCTCTCCCTGTCTCCGGGTAAA | |
| GPB04 HC | GAAGTTCAGCTTGTCGAAAGTGGCGGAGGTCTTGTAAAGCCAGGA GGGTCCCTGAAGTTGAGTTGTGCTGCCTCTGGCTTCACCTTTTCAC GGTATGCCATGTCCTGGGTTCGACAGATACCTGAGAAGATTCTTG AATGGGTGGCCGCAATAGACAGTAGCGGTGGTGACACCTATTACC TCGACACAGTCAAAGACCGCTTTACTATCTCACGCGATAACGCCA ACAATACCCTGCACTTGCAGATGCGATCACTTCGTTCAGAAGACA CTGCTCTTTACTATTGTGTACGCCAAGGGGGAGCATACTGGGGTC AGGGAACACTGGTTACCGTGTCTTCAGCTAGCACCAAAGGACCTA GTGTTTTTCCTCTTGCCCCTCAGGTTCAGCTTGTGCAATCAGGCGC TGAAGTCAAAAAACCAGGAGCAAGCGTGAAAGTAAGCTGTAAGG CTTCCGGTTACACTTTCACCGATTACGAAATGCACTGGGTACGCC AGGCTCCTGGACAATGTCTGGAATGGATGGGCGCACTTGATCCAA AAACAGGTGACACTGCTTACAGTCAAAAATTCAAAGGTCGTGTCA CTCTTACAGCAGATAAGTCCACCAGTACCGCTTATATGGAGCTTA GCTCCCTGACTTCCGAGGACACCGCCGTGTATTATTGTACAAGATT CTACTCATATACTTACTGGGGCCAAGGAACCCTGGTGACAGTGTC ATCTGCTAGCACCAAAGGACCTAGTGTTTTTCCTCTTGCCCCTTCC TCAAAGTCTACCTCTGGGGGGACAGCCGCTCTGGGCTGCCTGGTC AAGGATTATTTCCCAGAGCCTGTCACTGTCAGTTGGAACTCTGGA GCCTTGACTTCTGGTGTTCATACATTTCCTGCTGTCCTTCAAAGCA GCGGCTTGTACTCATTGTCTTCTGTTGTGACAGTACCCTCAAGCAG CCTCGGCACTCAGACATACATCTGCAATGTCAACCACAAACCCTC AAATACAAAGGTAGATAAAAAAGTCGAACCAAAGTCTTGTGACA AAACTCACACGTGCCCACCGTGCCCAGCACCTGAACTCCTGGGGG | 514 |
| | GACCGTCAGTCTTCCTCTTCCCCCCAAAACCCAAGGACACCCTCAT GATCTCCCGGACCCCTGAGGTCACATGCGTGGTGGTGGACGTGAG CCACGAAGACCCTGAGGTCAAGTTCAACTGGTACGTGGACGGCGT GGAGGTGCATAATGCCAAGACAAAGCCGCGGGAGGAGCAGTACA ACAGCACGTACCGTGTGGTCAGCGTCCTCACCGTCCTGCACCAGG ACTGGCTGAATGGCAAGGAGTACAAGTGCAAGGTCTCCAACAAA GCCCTCCCAGCCCCCATCGAGAAAACCATCTCCAAAGCCAAAGGG CAGCCCCGAGAACCACAGGTGTACACCCTGCCCCCATGCCGGGAG GAGATGACCAAGAACCAGGTCAGCCTGTGGTGCCTGGTCAAAGG CTTCTATCCCAGCGACATCGCCGTGGAGTGGGAGAGCAATGGGCA GCCGGAGAACAACTACAAGACCACGCCTCCCGTGCTGGACTCCGA CGGCTCCTTCTTCCTCTACAGCAAGCTCACCGTGGACAAGAGCAG GTGGCAGCAGGGGAACGTCTTCTCATGCTCCGTGATGCATGAGGC TCTGCACAACCACTACACGCAGAAGAGCCTCTCCCTGTCTCCGGG TAAA | |
| GPB04 LC | GACGTGGTTATGACTCAGACTCCTCTGACTCTCTCCGTCACTATTG GTCAGCCCGCTTCCATATCATGTAAATCATCACAATCTCTTCTTGA TAGCGATGGCAAGACTTATTTGAACTGGTTGTTGCAACGCCCAGG TCAGAGCCCTAAGAGACTTATCTATTTGGTGAGCAAACTCGACAG CGGTGCACCCGATCGTTTTACCGGAAGCGGCAGCGGCACCGATTT CACACTGAAGATCAGTAGGGTCGAAGCTGAAGACCTGGGAATCT ACTACTGCTGGCAAGGTACTCACTTTCCCCTGACTTTCGGCGCCGG TACTAAACTTGAGATCAAACGTACGGTGGCAGCTCCCAGCGTTTT TATCTTTCCCCCAGATGTTGTTATGACTCAGTCACCTCTCTCACTTC CTGTAACCCCTGGTGAACCAGCCTCTATAAGCTGCCGGTCAAGTC AAAGCCTGGTTCATAGCAACCGTAACACTTACCTTCACTGGTACTT GCAAAAACCTGGTCAGTCCCCACAACTCTTGATCTACAAAGTCTC CAATCGCTTCTCTGGAGTCCCTGACAGGTTTTCTGGTAGTGGATCA GGTACAGACTTCACTTTGAAAATCAGCCGCGTTGAGGCCGAGGAC GTGGGAGTGTATTATTGCTCTCAGAATACCCATGTACCCCCAACCT TTGGCTGTGGGACTAAACTCGAGATTAAACGTACGGTGGCAGCTC CCAGCGTTTTTATCTTTCCCCCATCCGACGAGCAGCTCAAGAGTGG CACTGCCTCTGTAGTTTGTTTGCTGAATAACTTCTATCCACGTGAA GCAAAAGTACAGTGGAAGGTCGATAATGCCCTTCAGAGCGGTAA CAGCCAAGAAAGTGTTACCGAGCAAGATTCCAAAGATTCCACTTA CAGTCTGTCCAGCACATTGACACTGAGTAAGGCTGATTACGAAAA ACACAAGGTGTACGCATGCGAGGTGACACACCAAGGTCTTTCATC TCCTGTAACTAAGAGCTTTAACCGGGGAGAATGTGGTGGTGGGGG CAGCGGGGGCGGAGGTAGTGGAGGCGGCGGTAGTGAACCAAAGA GTAGTGACAAAACTCACACGTGCCCACCGTGCCCAGCACCTGAAC TCCTGGGGGGACCGTCAGTCTTCCTCTTCCCCCCAAAACCCAAGG ACACCCTCATGATCTCCCGGACCCCTGAGGTCACATGCGTGGTGG TGGACGTGAGCCACGAAGACCCTGAGGTCAAGTTCAACTGGTACG TGGACGGCGTGGAGGTGCATAATGCCAAGACAAAGCCGCGGGAG GAGCAGTACAACAGCACGTACCGTGTGGTCAGCGTCCTCACCGTC | 515 |
| | CTGCACCAGGACTGGCTGAATGGCAAGGAGTACAAGTGCAAGGT CTCCAACAAAGCCCTCCCAGCCCCCATCGAGAAAACCATCTCCAA AGCCAAAGGGCAGCCCCGAGAACCACAGGTGTACACCCTGCCCC CATGCCGGGAGGAGATGACCAAGAACCAGGTCAGCCTGTGGTGC CTGGTCAAAGGCTTCTATCCCAGCGACATCGCCGTGGAGTGGGAG AGCAATGGGCAGCCGGAGAACAACTACAAGACCACGCCTCCCGT GCTGGACTCCGACGGCTCCTTCTTCCTCTACAGCAAGCTCACCGTG GACAAGAGCAGGTGGCAGCAGGGGAACGTCTTCTCATGCTCCGTG ATGCATGAGGCTCTGCACAACCACTACACGCAGAAGAGCCTCTCC CTGTCTCCGGGTAAA | |
| GPB06 HC | GAAGTGCAACTGGTCGAAACAGGCGGGGGACTGGTACAGCCCAA GGGATCTTTGAAACTTAGTTGTGCTGCTAGTGGGTTTACATTCAAT GCCTCCGCAATGAACTGGGTAAGACAAGCTCCTGGCAAGGGCCTG GAATGGGTGGCCCGTATTCGCTCTAAAAGTAATAACTACGCTATT TATTACGCTGATTCTGTAAAGGATCGGTTTACAATAAGTCGGGAC GACAGCCAATCCATGCTGTATCTCCAAATGAATAACCTGAAAACA GAGGATACTGCCATGTACTATTGTGTGCGGACCCAGGCTATTAC GGGAATCCCTGGTTCGCCTATTGGGGACAGGGCACTCTGGTTACC GTATCATCAGCTAGCACCAAAGGACCTAGTGTTTTCCTCTTGCCC CTCAGGTTCAGCTTGTGCAATCAGGCGCTGAAGTCAAAAAACCAG GAGCAAGCGTGAAAGTAAGCTGTAAGGCTTCCGGTTACACTTTCA CCGATTACGAAATGCACTGGGTACGCCAGGCTCCTGGACAATGTC TGGAATGGATGGGCGCACTTGATCCAAAAACAGGTGACACTGCTT ACAGTCAAAAATTCAAAGGTCGTGTCACTCTTACAGCAGATAAGT CCACCAGTACCGCTTATATGGAGCTTAGCTCCCTGACTTCCGAGG ACACCGCCGTGTATTATTGTACAAGATTCTACTCATATACTTACTG GGGCCAAGGAACCCTGGTGACAGTGTCATCTGCTAGCACCAAAGG ACCTAGTGTTTTCCTCTTGCCCCTTCCTCAAAGTCTACCTCTGGG GGGACAGCCGCTCTGGGCTGCCTGGTCAAGGATTATTTCCCAGAG CCTGTCACTGTCAGTTGGAACTCTGGAGCCTTGACTTCTGGTGTTC ATACATTTCCTGCTGTCCTTCAAAGCAGCGGCTTGTACTCATTGTC TTCTGTTGTGACAGTACCCTCAAGCAGCCTCGGCACTCAGACATA CATCTGCAATGTCAACCACAAACCCTCAAATACAAAGGTAGATAA AAAAGTCGAACCAAAGTCTTGTGACAAAACTCACACGTGCCCACC GTGCCCAGCACCTGAACTCCTGGGGGGACCGTCAGTCTTCCTCTTC CCCCCAAAACCCAAGGACACCCTCATGATCTCCCGGACCCCTGAG GTCACATGCGTGGTGGTGGACGTGAGCCACGAAGACCCTGAGGTC AAGTTCAACTGGTACGTGGACGGCGTGGAGGTGCATAATGCCAAG ACAAAGCCGCGGGAGGAGCAGTACAACAGCACGTACCGTGTGGT CAGCGTCCTCACCGTCCTGCACCAGGACTGGCTGAATGGCAAGGA GTACAAGTGCAAGGTCTCCAACAAAGCCCTCCCAGCCCCCATCGA GAAAACCATCTCCAAAGCCAAAGGGCAGCCCCGAGAACCACAGG TGTACACCCTGCCCCCATGCCGGGAGGAGATGACCAAGAACCAG GTCAGCCTGTGGTGCCTGGTCAAAGGCTTCTATCCCAGCGACATC GCCGTGGAGTGGGAGAGCAATGGGCAGCCGGAGAACAACTACAA | 516 |
| | GACCACGCCTCCCGTGCTGGACTCCGACGGCTCCTTCTTCCTCTAC AGCAAGCTCACCGTGGACAAGAGCAGGTGGCAGCAGGGGAACGT CTTCTCATGCTCCGTGATGCATGAGGCTCTGCACAACCACTACAC GCAGAAGAGCCTCTCCCTGTCTCCGGGTAAA | |
| GPB06 LC | CAAATTGTCCTCACCCAATCACCAGCTATAATGTCTGCTTTTCCCG GCGAGAAAGTAACCATGACTTGTAGCGCCTCTAGTAGCGTGTCAT ATATGTATTGGTATCAACAAAAGAGCGGTAGTTCACCTCGACTCC TTATCTACGACACAAGTAATCTCGCTAGTGGTGTCCCAGTCCGTTT CTCCGGGAGCGGCAGCGGCACATCATACTCCCTGACCATCTCCAG AATGGAGGCCGAGGACGCTGCCACATACTATTGTCAGCAGTGGAG CTCATATCCTTTGACATTCGGTGGAGGTACTAAACTCGAAATCAA GCGTACGGTGGCAGCTCCCAGCGTTTTTATCTTTCCCCCAGATGTT GTTATGACTCAGTCACCTCTCTCACTTCCTGTAACCCCTGGTGAAC CAGCCTCTATAAGCTGCCGGTCAAGTCAAAGCCTGGTTCATAGCA ACCGTAACACTTACCTTCACTGGTACTTGCAAAAACCTGGTCAGT CCCCACAACTCTTGATCTACAAAGTCTCCAATCGCTTCTCTGGAGT CCCTGACAGGTTTTCTGGTAGTGGATCAGGTACAGACTTCACTTTG AAAATCAGCCGCGTTGAGGCCGAGGACGTGGGAGTGTATTATTGC TCTCAGAATACCCATGTACCCCCAACCTTTGGCTGTGGGACTAAA CTCGAGATTAAACGTACGGTGGCAGCTCCCAGCGTTTTTATCTTTC CCCCATCCGACGAGCAGCTCAAGAGTGGCACTGCCTCTGTAGTTT GTTTGCTGAATAACTTCTATCCACGTGAAGCAAAAGTACAGTGGA AGGTCGATAATGCCCTTCAGAGCGGTAACAGCCAAGAAAGTGTTA CCGAGCAAGATTCCAAAGATTCCACTTACAGTCTGTCCAGCACAT TGACACTGAGTAAGGCTGATTACGAAAAACACAAGGTGTACGCAT GCGAGGTGACACACCAAGGTCTTTCATCTCCTGTAACTAAGAGCT TTAACCGGGGAGAATGTGGTGGTGGGGGCAGCGGGGGCGGAGGT AGTGGAGGCGGCGGTAGTGAACCAAAGAGTAGTGACAAAACTCA CACGTGCCCACCGTGCCCAGCACCTGAACTCCTGGGGGGACCGTC AGTCTTCCTCTTCCCCCCAAAACCCAAGGACACCCTCATGATCTCC CGGACCCCTGAGGTCACATGCGTGGTGGTGGACGTGAGCCACGAA GACCCTGAGGTCAAGTTCAACTGGTACGTGGACGGCGTGGAGGTG CATAATGCCAAGACAAAGCCGCGGGAGGAGCAGTACAACAGCAC GTACCGTGTGGTCAGCGTCCTCACCGTCCTGCACCAGGACTGGCT GAATGGCAAGGAGTACAAGTGCAAGGTCTCCAACAAAGCCCTCC CAGCCCCCATCGAGAAAACCATCTCCAAAGCCAAAGGGCAGCCC CGAGAACCACAGGTGTACACCCTGCCCCCATGCCGGGAGGAGAT GACCAAGAACCAGGTCAGCCTGTGGTGCCTGGTCAAAGGCTTCTA TCCCAGCGACATCGCCGTGGAGTGGGAGAGCAATGGGCAGCCGG AGAACAACTACAAGACCACGCCTCCCGTGCTGGACTCCGACGGCT CCTTCTTCCTCTACAGCAAGCTCACCGTGGACAAGAGCAGGTGGC AGCAGGGGAACGTCTTCTCATGCTCCGTGATGCATGAGGCTCTGC ACAACCACTACACGCAGAAGAGCCTCTCCCTGTCTCCGGGTAAA | 517 |

Table 46 below shows the polypeptide sequences of the heavy chain variable region (VH) and light chain variable region (VL) of the engineered antibodies targeting GPC-3. Table 47 below shows the nucleotide sequences of the heavy chain variable region (VH) and light chain variable region (VL) of the engineered antibodies targeting GPC-3.

**[Table 46]**

| Name | | Sequence | SEQ ID NO |
|---|---|---|---|
| GPM01 | VH | EVQLVESGGGLVKPGGSLKLSCAASGFTFSRYAMSWVRQIPEKCLEW VAAIDSSGGDTYYLDTVKDRFTISRDNANNTLHLQMRSLRSEDTALY YCVRQGGAYWGQGTLVTVSS | 81 |
| | VL | DVVMTQTPLTLSVTIGQPASISCKSSQSLLDSDGKTYLNWLLQRPGQS PKRLIYLVSKLDSGAPDRFTGSGSGTDFTLKISRVEAEDLGIYYCWQG THFPLTFGCGTKLEIK | 82 |
| GPM02 | VH | QVQLVQSGAEVKKPGASVKVSCKASGYTFTDYEMHWVRQAPGQCL EWMGALDPKTGDTAYSQKFKGRVTLTADKSTSTAYMELSSLTSEDT AVYYCTRFYSYTYWGQGTLVTVSS | 83 |
| | VL | DVVMTQSPLSLPVTPGEPASISCRSSQSLVHSNRNTYLHWYLQKPGQS PQLLIYKVSNRFSGVPDRFSGSGSGTDFTLKISRVEAEDVGVYYCSQN THVPPTFGCGTKLEIK | 84 |
| GPM04 | VH | EVQLVETGGGLVQPKGSLKLSCAASGFTFNASAMNWVRQAPGKCLE WVARIRSKSNNYAIYYADSVKDRFTISRDDSQSMLYLQMNNLKTEDT AMYYCVRDPGYYGNPWFAYWGQGTLVTVSS | 85 |
| | VL | QIVLTQSPAIMSAFPGEKVTMTCSASSSVSYMYWYQQKSGSSPRLLIY DTSNLASGVPVRFSGSGSGTSYSLTISRMEAEDAATYYCQQWSSYPLT FGCGTKLEIK | 86 |
| GPB01 | VH1 | QVQLVQSGAEVKKPGASVKVSCKASGYTFTDYEMHWVRQAPGQGL EWMGALDPKTGDTAYSQKFKGRVTLTADKSTSTAYMELSSLTSEDT AVYYCTRFYSYTYWGQGTLVTVSS | 87 |
| | VH2 | EVQLVESGGGLVKPGGSLKLSCAASGFTFSRYAMSWVRQIPEKCLEW VAAIDSSGGDTYYLDTVKDRFTISRDNANNTLHLQMRSLRSEDTALY YCVRQGGAYWGQGTLVTVSS | 81 |
| | VL1 | DVVMTQSPLSLPVTPGEPASISCRSSQSLVHSNRNTYLHWYLQKPGQS PQLLIYKVSNRFSGVPDRFSGSGSGTDFTLKISRVEAEDVGVYYCSQN THVPPTFGQGTKLEIK | 88 |
| | VL2 | DVVMTQTPLTLSVTIGQPASISCKSSQSLLDSDGKTYLNWLLQRPGQS PKRLIYLVSKLDSGAPDRFTGSGSGTDFTLKISRVEAEDLGIYYCWQG THFPLTFGCGTKLEIK | 82 |
| GPB03 | VH1 | QVQLVQSGAEVKKPGASVKVSCKASGYTFTDYEMHWVRQAPGQGL EWMGALDPKTGDTAYSQKFKGRVTLTADKSTSTAYMELSSLTSEDT AVYYCTRFYSYTYWGQGTLVTVSS | 87 |
| | VH2 | EVQLVETGGGLVQPKGSLKLSCAASGFTFNASAMNWVRQAPGKCLE WVARIRSKSNNYAIYYADSVKDRFTISRDDSQSMLYLQMNNLKTEDT AMYYCVRDPGYYGNPWFAYWGQGTLVTVSS | 85 |
| | VL1 | DVVMTQSPLSLPVTPGEPASISCRSSQSLVHSNRNTYLHWYLQKPGQS PQLLIYKVSNRFSGVPDRFSGSGSGTDFTLKISRVEAEDVGVYYCSQN THVPPTFGQGTKLEIK | 88 |
| | VL2 | QIVLTQSPAIMSAFPGEKVTMTCSASSSVSYMYWYQQKSGSSPRLLIY | 86 |
| | | DTSNLASGVPVRFSGSGSGTSYSLTISRMEAEDAATYYCQQWSSYPLT FGCGTKLEIK | |
| GPB04 | VH1 | EVQLVESGGGLVKPGGSLKLSCAASGFTFSRYAMSWVRQIPEKILEW VAAIDSSGGDTYYLDTVKDRFTISRDNANNTLHLQMRSLRSEDTALY YCVRQGGAYWGQGTLVTVSS | 89 |
| | VH2 | QVQLVQSGAEVKKPGASVKVSCKASGYTFTDYEMHWVRQAPGQCL EWMGALDPKTGDTAYSQKFKGRVTLTADKSTSTAYMELSSLTSEDT AVYYCTRFYSYTYWGQGTLVTVSS | 83 |
| | VL1 | DVVMTQTPLTLSVTIGQPASISCKSSQSLLDSDGKTYLNWLLQRPGQS PKRLIYLVSKLDSGAPDRFTGSGSGTDFTLKISRVEAEDLGIYYCWQG THFPLTFGAGTKLEIK | 90 |
| | VL2 | DVVMTQSPLSLPVTPGEPASISCRSSQSLVHSNRNTYLHWYLQKPGQS PQLLIYKVSNRFSGVPDRFSGSGSGTDFTLKISRVEAEDVGVYYCSQN THVPPTFGCGTKLEIK | 84 |
| GPB06 | VH1 | EVQLVETGGGLVQPKGSLKLSCAASGFTFNASAMNWVRQAPGKGLE WVARIRSKSNNYAIYYADSVKDRFTISRDDSQSMLYLQMNNLKTEDT AMYYCVRDPGYYGNPWFAYWGQGTLVTVSS | 91 |
| | VH2 | QVQLVQSGAEVKKPGASVKVSCKASGYTFTDYEMHWVRQAPGQCL EWMGALDPKTGDTAYSQKFKGRVTLTADKSTSTAYMELSSLTSEDT AVYYCTRFYSYTYWGQGTLVTVSS | 83 |
| | VL1 | QIVLTQSPAIMSAFPGEKVTMTCSASSSVSYMYWYQQKSGSSPRLLIY DTSNLASGVPVRFSGSGSGTSYSLTISRMEAEDAATYYCQQWSSYPLT FGGGTKLEIK | 92 |
| | VL2 | DVVMTQSPLSLPVTPGEPASISCRSSQSLVHSNRNTYLHWYLQKPGQS PQLLIYKVSNRFSGVPDRFSGSGSGTDFTLKISRVEAEDVGVYYCSQN THVPPTFGCGTKLEIK | 84 |

**[Table 47]**

| **Name** | | **Sequence** | **SEQ ID NO** |
|---|---|---|---|
| GPM01 | VH | GAAGTTCAGCTTGTCGAAAGTGGCGGAGGTCTTGTAAAGCCAGGAGGGTCCCTGAAGTTGA GTTGTGCTGCCTCTGGCTTCACCTTTTCACGGTATGCCATGTCCTGGGTTCGACAGATACC TGAGAAGTGTCTTGAATGGGTGGCCGCAATAGACAGTAGCGGTGGTGACACCTATTACCTC GACACAGTCAAAGACCGCTTTACTATCTCACGCGATAACGCCAACAATACCCTGCACTTGC AGATGCGATCACTTCGTTCAGAAGACACTGCTCTTTACTATTGTGTACGCCAAGGGGGAGC ATACTGGGGTCAGGGAACACTGGTTACCGTGTCTTCA | 518 |
| | VL | GACGTGGTTATGACTCAGACTCCTCTGACTCTCTCCGTCACTATTGGTCAGCCCGCTTCCA TATCATGTAAATCATCACAATCTCTTCTTGATAGCGATGGCAAGACTTATTTGAACTGGTT GTTGCAACGCCCAGGTCAGAGCCCTAAGAGACTTATCTATTTGGTGAGCAAACTCGACAGC GGTGCACCCGATCGTTTTACCGGAAGCGGCAGCGGCACCGATTTCACACTGAAGATCAGTA GGGTCGAAGCTGAAGACCTGGGAATCTACTACTGCTGGCAAGGTACTCACTTTCCCCTGAC TTTCGGCTGCGGTACTAAACTTGAGATCAAA | 519 |
| GPM02 | VH | CAGGTTCAGCTTGTGCAATCAGGCGCTGAAGTCAAAAAACCAGGAGCAAGCGTGAAAGTAA GCTGTAAGGCTTCCGGTTACACTTTCACCGATTACGAAATGCACTGGGTACGCCAGGCTCC TGGACAATGTCTGGAATGGATGGGCGCACTTGATCCAAAAACAGGTGACACTGCTTACAGT CAAAAATTCAAAGGTCGTGTCACTCTTACAGCAGATAAGTCCACCAGTACCGCTTATATGG AGCTTAGCTCCCTGACTTCCGAGGACACCGCCGTGTATTATTGTACAAGATTCTACTCATA TACTTACTGGGGCCAAGGAACCCTGGTGACAGTGTCATCT | 520 |
| | VL | GATGTTGTTATGACTCAGTCACCTCTCTCACTTCCTGTAACCCCTGGTGAACCAGCCTCTA TAAGCTGCCGGTCAAGTCAAAGCCTGGTTCATAGCAACCGTAACACTTACCTTCACTGGTA CTTGCAAAAACCTGGTCAGTCCCCACAACTCTTGATCTACAAAGTCTCCAATCGCTTCTCT GGAGTCCCTGACAGGTTTTCTGGTAGTGGATCAGGTACAGACTTCACTTTGAAAATCAGCC GCGTTGAGGCCGAGGACGTGGGAGTGTATTATTGCTCTCAGAATACCCATGTACCCCCAAC CTTTGGCTGTGGGACTAAACTCGAGATTAAA | 521 |
| GPM04 | VH | GAAGTGCAACTGGTCGAAACAGGCGGGGGACTGGTACAGCCCAAGGGATCTTTGAAACTTA GTTGTGCTGCTAGTGGGTTTACATTCAATGCCTCCGCAATGAACTGGGTAAGACAAGCTCC TGGCAAGTGCCTGGAATGGGTGGCCCGTATTCGCTCTAAAAGTAATAACTACGCTATTTAT TACGCTGATTCTGTAAAGGATCGGTTTACAATAAGTCGGGACGACAGCCAATCCATGCTGT ATCTCCAAATGAATAACCTGAAAACAGAGGATACTGCCATGTACTATTGTGTGCGGGACCC AGGCTATTACGGGAATCCCTGGTTCGCCTATTGGGGACAGGGCACTCTGGTTACCGTATCA TCA | 522 |
| | VL | CAAATTGTCCTCACCCAATCACCAGCTATAATGTCTGCTTTTCCCGGCGAGAAAGTAACCA TGACTTGTAGCGCCTCTAGTAGCGTGTCATATATGTATTGGTATCAACAAAAGAGCGGTAG TTCACCTCGACTCCTTATCTACGACACAAGTAATCTCGCTAGTGGTGTCCCAGTCCGTTTC TCCGGGAGCGGCAGCGGCACATCATACTCCCTGACCATCTCCAGAATGGAGGCCGAGGACG CTGCCACATACTATTGTCAGCAGTGGAGCTCATATCCTTTGACATTCGGTTGCGGTACTAA ACTCGAAATCAAG | 523 |
| GPB01 | VH1 | CAGGTGCAACTCGTTCAAAGCGGGGCCGAGGTGAAGAAACCAGGGGCCTCAGTTAAGGTGA | 434 |
| | | GTTGCAAGGCAAGTGGATACACTTTCACCGATTATGAAATGCATTGGGTGCGTCAGGCCCC AGGACAAGGACTGGAGTGGATGGGCGCTCTCGATCCTAAGACTGGTGATACTGCTTACTCT CAAAAGTTCAAAGGCCGAGTCACCTTGACCGCCGACAAGTCCACATCCACTGCATATATGG AATTGTCAAGTCTGACAAGCGAAGATACAGCCGTCTACTACTGCACCCGCTTTTATAGCTA TACATATTGGGGACAGGGGACCTTGGTTACTGTGTCATCT | |
| | VH2 | GAAGTTCAGCTTGTCGAAAGTGGCGGAGGTCTTGTAAAGCCAGGAGGGTCCCTGAAGTTGA GTTGTGCTGCCTCTGGCTTCACCTTTTCACGGTATGCCATGTCCTGGGTTCGACAGATACC TGAGAAGTGTCTTGAATGGGTGGCCGCAATAGACAGTAGCGGTGGTGACACCTATTACCTC GACACAGTCAAAGACCGCTTTACTATCTCACGCGATAACGCCAACAATACCCTGCACTTGC AGATGCGATCACTTCGTTCAGAAGACACTGCTCTTTACTATTGTGTACGCCAAGGGGGAGC ATACTGGGGTCAGGGAACACTGGTTACCGTGTCTTCA | 518 |
| | VL1 | GACGTGGTAATGACACAATCACCTTTGTCTCTTCCCGTAACCCCCGGTGAACCAGCCAGCA TCTCATGCAGAAGCAGTCAGTCACTGGTACATTCCAACCGTAATACTTATCTTCACTGGTA CTTGCAGAAGCCTGGGCAGTCTCCTCAACTTTTGATATATAAAGTGAGCAATCGGTTTAGC GGTGTCCCAGACCGCTTTTCTGGATCTGGAAGTGGAACAGACTTTACTCTGAAAATAAGCA GAGTCGAGGCAGAAGATGTCGGAGTTTACTACTGTAGCCAGAACACACACGTACCCCCAAC CTTTGGACAGGGCACAAAGTTGGAAATCAAG | 435 |
| | VL2 | GACGTGGTTATGACTCAGACTCCTCTGACTCTCTCGGTCACTATTGGTCAGCCCGCTTCCA TATCATGTAAATCATCACAATCTCTTCTTGATAGCGATGGCAAGACTTATTTGAACTGGTT GTTGCAACGCCCAGGTCAGAGCCCTAAGAGACTTATCTATTTGGTGAGCAAACTCGACAGC GGTGCACCCGATCGTTTTACCGGAAGCGGCAGCGGCACCGATTTCACACTGAAGATCAGTA GGGTCGAAGCTGAAGACCTGGGAATCTACTACTGCTGGCAAGGTACTCACTTTCCCCTGAC TTTCGGCTGCGGTACTAAACTTGAGATCAAA | 519 |
| GPB03 | VH1 | CAGGTGCAACTCGTTCAAAGCGGGGCCGAGGTGAAGAAACCAGGGGCCTCAGTTAAGGTGA GTTGCAAGGCAAGTGGATACACTTTCACCGATTATGAAATGCATTGGGTGCGTCAGGCCCC AGGACAAGGACTGGAGTGGATGGGCGCTCTCGATCCTAAGACTGGTGATACTGCTTACTCT CAAAAGTTCAAAGGCCGAGTCACCTTGACCGCCGACAAGTCCACATCCACTGCATATATGG AATTGTCAAGTCTGACAAGCGAAGATACAGCCGTCTACTACTGCACCCGCTTTTATAGCTA TACATATTGGGGACAGGGGACCTTGGTTACTGTGTCATCT | 434 |
| | VH2 | GAAGTGCAACTGGTCGAAACAGGCGGGGGACTGGTACAGCCCAAGGGATCTTTGAAACTTA GTTGTGCTGCTAGTGGGTTTACATTCAATGCCTCCGCAATGAACTGGGTAAGACAAGCTCC TGGCAAGTGCCTGGAATGGGTGGCCCGTATTCGCTCTAAAAGTAATAACTACGCTATTTAT TACGCTGATTCTGTAAAGGATCGGTTTACAATAAGTCGGGACGACAGCCAATCCATGCTGT ATCTCCAAATGAATAACCTGAAAACAGAGGATACTGCCATGTACTATTGTGTGCGGGACCC AGGCTATTACGGGAATCCCTGGTTCGCCTATTGGGGACAGGGCACTCTGGTTACCGTATCA TCA | 522 |
| | VL1 | GACGTGGTAATGACACAATCACCTTTGTCTCTTCCCGTAACCCCCGGTGAACCAGCCAGCA TCTCATGCAGAAGCAGTCAGTCACTGGTACATTCCAACCGTAATACTTATCTTCACTGGTA CTTGCAGAAGCCTGGGCAGTCTCCTCAACTTTTGATATATAAAGTGAGCAATCGGTTTAGC GGTGTCCCAGACCGCTTTTCTGGATCTGGAAGTGGAACAGACTTTACTCTGAAAATAAGCA | 435 |
| | | GAGTCGAGGCAGAAGATGTCGGAGTTTACTACTGTAGCCAGAACACACACGTACCCCCAAC CTTTGGACAGGGCACAAAGTTGGAAATCAAG | |
| | VL2 | CAAATTGTCCTCACCCAATCACCAGCTATAATGTCTGCTTTTCCCGGCGAGAAAGTAACCA TGACTTGTAGCGCCTCTAGTAGCGTGTCATATATGTATTGGTATCAACAAAAGAGCGGTAG TTCACCTCGACTCCTTATCTACGACACAAGTAATCTCGCTAGTGGTGTCCCAGTCCGTTTC TCCGGGAGCGGCAGCGGCACATCATACTCCCTGACCATCTCCAGAATGGAGGCCGAGGACG CTGCCACATACTATTGTCAGCAGTGGAGCTCATATCCTTTGACATTCGGTTGCGGTACTAA ACTCGAAATCAAG | 523 |
| GPB04 | VH1 | GAAGTTCAGCTTGTCGAAAGTGGCGGAGGTCTTGTAAAGCCAGGAGGGTCCCTGAAGTTGA GTTGTGCTGCCTCTGGCTTCACCTTTTCACGGTATGCCATGTCCTGGGTTCGACAGATACC TGAGAAGATTCTTGAATGGGTGGCCGCAATAGACAGTAGCGGTGGTGACACCTATTACCTC GACACAGTCAAAGACCGCTTTACTATCTCACGCGATAACGCCAACAATACCCTGCACTTGC AGATGCGATCACTTCGTTCAGAAGACACTGCTCTTTACTATTGTGTACGCCAAGGGGGAGC ATACTGGGGTCAGGGAACACTGGTTACCGTGTCTTCA | 524 |
| | VH2 | CAGGTTCAGCTTGTGCAATCAGGCGCTGAAGTCAAAAAACCAGGAGCAAGCGTGAAAGTAA GCTGTAAGGCTTCCGGTTACACTTTCACCGATTACGAAATGCACTGGGTACGCCAGGCTCC TGGACAATGTCTGGAATGGATGGGCGCACTTGATCCAAAAACAGGTGACACTGCTTACAGT CAAAAATTCAAAGGTCGTGTCACTCTTACAGCAGATAAGTCCACCAGTACCGCTTATATGG AGCTTAGCTCCCTGACTTCCGAGGACACCGCCGTGTATTATTGTACAAGATTCTACTCATA TACTTACTGGGGCCAAGGAACCCTGGTGACAGTGTCATCT | 520 |
| | VL1 | GACGTGGTTATGACTCAGACTCCTCTGACTCTCTCCGTCACTATTGGTCAGCCCGCTTCCA TATCATGTAAATCATCACAATCTCTTCTTGATAGCGATGGCAAGACTTATTTGAACTGGTT GTTGCAACGCCCAGGTCAGAGCCCTAAGAGACTTATCTATTTGGTGAGCAAACTCGACAGC GGTGCACCCGATCGTTTTACCGGAAGCGGCAGCGGCACCGATTTCACACTGAAGATCAGTA GGGTCGAAGCTGAAGACCTGGGAATCTACTACTGCTGGCAAGGTACTCACTTTCCCCTGAC TTTCGGCGCCGGTACTAAACTTGAGATCAAA | 525 |
| | VL2 | GATGTTGTTATGACTCAGTCACCTCTCTCACTTCCTGTAACCCCTGGTGAACCAGCCTCTA TAAGCTGCCGGTCAAGTCAAAGCCTGGTTCATAGCAACCGTAACACTTACCTTCACTGGTA CTTGCAAAAACCTGGTCAGTCCCCACAACTCTTGATCTACAAAGTCTCCAATCGCTTCTCT GGAGTCCCTGACAGGTTTTCTGGTAGTGGATCAGGTACAGACTTCACTTTGAAAATCAGCC GCGTTGAGGCCGAGGACGTGGGAGTGTATTATTGCTCTCAGAATACCCATGTACCCCCAAC CTTTGGCTGTGGGACTAAACTCGAGATTAAA | 521 |
| GPB06 | VH1 | GAAGTGCAACTGGTCGAAACAGGCGGGGGACTGGTACAGCCCAAGGGATCTTTGAAACTTA GTTGTGCTGCTAGTGGGTTTACATTCAATGCCTCCGCAATGAACTGGGTAAGACAAGCTCC TGGCAAGGGCCTGGAATGGGTGGCCCGTATTCGCTCTAAAAGTAATAACTACGCTATTTAT TACGCTGATTCTGTAAAGGATCGGTTTACAATAAGTCGGGACGACAGCCAATCCATGCTGT ATCTCCAAATGAATAACCTGAAAACAGAGGATACTGCCATGTACTATTGTGTGCGGGACCC AGGCTATTACGGGAATCCCTGGTTCGCCTATTGGGGACAGGGCACTCTGGTTACCGTATCA TCA | 526 |
| | VH2 | CAGGTTCAGCTTGTGCAATCAGGCGCTGAAGTCAAAAAACCAGGAGCAAGCGTGAAAGTAA | 520 |
| | | GCTGTAAGGCTTCCGGTTACACTTTCACCGATTACGAAATGCACTGGGTACGCCAGGCTCC TGGACAATGTCTGGAATGGATGGGCGCACTTGATCCAAAAACAGGTGACACTGCTTACAGT CAAAAATTCAAAGGTCGTGTCACTCTTACAGCAGATAAGTCCACCAGTACCGCTTATATGG AGCTTAGCTCCCTGACTTCCGAGGACACCGCCGTGTATTATTGTACAAGATTCTACTCATA TACTTACTGGGGCCAAGGAACCCTGGTGACAGTGTCATCT | |
| | VL1 | CAAATTGTCCTCACCCAATCACCAGCTATAATGTCTGCTTTTCCCGGCGAGAAAGTAACCA TGACTTGTAGCGCCTCTAGTAGCGTGTCATATATGTATTGGTATCAACAAAAGAGCGGTAG TTCACCTCGACTCCTTATCTACGACACAAGTAATCTCGCTAGTGGTGTCCCAGTCCGTTTC TCCGGGAGCGGCAGCGGCACATCATACTCCCTGACCATCTCCAGAATGGAGGCCGAGGACG CTGCCACATACTATTGTCAGCAGTGGAGCTCATATCCTTTGACATTCGGTGGAGGTACTAA ACTCGAAATCAAG | 527 |
| | VL2 | GATGTTGTTATGACTCAGTCACCTCTCTCACTTCCTGTAACCCCTGGTGAACCAGCCTCTA TAAGCTGCCGGTCAAGTCAAAGCCTGGTTCATAGCAACCGTAACACTTACCTTCACTGGTA CTTGCAAAAACCTGGTCAGTCCCCACAACTCTTGATCTACAAAGTCTCCAATCGCTTCTCT GGAGTCCCTGACAGGTTTTCTGGTAGTGGATCAGGTACAGACTTCACTTTGAAAATCAGCC GCGTTGAGGCCGAGGACGTGGGAGTGTATTATTGCTCTCAGAATACCCATGTACCCCCAAC CTTTGGCTGTGGGACTAAACTCGAGATTAAA | 521 |

Table 48 below shows the heavy chain and light chain CDR sequences of the engineered antibodies targeting GPC-3.

**[Table 48]**

| **Name** | CDR | **Sequence** | **SEQ ID NO** |
|---|---|---|---|
| GTM01 | CDR-H1 | RYAMS | 93 |
| | CDR-H2 | AIDSSGGDTYYLDTVKD | 94 |
| | CDR-H3 | QGGAY | 96 |
| | CDR-L1 | KSSQSLLDSDGKTYLN | 96 |
| | CDR-L2 | LVSKLDS | 97 |
| | CDR-L3 | WQGTHFPLT | 98 |
| GPM02 | CDR-H1 | DYEMH | 99 |
| | CDR-H2 | ALDPKTGDTAYSQKFKG | 100 |
| | CDR-H3 | FYSYTY | 101 |
| | CDR-L1 | RSSQSLVHSNRNTYLH | 102 |
| | CDR-L2 | KVSNRFS | 103 |
| | CDR-L3 | SQNTHVPPT | 104 |
| GPM04 | CDR-H1 | ASAMN | 106 |
| | CDR-H2 | RIRSKSNNYAIYYADSVKD | 106 |
| | CDR-H3 | DPGYYGNPWFAY | 107 |
| | CDR-L1 | SASSSVSYMY | 108 |
| | CDR-L2 | DTSNLAS | 109 |
| | CDR-L3 | QQWSSYPLT | 110 |
| GPB01 | V1 CDR-H1 | DYEMH | 99 |
| | V1 CDR-H2 | ALDPKTGDTAYSQKFKG | 100 |
| | V1 CDR-H3 | FYSYTY | 101 |
| | V1 CDR-L1 | RSSQSLVHSNRNTYLH | 102 |
| | V1 CDR-L2 | KVSNRFS | 103 |
| | V1 CDR-L3 | SQNTHVPPT | 104 |
| | V2 CDR-H1 | RYAMS | 93 |
| | V2 CDR-H2 | AIDSSGGDTYYLDTVKD | 94 |
| | V2 CDR-H3 | QGGAY | 95 |
| | V2 CDR-L1 | KSSQSLLDSDGKTYLN | 96 |
| | V2 CDR-L2 | LVSKLDS | 97 |
| | V2 CDR-L3 | WQGTHFPLT | 98 |
| GPB03 | V1 CDR-H1 | DYEMH | 99 |
| | V1 CDR-H2 | ALDPKTGDTAYSQKFKG | 100 |
| | V1 CDR-H3 | FYSYTY | 101 |
| | V1 CDR-L1 | RSSQSLVHSNRNTYLH | 102 |
| | V1 CDR-L2 | KVSNRFS | 103 |
| | V1 CDR-L3 | SQNTHVPPT | 104 |
| | V2 CDR-H1 | ASAMN | 106 |
| | V2 CDR-H2 | RIRSKSNNYAIYYADSVKD | 106 |
| | V2 CDR-H3 | DPGYYGNPWFAY | 107 |
| | V2 CDR-L1 | SASSSVSYMY | 108 |
| | V2 CDR-L2 | DTSNLAS | 109 |
| | V2 CDR-L3 | QQWSSYPLT | 110 |
| GPB04 | V1 CDR-H1 | RYAMS | 93 |
| | V1 CDR-H2 | AIDSSGGDTYYLDTVKD | 94 |
| | V1 CDR-H3 | QGGAY | 95 |
| | V1 CDR-L1 | KSSQSLLDSDGKTYLN | 96 |
| | V1 CDR-L2 | LVSKLDS | 97 |
| | V1 CDR-L3 | WQGTHFPLT | 98 |
| | V2 CDR-H1 | DYEMH | 99 |
| | V2 CDR-H2 | ALDPKTGDTAYSQKFKG | 100 |
| | V2 CDR-H3 | FYSYTY | 101 |
| | V2 CDR-L1 | RSSQSLVHSNRNTYLH | 102 |
| | V2 CDR-L2 | KVSNRFS | 103 |
| | V2 CDR-L3 | SQNTHVPPT | 104 |
| GPB06 | V1 CDR-H1 | ASAMN | 105 |
| | V1 CDR-H2 | RIRSKSNNYAIYYADSVKD | 106 |
| | V1 CDR-H3 | DPGYYGNPWFAY | 107 |
| | V1 CDR-L1 | SASSSVSYMY | 108 |
| | V1 CDR-L2 | DTSNLAS | 109 |
| | V1 CDR-L3 | QQWSSYPLT | 110 |
| | V2 CDR-H1 | DYEMH | 99 |
| | V2 CDR-H2 | ALDPKTGDTAYSQKFKG | 100 |
| | V2 CDR-H3 | FYSYTY | 101 |
| | V2 CDR-L1 | RSSQSLVHSNRNTYLH | 102 |
| | V2 CDR-L2 | KVSNRFS | 103 |
| | V2 CDR-L3 | SQNTHVPPT | 104 |

The GPC-3 protein binding constants of GPM01, GPM02, GPM04, GPB01, GPB03, GPB04, and GPB06 were determined using the Octet Red96e (Sartorius). In order to analyze the binding constants of the seven antibodies, the human GPC-3 recombinant protein (Sino Biologicals, 10088-H08H) was loaded onto the Anti-Penta-HIS (HIS1K) biosensor (Sartorius, 18-5120). Then the seven antibodies were added in a binding reaction (300 seconds) and a dissociation reaction (1,200 seconds) at various concentrations, and their affinities for GPC-3 were calculated (Figure 42, Table 49). Table 49 below illustrates the binding constants of the engineered antibodies targeting GPC-3.

**[Table 49]**

| Antibodies | Antigen | Binding mode | K_{D} (nM) | Kₐ (1/Ms) | K_{d} (1/s) |
|---|---|---|---|---|---|
| GPM01 | rhGPC3 | Monovalent | 2.7048 | 3.72E+05 | 1.01E-03 |
| GPM02 | rhGPC3 | Monovalent | 2.3799 | 1.92E+05 | 4.57E-04 |
| GPM04 | rhGPC3 | Monovalent | 38.3721 | 2.92E+04 | 1.12E-03 |
| GPB01 | rhGPC3 | Biparatopic | 0.5827 | 2.49E+05 | 1.45E-04 |
| GPB03 | rhGPC3 | Biparatopic | 0.1601 | 2.03E+05 | 3.25E-05 |
| GPB04 | rhGPC3 | Biparatopic | 0.2510 | 3.32E+05 | 8.33E-05 |
| GPB06 | rhGPC3 | Biparatopic | 0.3427 | 6.64E+04 | 2.28E-05 |

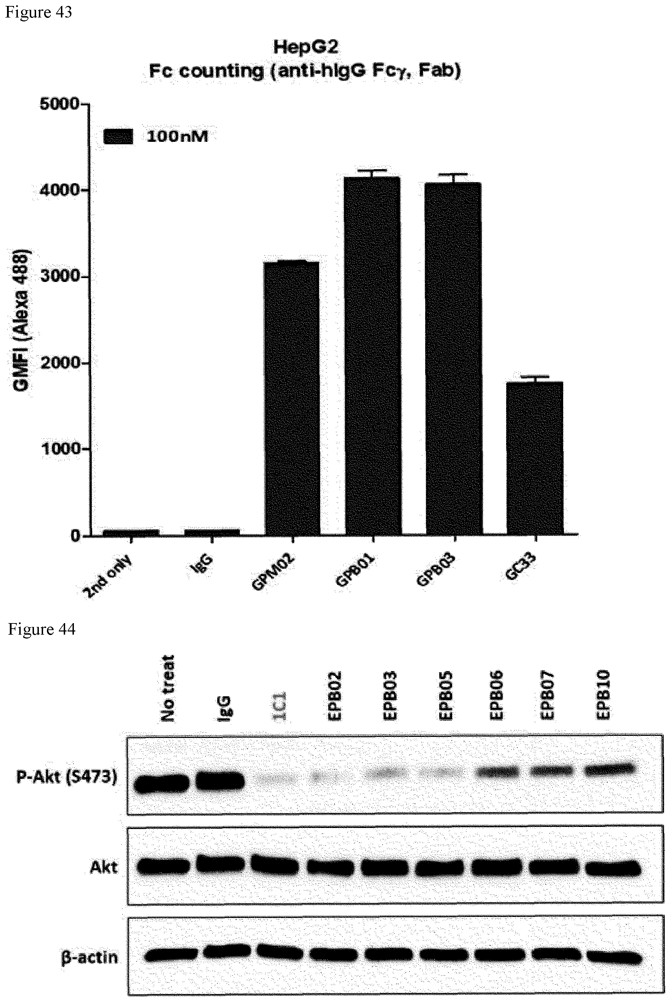

HepG2 liver cancer cell line was used to quantify the Fc loads on the surface of GPC-3 expressing cells. 100 nM human IgG1, GPM02, GPB01, GPB03, and GC33 were allowed to bind to the HepG2 cell line at 4°C for 30 minutes, and the Fc loads were quantified using the Alexa 488 fluorescence-conjugated anti-human IgG Fcγ Fab antibody (Jackson ImmunoResearch, 109-547-008) (Figure 43). GC33, which is a humanized antibody targeting GPC-3, was used as a positive control (Nakano et al., US7,919,086 B2). It was shown that higher Fc loads on the surface of cancer cells are induced by treatment of GPM02, GPB01, and GPB03 compared to GC33 (Figure 43).

### Example 18. Design, preparation and analysis of antibody structure targeting EPH receptor A2 (EphA2)

The variant light chain and heavy chain polypeptide sequences of the antibodies that specifically bind to the EPH receptor A2 (EphA2) protein are shown in Table 46. For EPB01, expression vectors consisting of the sequences corresponding to Fc-Hole (SEQ ID NO: 7), EPB01 HC (SEQ ID NO: 111), and EPB01 LC (SEQ ID NO: 112) were co-transfected into EXPICHO-S^{™} (Gibco, A29127) (Figure 41b, Table 50), and purification and analysis were performed in the same manner as described in Example 1. Expression, purification and analysis were performed for EPB02, EPB03, EPB04, EPB05, EPB06, EPB07, EPB08, EPB09, EPB10, EPB11, and EPB12 in the same manner as mentioned above (Table 50). The 12 antibodies have structures in which the variable regions that bind to two different epitopes of EphA2 are linked with a polypeptide linker (SEQ ID NO: 48, SEQ ID NO: 50), bind biparatopically to EphA2, and have two Fc domains (Figure 41b, Table 50).

**[Table 50]**

| **Name** | **Sequence** | **SEQ ID NO** |
|---|---|---|
| EPB01 HC | EVQLLESGGGLVQPGGSLRLSCAASGFTFSRYQMMWVRQAPGKGLEWVSSISPSGGVTL YADSVKGRFTISRDNSKNTLYLQMNSLRAEDTAVYYCTRELLGTVVVPVAWKMRGYFDY WGQLTLVTVSSASTKGPSVFPLAPEVQLLESGGGLVQPGGSLRLSCAASGFTFSHYMMA WVRQAPGKCLEWVSRIGPSGGPTHYADSVKGRFTISRDNSKNTLYLQMNSLRAEDTAVY YCAGYDSGYDYVAVAGPAEYFQHWGQGTLVTVSSASTKGPSVFPLAPSSKSTSGGTAAL GCLVKDYFPEPVTVSWNSGALTSGVHTFPAVLQSSGLYSLSSVVTVPSSSLGTQTYICN VNHKPSNTKVDKKVEPKSCDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNGKEY KCKVSNKALPAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEALHNHYT QKSLSLSPGK | 111 |
| EPB01 LC | DIQMTQSPGTLSVSPGERATLSCRASQSVSSNLAWYQQKPGQAPRLLIYGASTRATGIP ARFSGSGSGTEFTLTISSMQSEDFAVYYCQQYNNWPPLTFGGGTKVEIKRTVAAPSVFI FPPDIQMTQSPSSLSASVGDRVTITCRASQSISTWLAWYQQKPGKAPKLLIYKASNLHT GVPSRFSGSGSGTEFSLTISGLQPDDFATYYCQQYNSYSRTFGCGTKVEIKRTVAAPSV FIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGECGGGGSGGGGSGGGGSEPKS SDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTIS KAKGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 112 |
| EPB02 HC | EVQLVESGGGVVRPGGSLRLSCAASGFTVSDYSMNWVRQAPGKGLEWIGFIRNKANAYT TEYSASVKGRFTISRDDSKNTLYLQMNSLKTEDTAVYYCTTYPRYHAMDSWGQGTMVTV SSASTKGPSVFPLAPEVQLLESGGGLVQPGGSLRLSCAASGFTFSHYMMAWVRQAPGKC LEWVSRIGPSGGPTHYADSVKGRFTISRDNSKNTLYLQMNSLRAEDTAVYYCAGYDSGY DYVAVAGPAEYFQHWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNGQP ENNYKTTPPVLDSDGSFFLYSKLTVDKSRWQQGNVFSCSVMHEALHNHYTQKSLSLSPG K | 113 |
| EPB02 LC | AIQLTQSPSSLSASVGDRVTITCRASQSISNNLHWYLQKPGQSPQLLIYYGFQSISGVP SRFSGSGSGTDFTLTISSLQPEDFATYYCQQANSWPLTFGGGTKLEIKRTVAAPSVFIF PPDIQMTQSPSSLSASVGDRVTITCRASQSISTWLAWYQQKPGKAPKLLIYKASNLHTG VPSRFSGSGSGTEFSLTISGLQPDDFATYYCQQYNSYSRTFGCGTKVEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS | 114 |
| | DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | |
| EPB03 HC | QVQLLESGGGLVQPGGSLRLSCAASGFTFSSYTMSWVRQAPGQALEWMGTISSGGTYTY YPDSVKGRFTISRDNAKNSLYLQMNSLRAEDTAVYYCAREAIFTYWGRGTLVTVSSAST KGPSVFPLAPEVQLLESGGGLVQPGGSLRLSCAASGFTFSHYMMAWVRQAPGKCLEWVS RIGPSGGPTHYADSVKGRFTISRDNSKNTLYLQMNSLRAEDTAVYYCAGYDSGYDYVAV AGPAEYFQHWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVSNKALPAPIE KTISKAKGQPREPQVYTLPPCREEMTKNQVSLWCLVKGFYPSDIAVEWESNGQPENNYK TTPPVLDSDGSFFLYSKLTVDKSRWQQGNVFSCSVMHEALHNHYTQKSLSLSPGK | 115 |
| EPB03 LC | DIQLTQSPSSLSLSPGERVTLSCKASQDINNYLSWYQQKPDQAPKLLIKRANRLVDGVP DRFSGSGSGTDFTLKISRVEAEDVGVYYCLKYDEFPYTFGQGTRLEIKRTVAAPSVFIF PPDIQMTQSPSSLSASVGDRVTITCRASQSISTWLAWYQQKPGKAPKLLIYKASNLHTG VPSRFSGSGSGTEFSLTISGLQPDDFATYYCQQYNSYSRTFGCGTKVEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 116 |
| EPB04 HC | EVQLLESGGGLVQPGGSLRLSCAASGFTFSHYMMAWVRQAPGKGLEWVSRIGPSGGPTH YADSVKGRFTISRDNSKNTLYLQMNSLRAEDTAVYYCAGYDSGYDYVAVAGPAEYFQHW GQGTLVTVSSASTKGPSVFPLAPEVQLLESGGGLVQPGGSLRLSCAASGFTFSRYQMMW VRQAPGKCLEWVSSISPSGGVTLYADSVKGRFTISRDNSKNTLYLQMNSLRAEDTAVYY CTRELLGTVVVPVAWKMRGYFDYWGQLTLVTVSSASTKGPSVFPLAPSSKSTSGGTAAL GCLVKDYFPEPVTVSWNSGALTSGVHTFPAVLQSSGLYSLSSVVTVPSSSLGTQTYICN VNHKPSNTKVDKKVEPKSCDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNGKEY KCKVSNKALPAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEALHNHYT QKSLSLSPGK | 117 |
| EPB04 LC | DIQMTQSPSSLSASVGDRVTITCRASQSISTWLAWYQQKPGKAPKLLIYKASNLHTGVP SRFSGSGSGTEFSLTISGLQPDDFATYYCQQYNSYSRTFGQGTKVEIKRTVAAPSVFIF PPDIQMTQSPGTLSVSPGERATLSCRASQSVSSNLAWYQQKPGQAPRLLIYGASTRATG IPARFSGSGSGTEFTLTISSMQSEDFAVYYCQQYNNWPPLTFGCGTKVEIKRTVAAPSV FIFPPSDEQLKSGTASVVCLLNNFYPREAKVQWKVDNALQSGNSQESVTEQDSKDSTYS | 118 |
| | LSSTLTLSKADYEKHKVYACEVTHQGLSSPVTKSFNRGECGGGGSGGGGSGGGGSEPKS SDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTIS KAKGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | |
| EPB05 HC | EVQLLESGGGLVQPGGSLRLSCAASGFTFSHYMMAWVRQAPGKGLEWVSRIGPSGGPTH YADSVKGRFTISRDNSKNTLYLQMNSLRAEDTAVYYCAGYDSGYDYVAVAGPAEYFQHW GQGTLVTVSSASTKGPSVFPLAPEVQLVESGGGVVRPGGSLRLSCAASGFTVSDYSMNW VRQAPGKCLEWIGFIRNKANAYTTEYSASVKGRFTISRDDSKNTLYLQMNSLKTEDTAV YYCTTYPRYHAMDSWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNGQP ENNYKTTPPVLDSDGSFFLYSKLTVDKSRWQQGNVFSCSVMHEALHNHYTQKSLSLSPG K | 119 |
| EPB05 LC | DIQMTQSPSSLSASVGDRVTITCRASQSISTWLAWYQQKPGKAPKLLIYKASNLHTGVP SRFSGSGSGTEFSLTISGLQPDDFATYYCQQYNSYSRTFGQGTKVEIKRTVAAPSVFIF PPAIQLTQSPSSLSASVGDRVTITCRASQSISNNLHWYLQKPGQSPQLLIYYGFQSISG VPSRFSGSGSGTDFTLTISSLQPEDFATYYCQQANSWPLTFGCGTKLEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 120 |
| EPB06 HC | EVQLLESGGGLVQPGGSLRLSCAASGFTFSHYMMAWVRQAPGKGLEWVSRIGPSGGPTH YADSVKGRFTISRDNSKNTLYLQMNSLRAEDTAVYYCAGYDSGYDYVAVAGPAEYFQHW GQGTLVTVSSASTKGPSVFPLAPQVQLLESGGGLVQPGGSLRLSCAASGFTFSSYTMSW VRQAPGQCLEWMGTISSGGTYTYYPDSVKGRFTISRDNAKNSLYLQMNSLRAEDTAVYY CAREAIFTYWGRGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDPEVK FNWYVDGVEVHNAKTKPREEQYNSTYRVVSVLTVLHQDWLNGKEYKCKVSNKALPAPIE KTISKAKGQPREPQVYTLPPCREEMTKNQVSLWCLVKGFYPSDIAVEWESNGQPENNYK TTPPVLDSDGSFFLYSKLTVDKSRWQQGNVFSCSVMHEALHNHYTQKSLSLSPGK | 121 |
| EPB06 LC | DIQMTQSPSSLSASVGDRVTITCRASQSISTWLAWYQQKPGKAPKLLIYKASNLHTGVP SRFSGSGSGTEFSLTISGLQPDDFATYYCQQYNSYSRTFGQGTKVEIKRTVAAPSVFIF PPDIQLTQSPSSLSLSPGERVTLSCKASQDINNYLSWYQQKPDQAPKLLIKRANRLVDG VPDRFSGSGSGTDFTLKISRVEAEDVGVYYCLKYDEFPYTFGGGTRLEIKRTVAAPSVF | 122 |
| | IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | |
| EPB07 HC | EVQLVESGGGVVRPGGSLRLSCAASGFTVSDYSMNWVRQAPGKGLEWIGFIRNKANAYT TEYSASVKGRFTISRDDSKNTLYLQMNSLKTEDTAVYYCTTYPRYHAMDSWGQGTMVTV SSASTKGPSVFPLAPEVQLLESGGGLVQPGGSLRLSCAASGFTFSRYQMMWVRQAPGKG LEWVSSISPSGGVTLYADSVKGRFTISRDNSKNTLYLQMNSLRAEDTAVYYCTRELLGT VVVPVAWKMRGYFDYWGQLTLVTVSSASTKGPSVFPLAPSSKSTSGGTAALGCLVKDYF PEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKA LPAPIEKTISKAKGQPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYTQKSLSLSP GK | 123 |
| EPB07 LC | AIQLTQSPSSLSASVGDRVTITCRASQSISNNLHWYLQKPGQSPQLLIYYGFQSISGVP SRFSGSGSGTDFTLTISSLQPEDFATYYCQQANSWPLTFGGGTKLEIKRTVAAPSVFIF PPDIQMTQSPGTLSVSPGERATLSCRASQSVSSNLAWYQQKPGQAPRLLIYGASTRATG IPARFSGSGSGTEFTLTISSMQSEDFAVYYCQQYNNWPPLTFGGGTKVEIKRTVAAPSV FIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGECGGGGSGGGGSGGGGSEPKS SDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTIS KAKGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 124 |
| EPB08 HC | QVQLLESGGGLVQPGGSLRLSCAASGFTFSSYTMSWVRQAPGQALEWMGTISSGGTYTY YPDSVKGRFTISRDNAKNSLYLQMNSLRAEDTAVYYCAREAIFTYWGRGTLVTVSSAST KGPSVFPLAPEVQLLESGGGLVQPGGSLRLSCAASGFTFSRYQMMWVRQAPGKGLEWVS SISPSGGVTLYADSVKGRFTISRDNSKNTLYLQMNSLRAEDTAVYYCTRELLGTVVVPV AWKMRGYFDYWGQLTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPI EKTISKAKGQPREPQVYTLPPCREEMTKNQVSLWCLVKGFYPSDIAVEWESNGQPENNY KTTPPVLDSDGSFFLYSKLTVDKSRWQQGNVFSCSVMHEALHNHYTQKSLSLSPGK | 125 |
| EPB08 LC | DIQLTQSPSSLSLSPGERVTLSCKASQDINNYLSWYQQKPDQAPKLLIKRANRLVDGVP DRFSGSGSGTDFTLKISRVEAEDVGVYYCLKYDEFPYTFGQGTRLEIKRTVAAPSVFIF PPDIQMTQSPGTLSVSPGERATLSCRASQSVSSNLAWYQQKPGQAPRLLIYGASTRATG | 126 |
| | IPARFSGSGSGTEFTLTISSMQSEDFAVYYCQQYNNWPPLTFGGGTKVEIKRTVAAPSV FIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGECGGGGSGGGGSGGGGSEPKS SDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTIS KAKGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | |
| EPB09 HC | EVQLLESGGGLVQPGGSLRLSCAASGFTFSRYQMMWVRQAPGKGLEWVSSISPSGGVTL YADSVKGRFTISRDNSKNTLYLQMNSLRAEDTAVYYCTRELLGTVVVPVAWKMRGYFDY WGQLTLVTVSSASTKGPSVFPLAPEVQLVESGGGVVRPGGSLRLSCAASGFTVSDYSMN WVRQAPGKCLEWIGFIRNKANAYTTEYSASVKGRFTISRDDSKNTLYLQMNSLKTEDTA VYYCTTYPRYHAMDSWGQGTMVTVSSASTKGPSVFPLAPSSKSTSGGTAALGCLVKDYF PEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCKVSNKA LPAPIEKTISKAKGQPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYTQKSLSLSP GK | 127 |
| EPB09 LC | DIQMTQSPGTLSVSPGERATLSCRASQSVSSNLAWYQQKPGQAPRLLIYGASTRATGIP ARFSGSGSGTEFTLTISSMQSEDFAVYYCQQYNNWPPLTFGGGTKVEIKRTVAAPSVFI FPPAIQLTQSPSSLSASVGDRVTITCRASQSISNNLHWYLQKPGQSPQLLIYYGFQSIS GVPSRFSGSGSGTDFTLTISSLQPEDFATYYCQQANSWPLTFGCGTKLEIKRTVAAPSV FIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGECGGGGSGGGGSGGGGSEPKS SDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTIS KAKGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 128 |
| EPB10 HC | QVQLLESGGGLVQPGGSLRLSCAASGFTFSSYTMSWVRQAPGQALEWMGTISSGGTYTY YPDSVKGRFTISRDNAKNSLYLQMNSLRAEDTAVYYCAREAIFTYWGRGTLVTVSSAST KGPSVFPLAPEVQLVESGGGVVRPGGSLRLSCAASGFTVSDYSMNWVRQAPGKCLEWIG FIRNKANAYTTEYSASVKGRFTISRDDSKNTLYLQMNSLKTEDTAVYYCTTYPRYHAMD SWGQGTMVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKG QPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 129 |
| EPB10 LC | DIQLTQSPSSLSLSPGERVTLSCKASQDINNYLSWYQQKPDQAPKLLIKRANRLVDGVP DRFSGSGSGTDFTLKISRVEAEDVGVYYCLKYDEFPYTFGQGTRLEIKRTVAAPSVFIF | 130 |
| | PPAIQLTQSPSSLSASVGDRVTITCRASQSISNNLHWYLQKPGQSPQLLIYYGFQSISG VPSRFSGSGSGTDFTLTISSLQPEDFATYYCQQANSWPLTFGCGTKLEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | |
| EPB11 HC | EVQLLESGGGLVQPGGSLRLSCAASGFTFSRYQMMWVRQAPGKGLEWVSSISPSGGVTL YADSVKGRFTISRDNSKNTLYLQMNSLRAEDTAVYYCTRELLGTVVVPVAWKMRGYFDY WGQLTLVTVSSASTKGPSVFPLAPQVQLLESGGGLVQPGGSLRLSCAASGFTFSSYTMS WVRQAPGQCLEWMGTISSGGTYTYYPDSVKGRFTISRDNAKNSLYLQMNSLRAEDTAVY YCAREAIFTYWGRGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPI EKTISKAKGQPREPQVYTLPPCREEMTKNQVSLWCLVKGFYPSDIAVEWESNGQPENNY KTTPPVLDSDGSFFLYSKLTVDKSRWQQGNVFSCSVMHEALHNHYTQKSLSLSPGK | 131 |
| EPB11 LC | DIQMTQSPGTLSVSPGERATLSCRASQSVSSNLAWYQQKPGQAPRLLIYGASTRATGIP ARFSGSGSGTEFTLTISSMQSEDFAVYYCQQYNNWPPLTFGGGTKVEIKRTVAAPSVFI FPPDIQLTQSPSSLSLSPGERVTLSCKASQDINNYLSWYQQKPDQAPKLLIKRANRLVD GVPDRFSGSGSGTDFTLKISRVEAEDVGVYYCLKYDEFPYTFGCGTRLEIKRTVAAPSV FIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGECGGGGSGGGGSGGGGSEPKS SDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTIS KAKGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 132 |
| EPB12 HC | EVQLVESGGGVVRPGGSLRLSCAASGFTVSDYSMNWVRQAPGKGLEWIGFIRNKANAYT TEYSASVKGRFTISRDDSKNTLYLQMNSLKTEDTAVYYCTTYPRYHAMDSWGQGTMVTV SSASTKGPSVFPLAPQVQLLESGGGLVQPGGSLRLSCAASGFTFSSYTMSWVRQAPGQC LEWMGTISSGGTYTYYPDSVKGRFTISRDNAKNSLYLQMNSLRAEDTAVYYCAREAIFT YWGRGTLVTVSSASTKGPSVFPLAPSSKSTSGGTAALGCLVKDYFPEPVTVSWNSGALT SGVHTFPAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKG QPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 133 |
| EPB12 LC | AIQLTQSPSSLSASVGDRVTITCRASQSISNNLHWYLQKPGQSPQLLIYYGFQSISGVP SRFSGSGSGTDFTLTISSLQPEDFATYYCQQANSWPLTFGGGTKLEIKRTVAAPSVFIF | 134 |
| | PPDIQLTQSPSSLSLSPGERVTLSCKASQDINNYLSWYQQKPDQAPKLLIKRANRLVDG VPDRFSGSGSGTDFTLKISRVEAEDVGVYYCLKYDEFPYTFGCGTRLEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | |

**[Table 51]**

| **Name** | **Sequence** | **SEQ ID NO** |
|---|---|---|
| EPB01 HC | GAGGTGCAACTGCTTGAGTCAGGAGGAGGACTGGTTCAACCCGGCGGGAGCCTTCGACT CTCATGTGCAGCCTCTGGGTTTACATTTAGTCGTTACCAAATGATGTGGGTACGCCAGG CCCCCGGAAAAGGTTTGGAGTGGGTGAGCAGCATTTCTCCTTCAGGAGGAGTGACACTT TACGCAGACAGTGTTAAGGGTCGGTTCACAATCAGTCGTGATAATTCCAAGAACACTCT CTATCTCCAAATGAACTCCCTTCGAGCTGAGGACACTGCTGTTTATTACTGTACTCGGG AACTTCTCGGCACTGTGGTTGTTCCTGTGGCTTGGAAGATGCGTGGATACTTCGACTAT TGGGGGCAACTCACTCTTGTTACCGTCTCAAGCGCTAGCACCAAAGGACCTAGTGTTTT TCCTCTTGCCCCTGAGGTTCAACTCCTTGAATCCGGAGGAGGACTTGTCCAACCAGGCG GCAGCCTTAGGCTGTCCTGCGCTGCCTCAGGCTTTACATTCAGTCATTACATGATGGCC TGGGTTAGACAGGCACCCGGCAAATGTCTGGAATGGGTCAGCCGAATAGGACCATCAGG AGGTCCCACTCACTATGCCGATTCTGTAAAAGGGAGGTTTACAATTTCCAGAGACAATT CAAAGAATACCCTTTACCTCCAGATGAACTCATTGAGAGCCGAGGACACAGCCGTATAT TATTGCGCAGGTTATGATTCCGGTTACGATTACGTTGCAGTCGCAGGCCCAGCCGAATA CTTCCAACATTGGGGTCAGGGAACCCTCGTGACCGTGTCCAGTGCTAGCACCAAAGGAC CTAGTGTTTTTCCTCTTGCCCCTTCCTCAAAGTCTACCTCTGGGGGGACAGCCGCTCTG GGCTGCCTGGTCAAGGATTATTTCCCAGAGCCTGTCACTGTCAGTTGGAACTCTGGAGC CTTGACTTCTGGTGTTCATACATTTCCTGCTGTCCTTCAAAGCAGCGGCTTGTACTCAT TGTCTTCTGTTGTGACAGTACCCTCAAGCAGCCTCGGCACTCAGACATACATCTGCAAT GTCAACCACAAACCCTCAAATACAAAGGTAGATAAAAAAGTCGAACCAAAGTCTTGTGA CAAAACTCACACGTGCCCACCGTGCCCAGCACCTGAACTCCTGGGGGGACCGTCAGTCT TCCTCTTCCCCCCAAAACCCAAGGACACCCTCATGATCTCCCGGACCCCTGAGGTCACA TGCGTGGTGGTGGACGTGAGCCACGAAGACCCTGAGGTCAAGTTCAACTGGTACGTGGA CGGCGTGGAGGTGCATAATGCCAAGACAAAGCCGCGGGAGGAGCAGTACAACAGCACGT ACCGTGTGGTCAGCGTCCTCACCGTCCTGCACCAGGACTGGCTGAATGGCAAGGAGTAC AAGTGCAAGGTCTCCAACAAAGCCCTCCCAGCCCCCATCGAGAAAACCATCTCCAAAGC CAAAGGGCAGCCCCGAGAACCACAGGTGTACACCCTGCCCCCATGCCGGGAGGAGATGA CCAAGAACCAGGTCAGCCTGTGGTGCCTGGTCAAAGGCTTCTATCCCAGCGACATCGCC GTGGAGTGGGAGAGCAATGGGCAGCCGGAGAACAACTACAAGACCACGCCTCCCGTGCT GGACTCCGACGGCTCCTTCTTCCTCTACAGCAAGCTCACCGTGGACAAGAGCAGGTGGC AGCAGGGGAACGTCTTCTCATGCTCCGTGATGCATGAGGCTCTGCACAACCACTACACG CAGAAGAGCCTCTCCCTGTCTCCGGGTAAA | 528 |
| EPB01 LC | GACATACAGATGACTCAGAGCCCTGGTACACTGAGTGTATCTCCTGGGGAACGCGCCAC TCTGTCTTGCCGAGCCTCTCAAAGCGTTAGTTCCAACCTGGCTTGGTATCAGCAAAAAC CCGGTCAAGCACCTCGCTTGCTTATCTACGGGGCCTCCACACGGGCAACCGGCATTCCT GCACGCTTTAGTGGCTCTGGTAGCGGGACTGAATTCACTTTGACTATATCTTCCATGCA GAGTGAGGATTTCGCCGTTTATTATTGTCAACAGTACAATAACTGGCCTCCACTCACTT TTGGTGGTGGCACCAAAGTGGAGATAAAGCGTACGGTGGCAGCTCCCAGCGTTTTTATC TTTCCCCCAGACATACAGATGACACAAAGTCCAAGTAGCCTTTCTGCATCCGTTGGAGA | 529 |
| | GCGCGTAACCATAACTTGTCGAGCATCACAAAGCATAAGCACTTGGCTTGCTTGGTATC AGCAGAAGCCAGGCAAAGCACCCAAGCTGCTGATTTATAAAGCCTCTAATCTCCATACA GGAGTTCCCAGCCGTTTCTCAGGCAGCGGGTCTGGAACCGAATTCTCTCTGACCATCTC TGGCTTGCAACCTGATGACTTTGCAACATATTACTGTCAACAATATAATTCATATAGTC GTACTTTCGGGTGTGGCACTAAGGTGGAAATCAAACGTACGGTGGCAGCTCCCAGCGTT TTTATCTTTCCCCCATCCGACGAGCAGCTCAAGAGTGGCACTGCCTCTGTAGTTTGTTT GCTGAATAACTTCTATCCACGTGAAGCAAAAGTACAGTGGAAGGTCGATAATGCCCTTC AGAGCGGTAACAGCCAAGAAAGTGTTACCGAGCAAGATTCCAAAGATTCCACTTACAGT CTGTCCAGCACATTGACACTGAGTAAGGCTGATTACGAAAAACACAAGGTGTACGCATG CGAGGTGACACACCAAGGTCTTTCATCTCCTGTAACTAAGAGCTTTAACCGGGGAGAAT GTGGTGGTGGGGGCAGCGGGGGCGGAGGTAGTGGAGGCGGCGGTAGTGAACCAAAGAGT AGTGACAAAACTCACACGTGCCCACCGTGCCCAGCACCTGAACTCCTGGGGGGACCGTC AGTCTTCCTCTTCCCCCCAAAACCCAAGGACACCCTCATGATCTCCCGGACCCCTGAGG TCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGG AGTACAAGTGCAAGGTCTCCAACAAAGCCCTCCCAGCCCCCATCGAGAAAACCATCTCC AAAGCCAAAGGGCAGCCCCGAGAACCACAGGTGTACACCCTGCCCCCATGCCGGGAGGA GATGACCAAGAACCAGGTCAGCCTGTGGTGCCTGGTCAAAGGCTTCTATCCCAGCGACA TCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAG GTGGCAGCAGGGGAACGTCTTCTCATGCTCCGTGATGCATGAGGCTCTGCACAACCACT ACACGCAGAAGAGCCTCTCCCTGTCTCCGGGTAAA | |
| EPB02 HC | GAGGTCCAGCTGGTTGAGTCTGGAGGTGGTGTAGTCAGGCCAGGAGGTTCCCTGCGTCT GTCTTGCGCTGCCTCTGGGTTCACTGTGTCTGACTACAGTATGAACTGGGTTAGACAGG CTCCAGGGAAAGGTTTGGAATGGATAGGGTTTATTCGCAATAAAGCCAATGCCTATACC ACCGAATACAGCGCAAGCGTTAAAGGCAGGTTCACCATCAGTAGGGACGACTCTAAGAA TACCCTCTACCTGCAGATGAATAGCTTGAAGACAGAGGATACCGCTGTTTATTACTGCA CTACCTACCCCAGGTATCATGCTATGGACAGTTGGGGTCAAGGAACTATGGTCACCGTC TCCTCTGCTAGCACCAAAGGACCTAGTGTTTTTCCTCTTGCCCCTGAGGTTCAACTCCT TGAATCCGGAGGAGGACTTGTCCAACCAGGCGGCAGCCTTAGGCTGTCCTGCGCTGCCT CAGGCTTTACATTCAGTCATTACATGATGGCCTGGGTTAGACAGGCACCCGGCAAATGT CTGGAATGGGTCAGCCGAATAGGACCATCAGGAGGTCCCACTCACTATGCCGATTCTGT AAAAGGGAGGTTTACAATTTCCAGAGACAATTCAAAGAATACCCTTTACCTCCAGATGA ACTCATTGAGAGCCGAGGACACAGCCGTATATTATTGCGCAGGTTATGATTCCGGTTAC GATTACGTTGCAGTCGCAGGCCCAGCCGAATACTTCCAACATTGGGGTCAGGGAACCCT CGTGACCGTGTCCAGTGCTAGCACCAAAGGACCTAGTGTTTTTCCTCTTGCCCCTTCCT CAAAGTCTACCTCTGGGGGGACAGCCGCTCTGGGCTGCCTGGTCAAGGATTATTTCCCA GAGCCTGTCACTGTCAGTTGGAACTCTGGAGCCTTGACTTCTGGTGTTCATACATTTCC TGCTGTCCTTCAAAGCAGCGGCTTGTACTCATTGTCTTCTGTTGTGACAGTACCCTCAA | 530 |
| | GCAGCCTCGGCACTCAGACATACATCTGCAATGTCAACCACAAACCCTCAAATACAAAG GTAGATAAAAAAGTCGAACCAAAGTCTTGTGACAAAACTCACACGTGCCCACCGTGCCC AGCACCTGAACTCCTGGGGGGACCGTCAGTCTTCCTCTTCCCCCCAAAACCCAAGGACA CCCTCATGATCTCCCGGACCCCTGAGGTCACATGCGTGGTGGTGGACGTGAGCCACGAA GACCCTGAGGTCAAGTTCAACTGGTACGTGGACGGCGTGGAGGTGCATAATGCCAAGAC AAAGCCGCGGGAGGAGCAGTACAACAGCACGTACCGTGTGGTCAGCGTCCTCACCGTCC TGCACCAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCACAGGT GTACACCCTGCCCCCATGCCGGGAGGAGATGACCAAGAACCAGGTCAGCCTGTGGTGCC TGGTCAAAGGCTTCTATCCCAGCGACATCGCCGTGGAGTGGGAGAGCAATGGGCAGCCG GAGAACAACTACAAGACCACGCCTCCCGTGCTGGACTCCGACGGCTCCTTCTTCCTCTA CAGCAAGCTCACCGTGGACAAGAGCAGGTGGCAGCAGGGGAACGTCTTCTCATGCTCCG TGATGCATGAGGCTCTGCACAACCACTACACGCAGAAGAGCCTCTCCCTGTCTCCGGGT AAA | |
| EPB02 LC | GCCATACAGCTGACTCAATCACCTTCTTCTTTGAGCGCCTCTGTCGGTGATCGCGTTAC AATTACATGCCGAGCTTCTCAGTCTATCTCTAATAACCTCCACTGGTATCTCCAGAAAC CTGGACAGTCACCACAGCTTCTGATATATTACGGCTTTCAGTCTATAAGTGGAGTCCCA AGTAGATTTTCCGGTAGTGGATCTGGCACCGATTTTACTCTTACTATCTCTAGCCTCCA ACCTGAAGATTTCGCAACCTATTACTGCCAGCAGGCCAATTCCTGGCCCTTGACCTTCG GAGGTGGGACTAAGCTGGAAATCAAACGTACGGTGGCAGCTCCCAGCGTTTTTATCTTT CCCCCAGACATACAGATGACACAAAGTCCAAGTAGCCTTTCTGCATCCGTTGGAGACCG CGTAACCATAACTTGTCGAGCATCACAAAGCATAAGCACTTGGCTTGCTTGGTATCAGC AGAAGCCAGGCAAAGCACCCAAGCTGCTGATTTATAAAGCCTCTAATCTCCATACAGGA GTTCCCAGCCGTTTCTCAGGCAGCGGGTCTGGAACCGAATTCTCTCTGACCATCTCTGG CTTGCAACCTGATGACTTTGCAACATATTACTGTCAACAATATAATTCATATAGTCGTA CTTTCGGGTGTGGCACTAAGGTGGAAATCAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCATGCCGGGAGGAGAT GACCAAGAACCAGGTCAGCCTGTGGTGCCTGGTCAAAGGCTTCTATCCCAGCGACATCG | 531 |
| | CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |
| EPB03 HC | CAAGTGCAACTCCTTGAAAGTGGAGGTGGCCTTGTCCAGCCAGGCGGTTCACTGCGTTT GAGCTGTGCCGCATCCGGCTTTACCTTTTCCAGTTACACAATGAGTTGGGTCCGTCAAG CACCCGGGCAAGCTTTGGAATGGATGGGAACTATTTCATCTGGTGGGACTTACACTTAC TATGCCGATAGCGTCAAGGGACGTTTTACCATAAGCCGGGACAACGCTAAAAATTCACT CTATCTTCAGATGAACAGTCTTAGGGCTGAGGATACCGCTGTTTATTATTGCGCCCGAG AAGCCATTTTCACCTACTGGGGGCGCGGTACACTTGTCACCGTTAGCAGTGCTAGCACC AAAGGACCTAGTGTTTTTCCTCTTGCCCCTGAGGTTCAACTCCTTGAATCCGGAGGAGG ACTTGTCCAACCAGGCGGCAGCCTTAGGCTGTCCTGCGCTGCCTCAGGCTTTACATTCA GTCATTACATGATGGCCTGGGTTAGACAGGCACCCGGCAAATGTCTGGAATGGGTCAGC CGAATAGGACCATCAGGAGGTCCCACTCACTATGCCGATTCTGTAAAAGGGAGGTTTAC AATTTCCAGAGACAATTCAAAGAATACCCTTTACCTCCAGATGAACTCATTGAGAGCCG AGGACACAGCCGTATATTATTGCGCAGGTTATGATTCCGGTTACGATTACGTTGCAGTC GCAGGCCCAGCCGAATACTTCCAACATTGGGGTCAGGGAACCCTCGTGACCGTGTCCAG TGCTAGCACCAAAGGACCTAGTGTTTTTCCTCTTGCCCCTTCCTCAAAGTCTACCTCTG GGGGGACAGCCGCTCTGGGCTGCCTGGTCAAGGATTATTTCCCAGAGCCTGTCACTGTC AGTTGGAACTCTGGAGCCTTGACTTCTGGTGTTCATACATTTCCTGCTGTCCTTCAAAG CAGCGGCTTGTACTCATTGTCTTCTGTTGTGACAGTACCCTCAAGCAGCCTCGGCACTC AGACATACATCTGCAATGTCAACCACAAACCCTCAAATACAAAGGTAGATAAAAAAGTC GAACCAAAGTCTTGTGACAAAACTCACACGTGCCCACCGTGCCCAGCACCTGAACTCCT GGGGGGACCGTCAGTCTTCCTCTTCCCCCCAAAACCCAAGGACACCCTCATGATCTCCC GGACCCCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGGGAGGA GCAGTACAACAGCACGTACCGTGTGGTCAGCGTCCTCACCGTCCTGCACCAGGACTGGC TGAATGGCAAGGAGTACAAGTGCAAGGTCTCCAACAAAGCCCTCCCAGCCCCCATCGAG AAAACCATCTCCAAAGCCAAAGGGCAGCCCCGAGAACCACAGGTGTACACCCTGCCCCC ATGCCGGGAGGAGATGACCAAGAACCAGGTCAGCCTGTGGTGCCTGGTCAAAGGCTTCT ATCCCAGCGACATCGCCGTGGAGTGGGAGAGCAATGGGCAGCCGGAGAACAACTACAAG ACCACGCCTCCCGTGCTGGACTCCGACGGCTCCTTCTTCCTCTACAGCAAGCTCACCGT GGACAAGAGCAGGTGGCAGCAGGGGAACGTCTTCTCATGCTCCGTGATGCATGAGGCTC TGCACAACCACTACACGCAGAAGAGCCTCTCCCTGTCTCCGGGTAAA | 532 |
| EPB03 LC | GACATACAACTCACACAAAGTCCAAGCTCTCTTTCCCTTAGTCCAGGAGAACGCGTAAC TCTCTCATGTAAAGCCTCCCAGGATATCAATAACTATCTCTCTTGGTACCAACAAAAGC CTGACCAAGCTCCTAAGCTGCTTATCAAACGCGCCAATCGGTTGGTAGACGGAGTTCCC GACAGGTTCAGTGGGTCAGGCTCAGGTACAGATTTTACCTTGAAGATATCACGCGTGGA AGCCGAAGACGTAGGGGTATATTATTGTCTGAAATATGACGAGTTCCCCTATACTTTTG GGCAAGGAACTCGCCTGGAAATAAAACGTACGGTGGCAGCTCCCAGCGTTTTTATCTTT | 533 |
| | GCCCCAGACATACAGATGACACAAAGTCCAAGTAGCCTTTCTGCATCCGTTGGAGACCG CGTAACCATAACTTGTCGAGCATCACAAAGCATAAGCACTTGGCTTGCTTGGTATCAGC AGAAGCCAGGCAAAGCACCCAAGCTGCTGATTTATAAAGCCTCTAATCTCCATACAGGA GTTCCCAGCCGTTTCTCAGGCAGCGGGTCTGGAACCGAATTCTCTCTGACCATCTCTGG CTTGCAACCTGATGACTTTGCAACATATTACTGTCAACAATATAATTCATATAGTCGTA CTTTCGGGTGTGGCACTAAGGTGGAAATCAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCATGCCGGGAGGAGAT GACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |
| EPB04 HC | GAGGTTCAACTCCTTGAATCCGGAGGAGGACTTGTCCAACCAGGCGGCAGCCTTAGGCT GTCCTGCGCTGCCTCAGGCTTTACATTCAGTCATTACATGATGGCCTGGGTTAGACAGG CACCCGGCAAAGGTCTGGAATGGGTCAGCCGAATAGGACCATCAGGAGGTCCCACTCAC TATGCCGATTCTGTAAAAGGGAGGTTTACAATTTCCAGAGACAATTCAAAGAATACCCT TTACCTCCAGATGAACTCATTGAGAGCCGAGGACACAGCCGTATATTATTGCGCAGGTT ATGATTCCGGTTACGATTACGTTGCAGTCGCAGGCCCAGCCGAATACTTCCAACATTGG GGTCAGGGAACCCTCGTGACCGTGTCCAGTGCTAGCACCAAAGGACCTAGTGTTTTTCC TCTTGCCCCTGAGGTGCAACTGCTTGAGTCAGGAGGAGGACTGGTTCAACCCGGCGGGA GCCTTCGACTCTCATGTGCAGCCTCTGGGTTTACATTTAGTCGTTACCAAATGATGTGG GTACGCCAGGCCCCCGGAAAATGTTTGGAGTGGGTGAGCAGCATTTCTCCTTCAGGAGG AGTGACACTTTACGCAGACAGTGTTAAGGGTCGGTTCACAATCAGTCGTGATAATTCCA AGAACACTCTCTATCTCCAAATGAACTCCCTTCGAGCTGAGGACACTGCTGTTTATTAC TGTACTCGGGAACTTCTCGGCACTGTGGTTGTTCCTGTGGCTTGGAAGATGCGTGGATA CTTCGACTATTGGGGGCAACTCACTCTTGTTACCGTCTCAAGCGCTAGCACCAAAGGAC CTAGTGTTTTTCCTCTTGCCCCTTCCTCAAAGTCTACCTCTGGGGGGACAGCCGCTCTG GGCTGCCTGGTCAAGGATTATTTCCCAGAGCCTGTCACTGTCAGTTGGAACTCTGGAGC | 534 |
| | CTTGACTTCTGGTGTTCATACATTTCCTGCTGTCCTTCAAAGCAGCGGCTTGTACTCAT TGTCTTCTGTTGTGACAGTACCCTCAAGCAGCCTCGGCACTCAGACATACATCTGCAAT GTCAACCACAAACCCTCAAATACAAAGGTAGATAAAAAAGTCGAACCAAAGTCTTGTGA CAAAACTCACACGTGCCCACCGTGCCCAGCACCTGAACTCCTGGGGGGACCGTCAGTCT TCCTCTTCCCCCCAAAACCCAAGGACACCCTCATGATCTCCCGGACCCCTGAGGTCACA TGCGTGGTGGTGGACGTGAGCCACGAAGACCCTGAGGTCAAGTTCAACTGGTACGTGGA CGGCGTGGAGGTGCATAATGCCAAGACAAAGCCGCGGGAGGAGCAGTACAACAGCACGT ACCGTGTGGTCAGCGTCCTCACCGTCCTGCACCAGGACTGGCTGAATGGCAAGGAGTAC AAGTGCAAGGTCTCCAACAAAGCCCTCCCAGCCCCCATCGAGAAAACCATCTCCAAAGC CAAAGGGCAGCCCCGAGAACCACAGGTGTACACCCTGCCCCCATGCCGGGAGGAGATGA CCAAGAACCAGGTCAGCCTGTGGTGCCTGGTCAAAGGCTTCTATCCCAGCGACATCGCC GTGGAGTGGGAGAGCAATGGGCAGCCGGAGAACAACTACAAGACCACGCCTCCCGTGCT GGACTCCGACGGCTCCTTCTTCCTCTACAGCAAGCTCACCGTGGACAAGAGCAGGTGGC AGCAGGGGAACGTCTTCTCATGCTCCGTGATGCATGAGGCTCTGCACAACCACTACACG CAGAAGAGCCTCTCCCTGTCTCCGGGTAAA | |
| EPB04 LC | GACATACAGATGACACAAAGTCCAAGTAGCCTTTCTGCATCCGTTGGAGACCGCGTAAC CATAACTTGTCGAGCATCACAAAGCATAAGCACTTGGCTTGCTTGGTATCAGCAGAAGC CAGGCAAAGCACCCAAGCTGCTGATTTATAAAGCCTCTAATCTCCATACAGGAGTTCCC AGCCGTTTCTCAGGCAGCGGGTCTGGAACCGAATTCTCTCTGACCATCTCTGGCTTGCA ACCTGATGACTTTGCAACATATTACTGTCAACAATATAATTCATATAGTCGTACTTTCG GGCAAGGCACTAAGGTGGAAATCAAACGTACGGTGGCAGCTCCCAGCGTTTTTATCTTT CCCCCAGACATACAGATGACTCAGAGCCCTGGTACACTGAGTGTATCTCCTGGGGAACG CGCCACTCTGTCTTGCCGAGCCTCTCAAAGCGTTAGTTCCAACCTGGCTTGGTATCAGC AAAAACCCGGTCAAGCACCTCGCTTGCTTATCTACGGGGCCTCCACACGGGCAACCGGC ATTCCTGCACGCTTTAGTGGCTCTGGTAGCGGGACTGAATTCACTTTGACTATATCTTC CATGCAGAGTGAGGATTTCGCCGTTTATTATTGTCAACAGTACAATAACTGGCCTCCAC TCACTTTTGGTTGTGGCACCAAAGTGGAGATAAAGCGTACGGTGGCAGCTCCCAGCGTT TTTATCTTTCCCCCATCCGACGAGCAGCTCAAGAGTGGCACTGCCTCTGTAGTTTGTTT GCTGAATAACTTCTATCCACGTGAAGCAAAAGTACAGTGGAAGGTCGATAATGCCCTTC AGAGCGGTAACAGCCAAGAAAGTGTTACCGAGCAAGATTCCAAAGATTCCACTTACAGT CTGTCCAGCACATTGACACTGAGTAAGGCTGATTACGAAAAACACAAGGTGTACGCATG CGAGGTGACACACCAAGGTCTTTCATCTCCTGTAACTAAGAGCTTTAACCGGGGAGAAT GTGGTGGTGGGGGCAGCGGGGGCGGAGGTAGTGGAGGCGGCGGTAGTGAACCAAAGAGT AGTGACAAAACTCACACGTGCCCACCGTGCCCAGCACCTGAACTCCTGGGGGGACCGTC AGTCTTCCTCTTCCCCCCAAAACCCAAGGACACCCTCATGATCTCCCGGACCCCTGAGG TCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGG AGTACAAGTGCAAGGTCTCCAACAAAGCCCTCCCAGCCCCCATCGAGAAAACCATCTCC AAAGCCAAAGGGCAGCCCCGAGAACCACAGGTGTACACCCTGCCCCCATGCCGGGAGGA | 535 |
| | GATGACCAAGAACCAGGTCAGCCTGTGGTGCCTGGTCAAAGGCTTCTATCCCAGCGACA TCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAG GTGGCAGCAGGGGAACGTCTTCTCATGCTCCGTGATGCATGAGGCTCTGCACAACCACT ACACGCAGAAGAGCCTCTCCCTGTCTCCGGGTAAA [?] | |
| EPB05 HC | GAGGTTCAACTCCTTGAATCCGGAGGAGGACTTGTCCAACCAGGCGGCAGCCTTAGGCT GTCCTGCGCTGCCTCAGGCTTTACATTCAGTCATTACATGATGGCCTGGGTTAGACAGG CACCCGGCAAAGGTCTGGAATGGGTCAGCCGAATAGGACCATCAGGAGGTCCCACTCAC TATGCCGATTCTGTAAAAGGGAGGTTTACAATTTCCAGAGACAATTCAAAGAATACCCT TTACCTCCAGATGAACTCATTGAGAGCCGAGGACACAGCCGTATATTATTGCGCAGGTT ATGATTCCGGTTACGATTACGTTGCAGTCGCAGGCCCAGCCGAATACTTCCAACATTGG GGTCAGGGAACCCTCGTGACCGTGTCCAGTGCTAGCACCAAAGGACCTAGTGTTTTTCC TCTTGCCCCTGAGGTCCAGCTGGTTGAGTCTGGAGGTGGTGTAGTCAGGCCAGGAGGTT CCCTGCGTCTGTCTTGCGCTGCCTCTGGGTTCACTGTGTCTGACTACAGTATGAACTGG GTTAGACAGGCTCCAGGGAAATGTTTGGAATGGATAGGGTTTATTCGCAATAAAGCCAA TGCCTATACCACCGAATACAGCGCAAGCGTTAAAGGCAGGTTCACCATCAGTAGGGACG ACTCTAAGAATACCCTCTACCTGCAGATGAATAGCTTGAAGACAGAGGATACCGCTGTT TATTACTGCACTACCTACCCCAGGTATCATGCTATGGACAGTTGGGGTCAAGGAACTAT GGTCACCGTCTCCTCTGCTAGCACCAAAGGACCTAGTGTTTTTCCTCTTGCCCCTTCCT CAAAGTCTACCTCTGGGGGGACAGCCGCTCTGGGCTGCCTGGTCAAGGATTATTTCCCA GAGCCTGTCACTGTCAGTTGGAACTCTGGAGCCTTGACTTCTGGTGTTCATACATTTCC TGCTGTCCTTCAAAGCAGCGGCTTGTACTCATTGTCTTCTGTTGTGACAGTACCCTCAA GCAGCCTCGGCACTCAGACATACATCTGCAATGTCAACCACAAACCCTCAAATACAAAG GTAGATAAAAAAGTCGAACCAAAGTCTTGTGACAAAACTCACACGTGCCCACCGTGCCC AGCACCTGAACTCCTGGGGGGACCGTCAGTCTTCCTCTTCCCCCCAAAACCCAAGGACA CCCTCATGATCTCCCGGACCCCTGAGGTCACATGCGTGGTGGTGGACGTGAGCCACGAA GACCCTGAGGTCAAGTTCAACTGGTACGTGGACGGCGTGGAGGTGCATAATGCCAAGAC AAAGCCGCGGGAGGAGCAGTACAACAGCACGTACCGTGTGGTCAGCGTCCTCACCGTCC TGCACCAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCACAGGT GTACACCCTGCCCCCATGCCGGGAGGAGATGACCAAGAACCAGGTCAGCCTGTGGTGCC TGGTCAAAGGCTTCTATCCCAGCGACATCGCCGTGGAGTGGGAGAGCAATGGGCAGCCG GAGAACAACTACAAGACCACGCCTCCCGTGCTGGACTCCGACGGCTCCTTCTTCCTCTA CAGCAAGCTCACCGTGGACAAGAGCAGGTGGCAGCAGGGGAACGTCTTCTCATGCTCCG TGATGCATGAGGCTCTGCACAACCACTACACGCAGAAGAGCCTCTCCCTGTCTCCGGGT AAA | 536 |
| EPB05 LC | GACATACAGATGACACAAAGTCCAAGTAGCCTTTCTGCATCCGTTGGAGACCGCGTAAC CATAACTTGTCGAGCATCACAAAGCATAAGCACTTGGCTTGCTTGGTATCAGCAGAAGC CAGGCAAAGCACCCAAGCTGCTGATTTATAAAGCCTCTAATCTCCATACAGGAGTTCCC AGCCGTTTCTCAGGCAGCGGGTCTGGAACCGAATTCTCTCTGACCATCTCTGGCTTGCA | 537 |
| | ACCTGATGACTTTGCAACATATTACTGTCAACAATATAATTCATATAGTCGTACTTTCG GGCAAGGCACTAAGGTGGAAATCAAACGTACGGTGGCAGCTCCCAGCGTTTTTATCTTT CCCCCAGCCATACAGCTGACTCAATCACCTTCTTCTTTGAGCGCCTCTGTCGGTGATCG CGTTACAATTACATGCCGAGCTTCTCAGTCTATCTCTAATAACCTCCACTGGTATCTCC AGAAACCTGGACAGTCACCACAGCTTCTGATATATTACGGCTTTCAGTCTATAAGTGGA GTCCCAAGTAGATTTTCCGGTAGTGGATCTGGCACCGATTTTACTCTTACTATCTCTAG CCTCCAACCTGAAGATTTCGCAACCTATTACTGCCAGCAGGCCAATTCCTGGCCCTTGA CCTTCGGATGTGGGACTAAGCTGGAAATCAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCATGCCGGGAGGAGAT GACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |
| EPB06 HC | GAGGTTCAACTCCTTGAATCCGGAGGAGGACTTGTCCAACCAGGCGGCAGCCTTAGGCT GTCCTGCGCTGCCTCAGGCTTTACATTCAGTCATTACATGATGGCCTGGGTTAGACAGG CACCCGGCAAAGGTCTGGAATGGGTCAGCCGAATAGGACCATCAGGAGGTCCCACTCAC TATGCCGATTCTGTAAAAGGGAGGTTTACAATTTCCAGAGACAATTCAAAGAATACCCT TTACCTCCAGATGAACTCATTGAGAGCCGAGGACACAGCCGTATATTATTGCGCAGGTT ATGATTCCGGTTACGATTACGTTGCAGTCGCAGGCCCAGCCGAATACTTCCAACATTGG GGTCAGGGAACCCTCGTGACCGTGTCCAGTGCTAGCACCAAAGGACCTAGTGTTTTTCC TCTTGCCCCTCAAGTGCAACTCCTTGAAAGTGGAGGTGGCCTTGTCCAGCCAGGCGGTT CACTGCGTTTGAGCTGTGCCGCATCCGGCTTTACCTTTTCCAGTTACACAATGAGTTGG GTCCGTCAAGCACCCGGGCAATGTTTGGAATGGATGGGAACTATTTCATCTGGTGGGAC TTACACTTACTATCCCGATAGCGTCAAGGGACGTTTTACCATAAGCCGGGACAACGCTA AAAATTCACTCTATCTTCAGATGAACAGTCTTAGGGCTGAGGATACCGCTGTTTATTAT TGCGCCCGAGAAGCCATTTTCACCTACTGGGGGCGCGGTACACTTGTCACCGTTAGCAG TGCTAGCACCAAAGGACCTAGTGTTTTTCCTCTTGCCCCTTCCTCAAAGTCTACCTCTG | 538 |
| | GGGGGACAGCCGCTCTGGGCTGCCTGGTCAAGGATTATTTCCCAGAGCCTGTCACTGTC AGTTGGAACTCTGGAGCCTTGACTTCTGGTGTTCATACATTTCCTGCTGTCCTTCAAAG CAGCGGCTTGTACTCATTGTCTTCTGTTGTGACAGTACCCTCAAGCAGCCTCGGCACTC AGACATACATCTGCAATGTCAACCACAAACCCTCAAATACAAAGGTAGATAAAAAAGTC GAACCAAAGTCTTGTGACAAAACTCACACGTGCCCACCGTGCCCAGCACCTGAACTCCT GGGGGGACCGTCAGTCTTCCTCTTCCCCCCAAAACCCAAGGACACCCTCATGATCTCCC GGACCCCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGGGAGGA GCAGTACAACAGCACGTACCGTGTGGTCAGCGTCCTCACCGTCCTGCACCAGGACTGGC TGAATGGCAAGGAGTACAAGTGCAAGGTCTCCAACAAAGCCCTCCCAGCCCCCATCGAG AAAACCATCTCCAAAGCCAAAGGGCAGCCCCGAGAACCACAGGTGTACACCCTGCCCCC ATGCCGGGAGGAGATGACCAAGAACCAGGTCAGCCTGTGGTGCCTGGTCAAAGGCTTCT ATCCCAGCGACATCGCCGTGGAGTGGGAGAGCAATGGGCAGCCGGAGAACAACTACAAG ACCACGCCTCCCGTGCTGGACTCCGACGGCTCCTTCTTCCTCTACAGCAAGCTCACCGT GGACAAGAGCAGGTGGCAGCAGGGGAACGTCTTCTCATGCTCCGTGATGCATGAGGCTC TGCACAACCACTACACGCAGAAGAGCCTCTCCCTGTCTCCGGGTAAA | |
| EPB06 LC | GACATACAGATGACACAAAGTCCAAGTAGCCTTTCTGCATCCGTTGGAGACCGCGTAAC CATAACTTGTCGAGCATCACAAAGCATAAGCACTTGGCTTGCTTGGTATCAGCAGAAGC CAGGCAAAGCACCCAAGCTGCTGATTTATAAAGCCTCTAATCTCCATACAGGAGTTCCC AGCCGTTTCTCAGGCAGCGGGTCTGGAACCGAATTCTCTCTGACCATCTCTGGCTTGCA ACCTGATGACTTTGCAACATATTACTGTCAACAATATAATTCATATAGTCGTACTTTCG GGCAAGGCACTAAGGTGGAAATCAAACGTACGGTGGCAGCTCCCAGCGTTTTTATCTTT CCCCCAGACATACAACTCACACAAAGTCCAAGCTCTCTTTCCCTTAGTCCAGGAGAACG CGTAACTCTCTCATGTAAAGCCTCCCAGGATATCAATAACTATCTCTCTTGGTACCAAC AAAAGCCTGACCAAGCTCCTAAGCTGCTTATCAAACGCGCCAATCGGTTGGTAGACGGA GTTCCCGACAGGTTCAGTGGGTCAGGCTCAGGTACAGATTTTACCTTGAAGATATCACG CGTGGAAGCCGAAGACGTAGGGGTATATTATTGTCTGAAATATGACGAGTTCCCCTATA CTTTTGGGTGTGGAACTCGCCTGGAAATAAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA | 539 |
| | GGCAAAGGGCAGCCCCGAGAACCACAGGTGTACACCCTGCCCCCATGCCGGGAGGAGAT GACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |
| EPB07 HC | GAGGTCCAGCTGGTTGAGTCTGGAGGTGGTGTAGTCAGGCCAGGAGGTTCCCTGCGTCT GTCTTGCGCTGCCTCTGGGTTCACTGTGTCTGACTACAGTATGAACTGGGTTAGACAGG CTCCAGGGAAAGGTTTGGAATGGATAGGGTTTATTCGCAATAAAGCCAATGCCTATACC ACCGAATACAGCGCAAGCGTTAAAGGCAGGTTCACCATCAGTAGGGACGACTCTAAGAA TACCCTCTACCTGCAGATGAATAGCTTGAAGACAGAGGATACCGCTGTTTATTACTGCA CTACCTACCCCAGGTATCATGCTATGGACAGTTGGGGTCAAGGAACTATGGTCACCGTC TCCTCTGCTAGCACCAAAGGACCTAGTGTTTTTCCTCTTGCCCCTGAGGTGCAACTGCT TGAGTCAGGAGGAGGACTGGTTCAACCCGGCGGGAGCCTTCGACTCTCATGTGCAGCCT CTGGGTTTACATTTAGTCGTTACCAAATGATGTGGGTACGCCAGGCCCCCGGAAAATGT TTGGAGTGGGTGAGCAGCATTTCTCCTTCAGGAGGAGTGACACTTTACGCAGACAGTGT TAAGGGTCGGTTCACAATCAGTCGTGATAATTCCAAGAACACTCTCTATCTCCAAATGA ACTCCCTTCGAGCTGAGGACACTGCTGTTTATTACTGTACTCGGGAACTTCTCGGCACT GTGGTTGTTCCTGTGGCTTGGAAGATGCGTGGATACTTCGACTATTGGGGGCAACTCAC TCTTGTTACCGTCTCAAGCGCTAGCACCAAAGGACCTAGTGTTTTTCCTCTTGCCCCTT CCTCAAAGTCTACCTCTGGGGGGACAGCCGCTCTGGGCTGCCTGGTCAAGGATTATTTC CCAGAGCCTGTCACTGTCAGTTGGAACTCTGGAGCCTTGACTTCTGGTGTTCATACATT TCCTGCTGTCCTTCAAAGCAGCGGCTTGTACTCATTGTCTTCTGTTGTGACAGTACCCT CAAGCAGCCTCGGCACTCAGACATACATCTGCAATGTCAACCACAAACCCTCAAATACA AAGGTAGATAAAAAAGTCGAACCAAAGTCTTGTGACAAAACTCACACGTGCCCACCGTG CCCAGCACCTGAACTCCTGGGGGGACCGTCAGTCTTCCTCTTCCCCCCAAAACCCAAGG ACACCCTCATGATCTCCCGGACCCCTGAGGTCACATGCGTGGTGGTGGACGTGAGCCAC GAAGACCCTGAGGTCAAGTTCAACTGGTACGTGGACGGCGTGGAGGTGCATAATGCCAA GACAAAGCCGCGGGAGGAGCAGTACAACAGCACGTACCGTGTGGTCAGCGTCCTCACCG TCCTGCACCAGGACTGGCTGAATGGCAAGGAGTACAAGTGCAAGGTCTCCAACAAAGCC CTCCCAGCCCCCATCGAGAAAACCATCTCCAAAGCCAAAGGGCAGCCCCGAGAACCACA GGTGTACACCCTGCCCCCATGCCGGGAGGAGATGACCAAGAACCAGGTCAGCCTGTGGT GCCTGGTCAAAGGCTTCTATCCCAGCGACATCGCCGTGGAGTGGGAGAGCAATGGGCAG CCGGAGAACAACTACAAGACCACGCCTCCCGTGCTGGACTCCGACGGCTCCTTCTTCCT CTACAGCAAGCTCACCGTGGACAAGAGCAGGTGGCAGCAGGGGAACGTCTTCTCATGCT CCGTGATGCATGAGGCTCTGCACAACCACTACACGCAGAAGAGCCTCTCCCTGTCTCCG GGTAAA | 540 |
| EPB07 LC | GCCATACAGCTGACTCAATCACCTTCTTCTTTGAGCGCCTCTGTCGGTGATCGCGTTAC AATTACATGCCGAGCTTCTCAGTCTATCTCTAATAACCTCCACTGGTATCTCCAGAAAC CTGGACAGTCACCACAGCTTCTGATATATTACGGCTTTCAGTCTATAAGTGGAGTCCCA | 541 |
| | AGTAGATTTTCCGGTAGTGGATCTGGCACCGATTTTACTCTTACTATCTCTAGCCTCCA ACCTGAAGATTTCGCAACCTATTACTGCCAGCAGGCCAATTCCTGGCGCTTGACCTTCG GAGGTGGGACTAAGCTGGAAATCAAACGTACGGTGGCAGCTCCCAGCGTTTTTATCTTT CCCCCAGACATACAGATGACTCAGAGCCCTGGTACACTGAGTGTATCTCCTGGGGAACG CGCCACTCTGTCTTGCCGAGCCTCTCAAAGCGTTAGTTCCAACCTGGCTTGGTATCAGC AAAAACCCGGTCAAGCACCTCGCTTGCTTATCTACGGGGCCTCCACACGGGCAACCGGC ATTCCTGCACGCTTTAGTGGCTCTGGTAGCGGGACTGAATTCACTTTGACTATATCTTC CATGCAGAGTGAGGATTTCGCCGTTTATTATTGTCAACAGTACAATAACTGGCCTCCAC TCACTTTTGGTTGTGGCACCAAAGTGGAGATAAAGCGTACGGTGGCAGCTCCCAGCGTT TTTATCTTTCCCCCATCCGACGAGCAGCTCAAGAGTGGCACTGCCTCTGTAGTTTGTTT GCTGAATAACTTCTATCCACGTGAAGCAAAAGTACAGTGGAAGGTCGATAATGCCCTTC AGAGCGGTAACAGCCAAGAAAGTGTTACCGAGCAAGATTCCAAAGATTCCACTTACAGT CTGTCCAGCACATTGACACTGAGTAAGGCTGATTACGAAAAACACAAGGTGTACGCATG CGAGGTGACACACCAAGGTCTTTCATCTCCTGTAACTAAGAGCTTTAACCGGGGAGAAT GTGGTGGTGGGGGCAGCGGGGGCGGAGGTAGTGGAGGCGGCGGTAGTGAACCAAAGAGT AGTGACAAAACTCACACGTGCCCACCGTGCCCAGCACCTGAACTCCTGGGGGGACCGTC AGTCTTCCTCTTCCCCCCAAAACCCAAGGACACCCTCATGATCTCCCGGACCCCTGAGG TCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGG AGTACAAGTGCAAGGTCTCCAACAAAGCCCTCCCAGCCCCCATCGAGAAAACCATCTCC AAAGCCAAAGGGCAGCCCCGAGAACCACAGGTGTACACCCTGCCCCCATGCCGGGAGGA GATGACCAAGAACCAGGTCAGCCTGTGGTGCCTGGTCAAAGGCTTCTATCCCAGCGACA TCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAG GTGGCAGCAGGGGAACGTCTTCTCATGCTCCGTGATGCATGAGGCTCTGCACAACCACT ACACGCAGAAGAGCCTCTCCCTGTCTCCGGGTAAA | |
| EPB08 HC | CAAGTGCAACTCCTTGAAAGTGGAGGTGGCCTTGTCCAGCCAGGCGGTTCACTGCGTTT GAGCTGTGCCGCATCCGGCTTTACCTTTTCCAGTTACACAATGAGTTGGGTCCGTCAAG CACCCGGGCAAGCTTTGGAATGGATGGGAACTATTTCATCTGGTGGGACTTACACTTAC TATCCCGATAGCGTCAAGGGACGTTTTACCATAAGCCGGGACAACGCTAAAAATTCACT CTATCTTCAGATGAACAGTCTTAGGGCTGAGGATACCGCTGTTTATTATTGCGCCCGAG AAGCCATTTTCACCTACTGGGGGCGCGGTACACTTGTCACCGTTAGCAGTGCTAGCACC AAAGGACCTAGTGTTTTTCCTCTTGCCCCTGAGGTGCAACTGCTTGAGTCAGGAGGAGG ACTGGTTCAACCCGGCGGGAGCCTTCGACTCTCATGTGCAGCCTCTGGGTTTACATTTA GTCGTTACCAAATGATGTGGGTACGCCAGGCCCCCGGAAAATGTTTGGAGTGGGTGAGC AGCATTTCTCCTTCAGGAGGAGTGACACTTTACGCAGACAGTGTTAAGGGTCGGTTCAC AATCAGTCGTGATAATTCCAAGAACACTCTCTATCTCCAAATGAACTCCCTTCGAGCTG AGGACACTGCTGTTTATTACTGTACTCGGGAACTTCTCGGCACTGTGGTTGTTCCTGTG GCTTGGAAGATGCGTGGATACTTCGACTATTGGGGGCAACTCACTCTTGTTACCGTCTC | 542 |
| | AAGCGCTAGCACCAAAGGACCTAGTGTTTTTCCTCTTGCCCCTTCCTCAAAGTCTACCT CTGGGGGGACAGCCGCTCTGGGCTGCCTGGTCAAGGATTATTTCCCAGAGCCTGTCACT GTCAGTTGGAACTCTGGAGCCTTGACTTCTGGTGTTCATACATTTCCTGCTGTCCTTCA AAGCAGCGGCTTGTACTCATTGTCTTCTGTTGTGACAGTACCCTCAAGCAGCCTCGGCA CTCAGACATACATCTGCAATGTCAACCACAAACCCTCAAATACAAAGGTAGATAAAAAA GTCGAACCAAAGTCTTGTGACAAAACTCACACGTGCCCACCGTGCCCAGCACCTGAACT CCTGGGGGGACCGTCAGTCTTCCTCTTCCCCCCAAAACCCAAGGACACCCTCATGATCT CCCGGACCCCTGAGGTCACATGCGTGGTGGTGGACGTGAGCCACGAAGACCCTGAGGTC AAGTTCAACTGGTACGTGGACGGCGTGGAGGTGCATAATGCCAAGACAAAGCCGCGGGA GGAGCAGTACAACAGCACGTACCGTGTGGTCAGCGTCCTCACCGTCCTGCACCAGGACT GGCTGAATGGCAAGGAGTACAAGTGCAAGGTCTCCAACAAAGCCCTCCCAGCCCCCATC GAGAAAACCATCTCCAAAGCCAAAGGGCAGCCCCGAGAACCACAGGTGTACACCCTGCC CCCATGCCGGGAGGAGATGACCAAGAACCAGGTCAGCCTGTGGTGCCTGGTCAAAGGCT TCTATCCCAGCGACATCGCCGTGGAGTGGGAGAGCAATGGGCAGCCGGAGAACAACTAC AAGACCACGCCTCCCGTGCTGGACTCCGACGGCTCCTTCTTCCTCTACAGCAAGCTCAC CGTGGACAAGAGCAGGTGGCAGCAGGGGAACGTCTTCTCATGCTCCGTGATGCATGAGG CTCTGCACAACCACTACACGCAGAAGAGCCTCTCCCTGTCTCCGGGTAAA | |
| EPB08 LC | GACATACAACTCACACAAAGTCCAAGCTCTCTTTCCCTTAGTCCAGGAGAACGCGTAAC TCTCTCATGTAAAGCCTCCCAGGATATCAATAACTATCTCTCTTGGTACCAACAAAAGC CTGACCAAGCTCCTAAGCTGCTTATCAAACGCGCCAATCGGTTGGTAGACGGAGTTCCC GACAGGTTCAGTGGGTCAGGCTCAGGTACAGATTTTACCTTGAAGATATCACGCGTGGA AGCCGAAGACGTAGGGGTATATTATTGTCTGAAATATGACGAGTTCCCCTATACTTTTG GGCAAGGAACTCGCCTGGAAATAAAACGTACGGTGGCAGCTCCCAGCGTTTTTATCTTT CCCCCAGACATACAGATGACTCAGAGCCCTGGTACACTGAGTGTATCTCCTGGGGAACG CGCCACTCTGTCTTGCCGAGCCTCTCAAAGCGTTAGTTCCAACCTGGCTTGGTATCAGC AAAAACCCGGTCAAGCACCTCGCTTGCTTATCTACGGGGCCTCCACACGGGCAACCGGC ATTCCTGCACGCTTTAGTGGCTCTGGTAGCGGGACTGAATTCACTTTGACTATATCTTC CATGCAGAGTGAGGATTTCGCCGTTTATTATTGTCAACAGTACAATAACTGGCCTCCAC TCACTTTTGGTTGTGGCACCAAAGTGGAGATAAAGCGTACGGTGGCAGCTCCCAGCGTT TTTATCTTTCCCCCATCCGACGAGCAGCTCAAGAGTGGCACTGCCTCTGTAGTTTGTTT GCTGAATAACTTCTATCCACGTGAAGCAAAAGTACAGTGGAAGGTCGATAATGCCCTTC AGAGCGGTAACAGCCAAGAAAGTGTTACCGAGCAAGATTCCAAAGATTCCACTTACAGT CTGTCCAGCACATTGACACTGAGTAAGGCTGATTACGAAAAACACAAGGTGTACGCATG CGAGGTGACACACCAAGGTCTTTCATCTCCTGTAACTAAGAGCTTTAACCGGGGAGAAT GTGGTGGTGGGGGCAGCGGGGGCGGAGGTAGTGGAGGCGGCGGTAGTGAACCAAAGAGT AGTGACAAAACTCACACGTGCCCACCGTGCCCAGCACCTGAACTCCTGGGGGGACCGTC AGTCTTCCTCTTCCCCCCAAAACCCAAGGACACCCTCATGATCTCCCGGACCCCTGAGG TCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGG | 543 |
| | AGTACAAGTGCAAGGTCTCCAACAAAGCCCTCCCAGCCCCCATCGAGAAAACCATCTCC AAAGCCAAAGGGCAGCCCCGAGAACCACAGGTGTACACCCTGCCCCCATGCCGGGAGGA GATGACCAAGAACCAGGTCAGCCTGTGGTGCCTGGTCAAAGGCTTCTATCCCAGCGACA TCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAG GTGGCAGCAGGGGAACGTCTTCTCATGCTCCGTGATGCATGAGGCTCTGCACAACCACT ACACGCAGAAGAGCCTCTCCCTGTCTCCGGGTAAA | |
| EPB09 HC | GAGGTGCAACTGCTTGAGTCAGGAGGAGGACTGGTTCAACCCGGCGGGAGCCTTCGACT CTCATGTGCAGCCTCTGGGTTTACATTTAGTCGTTACCAAATGATGTGGGTACGCCAGG CCCCCGGAAAAGGTTTGGAGTGGGTGAGCAGCATTTCTCCTTCAGGAGGAGTGACACTT TACGCAGACAGTGTTAAGGGTCGGTTCACAATCAGTCGTGATAATTCCAAGAACACTCT CTATCTCCAAATGAACTCCCTTCGAGCTGAGGACACTGCTGTTTATTACTGTACTCGGG AACTTCTCGGCACTGTGGTTGTTCCTGTGGCTTGGAAGATGCGTGGATACTTCGACTAT TGGGGGCAACTCACTCTTGTTACCGTCTCAAGCGCTAGCACCAAAGGACCTAGTGTTTT TCCTCTTGCCCCTGAGGTCCAGCTGGTTGAGTCTGGAGGTGGTGTAGTCAGGCCAGGAG GTTCCCTGCGTCTGTCTTGCGCTGCCTCTGGGTTCACTGTGTCTGACTACAGTATGAAC TGGGTTAGACAGGCTCCAGGGAAATGTTTGGAATGGATAGGGTTTATTCGCAATAAAGC CAATGCCTATACCACCGAATACAGCGCAAGCGTTAAAGGCAGGTTCACCATCAGTAGGG ACGACTCTAAGAATACCCTCTACCTGCAGATGAATAGCTTGAAGACAGAGGATACCGCT GTTTATTACTGCACTACCTACCCCAGGTATCATGCTATGGACAGTTGGGGTCAAGGAAC TATGGTCACCGTCTCCTCTGCTAGCACCAAAGGACCTAGTGTTTTTCCTCTTGCCCCTT CCTCAAAGTCTACCTCTGGGGGGACAGCCGCTCTGGGCTGCCTGGTCAAGGATTATTTC CCAGAGCCTGTCACTGTCAGTTGGAACTCTGGAGCCTTGACTTCTGGTGTTCATACATT TCCTGCTGTCCTTCAAAGCAGCGGCTTGTACTCATTGTCTTCTGTTGTGACAGTACCCT CAAGCAGCCTCGGCACTCAGACATACATCTGCAATGTCAACCACAAACCCTCAAATACA AAGGTAGATAAAAAAGTCGAACCAAAGTCTTGTGACAAAACTCACACGTGCCCACCGTG CCCAGCACCTGAACTCCTGGGGGGACCGTCAGTCTTCCTCTTCCCCCCAAAACCCAAGG ACACCCTCATGATCTCCCGGACCCCTGAGGTCACATGCGTGGTGGTGGACGTGAGCCAC GAAGACCCTGAGGTCAAGTTCAACTGGTACGTGGACGGCGTGGAGGTGCATAATGCCAA GACAAAGCCGCGGGAGGAGCAGTACAACAGCACGTACCGTGTGGTCAGCGTCCTCACCG TCCTGCACCAGGACTGGCTGAATGGCAAGGAGTACAAGTGCAAGGTCTCCAACAAAGCC CTCCCAGCCCCCATCGAGAAAACCATCTCCAAAGCCAAAGGGCAGCCCCGAGAACCACA GGTGTACACCCTGCCCCCATGCCGGGAGGAGATGACCAAGAACCAGGTCAGCCTGTGGT GCCTGGTCAAAGGCTTCTATCCCAGCGACATCGCCGTGGAGTGGGAGAGCAATGGGCAG CCGGAGAACAACTACAAGACCACGCCTCCCGTGCTGGACTCCGACGGCTCCTTCTTCCT CTACAGCAAGCTCACCGTGGACAAGAGCAGGTGGCAGCAGGGGAACGTCTTCTCATGCT CCGTGATGCATGAGGCTCTGCACAACCACTACACGCAGAAGAGCCTCTCCCTGTCTCCG GGTAAA | 544 |
| EPB09 LC | GACATACAGATGACTCAGAGCCCTGGTACACTGAGTGTATCTCCTGGGGAACGCGCCAC TCTGTCTTGCCGAGCCTCTCAAAGCGTTAGTTCCAACCTGGCTTGGTATCAGCAAAAAC | 545 |
| | CCGGTCAAGCACCTCGCTTGCTTATCTACGGGGCCTCCACACGGGCAACCGGCATTCCT GCACGCTTTAGTGGCTCTGGTAGCGGGACTGAATTCACTTTGACTATATCTTCCATGCA GAGTGAGGATTTCGCCGTTTATTATTGTCAACAGTACAATAACTGGCCTCCACTCACTT TTGGTGGTGGCACCAAAGTGGAGATAAAGCGTACGGTGGCAGCTCCCAGCGTTTTTATC TTTCCCCCAGCCATACAGCTGACTCAATCACCTTCTTCTTTGAGCGCCTCTGTCGGTGA TCGCGTTACAATTACATGCCGAGCTTCTCAGTCTATCTCTAATAACCTCCACTGGTATC TCCAGAAACCTGGACAGTCACCACAGCTTCTGATATATTACGGCTTTCAGTCTATAAGT GGAGTCCCAAGTAGATTTTCCGGTAGTGGATCTGGCACCGATTTTACTCTTACTATCTC TAGCCTCCAACCTGAAGATTTCGCAACCTATTACTGCCAGCAGGCCAATTCCTGGCCCT TGACCTTCGGATGTGGGACTAAGCTGGAAATCAAACGTACGGTGGCAGCTCCCAGCGTT TTTATCTTTCCCCCATCCGACGAGCAGCTCAAGAGTGGCACTGCCTCTGTAGTTTGTTT GCTGAATAACTTCTATCCACGTGAAGCAAAAGTACAGTGGAAGGTCGATAATGCCCTTC AGAGCGGTAACAGCCAAGAAAGTGTTACCGAGCAAGATTCCAAAGATTCCACTTACAGT CTGTCCAGCACATTGACACTGAGTAAGGCTGATTACGAAAAACACAAGGTGTACGCATG CGAGGTGACACACCAAGGTCTTTCATCTCCTGTAACTAAGAGCTTTAACCGGGGAGAAT GTGGTGGTGGGGGCAGCGGGGGCGGAGGTAGTGGAGGCGGCGGTAGTGAACCAAAGAGT AGTGACAAAACTCACACGTGCCCACCGTGCCCAGCACCTGAACTCCTGGGGGGACCGTC AGTCTTCCTCTTCCCCCCAAAACCCAAGGACACCCTCATGATCTCCCGGACCCCTGAGG TCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGG AGTACAAGTGCAAGGTCTCCAACAAAGCCCTCCCAGCCCCCATCGAGAAAACCATCTCC AAAGCCAAAGGGCAGCCCCGAGAACCACAGGTGTACACCCTGCCCCCATGCCGGGAGGA GATGACCAAGAACCAGGTCAGCCTGTGGTGCCTGGTCAAAGGCTTCTATCCCAGCGACA TCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAG GTGGCAGCAGGGGAACGTCTTCTCATGCTCCGTGATGCATGAGGCTCTGCACAACCACT ACACGCAGAAGAGCCTCTCCCTGTCTCCGGGTAAA | |
| EPB10 HC | CAAGTGCAACTCCTTGAAAGTGGAGGTGGCCTTGTCCAGCCAGGCGGTTCACTGCGTTT GAGCTGTGCCGCATCCGGCTTTACCTTTTCCAGTTACACAATGAGTTGGGTCCGTCAAG CACCCGGGCAAGCTTTGGAATGGATGGGAACTATTTCATCTGGTGGGACTTACACTTAC TATCCCGATAGCGTCAAGGGACGTTTTACCATAAGCCGGGACAACGCTAAAAATTCACT CTATCTTCAGATGAACAGTCTTAGGGCTGAGGATACCGCTGTTTATTATTGCGCCCGAG AAGCCATTTTCACCTACTGGGGGCGCGGTACACTTGTCACCGTTAGCAGTGCTAGCACC AAAGGACCTAGTGTTTTTCCTCTTGCCCCTGAGGTCCAGCTGGTTGAGTCTGGAGGTGG TGTAGTCAGGCCAGGAGGTTCCCTGCGTCTGTCTTGCGCTGCCTCTGGGTTCACTGTGT CTGACTACAGTATGAACTGGGTTAGACAGGCTCCAGGGAAATGTTTGGAATGGATAGGG TTTATTCGCAATAAAGCCAATGCCTATACCACCGAATACAGCGCAAGCGTTAAAGGCAG GTTCACCATCAGTAGGGACGACTCTAAGAATACCCTCTACCTGCAGATGAATAGCTTGA AGACAGAGGATACCGCTGTTTATTACTGCACTACCTACCCCAGGTATCATGCTATGGAC | 546 |
| | AGTTGGGGTCAAGGAACTATGGTCACCGTCTCCTCTGCTAGCACCAAAGGACCTAGTGT TTTTCCTCTTGCCCCTTGCTCAAAGTCTACCTCTGGGGGGACAGCCGCTCTGGGCTGCC TGGTCAAGGATTATTTCCCAGAGCCTGTCACTGTCAGTTGGAACTCTGGAGCCTTGACT TCTGGTGTTCATACATTTCCTGCTGTCCTTCAAAGCAGCGGCTTGTACTCATTGTCTTC TGTTGTGACAGTACCCTCAAGCAGCCTCGGCACTCAGACATACATCTGCAATGTCAACC ACAAACCCTCAAATACAAAGGTAGATAAAAAAGTCGAACCAAAGTCTTGTGACAAAACT CACACGTGCCCACCGTGCCCAGCACCTGAACTCCTGGGGGGACCGTCAGTCTTCCTCTT CCCCCCAAAACCCAAGGACACCCTCATGATCTCCCGGACCCCTGAGGTCACATGCGTGG TGGTGGACGTGAGCCACGAAGACCCTGAGGTCAAGTTCAACTGGTACGTGGACGGCGTG GAGGTGCATAATGCCAAGACAAAGCCGCGGGAGGAGCAGTACAACAGCACGTACCGTGT GGTCAGCGTCCTCACCGTCCTGCACCAGGACTGGCTGAATGGCAAGGAGTACAAGTGCA AGGTCTCCAACAAAGCCCTCCCAGCCCCCATCGAGAAAACCATCTCCAAAGCCAAAGGG CAGCCCCGAGAACCACAGGTGTACACCCTGCCCCCATGCCGGGAGGAGATGACCAAGAA CCAGGTCAGCCTGTGGTGCCTGGTCAAAGGCTTCTATCCCAGCGACATCGCCGTGGAGT GGGAGAGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAGCAGGG GAACGTCTTCTCATGCTCCGTGATGCATGAGGCTCTGCACAACCACTACACGCAGAAGA GCCTCTCCCTGTCTCCGGGTAAA | |
| EFB10 LC | GACATACAACTCACACAAAGTCCAAGCTCTCTTTCCCTTAGTCCAGGAGAACGCGTAAC TCTCTCATGTAAAGCCTCCCAGGATATCAATAACTATCTCTCTTGGTACCAACAAAAGC CTGACCAAGCTCCTAAGCTGCTTATCAAACGCGCCAATCGGTTGGTAGACGGAGTTCCC GACAGGTTCAGTGGGTCAGGCTCAGGTACAGATTTTACCTTGAAGATATCACGCGTGGA AGCCGAAGACGTAGGGGTATATTATTGTCTGAAATATGACGAGTTCCCCTATACTTTTG GGCAAGGAACTCGCCTGGAAATAAAACGTACGGTGGCAGCTCCCAGCGTTTTTATCTTT CCCCCAGCCATACAGCTGACTCAATCACCTTCTTCTTTGAGCGCCTCTGTCGGTGATCG CGTTACAATTACATGCCGAGCTTCTCAGTCTATCTCTAATAACCTCCACTGGTATCTCC AGAAACCTGGACAGTCACCACAGCTTCTGATATATTACGGCTTTCAGTCTATAAGTGGA GTCCCAAGTAGATTTTCCGGTAGTGGATCTGGCACCGATTTTACTCTTACTATCTCTAG CCTCCAACCTGAAGATTTCGCAACCTATTACTGCCAGCAGGCCAATTCCTGGCCCTTGA CCTTCGGATGTGGGACTAAGCTGGAAATCAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC | 547 |
| | GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCATGCCGGGAGGAGAT GACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |
| EPB11 HC | GAGGTGCAACTGCTTGAGTCAGGAGGAGGACTGGTTCAACCCGGCGGGAGCCTTCGACT CTCATGTGCAGCCTCTGGGTTTACATTTAGTCGTTACCAAATGATGTGGGTACGCCAGG CCCCCGGAAAAGGTTTGGAGTGGGTGAGCAGCATTTCTCCTTCAGGAGGAGTGACACTT TACGCAGACAGTGTTAAGGGTCGGTTCACAATCAGTCGTGATAATTCCAAGAACACTCT CTATCTCCAAATGAACTCCCTTCGAGCTGAGGACACTGCTGTTTATTACTGTACTCGGG AACTTCTCGGCACTGTGGTTGTTCCTGTGGCTTGGAAGATGCGTGGATACTTCGACTAT TGGGGGCAACTCACTCTTGTTACCGTCTCAAGCGCTAGCACCAAAGGACCTAGTGTTTT TCCTCTTGCCCCTCAAGTGCAACTCCTTGAAAGTGGAGGTGGCCTTGTCCAGCCAGGCG GTTCACTGCGTTTGAGCTGTGCCGCATCCGGCTTTACCTTTTCCAGTTACACAATGAGT TGGGTCCGTCAAGCACCCGGGCAATGTTTGGAATGGATGGGAACTATTTCATCTGGTGG GACTTACACTTACTATCCCGATAGCGTCAAGGGACGTTTTACCATAAGCCGGGACAACG CTAAAAATTCACTCTATCTTCAGATGAACAGTCTTAGGGCTGAGGATACCGCTGTTTAT TATTGCGCCCGAGAAGCCATTTTCACCTACTGGGGGCGCGGTACACTTGTCACCGTTAG CAGTGCTAGCACCAAAGGACCTAGTGTTTTTCCTCTTGCCCCTTCCTCAAAGTCTACCT CTGGGGGGACAGCCGCTCTGGGCTGCCTGGTCAAGGATTATTTCCCAGAGCCTGTCACT GTCAGTTGGAACTCTGGAGCCTTGACTTCTGGTGTTCATACATTTCCTGCTGTCCTTCA AAGCAGCGGCTTGTACTCATTGTCTTCTGTTGTGACAGTACCCTCAAGCAGCCTCGGCA CTCAGACATACATCTGCAATGTCAACCACAAACCCTCAAATACAAAGGTAGATAAAAAA GTCGAACCAAAGTCTTGTGACAAAACTCACACGTGCCCACCGTGCCCAGCACCTGAACT CCTGGGGGGACCGTCAGTCTTCCTCTTCCCCCCAAAACCCAAGGACACCCTCATGATCT CCCGGACCCCTGAGGTCACATGCGTGGTGGTGGACGTGAGCCACGAAGACCCTGAGGTC AAGTTCAACTGGTACGTGGACGGCGTGGAGGTGCATAATGCCAAGACAAAGCCGCGGGA GGAGCAGTACAACAGCACGTACCGTGTGGTCAGCGTCCTCACCGTCCTGCACCAGGACT GGCTGAATGGCAAGGAGTACAAGTGCAAGGTCTCCAACAAAGCCCTCCCAGCCCCCATC GAGAAAACCATCTCCAAAGCCAAAGGGCAGCCCCGAGAACCACAGGTGTACACCCTGCC CCCATGCCGGGAGGAGATGACCAAGAACCAGGTCAGCCTGTGGTGCCTGGTCAAAGGCT TCTATCCCAGCGACATCGCCGTGGAGTGGGAGAGCAATGGGCAGCCGGAGAACAACTAC AAGACCACGCCTCCCGTGCTGGACTCCGACGGCTCCTTCTTCCTCTACAGCAAGCTCAC CGTGGACAAGAGCAGGTGGCAGCAGGGGAACGTCTTCTCATGCTCCGTGATGCATGAGG CTCTGCACAACCACTACACGCAGAAGAGCCTCTCCCTGTCTCCGGGTAAA | 548 |
| EPB11 LC | GACATACAGATGACTCAGAGCCCTGGTACACTGAGTGTATCTCCTGGGGAACGCGCCAC TCTGTCTTGCCGAGCCTCTCAAAGCGTTAGTTCCAACCTGGCTTGGTATCAGCAAAAAC | 540 |
| | CCGGTCAAGCACCTCGCTTGCTTATCTACGGGGCCTCCACACGGGCAACCGGCATTCCT GCACGCTTTAGTGGCTCTGGTAGCGGGACTGAATTCACTTTGACTATATCTTCCATGCA GAGTGAGGATTTCGCCGTTTATTATTGTCAACAGTACAATAACTGGCCTCCACTCACTT TTGGTGGTGGCACCAAAGTGGAGATAAAGCGTACGGTGGCAGCTCCCAGCGTTTTTATC TTTCCCCCAGACATACAACTCACACAAAGTCCAAGCTCTCTTTCCCTTAGTCCAGGAGA ACGCGTAACTCTCTCATGTAAAGCCTCCCAGGATATCAATAACTATCTCTCTTGGTACC AACAAAAGCCTGACCAAGCTCCTAAGCTGCTTATCAAACGCGCCAATCGGTTGGTAGAC GGAGTTCCCGACAGGTTCAGTGGGTCAGGCTCAGGTACAGATTTTACCTTGAAGATATC ACGCGTGGAAGCCGAAGACGTAGGGGTATATTATTGTCTGAAATATGACGAGTTCCCCT ATACTTTTGGGTGTGGAACTCGCCTGGAAATAAAACGTACGGTGGCAGCTCCCAGCGTT TTTATCTTTCCCCCATCCGACGAGCAGCTCAAGAGTGGCACTGCCTCTGTAGTTTGTTT GCTGAATAACTTCTATCCACGTGAAGCAAAAGTACAGTGGAAGGTCGATAATGCCCTTC AGAGCGGTAACAGCCAAGAAAGTGTTACCGAGCAAGATTCCAAAGATTCCACTTACAGT CTGTCCAGCACATTGACACTGAGTAAGGCTGATTACGAAAAACACAAGGTGTACGCATG CGAGGTGACACACCAAGGTCTTTCATCTCCTGTAACTAAGAGCTTTAACCGGGGAGAAT GTGGTGGTGGGGGCAGCGGGGGCGGAGGTAGTGGAGGCGGCGGTAGTGAACCAAAGAGT AGTGACAAAACTCACACGTGCCCACCGTGCCCAGCACCTGAACTCCTGGGGGGACCGTC AGTCTTCCTCTTCCCCCCAAAACCCAAGGACACCCTCATGATCTCCCGGACCCCTGAGG TCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGG AGTACAAGTGCAAGGTCTCCAACAAAGCCCTCCCAGCCCCCATCGAGAAAACCATCTCC AAAGCCAAAGGGCAGCCCCGAGAACCACAGGTGTACACCCTGCCCCCATGCCGGGAGGA GATGACCAAGAACCAGGTCAGCCTGTGGTGCCTGGTCAAAGGCTTCTATCCCAGCGACA TCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAG GTGGCAGCAGGGGAACGTCTTCTCATGCTCCGTGATGCATGAGGCTCTGCACAACCACT ACACGCAGAAGAGCCTCTCCCTGTCTCCGGGTAAA | |
| EPB12 HC | GAGGTCCAGCTGGTTGAGTCTGGAGGTGGTGTAGTCAGGCCAGGAGGTTCCCTGCGTCT GTCTTGCGCTGCCTCTGGGTTCACTGTGTCTGACTACAGTATGAACTGGGTTAGACAGG CTCCAGGGAAAGGTTTGGAATGGATAGGGTTTATTCGCAATAAAGCCAATGCCTATACC ACCGAATACAGCGCAAGCGTTAAAGGCAGGTTCACCATCAGTAGGGACGACTCTAAGAA TACCCTCTACCTGCAGATGAATAGCTTGAAGACAGAGGATACCGCTGTTTATTACTGCA CTACCTACCCCAGGTATCATGCTATGGACAGTTGGGGTCAAGGAACTATGGTCACCGTC TCCTCTGCTAGCACCAAAGGACCTAGTGTTTTTCCTCTTGCCCCTCAAGTGCAACTCCT TGAAAGTGGAGGTGGCCTTGTCCAGCCAGGCGGTTCACTGCGTTTGAGCTGTGCCGCAT CCGGCTTTACCTTTTCCAGTTACACAATGAGTTGGGTCCGTCAAGCACCCGGGCAATGT TTGGAATGGATGGGAACTATTTCATCTGGTGGGACTTACACTTACTATCCCGATAGCGT CAAGGGACGTTTTACCATAAGCCGGGACAACGCTAAAAATTCACTCTATCTTCAGATGA ACAGTCTTAGGGCTGAGGATACCGCTGTTTATTATTGCGCCCGAGAAGCCATTTTCACC | 550 |
| | TACTGGGGGCGCGGTACACTTGTCACCGTTAGCAGTGCTAGCACCAAAGGACCTAGTGT TTTTCCTCTTGCCCCTTCCTCAAAGTCTACCTCTGGGGGGACAGCCGCTCTGGGCTGCC TGGTCAAGGATTATTTCCCAGAGCCTGTCACTGTCAGTTGGAACTCTGGAGCCTTGACT TCTGGTGTTCATACATTTCCTGCTGTCCTTCAAAGCAGCGGCTTGTACTCATTGTCTTC TGTTGTGACAGTACCCTCAAGCAGCCTGGGCACTCAGACATACATCTGCAATGTCAACC ACAAACCCTCAAATACAAAGGTAGATAAAAAAGTCGAACCAAAGTCTTGTGACAAAACT CACACGTGCCCACCGTGCCCAGCACCTGAACTCCTGGGGGGACCGTCAGTCTTCCTCTT CCCCCCAAAACCCAAGGACACCCTCATGATCTCCCGGACCCCTGAGGTCACATGCGTGG TGGTGGACGTGAGCCACGAAGACCCTGAGGTCAAGTTCAACTGGTACGTGGACGGCGTG GAGGTGCATAATGCCAAGACAAAGCCGCGGGAGGAGCAGTACAACAGCACGTACCGTGT GGTCAGCGTCCTCACCGTCCTGCACCAGGACTGGCTGAATGGCAAGGAGTACAAGTGCA AGGTCTCCAACAAAGCCCTCCCAGCCCCCATCGAGAAAACCATCTCCAAAGCCAAAGGG CAGCCCCGAGAACCACAGGTGTACACCCTGCCCCCATGCCGGGAGGAGATGACCAAGAA CCAGGTCAGCCTGTGGTGCCTGGTCAAAGGCTTCTATCCCAGCGACATCGCCGTGGAGT GGGAGAGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAGCAGGG GAACGTCTTCTCATGCTCCGTGATGCATGAGGCTCTGCACAACCACTACACGCAGAAGA GCCTCTCCCTGTCTCCGGGTAAA | |
| EPB12 LC | GCCATACAGCTGACTCAATCACCTTCTTCTTTGAGCGCCTCTGTCGGTGATCGCGTTAC AATTACATGCCGAGCTTCTCAGTCTATCTCTAATAACCTCCACTGGTATCTCCAGAAAC CTGGACAGTCACCACAGCTTCTGATATATTACGGCTTTCAGTCTATAAGTGGAGTCCCA AGTAGATTTTCCGGTAGTGGATCTGGCACCGATTTTACTCTTACTATCTCTAGCCTCCA ACCTGAAGATTTCGCAACCTATTACTGCCAGCAGGCCAATTCCTGGCCCTTGACCTTCG GAGGTGGGACTAAGCTGGAAATCAAACGTACGGTGGCAGCTCCCAGCGTTTTTATCTTT CCCCCAGACATACAACTCACACAAAGTCCAAGCTCTCTTTCCCTTAGTCCAGGAGAACG CGTAACTCTCTCATGTAAAGCCTCCCAGGATATCAATAACTATCTCTCTTGGTACCAAC AAAAGCCTGACCAAGCTCCTAAGCTGCTTATCAAACGCGCCAATCGGTTGGTAGACGGA GTTCCCGACAGGTTCAGTGGGTCAGGCTCAGGTACAGATTTTACCTTGAAGATATCACG CGTGGAAGCCGAAGACGTAGGGGTATATTATTGTCTGAAATATGACGAGTTCCCCTATA CTTTTGGGTGTGGAACTCGCCTGGAAATAAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC | 551 |
| | GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCATGCCGGGAGGAGAT GACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |

Table 52 below shows the polypeptide sequences of the heavy chain variable region (VH) and light chain variable region (VL) of the engineered antibodies targeting EphA2. Table 53 below shows the nucleotide sequences of the heavy chain variable region (VH) and light chain variable region (VL) of the engineered antibodies targeting EphA2.

**[Table 52]**

| **Name** | | **Sequence** | **SEQ ID NO** |
|---|---|---|---|
| EPD01 | VH1 | EVQLLESGGGLVQPGGSLRLSCAASGFTFSRYQMMWVRQAPGKGLEWVSSISPSGGVTLYA DSVKGRFTISRDNSKNTLYLQMNSLRAEDTAVYYCTRELLGTVVVPVAWKMRGYFDYWGQL TLVTVSS | 135 |
| | VH2 | EVQLLESGGGLVQPGGSLRLSCAASGFTFSHYMMAWVRQAPGKCLEWVSRIGPSGGPTHYA DSVKGRFTISRDNSKNTLYLQMNSLRAEDTAVYYCAGYDSGYDYVAVAGPAEYFQHWGQGT LVTVSS | 136 |
| | VL1 | DIQMTQSPGTLSVSPGERATLSCRASQSVSSNLAWYQQKPGQAPRLLIYGASTRATGIPAR FSGSGSGTEFTLTISSMQSEDFAVYYCQQYNNWPPLTFGGGTKVEIK | 137 |
| | VL2 | DIQMTQSPSSLSASVGDRVTITCRASQSISTWLAWYQQKPGKAPKLLIYKASNLHTGVPSR FSGSGSGTEFSLTISGLQPDDFATYYCQQYNSYSRTFGGGTKVEIK | 138 |
| EPB02 | VH1 | EVQLVESGGGVVRPGGSLRLSCAASGFTVSDYSMNWVRQAPGKGLEWIGFIRNKANAYTTE YSASVKGRFTISRDDSKNTLYLQMNSLKTEDTAVYYCTTYPRYHAMDSWGQGTMVTVSS | 139 |
| | VH2 | EVQLLESGGGLVQPGGSLRLSCAASGFTFSHYMMAWVRQAPGKCLEWVSRIGPSGGPTHYA DSVKGRFTISRDNSKNTLYLQMNSLRAEDTAVYYCAGYDSGYDYVAVAGPAEYFQHWGQGT LVTVSS | 136 |
| | VL1 | AIQLTQSPSSLSASVGDRVTITCRASQSISNNLHWYLQKPGQSPQLLIYYGFQSISGVPSR FSGSGSGTDFTLTISSLQPEDFATYYCQQANSWPLTFGGGTKLEIK | 140 |
| | VL2 | DIQMTQSPSSLSASVGDRVTITCRASQSISTWLAWYQQKPGKAPKLLIYKASNLHTGVPSR FSGSGSGTEFSLTISGLQPDDFATYYCQQYNSYSRTFGCGTKVEIK | 138 |
| EP B03 | VH1 | QVQLLESGGGLVQPGGSLRLSCAASGFTFSSYTMSWVRQAPGQALEWMGTISSGGTYTYYP DSVKGRFTISRDNAKNSLYLQMNSLRAEDTAVYYCAREAIFTYWGRGTLVTVSS | 141 |
| | VH2 | EVQLLESGGGLVQPGGSLRLSCAASGFTFSHYMMAWVRQAPGKCLEWVSRIGPSGGPTHYA DSVKGRFTISRDNSKNTLYLQMNSLRAEDTAVYYCAGYDSGYDYVAVAGPAEYFQHWGQGT LVTVSS | 136 |
| | VL1 | DIQLTQSPSSLSLSPGERVTLSCKASQDINNYLSWYQQKPDQAPKLLIKRANRLVDGVPDR FSGSGSGTDFTLKISRVEAEDVGVYYCLKYDEFPYTFGQGTRLEIK | 142 |
| | VL2 | DIQMTQSPSSLSASVGDRVTITCRASQSISTWLAWYQQKPGKAPKLLIYKASNLHTGVPSR FSGSGSGTEFSLTISGLQPDDFATYYCQQYNSYSRTFGCGTKVEIK | 138 |
| EPB04 | VH1 | EVQLLESGGGLVQPGGSLRLSCAASGFTFSHYMMAWVRQAPGKGLEWVSRIGPSGGPTHYA DSVKGRFTISRDNSKNTLYLQMNSLRAEDTAVYYCAGYDSGYDYVAVAGPAEYFQHWGQGT LVTVSS | 143 |
| | VH2 | EVQLLESGGGLVQPGGSLRLSCAASGFTFSRYQMMWVRQAPGKCLEWVSSISPSGGVTLYA DSVKGRFTISRDNSKNTLYLQMNSLRAEDTAVYYCTRELLGTVVVPVAWKMRGYFDYWGQL TLVTVSS | 144 |
| | VL1 | DIQMTQSPSSLSASVGDRVTITCRASQSISTWLAWYQQKPGKAPKLLIYKASNLHTGVPSR FSGSGSGTEFSLTISGLQPDDFATYYCQQYNSYSRTFGQGTKVEIK | 145 |
| | VL2 | DIQMTQSPGTLSVSPGERATLSCRASQSVSSNLAWYQQKPGQAPRLLIYGASTRATGIPAR FSGSGSGTEFTLTISSMQSEDFAVYYCQQYNNWPPLTFGCGTKVEIK | 146 |
| EPB05 | VH1 | EVQLLESGGGLVQPGGSLRLSCAASGFTFSHYMMAWVRQAPGKGLEWVSRIGPSGGPTHYA DSVKGRFTISRDNSKNTLYLQMNSLRAEDTAVYYCAGYDSGYDYVAVAGPAEYFQHWGQGT LVTVSS | 143 |
| | VH2 | EVQLVESGGGVVRPGGSLRLSCAASGFTVSDYSMNWVRQAPGKCLEWIGFIRNKANAYTTE YSASVKGRFTISRDDSKNTLYLQMNSLKTEDTAVYYCTTYPRYHAMDSWGQGTMVTVSS | 147 |
| | VL1 | DIQMTQSPSSLSASVGDRVTITCRASQSISTWLAWYQQKPGKAPKLLIYKASNLHTGVPSR FSGSGSGTEFSLTISGLQPDDFATYYCQQYNSYSRTFGQGTKVEIK | 145 |
| | VL2 | AIQLTQSPSSLSASVGDRVTITCRASQSISNNLHWYLQKPGQSPQLLIYYGFQSISGVPSR FSGSGSGTDFTLTISSLQPEDFATYYCQQANSWPLTFGGGTKLEIK | 148 |
| EPB06 | VH1 | EVQLLESGGGLVQPGGSLRLSCAASGFTFSHYMMAWVRQAPGKGLEWVSRIGPSGGPTHYA DSVKGRFTISRDNSKNTLYLQMNSLRAEDTAVYYCAGYDSGYDYVAVAGPAEYFQHWGQGT LVTVSS | 143 |
| | VH2 | QVQLLESGGGLVQPGGSLRLSCAASGFTFSSYTMSWVRQAPGQCLEWMGTISSGGTYTYYP DSVKGRFTISRDNAKNSLYLQMNSLRAEDTAVYYCAREAIFTYWGRGTLVTVSS | 140 |
| | VL1 | DIQMTQSPSSLSASVGDRVTITCRASQSISTWLAWYQQKPGKAPKLLIYKASNLHTGVPSR FSGSGSGTEFSLTISGLQPDDFATYYCQQYNSYSRTFGQGTKVEIK | 146 |
| | VL2 | DIQLTQSPSSLSLSPGERVTLSCKASQDINNYLSWYQQKPDQAPKLLIKRANRLVDGVPDR FSGSGSGTDFTLKISRVEAEDVGVYYCLKYDEFPYTFGGGTKLEIK | 150 |
| EPB07 | VH1 | EVQLVESGGGVVRPGGSLRLSCAASGFTVSDYSMNWVRQAPGKGLEWIGFIRNKANAYTTE YSASVKGRFTISRDDSKNTLYLQMNSLKTEDTAVYYCTTYPRYHAMDSWGQGTMVTVSS | 139 |
| | VH2 | EVQLLESGGGLVQPGGSLRLSCAASGFTFSRYQMMWVRQAPGKCLEWVSSISPSGGVTLYA DSVKGRFTISRDNSKNTLYLQMNSLRAEDTAVYYCTRELLGTVVVPVAWKMRGYFDYWGQL TLVTVSS | 144 |
| | VL1 | AIQLTQSPSSLSASVGDRVTITCRASQSISNNLHWYLQKPGQSPQLLIYYGFQSISGVPSR FSGSGSGTDFTLTISSLQPEDFATYYCQQANSWPLTFGGGTKLEIK | 140 |
| | VL2 | DIQMTQSPGTLSVSPGERATLSCRASQSVSSNLAWYQQKPGQAPRLLIYGASTRATGIPAR FSGSGSGTEFTLTISSMQSEDFAVYYCQQYNNWPPLTFGCGTKVEIK | 146 |
| EPB08 | VH1 | QVQLLESGGGLVQPGGSLRLSCAASGFTFSSYTMSWVRQAPGQALEWMGTISSGGTYTYYP DSVKGRFTISRDNAKNSLYLQMNSLRAEDTAVYYCAREAIFTYWGRGTLVTVSS | 141 |
| | VH2 | EVQLLESGGGLVQPGGSLRLSCAASGFTFSRYQMMWVRQAPGKCLEWVSSISPSGGVTLYA DSVKGRFTISRDNSKNTLYLQMNSLRAEDTAVYYCTRELLGTVVVPVAWKMRGYFDYWGQL TLVTVSS | 144 |
| | VL1 | DIQLTQSPSSLSLSPGERVTLSCKASQDINNYLSWYQQKPDQAPKLLIKRANRLVDGVPDR FSGSGSGTDFTLKISRVEAEDVGVYYCLKYDEFPYTFGQGTRLEIK | 142 |
| | VL2 | DIQMTQSPGTLSVSPGERATLSCRASQSVSSNLAWYQQKPGQAPRLLIYGASTRATGIPAR FSGSGSGTEFTLTISSMQSEDFAVYYCQQYNNWPPLTFGCGTKVEIK | 146 |
| EPB09 | VH1 | EVQLLESGGGLVQPGGSLRLSCAASGFTFSRYQMMWVRQAPGKGLEWVSSISPSGGVTLYA DSVKGRFTISRDNSKNTLYLQMNSLRAEDTAVYYCTRELLGTVVVPVAWKMRGYFDYWGQL TLVTVSS | 135 |
| | VH2 | EVQLVESGGGVVRPGGSLRLSCAASGFTVSDYSMNWVRQAPGKCLEWIGFIRNKANAYTTE YSASVKGRFTISRDDSKNTLYLQMNSLKTEDTAVYYCTTYPRYHAMDSWGQGTMVTVSS | 14 7 |
| | VL1 | DIQMTQSPGTLSVSPGERATLSCRASQSVSSNLAWYQQKPGQAPRLLIYGASTRATGIPAR FSGSGSGTEFTLTISSMQSEDFAVYYCQQYNNWPPLTFGGGTKVEIK | 137 |
| | VL2 | AIQLTQSPSSLSASVGDRVTITCRASQSISNNLHWYLQKPGQSPQLLIYYGFQSISGVPSR FSGSGSGTDFTLTISSLQPEDFATYYCQQANSWPLTFGGGTKLEIK | 148 |
| EPB10 | VH1 | QVQLLESGGGLVQPGGSLRLSCAASGFTFSSYTMSWVRQAPGQALEWMGTISSGGTYTYYP DSVKGRFTISRDNAKNSLYLQMNSLRAEDTAVYYCAREAIFTYWGRGTLVTVSS | 141 |
| | VH2 | EVQLVESGGGVVRPGGSLRLSCAASGFTVSDYSMNWVRQAPGKCLEWIGFIRNKANAYTTE YSASVKGRFTISRDDSKNTLYLQMNSLKTEDTAVYYCTTYPRYHAMDSWGQGTMVTVSS | 147 |
| | VL1 | DIQLTQSPSSLSLSPGERVTLSCKASQDINNYLSWYQQKPDQAPKLLIKRANRLVDGVPDR FSGSGSGTDFTLKISRVEAEDVGVYYCLKYDEFPYTFGQGTRLEIK | 142 |
| | VL2 | AIQLTQSPSSLSASVGDRVTITCRASQSISNNLHWYLQKPGQSPQLLIYYGFQSISGVPSR FSGSGSGTDFTLTISSLQPEDFATYYCQQANSWPLTFGCGTKLEIK | 148 |
| EPB11 | VH1 | EVQLLESGGGLVQPGGSLRLSCAASGFTFSRYQMMWVRQAPGKGLEWVSSISPSGGVTLYA DSVKGRFTISRDNSKNTLYLQMNSLRAEDTAVYYCTRELLGTVVVPVAWKMRGYFDYWGQL TLVTVSS | 135 |
| | VH2 | QVQLLESGGGLVQPGGSLRLSCAASGFTFSSYTMSWVRQAPGQCLEWMGTISSGGTYTYYP DSVKGRFTISRDNAKNSLYLQMNSLRAEDTAVYYCAREAIFTYWGRGTLVTVSS [?] | 149 |
| | VL1 | DIQMTQSPGTLSVSPGERATLSCRASQSVSSNLAWYQQKPGQAPRLLIYGASTRATGIPAR FSGSGSGTEFTLTISSMQSEDFAVYYCQQYNNWPPLTFGGGTKVEIK | 137 |
| | VL2 | DIQLTQSPSSLSLSPGERVTLSCKASQDINNYLSWYQQKPDQAPKLLIKRANRLVDGVPDR FSGSGSGTDFTLKISRVEAEDVGVYYCLKYDEFPYTFGCGTRLEIK | 150 |
| EPB12 | VH1 | EVQLVESGGGVVRPGGSLRLSCAASGFTVSDYSMNWVRQAPGKGLEWIGFIRNKANAYTTE YSASVKGRFTISRDDSKNTLYLQMNSLKTEDTAVYYCTTYPRYHAMDSWGQGTMVTVSS | 139 |
| | VH2 | QVQLLESGGGLVQPGGSLRLSCAASGFTFSSYTMSWVRQAPGQCLEWMGTISSGGTYTYYP DSVKGRFTISRDNAKNSLYLQMNSLRAEDTAVYYCAREAIFTYWGRGTLVTVSS | 149 |
| | VL1 | AIQLTQSPSSLSASVGDRVTITCRASQSISNNLHWYLQKPGQSPQLLIYYGFQSISGVPSR FSGSGSGTDFTLTISSLQPEDFATYYCQQANSWPLTFGGGTKLEIK | 140 |
| | VL2 | DIQLTQSPSSLSLSPGERVTLSCKASQDINNYLSWYQQKPDQAPKLLIKRANRLVDGVPDR FSGSGSGTDFTLKISRVEAEDVGVYYCLKYDEFPYTFGCGTRLEIK | 150 |

**[Table 53]**

| **Name** | | **Sequence** | **SEQ ID NO** |
|---|---|---|---|
| EPB01 | VH 1 | GAGGTGCAACTGCTTGAGTCAGGAGGAGGACTGGTTCAACCCGGCGGGAGCCTTCGACTCT CATGTGCAGCCTCTGGGTTTACATTTAGTCGTTACCAAATGATGTGGGTACGCCAGGCCCC CGGAAAAGGTTTGGAGTGGGTGAGCAGCATTTCTCCTTCAGGAGGAGTGACACTTTACGCA GACAGTGTTAAGGGTCGGTTCACAATCAGTCGTGATAATTCCAAGAACACTCTCTATCTCC AAATGAACTCCCTTCGAGCTGAGGACACTGCTGTTTATTACTGTACTCGGGAACTTCTCGG CACTGTGGTTGTTCCTGTGGCTTGGAAGATGCGTGGATACTTCGACTATTGGGGGCAACTC ACTCTTGTTACCGTCTCAAGC | 552 |
| | VH2 | GAGGTTCAACTCCTTGAATCCGGAGGAGGACTTGTCCAACCAGGCGGCAGCCTTAGGCTGT CCTGCGCTGCCTCAGGCTTTACATTCAGTCATTACATGATGGCCTGGGTTAGACAGGCACC CGGCAAATGTCTGGAATGGGTCAGCCGAATAGGACCATCAGGAGGTCCCACTCACTATGCC GATTCTGTAAAAGGGAGGTTTACAATTTCCAGAGACAATTCAAAGAATACCCTTTACCTCC AGATGAACTCATTGAGAGCCGAGGACACAGCCGTATATTATTGCGCAGGTTATGATTCCGG TTACGATTACGTTGCAGTCGCAGGCCCAGCCGAATACTTCCAACATTGGGGTCAGGGAACC CTCGTGACCGTGTCCAGT | 553 |
| | VL1 | GACATACAGATGACTCAGAGCCCTGGTACACTGAGTGTATCTCCTGGGGAACGCGCCACTC TGTCTTGCCGAGCCTCTCAAAGCGTTAGTTCCAACCTGGCTTGGTATCAGCAAAAACCCGG TCAAGCACCTCGCTTGCTTATCTACGGGGCCTCCACACGGGCAACCGGCATTCCTGCACGC TTTAGTGGCTCTGGTAGCGGGACTGAATTCACTTTGACTATATCTTCCATGCAGAGTGAGG ATTTCGCCGTTTATTATTGTCAACAGTACAATAACTGGCCTCCACTCACTTTTGGTGGTGG CACCAAAGTGGAGATAAAG | 554 |
| | VL2 | GACATACAGATGACACAAAGTCCAAGTAGCCTTTCTGCATCCGTTGGAGACCGCGTAACCA TAACTTGTCGAGCATCACAAAGCATAAGCACTTGGCTTGCTTGGTATCAGCAGAAGCCAGG CAAAGCACCCAAGCTGCTGATTTATAAAGCCTCTAATCTCCATACAGGAGTTCCCAGCCGT TTCTCAGGCAGCGGGTCTGGAACCGAATTCTCTCTGACCATCTCTGGCTTGCAACCTGATG ACTTTGCAACATATTACTGTCAACAATATAATTCATATAGTCGTACTTTCGGGTGTGGCAC TAAGGTGGAAATCAAA | 555 |
| EPB02 | VH1 | GAGGTCCAGCTGGTTGAGTCTGGAGGTGGTGTAGTCAGGCCAGGAGGTTCCCTGCGTCTGT CTTGCGCTGCCTCTGGGTTCACTGTGTCTGACTACAGTATGAACTGGGTTAGACAGGCTCC AGGGAAAGGTTTGGAATGGATAGGGTTTATTCGCAATAAAGCCAATGCCTATACCACCGAA TACAGCGCAAGCGTTAAAGGCAGGTTCACCATCAGTAGGGACGACTCTAAGAATACCCTCT ACCTGCAGATGAATAGCTTGAAGACAGAGGATACCGCTGTTTATTACTGCACTACCTACCC CAGGTATCATGCTATGGACAGTTGGGGTCAAGGAACTATGGTCACCGTCTCCTCT | 556 |
| | VH2 | GAGGTTCAACTCCTTGAATCCGGAGGAGGACTTGTCCAACCAGGCGGCAGCCTTAGGCTGT CCTGCGCTGCCTCAGGCTTTACATTCAGTCATTACATGATGGCCTGGGTTAGACAGGCACC CGGCAAATGTCTGGAATGGGTCAGCCGAATAGGACCATCAGGAGGTCCCACTCACTATGCC GATTCTGTAAAAGGGAGGTTTACAATTTCCAGAGACAATTCAAAGAATACCCTTTACCTCC AGATGAACTCATTGAGAGCCGAGGACACAGCCGTATATTATTGCGCAGGTTATGATTCCGG TTACGATTACGTTGCAGTCGCAGGCCCAGCCGAATACTTCCAACATTGGGGTCAGGGAACC | 553 |
| | | CTCGTGACCGTGTCCAGT | |
| | VL1 | GCCATACAGCTGACTCAATCACCTTCTTCTTTGAGCGCCTCTGTCGGTGATCGCGTTACAA TTACATGCCGAGCTTCTCAGTCTATCTCTAATAACCTCCACTGGTATCTCCAGAAACCTGG ACAGTCACCACAGCTTCTGATATATTACGGCTTTCAGTCTATAAGTGGAGTCCCAAGTAGA TTTTCCGGTAGTGGATCTGGCACCGATTTTACTCTTACTATCTCTAGCCTCCAACCTGAAG ATTTCGCAACCTATTACTGCCAGCAGGCCAATTCCTGGCCCTTGACCTTCGGAGGTGGGAC TAAGCTGGAAATCAAA | 557 |
| | VL2 | GACATACAGATGACACAAAGTCCAAGTAGCCTTTCTGCATCCGTTGGAGACCGCGTAACCA TAACTTGTCGAGCATCACAAAGCATAAGCACTTGGCTTGCTTGGTATCAGCAGAAGCCAGG CAAAGCACCCAAGCTGCTGATTTATAAAGCCTCTAATCTCCATACAGGAGTTCCCAGCCGT TTCTCAGGCAGCGGGTCTGGAACCGAATTCTCTCTGACCATCTCTGGCTTGCAACCTGATG ACTTTGCAACATATTACTGTCAACAATATAATTCATATAGTCGTACTTTCGGGTGTGGCAC TAAGGTGGAAATCAAA | 555 |
| EPB03 | VH1 | CAAGTGCAACTCCTTGAAAGTGGAGGTGGCCTTGTCCAGCCAGGCGGTTCACTGCGTTTGA GCTGTGCCGCATCCGGCTTTACCTTTTCCAGTTACACAATGAGTTGGGTCCGTCAAGCACC CGGGCAAGCTTTGGAATGGATGGGAACTATTTCATCTGGTGGGACTTACACTTACTATCCC GATAGCGTCAAGGGACGTTTTACCATAAGCCGGGACAACGCTAAAAATTCACTCTATCTTC AGATGAACAGTCTTAGGGCTGAGGATACCGCTGTTTATTATTGCGCCCGAGAAGCCATTTT CACCTACTGGGGGCGCGGTACACTTGTCACCGTTAGCAGT | 558 |
| | VH2 | GAGGTTCAACTCCTTGAATCCGGAGGAGGACTTGTCCAACCAGGCGGCAGCCTTAGGCTGT CCTGCGCTGCCTCAGGCTTTACATTCAGTCATTACATGATGGCCTGGGTTAGACAGGCACC CGGCAAATGTCTGGAATGGGTCAGCCGAATAGGACCATCAGGAGGTCCCACTCACTATGCC GATTCTGTAAAAGGGAGGTTTACAATTTCCAGAGACAATTCAAAGAATACCCTTTACCTCC AGATGAACTCATTGAGAGCCGAGGACACAGCCGTATATTATTGCGCAGGTTATGATTCCGG TTACGATTACGTTGCAGTCGCAGGCCCAGCCGAATACTTCCAACATTGGGGTCAGGGAACC CTCGTGACCGTGTCCAGT | 553 |
| | VL1 | GACATACAACTCACACAAAGTCCAAGCTCTCTTTCCCTTAGTCCAGGAGAACGCGTAACTC TCTCATGTAAAGCCTCCCAGGATATCAATAACTATCTCTCTTGGTACCAACAAAAGCCTGA CCAAGCTCCTAAGCTGCTTATCAAACGCGCCAATCGGTTGGTAGACGGAGTTCCCGACAGG TTCAGTGGGTCAGGCTCAGGTACAGATTTTACCTTGAAGATATCACGCGTGGAAGCCGAAG ACGTAGGGGTATATTATTGTCTGAAATATGACGAGTTCCCCTATACTTTTGGGCAAGGAAC TCGCCTGGAAATAAAA | 559 |
| | VL | GACATACAGATGACACAAAGTCCAAGTAGCCTTTCTGCATCCGTTGGAGACCGCGTAACCA TAACTTGTCGAGCATCACAAAGCATAAGCACTTGGCTTGCTTGGTATCAGCAGAAGCCAGG CAAAGCACCCAAGCTGCTGATTTATAAAGCCTCTAATCTCCATACAGGAGTTCCCAGCCGT TTCTCAGGCAGCGGGTCTGGAACCGAATTCTCTCTGACCATCTCTGGCTTGCAACCTGATG ACTTTGCAACATATTACTGTCAACAATATAATTCATATAGTCGTACTTTCGGGTGTGGCAC TAAGGTGGAAATCAAA | 555 |
| EPB04 | VH1 | GAGGTACAGTTGCTGGAGTCAGGAGGTGGATTGGTCCAACCCGGAGGATCTCTTCGTCTGT CCTGCGCCGCCTCAGGATTTACCTTCTCTCATTATATGATGGCATGGGTACGTCAGGCTCC | 450 |
| | | AGGCAAAGGTCTGGAATGGGTTAGTCGGATTGGTCCCTCAGGGGGTCCTACCCATTATGCC GATTCTGTAAAGGGCCGTTTTACCATAAGCAGAGACAACTCTAAGAACACCCTTTACCTTC AGATGAATAGCCTGAGGGCTGAGGATACCGCAGTGTATTACTGCGCAGGCTATGACTCTGG GTACGACTATGTCGCCGTAGCAGGACCTGCCGAGTATTTTCAACACTGGGGACAGGGGACC CTTGTCACAGTTTCTAGT | |
| | VH2 | GAGGTGCAACTGCTTGAGTCAGGAGGAGGACTGGTTCAACCCGGCGGGAGCCTTCGACTCT CATGTGCAGCCTCTGGGTTTACATTTAGTCGTTACCAAATGATGTGGGTACGCCAGGCCCC CGGAAAATGTTTGGAGTGGGTGAGCAGCATTTCTCCTTCAGGAGGAGTGACACTTTACGCA GACAGTGTTAAGGGTCGGTTCACAATCAGTCGTGATAATTCCAAGAACACTCTCTATCTCC AAATGAACTCCCTTCGAGCTGAGGACACTGCTGTTTATTACTGTACTCGGGAACTTCTCGG CACTGTGGTTGTTCCTGTGGCTTGGAAGATGCGTGGATACTTCGACTATTGGGGGCAACTC ACTCTTGTTACCGTCTCAAGC | 560 |
| | VL1 | GATATTCAAATGACACAAAGCCCAAGTTCCTTGTCCGCCTCAGTTGGTGATCGTGTGACAA TAACCTGTCGGGCTTCACAATCCATATCTACATGGCTGGCTTGGTACCAGCAAAAGCCAGG TAAAGCCCCAAAACTCCTGATTTACAAGGCAAGTAACTTGCATACTGGGGTACCCAGCCGT TTCTCTGGGTCAGGCTCTGGGACAGAGTTTAGTCTTACAATTTCTGGTCTGCAACCCGATG ACTTCGCTACCTATTACTGTCAACAATATAATAGTTATTCTCGAACATTTGGTCAGGGAAC AAAAGTGGAAATCAAA | 451 |
| | VL2 | GACATACAGATGACTCAGAGCCCTGGTACACTGAGTGTATCTCCTGGGGAACGCGCCACTC TGTCTTGCCGAGCCTCTCAAAGCGTTAGTTCCAACCTGGCTTGGTATCAGCAAAAACCCGG TCAAGCACCTCGCTTGCTTATCTACGGGGCCTCCACACGGGCAACCGGCATTCCTGCACGC TTTAGTGGCTCTGGTAGCGGGACTGAATTCACTTTGACTATATCTTCCATGCAGAGTGAGG ATTTCGCCGTTTATTATTGTCAACAGTACAATAACTGGCCTCCACTCACTTTTGGTTGTGG CACCAAAGTGGAGATAAAG | 561 |
| EPB05 | VH1 | GAGGTACAGTTGCTGGAGTCAGGAGGTGGATTGGTCCAACCCGGAGGATCTCTTCGTCTGT CCTGCGCCGCCTCAGGATTTACCTTCTCTCATTATATGATGGCATGGGTACGTCAGGCTCC AGGCAAAGGTCTGGAATGGGTTAGTCGGATTGGTCCCTCAGGGGGTCCTACCCATTATGCC GATTCTGTAAAGGGCCGTTTTACCATAAGCAGAGACAACTCTAAGAACACCCTTTACCTTC AGATGAATAGCCTGAGGGCTGAGGATACCGCAGTGTATTACTGCGCAGGCTATGACTCTGG GTACGACTATGTCGCCGTAGCAGGACCTGCCGAGTATTTTCAACACTGGGGACAGGGGACC CTTGTCACAGTTTCTAGT | 450 |
| | VH2 | GAGGTCCAGCTGGTTGAGTCTGGAGGTGGTGTAGTCAGGCCAGGAGGTTCCCTGCGTCTGT CTTGCGCTGCCTCTGGGTTCACTGTGTCTGACTACAGTATGAACTGGGTTAGACAGGCTCC AGGGAAATGTTTGGAATGGATAGGGTTTATTCGCAATAAAGCCAATGCCTATACCACCGAA TACAGCGCAAGCGTTAAAGGCAGGTTCACCATCAGTAGGGACGACTCTAAGAATACCCTCT ACCTGCAGATGAATAGCTTGAAGACAGAGGATACCGCTGTTTATTACTGCACTACCTACCC CAGGTATCATGCTATGGACAGTTGGGGTCAAGGAACTATGGTCACCGTCTCCTCT | 562 |
| | VL1 | GATATTCAAATGACACAAAGCCCAAGTTCCTTGTCCGCCTCAGTTGGTGATCGTGTGACAA TAACCTGTCGGGCTTCACAATCCATATCTACATGGCTGGCTTGGTACCAGCAAAAGCCAGG TAAAGCCCCAAAACTCCTGATTTACAAGGCAAGTAACTTGCATACTGGGGTACCCAGCCGT | 451 |
| | | TTCTCTGGGTCAGGCTCTGGGACAGAGTTTAGTCTTACAATTTCTGGTCTGCAACCCGATG ACTTCGCTACCTATTACTGTCAACAATATAATAGTTATTCTCGAACATTTGGTCAGGGAAC AAAAGTGGAAATCAAA | |
| | VL2 | GCCATACAGCTGACTCAATCACCTTCTTCTTTGAGCGCCTCTGTCGGTGATCGCGTTACAA TTACATGCCGAGCTTCTCAGTCTATCTCTAATAACCTCCACTGGTATCTCCAGAAACCTGG ACAGTCACCACAGCTTCTGATATATTACGGCTTTCAGTCTATAAGTGGAGTCCCAAGTAGA TTTTCCGGTAGTGGATCTGGCACCGATTTTACTCTTACTATCTCTAGCCTCCAACCTGAAG ATTTCGCAACCTATTACTGCCAGCAGGCCAATTCCTGGCCCTTGACCTTCGGATGTGGGAC TAAGCTGGAAATCAAA | 563 |
| EPB06 | VH1 | GAGGTACAGTTGCTGGAGTCAGGAGGTGGATTGGTCCAACCCGGAGGATCTCTTCGTCTGT CCTGCGCCGCCTCAGGATTTACCTTCTCTCATTATATGATGGCATGGGTACGTCAGGCTCC AGGCAAAGGTCTGGAATGGGTTAGTCGGATTGGTCCCTCAGGGGGTCCTACCCATTATGCC GATTCTGTAAAGGGCCGTTTTACCATAAGCAGAGACAACTCTAAGAACACCCTTTACCTTC AGATGAATAGCCTGAGGGCTGAGGATACCGCAGTGTATTACTGCGCAGGCTATGACTCTGG GTACGACTATGTCGCCGTAGCAGGACCTGCCGAGTATTTTCAACACTGGGGACAGGGGACC CTTGTCACAGTTTCTAGT | 450 |
| | VH2 | CAAGTGCAACTCCTTGAAAGTGGAGGTGGCCTTGTCCAGCCAGGCGGTTCACTGCGTTTGA GCTGTGCCGCATCCGGCTTTACCTTTTCCAGTTACACAATGAGTTGGGTCCGTCAAGCACC CGGGCAATGTTTGGAATGGATGGGAACTATTTCATCTGGTGGGACTTACACTTACTATCCC GATAGCGTCAAGGGACGTTTTACCATAAGCCGGGACAACGCTAAAAATTCACTCTATCTTC AGATGAACAGTCTTAGGGCTGAGGATACCGCTGTTTATTATTGCGCCCGAGAAGCCATTTT CACCTACTGGGGGCGCGGTACACTTGTCACCGTTAGCAGT | 564 |
| | VL1 | GATATTCAAATGACACAAAGCCCAAGTTCCTTGTCCGCCTCAGTTGGTGATCGTGTGACAA TAACCTGTCGGGCTTCACAATCCATATCTACATGGCTGGCTTGGTACCAGCAAAAGCCAGG TAAAGCCCCAAAACTCCTGATTTACAAGGCAAGTAACTTGCATACTGGGGTACCCAGCCGT TTCTCTGGGTCAGGCTCTGGGACAGAGTTTAGTCTTACAATTTCTGGTCTGCAACCCGATG ACTTCGCTACCTATTACTGTCAACAATATAATAGTTATTCTCGAACATTTGGTCAGGGAAC AAAAGTGGAAATCAAA | 451 |
| | VL2 | GACATACAACTCACACAAAGTCCAAGCTCTCTTTCCCTTAGTCCAGGAGAACGCGTAACTC TCTCATGTAAAGCCTCCCAGGATATCAATAACTATCTCTCTTGGTACCAACAAAAGCCTGA CCAAGCTCCTAAGCTGCTTATCAAACGGCCAATCGGTTGGTAGACGGAGTTCCCGACAGG TTCAGTGGGTCAGGCTCAGGTACAGATTTTACCTTGAAGATATCACGCGTGGAAGCCGAAG ACGTAGGGGTATATTATTGTCTGAAATATGACGAGTTCCCCTATACTTTTGGGTGTGGAAC TCGCCTGGAAATAAAA | 565 |
| EP-B07 | VH1 | GAGGTCCAGCTGGTTGAGTCTGGAGGTGGTGTAGTCAGGCCAGGAGGTTCCCTGCGTCTGT CTTGCGCTGCCTCTGGGTTCACTGTGTCTGACTACAGTATGAACTGGGTTAGACAGGCTCC AGGGAAAGGTTTGGAATGGATAGGGTTTATTCGCAATAAAGCCAATGCCTATACCACCGAA TACAGCGCAAGCGTTAAAGGCAGGTTCACCATCAGTAGGGACGACTCTAAGAATACCCTCT ACCTGCAGATGAATAGCTTGAAGACAGAGGATACCGCTGTTTATTACTGCACTACCTACCC CAGGTATCATGCTATGGACAGTTGGGGTCAAGGAACTATGGTCACCGTCTCCTCT | 556 |
| | VH2 | GAGGTGCAACTGCTTGAGTCAGGAGGAGGACTGGTTCAACCCGGCGGGAGCCTTCGACTCT CATGTGCAGCCTCTGGGTTTACATTTAGTCGTTACCAAATGATGTGGGTACGCCAGGCCCC CGGAAAATGTTTGGAGTGGGTGAGCAGCATTTCTCCTTCAGGAGGAGTGACACTTTACGCA GACAGTGTTAAGGGTCGGTTCACAATCAGTCGTGATAATTCCAAGAACACTCTCTATCTCC AAATGAACTCCCTTCGAGCTGAGGACACTGCTGTTTATTACTGTACTCGGGAACTTCTCGG CACTGTGGTTGTTCCTGTGGCTTGGAAGATGCGTGGATACTTCGACTATTGGGGGCAACTC ACTCTTGTTACCGTCTCAAGC | 560 |
| | VL1 | GCCATACAGCTGACTCAATCACCTTCTTCTTTGAGCGCCTCTGTCGGTGATCGCGTTACAA TTACATGCCGAGCTTCTCAGTCTATCTCTAATAACCTCCACTGGTATCTCCAGAAACCTGG ACAGTCACCACAGCTTCTGATATATTACGGCTTTCAGTCTATAAGTGGAGTCCCAAGTAGA TTTTCCGGTAGTGGATCTGGCACCGATTTTACTCTTACTATCTCTAGCCTCCAACCTGAAG ATTTCGCAACCTATTACTGCCAGCAGGCCAATTCCTGGCCCTTGACCTTCGGAGGTGGGAC TAAGCTGGAAATCAAA | 557 |
| | VL2 | GACATACAGATGACTCAGAGCCCTGGTACACTGAGTGTATCTCCTGGGGAACGCGCCACTC TGTCTTGCCGAGCCTCTCAAAGCGTTAGTTCCAACCTGGCTTGGTATCAGCAAAAACCCGG TCAAGCACCTCGCTTGCTTATCTACGGGGCCTCCACACGGGCAACCGGCATTCCTGCACGC TTTAGTGGCTCTGGTAGCGGGACTGAATTCACTTTGACTATATCTTCCATGCAGAGTGAGG ATTTCGCCGTTTATTATTGTCAACAGTACAATAACTGGCCTCCACTCACTTTTGGTTGTGG CACCAAAGTGGAGATAAAG | 561 |
| EFB08 | VH1 | CAAGTGCAACTCCTTGAAAGTGGAGGTGGCCTTGTCCAGCCAGGCGGTTCACTGCGTTTGA GCTGTGCCGCATCCGGCTTTACCTTTTCCAGTTACACAATGAGTTGGGTCCGTCAAGCACC CGGGCAAGCTTTGGAATGGATGGGAACTATTTCATCTGGTGGGACTTACACTTACTATCCC GATAGCGTCAAGGGACGTTTTACCATAAGCCGGGACAACGCTAAAAATTCACTCTATCTTC AGATGAACAGTCTTAGGGCTGAGGATACCGCTGTTTATTATTGCGCCCGAGAAGCCATTTT CACCTACTGGGGGCGCGGTACACTTGTCACCGTTAGCAGT | 558 |
| | VH2 | GAGGTGCAACTGCTTGAGTCAGGAGGAGGACTGGTTCAACCCGGCGGGAGCCTTCGACTCT CATGTGCAGCCTCTGGGTTTACATTTAGTCGTTACCAAATGATGTGGGTACGCCAGGCCCC CGGAAAATGTTTGGAGTGGGTGAGCAGCATTTCTCCTTCAGGAGGAGTGACACTTTACGCA GACAGTGTTAAGGGTCGGTTCACAATCAGTCGTGATAATTCCAAGAACACTCTCTATCTCC AAATGAACTCCCTTCGAGCTGAGGACACTGCTGTTTATTACTGTACTCGGGAACTTCTCGG CACTGTGGTTGTTCCTGTGGCTTGGAAGATGCGTGGATACTTCGACTATTGGGGGCAACTC ACTCTTGTTACCGTCTCAAGC | 560 |
| | VL1 | GACATACAACTCACACAAAGTCCAAGCTCTCTTTCCCTTAGTCCAGGAGAACGCGTAACTC TCTCATGTAAAGCCTCCCAGGATATCAATAACTATCTCTCTTGGTACCAACAAAAGCCTGA CCAAGCTCCTAAGCTGCTTATCAAACGCGCCAATCGGTTGGTAGACGGAGTTCCCGACAGG TTCAGTGGGTCAGGCTCAGGTACAGATTTTACCTTGAAGATATCACGCGTGGAAGCCGAAG ACGTAGGGGTATATTATTGTCTGAAATATGACGAGTTCCCCTATACTTTTGGGCAAGGAAC TCGCCTGGAAATAAAA | 559 |
| | VL2 | GACATACAGATGACTCAGAGCCCTGGTACACTGAGTGTATCTCCTGGGGAACGCGCCACTC TGTCTTGCCGAGCCTCTCAAAGCGTTAGTTCCAACCTGGCTTGGTATCAGCAAAAACCCGG | 561 |
| | | TCAAGCACCTCGCTTGCTTATCTACGGGGCCTCCACACGGGCAACCGGCATTCCTGCACGC TTTAGTGGCTCTGGTAGCGGGACTGAATTCACTTTGACTATATCTTCCATGCAGAGTGAGG ATTTCGCCGTTTATTATTGTCAACAGTACAATAACTGGCCTCCACTCACTTTTGGTTGTGG CACCAAAGTGGAGATAAAG | |
| EPB09 | VH1 | GAGGTGCAACTGCTTGAGTCAGGAGGAGGACTGGTTCAACCCGGCGGGAGCCTTCGACTCT CATGTGCAGCCTCTGGGTTTACATTTAGTCGTTACCAAATGATGTGGGTACGCCAGGCCCC CGGAAAAGGTTTGGAGTGGGTGAGCAGCATTTCTCCTTCAGGAGGAGTGACACTTTACGCA GACAGTGTTAAGGGTCGGTTCACAATCAGTCGTGATAATTCCAAGAACACTCTCTATCTCC 5 AAATGAACTCCCTTCGAGCTGAGGACACTGCTGTTTATTACTGTACTCGGGAACTTCTCGG CACTGTGGTTGTTCCTGTGGCTTGGAAGATGCGTGGATACTTCGACTATTGGGGGCAACTC ACTCTTGTTACCGTCTCAAGC | 552 |
| | VH2 | GAGGTCCAGCTGGTTGAGTCTGGAGGTGGTGTAGTCAGGCCAGGAGGTTCCCTGCGTCTGT CTTGCGCTGCCTCTGGGTTCACTGTGTCTGACTACAGTATGAACTGGGTTAGACAGGCTCC AGGGAAATGTTTGGAATGGATAGGGTTTATTCGCAATAAAGCCAATGCCTATACCACCGAA TACAGCGCAAGCGTTAAAGGCAGGTTCACCATCAGTAGGGACGACTCTAAGAATACCCTCT ACCTGCAGATGAATAGCTTGAAGACAGAGGATACCGCTGTTTATTACTGCACTACCTACCC CAGGTATCATGCTATGGACAGTTGGGGTCAAGGAACTATGGTCACCGTCTCCTCT | 562 |
| | VL1 | GACATACAGATGACTCAGAGCCCTGGTACACTGAGTGTATCTCCTGGGGAACGCGCCACTC TGTCTTGCCGAGCCTCTCAAAGCGTTAGTTCCAACCTGGCTTGGTATCAGCAAAAACCCGG TCAAGCACCTCGCTTGCTTATCTACGGGGCCTCCACACGGGCAACCGGCATTCCTGCACGC TTTAGTGGCTCTGGTAGCGGGACTGAATTCACTTTGACTATATCTTCCATGCAGAGTGAGG ATTTCGCCGTTTATTATTGTCAACAGTACAATAACTGGCCTCCACTCACTTTTGGTGGTGG CACCAAAGTGGAGATAAAG | 554 |
| | VL2 | GCCATACAGCTGACTCAATCACCTTCTTCTTTGAGCGCCTCTGTCGGTGATCGCGTTACAA TTACATGCCGAGCTTCTCAGTCTATCTCTAATAACCTCCACTGGTATCTCCAGAAACCTGG ACAGTCACCACAGCTTCTGATATATTACGGCTTTCAGTCTATAAGTGGAGTCCCAAGTAGA TTTTCCGGTAGTGGATCTGGCACCGATTTTACTCTTACTATCTCTAGCCTCCAACCTGAAG ATTTCGCAACCTATTACTGCCAGCAGGCCAATTCCTGGCCCTTGACCTTCGGATGTGGGAC TAAGCTGGAAATCAAA | 563 |
| EPB10 | VH1 | CAAGTGCAACTCCTTGAAAGTGGAGGTGGCCTTGTCCAGCCAGGCGGTTCACTGCGTTTGA GCTGTGCCGCATCCGGCTTTACCTTTTCCAGTTACACAATGAGTTGGGTCCGTCAAGCACC CGGGCAAGCTTTGGAATGGATGGGAACTATTTCATCTGGTGGGACTTACACTTACTATCCC GATAGCGTCAAGGGACGTTTTACCATAAGCCGGGACAACGCTAAAAATTCACTCTATCTTC AGATGAACAGTCTTAGGGCTGAGGATACCGCTGTTTATTATTGCGCCCGAGAAGCCATTTT CACCTACTGGGGGCGCGGTACACTTGTCACCGTTAGCAGT | 558 |
| | VH2 | GAGGTCCAGCTGGTTGAGTCTGGAGGTGGTGTAGTCAGGCCAGGAGGTTCCCTGCGTCTGT CTTGCGCTGCCTCTGGGTTCACTGTGTCTGACTACAGTATGAACTGGGTTAGACAGGCTCC AGGGAAATGTTTGGAATGGATAGGGTTTATTCGCAATAAAGCCAATGCCTATACCACCGAA TACAGCGCAAGCGTTAAAGGCAGGTTCACCATCAGTAGGGACGACTCTAAGAATACCCTCT ACCTGCAGATGAATAGCTTGAAGACAGAGGATACCGCTGTTTATTACTGCACTACCTACCC | 562 |
| | | CAGGTATCATGCTATGGACAGTTGGGGTCAAGGAACTATGGTCACCGTCTCCTCT | |
| | VL1 | GACATACAACTCACACAAAGTCCAAGCTCTCTTTCCCTTAGTCCAGGAGAACGCGTAACTC TCTCATGTAAAGCCTCCCAGGATATCAATAACTATCTCTCTTGGTACCAACAAAAGCCTGA CCAAGCTCCTAAGCTGCTTATCAAACGCGCCAATCGGTTGGTAGACGGAGTTCCCGACAGG TTCAGTGGGTCAGGCTCAGGTACAGATTTTACCTTGAAGATATCACGCGTGGAAGCCGAAG ACGTAGGGGTATATTATTGTCTGAAATATGACGAGTTCCCCTATACTTTTGGGCAAGGAAC TCGCCTGGAAATAAAA | 559 |
| | VL2 | GCCATACAGCTGACTCAATCACCTTCTTCTTTGAGCGCCTCTGTCGGTGATCGCGTTACAA TTACATGCCGAGCTTCTCAGTCTATCTCTAATAACCTCCACTGGTATCTCCAGAAACCTGG ACAGTCACCACAGCTTCTGATATATTACGGCTTTCAGTCTATAAGTGGAGTCCCAAGTAGA TTTTCCGGTAGTGGATCTGGCACCGATTTTACTCTTACTATCTCTAGCCTCCAACCTGAAG ATTTCGCAACCTATTACTGCCAGCAGGCCAATTCCTGGCCCTTGACCTTCGGATGTGGGAC TAAGCTGGAAATCAAA | 563 |
| EPB11 | VH1 | GAGGTGCAACTGCTTGAGTCAGGAGGAGGACTGGTTCAACCCGGCGGGAGCCTTCGACTCT CATGTGCAGCCTCTGGGTTTACATTTAGTCGTTACCAAATGATGTGGGTACGCCAGGCCCC CGGAAAAGGTTTGGAGTGGGTGAGCAGCATTTCTCCTTCAGGAGGAGTGACACTTTACGCA GACAGTGTTAAGGGTCGGTTCACAATCAGTCGTGATAATTCCAAGAACACTCTCTATCTCC AAATGAACTCCCTTCGAGCTGAGGACACTGCTGTTTATTACTGTACTCGGGAACTTCTCGG CACTGTGGTTGTTCCTGTGGCTTGGAAGATGCGTGGATACTTCGACTATTGGGGGCAACTC ACTCTTGTTACCGTCTCAAGC | 552 |
| | VH2 | CAAGTGCAACTCCTTGAAAGTGGAGGTGGCCTTGTCCAGCCAGGCGGTTCACTGCGTTTGA GCTGTGCCGCATCCGGCTTTACCTTTTCCAGTTACACAATGAGTTGGGTCCGTCAAGCACC CGGGCAATGTTTGGAATGGATGGGAACTATTTCATCTGGTGGGACTTACACTTACTATCCC GATAGCGTCAAGGGACGTTTTACCATAAGCCGGGACAACGCTAAAAATTCACTCTATCTTC AGATGAACAGTCTTAGGGCTGAGGATACCGCTGTTTATTATTGCGCCCGAGAAGCCATTTT CACCTACTGGGGGCGCGGTACACTTGTCACCGTTAGCAGT | 564 |
| | VL1 | GACATACAGATGACTCAGAGCCCTGGTACACTGAGTGTATCTCCTGGGGAACGCGCCACTC TGTCTTGCCGAGCCTCTCAAAGCGTTAGTTCCAACCTGGCTTGGTATCAGCAAAAACCCGG TCAAGCACCTCGCTTGCTTATCTACGGGGCCTCCACACGGGCAACCGGCATTCCTGCACGC TTTAGTGGCTCTGGTAGCGGGACTGAATTCACTTTGACTATATCTTCCATGCAGAGTGAGG ATTTCGCCGTTTATTATTGTCAACAGTACAATAACTGGCCTCCACTCACTTTTGGTGGTGG CACCAAAGTGGAGATAAAG | 554 |
| | VL2 | GACATACAACTCACACAAAGTCCAAGCTCTCTTTCCCTTAGTCCAGGAGAACGCGTAACTC TCTCATGTAAAGCCTCCCAGGATATCAATAACTATCTCTCTTGGTACCAACAAAAGCCTGA CCAAGCTCCTAAGCTGCTTATCAAACGCGCCAATCGGTTGGTAGACGGAGTTCCCGACAGG TTCAGTGGGTCAGGCTCAGGTACAGATTTTACCTTGAAGATATCACGCGTGGAAGCCGAAG ACGTAGGGGTATATTATTGTCTGAAATATGACGAGTTCCCCTATACTTTTGGGTGTGGAAC TCGCCTGGAAATAAAA | 565 |
| EPB12 | VH1 | GAGGTCCAGCTGGTTGAGTCTGGAGGTGGTGTAGTCAGGCCAGGAGGTTCCCTGCGTCTGT CTTGCGCTGCCTCTGGGTTCACTGTGTCTGACTACAGTATGAACTGGGTTAGACAGGCTCC | 556 |
| | | AGGGAAAGGTTTGGAATGGATAGGGTTTATTCGCAATAAAGCCAATGCCTATACCACCGAA TACAGCGCAAGCGTTAAAGGCAGGTTCACCATCAGTAGGGACGACTCTAAGAATACCCTCT ACCTGCAGATGAATAGCTTGAAGACAGAGGATACCGCTGTTTATTACTGCACTACCTACCC CAGGTATCATGCTATGGACAGTTGGGGTCAAGGAACTATGGTCACCGTCTCCTCT | |
| | VH2 | CAAGTGCAACTCCTTGAAAGTGGAGGTGGCCTTGTCCAGCCAGGCGGTTCACTGCGTTTGA GCTGTGCCGCATCCGGCTTTACCTTTTCCAGTTACACAATGAGTTGGGTCCGTCAAGCACC CGGGCAATGTTTGGAATGGATGGGAACTATTTCATCTGGTGGGACTTACACTTACTATCCC GATAGCGTCAAGGGACGTTTTACCATAAGCCGGGACAACGCTAAAAATTCACTCTATCTTC AGATGAACAGTCTTAGGGCTGAGGATACCGCTGTTTATTATTGCGCCCGAGAAGCCATTTT CACCTACTGGGGGCGCGGTACACTTGTCACCGTTAGCAGT | 564 |
| | VL1 | GCCATACAGCTGACTCAATCACCTTCTTCTTTGAGCGCCTCTGTCGGTGATCGCGTTACAA TTACATGCCGAGCTTCTCAGTCTATCTCTAATAACCTCCACTGGTATCTCCAGAAACCTGG ACAGTCACCACAGCTTCTGATATATTACGGCTTTCAGTCTATAAGTGGAGTCCCAAGTAGA TTTTCCGGTAGTGGATCTGGCACCGATTTTACTCTTACTATCTCTAGCCTCCAACCTGAAG ATTTCGCAACCTATTACTGCCAGCAGGCCAATTCCTGGCCCTTGACCTTCGGAGGTGGGAC TAAGCTGGAAATCAAA | 557 |
| | VL2 | GACATACAACTCACACAAAGTCCAAGCTCTCTTTCCCTTAGTCCAGGAGAACGCGTAACTC TCTCATGTAAAGCCTCCCAGGATATCAATAACTATCTCTCTTGGTACCAACAAAAGCCTGA CCAAGCTCCTAAGCTGCTTATCAAACGCGCCAATCGGTTGGTAGACGGAGTTCCCGACAGG TTCAGTGGGTCAGGCTCAGGTACAGATTTTACCTTGAAGATATCACGCGTGGAAGCCGAAG ACGTAGGGGTATATTATTGTCTGAAATATGACGAGTTCCCCTATACTTTTGGGTGTGGAAC TCGCCTGGAAATAAAA | 565 |

Table 54 below shows the heavy chain and light chain CDR sequences of the engineered antibodies targeting EphA2.

**[Table 54]**

| **Name** | CDR | **Sequence** | **SEQ ID NO** |
|---|---|---|---|
| EPB01 | V1 CDR-H1 | RYQMM | 151 |
| | V1 CDR-H2 | SISPSGGVTLYADSVKG | 152 |
| | V1 CDR-H3 | ELLGTVVVPVAWKMRGYFDY | 153 |
| | V1 CDR-L1 | RASQSVSSNLA | 154 |
| | V1 CDR-L2 | GASTRAT | 155 |
| | V1 CDR-L3 | QQYNNWPPLT | 156 |
| | V2 CDR-H1 | HYMMA | 157 |
| | V2 CDR-H2 | RIGPSGGPTHYADSVKG | 158 |
| | V2 CDR-H3 | YDSGYDYVAVAGPAEYFQH | 159 |
| | V2 CDR-L1 | RASQSISTWLA | 160 |
| | V2 CDR-L2 | KASNLHT | 161 |
| | V2 CDR-L3 | QQYNSYSRT | 162 |
| EPB02 | V1 CDR-H1 | DYSMN | 163 |
| | V1 CDR-H2 | FIRNKANAYTTEYSASVKG | 164 |
| | V1 CDR-H3 | YPRYHAMDSW | 165 |
| | V1 CDR-L1 | RASQSISNNLH | 166 |
| | V1 CDR-L2 | YGFQSIS | 167 |
| | V1 CDR-L3 | QQANSWPLT | 168 |
| | V2 CDR-H1 | HYMMA | 157 |
| | V2 CDR-H2 | RIGPSGGPTHYADSVKG | 158 |
| | V2 CDR-H3 | YDSGYDYVAVAGPAEYFQH | 159 |
| | V2 CDR-L1 | RASQSISTWLA | 160 |
| | V2 CDR-L2 | KASNLHT | 161 |
| | V2 CDR-L3 | QQYNSYSRT | 162 |
| EPB03 | V1 CDR-H1 | SYTMS | 169 |
| | V1 CDR-H2 | TISSGGTYTYYPDSVKG | 170 |
| | V1 CDR-H3 | EAIFTYW | 171 |
| | V1 CDR-L1 | KASQDINNYLS | 172 |
| | V1 CDR-L2 | RANRLVD | 173 |
| | V1 CDR-L3 | LKYDEFPYT | 174 |
| | V2 CDR-H1 | HYMMA | 157 |
| | V2 CDR-H2 | RIGFSGGPTHYADSVKG | 158 |
| | V2 CDR-H3 | YDSGYDYVAVAGPAEYFQH | 159 |
| | V2 CDR-L1 | RASQSISIWLA | 160 |
| | V2 CDR-L2 | KASNLHT | 161 |
| | V2 CDR-L3 | QQYNSYSRT | 162 |
| EPB04 | V1 CDR-H1 | HYMMA | 157 |
| | V1 CDR-H2 | RIGPSGGPTHYADSVKG | 158 |
| | V1 CDR-H3 | YDSGYUYVAVAGPAEYTQH | 150 |
| | V1 CDR-L1 | RASQSISTWLA | 160 |
| | V1 CDR-L2 | KASNLHT | 161 |
| | V1 CDR-L3 | QQYNSYSRT | 162 |
| | V2 CDR-H1 | RYQMM | 151 |
| | V2 CDR-H2 | SISPSGGVTLYADSVKG | 152 |
| | V2 CDR-H3 | ELLGTVVVAWKMRGYFDY | 153 |
| | V2 CDR-L1 | RASQSVSSNLA | 154 |
| | V2 CDR-L2 | GASTRAT | 155 |
| | V2 CDR-L3 | QQYNNWPPLT | 156 |
| EPB05 | V1 COR-H1 | HYMMA | 157 |
| | V1 CDR-H2 | RIGPSGGPTHYADSVKG | 158 |
| | V1 CDR-H3 | YDSGYDYVAVAGPAEYFQH | 159 |
| | V1 CDR-L1 | RASQSISTWLA | 160 |
| | VI CDR-L2 | KASNLHT | 161 |
| | V1 CDR-L3 | QQYNSYSRT | 162 |
| | V2 CDR-H1 | DYSMN | 163 |
| | V2 CDR-H2 | FIRNKANAYTTEYSASVKG | 164 |
| | V2 CDR-H3 | YPRYHAMDSW | 165 |
| | V2 CDE-L1 | RASQSISNNLH | 166 |
| | V2 CDR-L2 | YGFQSIS | 167 |
| | V2 CD2-L3 | QQANSWPLT | 168 |
| EPB06 | V1 CDR-H1 | HYMMA | 157 |
| | V1 CDR-H2 | RIGPSGGPTHYADSVKG | 158 |
| | V1 CDR-H3 | YDSGYDYVAVAGPAEYFQH | 159 |
| | V1 CDR-L1 | RASQSISTWLA | 160 |
| | V1 CDR-L2 | KASNLHT | 161 |
| | V1 CDR-L3 | QQYNSYSRT | 162 |
| | V2 CDR-H1 | SYTMS | 169 |
| | V2 CDR-H2 | TISSGGTYTYYPDSVKG | 170 |
| | V2 CDR-H3 | EAIFTYW | 171 |
| | V2 CDR-L1 | KASQDINNYLS | 172 |
| | V2 CDR-L2 | RANRLVD | 173 |
| | V2 CDR-L3 | LKYDEFPYT | 174 |
| EPB07 | V1 CDR-H1 | DYSMN | 163 |
| | V1 CDR-H2 | FIRNKANAYTTEYSASVKG | 164 |
| | V1 CDR-H3 | YPRYHAMDSW | 165 |
| | V1 CDR-L1 | RASQSISNNLH | 166 |
| | V1 CDR-L2 | YGFQSIS | 167 |
| | V1 CDR-L3 | QQANSWPLT | 168 |
| | V2 CDR-H1 | RYQMM | 151 |
| | V2 CDR-H2 | SISPSGGVTLYADSVKG | 152 |
| | V2 CDR-H3 | ELLGTVVVPVAWKMRGYFDY | 153 |
| | V2 CDR-L1 | RASQSVSSNLA | 154 |
| | V2 CDR-L2 | GASTRAT | 155 |
| | V2 CDR-L3 | QQYNNPPLT | 156 |
| EPB08 | V1 CDR-H1 | SYTMS | 169 |
| | V1 CDR-H2 | TISSGGTYTYYPDSVKG | 170 |
| | V1 CDR-H3 | EAIFTYW | 171 |
| | V1 CDR-L1 | KASQDINNYLS | 172 |
| | V1 CDR-L2 | RANRLVD | 173 |
| | V1 CDR-L3 | LKYDEFPYT | 174 |
| | V2 CDR-H1 | RYQMM | 151 |
| | V2 CDR-H2 | SISPSGGVTLYADSVKG | 152 |
| | V2 CDR-H3 | ELLGTVVVAWKMRGYFDY | 153 |
| | V2 CDR-L1 | RASQSVSSNLA | 154 |
| | V2 CDR-L2 | GASTRAT | 155 |
| | V2 CDR-L3 | QQYNNWPPLT | 156 |
| EPB09 | V1 CDR-H1 | RYQMM | 151 |
| | V1 CDR-H2 | SISPSGGVTLYADSVKG | 152 |
| | V1 CDR-H3 | ELLGTVVVPVAWKMRGYFDY | 153 |
| | V1 CDR-L1 | RASQSVSSNLA | 154 |
| | V1 CDR-L2 | GASTRAT | 155 |
| | V1 CDR-L3 | QQYTWPPLT | 156 |
| | V2 CDR-H1 | DYSMN | 163 |
| | V2 CDR-H2 | FLRNKANAYTTEYSASVKG | 164 |
| | V2 CDR-H3 | YTRYHAMDSW | 165 |
| | V2 CDR-L1 | RASQSISNNLH | 166 |
| | V2 CDR-L2 | YGFQSIS | 167 |
| | V2 CDR-L3 | QQANSWPLT | 168 |
| EPB10 | V1 CDR-H1 | SYTMS | 169 |
| | V1 CDR-H2 | TISSGGTYTYYPDSVKG | 170 |
| | V1 CDR-H3 | EAIFTYW | 171 |
| | V1 CDR-L1 | KASQDINNYLS | 172 |
| | V1 CDR-L2 | RANRLVD | 173 |
| | V1 CDR-L3 | LKYDEFPYT | 174 |
| | V2 CDR-H1 | DYSMN | 163 |
| | V2 CDR-H2 | FIRNKANAYTTEYSASVKG | 164 |
| | V2 CDR-H3 | YPRYHAMDSW | 165 |
| | V2 CDR-L1 | RASQSISNNLH | 166 |
| | V2 CDR-L2 | YGFQSIS | 167 |
| | V2 CDR-L3 | QQANSPLT | 168 |
| EFB11 | V1 CDR-H1 | RYQMM | 151 |
| | V1 CDR-H2 | SISPSGGVTLYADSVKG | 152 |
| | V1 CDR-H3 | ELLGTVVVFVAWKMRGYFDY | 153 |
| | Y1 CDR-L1 | RASQSVSSNLA | 154 |
| | V1 CDR-L2 | GASTRAT | 155. |
| | V1 CDR-L3 | QQYNNWPPLT | 156 |
| | V2 CDR-H1 | SYTMS | 169 |
| | V2 CDR-H2 | TISSGGTYTYYPDSVKG | 170 |
| | V2 CDR-H3 | EAIFTYW | 17 1 |
| | V2 CDR-L1 | KASQDINNYLS | 172 |
| | V2 CDR-L2 | RANRLVD | 17 3 |
| | V2 CDR-L3 | LKYDEFPYT | 174 |
| EPB12 | V1 CDR-H1 | DYSMN | 163 |
| | V1 CDR-H2 | FIRNKANAYTTEYSASVKG | 164 |
| | V1 CDR-H3 | YPRYHAMDSW | 165 |
| | V1 CDR-L1 | RASQSISNNLH | 166 |
| | V1 CDR-L2 | YGFQSIS | 167 |
| | V1 CDR-L3 | QQANSWPLT | 168 |
| | V2 CDR-H1 | SYTMS | 169 |
| | V2 CDR-H2 | TISSGGTYTYYPDSVKG | 170 |
| | V2 CDR-H3 | EAIFTYW | 171 |
| | V2 CDR-L1 | KASQDINNYLS | 172 |
| | V2 CDR-L2 | RANRLVD | 173 |
| | V2 CDR-L3 | LKYDEFPYT | 174 |

The EphA2 protein binding constants of EPB01, EPB02, EPB03, EPB04, EPB05, EPB06, EPB07, EPB08, EPB09, EPB10, EPB11, and EPB12 were determined using Octet Red96e (Sartorius). In order to analyze the binding constants of the twelve biparatopic antibodies, the human EphA2 recombinant protein (Sino Biologicals, 13926-H08H) was loaded onto the Anti-Penta-HIS (HIS1K) biosensor (Sartorius, 18-5120), and then the twelve antibodies were added in a binding reaction (300 seconds) and a dissociation reaction (1,200 seconds) at various concentrations. Based on the above, the affinity for EphA2 was calculated (Table 55). Table 55 below illustrates a result obtained by analyzing the binding constants of the engineered antibodies targeting EphA2.

**[Table 55]**

| Antibodies | K_{D} (nM) | Kₐ (1/Ms) | K_{d} (1/s) |
|---|---|---|---|
| EPB01 | 9.8579 | 2.32E+05 | 2.29E-03 |
| EPB02 | <0.001 | 2.15E+05 | <1.0E-07 |
| EPB03 | 0.7944 | 4.91E+05 | 3.90E-04 |
| EPB04 | 2.8644 | 4.95E+05 | 1.42E-03 |
| EPB05 | <0.001 | 3.42E+05 | <1.0E-07 |
| EPB06 | <0.001 | 3.84E+05 | <1.0E-07 |
| EPB07 | 0.0795 | 2.03E+05 | 1.62E-05 |
| EPB08 | 3.2174 | 6.58E+05 | 2.12E-03 |
| EPB09 | 2.3522 | 2.64E+05 | 6.20E-04 |
| EPB10 | 0.1878 | 3.92E+05 | 7.36E-05 |
| EPB11 | 0.5349 | 2.98E+05 | 1.59E-04 |
| EPB12 | 0.8657 | 5.44E+05 | 4.71E-04 |

PC-3 prostate cancer cell line was used to analyze the ability of antibodies to inhibit EphA2 signaling. PC-3 cancer cell lysate treated with each antibody at a concentration of 50 nM for 30 minutes was analyzed by western blot. The 1C1 humanized antibody that targets human EphA2 was used as a positive control and was prepared based on the sequence published in the literature (Kinch et al., US 20090304721 A1). Akt Rabbit mAb (Cell Signaling Technology, 9272), Phospho-Akt (Ser473) (D9E) XP^{®} Rabbit mAb (Cell Signaling Technology, 4060), and β-Actin (13E5) Rabbit mAb (Cell Signaling Technology, 4970) were used as primary antibodies for analysis, and anti-rabbit IgG, HRP-linked antibody (Cell Signaling Technology, 7074) was used as secondary antibody. In the analysis of AKT signaling pathway inhibition, EPB02, EPB03, and EPB05 showed a similar level of inhibition as the positive control 1C1 (Figure 44).

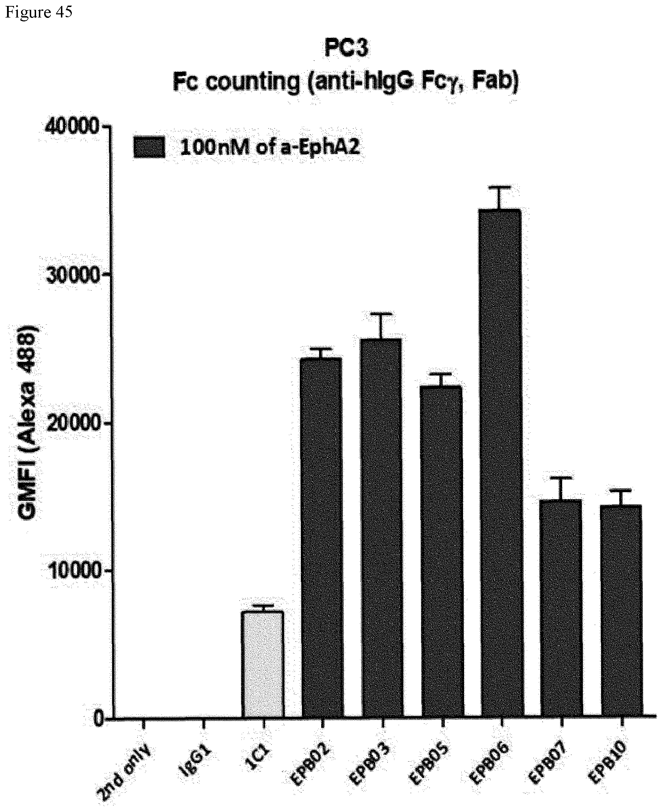

PC-3 prostate cancer cell line was used to quantify the Fc loads on the surface of EphA2 expressing cells. 100 nM antibodies were allowed to bind to the PC-3 cell line at 4°C for 30 minutes, and the Fc loads were quantified using the Alexa 488 fluorescence-conjugated anti-human IgG Fcγ Fab antibody (Jackson ImmunoResearch, 109-547-008) (Figure 44). It was shown that higher Fc loads on the surface of cancer cells are induced by treatment of EPB02, EPB03, EPB05, EPB06, EPB07, and EPB10 compared to treatment of 1C1 humanized antibody that targets EphA2 (Figure 45).

### Example 19. Design, preparation and analysis of antibody structure targeting MET

The variant light chain and heavy chain polypeptide sequences of the antibodies that specifically bind to the MET protein are shown in Table 56. For MEM01, expression vectors consisting of the sequences corresponding to Fc-Hole (SEQ ID NO: 7), MEM01 HC (SEQ ID NO: 568), and MEM01 LC (SEQ ID NO: 569) were co-transfected into EXPICHO-S^{™} (Gibco, A29127) (Figure 41a, Table 56), and purification and analysis were performed in the same manner as described in Example 1. Expression, purification and analysis were performed for MEM06 in the same manner as mentioned above (Table 56, Table 57).

**[Table 56]**

| Name | Sequence | SEQ ID NO |
|---|---|---|
| MEM01 HC | EVQLVESGGGLVQPGGSLRLSCAASGYTFTSYWLHWVRQAPGKCLE WVGMIDPSNSDTRFNPNFKDRFTISADTSKNTAYLQMNSLRAEDTAV YYCATYRSYVTPLDYWGQGTLVTVSSASTKGPSVFPLAPSSKSTSGG TAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPE LLGGPSVFLFPPKPKDTLMISRTPEVTCVVVDVSHEDPEVKFNWYVD GVEVHNAKTKPREEQYNSTYRVVSVLTVLHQDWLNGKEYKCKVSN KALPAPIEKTISKAKGQPREPQVYTLPPCREEMTKNQVSLWCLVKGF YPSDIAVEWESNGQPENNYKTTPPVLDSDGSFFLYSKLTVDKSRWQQ GNVFSCSVMHEALHNHYTQKSLSLSPGK | 568 |
| MEM01 LC | DIQMTQSPSSLSASVGDRVTITCKSSQSLLYTSSQKNYLAWYQQKPG KAPKLLIYWASTRESGVPSRFSGSGSGTDFTLTISSLQPEDFATYYCQ QYYAYPWTFGCGTKVEIKRTVAAPSVFIFPPSDEQLKSGTASVVCLL NNFYPREAKVQWKVDNALQSGNSQESVTEQDSKDSTYSLSSTLTLSK ADYEKHKVYACEVTHQGLSSPVTKSFNRGECGGGGSGGGGSGGGGS EPKSSDKTHTCPPCPAPELLGGPSVFLFPPKPKDTLMISRTPEVTCVVV DVSHEDPEVKFNWYVDGVEVHNAKTKPREEQYNSTYRVVSVLTVL HQDWLNGKEYKCKVSNKALPAPIEKTISKAKGQPREPQVYTLPPCRE EMTKNQVSLWCLVKGFYPSDIAVEWESNGQPENNYKTTPPVLDSDG SFFLYSKLTVDKSRWQQGNVFSCSVMHEALHNHYTQKSLSLSPGK | 569 |
| MEM06 HC | QVQLVQSGAEVKKPGASVKVSCKASGYTFTSYGFSWVRQAPGQCLE WMGWISASNGNTYYAQKLQGRVTMTTDTSTSTAYMELRSLRSDDT AVYYCARVYADYADYWGQGTLVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDKTHTCPPCPA PELLGGPSVFLFPPKPKDTLMISRTPEVTCVVVDVSHEDPEVKFNWY VDGVEVHNAKTKPREEQYNSTYRVVSVLTVLHQDWLNGKEYKCKV SNKALPAPIEKTISKAKGQPREPQVYTLPPCREEMTKNQVSLWCLVK GFYPSDIAVEWESNGQPENNYKTTPPVLDSDGSFFLYSKLTVDKSRW QQGNVFSCSVMHEALHNHYTQKSLSLSPGK | 570 |
| MEM06 LC | DIQMTQSPSSVSASVGDRVTITCRASQGINTWLAWYQQKPGKAPKLL IYAASSLKSGVPSRFSGSGSGTDFTLTISSLQPEDFATYYCQQANSFPL TFGCGTKVEIKRTVAAPSVFIFPPSDEQLKSGTASVVCLLNNFYPREA KVQWKVDNALQSGNSQESVTEQDSKDSTYSLSSTLTLSKADYEKHK VYACEVTHQGLSSPVTKSFNRGECGGGGSGGGGSGGGGSEPKSSDK THTCPPCPAPELLGGPSVFLFPPKPKDTLMISRTPEVTCVVVDVSHED PEVKFNWYVDGVEVHNAKTKPREEQYNSTYRVVSVLTVLHQDWLN GKEYKCKVSNKALPAPIEKTISKAKGQPREPQVYTLPPCREEMTKNQ VSLWCLVKGFYPSDIAVEWESNGQPENNYKTTPPVLDSDGSFFLYSK LTVDKSRWQQGNVFSCSVMHEALHNHYTQKSLSLSPGK | 571 |

**[Table 57]**

| Name | Sequence | SEQ ID NO |
|---|---|---|
| MEM01 HC | GAAGTCCAATTGGTCGAGAGTGGAGGCGGGTTGGTACAGCCAGG AGGTTCACTTAGGTTGTCCTGCGCCGCCAGTGGTTACACTTTCACA TCTTATTGGCTGCACTGGGTGCGCCAAGCTCCTGGGAAGTGTCTC GAATGGGTGGGTATGATCGATCCATCTAATTCCGACACACGGTTT AACCCAAATTTTAAGGATAGATTTACAATTAGTGCTGACACTTCA AAAAACACAGCATACCTCCAGATGAACAGCCTGCGTGCTGAGGAT ACTGCTGTCTACTACTGTGCAACTTACCGCTCCTATGTCACACCTT TGGATTATTGGGGCCAGGGGACTCTGGTGACCGTGAGTTCTGCTA GCACCAAAGGACCTAGTGTTTTTCCTCTTGCCCCTTCCTCAAAGTC TACCTCTGGGGGGACAGCCGCTCTGGGCTGCCTGGTCAAGGATTA TTTCCCAGAGCCTGTCACTGTCAGTTGGAACTCTGGAGCCTTGACT TCTGGTGTTCATACATTTCCTGCTGTCCTTCAAAGCAGCGGCTTGT ACTCATTGTCTTCTGTTGTGACAGTACCCTCAAGCAGCCTCGGCAC TCAGACATACATCTGCAATGTCAACCACAAACCCTCAAATACAAA GGTAGATAAAAAAGTCGAACCAAAGTCTTGTGACAAAACTCACA CGTGCCCACCGTGCCCAGCACCTGAACTCCTGGGGGGACCGTCAG TCTTCCTCTTCCCCCCAAAACCCAAGGACACCCTCATGATCTCCCG GACCCCTGAGGTCACATGCGTGGTGGTGGACGTGAGCCACGAAG ACCCTGAGGTCAAGTTCAACTGGTACGTGGACGGCGTGGAGGTGC ATAATGCCAAGACAAAGCCGCGGGAGGAGCAGTACAACAGCACG TACCGTGTGGTCAGCGTCCTCACCGTCCTGCACCAGGACTGGCTG AATGGCAAGGAGTACAAGTGCAAGGTCTCCAACAAAGCCCTCCC AGCCCCCATCGAGAAAACCATCTCCAAAGCCAAAGGGCAGCCCC GAGAACCACAGGTGTACACCCTGCCCCCATGCCGGGAGGAGATG ACCAAGAACCAGGTCAGCCTGTGGTGCCTGGTCAAAGGCTTCTAT CCCAGCGACATCGCCGTGGAGTGGGAGAGCAATGGGCAGCCGGA GAACAACTACAAGACCACGCCTCCCGTGCTGGACTCCGACGGCTC CTTCTTCCTCTACAGCAAGCTCACCGTGGACAAGAGCAGGTGGCA GCAGGGGAACGTCTTCTCATGCTCCGTGATGCATGAGGCTCTGCA CAACCACTACACGCAGAAGAGCCTCTCCCTGTCTCCGGGTAAA | 572 |
| MEM01 LC | GATATACAGATGACACAAAGTCCCTCATCACTTTCTGCCTCCGTTG GAGATCGTGTGACCATTACCTGTAAGAGTTCCCAATCACTGCTTTA TACCTCTTCACAAAAAAATTACCTCGCTTGGTACCAGCAGAAGCC AGGTAAAGCACCTAAGCTGTTGATCTATTGGGCCTCCACTAGAGA GTCAGGCGTGCCCAGCCGTTTCTCCGGTTCAGGGAGTGGGACAGA CTTTACCTTGACCATTTCTTCTTTGCAACCTGAAGACTTCGCCACA TACTATTGTCAGCAATATTACGCATATCCATGGACCTTTGGGTGTG GAACCAAAGTCGAAATAAAACGTACGGTGGCAGCTCCCAGCGTTT TTATCTTTCCCCCATCCGACGAGCAGCTCAAGAGTGGCACTGCCTC TGTAGTTTGTTTGCTGAATAACTTCTATCCACGTGAAGCAAAAGTA CAGTGGAAGGTCGATAATGCCCTTCAGAGCGGTAACAGCCAAGA AAGTGTTACCGAGCAAGATTCCAAAGATTCCACTTACAGTCTGTC CAGCACATTGACACTGAGTAAGGCTGATTACGAAAAACACAAGG TGTACGCATGCGAGGTGACACACCAAGGTCTTTCATCTCCTGTAA CTAAGAGCTTTAACCGGGGAGAATGTGGTGGTGGGGGCAGCGGG | 573 |
| | GGCGGAGGTAGTGGAGGCGGCGGTAGTGAACCAAAGAGTAGTGA CAAAACTCACACGTGCCCACCGTGCCCAGCACCTGAACTCCTGGG GGGACCGTCAGTCTTCCTCTTCCCCCCAAAACCCAAGGACACCCT CATGATCTCCCGGACCCCTGAGGTCACATGCGTGGTGGTGGACGT GAGCCACGAAGACCCTGAGGTCAAGTTCAACTGGTACGTGGACG GCGTGGAGGTGCATAATGCCAAGACAAAGCCGCGGGAGGAGCAG TACAACAGCACGTACCGTGTGGTCAGCGTCCTCACCGTCCTGCAC CAGGACTGGCTGAATGGCAAGGAGTACAAGTGCAAGGTCTCCAA CAAAGCCCTCCCAGCCCCCATCGAGAAAACCATCTCCAAAGCCAA AGGGCAGCCCCGAGAACCACAGGTGTACACCCTGCCCCCATGCCG GGAGGAGATGACCAAGAACCAGGTCAGCCTGTGGTGCCTGGTCA AAGGCTTCTATCCCAGCGACATCGCCGTGGAGTGGGAGAGCAATG GGCAGCCGGAGAACAACTACAAGACCACGCCTCCCGTGCTGGACT CCGACGGCTCCTTCTTCCTCTACAGCAAGCTCACCGTGGACAAGA GCAGGTGGCAGCAGGGGAACGTCTTCTCATGCTCCGTGATGCATG AGGCTCTGCACAACCACTACACGCAGAAGAGCCTCTCCCTGTCTC CGGGTAAA | |
| MEM06 HC | CAAGTGCAGCTTGTTCAATCAGGGGCCGAGGTTAAGAAACCAGGT GCTTCCGTCAAGGTCTCCTGCAAGGCTTCCGGCTACACTTTTACCA GTTATGGGTTCAGTTGGGTTAGACAGGCCCCAGGGCAGTGTCTCG AATGGATGGGATGGATTTCCGCATCTAACGGGAATACTTACTATG CCCAGAAACTTCAAGGTAGGGTTACCATGACTACCGATACTTCCA CTAGTACAGCCTACATGGAACTCAGATCACTCCGTTCAGATGACA CCGCAGTATATTACTGTGCAAGGGTATATGCTGATTATGCCGATTA TTGGGGGCAAGGAACACTTGTCACAGTATCCAGCGCTAGCACCAA AGGACCTAGTGTTTTTCCTCTTGCCCCTTCCTCAAAGTCTACCTCT GGGGGGACAGCCGCTCTGGGCTGCCTGGTCAAGGATTATTTCCCA GAGCCTGTCACTGTCAGTTGGAACTCTGGAGCCTTGACTTCTGGTG TTCATACATTTCCTGCTGTCCTTCAAAGCAGCGGCTTGTACTCATT GTCTTCTGTTGTGACAGTACCCTCAAGCAGCCTCGGCACTCAGAC ATACATCTGCAATGTCAACCACAAACCCTCAAATACAAAGGTAGA TAAAAAAGTCGAACCAAAGTCTTGTGACAAAACTCACACGTGCCC ACCGTGCCCAGCACCTGAACTCCTGGGGGGACCGTCAGTCTTCCT CTTCCCCCCAAAACCCAAGGACACCCTCATGATCTCCCGGACCCC TGAGGTCACATGCGTGGTGGTGGACGTGAGCCACGAAGACCCTGA GGTCAAGTTCAACTGGTACGTGGACGGCGTGGAGGTGCATAATGC CAAGACAAAGCCGCGGGAGGAGCAGTACAACAGCACGTACCGTG TGGTCAGCGTCCTCACCGTCCTGCACCAGGACTGGCTGAATGGCA AGGAGTACAAGTGCAAGGTCTCCAACAAAGCCCTCCCAGCCCCCA TCGAGAAAACCATCTCCAAAGCCAAAGGGCAGCCCCGAGAACCA CAGGTGTACACCCTGCCCCCATGCCGGGAGGAGATGACCAAGAA CCAGGTCAGCCTGTGGTGCCTGGTCAAAGGCTTCTATCCCAGCGA CATCGCCGTGGAGTGGGAGAGCAATGGGCAGCCGGAGAACAACT ACAAGACCACGCCTCCCGTGCTGGACTCCGACGGCTCCTTCTTCCT CTACAGCAAGCTCACCGTGGACAAGAGCAGGTGGCAGCAGGGGA | 574 |
| | ACGTCTTCTCATGCTCCGTGATGCATGAGGCTCTGCACAACCACTA CACGCAGAAGAGCCTCTCCCTGTCTCCGGGTAAA | |
| MEM06 LC | GATATCCAAATGACACAGTCACCCTCAAGTGTTAGCGCAAGTGTC GGGGACAGGGTGACCATCACATGCAGAGCTTCCCAGGGTATCAAT ACATGGCTGGCATGGTATCAACAGAAACCCGGAAAAGCACCAAA ATTGCTTATTTATGCAGCTTCTAGCTTGAAGAGTGGGGTTCCCTCT CGTTTCTCTGGTTCAGGAAGCGGTACTGACTTTACCTTGACCATCA GTAGCTTGCAGCCCGAAGATTTCGCTACATATTATTGCCAACAGG CCAACTCTTTTCCCCTGACATTCGGTTGTGGCACTAAAGTGGAAAT TAAGCGTACGGTGGCAGCTCCCAGCGTTTTTATCTTTCCCCCATCC GACGAGCAGCTCAAGAGTGGCACTGCCTCTGTAGTTTGTTTGCTG AATAACTTCTATCCACGTGAAGCAAAAGTACAGTGGAAGGTCGAT AATGCCCTTCAGAGCGGTAACAGCCAAGAAAGTGTTACCGAGCA AGATTCCAAAGATTCCACTTACAGTCTGTCCAGCACATTGACACT GAGTAAGGCTGATTACGAAAAACACAAGGTGTACGCATGCGAGG TGACACACCAAGGTCTTTCATCTCCTGTAACTAAGAGCTTTAACCG GGGAGAATGTGGTGGTGGGGGCAGCGGGGGCGGAGGTAGTGGAG GCGGCGGTAGTGAACCAAAGAGTAGTGACAAAACTCACACGTGC CCACCGTGCCCAGCACCTGAACTCCTGGGGGGACCGTCAGTCTTC CTCTTCCCCCCAAAACCCAAGGACACCCTCATGATCTCCCGGACC CCTGAGGTCACATGCGTGGTGGTGGACGTGAGCCACGAAGACCCT GAGGTCAAGTTCAACTGGTACGTGGACGGCGTGGAGGTGCATAAT GCCAAGACAAAGCCGCGGGAGGAGCAGTACAACAGCACGTACCG TGTGGTCAGCGTCCTCACCGTCCTGCACCAGGACTGGCTGAATGG CAAGGAGTACAAGTGCAAGGTCTCCAACAAAGCCCTCCCAGCCCC CATCGAGAAAACCATCTCCAAAGCCAAAGGGCAGCCCCGAGAAC CACAGGTGTACACCCTGCCCCCATGCCGGGAGGAGATGACCAAG AACCAGGTCAGCCTGTGGTGCCTGGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTTC CTCTACAGCAAGCTCACCGTGGACAAGAGCAGGTGGCAGCAGGG GAACGTCTTCTCATGCTCCGTGATGCATGAGGCTCTGCACAACCA CTACACGCAGAAGAGCCTCTCCCTGTCTCCGGGTAAA | 575 |

Table 58 below shows the polypeptide sequences of the heavy chain variable region (VH) and light chain variable region (VL) of the engineered antibodies targeting MET. Table 59 below shows the nucleotide sequences of the heavy chain variable region (VH) and light chain variable region (VL) of the engineered antibodies targeting MET.

**[Table 58]**

| Name | | Sequence | SEQ ID NO |
|---|---|---|---|
| MEM01 | VH | EVQLVESGGGLVQPGGSLRLSCAASGYTFTSYWLHWVRQAPGKCLE WVGMIDPSNSDTRFNPNFKDRFTISADTSKNTAYLQMNSLRAEDTAV YYCATYRSYVTPLDYWGQGTLVTVSS | 576 |
| | VL | DIQMTQSPSSLSASVGDRVTITCKSSQSLLYTSSQKNYLAWYQQKPGK APKLLIYWASTRESGVPSRFSGSGSGTDFTLTISSLQPEDFATYYCQQY | 577 |
| | | YAYPWTFGCGTKVEIK | |
| MEM06 | VH | QVQLVQSGAEVKKPGASVKVSCKASGYTFTSYGFSWVRQAPGQCLE WMGWISASNGNTYYAQKLQGRVTMTTDTSTSTAYMELRSLRSDDTA VYYCARVYADYADYWGQGTLVTVSS | 578 |
| | VL | DIQMTQSPSSVSASVGDRVTITCRASQGINTWLAWYQQKPGKAPKLLI YAASSLKSGVPSRFSGSGSGTDFTLTISSLQPEDFATYYCQQANSFPLT FGCGTKVEIK | 579 |

**[Table 59]**

| Name | | Sequence | SEQ ID NO |
|---|---|---|---|
| MEM01 | VH | GAAGTCCAATTGGTCGAGAGTGGAGGCGGGTTGGTACAGCCAGGA GGTTCACTTAGGTTGTCCTGCGCCGCCAGTGGTTACACTTTCACAT CTTATTGGCTGCACTGGGTGCGCCAAGCTCCTGGGAAGTGTCTCGA ATGGGTGGGTATGATCGATCCATCTAATTCCGACACACGGTTTAAC CCAAATTTTAAGGATAGATTTACAATTAGTGCTGACACTTCAAAAA ACACAGCATACCTCCAGATGAACAGCCTGCGTGCTGAGGATACTG CTGTCTACTACTGTGCAACTTACCGCTCCTATGTCACACCTTTGGAT TATTGGGGCCAGGGGACTCTGGTGACCGTGAGTTCT | 580 |
| | VL | GATATACAGATGACACAAAGTCCCTCATCACTTTCTGCCTCCGTTG GAGATCGTGTGACCATTACCTGTAAGAGTTCCCAATCACTGCTTTA TACCTCTTCACAAAAAAATTACCTCGCTTGGTACCAGCAGAAGCCA GGTAAAGCACCTAAGCTGTTGATCTATTGGGCCTCCACTAGAGAGT CAGGCGTGCCCAGCCGTTTCTCCGGTTCAGGGAGTGGGACAGACTT TACCTTGACCATTTCTTCTTTGCAACCTGAAGACTTCGCCACATACT ATTGTCAGCAATATTACGCATATCCATGGACCTTTGGGTGTGGAAC CAAAGTCGAAATAAAA | 581 |
| MEM06 | VH | CAAGTGCAGCTTGTTCAATCAGGGGCCGAGGTTAAGAAACCAGGT GCTTCCGTCAAGGTCTCCTGCAAGGCTTCCGGCTACACTTTTACCA GTTATGGGTTCAGTTGGGTTAGACAGGCCCCAGGGCAGTGTCTCGA ATGGATGGGATGGATTTCCGCATCTAACGGGAATACTTACTATGCC CAGAAACTTCAAGGTAGGGTTACCATGACTACCGATACTTCCACTA GTACAGCCTACATGGAACTCAGATCACTCCGTTCAGATGACACCGC AGTATATTACTGTGCAAGGGTATATGCTGATTATGCCGATTATTGG GGGCAAGGAACACTTGTCACAGTATCCAGC | 582 |
| | VL | GATATCCAAATGACACAGTCACCCTCAAGTGTTAGCGCAAGTGTC GGGGACAGGGTGACCATCACATGCAGAGCTTCCCAGGGTATCAAT ACATGGCTGGCATGGTATCAACAGAAACCCGGAAAAGCACCAAAA TTGCTTATTTATGCAGCTTCTAGCTTGAAGAGTGGGGTTCCCTCTCG TTTCTCTGGTTCAGGAAGCGGTACTGACTTTACCTTGACCATCAGT AGCTTGCAGCCCGAAGATTTCGCTACATATTATTGCCAACAGGCCA ACTCTTTTCCCCTGACATTCGGTTGTGGCACTAAAGTGGAAATTAA G | 583 |

Table 60 below shows the heavy chain and light chain CDR sequences of the engineered antibodies targeting MET.

**[Table 60]**

| Name | CDR | Sequence | SEQ ID NO |
|---|---|---|---|
| MEM01 | CDR-H1 | SYWLH | 236 |
| | CDR-H2 | MIDPSNSDTRFNPNFKD | 237 |
| | CDR-H3 | YRSYVTPLDY | 238 |
| | CDR-L1 | KSSQSLLYTSSQKNYLA | 239 |
| | CDR-L2 | WASTRES | 240 |
| | CDR-L3 | QQYYAYPWT | 241 |
| MEM06 | CDR-H1 | SYGFS | 584 |
| | CDR-H2 | WISASNGNTYYAQKLQG | 585 |
| | CDR-H3 | VYADYADY | 586 |
| | CDR-L1 | QQANSFPLT | 587 |
| | CDR-L2 | AASSLKS | 588 |
| | CDR-L3 | QQANSFPLT | 589 |

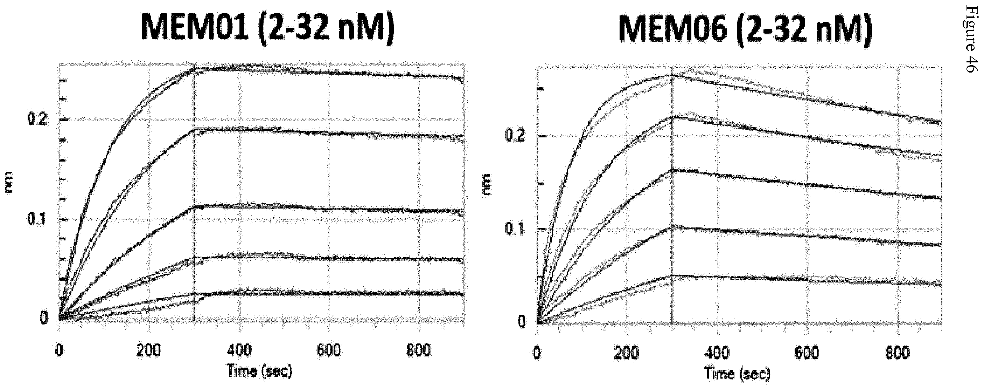

The MET protein binding constants of MEM01 and MEM06 were determined using Octet Red96e (Sartorius). In order to analyze the binding constants of the antibodies, the antibodies were loaded onto the anti-human Fab-CH1 2nd generation (FAB2G) biosensor (Sartorius, 18-5125). Then, the human MET recombinant protein (Sino Biologicals, 10692-H08H) were added in a binding reaction (300 seconds) and a dissociation reaction (600 seconds) at various concentrations (Figure 46), and the affinities of antibodies for MET was calculated (Figure 46, Table 61). Table 61 below illustrates the binding constants of the engineered antibodies targeting MET.

**[Table 61]**

| Antibodies | K_{D} (nM) | Kₐ (1/Ms) | K_{d} (1/s) |
|---|---|---|---|
| MEM01 | 0.1861 | 3.31E+05 | 6.16E-05 |
| MEM06 | 0.7881 | 4.20E+05 | 3.31E-04 |

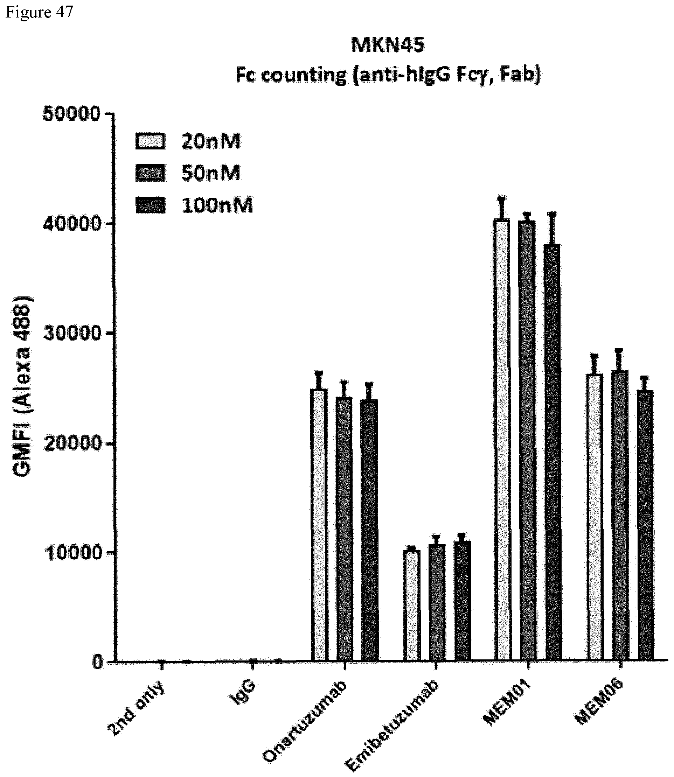

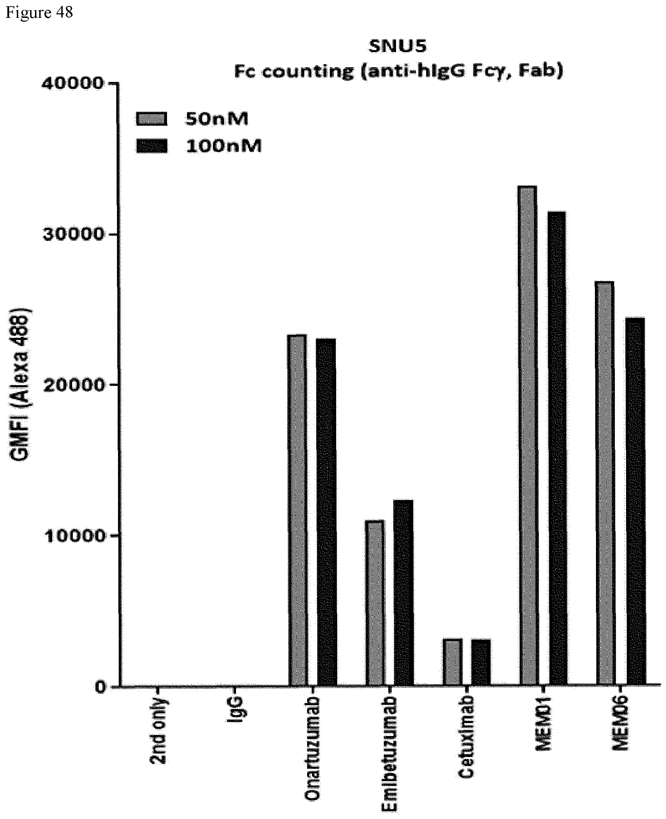

MKN45 and SNU-5 gastric cancer cell lines were used to quantify the Fc loads on the surface of MET expressing cells. The human IgG1 control, onartuzumab (produced in CHO cell line), emibetuzumab, MEM01, and MEM06 antibodies were allowed to bind to the cell lines at 4°C for 30 minutes, and the Fc loads were quantified using the Alexa 488 fluorescence-conjugated anti-human IgG Fcγ Fab antibody (Jackson ImmunoResearch, 109-547-008) (Figures 47 and 48). It was shown that high Fc loads on the surface of MET expressing cancer cells are induced by treatment of MEM01 compared to onartuzumab and emibetuzumab, which target MET (Figures 47 and 48). Treatment of MEM06 induced higher Fc loads on the surface of MET expressing cancer cells compared to emibetuzumab, but a similar level when compared to onartuzumab (Figures 47 and 48).

### Example 20. Design, preparation and analysis of antibody structure targeting EGFR

The variant light chain and heavy chain polypeptide sequences of the antibodies that specifically bind to the EGFR protein are shown in Table 62. For EGM01, expression vectors consisting of the sequences corresponding to Fc-Hole (SEQ ID NO: 7), EGM01 HC (SEQ ID NO: 590), and EGF01 LC (SEQ ID NO: 591) were co-transfected into EXPICHO-S^{™} (Gibco, A29127) (Figure 41a, Table 62), and purification and analysis were performed in the same manner as described in Example 1. Expression, purification and analysis were performed for EGM02, EGM03, EGM04, EGM05, and EGM06 in the same manner as mentioned above (Table 62).

**[Table 62]**

| Name | Sequence | SEQ ID NO |
|---|---|---|
| EGM01 HC | QVQLKQSGPGLVQPSQSLSITCTVSGFSLTNYGVHWVRQSPGKCLEW LGVIWSGGNTDYNTPFTSRLSINKDNSKSQVFFKMNSLQSNDTAIYY CARALTYYDYEFAYWGQGTLVTVSAASTKGPSVFPLAPSSKSTSGGT AALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCREEMTKNQVSLWCLVKGFY PSDIAVEWESNGQPENNYKTTPPVLDSDGSFFLYSKLTVDKSRWQQG NVFSCSVMHEALHNHYTQKSLSLSPGK | 590 |
| EGM01 LC | DILLTQSPVILSVSPGERVSFSCRASQSIGTNIHWYQQRTNGSPRLLIK YASESISGIPSRFSGSGSGTDFTLSINSVESEDIADYYCQQNNNWPTTF GCGTKLELKRTVAAPSVFIFPPSDEQLKSGTASVVCLLNNFYPREAKV QWKVDNALQSGNSQESVTEQDSKDSTYSLSSTLTLSKADYEKHKVY ACEVTHQGLSSPVTKSFNRGECGGGGSGGGGSGGGGSEPKSSDKTHT CPPCPAPELLGGPSVFLFPPKPKDTLMISRTPEVTCVVVDVSHEDPEV KFNWYVDGVEVHNAKTKPREEQYNSTYRVVSVLTVLHQDWLNGKE YKCKVSNKALPAPIEKTISKAKGQPREPQVYTLPPCREEMTKNQVSL WCLVKGFYPSDIAVEWESNGQPENNYKTTPPVLDSDGSFFLYSKLTV DKSRWQQGNVFSCSVMHEALHNHYTQKSLSLSPGK | 591 |
| EGM02 HC | QVQLQESGPGLVKPSQTLSLTCTVSGGSISSGDYYWSWIRQPPGKCLE WIGYIYYSGSTDYNPSLKSRVTMSVDTSKNQFSLKVNSVTAADTAVY YCARVSIFGVGTFDYWGQGTLVTVSSASTKGPSVFPLAPSSKSTSGGT AALGCLVKDYFPEPVTVSWNSGALTSGVHTFPAVLQSSGLYSLSSVV TVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDG VEVHNAKTKPREEQYNSTYRVVSVLTVLHQDWLNGKEYKCKVSNK ALPAPIEKTISKAKGQPREPQVYTLPPCREEMTKNQVSLWCLVKGFY PSDIAVEWESNGQPENNYKTTPPVLDSDGSFFLYSKLTVDKSRWQQG NVFSCSVMHEALHNHYTQKSLSLSPGK | 592 |
| EGM02 LC | EIVMTQSPATLSLSPGERATLSCRASQSVSSYLAWYQQKPGQAPRLLI YDASNRATGIPARFSGSGSGTDFTLTISSLEPEDFAVYYCHQYGSTPLT FGCGTKAEIKRTVAAPSVFIFPPSDEQLKSGTASVVCLLNNFYPREAK | 593 |
| | VQWKVDNALQSGNSQESVTEQDSKDSTYSLSSTLTLSKADYEKHKV YACEVTHQGLSSPVTKSFNRGECGGGGSGGGGSGGGGSEPKSSDKT HTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQV SLWCLVKGFYPSDIAVEWESNGQPENNYKTTPPVLDSDGSFFLYSKL TVDKSRWQQGNVFSCSVMHEALHNHYTQKSLSLSPGK | |
| EGM03 HC | QVQLQESGPGLVKPSETLSLTCTVSGGSVSSGDYYWTWIRQSPGKCL EWIGHIYYSGNTNYNPSLKSRLTISIDTSKTQFSLKLSSVTAADTAIYY CVRDRVTGAFDIWGQGTMVTVSSASTKGPSVFPLAPSSKSTSGGTAA LGCLVKDYFPEPVTVSWNSGALTSGVHTFPAVLQSSGLYSLSSVVTV PSSSLGTQTYICNVNHKPSNTKVDKKVEPKSCDKTHTCPPCPAPELLG GPSVFLFPPKPKDTLMISRTPEVTCVVVDVSHEDPEVKFNWYVDGVE VHNAKTKPREEQYNSTYRVVSVLTVLHQDWLNGKEYKCKVSNKAL PAPIEKTISKAKGQPREPQVYTLPPCREEMTKNQVSLWCLVKGFYPS DIAVEWESNGQPENNYKTTPPVLDSDGSFFLYSKLTVDKSRWQQGN VFSCSVMHEALHNHYTQKSLSLSPGK | 594 |
| EGM03 LC | DIQMTQSPSSLSASVGDRVTITCQASQDISNYLNWYQQKPGKAPKLLI YDASNLETGVPSRFSGSGSGTDFTFTISSLQPEDIATYFCQHFDHLPLA FGCGTKVEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGECGGGGSGGGGSGGGGSEPKSSDKT HTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQV SLWCLVKGFYPSDIAVEWESNGQPENNYKTTPPVLDSDGSFFLYSKL TVDKSRWQQGNVFSCSVMHEALHNHYTQKSLSLSPGK | 595 |
| EGM04 HC | QVQLVQSGAEVKKPGASVKVSCKASGYTFTSHWMHWVRQAPGQCL EWIGEFNPSNGRTNYNEKFKSKATMTVDTSTNTAYMELSSLRSEDTA VYYCASRDYDYDGRYFDYWGQGTLVTVSSASTKGPSVFPLAPSSKS TSGGTAALGCLVKDYFPEPVTVSWNSGALTSGVHTFPAVLQSSGLYS LSSVVTVPSSSLGTQTYICNVNHKPSNTKVDKKVEPKSCDKTHTCPPC PAPELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPCREEMTKNQVSLWCLV KGFYPSDIAVEWESNGQPENNYKTTPPVLDSDGSFFLYSKLTVDKSR WQQGNVFSCSVMHEALHNHYTQKSLSLSPGK | 596 |
| EGM04 LC | DIQMTQSPSSLSASVGDRVTITCSASSSVTYMYWYQQKPGKAPKLLI YDTSNLASGVPSRFSGSGSGTDYTFTISSLQPEDIATYYCQQWSSHIFT FGCGTKVEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGECGGGGSGGGGSGGGGSEPKSSDKT HTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQV | 597 |
| | SLWCLVKGFYPSDIAVEWESNGQPENNYKTTPPVLDSDGSFFLYSKL TVDKSRWQQGNVFSCSVMHEALHNHYTQKSLSLSPGK | |
| EGM05 HC | QVQLVQSGAEVKKPGSSVKVSCKASGFTFTDYKIHWVRQAPGQCLE WMGYFNPNSGYSTYAQKFQGRVTITADKSTSTAYMELSSLRSEDTA VYYCARLSPGGYYVMDAWGQGTTVTVSSASTKGPSVFPLAPSSKST SGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCP APELLGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPCREEMTKNQVSLWCLV KGFYPSDIAVEWESNGQPENNYKTTPPVLDSDGSFFLYSKLTVDKSR WQQGNVFSCSVMHEALHNHYTQKSLSLSPGK | 598 |
| EGM05 LC | DIQMTQSPSSLSASVGDRVTITCRASQGINNYLNWYQQKPGKAPKRL IYNTNNLQTGVPSRFSGSGSGTEFTLTISSLQPEDFATYYCLQHNSFPT FGCGTKLEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGECGGGGSGGGGSGGGGSEPKSSDKT HTCPPCPAPELLGGPSVFLFPPKPKDTLMISRTPEVTCVVVDVSHEDP EVKFNWYVDGVEVHNAKTKPREEQYNSTYRVVSVLTVLHQDWLNG KEYKCKVSNKALPAPIEKTISKAKGQPREPQVYTLPPCREEMTKNQV SLWCLVKGFYPSDIAVEWESNGQPENNYKTTPPVLDSDGSFFLYSKL TVDKSRWQQGNVFSCSVMHEALHNHYTQKSLSLSPGK | 599 |

Table 63 below shows the heavy chain and light chain nucleotide sequences of the engineered antibodies targeting EGFR.

**[Table 63]**

| Name | Sequence | SEQ ID NO |
|---|---|---|
| EGM01 HC | CAAGTACAGTTGAAACAATCAGGGCCTGGTTTGGTGCAGCCTTCC CAATCACTTAGCATCACCTGCACTGTCTCAGGGTTCAGTCTTACAA ACTACGGCGTACACTGGGTACGGCAAAGCCCTGGTAAGTGCCTGG AGTGGCTTGGGGTTATATGGTCTGGAGGGAATACCGACTATAACA CACCCTTTACCAGCAGGCTGTCCATCAATAAAGATAACTCTAAAT CCCAGGTCTTCTTTAAAATGAACTCCCTCCAGTCTAATGACACTGC CATATATTACTGTGCTAGAGCATTGACTTACTACGATTATGAGTTC GCATATTGGGGACAGGGTACTCTGGTCACCGTATCCGCTGCTAGC ACCAAAGGACCTAGTGTTTTTCCTCTTGCCCCTTCCTCAAAGTCTA CCTCTGGGGGGACAGCCGCTCTGGGCTGCCTGGTCAAGGATTATT TCCCAGAGCCTGTCACTGTCAGTTGGAACTCTGGAGCCTTGACTTC TGGTGTTCATACATTTCCTGCTGTCCTTCAAAGCAGCGGCTTGTAC TCATTGTCTTCTGTTGTGACAGTACCCTCAAGCAGCCTCGGCACTC AGACATACATCTGCAATGTCAACCACAAACCCTCAAATACAAAGG TAGATAAAAAAGTCGAACCAAAGTCTTGTGACAAAACTCACACGT GCCCACCGTGCCCAGCACCTGAACTCCTGGGGGGACCGTCAGTCT TCCTCTTCCCCCCAAAACCCAAGGACACCCTCATGATCTCCCGGA CCCCTGAGGTCACATGCGTGGTGGTGGACGTGAGCCACGAAGACC | 600 |
| | CTGAGGTCAAGTTCAACTGGTACGTGGACGGCGTGGAGGTGCATA ATGCCAAGACAAAGCCGCGGGAGGAGCAGTACAACAGCACGTAC CGTGTGGTCAGCGTCCTCACCGTCCTGCACCAGGACTGGCTGAAT GGCAAGGAGTACAAGTGCAAGGTCTCCAACAAAGCCCTCCCAGC CCCCATCGAGAAAACCATCTCCAAAGCCAAAGGGCAGCCCCGAG AACCACAGGTGTACACCCTGCCCCCATGCCGGGAGGAGATGACCA AGAACCAGGTCAGCCTGTGGTGCCTGGTCAAAGGCTTCTATCCCA GCGACATCGCCGTGGAGTGGGAGAGCAATGGGCAGCCGGAGAAC AACTACAAGACCACGCCTCCCGTGCTGGACTCCGACGGCTCCTTC TTCCTCTACAGCAAGCTCACCGTGGACAAGAGCAGGTGGCAGCAG GGGAACGTCTTCTCATGCTCCGTGATGCATGAGGCTCTGCACAAC CACTACACGCAGAAGAGCCTCTCCCTGTCTCCGGGTAAA | |
| EGM01 LC | GACATCTTGCTTACTCAATCACCTGTAATACTTTCAGTTTCACCAG GTGAACGCGTTAGCTTCTCTTGTAGAGCCTCCCAATCTATAGGTAC TAATATCCATTGGTATCAGCAGAGAACCAACGGGTCTCCTCGTTT GCTCATTAAATATGCAAGCGAATCAATCTCAGGGATTCCTAGCCG TTTTAGTGGCTCTGGCAGTGGTACTGATTTCACACTCAGCATCAAT TCTGTAGAGAGCGAAGATATTGCAGACTACTATTGCCAACAGAAC AATAATTGGCCCACAACCTTCGGGTGTGGCACAAAATTGGAACTC AAACGTACGGTGGCAGCTCCCAGCGTTTTTATCTTTCCCCCATCCG ACGAGCAGCTCAAGAGTGGCACTGCCTCTGTAGTTTGTTTGCTGA ATAACTTCTATCCACGTGAAGCAAAAGTACAGTGGAAGGTCGATA ATGCCCTTCAGAGCGGTAACAGCCAAGAAAGTGTTACCGAGCAA GATTCCAAAGATTCCACTTACAGTCTGTCCAGCACATTGACACTG AGTAAGGCTGATTACGAAAAACACAAGGTGTACGCATGCGAGGT GACACACCAAGGTCTTTCATCTCCTGTAACTAAGAGCTTTAACCG GGGAGAATGTGGTGGTGGGGGCAGCGGGGGCGGAGGTAGTGGAG GCGGCGGTAGTGAACCAAAGAGTAGTGACAAAACTCACACGTGC CCACCGTGCCCAGCACCTGAACTCCTGGGGGGACCGTCAGTCTTC CTCTTCCCCCCAAAACCCAAGGACACCCTCATGATCTCCCGGACC CCTGAGGTCACATGCGTGGTGGTGGACGTGAGCCACGAAGACCCT GAGGTCAAGTTCAACTGGTACGTGGACGGCGTGGAGGTGCATAAT GCCAAGACAAAGCCGCGGGAGGAGCAGTACAACAGCACGTACCG TGTGGTCAGCGTCCTCACCGTCCTGCACCAGGACTGGCTGAATGG CAAGGAGTACAAGTGCAAGGTCTCCAACAAAGCCCTCCCAGCCCC CATCGAGAAAACCATCTCCAAAGCCAAAGGGCAGCCCCGAGAAC CACAGGTGTACACCCTGCCCCCATGCCGGGAGGAGATGACCAAG AACCAGGTCAGCCTGTGGTGCCTGGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTTC CTCTACAGCAAGCTCACCGTGGACAAGAGCAGGTGGCAGCAGGG GAACGTCTTCTCATGCTCCGTGATGCATGAGGCTCTGCACAACCA CTACACGCAGAAGAGCCTCTCCCTGTCTCCGGGTAAA | 601 |
| EGM02 HC | CAAGTGCAGCTTCAAGAGTCTGGACCAGGGCTGGTCAAGCCCTCC CAAACCTTGAGCCTCACCTGTACTGTTTCCGGGGGCAGCATAAGT | 602 |
| | TCTGGTGATTACTACTGGAGTTGGATACGCCAACCTCCCGGAAAA TGTCTGGAGTGGATTGGGTATATCTATTATAGTGGCTCAACAGACT ACAATCCTTCTCTCAAGAGTCGGGTAACTATGAGCGTAGATACAA GTAAAAACCAATTTTCCCTTAAAGTCAATAGCGTTACAGCCGCTG ACACTGCAGTTTACTACTGTGCCCGTGTTTCAATCTTCGGTGTCGG CACTTTCGATTACTGGGGTCAGGGTACTCTGGTTACCGTGTCATCC GCTAGCACCAAAGGACCTAGTGTTTTTCCTCTTGCCCCTTCCTCAA AGTCTACCTCTGGGGGGACAGCCGCTCTGGGCTGCCTGGTCAAGG ATTATTTCCCAGAGCCTGTCACTGTCAGTTGGAACTCTGGAGCCTT GACTTCTGGTGTTCATACATTTCCTGCTGTCCTTCAAAGCAGCGGC TTGTACTCATTGTCTTCTGTTGTGACAGTACCCTCAAGCAGCCTCG GCACTCAGACATACATCTGCAATGTCAACCACAAACCCTCAAATA CAAAGGTAGATAAAAAAGTCGAACCAAAGTCTTGTGACAAAACT CACACGTGCCCACCGTGCCCAGCACCTGAACTCCTGGGGGGACCG TCAGTCTTCCTCTTCCCCCCAAAACCCAAGGACACCCTCATGATCT CCCGGACCCCTGAGGTCACATGCGTGGTGGTGGACGTGAGCCACG AAGACCCTGAGGTCAAGTTCAACTGGTACGTGGACGGCGTGGAG GTGCATAATGCCAAGACAAAGCCGCGGGAGGAGCAGTACAACAG CACGTACCGTGTGGTCAGCGTCCTCACCGTCCTGCACCAGGACTG GCTGAATGGCAAGGAGTACAAGTGCAAGGTCTCCAACAAAGCCC TCCCAGCCCCCATCGAGAAAACCATCTCCAAAGCCAAAGGGCAGC CCCGAGAACCACAGGTGTACACCCTGCCCCCATGCCGGGAGGAG ATGACCAAGAACCAGGTCAGCCTGTGGTGCCTGGTCAAAGGCTTC TATCCCAGCGACATCGCCGTGGAGTGGGAGAGCAATGGGCAGCC GGAGAACAACTACAAGACCACGCCTCCCGTGCTGGACTCCGACGG CTCCTTCTTCCTCTACAGCAAGCTCACCGTGGACAAGAGCAGGTG GCAGCAGGGGAACGTCTTCTCATGCTCCGTGATGCATGAGGCTCT GCACAACCACTACACGCAGAAGAGCCTCTCCCTGTCTCCGGGTAA A | |
| EGM02 LC | GAGATTGTTATGACCCAAAGCCCTGCAACACTTAGTTTGTCTCCA GGAGAGCGCGCCACCCTTTCTTGTCGTGCATCCCAAAGCGTTAGC AGCTATCTCGCCTGGTATCAGCAGAAACCCGGACAGGCTCCACGA TTGCTGATCTACGACGCAAGTAATAGAGCTACAGGAATACCTGCT CGTTTCTCAGGCTCTGGATCTGGCACTGATTTCACCTTGACCATAA GCAGCCTGGAGCCCGAGGATTTCGCTGTATATTATTGCCATCAAT ACGGGAGTACCCCCCTCACATTCGGTTGCGGGACTAAGGCCGAAA TTAAACGTACGGTGGCAGCTCCCAGCGTTTTTATCTTTCCCCCATC CGACGAGCAGCTCAAGAGTGGCACTGCCTCTGTAGTTTGTTTGCT GAATAACTTCTATCCACGTGAAGCAAAAGTACAGTGGAAGGTCGA TAATGCCCTTCAGAGCGGTAACAGCCAAGAAAGTGTTACCGAGCA AGATTCCAAAGATTCCACTTACAGTCTGTCCAGCACATTGACACT GAGTAAGGCTGATTACGAAAAACACAAGGTGTACGCATGCGAGG TGACACACCAAGGTCTTTCATCTCCTGTAACTAAGAGCTTTAACCG GGGAGAATGTGGTGGTGGGGGCAGCGGGGGCGGAGGTAGTGGAG GCGGCGGTAGTGAACCAAAGAGTAGTGACAAAACTCACACGTGC | 603 |
| | CCACCGTGCCCAGCACCTGAACTCCTGGGGGGACCGTCAGTCTTC CTCTTCCCCCCAAAACCCAAGGACACCCTCATGATCTCCCGGACC CCTGAGGTCACATGCGTGGTGGTGGACGTGAGCCACGAAGACCCT GAGGTCAAGTTCAACTGGTACGTGGACGGCGTGGAGGTGCATAAT GCCAAGACAAAGCCGCGGGAGGAGCAGTACAACAGCACGTACCG TGTGGTCAGCGTCCTCACCGTCCTGCACCAGGACTGGCTGAATGG CAAGGAGTACAAGTGCAAGGTCTCCAACAAAGCCCTCCCAGCCCC CATCGAGAAAACCATCTCCAAAGCCAAAGGGCAGCCCCGAGAAC CACAGGTGTACACCCTGCCCCCATGCCGGGAGGAGATGACCAAG AACCAGGTCAGCCTGTGGTGCCTGGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTTC CTCTACAGCAAGCTCACCGTGGACAAGAGCAGGTGGCAGCAGGG GAACGTCTTCTCATGCTCCGTGATGCATGAGGCTCTGCACAACCA CTACACGCAGAAGAGCCTCTCCCTGTCTCCGGGTAAA | |
| EGM03 HC | CAGGTACAACTTCAAGAAAGTGGTCCAGGTCTTGTAAAACCCTCA GAAACTTTGAGTCTCACTTGTACCGTCAGTGGCGGAAGTGTAAGT TCTGGCGATTACTACTGGACCTGGATACGCCAGTCTCCAGGCAAA TGTCTGGAGTGGATAGGCCACATCTACTACAGCGGGAACACCAAT TACAATCCATCTCTTAAATCAAGGTTGACAATTTCAATAGACACC AGTAAGACCCAGTTTTCTCTCAAACTTAGCAGTGTAACAGCAGCA GATACTGCAATCTACTACTGCGTTAGAGACCGTGTTACAGGGGCT TTCGACATCTGGGGGCAGGGAACTATGGTTACCGTCTCTTCTGCTA GCACCAAAGGACCTAGTGTTTTTCCTCTTGCCCCTTCCTCAAAGTC TACCTCTGGGGGGACAGCCGCTCTGGGCTGCCTGGTCAAGGATTA TTTCCCAGAGCCTGTCACTGTCAGTTGGAACTCTGGAGCCTTGACT TCTGGTGTTCATACATTTCCTGCTGTCCTTCAAAGCAGCGGCTTGT ACTCATTGTCTTCTGTTGTGACAGTACCCTCAAGCAGCCTCGGCAC TCAGACATACATCTGCAATGTCAACCACAAACCCTCAAATACAAA GGTAGATAAAAAAGTCGAACCAAAGTCTTGTGACAAAACTCACA CGTGCCCACCGTGCCCAGCACCTGAACTCCTGGGGGGACCGTCAG TCTTCCTCTTCCCCCCAAAACCCAAGGACACCCTCATGATCTCCCG GACCCCTGAGGTCACATGCGTGGTGGTGGACGTGAGCCACGAAG ACCCTGAGGTCAAGTTCAACTGGTACGTGGACGGCGTGGAGGTGC ATAATGCCAAGACAAAGCCGCGGGAGGAGCAGTACAACAGCACG TACCGTGTGGTCAGCGTCCTCACCGTCCTGCACCAGGACTGGCTG AATGGCAAGGAGTACAAGTGCAAGGTCTCCAACAAAGCCCTCCC AGCCCCCATCGAGAAAACCATCTCCAAAGCCAAAGGGCAGCCCC GAGAACCACAGGTGTACACCCTGCCCCCATGCCGGGAGGAGATG ACCAAGAACCAGGTCAGCCTGTGGTGCCTGGTCAAAGGCTTCTAT CCCAGCGACATCGCCGTGGAGTGGGAGAGCAATGGGCAGCCGGA GAACAACTACAAGACCACGCCTCCCGTGCTGGACTCCGACGGCTC CTTCTTCCTCTACAGCAAGCTCACCGTGGACAAGAGCAGGTGGCA GCAGGGGAACGTCTTCTCATGCTCCGTGATGCATGAGGCTCTGCA CAACCACTACACGCAGAAGAGCCTCTCCCTGTCTCCGGGTAAA | 604 |
| EGM03 LC | GACATACAGATGACACAGAGTCCTAGCTCATTGTCAGCCTCTGTC GGTGACCGCGTCACTATCACCTGCCAAGCCAGCCAAGACATATCA AATTATCTTAACTGGTACCAGCAAAAGCCTGGAAAGGCTCCAAAA CTGCTGATTTACGACGCCTCTAATTTGGAGACCGGGGTTCCCTCTA GGTTCAGCGGGTCTGGTTCAGGCACCGACTTTACATTCACTATCTC AAGTCTCCAGCCAGAGGACATCGCTACATACTTTTGCCAACATTTT GACCATCTGCCCCTGGCATTTGGGTGTGGCACCAAGGTAGAAATT AAGCGTACGGTGGCAGCTCCCAGCGTTTTTATCTTTCCCCCATCCG ACGAGCAGCTCAAGAGTGGCACTGCCTCTGTAGTTTGTTTGCTGA ATAACTTCTATCCACGTGAAGCAAAAGTACAGTGGAAGGTCGATA ATGCCCTTCAGAGCGGTAACAGCCAAGAAAGTGTTACCGAGCAA GATTCCAAAGATTCCACTTACAGTCTGTCCAGCACATTGACACTG AGTAAGGCTGATTACGAAAAACACAAGGTGTACGCATGCGAGGT GACACACCAAGGTCTTTCATCTCCTGTAACTAAGAGCTTTAACCG GGGAGAATGTGGTGGTGGGGGCAGCGGGGGCGGAGGTAGTGGAG GCGGCGGTAGTGAACCAAAGAGTAGTGACAAAACTCACACGTGC CCACCGTGCCCAGCACCTGAACTCCTGGGGGGACCGTCAGTCTTC CTCTTCCCCCCAAAACCCAAGGACACCCTCATGATCTCCCGGACC CCTGAGGTCACATGCGTGGTGGTGGACGTGAGCCACGAAGACCCT GAGGTCAAGTTCAACTGGTACGTGGACGGCGTGGAGGTGCATAAT GCCAAGACAAAGCCGCGGGAGGAGCAGTACAACAGCACGTACCG TGTGGTCAGCGTCCTCACCGTCCTGCACCAGGACTGGCTGAATGG CAAGGAGTACAAGTGCAAGGTCTCCAACAAAGCCCTCCCAGCCCC CATCGAGAAAACCATCTCCAAAGCCAAAGGGCAGCCCCGAGAAC CACAGGTGTACACCCTGCCCCCATGCCGGGAGGAGATGACCAAG AACCAGGTCAGCCTGTGGTGCCTGGTCAAAGGCTTCTATCCCAGC GACATCGCCGTGGAGTGGGAGAGCAATGGGCAGCCGGAGAACAA CTACAAGACCACGCCTCCCGTGCTGGACTCCGACGGCTCCTTCTTC CTCTACAGCAAGCTCACCGTGGACAAGAGCAGGTGGCAGCAGGG GAACGTCTTCTCATGCTCCGTGATGCATGAGGCTCTGCACAACCA CTACACGCAGAAGAGCCTCTCCCTGTCTCCGGGTAAA | 605 |
| EGM04 HC | CAAGTCCAACTGGTCCAATCTGGGGCAGAGGTCAAGAAACCTGGC GCAAGCGTAAAGGTATCCTGTAAAGCATCTGGCTACACATTCACT TCACATTGGATGCACTGGGTTCGGCAGGCACCTGGGCAATGTCTT GAATGGATTGGGGAGTTTAACCCCAGTAACGGGAGGACTAACTAC AATGAAAAGTTCAAGTCCAAAGCAACCATGACCGTCGATACCAGC ACAAACACTGCCTACATGGAACTTTCATCATTGCGATCTGAAGAC ACAGCAGTATATTACTGTGCCAGTAGGGATTACGACTACGACGGT CGCTACTTCGACTATTGGGGGCAAGGTACTTTGGTAACAGTGAGT AGTGCTAGCACCAAAGGACCTAGTGTTTTTCCTCTTGCCCCTTCCT CAAAGTCTACCTCTGGGGGGACAGCCGCTCTGGGCTGCCTGGTCA AGGATTATTTCCCAGAGCCTGTCACTGTCAGTTGGAACTCTGGAG CCTTGACTTCTGGTGTTCATACATTTCCTGCTGTCCTTCAAAGCAG CGGCTTGTACTCATTGTCTTCTGTTGTGACAGTACCCTCAAGCAGC CTCGGCACTCAGACATACATCTGCAATGTCAACCACAAACCCTCA | 606 |
| | AATACAAAGGTAGATAAAAAAGTCGAACCAAAGTCTTGTGACAA AACTCACACGTGCCCACCGTGCCCAGCACCTGAACTCCTGGGGGG ACCGTCAGTCTTCCTCTTCCCCCCAAAACCCAAGGACACCCTCATG ATCTCCCGGACCCCTGAGGTCACATGCGTGGTGGTGGACGTGAGC CACGAAGACCCTGAGGTCAAGTTCAACTGGTACGTGGACGGCGTG GAGGTGCATAATGCCAAGACAAAGCCGCGGGAGGAGCAGTACAA CAGCACGTACCGTGTGGTCAGCGTCCTCACCGTCCTGCACCAGGA CTGGCTGAATGGCAAGGAGTACAAGTGCAAGGTCTCCAACAAAG CCCTCCCAGCCCCCATCGAGAAAACCATCTCCAAAGCCAAAGGGC AGCCCCGAGAACCACAGGTGTACACCCTGCCCCCATGCCGGGAGG AGATGACCAAGAACCAGGTCAGCCTGTGGTGCCTGGTCAAAGGCT TCTATCCCAGCGACATCGCCGTGGAGTGGGAGAGCAATGGGCAGC CGGAGAACAACTACAAGACCACGCCTCCCGTGCTGGACTCCGACG GCTCCTTCTTCCTCTACAGCAAGCTCACCGTGGACAAGAGCAGGT GGCAGCAGGGGAACGTCTTCTCATGCTCCGTGATGCATGAGGCTC TGCACAACCACTACACGCAGAAGAGCCTCTCCCTGTCTCCGGGTA AA | |
| EGM04 LC | GACATACAGATGACACAATCCCCATCTAGCCTGTCCGCAAGTGTT GGGGACCGTGTCACTATAACATGCTCAGCATCATCATCAGTGACT TATATGTACTGGTACCAGCAAAAGCCCGGAAAGGCACCTAAACTG CTCATTTATGACACCAGCAATCTTGCTTCCGGGGTTCCTTCTCGAT TTTCCGGTTCTGGCAGCGGTACTGACTATACTTTTACTATCAGTTC TCTGCAACCTGAAGACATCGCAACTTACTATTGTCAACAATGGTC CAGCCACATCTTTACTTTTGGATGTGGGACAAAAGTCGAAATTAA ACGTACGGTGGCAGCTCCCAGCGTTTTTATCTTTCCCCCATCCGAC GAGCAGCTCAAGAGTGGCACTGCCTCTGTAGTTTGTTTGCTGAAT AACTTCTATCCACGTGAAGCAAAAGTACAGTGGAAGGTCGATAAT GCCCTTCAGAGCGGTAACAGCCAAGAAAGTGTTACCGAGCAAGA TTCCAAAGATTCCACTTACAGTCTGTCCAGCACATTGACACTGAGT AAGGCTGATTACGAAAAACACAAGGTGTACGCATGCGAGGTGAC ACACCAAGGTCTTTCATCTCCTGTAACTAAGAGCTTTAACCGGGG AGAATGTGGTGGTGGGGGCAGCGGGGGCGGAGGTAGTGGAGGCG GCGGTAGTGAACCAAAGAGTAGTGACAAAACTCACACGTGCCCA CCGTGCCCAGCACCTGAACTCCTGGGGGGACCGTCAGTCTTCCTC TTCCCCCCAAAACCCAAGGACACCCTCATGATCTCCCGGACCCCT GAGGTCACATGCGTGGTGGTGGACGTGAGCCACGAAGACCCTGA GGTCAAGTTCAACTGGTACGTGGACGGCGTGGAGGTGCATAATGC CAAGACAAAGCCGCGGGAGGAGCAGTACAACAGCACGTACCGTG TGGTCAGCGTCCTCACCGTCCTGCACCAGGACTGGCTGAATGGCA AGGAGTACAAGTGCAAGGTCTCCAACAAAGCCCTCCCAGCCCCCA TCGAGAAAACCATCTCCAAAGCCAAAGGGCAGCCCCGAGAACCA CAGGTGTACACCCTGCCCCCATGCCGGGAGGAGATGACCAAGAA CCAGGTCAGCCTGTGGTGCCTGGTCAAAGGCTTCTATCCCAGCGA CATCGCCGTGGAGTGGGAGAGCAATGGGCAGCCGGAGAACAACT ACAAGACCACGCCTCCCGTGCTGGACTCCGACGGCTCCTTCTTCCT | 607 |
| | CTACAGCAAGCTCACCGTGGACAAGAGCAGGTGGCAGCAGGGGA ACGTCTTCTCATGCTCCGTGATGCATGAGGCTCTGCACAACCACTA CACGCAGAAGAGCCTCTCCCTGTCTCCGGGTAAA | |
| EGM05 HC | CAAGTACAACTTGTTCAATCTGGAGCAGAGGTTAAGAAACCCGGA TCTTCCGTTAAGGTTAGTTGCAAGGCCTCCGGGTTTACTTTCACTG ACTATAAAATCCATTGGGTCCGGCAGGCCCCTGGGCAGTGTCTTG AATGGATGGGCTACTTCAACCCAAATTCTGGTTATTCCACTTATGC CCAGAAGTTTCAAGGCAGGGTCACCATCACTGCCGACAAGTCTAC TTCAACCGCCTATATGGAACTTAGCAGTCTGCGATCAGAGGATAC AGCAGTCTACTACTGCGCCAGACTGTCACCCGGCGGTTATTATGT GATGGATGCTTGGGGCCAGGGCACCACTGTTACAGTATCCTCTGC TAGCACCAAAGGACCTAGTGTTTTTCCTCTTGCCCCTTCCTCAAAG TCTACCTCTGGGGGGACAGCCGCTCTGGGCTGCCTGGTCAAGGAT TATTTCCCAGAGCCTGTCACTGTCAGTTGGAACTCTGGAGCCTTGA CTTCTGGTGTTCATACATTTCCTGCTGTCCTTCAAAGCAGCGGCTT GTACTCATTGTCTTCTGTTGTGACAGTACCCTCAAGCAGCCTCGGC ACTCAGACATACATCTGCAATGTCAACCACAAACCCTCAAATACA AAGGTAGATAAAAAAGTCGAACCAAAGTCTTGTGACAAAACTCA CACGTGCCCACCGTGCCCAGCACCTGAACTCCTGGGGGGACCGTC AGTCTTCCTCTTCCCCCCAAAACCCAAGGACACCCTCATGATCTCC CGGACCCCTGAGGTCACATGCGTGGTGGTGGACGTGAGCCACGAA GACCCTGAGGTCAAGTTCAACTGGTACGTGGACGGCGTGGAGGTG CATAATGCCAAGACAAAGCCGCGGGAGGAGCAGTACAACAGCAC GTACCGTGTGGTCAGCGTCCTCACCGTCCTGCACCAGGACTGGCT GAATGGCAAGGAGTACAAGTGCAAGGTCTCCAACAAAGCCCTCC CAGCCCCCATCGAGAAAACCATCTCCAAAGCCAAAGGGCAGCCC CGAGAACCACAGGTGTACACCCTGCCCCCATGCCGGGAGGAGAT GACCAAGAACCAGGTCAGCCTGTGGTGCCTGGTCAAAGGCTTCTA TCCCAGCGACATCGCCGTGGAGTGGGAGAGCAATGGGCAGCCGG AGAACAACTACAAGACCACGCCTCCCGTGCTGGACTCCGACGGCT CCTTCTTCCTCTACAGCAAGCTCACCGTGGACAAGAGCAGGTGGC AGCAGGGGAACGTCTTCTCATGCTCCGTGATGCATGAGGCTCTGC ACAACCACTACACGCAGAAGAGCCTCTCCCTGTCTCCGGGTAAA | 608 |
| EGM05 LC | GACATTCAGATGACTCAGTCCCCATCCTCTCTGTCTGCCAGCGTGG GAGATAGGGTCACCATAACTTGTCGGGCATCCCAAGGGATCAATA ACTACCTCAATTGGTATCAACAAAAACCTGGCAAGGCTCCTAAAA GGCTGATTTATAACACTAACAATCTCCAAACCGGGGTGCCAAGTC GCTTTAGTGGGTCAGGGAGTGGAACAGAGTTTACTCTTACTATCTC CAGCCTCCAGCCCGAGGACTTTGCCACTTACTATTGCCTCCAACAC AACTCATTTCCAACATTTGGTTGTGGCACTAAACTTGAAATTAAAC GTACGGTGGCAGCTCCCAGCGTTTTTATCTTTCCCCCATCCGACGA GCAGCTCAAGAGTGGCACTGCCTCTGTAGTTTGTTTGCTGAATAAC TTCTATCCACGTGAAGCAAAAGTACAGTGGAAGGTCGATAATGCC CTTCAGAGCGGTAACAGCCAAGAAAGTGTTACCGAGCAAGATTCC AAAGATTCCACTTACAGTCTGTCCAGCACATTGACACTGAGTAAG | 609 |
| | GCTGATTACGAAAAACACAAGGTGTACGCATGCGAGGTGACACA CCAAGGTCTTTCATCTCCTGTAACTAAGAGCTTTAACCGGGGAGA ATGTGGTGGTGGGGGCAGCGGGGGCGGAGGTAGTGGAGGCGGCG GTAGTGAACCAAAGAGTAGTGACAAAACTCACACGTGCCCACCGT GCCCAGCACCTGAACTCCTGGGGGGACCGTCAGTCTTCCTCTTCCC CCCAAAACCCAAGGACACCCTCATGATCTCCCGGACCCCTGAGGT CACATGCGTGGTGGTGGACGTGAGCCACGAAGACCCTGAGGTCA AGTTCAACTGGTACGTGGACGGCGTGGAGGTGCATAATGCCAAGA CAAAGCCGCGGGAGGAGCAGTACAACAGCACGTACCGTGTGGTC AGCGTCCTCACCGTCCTGCACCAGGACTGGCTGAATGGCAAGGAG TACAAGTGCAAGGTCTCCAACAAAGCCCTCCCAGCCCCCATCGAG AAAACCATCTCCAAAGCCAAAGGGCAGCCCCGAGAACCACAGGT GTACACCCTGCCCCCATGCCGGGAGGAGATGACCAAGAACCAGG TCAGCCTGTGGTGCCTGGTCAAAGGCTTCTATCCCAGCGACATCG CCGTGGAGTGGGAGAGCAATGGGCAGCCGGAGAACAACTACAAG ACCACGCCTCCCGTGCTGGACTCCGACGGCTCCTTCTTCCTCTACA GCAAGCTCACCGTGGACAAGAGCAGGTGGCAGCAGGGGAACGTC TTCTCATGCTCCGTGATGCATGAGGCTCTGCACAACCACTACACG CAGAAGAGCCTCTCCCTGTCTCCGGGTAAA | |

Table 64 below shows the polypeptide sequences of the heavy chain variable region (VH) and light chain variable region (VL) of the engineered antibodies targeting EGFR. Table 65 below shows the nucleotide sequences of the heavy chain variable region (VH) and light chain variable region (VL) of the engineered antibodies targeting EGFR.

**[Table 64]**

| **Name** | | **Sequence** | **SEQ ID NO** |
|---|---|---|---|
| EGM01 | VH | QVQLKQSGPGLVQPSQSLSITCTVSGFSLTNYGVHWVRQSPGKCLEWLGVIWSGGNTDYNT PFTSRLSINKDNSKSQVFFKMNSLQSNDTAIYYCARALTYYDYEFAYWGQGTLVTVSA | 610 |
| | VL | DILLTQSPVILSVSPGERVSFSCRASQSIGTNIHWYQQRTNGSPRLLIKYASESISGIPSR FSGSGSGTDFTLSINSVESEDIADYYCQQNNNWPTTFGCGTKLELK | 611 |
| EGM02 | VH | QVQLQESGPGLVKPSQTLSLTCTVSGGSISSGDYYWSWIRQPPGKCLEWIGYIYYSGSTDY NPSLKSRVTMSVDTSKNQFSLKVNSVTAADTAVYYCARVSIFGVGTFDYWGQGTLVTVSS | 612 |
| | VL | EIVMTQSPATLSLSPGERATLSCRASQSVSSYLAWYQQKPGQAPRLLIYDASNRATGIPAR FSGSGSGTDFTLTISSLEPEDFAVYYCHQYGSTPLTFGCGTKAEIK | 613 |
| EGM03 | VH | QVQLQESGPGLVKPSETLSLTCTVSGGSVSSGDYYWTWIRQSPGKCLEWIGHIYYSGNTNY NPSLKSRLTISIDTSKTQFSLKLSSVTAADTAIYYCVRDRVTGAFDIWGQGTMVTVSS | 614 |
| | VL | DIQMTQSPSSLSASVGDRVTITCQASQDISNYLNWYQQKPGKAPKLLIYDASNLETGVPSR FSGSGSGTDFTFTISSLQPEDIATYFCQHFDHLPLAFGCGTKVEIK | 615 |
| EGM04 | VH | QVQLVQSGAEVKKPGASVKVSCKASGYTFTSHWMHWVRQAPGQCLEWIGEFNPSNGRTNYN EKFKSKATMTVDTSTNTAYMELSSLRSEDTAVYYCASRDYDYDGRYFDYWGQGTLVTVSS | 616 |
| | VL | DIQMTQSPSSLSASVGDRVTITCSASSSVTYMYWYQQKPGKAPKLLIYDTSNLASGVPSRF SGSGSGTDYTFTISSLQPEDIATYYCQQWSSHIFTFGCGTKVEIK | 617 |
| EGM05 | VH | QVQLVQSGAEVKKPGSSVKVSCKASGFTFTDYKIHWVRQAPGQCLEWMGYFNPNSGYSTYA QKFQGRVTITADKSTSTAYMELSSLRSEDTAVYYCARLSPGGYYVMDAWGQGTTVTVSS | 618 |
| | VL | DIQMTQSPSSLSASVGDRVTITCRASQGINNYLNWYQQKPGKAPKRLIYNTNNLQTGVPSR FSGSGSGTEFTLTISSLQPEDFATYYCLQHNSFPTFGGGTKLEIK | 619 |

**[Table 65]**

| **Name** | | **Sequence** | **SEQ ID** NO |
|---|---|---|---|
| EGM01 | VH | CAAGTACAGTTGAAACAATCAGGGCCTGGTTTGGTGCAGCCTTCCCAATCACTTAGCATCA CCTGCACTGTCTCAGGGTTCAGTCTTACAAACTACGGCGTACACTGGGTACGGCAAAGCCC TGGTAAGTGCCTGGAGTGGCTTGGGGTTATATGGTCTGGAGGGAATACCGACTATAACACA CCCTTTACCAGCAGGCTGTCCATCAATAAAGATAACTCTAAATCCCAGGTCTTCTTTAAAA TGAACTCCCTCCAGTCTAATGACACTGCCATATATTACTGTGCTAGAGCATTGACTTACTA CGATTATGAGTTCGCATATTGGGGACAGGGTACTCTGGTCACCGTATCCGCT | 620 |
| | VL | GACATCTTGCTTACTCAATCACCTGTAATACTTTCAGTTTCACCAGGTGAACGCGTTAGCT TCTCTTGTAGAGCCTCCCAATCTATAGGTACTAATATCCATTGGTATCAGCAGAGAACCAA CGGGTCTCCTCGTTTGCTCATTAAATATGCAAGCGAATCAATCTCAGGGATTCCTAGCCGT TTTAGTGGCTCTGGCAGTGGTACTGATTTCACACTCAGCATCAATTCTGTAGAGAGCGAAG ATATTGCAGACTACTATTGCCAACAGAACAATAATTGGCCCACAACCTTCGGGTGTGGCAC AAAATTGGAACTCAAA | 621 |
| EGM02 | VH | CAAGTGCAGCTTCAAGAGTCTGGACCAGGGCTGGTCAAGCCCTCCCAAACCTTGAGCCTCA CCTGTACTGTTTCCGGGGGCAGCATAAGTTCTGGTGATTACTACTGGAGTTGGATACGCCA ACCTCCCGGAAAATGTCTGGAGTGGATTGGGTATATCTATTATAGTGGCTCAACAGACTAC AATCCTTCTCTCAAGAGTCGGGTAACTATGAGCGTAGATACAAGTAAAAACCAATTTTCCC TTAAAGTCAATAGCGTTACAGCCGCTGACACTGCAGTTTACTACTGTGCCCGTGTTTCAAT CTTCGGTGTCGGCACTTTCGATTACTGGGGTCAGGGTACTCTGGTTACCGTGTCATCC | 622 |
| | VL | GAGATTGTTATGACCCAAAGCCCTGCAACACTTAGTTTGTCTCCAGGAGAGCGCGCCACCC TTTCTTGTCGTGCATCCCAAAGCGTTAGCAGCTATCTCGCCTGGTATCAGCAGAAACCCGG ACAGGCTCCACGATTGCTGATCTACGACGCAAGTAATAGAGCTACAGGAATACCTGCTCGT TTCTCAGGCTCTGGATCTGGCACTGATTTCACCTTGACCATAAGCAGCCTGGAGCCCGAGG ATTTCGCTGTATATTATTGCCATCAATACGGGAGTACCCCCCTCACATTCGGTTGCGGGAC TAAGGCCGAAATTAAA | 623 |
| EGM03 | VH | GACATACAGATGACACAGAGTCCTAGCTCATTGTCAGCCTCTGTCGGTGACCGCGTCACTA TCACCTGCCAAGCCAGCCAAGACATATCAAATTATCTTAACTGGTACCAGCAAAAGCCTGG AAAGGCTCCAAAACTGCTGATTTACGACGCCTCTAATTTGGAGACCGGGGTTCCCTCTAGG TTCAGCGGGTCTGGTTCAGGCACCGACTTTACATTCACTATCTCAAGTCTCCAGCCAGAGG ACATCGCTACATACTTTTGCCAACATTTTGACCATCTGCCCCTGGCATTTGGGTGTGGCAC CAAGGTAGAAATTAAG | 624 |
| | VL | CAGGTACAACTTCAAGAAAGTGGTCCAGGTCTTGTAAAACCCTCAGAAACTTTGAGTCTCA CTTGTACCGTCAGTGGCGGAAGTGTAAGTTCTGGCGATTACTACTGGACCTGGATACGCCA GTCTCCAGGCAAATGTCTGGAGTGGATAGGCCACATCTACTACAGCGGGAACACCAATTAC AATCCATCTCTTAAATCAAGGTTGACAATTTCAATAGACACCAGTAAGACCCAGTTTTCTC TCAAACTTAGCAGTGTAACAGCAGCAGATACTGCAATCTACTACTGCGTTAGAGACCGTGT TACAGGGGCTTTCGACATCTGGGGGCAGGGAACTATGGTTACCGTCTCTTCT | 625 |
| EGM04 | VH | CAAGTCCAACTGGTCCAATCTGGGGCAGAGGTCAAGAAACCTGGCGCAAGCGTAAAGGTAT CCTGTAAAGCATCTGGCTACACATTCACTTCACATTGGATGCACTGGGTTCGGCAGGCACC | 626 |
| | | TGGGCAATGTCTTGAATGGATTGGGGAGTTTAACCCCAGTAACGGGAGGACTAACTACAAT GAAAAGTTCAAGTCCAAAGCAACCATGACCGTCGATACCAGCACAAACACTGCCTACATGG AACTTTCATCATTGCGATCTGAAGACACAGCAGTATATTACTGTGCCAGTAGGGATTACGA CTACGACGGTCGCTACTTCGACTATTGGGGGCAAGGTACTTTGGTAACAGTGAGTAGT | |
| | VL | GACATACAGATGACACAATCCCCATCTAGCCTGTCCGCAAGTGTTGGGGACCGTGTCACTA TAACATGCTCAGCATCATCATCAGTGACTTATATGTACTGGTACCAGCAAAAGCCCGGAAA GGCACCTAAACTGCTCATTTATGACACCAGCAATCTTGCTTCCGGGGTTCCTTCTCGATTT TCCGGTTCTGGCAGCGGTACTGACTATACTTTTACTATCAGTTCTCTGCAACCTGAAGACA TCGCAACTTACTATTGTCAACAATGGTCCAGCCACATCTTTACTTTTGGATGTGGGACAAA AGTCGAAATTAAA | 627 |
| EGM05 | VH | CAAGTACAACTTGTTCAATCTGGAGCAGAGGTTAAGAAACCCGGATCTTCCGTTAAGGTTA GTTGCAAGGCCTCCGGGTTTACTTTCACTGACTATAAAATCCATTGGGTCCGGCAGGCCCC TGGGCAGTGTCTTGAATGGATGGGCTACTTCAACCCAAATTCTGGTTATTCCACTTATGCC CAGAAGTTTCAAGGCAGGGTCACCATCACTGCCGACAAGTCTACTTCAACCGCCTATATGG AACTTAGCAGTCTGCGATCAGAGGATACAGCAGTCTACTACTGCGCCAGACTGTCACCCGG CGGTTATTATGTGATGGATGCTTGGGGCCAGGGCACCACTGTTACAGTATCCTCT | 628 |
| | VL | GACATTCAGATGACTCAGTCCCCATCCTCTCTGTCTGCCAGCGTGGGAGATAGGGTCACCA TAACTTGTCGGGCATCCCAAGGGATCAATAACTACCTCAATTGGTATCAACAAAAACCTGG CAAGGCTCCTAAAAGGCTGATTTATAACACTAACAATCTCCAAACCGGGGTGCCAAGTCGC TTTAGTGGGTCAGGGAGTGGAACAGAGTTTACTCTTACTATCTCCAGCCTCCAGCCCGAGG ACTTTGCCACTTACTATTGCCTCCAACACAACTCATTTCCAACATTTGGTTGTGGCACTAA ACTTGAAATTAAA | 629 |

Table 66 below shows the heavy chain and light chain CDR sequences of the engineered antibodies targeting EGFR.

**[Table 66]**

| Name | CDR | Sequence | SEQ ID NO |
|---|---|---|---|
| EGM01 | CDR-H1 | NYGVH | 175 |
| | CDR-H2 | VIWSGGNTDYNTPFTS | 176 |
| | CDR-H3 | ALTYYDYEFAY | 177 |
| | CDR-L1 | RASQSIGTNIH | 178 |
| | CDR-L2 | YASESIS | 179 |
| | CDR-L3 | QQNNNWPTT | 180 |
| EGM02 | CDR-H1 | DYYWS | 187 |
| | CDR-H2 | YIYYSGSTDYNPSLKS | 188 |
| | CDR-H3 | VSIFGVGTFDY | 189 |
| | CDR-L1 | RASQSVSSYLA | 190 |
| | CDR-L2 | DASNRAT | 191 |
| | CDR-L3 | HQYGSTPLT | 192 |
| EGM03 | CDR-H1 | DYYWT | 181 |
| | CDR-H2 | HIYYSGNTNYNPSLKS | 182 |
| | CDR-H3 | DRVTGAFDI | 183 |
| | CDR-L1 | QASQDISNYLN | 184 |
| | CDR-L2 | DASNLET | 185 |
| | CDR-L3 | QHFDHLPLA | 186 |
| EGM04 | CDR-H1 | SHWMH | 630 |
| | CDR-H2 | EFNPSNGRTNYNEKFKS | 631 |
| | CDR-H3 | RDYDYDGRYFDY | 632 |
| | CDR-L1 | SASSSVTYMY | 633 |
| | CDR-L2 | DTSNLAS | 634 |
| | CDR-L3 | QQWSSHIFT | 635 |
| EGM05 | CDR-H1 | DYKIH | 193 |
| | CDR-H2 | YFNPNSGYSTYAQKFQG | 194 |
| | CDR-H3 | LSPGGYYVMDA | 195 |
| | CDR-L1 | RASQGINNYLN | 196 |
| | CDR-L2 | NTNNLQT | 197 |
| | CDR-L3 | LQHNSFPT | 198 |

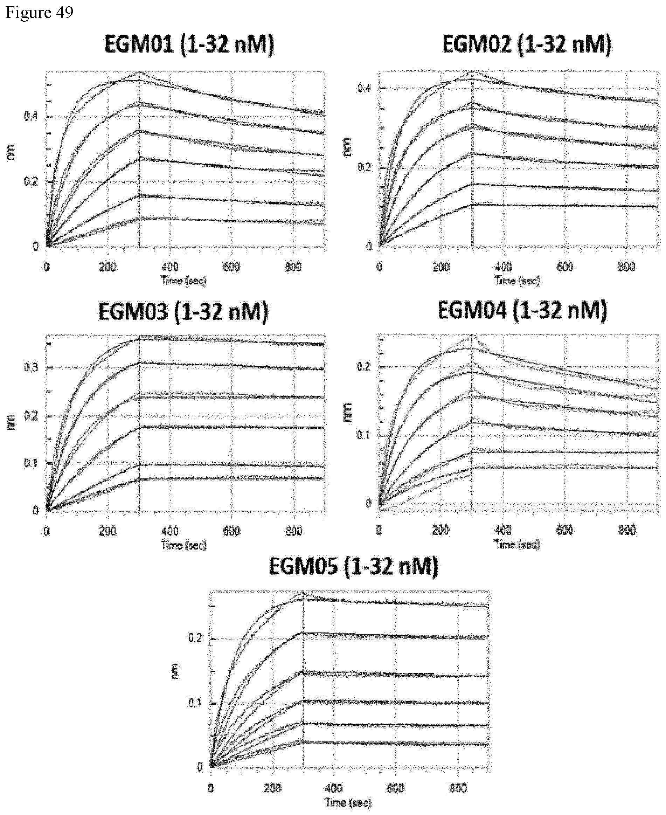

The EGFR protein binding constants of EGM01 to EGM05 were determined using the Octet Red96e (Sartorius). In order to analyze the binding constants of the antibodies, the antibodies were loaded onto the anti-human Fab-CH1 2nd generation (FAB2G) biosensor (Sartorius, 18-5125). Then, the human EGFR recombinant proteins (Sino Biologicals, 10692-H08H) were added in a binding reaction (300 seconds) and a dissociation reaction (600 seconds) at various concentrations (Figure 49), and the affinities of antibodies for EGFR were calculated (Figure 49, Table 67). Table 67 below illustrates the binding constants of the engineered antibodies targeting EGFR.

**[Table 67]**

| Antibodies | K_{D} (nM) | Kₐ (1/Ms) | K_{d} (1/s) |
|---|---|---|---|
| EGM01 | 0.6446 | 6.00E+05 | 3.87E-04 |
| EGM02 | 0.3946 | 7.30E+05 | 2.88E-04 |
| EGM03 | 0.1518 | 4.28E+05 | 6.51E-05 |
| EGM04 | 0.6421 | 6.31E+05 | 4.05E-04 |
| EGM05 | 0.1989 | 4.08E+05 | 8.11E-05 |

### Example 21. Design, preparation and analysis of novel antibody targeting CD33

The variant light chain and heavy chain polypeptide sequences of the antibodies that specifically recognize the CD33 protein are shown in Table 68. For GPM01, expression vector consisting of the sequences corresponding to Fc-Hole (SEQ ID NO: 7), 33-1 HC (SEQ ID NO: 636), and 33-1 LC (SEQ ID NO: 637) were co-transfected into EXPICHO-S^{™} (Gibco, A29127) (Figure 41a, Table 64), and purification and analysis were performed in the same manner as described in Example 1. Expression, purification and analysis were performed for 33-2, 33-3, 33-4, 33-5, 33-6, and 33-7 in the same manner as mentioned above (Figures 41a to 41b, and Tables 68 and Table 69). 33-1, 33-2, and 33-3 bind monovalently to different epitopes of the antigen and have structures consisting of two Fc domains (Figure 41a). 33-4, 33-5, 33-6, and 33-7 have structures in which the variable regions of the CD33 antibody are linked with a polypeptide linker (SEQ ID NO: 48, SEQ ID NO: 50), and bind biparatopically to CD33, and have two Fc domains (Figure 41b).

**[Table 68]**

| **Name** | **Sequence** | **SEQ ID NO** |
|---|---|---|
| 33-1 HC | EVQLVQSGAEVKKPGSSVKVSCKASGYTITDSNIHWVRQAPGQCLEWIGYIYPYNGGTD YNQKFKNRATLTVDNPTNTAYMELSSLRSEDTAFYYCVNGNPWLAYWGQGTLVTVSSAS TKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPCR EEMTKNQVSLWCLVKGFYPSDIAVEWESNGQPENNYKTTPPVLDSDGSFFLYSKLTVDK SRWQQGNVFSCSVMHEALHNHYTQKSLSLSPGK | 636 |
| 33-1 LC | DIQLTQSPSTLSASVGDRVTITCRASESLDNYGIRFLTWFQQKPGKAPKLLMYAASNQG SGVPSRFSGSGSGTEFTLTISSLQPDDFATYYCQQTKEVPWSFGQGTKVEVKRTVAAPS VFIFPPSDEQLKSGTASVVCLLNNFYPREAKVQWKVDNALQSGNSQESVTEQDSKDSTY SLSSTLTLSKADYEKHKVYACEVTHQGLSSPVTKSFNRGECGGGGSGGGGSGGGGSEPK SSDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTI SKAKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | 637 |
| 33-2 HC | QVQLVQSGAEVKKPGSSVKVSCKASGYTFTDYNMHWVRQAPGQCLEWIGYIYPYNGGTG YNQKFKSKATITADESTNTAYMELSSLRSEDTAVYYCARGRPAMDYWGQGTLVTVSSAS TKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPCR EEMTKNQVSLWCLVKGFYPSDIAVEWESNGQPENNYKTTPPVLDSDGSFFLYSKLTVDK SRWQQGNVFSCSVMHEALHNHYTQKSLSLSPGK | 638 |
| 33-2 LC | DIQMTQSPSSLSASVGDRVTITCRASESVDNYGISFMNWFQQKPGKAPKLLIYAASNQG SGVPSRFSGSGSGTDFTLTISSLQPDDFATYYCQQSKEVPWTFGQGTKVEIKRTVAAPS VFIFPPSDEQLKSGTASVVCLLNNFYPREAKVQWKVDNALQSGNSQESVTEQDSKDSTY SLSSTLTLSKADYEKHKVYACEVTHQGLSSPVTKSFNRGECGGGGSGGGGSGGGGSEPK SSDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTI SKAKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | 639 |
| 33-3 HC | QVQLQQPGAEVVKPGASVKMSCKASGYTFTSYYIHWIKQTPGQCLEWVGVIYPGNDDIS YNQKFQGKATLTADKSSTTAYMQLSSLTSEDSAVYYCAREVRLRYFDVWGQGTTVTVSS ASTKGPSVFPLAPSSKSTSGGTAALGCLVKDYFPEPVTVSWNSGALTSGVHTFPAVLQS SGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPP | 640 |
| | CREEMTKNQVSLWCLVKGFYPSDIAVEWESNGQPENNYKTTPPVLDSDGSFFLYSKLTV DKSRWQQGNVFSCSVMHEALHNHYTQKSLSLSPGK | |
| 33-3 LC | EIVLTQSPGSLAVSPGERVTMSCKSSQSVFFSSSQKNYLAWYQQIPGQSPRLLIYWAST RESGVPDRFTGSGSGTDFTLTISSVQPEDLAIYYCHQYLSSRTFGCGTKLEIKRTVAAP SVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSGGGGSEP KSSDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKT ISKAKGQPREPQVYTLPPCREEMTKNQVSLWCLVKGFYPSDIAVEWESNGQPENNYKTT PPVLDSDGSFFLYSKLTVDKSRWQQGNVFSCSVMHEALHNHYTQKSLSLSPGK | 641 |
| 33-4 HC | EVQLVQSGAEVKKPGSSVKVSCKASGYTITDSNIHWVRQAPGQSLEWIGYIYPYNGGTD YNQKFKNRATLTVDNPTNTAYMELSSLRSEDTAFYYCVNGNPWLAYWGQGTLVTVSSAS TKGPSVFPLAPQVQLVQSGAEVKKPGSSVKVSCKASGYTFTDYNMHWVRQAPGQCLEWI GYIYPYNGGTGYNQKFKSKATITADESTNTAYMELSSLRSEDTAVYYCARGRPAMDYWG QGTLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPR EPQVYTLPPCREEMTKNQVSLWCLVKGFYPSDIAVEWESNGQPENNYKTTPPVLDSDGS FFLYSKLTVDKSRWQQGNVFSCSVMHEALHNHYTQKSLSLSPGK | 642 |
| 33-4 LC | DIQLTQSPSTLSASVGDRVTITCRASESLDNYGIRFLTWFQQKPGKAPKLLMYAASNQG SGVPSRFSGSGSGTEFTLTISSLQPDDFATYYCQQTKEVPWSFGQGTKVEVKRTVAAPS VFIFPPDIQMTQSPSSLSASVGDRVTITCRASESVDNYGISFMNWFQQKPGKAPKLLIY AASNQGSGVPSRFSGSGSGTDFTLTISSLQPDDFATYYCQQSKEVPWTFGCGTKVEIKR TVAAPSVFIFPPSDEQLKSGTASVVCLLNNFYPREAKVQWKVDNALQSGNSQESVTEQD SKDSTYSLSSTLTLSKADYEKHKVYACEVTHQGLSSPVTKSFNRGECGGGGSGGGGSGG GGSEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKALPA PIEKTISKAKGQPREPQVYTLPPCREEMTKNQVSLWCLVKGFYPSDIAVEWESNGQPEN NYKTTPPVLDSDGSFFLYSKLTVDKSRWQQGNVFSCSVMHEALHNHYTQKSLSLSPGK | 643 |
| 33-5 HC | QVQLQQPGAEVVKPGASVKMSCKASGYTFTSYYIHWIKQTPGQGLEWVGVIYPGNDDIS YNQKFQGKATLTADKSSTTAYMQLSSLTSEDSAVYYCAREVRLRYFDVWGQGTTVTVSS ASTKGPSVFPLAPQVQLVQSGAEVKKPGSSVKVSCKASGYTFTDYNMHWVRQAPGQCLE WIGYIYPYNGGTGYNQKFKSKATITADESTNTAYMELSSLRSEDTAVYYCARGRPAMDY WGQGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQ PREPQVYTLPPCREEMTKNQVSLWCLVKGFYPSDIAVEWESNGQPENNYKTTPPVLDSD GSFFLYSKLTVDKSRWQQGNVFSCSVMHEALHNHYTQKSLSLSPGK | 644 |
| 33-6 LC | EIVLTQSPGSLAVSPGERVTMSCKSSQSVFFSSSQKNYLAWYQQIPGQSPRLLIYWAST RESGVPDRFTGSGSGTDFTLTISSVQPEDLAIYYCHQYLSSRTFGQGTKLEIKRTVAAP SVFIFPPDIQMTQSPSSLSASVGDRVTITCRASESVDNYGISFMNWFQQKPGKAPKLLI YAASNQGSGVPSRFSGSGSGTDFTLTISSLQPDDFATYYCQQSKEVPWTFGCGTKVEIK RTVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGECGGGGSGGGGSG GGGSEPKSSDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNKALP APIEKTISKAKGQPREPQVYTLPPCREEMTKNQVSLWCLVKGFYPSDIAVEWESNGQPE NNYKTTPPVLDSDGSFFLYSKLTVDKSRWQQGNVFSCSVMHEALHNHYTQKSLSLSPGK | 645 |
| 33-6 HC | QVQLVQSGAEVKKPGSSVKVSCKASGYTFTDYNMHWVRQAPGQGLEWIGYIYPYNGGTG YNQKFKSKATITADESTNTAYMELSSLRSEDTAVYYCARGRPAMDYWGQGTLVTVSSAS TKGPSVFPLAPEVQLVQSGAEVKKPGSSVKVSCKASGYTITDSNIHWVRQAPGQCLEWI GYIYPYNGGTDYNQKFKNRATLTVDNPTNTAYMELSSLRSEDTAFYYCVNGNPWLAYWG QGTLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPR EPQVYTLPPCREEMTKNQVSLWCLVKGFYPSDIAVEWESNGQPENNYKTTPPVLDSDGS FFLYSKLTVDKSRWQQGNVFSCSVMHEALHNHYTQKSLSLSPGK | 546 |
| 33-6 LC | DIQMTQSPSSLSASVGDRVTITCRASESVDNYGISFMNWFQQKPGKAPKLLIYAASNQG SGVPSRFSGSGSGTDFTLTISSLQPDDFATYYCQQSKEVPWTFGQGTKVEIKRTVAAPS VFIFPPSDEQLKSGTASVVCLLNNFYPREAKVQWKVDNALQSGNSQESVTEQDSKDSTY SLSSTLTLSKADYEKHKVYACEVTHQGLSSPVTKSFNRGECGGGGSGGGGSGGGGSEPK SSDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTI SKAKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | 647 |
| 33-7 HC | QVQLVQSGAEVKKPGSSVKVSCKASGYTFTDYNMHWVRQAPGQGLEWIGYIYPYNGGTG YNQKFKSKATITADESTNTAYMELSSLRSEDTAVYYCARGRPAMDYWGQGTLVTVSSAS TKGPSVFPLAPQVQLQQPGAEVVKPGASVKMSCKASGYTFTSYYIHWIKQTPGQCLEWV GVIYPGNDDISYNQKFQGKATLTADKSSTTAYMQLSSLTSEDSAVYYCAREVRLRYFDV WGQGTTVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQ PREPQVYTLPPCREEMTKNQVSLWCLVKGFYPSDIAVEWESNGQPENNYKTTPPVLDSD GSFFLYSKLTVDKSRWQQGNVFSCSVMHEALHNHYTQKSLSLSPGK | 648 |
| 33-7 LC | DIQMTQSPSSLSASVGDRVTITCRASESVDNYGISFMNWFQQKPGKAPKLLIYAASNQG SGVPSRFSGSGSGTDFTLTISSLQPDDFATYYCQQSKEVPWTFGQGTKVEIKRTVAAPS | 349 |
| | VFIFPPEIVLTQSPGSLAVSPGERVTMSCKSSQSVFFSSSQKNYLAWYQQIPGQSPRLL IYWASTRESGVPDRFTGSGSGTDFTLTISSVQPEDLAIYYCHQYLSSRTFGCGTKLEIK RTVAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGECGGGGSGGGGSG GGGSEPKSSDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNKALP APIEKTISKAKGQPREPQVYTLPPCREEMTKNQVSLWCLVKGFYPSDIAVEWESNGQPE NNYKTTPPVLDSDGSFFLYSKLTVDKSRWQQGNVFSCSVMHEALHNHYTQKSLSLSPGK | |

**[Table 69]**

| **Name** | **Sequence** | **SEQ ID NO** |
|---|---|---|
| 33-1 HC | GAGGTTCAGTTGGTTCAGTCAGGAGCAGAGGTCAAAAAACCTGGAAGCTCTGTCAAAGT TAGTTGTAAGGCCAGTGGATACACCATAACCGATTCAAATATACATTGGGTTAGGCAAG CACCAGGACAGTGCTTGGAATGGATCGGGTACATCTATCCATATAATGGGGGCACCGAT TACAACCAAAAGTTTAAGAATCGCGCCACACTCACTGTTGATAATCCAACCAATACAGC ATACATGGAGTTGAGCAGTCTTCGGTCCGAGGACACTGCTTTTTACTATTGTGTGAACG GTAACCCATGGTTGGCCTATTGGGGCCAAGGTACACTTGTAACAGTTTCATCTGCTAGC ACCAAAGGACCTAGTGTTTTTCCTCTTGCCCCTTCCTCAAAGTCTACCTCTGGGGGGAC AGCCGCTCTGGGCTGCCTGGTCAAGGATTATTTCCCAGAGCCTGTCACTGTCAGTTGGA ACTCTGGAGCCTTGACTTCTGGTGTTCATACATTTCCTGCTGTCCTTCAAAGCAGCGGC TTGTACTCATTGTCTTCTGTTGTGACAGTACCCTCAAGCAGCCTCGGCACTCAGACATA CATCTGCAATGTCAACCACAAACCCTCAAATACAAAGGTAGATAAAAAAGTCGAACCAA AGTCTTGTGACAAAACTCACACGTGCCCACCGTGCCCAGCACCTGAACTCCTGGGGGGA CCGTCAGTCTTCCTCTTCCCCCCAAAACCCAAGGACACCCTCATGATCTCCCGGACCCC TGAGGTCACATGCGTGGTGGTGGACGTGAGCCACGAAGACCCTGAGGTCAAGTTCAACT GGTACGTGGACGGCGTGGAGGTGCATAATGCCAAGACAAAGCCGCGGGAGGAGCAGTAC AACAGCACGTACCGTGTGGTCAGCGTCCTCACCGTCCTGCACCAGGACTGGCTGAATGG CAAGGAGTACAAGTGCAAGGTCTCCAACAAAGCCCTCCCAGCCCCCATCGAGAAAACCA TCTCCAAAGCCAAAGGGCAGCCCCGAGAACCACAGGTGTACACCCTGCCCCCATGCCGG GAGGAGATGACCAAGAACCAGGTCAGCCTGTGGTGCCTGGTCAAAGGCTTCTATCCCAG CGACATCGCCGTGGAGTGGGAGAGCAATGGGCAGCCGGAGAACAACTACAAGACCACGC CTCCCGTGCTGGACTCCGACGGCTCCTTCTTCCTCTACAGCAAGCTCACCGTGGACAAG AGCAGGTGGCAGCAGGGGAACGTCTTCTCATGCTCCGTGATGCATGAGGCTCTGCACAA CCACTACACGCAGAAGAGCCTCTCCCTGTCTCCGGGTAAA | 650 |
| 33-1 LC | GATATACAACTGACTCAGAGTCCCAGCACTCTCAGTGCAAGTGTAGGCGATAGAGTAAC TATAACCTGTCGCGCCTCAGAATCTCTTGATAATTATGGGATCCGATTTCTTACTTGGT TTCAGCAAAAGCCTGGTAAAGCTCCTAAATTGCTCATGTATGCCGCCAGTAATCAGGGT TCAGGAGTTCCTAGTCGTTTCTCTGGGTCAGGAAGCGGCACAGAATTTACCCTTACAAT TTCCAGCCTCCAGCCCGACGATTTCGCCACTTACTATTGCCAACAAACTAAAGAGGTTC CTTGGAGTTTTGGGTGTGGCACCAAGGTAGAAGTAAAACGTACGGTGGCAGCTCCCAGC GTTTTTATCTTTCCCCCATCCGACGAGCAGCTCAAGAGTGGCACTGCCTCTGTAGTTTG TTTGCTGAATAACTTCTATCCACGTGAAGCAAAAGTACAGTGGAAGGTCGATAATGCCC TTCAGAGCGGTAACAGCCAAGAAAGTGTTACCGAGCAAGATTCCAAAGATTCCACTTAC AGTCTGTCCAGCACATTGACACTGAGTAAGGCTGATTACGAAAAACACAAGGTGTACGC ATGCGAGGTGACACACCAAGGTCTTTCATCTCCTGTAACTAAGAGCTTTAACCGGGGAG AATGTGGTGGTGGGGGCAGCGGGGGCGGAGGTAGTGGAGGCGGCGGTAGTGAACCAAAG AGTAGTGACAAAACTCACACGTGCCCACCGTGCCCAGCACCTGAACTCCTGGGGGGACC GTCAGTCTTCCTCTTCCCCCCAAAACCCAAGGACACCCTCATGATCTCCCGGACCCCTG AGGTCACATGCGTGGTGGTGGACGTGAGCCACGAAGACCCTGAGGTCAAGTTCAACTGG | 651 |
| | TACGTGGACGGCGTGGAGGTGCATAATGCCAAGACAAAGCCGCGGGAGGAGCAGTACAA CAGCACGTACCGTGTGGTCAGCGTCCTCACCGTCCTGCACCAGGACTGGCTGAATGGCA AGGAGTACAAGTGCAAGGTCTCCAACAAAGCCCTCCCAGCCCCCATCGAGAAAACCATC TCCAAAGCCAAAGGGCAGCCCCGAGAACCACAGGTGTACACCCTGCCCCCATGCCGGGA GGAGATGACCAAGAACCAGGTCAGCCTGTGGTGCCTGGTCAAAGGCTTCTATCCCAGCG ACATCGCCGTGGAGTGGGAGAGCAATGGGCAGCCGGAGAACAACTACAAGACCACGCCT CCCGTGCTGGACTCCGACGGCTCCTTCTTCCTCTACAGCAAGCTCACCGTGGACAAGAG CAGGTGGCAGCAGGGGAACGTCTTCTCATGCTCCGTGATGCATGAGGCTCTGCACAACC ACTACACGCAGAAGAGCCTCTCCCTGTCTCCGGGTAAA | |
| 33-2 HC | CAGGTACAGCTTGTGCAATCTGGAGCTGAGGTCAAAAAACCAGGAAGTTCTGTAAAGGT GTCTTGTAAGGCATCTGGTTACACATTTACCGATTACAACATGCATTGGGTACGTCAAG CCCCTGGCCAGTGCCTGGAATGGATCGGATATATATACCCCTACAACGGTGGAACAGGT TACAACCAAAAATTCAAAAGCAAGGCTACTATCACTGCCGACGAGAGCACTAACACCGC TTACATGGAACTGTCTTCCTTGCGTTCAGAAGACACTGCCGTGTATTATTGTGCTCGGG GGCGACCCGCAATGGACTATTGGGGCCAAGGTACATTGGTGACTGTCAGTTCCGCTAGC ACCAAAGGACCTAGTGTTTTTCCTCTTGCCCCTTCCTCAAAGTCTACCTCTGGGGGGAC AGCCGCTCTGGGCTGCCTGGTCAAGGATTATTTCCCAGAGCCTGTCACTGTCAGTTGGA ACTCTGGAGCCTTGACTTCTGGTGTTCATACATTTCCTGCTGTCCTTCAAAGCAGCGGC TTGTACTCATTGTCTTCTGTTGTGACAGTACCCTCAAGCAGCCTCGGCACTCAGACATA CATCTGCAATGTCAACCACAAACCCTCAAATACAAAGGTAGATAAAAAAGTCGAACCAA AGTCTTGTGACAAAACTCACACGTGCCCACCGTGCCCAGCACCTGAACTCCTGGGGGGA CCGTCAGTCTTCCTCTTCCCCCCAAAACCCAAGGACACCCTCATGATCTCCCGGACCCC TGAGGTCACATGCGTGGTGGTGGACGTGAGCCACGAAGACCCTGAGGTCAAGTTCAACT GGTACGTGGACGGCGTGGAGGTGCATAATGCCAAGACAAAGCCGCGGGAGGAGCAGTAC AACAGCACGTACCGTGTGGTCAGCGTCCTCACCGTCCTGCACCAGGACTGGCTGAATGG CAAGGAGTACAAGTGCAAGGTCTCCAACAAAGCCCTCCCAGCCCCCATCGAGAAAACCA TCTCCAAAGCCAAAGGGCAGCCCCGAGAACCACAGGTGTACACCCTGCCCCCATGCCGG GAGGAGATGACCAAGAACCAGGTCAGCCTGTGGTGCCTGGTCAAAGGCTTCTATCCCAG CGACATCGCCGTGGAGTGGGAGAGCAATGGGCAGCCGGAGAACAACTACAAGACCACGC CTCCCGTGCTGGACTCCGACGGCTCCTTCTTCCTCTACAGCAAGCTCACCGTGGACAAG AGCAGGTGGCAGCAGGGGAACGTCTTCTCATGCTCCGTGATGCATGAGGCTCTGCACAA CCACTACACGCAGAAGAGCCTCTCCCTGTCTCCGGGTAAA | 652 |
| 33-2 LC | GACATCCAAATGACACAGTCCCCTAGCTCACTTTCAGCAAGCGTGGGGGACCGAGTTAC TATCACATGCCGCGCAAGTGAGTCTGTGGACAACTATGGAATATCATTCATGAACTGGT TCCAGCAGAAACCTGGGAAAGCACCCAAGCTGCTTATCTACGCAGCAAGTAATCAGGGT AGTGGCGTCCCTTCTCGATTCAGTGGGAGCGGTAGCGGCACCGACTTCACCCTTACCAT CTCATCACTTCAACCCGATGATTTTGCTACCTACTATTGCCAGCAATCCAAGGAAGTTC CTTGGACCTTCGGGTGTGGGACAAAGGTAGAGATTAAACGTACGGTGGCAGCTCCCAGC GTTTTTATCTTTCCCCCATCCGACGAGCAGCTCAAGAGTGGCACTGCCTCTGTAGTTTG TTTGCTGAATAACTTCTATCCACGTGAAGCAAAAGTACAGTGGAAGGTCGATAATGCCC | 653 |
| | TTCAGAGCGGTAACAGCCAAGAAAGTGTTACCGAGCAAGATTCCAAAGATTCCACTTAC AGTCTGTCCAGCACATTGACACTGAGTAAGGCTGATTACGAAAAACACAAGGTGTACGC ATGCGAGGTGACACACCAAGGTCTTTCATCTCCTGTAACTAAGAGCTTTAACCGGGGAG AATGTGGTGGTGGGGGCAGCGGGGGCGGAGGTAGTGGAGGCGGCGGTAGTGAACCAAAG AGTAGTGACAAAACTCACACGTGCCCACCGTGCCCAGCACCTGAACTCCTGGGGGGACC GTCAGTCTTCCTCTTCCCCCCAAAACCCAAGGACACCCTCATGATCTCCCGGACCCCTG AGGTCACATGCGTGGTGGTGGACGTGAGCCACGAAGACCCTGAGGTCAAGTTCAACTGG TACGTGGACGGCGTGGAGGTGCATAATGCCAAGACAAAGCCGCGGGAGGAGCAGTACAA CAGCACGTACCGTGTGGTCAGCGTCCTCACCGTCCTGCACCAGGACTGGCTGAATGGCA AGGAGTACAAGTGCAAGGTCTCCAACAAAGCCCTCCCAGCCCCCATCGAGAAAACCATC TCCAAAGCCAAAGGGCAGCCCCGAGAACCACAGGTGTACACCCTGCCCCCATGCCGGGA GGAGATGACCAAGAACCAGGTCAGCCTGTGGTGCCTGGTCAAAGGCTTCTATCCCAGCG ACATCGCCGTGGAGTGGGAGAGCAATGGGCAGCCGGAGAACAACTACAAGACCACGCCT CCCGTGCTGGACTCCGACGGCTCCTTCTTCCTCTACAGCAAGCTCACCGTGGACAAGAG CAGGTGGCAGCAGGGGAACGTCTTCTCATGCTCCGTGATGCATGAGGCTCTGCACAACC ACTACACGCAGAAGAGCCTCTCCCTGTCTCCGGGTAAA | |
| 33-3 HC | CAAGTCCAACTTCAGCAGCCTGGAGCTGAGGTAGTGAAACCCGGCGCATCTGTAAAAAT GAGCTGCAAAGCATCAGGTTACACATTTACATCCTACTACATCCATTGGATCAAGCAAA CACCAGGCCAATGTCTTGAGTGGGTTGGCGTCATTTACCCAGGAAACGATGATATATCT TACAATCAGAAATTTCAAGGGAAAGCCACACTTACAGCCGACAAGAGTTCCACAACTGC ATATATGCAACTCTCCTCCCTGACATCTGAAGACAGTGCCGTATACTATTGTGCTCGTG AAGTCAGGCTCAGATACTTTGACGTGTGGGGTCAAGGCACAACCGTCACCGTCAGTAGC GCTAGCACCAAAGGACCTAGTGTTTTTCCTCTTGCCCCTTCCTCAAAGTCTACCTCTGG GGGGACAGCCGCTCTGGGCTGCCTGGTCAAGGATTATTTCCCAGAGCCTGTCACTGTCA GTTGGAACTCTGGAGCCTTGACTTCTGGTGTTCATACATTTCCTGCTGTCCTTCAAAGC AGCGGCTTGTACTCATTGTCTTCTGTTGTGACAGTACCCTCAAGCAGCCTCGGCACTCA GACATACATCTGCAATGTCAACCACAAACCCTCAAATACAAAGGTAGATAAAAAAGTCG AACCAAAGTCTTGTGACAAAACTCACACGTGCCCACCGTGCCCAGCACCTGAACTCCTG GGGGGACCGTCAGTCTTCCTCTTCCCCCCAAAACCCAAGGACACCCTCATGATCTCCCG GACCCCTGAGGTCACATGCGTGGTGGTGGACGTGAGCCACGAAGACCCTGAGGTCAAGT TCAACTGGTACGTGGACGGCGTGGAGGTGCATAATGCCAAGACAAAGCCGCGGGAGGAG CAGTACAACAGCACGTACCGTGTGGTCAGCGTCCTCACCGTCCTGCACCAGGACTGGCT GAATGGCAAGGAGTACAAGTGCAAGGTCTCCAACAAAGCCCTCCCAGCCCCCATCGAGA AAACCATCTCCAAAGCCAAAGGGCAGCCCCGAGAACCACAGGTGTACACCCTGCCCCCA TGCCGGGAGGAGATGACCAAGAACCAGGTCAGCCTGTGGTGCCTGGTCAAAGGCTTCTA TCCCAGCGACATCGCCGTGGAGTGGGAGAGCAATGGGCAGCCGGAGAACAACTACAAGA CCACGCCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAGGCTCT GCACAACCACTACACGCAGAAGAGCCTCTCCCTGTCTCCGGGTAAA | 654 |
| 33-3 LC | GAGATAGTCCTCACCCAATCACCAGGTAGTCTGGCTGTTAGCCCAGGTGAGCGAGTTAC [?] | 655 |
| | TATGTCTTGCAAGTCCTCTCAATCTGTGTTCTTTTCATCAAGTCAGAAGAATTATCTTG CCTGGTATCAGCAGATTCCTGGTCAATCTCCCCGACTGCTTATTTACTGGGCTTCAACT CGGGAGTCAGGGGTTCCCGACCGATTCACAGGGTCTGGGTCAGGAACTGACTTTACCCT TACTATCAGCTCTGTGCAGCCTGAAGACCTCGCAATATATTATTGTCATCAGTACCTCT CTTCTCGCACTTTTGGATGTGGCACCAAATTGGAGATTAAGCGTACGGTGGCAGCTCCC AGCGTTTTTATCTTTCCCCCATCCGACGAGCAGCTCAAGAGTGGCACTGCCTCTGTAGT TTGTTTGCTGAATAACTTCTATCCACGTGAAGCAAAAGTACAGTGGAAGGTCGATAATG CCCTTCAGAGCGGTAACAGCCAAGAAAGTGTTACCGAGCAAGATTCCAAAGATTCCACT TACAGTCTGTCCAGCACATTGACACTGAGTAAGGCTGATTACGAAAAACACAAGGTGTA CGCATGCGAGGTGACACACCAAGGTCTTTCATCTCCTGTAACTAAGAGCTTTAACCGGG GAGAATGTGGTGGTGGGGGCAGCGGGGGCGGAGGTAGTGGAGGCGGCGGTAGTGAACCA AAGAGTAGTGACAAAACTCACACGTGCCCACCGTGCCCAGCACCTGAACTCCTGGGGGG ACCGTCAGTCTTCCTCTTCCCCCCAAAACCCAAGGACACCCTCATGATCTCCCGGACCC CTGAGGTCACATGCGTGGTGGTGGACGTGAGCCACGAAGACCCTGAGGTCAAGTTCAAC TGGTACGTGGACGGCGTGGAGGTGCATAATGCCAAGACAAAGCCGCGGGAGGAGCAGTA CAACAGCACGTACCGTGTGGTCAGCGTCCTCACCGTCCTGCACCAGGACTGGCTGAATG GCAAGGAGTACAAGTGCAAGGTCTCCAACAAAGCCCTCCCAGCCCCCATCGAGAAAACC ATCTCCAAAGCCAAAGGGCAGCCCCGAGAACCACAGGTGTACACCCTGCCCCCATGCCG GGAGGAGATGACCAAGAACCAGGTCAGCCTGTGGTGCCTGGTCAAAGGCTTCTATCCCA GCGACATCGCCGTGGAGTGGGAGAGCAATGGGCAGCCGGAGAACAACTACAAGACCACG CCTCCCGTGCTGGACTCCGACGGCTCCTTCTTCCTCTACAGCAAGCTCACCGTGGACAA GAGCAGGTGGCAGCAGGGGAACGTCTTCTCATGCTCCGTGATGCATGAGGCTCTGCACA ACCACTACACGCAGAAGAGCCTCTCCCTGTCTCCGGGTAAA | |
| 33-4 HC | GAGGTTCAGTTGGTTCAGTCAGGAGCAGAGGTCAAAAAACCTGGAAGCTCTGTCAAAGT TAGTTGTAAGGCCAGTGGATACACCATAACCGATTCAAATATACATTGGGTTAGGCAAG CACCAGGACAGTCCTTGGAATGGATCGGGTACATCTATCCATATAATGGGGGCACCGAT TACAACCAAAAGTTTAAGAATCGCGCCACACTCACTGTTGATAATCCAACCAATACAGC ATACATGGAGTTGAGCAGTCTTCGGTCCGAGGACACTGCTTTTTACTATTGTGTGAACG GTAACCCATGGTTGGCCTATTGGGGCCAAGGTACACTTGTAACAGTTTCATCCGCTAGC ACCAAAGGACCTAGTGTTTTTCCTCTTGCCCCTCAGGTACAGCTTGTGCAATCTGGAGC TGAGGTCAAAAAACCAGGAAGTTCTGTAAAGGTGTCTTGTAAGGCATCTGGTTACACAT TTACCGATTACAACATGCATTGGGTACGTCAAGCCCCTGGCCAGTGCCTGGAATGGATC GGATATATATACCCCTACAACGGTGGAACAGGTTACAACCAAAAATTCAAAAGCAAGGC TACTATCACTGCCGACGAGAGCACTAACACCGCTTACATGGAACTGTCTTCCTTGCGTT CAGAAGACACTGCCGTGTATTATTGTGCTCGGGGGCGACCCGCAATGGACTATTGGGGC CAAGGTACATTGGTGACTGTCAGTTCCGCTAGCACCAAAGGACCTAGTGTTTTTCCTCT TGCCCCTTCCTCAAAGTCTACCTCTGGGGGGACAGCCGCTCTGGGCTGCCTGGTCAAGG ATTATTTCCCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCTGTTGTGAC AGTACCCTCAAGCAGCCTCGGCACTCAGACATACATCTGCAATGTCAACCACAAACCCT | 666 |
| | CAAATACAAAGGTAGATAAAAAAGTCGAACCAAAGTCTTGTGACAAAACTCACACGTGC CCACCGTGCCCAGCACCTGAACTCCTGGGGGGACCGTCAGTCTTCCTCTTCCCCCCAAA ACCCAAGGACACCCTCATGATCTCCCGGACCCCTGAGGTCACATGCGTGGTGGTGGACG TGAGCCACGAAGACCCTGAGGTCAAGTTCAACTGGTACGTGGACGGCGTGGAGGTGCAT AATGCCAAGACAAAGCCGCGGGAGGAGCAGTACAACAGCACGTACCGTGTGGTCAGCGT CCTCACCGTCCTGCACCAGGACTGGCTGAATGGCAAGGAGTACAAGTGCAAGGTCTCCA ACAAAGCCCTCCCAGCCCCCATCGAGAAAACCATCTCCAAAGCCAAAGGGCAGCCCCGA GAACCACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAGAGCA ATGGGCAGCCGGAGAACAACTACAAGACCACGCCTCCCGTGCTGGACTCCGACGGCTCC TTCTTCCTCTACAGCAAGCTCACCGTGGACAAGAGCAGGTGGCAGCAGGGGAACGTCTT CTCATGCTCCGTGATGCATGAGGCTCTGCACAACCACTACACGCAGAAGAGCCTCTCCC TGTCTCCGGGTAAA | |
| 33-4 LC | GATATACAACTGACTCAGAGTCCCAGCACTCTCAGTGCAAGTGTAGGCGATAGAGTAAC TATAACCTGTCGCGCCTCAGAATCTCTTGATAATTATGGGATCCGATTTCTTACTTGGT TTCAGCAAAAGCCTGGTAAAGCTCCTAAATTGCTCATGTATGCCGCCAGTAATCAGGGT TCAGGAGTTCCTAGTCGTTTCTCTGGGTCAGGAAGCGGCACAGAATTTACCCTTACAAT TTCCAGCCTCCAGCCCGACGATTTCGCCACTTACTATTGCCAACAAACTAAAGAGGTTC CTTGGAGTTTTGGGCAAGGCACCAAGGTAGAAGTAAAACGTACGGTGGCCGCTCCCTCC GTTTTTATCTTTCCCCCAGACATCCAAATGACACAGTCCCCTAGCTCACTTTCAGCAAG CGTGGGGGACCGAGTTACTATCACATGCCGCGCAAGTGAGTCTGTGGACAACTATGGAA TATCATTCATGAACTGGTTCCAGCAGAAACCTGGGAAAGCACCCAAGCTGCTTATCTAC GCAGCAAGTAATCAGGGTAGTGGCGTCCCTTCTCGATTCAGTGGGAGCGGTAGCGGCAC CGACTTCACCCTTACCATCTCATCACTTCAACCCGATGATTTTGCTACCTACTATTGCC AGCAATCCAAGGAAGTTCCTTGGACCTTCGGGTGTGGGACAAAGGTAGAGATTAAACGT ACGGTGGCAGCTCCCAGCGTTTTTATCTTTCCCCCATCCGACGAGCAGCTCAAGAGTGG CACTGCCTCTGTAGTTTGTTTGCTGAATAACTTCTATCCACGTGAAGCAAAAGTACAGT GGAAGGTCGATAATGCCCTTCAGAGCGGTAACAGCCAAGAAAGTGTTACCGAGCAAGAT TCCAAAGATTCCACTTACAGTCTGTCCAGCACATTGACACTGAGTAAGGCTGATTACGA AAAACACAAGGTGTACGCATGCGAGGTGACACACCAAGGTCTTTCATCTCCTGTAACTA AGAGCTTTAACCGGGGAGAATGTGGTGGTGGGGGCAGCGGGGGCGGAGGTAGTGGAGGC GGCGGTAGTGAACCAAAGAGTAGTGACAAAACTCACACGTGCCCACCGTGCCCAGCACC TGAACTCCTGGGGGGACCGTCAGTCTTCCTCTTCCCCCCAAAACCCAAGGACACCCTCA TGATCTCCCGGACCCCTGAGGTCACATGCGTGGTGGTGGACGTGAGCCACGAAGACCCT GAGGTCAAGTTCAACTGGTACGTGGACGGCGTGGAGGTGCATAATGCCAAGACAAAGCC GCGGGAGGAGCAGTACAACAGCACGTACCGTGTGGTCAGCGTCCTCACCGTCCTGCACC AGGACTGGCTGAATGGCAAGGAGTACAAGTGCAAGGTCTCCAACAAAGCCCTCCCAGCC CCCATCGAGAAAACCATCTCCAAAGCCAAAGGGCAGCCCCGAGAACCACAGGTGTACAC CCTGCCCCCATGCCGGGAGGAGATGACCAAGAACCAGGTCAGCCTGTGGTGCCTGGTCA AAGGCTTCTATCCCAGCGACATCGCCGTGGAGTGGGAGAGCAATGGGCAGCCGGAGAAC | 657 |
| | AACTACAAGACCACGCCTCCCGTGCTGGACTCCGACGGCTCCTTCTTCCTCTACAGCAA GCTCACCGTGGACAAGAGCAGGTGGCAGCAGGGGAACGTCTTCTCATGCTCCGTGATGC ATGAGGCTCTGCACAACCACTACACGCAGAAGAGCCTCTCCCTGTCTCCGGGTAAA | |
| 33-5 HC | CAAGTCCAACTTCAGCAGCCTGGAGCTGAGGTAGTGAAACCCGGGGCATCTGTAAAAAT GAGCTGCAAAGCATCAGGTTACACATTTACATCCTACTACATCCATTGGATCAAGCAAA CACCAGGCCAAGGTCTTGAGTGGGTTGGCGTCATTTACCCAGGAAACGATGATATATCT TACAATCAGAAATTTCAAGGGAAAGCCACACTTACAGCCGACAAGAGTTCCACAACTGC ATATATGCAACTCTCCTCCCTGACATCTGAAGACAGTGCCGTATACTATTGTGCTCGTG AAGTCAGGCTCAGATACTTTGACGTGTGGGGTCAAGGCACAACCGTCACCGTCAGTAGC GCTAGCACCAAAGGACCTAGTGTTTTTCCTCTTGCCCCTCAGGTACAGCTTGTGCAATC TGGAGCTGAGGTCAAAAAACCAGGAAGTTCTGTAAAGGTGTCTTGTAAGGCATCTGGTT ACACATTTACCGATTACAACATGCATTGGGTACGTCAAGCCCCTGGCCAGTGCCTGGAA TGGATCGGATATATATACCCCTACAACGGTGGAACAGGTTACAACCAAAAATTCAAAAG CAAGGCTACTATCACTGCCGACGAGAGCACTAACACCGCTTACATGGAACTGTCTTCCT TGCGTTCAGAAGACACTGCCGTGTATTATTGTGCTCGGGGGCGACCCGCAATGGACTAT TGGGGCCAAGGTACATTGGTGACTGTCAGTTCCGCTAGCACCAAAGGACCTAGTGTTTT TCCTCTTGCCCCTTCCTCAAAGTCTACCTCTGGGGGGACAGCCGCTCTGGGCTGCCTGG TCAAGGATTATTTCCCAGAGCCTGTCACTGTCAGTTGGAACTCTGGAGCCTTGACTTCT GGTGTTCATACATTTCCTGCTGTCCTTCAAAGCAGCGGCTTGTACTCATTGTCTTCTGT TGTGACAGTACCCTCAAGCAGCCTCGGCACTCAGACATACATCTGCAATGTCAACCACA AACCCTCAAATACAAAGGTAGATAAAAAAGTCGAACCAAAGTCTTGTGACAAAACTCAC ACGTGCCCACCGTGCCCAGCACCTGAACTCCTGGGGGGACCGTCAGTCTTCCTCTTCCC CCCAAAACCCAAGGACACCCTCATGATCTCCCGGACCCCTGAGGTCACATGCGTGGTGG TGGACGTGAGCCACGAAGACCCTGAGGTCAAGTTCAACTGGTACGTGGACGGCGTGGAG GTGCATAATGCCAAGACAAAGCCGCGGGAGGAGCAGTACAACAGCACGTACCGTGTGGT CAGCGTCCTCACCGTCCTGCACCAGGACTGGCTGAATGGCAAGGAGTACAAGTGCAAGG TCTCCAACAAAGCCCTCCCAGCCCCCATCGAGAAAACCATCTCCAAAGCCAAAGGGCAG CCCCGAGAACCACAGGTGTACACCCTGCCCCCATGCCGGGAGGAGATGACCAAGAACCA GGTCAGCCTGTGGTGCCTGGTCAAAGGCTTCTATCCCAGCGACATCGCCGTGGAGTGGG AGAGCAATGGGCAGCCGGAGAACAACTACAAGACCACGCCTCCCGTGCTGGACTCCGAC GGCTCCTTCTTCCTCTACAGCAAGCTCACCGTGGACAAGAGCAGGTGGCAGCAGGGGAA CGTCTTCTCATGCTCCGTGATGCATGAGGCTCTGCACAACCACTACACGCAGAAGAGCC TCTCCCTGTCTCCGGGTAAA | 658 |
| 33-5 LC | GAGATAGTCCTCACCCAATCACCAGGTAGTCTGGCTGTTAGCCCAGGTGAGCGAGTTAC TATGTCTTGCAAGTCCTCTCAATCTGTGTTCTTTTCATCAAGTCAGAAGAATTATCTTG CCTGGTATCAGCAGATTCCTGGTCAATCTCCCCGACTGCTTATTTACTGGGCTTCAACT CGGGAGTCAGGGGTTCCCGACCGATTCACAGGGTCTGGGTCAGGAACTGACTTTACCCT TACTATCAGCTCTGTGCAGCCTGAAGACCTCGCAATATATTATTGTCATCAGTACCTCT CTTCTCGCACTTTTGGACAGGGCACCAAATTGGAGATTAAGCGTACGGTGGCCGCTCCC TCCGTTTTTATCTTTCCCCCAGACATCCAAATGACACAGTCCCCTAGCTCACTTTCAGC | 659 |
| | AAGCGTGGGGGACCGAGTTACTATCACATGCCGCGCAAGTGAGTCTGTGGACAACTATG GAATATCATTCATGAACTGGTTCCAGCAGAAACCTGGGAAAGCACCCAAGCTGCTTATC TACGCAGCAAGTAATCAGGGTAGTGGCGTCCCTTCTCGATTCAGTGGGAGCGGTAGCGG CACCGACTTCACCCTTACCATCTCATCACTTCAACCCGATGATTTTGCTACCTACTATT GCCAGCAATCCAAGGAAGTTCCTTGGACCTTCGGGTGTGGGACAAAGGTAGAGATTAAA CGTACGGTGGCAGCTCCCAGCGTTTTTATCTTTCCCCCATCCGACGAGCAGCTCAAGAG TGGCACTGCCTCTGTAGTTTGTTTGCTGAATAACTTCTATCCACGTGAAGCAAAAGTAC AGTGGAAGGTCGATAATGCCCTTCAGAGCGGTAACAGCCAAGAAAGTGTTACCGAGCAA GATTCCAAAGATTCCACTTACAGTCTGTCCAGCACATTGACACTGAGTAAGGCTGATTA CGAAAAACACAAGGTGTACGCATGCGAGGTGACACACCAAGGTCTTTCATCTCCTGTAA CTAAGAGCTTTAACCGGGGAGAATGTGGTGGTGGGGGCAGCGGGGGCGGAGGTAGTGGA GGCGGCGGTAGTGAACCAAAGAGTAGTGACAAAACTCACACGTGCCCACCGTGCCCAGC ACCTGAACTCCTGGGGGGACCGTCAGTCTTCCTCTTCCCCCCAAAACCCAAGGACACCC TCATGATCTCCCGGACCCCTGAGGTCACATGCGTGGTGGTGGACGTGAGCCACGAAGAC CCTGAGGTCAAGTTCAACTGGTACGTGGACGGCGTGGAGGTGCATAATGCCAAGACAAA GCCGCGGGAGGAGCAGTACAACAGCACGTACCGTGTGGTCAGCGTCCTCACCGTCCTGC ACCAGGACTGGCTGAATGGCAAGGAGTACAAGTGCAAGGTCTCCAACAAAGCCCTCCCA GCCCCCATCGAGAAAACCATCTCCAAAGCCAAAGGGCAGCCCCGAGAACCACAGGTGTA CACCCTGCCCCCATGCCGGGAGGAGATGACCAAGAACCAGGTCAGCCTGTGGTGCCTGG TCAAAGGCTTCTATCCCAGCGACATCGCCGTGGAGTGGGAGAGCAATGGGCAGCCGGAG AACAACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCCGTGA TGCATGAGGCTCTGCACAACCACTACACGCAGAAGAGCCTCTCCCTGTCTCCGGGTAAA | |
| 33-6 HC | CAGGTACAGCTTGTGCAATCTGGAGCTGAGGTCAAAAAACCAGGAAGTTCTGTAAAGGT GTCTTGTAAGGCATCTGGTTACACATTTACCGATTACAACATGCATTGGGTACGTCAAG CCCCTGGCCAGGGCCTGGAATGGATCGGATATATATACCCCTACAACGGTGGAACAGGT TACAACCAAAAATTCAAAAGCAAGGCTACTATCACTGCCGACGAGAGCACTAACACCGC TTACATGGAACTGTCTTCCTTGCGTTCAGAAGACACTGCCGTGTATTATTGTGCTCGGG GGCGACCCGCAATGGACTATTGGGGCCAAGGTACATTGGTGACTGTCAGTTCCGCTAGC ACCAAAGGACCTAGTGTTTTTCCTCTTGCCCCTGAGGTTCAGTTGGTTCAGTCAGGAGC AGAGGTCAAAAAACCTGGAAGCTCTGTCAAAGTTAGTTGTAAGGCCAGTGGATACACCA TAACCGATTCAAATATACATTGGGTTAGGCAAGCACCAGGACAGTGCTTGGAATGGATC GGGTACATCTATCCATATAATGGGGGCACCGATTACAACCAAAAGTTTAAGAATCGCGC CACACTCACTGTTGATAATCCAACCAATACAGCATACATGGAGTTGAGCAGTCTTCGGT CCGAGGACACTGCTTTTTACTATTGTGTGAACGGTAACCCATGGTTGGCCTATTGGGGC CAAGGTACACTTGTAACAGTTTCATCTGCTAGCACCAAAGGACCTAGTGTTTTTCCTCT TGCCCCTTCCTCAAAGTCTACCTCTGGGGGGACAGCCGCTCTGGGCTGCCTGGTCAAGG ATTATTTCCCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCTGTTGTGAC AGTACCCTCAAGCAGCCTCGGCACTCAGACATACATCTGCAATGTCAACCACAAACCCT | 660 |
| | CAAATACAAAGGTAGATAAAAAAGTCGAACCAAAGTCTTGTGACAAAACTCACACGTGC CCACCGTGCCCAGCACCTGAACTCCTGGGGGGACCGTCAGTCTTCCTCTTCCCCCCAAA ACCCAAGGACACCCTCATGATCTCCCGGACCCCTGAGGTCACATGCGTGGTGGTGGACG TGAGCCACGAAGACCCTGAGGTCAAGTTCAACTGGTACGTGGACGGCGTGGAGGTGCAT AATGCCAAGACAAAGCCGCGGGAGGAGCAGTACAACAGCACGTACCGTGTGGTCAGCGT CCTCACCGTCCTGCACCAGGACTGGCTGAATGGCAAGGAGTACAAGTGCAAGGTCTCCA ACAAAGCCCTCCCAGCCCCCATCGAGAAAACCATCTCCAAAGCCAAAGGGCAGCCCCGA GAACCACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAGAGCA ATGGGCAGCCGGAGAACAACTACAAGACCACGCCTCCCGTGCTGGACTCCGACGGCTCC TTCTTCCTCTACAGCAAGCTCACCGTGGACAAGAGCAGGTGGCAGCAGGGGAACGTCTT CTCATGCTCCGTGATGCATGAGGCTCTGCACAACCACTACACGCAGAAGAGCCTCTCCC TGTCTCCGGGTAAA | |
| 33-6 LC | GACATCCAAATGACACAGTCCCCTAGCTCACTTTCAGCAAGCGTGGGGGACCGAGTTAC TATCACATGCCGCGCAAGTGAGTCTGTGGACAACTATGGAATATCATTCATGAACTGGT TCCAGCAGAAACCTGGGAAAGCACCCAAGCTGCTTATCTACGCAGCAAGTAATCAGGGT AGTGGCGTCCCTTCTCGATTCAGTGGGAGCGGTAGCGGCACCGACTTCACCCTTACCAT CTCATCACTTCAACCCGATGATTTTGCTACCTACTATTGCCAGCAATCCAAGGAAGTTC CTTGGACCTTCGGGCAAGGGACAAAGGTAGAGATTAAACGTACGGTGGCAGCTCCCAGC GTTTTTATCTTTCCCCCATCCGACGAGCAGCTCAAGAGTGGCACTGCCTCTGTAGTTTG TTTGCTGAATAACTTCTATCCACGTGAAGCAAAAGTACAGTGGAAGGTCGATAATGCCC TTCAGAGCGGTAACAGCCAAGAAAGTGTTACCGAGCAAGATTCCAAAGATTCCACTTAC AGTCTGTCCAGCACATTGACACTGAGTAAGGCTGATTACGAAAAACACAAGGTGTACGC ATGCGAGGTGACACACCAAGGTCTTTCATCTCCTGTAACTAAGAGCTTTAACCGGGGAG AATGTGGTGGTGGGGGCAGCGGGGGCGGAGGTAGTGGAGGCGGCGGTAGTGAACCAAAG AGTAGTGACAAAACTCACACGTGCCCACCGTGCCCAGCACCTGAACTCCTGGGGGGACC GTCAGTCTTCCTCTTCCCCCCAAAACCCAAGGACACCCTCATGATCTCCCGGACCCCTG AGGTCACATGCGTGGTGGTGGACGTGAGCCACGAAGACCCTGAGGTCAAGTTCAACTGG TACGTGGACGGCGTGGAGGTGCATAATGCCAAGACAAAGCCGCGGGAGGAGCAGTACAA CAGCACGTACCGTGTGGTCAGCGTCCTCACCGTCCTGCACCAGGACTGGCTGAATGGCA AGGAGTACAAGTGCAAGGTCTCCAACAAAGCCCTCCCAGCCCCCATCGAGAAAACCATC TCCAAAGCCAAAGGGCAGCCCCGAGAACCACAGGTGTACACCCTGCCCCCATGCCGGGA GGAGATGACCAAGAACCAGGTCAGCCTGTGGTGCCTGGTCAAAGGCTTCTATCCCAGCG ACATCGCCGTGGAGTGGGAGAGCAATGGGCAGCCGGAGAACAACTACAAGACCACGCCT CCCGTGCTGGACTCCGACGGCTCCTTCTTCCTCTACAGCAAGCTCACCGTGGACAAGAG CAGGTGGCAGCAGGGGAACGTCTTCTCATGCTCCGTGATGCATGAGGCTCTGCACAACC ACTACACGCAGAAGAGCCTCTCCCTGTCTCCGGGTAAA | 661 |
| 33-7 HC | CAGGTACAGCTTGTGCAATCTGGAGCTGAGGTCAAAAAACCAGGAAGTTCTGTAAAGGT GTCTTGTAAGGCATCTGGTTACACATTTACCGATTACAACATGCATTGGGTACGTCAAG CCCCTGGCCAGGGCCTGGAATGGATCGGATATATATACCCCTACAACGGTGGAACAGGT | 662 |
| | TACAACCAAAAATTCAAAAGCAAGGCTACTATCACTGCCGACGAGAGCACTAACACCGC TTACATGGAACTGTCTTCCTTGCGTTCAGAAGACACTGCCGTGTATTATTGTGCTCGGG GGCGACCCGCAATGGACTATTGGGGCCAAGGTACATTGGTGACTGTCAGTTCCGCTAGC ACCAAAGGACCTAGTGTTTTTCCTCTTGCCCCTCAAGTCCAACTTCAGCAGCCTGGAGC TGAGGTAGTGAAACCCGGCGCATCTGTAAAAATGAGCTGCAAAGCATCAGGTTACACAT TTACATCCTACTACATCCATTGGATCAAGCAAACACCAGGCCAATGTCTTGAGTGGGTT GGCGTCATTTACCCAGGAAACGATGATATATCTTACAATCAGAAATTTCAAGGGAAAGC CACACTTACAGCCGACAAGAGTTCCACAACTGCATATATGCAACTCTCCTCCCTGACAT CTGAAGACAGTGCCGTATACTATTGTGCTCGTGAAGTCAGGCTCAGATACTTTGACGTG TGGGGTCAAGGCACAACCGTCACCGTCAGTAGCGCTAGCACCAAAGGACCTAGTGTTTT TCCTCTTGCCCCTTCCTCAAAGTCTACCTCTGGGGGGACAGCCGCTCTGGGCTGCCTGG TCAAGGATTATTTCCCAGAGCCTGTCACTGTCAGTTGGAACTCTGGAGCCTTGACTTCT GGTGTTCATACATTTCCTGCTGTCCTTCAAAGCAGCGGCTTGTACTCATTGTCTTCTGT TGTGACAGTACCCTCAAGCAGCCTCGGCACTCAGACATACATCTGCAATGTCAACCACA AACCCTCAAATACAAAGGTAGATAAAAAAGTCGAACCAAAGTCTTGTGACAAAACTCAC ACGTGCCCACCGTGCCCAGCACCTGAACTCCTGGGGGGACCGTCAGTCTTCCTCTTCCC CCCAAAACCCAAGGACACCCTCATGATCTCCCGGACCCCTGAGGTCACATGCGTGGTGG TGGACGTGAGCCACGAAGACCCTGAGGTCAAGTTCAACTGGTACGTGGACGGCGTGGAG GTGCATAATGCCAAGACAAAGCCGCGGGAGGAGCAGTACAACAGCACGTACCGTGTGGT CAGCGTCCTCACCGTCCTGCACCAGGACTGGCTGAATGGCAAGGAGTACAAGTGCAAGG TCTCCAACAAAGCCCTCCCAGCCCCCATCGAGAAAACCATCTCCAAAGCCAAAGGGCAG CCCCGAGAACCACAGGTGTACACCCTGCCCCCATGCCGGGAGGAGATGACCAAGAACCA GGTCAGCCTGTGGTGCCTGGTCAAAGGCTTCTATCCCAGCGACATCGCCGTGGAGTGGG AGAGCAATGGGCAGCCGGAGAACAACTACAAGACCACGCCTCCCGTGCTGGACTCCGAC GGCTCCTTCTTCCTCTACAGCAAGCTCACCGTGGACAAGAGCAGGTGGCAGCAGGGGAA CGTCTTCTCATGCTCCGTGATGCATGAGGCTCTGCACAACCACTACACGCAGAAGAGCC TCTCCCTGTCTCCGGGTAAA | |
| 33-7 LC | GACATCCAAATGACACAGTCCCCTAGCTCACTTTCAGCAAGCGTGGGGGACCGAGTTAC TATCACATGCCGCGCAAGTGAGTCTGTGGACAACTATGGAATATCATTCATGAACTGGT TCCAGCAGAAACCTGGGAAAGCACCCAAGCTGCTTATCTACGCAGCAAGTAATCAGGGT AGTGGCGTCCCTTCTCGATTCAGTGGGAGCGGTAGCGGCACCGACTTCACCCTTACCAT CTCATCACTTCAACCCGATGATTTTGCTACCTACTATTGCCAGCAATCCAAGGAAGTTC CTTGGACCTTCGGGCAAGGGACAAAGGTAGAGATTAAACGTACGGTGGCCGCTCCCTCC GTTTTTATCTTTCCCCCAGAGATAGTCCTCACCCAATCACCAGGTAGTCTGGCTGTTAG CCCAGGTGAGCGAGTTACTATGTCTTGCAAGTCCTCTCAATCTGTGTTCTTTTCATCAA GTCAGAAGAATTATCTTGCCTGGTATCAGCAGATTCCTGGTCAATCTCCCCGACTGCTT ATTTACTGGGCTTCAACTCGGGAGTCAGGGGTTCCCGACCGATTCACAGGGTCTGGGTC AGGAACTGACTTTACCCTTACTATCAGCTCTGTGCAGCCTGAAGACCTCGCAATATATT ATTGTCATCAGTACCTCTCTTCTCGCACTTTTGGATGTGGCACCAAATTGGAGATTAAG CGTACGGTGGCAGCTCCCAGCGTTTTTATCTTTCCCCCATCCGACGAGCAGCTCAAGAG | 663 |
| | TGGCACTGCCTCTGTAGTTTGTTTGCTGAATAACTTCTATCCACGTGAAGCAAAAGTAC AGTGGAAGGTCGATAATGCCCTTCAGAGCGGTAACAGCCAAGAAAGTGTTACCGAGCAA GATTCCAAAGATTCCACTTACAGTCTGTCCAGCACATTGACACTGAGTAAGGCTGATTA CGAAAAACACAAGGTGTACGCATGCGAGGTGACACACCAAGGTCTTTCATCTCCTGTAA CTAAGAGCTTTAACCGGGGAGAATGTGGTGGTGGGGGCAGCGGGGGCGGAGGTAGTGGA GGCGGCGGTAGTGAACCAAAGAGTAGTGACAAAACTCACACGTGCCCACCGTGCCCAGC ACCTGAACTCCTGGGGGGACCGTCAGTCTTCCTCTTCCCCCCAAAACCCAAGGACACCC TCATGATCTCCCGGACCCCTGAGGTCACATGCGTGGTGGTGGACGTGAGCCACGAAGAC CCTGAGGTCAAGTTCAACTGGTACGTGGACGGCGTGGAGGTGCATAATGCCAAGACAAA GCCGCGGGAGGAGCAGTACAACAGCACGTACCGTGTGGTCAGCGTCCTCACCGTCCTGC ACCAGGACTGGCTGAATGGCAAGGAGTACAAGTGCAAGGTCTCCAACAAAGCCCTCCCA GCCCCCATCGAGAAAACCATCTCCAAAGCCAAAGGGCAGCCCCGAGAACCACAGGTGTA CACCCTGCCCCCATGCCGGGAGGAGATGACCAAGAACCAGGTCAGCCTGTGGTGCCTGG TCAAAGGCTTCTATCCCAGCGACATCGCCGTGGAGTGGGAGAGCAATGGGCAGCCGGAG AACAACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCCGTGA TGCATGAGGCTCTGCACAACCACTACACGCAGAAGAGCCTCTCCCTGTCTCCGGGTAAA | |

Table 70 below shows the polypeptide sequences of the heavy chain variable region (VH) and light chain variable region (VL) of the engineered antibodies targeting CD33. Table 71 below shows the nucleotide sequences of the heavy chain variable region (VH) and light chain variable region (VL) of the engineered antibodies targeting CD33.

**[Table 70]**

| **Name** | | **Sequence** | **SEQ ID NO** |
|---|---|---|---|
| 33-1 | VH | EVQLVQSGAEVKKPGSSVKVSCKASGYTITDSNIHWVRQAPGQCLEWIGYIYPYNGGTDYN QKFKNRATLTVDNPTNTAYMELSSLRSEDTAFYYCVNGNPWLAYWGQGTLVTVSS | 664 |
| | VL | DIQLTQSPSTLSASVGDRVTITCRASESLDNYGIRFLTWFQQKPGKAPKLLMYAASNQGSG VPSRFSGSGSGTEFTLTISSLQPDDFATYYCQQTKEVPWSFGCGTKVEVK | 665 |
| 33-2 | VH | QVQLVQSGAEVKKPGSSVKVSCKASGYTFTDYNMHWVRQAPGQCLEWIGYIYPYNGGTGYN QKFKSKATITADESTNTAYMELSSLRSEDTAVYYCARGRPAMDYWGQGTLVTVSS | 666 |
| | VL | DIQMTQSPSSLSASVGDRVTITCRASESVDNYGISFMNWFQQKPGKAPKLLIYAASNQGSG VPSRFSGSGSGTDFTLTISSLQPDDFATYYCQQSKEVPWTFGCGTKVEIK | 667 |
| 33-3 | VH | QVQLQQPGAEVVKPGASVKMSCKASGYTFTSYYIHWIKQTPGQCLEWVGVIYPGNDDISYN QKFQGKATLTADKSSTTAYMQLSSLTSEDSAVYYCAREVRLRYFDVWGQGTTVTVSS | 668 |
| | VL | EIVLTQSPGSLAVSPGERVTMSCKSSQSVFFSSSQKNYLAWYQQIPGQSPRLLIYWASTRE SGVPDRFTGSGSGTDFTLTISSVQPEDLAIYYCHQYLSSRTFGCGTKLEIK | 669 |
| 33-4 | VH1 | | 670 |
| | VH2 | QVQLVQSGAEVKKPGSSVKVSCKASGYTFTDYNMHWVRQAPGQCLEWIGYIYPYNGGTGYN QKFKSKATITADESTNTAYMELSSLRSEDTAVYYCARGRPAMDYWGQGTLVTVSS | 666 |
| | VL1 | | 671 |
| | VL2 | DIQMTQSPSSLSASVGDRVTITCRASESVDNYGISFMNWFQQKPGKAPKLLIYAASNQGSG VPSRFSGSGSGTDFTLTISSLQPDDFATYYCQQSKEVPWTFGCGTKVEIK | 667 |
| 33-5 | VH1 | QVQLQQPGAEVVKPGASVKMSCKASGYTFTSYYIHWIKQTPGQGLEWVGVIYPGNDDISYN QKFQGKATLTADKSSTTAYMQLSSLTSEDSAVYYCAREVRLRYFDVWGQGTTVTVSS | 672 |
| | VH2 | QVQLVQSGAEVKKPGSSVKVSCKASGYTFTDYNMHWVRQAPGQCLEWIGYIYPYNGGTGYN QKFKSKATITADESTNTAYMELSSLRSEDTAVYYCARGRPAMDYWGQGTLVTVSS | 666 |
| | VL1 | EIVLTQSPGSLAVSPGERVTMSCKSSQSVFFSSSQKNYLAWYQQIPGQSPRLLIYWASTRE SGVPDRFTGSGSGTDFTLTISSVQPEDLAIYYCHQYLSSRTFGQGTKLEIK | 673 |
| | VL2 | DIQMTQSPSSLSASVGDRVTITCRASESVDNYGISFMNWFQQKPGKAPKLLIYAASNQGSG VPSRFSGSGSGTDFTLTISSLQPDDFATYYCQQSKEVPWTFGQGTKVEIK | 667 |
| 33-6 | VH1 | QVQLVQSGAEVKKPGSSVKVSCKASGYTFTDYNMHWVRQAPGQGLEWIGYIYPYNGGTGYN QKFKSKATITADESTNTAYMELSSLRSEDTAVYYCARGRPAMDYWGQGTLVTVSS | 674 |
| | VH2 | EVQLVQSGAEVKKPGSSVKVSCKASGYTITDSNIHWVRQAPGQCLEWIGYIYPYNGGTDYN QKFKNRATLTVDNPTNTAYMELSSLRSEDTAFYYCVNGNPWLAYWGQGTLVTVSS | 664 |
| | VL1 | DIQMTQSPSSLSASVGDRVTITCRASESVDNYGISFMNWFQQKPGKAPKLLIYAASNQGSG VPSRFSGSGSGTDFTLTISSLQPDDFATYYCQQSKEVPWTFGQGTKVEIK | 675 |
| | VL2 | DIQLTQSPSTLSASVGDRVTITCRASESLDNYGIRFLTWFQQKPGKAPKLLMYAASNQGSG VPSRFSGSGSGTEFTLTISSLQPDDFATYYCQQTKEVPWSFGCGTKVEVK | 665 |
| 33-7 | VH1 | QVQLVQSGAEVKKPGSSVKVSCKASGYTFTDYNMHWVRQAPGQGLEWIGYIYPYNGGTGYN QKFKSKATITADESTNTAYMELSSLRSEDTAVYYCARGRPAMDYWGQGTLVTVSS | 674 |
| | VH2 | QVQLQQPGAEVVKPGASVKMSCKASGYTFTSYYIHWIKQTPGQCLEWVGVIYPGNDDISYN QKFQGKATLTADKSSTTAYMQLSSLTSEDSAVYYCAREVRLRYFDVWGQGTTVTVSS | 668 |
| | VL1 | DIQMTQSPSSLSASVGDRVTITCRASESVDNYGISFMNWFQQKPGKAPKLLIYAASNQGSG VPSRFSGSGSGTDFTLTISSLQPDDFATYYCQQSKEVPWTFGQGTKVEIK | 675 |
| | VL2 | EIVLTQSPGSLAVSPGERVTMSCKSSQSVFFSSSQKNYLAWYQQIPGQSPRLLIYWASTRE SGVPDRFTGSGSGTDFTLTISSVQPEDLAIYYCHQYLSSRTFGCGTKLEIK | 669 |

**[Table 71]**

| **Name** | | **Sequence** | **SEQ ID NO** |
|---|---|---|---|
| 33-1 | VH | GAGGTTCAGTTGGTTCAGTCAGGAGCAGAGGTCAAAAAACCTGGAAGCTCTGTCAAAGTTA GTTGTAAGGCCAGTGGATACACCATAACCGATTCAAATATACATTGGGTTAGGCAAGCACC AGGACAGTGCTTGGAATGGATCGGGTACATCTATCCATATAATGGGGGCACCGATTACAAC CAAAAGTTTAAGAATCGCGCCACACTCACTGTTGATAATCCAACCAATACAGCATACATGG AGTTGAGCAGTCTTCGGTCCGAGGACACTGCTTTTTACTATTGTGTGAACGGTAACCCATG GTTGGCCTATTGGGGCCAAGGTACACTTGTAACAGTTTCATCT | 676 |
| | VL | GATATACAACTGACTCAGAGTCCCAGCACTCTCAGTGCAAGTGTAGGCGATAGAGTAACTA TAACCTGTCGCGCCTCAGAATCTCTTGATAATTATGGGATCCGATTTCTTACTTGGTTTCA GCAAAAGCCTGGTAAAGCTCCTAAATTGCTCATGTATGCCGCCAGTAATCAGGGTTCAGGA GTTCCTAGTCGTTTCTCTGGGTCAGGAAGCGGCACAGAATTTACCCTTACAATTTCCAGCC TCCAGCCCGACGATTTCGCCACTTACTATTGCCAACAAACTAAAGAGGTTCCTTGGAGTTT TGGGTGTGGCACCAAGGTAGAAGTAAAA | 677 |
| 33-2 | VH | CAGGTACAGCTTGTGCAATCTGGAGCTGAGGTCAAAAAACCAGGAAGTTCTGTAAAGGTGT CTTGTAAGGCATCTGGTTACACATTTACCGATTACAACATGCATTGGGTACGTCAAGCCCC TGGCCAGTGCCTGGAATGGATCGGATATATATACCCCTACAACGGTGGAACAGGTTACAAC CAAAAATTCAAAAGCAAGGCTACTATCACTGCCGACGAGAGCACTAACACCGCTTACATGG AACTGTCTTCCTTGCGTTCAGAAGACACTGCCGTGTATTATTGTGCTCGGGGGCGACCCGC AATGGACTATTGGGGCCAAGGTACATTGGTGACTGTCAGTTCC | 678 |
| | VL | GACATCCAAATGACACAGTCCCCTAGCTCACTTTCAGCAAGCGTGGGGGACCGAGTTACTA TCACATGCCGCGCAAGTGAGTCTGTGGACAACTATGGAATATCATTCATGAACTGGTTCCA GCAGAAACCTGGGAAAGCACCCAAGCTGCTTATCTACGCAGCAAGTAATCAGGGTAGTGGC GTCCCTTCTCGATTCAGTGGGAGCGGTAGCGGCACCGACTTCACCCTTACCATCTCATCAC TTCAACCCGATGATTTTGCTACCTACTATTGCCAGCAATCCAAGGAAGTTCCTTGGACCTT CGGGTGTGGGACAAAGGTAGAGATTAAA | 679 |
| 33-3 | VH | CAAGTCCAACTTCAGCAGCCTGGAGCTGAGGTAGTGAAACCCGGCGCATCTGTAAAAATGA GCTGCAAAGCATCAGGTTACACATTTACATCCTACTACATCCATTGGATCAAGCAAACACC AGGCCAATGTCTTGAGTGGGTTGGCGTCATTTACCCAGGAAACGATGATATATCTTACAAT CAGAAATTTCAAGGGAAAGCCACACTTACAGCCGACAAGAGTTCCACAACTGCATATATGC AACTCTCCTCCCTGACATCTGAAGACAGTGCCGTATACTATTGTGCTCGTGAAGTCAGGCT CAGATACTTTGACGTGTGGGGTCAAGGCACAACCGTCACCGTCAGTAGC | 680 |
| | VL | GAGATAGTCCTCACCCAATCACCAGGTAGTCTGGCTGTTAGCCCAGGTGAGCGAGTTACTA TGTCTTGCAAGTCCTCTCAATCTGTGTTCTTTTCATCAAGTCAGAAGAATTATCTTGCCTG GTATCAGCAGATTCCTGGTCAATCTCCCCGACTGCTTATTTACTGGGCTTCAACTCGGGAG TCAGGGGTTCCCGACCGATTCACAGGGTCTGGGTCAGGAACTGACTTTACCCTTACTATCA GCTCTGTGCAGCCTGAAGACCTCGCAATATATTATTGTCATCAGTACCTCTCTTCTCGCAC TTTTGGATGTGGCACCAAATTGGAGATTAAG | 681 |
| 33-4 | VH1 | GAGGTTCAGTTGGTTCAGTCAGGAGCAGAGGTCAAAAAACCTGGAAGCTCTGTCAAAGTTA GTTGTAAGGCCAGTGGATACACCATAACCGATTCAAATATACATTGGGTTAGGCAAGCACC | 682 |
| | | AGGACAGTCCTTGGAATGGATCGGGTACATCTATCCATATAATGGGGGCACCGATTACAAC CAAAAGTTTAAGAATCGCGCCACACTCACTGTTGATAATCCAACCAATACAGCATACATGG AGTTGAGCAGTCTTCGGTCCGAGGACACTGCTTTTTACTATTGTGTGAACGGTAACCCATG GTTGGCCTATTGGGGCCAAGGTACACTTGTAACAGTTTCATCC | |
| | VH2 | CAGGTACAGCTTGTGCAATCTGGAGCTGAGGTCAAAAAACCAGGAAGTTCTGTAAAGGTGT CTTGTAAGGCATCTGGTTACACATTTACCGATTACAACATGCATTGGGTACGTCAAGCCCC TGGCCAGTGCCTGGAATGGATCGGATATATATACCCCTACAACGGTGGAACAGGTTACAAC CAAAAATTCAAAAGCAAGGCTACTATCACTGCCGACGAGAGCACTAACACCGCTTACATGG AACTGTCTTCCTTGCGTTCAGAAGACACTGCCGTGTATTATTGTGCTCGGGGGCGACCCGC AATGGACTATTGGGGCCAAGGTACATTGGTGACTGTCAGTTCC | 678 |
| | VL1 | GATATACAACTGACTCAGAGTCCCAGCACTCTCAGTGCAAGTGTAGGCGATAGAGTAACTA TAACCTGTCGCGCCTCAGAATCTCTTGATAATTATGGGATCCGATTTCTTACTTGGTTTCA GCAAAAGCCTGGTAAAGCTCCTAAATTGCTCATGTATGCCGCCAGTAATCAGGGTTCAGGA GTTCCTAGTCGTTTCTCTGGGTCAGGAAGCGGCACAGAATTTACCCTTACAATTTCCAGCC TCCAGCCCGACGATTTCGCCACTTACTATTGCCAACAAACTAAAGAGGTTCCTTGGAGTTT TGGGCAAGGCACCAAGGTAGAAGTAAAA | 683 |
| | VL2 | GACATCCAAATGACACAGTCCCCTAGCTCACTTTCAGCAAGCGTGGGGGACCGAGTTACTA TCACATGCCGCGCAAGTGAGTCTGTGGACAACTATGGAATATCATTCATGAACTGGTTCCA GCAGAAACCTGGGAAAGCACCCAAGCTGCTTATCTACGCAGCAAGTAATCAGGGTAGTGGC GTCCCTTCTCGATTCAGTGGGAGCGGTAGCGGCACCGACTTCACCCTTACCATCTCATCAC TTCAACCCGATGATTTTGCTACCTACTATTGCCAGCAATCCAAGGAAGTTCCTTGGACCTT CGGGTGTGGGACAAAGGTAGAGATTAAA | 679 |
| 33-5 | V-H1 | CAAGTCCAACTTCAGCAGCCTGGAGCTGAGGTAGTGAAACCCGGCGCATCTGTAAAAATGA GCTGCAAAGCATCAGGTTACACATTTACATCCTACTACATCCATTGGATCAAGCAAACACC AGGCCAAGGTCTTGAGTGGGTTGGCGTCATTTACCCAGGAAACGATGATATATCTTACAAT CAGAAATTTCAAGGGAAAGCCACACTTACAGCCGACAAGAGTTCCACAACTGCATATATGC AACTCTCCTCCCTGACATCTGAAGACAGTGCCGTATACTATTGTGCTCGTGAAGTCAGGCT CAGATACTTTGACGTGTGGGGTCAAGGCACAACCGTCACCGTCAGTAGC | 684 |
| | VH2 | CAGGTACAGCTTGTGCAATCTGGAGCTGAGGTCAAAAAACCAGGAAGTTCTGTAAAGGTGT CTTGTAAGGCATCTGGTTACACATTTACCGATTACAACATGCATTGGGTACGTCAAGCCCC TGGCCAGTGCCTGGAATGGATCGGATATATATACCCCTACAACGGTGGAACAGGTTACAAC CAAAAATTCAAAAGCAAGGCTACTATCACTGCCGACGAGAGCACTAACACCGCTTACATGG AACTGTCTTCCTTGCGTTCAGAAGACACTGCCGTGTATTATTGTGCTCGGGGGCGACCCGC AATGGACTATTGGGGCCAAGGTACATTGGTGACTGTCAGTTCC | 678 |
| | VL1 | GAGATAGTCCTCACCCAATCACCAGGTAGTCTGGCTGTTAGCCCAGGTGAGCGAGTTACTA TGTCTTGCAAGTCCTCTCAATCTGTGTTCTTTTCATCAAGTCAGAAGAATTATCTTGCCTG GTATCAGCAGATTCCTGGTCAATCTCCCCGACTGCTTATTTACTGGGCTTCAACTCGGGAG TCAGGGGTTCCCGACCGATTCACAGGGTCTGGGTCAGGAACTGACTTTACCCTTACTATCA GCTCTGTGCAGCCTGAAGACCTCGCAATATATTATTGTCATCAGTACCTCTCTTCTCGCAC TTTTGGACAGGGCACCAAATTGGAGATTAAG | 685 |
| | VL2 | GACATCCAAATGACACAGTCCCCTAGCTCACTTTCAGCAAGCGTGGGGGACCGAGTTACTA TCACATGCCGCGCAAGTGAGTCTGTGGACAACTATGGAATATCATTCATGAACTGGTTCCA GCAGAAACCTGGGAAAGCACCCAAGCTGCTTATCTACGCAGCAAGTAATCAGGGTAGTGGC GTCCCTTCTCGATTCAGTGGGAGCGGTAGCGGCACCGACTTCACCCTTACCATCTCATCAC TTCAACCCGATGATTTTGCTACCTACTATTGCCAGCAATCCAAGGAAGTTCCTTGGACCTT CGGGTGTGGGACAAAGGTAGAGATTAAA | 679 |
| 33-6 | VH1 | CAGGTACAGCTTGTGCAATCTGGAGCTGAGGTCAAAAAACCAGGAAGTTCTGTAAAGGTGT CTTGTAAGGCATCTGGTTACACATTTACCGATTACAACATGCATTGGGTACGTCAAGCCCC TGGCCAGGGCCTGGAATGGATCGGATATATATACCCCTACAACGGTGGAACAGGTTACAAC CAAAAATTCAAAAGCAAGGCTACTATCACTGCCGACGAGAGCACTAACACCGCTTACATGG AACTGTCTTCCTTGCGTTCAGAAGACACTGCCGTGTATTATTGTGCTCGGGGGCGACCCGC AATGGACTATTGGGGCCAAGGTACATTGGTGACTGTCAGTTCC | 686 |
| | VH2 | GAGGTTCAGTTGGTTCAGTCAGGAGCAGAGGTCAAAAAACCTGGAAGCTCTGTCAAAGTTA GTTGTAAGGCCAGTGGATACACCATAACCGATTCAAATATACATTGGGTTAGGCAAGCACC AGGACAGTGCTTGGAATGGATCGGGTACATCTATCCATATAATGGGGGCACCGATTACAAC CAAAAGTTTAAGAATCGCGCCACACTCACTGTTGATAATCCAACCAATACAGCATACATGG AGTTGAGCAGTCTTCGGTCCGAGGACACTGCTTTTTACTATTGTGTGAACGGTAACCCATG GTTGGCCTATTGGGGCCAAGGTACACTTGTAACAGTTTCATCT | 676 |
| | VL1 | GACATCCAAATGACACAGTCCCCTAGCTCACTTTCAGCAAGCGTGGGGGACCGAGTTACTA TCACATGCCGCGCAAGTGAGTCTGTGGACAACTATGGAATATCATTCATGAACTGGTTCCA GCAGAAACCTGGGAAAGCACCCAAGCTGCTTATCTACGCAGCAAGTAATCAGGGTAGTGGC GTCCCTTCTCGATTCAGTGGGAGCGGTAGCGGCACCGACTTCACCCTTACCATCTCATCAC TTCAACCCGATGATTTTGCTACCTACTATTGCCAGCAATCCAAGGAAGTTCCTTGGACCTT CGGGCAAGGGACAAAGGTAGAGATTAAA | 687 |
| | VL2 | GATATACAACTGACTCAGAGTCCCAGCACTCTCAGTGCAAGTGTAGGCGATAGAGTAACTA TAACCTGTCGCGCCTCAGAATCTCTTGATAATTATGGGATCCGATTTCTTACTTGGTTTCA GCAAAAGCCTGGTAAAGCTCCTAAATTGCTCATGTATGCCGCCAGTAATCAGGGTTCAGGA GTTCCTAGTCGTTTCTCTGGGTCAGGAAGCGGCACAGAATTTACCCTTACAATTTCCAGCC TCCAGCCCGACGATTTCGCCACTTACTATTGCCAACAAACTAAAGAGGTTCCTTGGAGTTT TGGGTGTGGCACCAAGGTAGAAGTAAAA | 677 |
| 33-7 | VH1 | CAGGTACAGCTTGTGCAATCTGGAGCTGAGGTCAAAAAACCAGGAAGTTCTGTAAAGGTGT CTTGTAAGGCATCTGGTTACACATTTACCGATTACAACATGCATTGGGTACGTCAAGCCCC TGGCCAGGGCCTGGAATGGATCGGATATATATACCCCTACAACGGTGGAACAGGTTACAAC CAAAAATTCAAAAGCAAGGCTACTATCACTGCCGACGAGAGCACTAACACCGCTTACATGG AACTGTCTTCCTTGCGTTCAGAAGACACTGCCGTGTATTATTGTGCTCGGGGGCGACCCGC AATGGACTATTGGGGCCAAGGTACATTGGTGACTGTCAGTTCC | 686 |
| | VH2 | CAAGTCCAACTTCAGCAGCCTGGAGCTGAGGTAGTGAAACCCGGCGCATCTGTAAAAATGA GCTGCAAAGCATCAGGTTACACATTTACATCCTACTACATCCATTGGATCAAGCAAACACC AGGCCAATGTCTTGAGTGGGTTGGCGTCATTTACCCAGGAAACGATGATATATCTTACAAT CAGAAATTTCAAGGGAAAGCCACACTTACAGCCGACAAGAGTTCCACAACTGCATATATGC | 680 |
| | | AACTCTCCTCCCTGACATCTGAAGACAGTGCCGTATACTATTGTGCTCGTGAAGTCAGGCT CAGATACTTTGACGTGTGGGGTCAAGGCACAACCGTCACCGTCAGTAGC | |
| | VL1 | GACATCCAAATGACACAGTCCCCTAGCTCACTTTCAGCAAGCGTGGGGGACCGAGTTACTA TCACATGCCGCGCAAGTGAGTCTGTGGACAACTATGGAATATCATTCATGAACTGGTTCCA GCAGAAACCTGGGAAAGCACCCAAGCTGCTTATCTACGCAGCAAGTAATCAGGGTAGTGGC GTCCCTTCTCGATTCAGTGGGAGCGGTAGCGGCACCGACTTCACCCTTACCATCTCATCAC TTCAACCCGATGATTTTGCTACCTACTATTGCCAGCAATCCAAGGAAGTTCCTTGGACCTT CGGGCAAGGGACAAAGGTAGAGATTAAA | 587 |
| | VL2 | GAGATAGTCCTCACCCAATCACCAGGTAGTCTGGCTGTTAGCCCAGGTGAGCGAGTTACTA TGTCTTGCAAGTCCTCTCAATCTGTGTTCTTTTCATCAAGTCAGAAGAATTATCTTGCCTG GTATCAGCAGATTCCTGGTCAATCTCCCCGACTGCTTATTTACTGGGCTTCAACTCGGGAG TCAGGGGTTCCCGACCGATTCACAGGGTCTGGGTCAGGAACTGACTTTACCCTTACTATCA GCTCTGTGCAGCCTGAAGACCTCGCAATATATTATTGTCATCAGTACCTCTCTTCTCGCAC TTTTGGATGTGGCACCAAATTGGAGATTAAG | 581 |

Table 72 below shows the heavy chain and light chain CDR sequences of the engineered antibodies targeting CD33.

**[Table 72]**

| **Name** | CDR | **Sequence** | **SEQ ID NO** |
|---|---|---|---|
| 33-1 | CDR-H1 | DSNIH | 688 |
| | CDR-H2 | YIYPYNGGTDYNQKFKN | 689 |
| | CDR-H3 | GNPWLAY | 690 |
| | CDR-L1 | RASESLDNYGIRFLT | 691 |
| | CDR-L2 | AASNQGS | 692 |
| | CDR-L3 | QQTKEVPWS | 693 |
| 33-2 | CDR-H1 | DYNMH | 694 |
| | CDR-H2 | YIYPYNGGTGYNQKFKS | 695 |
| | CDR-H3 | GRPAMDY | 696 |
| | CDR-L1 | RASESVDNYGISFMN | 697 |
| | CDR-L2 | AASNQGS | 698 |
| | CDR-L3 | QQSKEVPWT | 699 |
| 33-3 | CDR-H1 | SYYIH | 700 |
| | CDR-H2 | VIYPGNDDISYNQKFQG | 701 |
| | CDR-H3 | EVRLRYFDV | 702 |
| | CDR-L1 | KSSQSVFFSSSQKNYLA | 703 |
| | CDR-L2 | WASTRES | 704 |
| | CDR-L3 | HQYLSSRT | 705 |
| 33-4 | V1 CDR-H1 | DSNIH | 638 |
| | V1 CDR-H2 | YIYPYNGGTDYNQKFKN | 689 |
| | V1 CDR-H3 | GNPWLAY | 690 |
| | V1 CDR-L1 | RASESLDNYGIRFLT | 691 |
| | V1 CDR-L2 | AASNQGS | 692 |
| | V1 CDR-L3 | QQTKEVPWS | 693 |
| | V2 CDR-H1 | DYNMH | 694 |
| | V2 CDR-H2 | YIYPYNGGTGYNQKFKS | 695 |
| | V2 CDR-H3 | GRPAMDY | 696 |
| | V2 CBR-L1 | RASESVDNYGISFMN | 69 7 |
| | V2 CDR-L2 | AASNQGS | 698 |
| | V2 CDR-L3 | QQSKEVPWT | 699 |
| 33-5 | V1 CDR-H1 | SYYIH | 700 |
| | V1 CDR-H2 | VIYPGNDDISYNQKFQG | 701 |
| | V1 CDR-H3 | EVRLRYFDV | 702 |
| | V1 CDR-L1 | KSSQSVFFSSSQKNYLA | 703 |
| | V1 CDR-L2 | WASTRES | 704 |
| | V1 CDR-L3 | HQYLSSRT | 705 |
| | V2 CDR-H1 | DYNMH | 694 |
| | V2 CDR-H2 | YIYPYNGGTGYNQKFKS | 695 |
| | V2 CDR-H3 | GRPAMDY | 696 |
| | V2 CDR-L1 | RASESVDNYGISFMN | 697 |
| | V2 CDR-L2 | AASNQGS | 698 |
| | V2 CDR-L3 | QQSKEVPWT | 699 |
| 33-6 | V1 CDR-H1 | DYNMH | 694 |
| | V1 CDR-H2 | YIYPYNGGTGYNQKFKS | 695 |
| | V1 CDR-H3 | GRPAMDY | 696 |
| | V1 CDR-L1 | RASESVDNYGISFMN | 697 |
| | V1 CDR-L2 | AASNQGS | 698 |
| | V1 CDR-L3 | QQSKEVPWT | 699 |
| | V2 CDR-H1 | DSNIH | 638 |
| | V2 CDR-H2 | YIYPYNGGTDYNQKFKN | 689 |
| | V2 CDR-H3 | GNPWLAY | 690 |
| | V2 CDR-L1 | RASESLDNYGIRFLT | 691 |
| | V2 CDR-L2 | AASNQGS | 692 |
| | V2 CDR-L3 | QQTKEVPWS | 693 |
| 33-7 | V1 CDR-H1 | DYNMH | 694 |
| | V1 CDR-H2 | YIYPYNGGTGYNQKFKS | 695 |
| | V1 CDR-H3 | GRPAMDY | 696 |
| | V1 CDR-L1 | RASESVDNYGISFMN | 697 |
| | V1 CDR-L2 | AASNQGS | 608 |
| | V1 CDR-L3 | QQSKEVPWT | 699 |
| | V2 CDR-H1 | DYNMH | 694 |
| | V2 CDR-H2 | YIYPYNGGTGYNQKFKS | 695 |
| | V2 CDR-H3 | GRPAMDY | 696 |
| | V2 CDR-L1 | RASESVDNYGISFMN | 697 |
| | V2 CDR-L2 | AASNQGS | 698 |
| | V2 CDR-L3 | QQSKEVPWT | 699 |

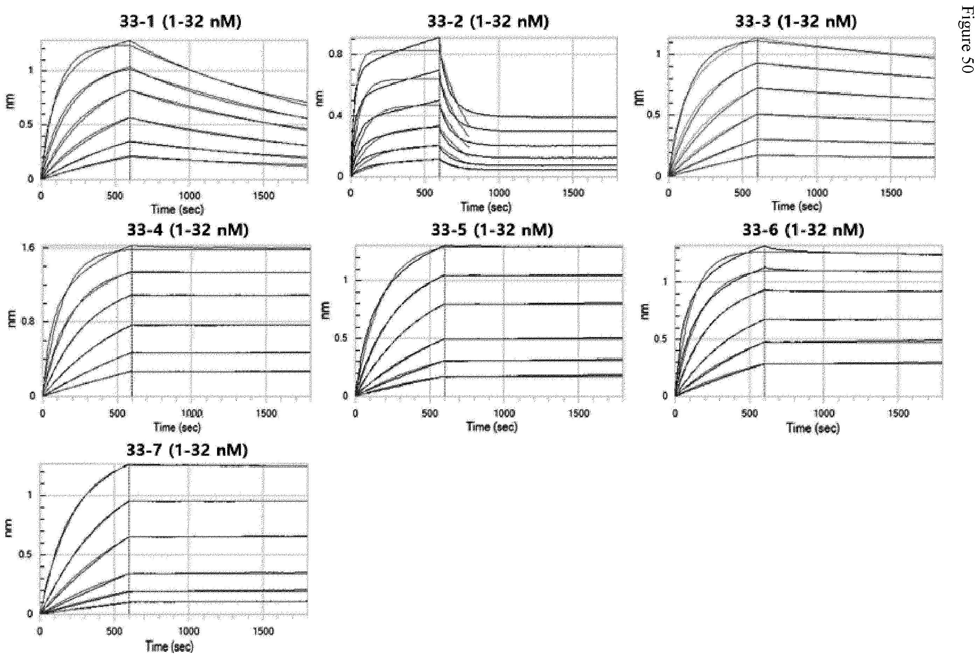

The CD33 protein binding constants of 33-1, 33-2, 33-3, 33-4, 33-5, 33-6, and 33-7 were determined using Octet Red96e (Sartorius). In order to analyze the binding constants of the seven antibodies, the human CD33 recombinant protein (Sino Biologicals, 12238-H08H) was loaded onto the Anti-Penta-HIS (HIS1K) biosensor (Sartorius, 18-5120). Then, the seven antibodies were added in a binding reaction (600 seconds) and a dissociation reaction (1,200 seconds) at various concentrations, and their affinities for CD33 were calculated (Figure 50, Table 73). Table 73 below illustrates the binding constants of the engineered antibodies targeting CD33.

**[Table 73]**

| Antibodies | Antigen | Binding mode | K_{D} (nM) | Kₐ (1/Ms) | K_{d} (1/s) |
|---|---|---|---|---|---|
| 33-1 | rhCD33 | Monovalent | 1.7835 | 2.80E+05 | 4.99E-04 |
| 33-2 | rhCD33 | Monovalent | 5.5540 | 8.01E+05 | 4.45E-03 |
| 33-3 | rhCD33 | Monovalent | 0.5163 | 2.24E+05 | 1.16E-04 |
| 33-4 | rhCD33 | Biparatopic | 0.0851 | 2.79E+05 | 8.11E+02 |
| 33-5 | rhCD33 | Biparatopic | 0.0641 | 2.01E+05 | 4.07E+02 |
| 33-6 | rhCD33 | Biparatopic | 0.0772 | 3.60E+05 | 1.08E+03 |
| 33-7 | rhCD33 | Biparatopic | 0.1290 | 1.37E+05 | 1.24E+02 |

### Example 22. Design, preparation and analysis of antibody structure targeting CEACAM5

The light chain and heavy chain variant polypeptide sequences of the antibodies that specifically bind to the CEACAM5 protein are shown in Table 74. For CEA01, expression vectors consisting of the sequences corresponding to Fc-Hole (SEQ ID NO: 7), CEA01 HC (SEQ ID NO: 590), and CEA01 LC (SEQ ID NO: 591) were co-transfected into EXPICHO-S^{™} (Gibco, A29127) (Figure 41a, Table 74), and purification and analysis were performed in the same manner as described in Example 1. Expression, purification and analysis were performed for CEA02, CEA03, and CEA04 in the same manner as mentioned above (Table 74).

**[Table 74]**

| **Name** | **Sequence** | **SEQ ID NO** |
|---|---|---|
| CEA01 HC | EVQLVESGGGVVQPGRSLRLSCSASGFDFTTYWMSWVRQAPGKCLEWIGEIHPDSSTIN YAPSLKDRFTISRDNAKNTLFLQMDSLRPEDTGVYFCASLYFGFPWFAYWGQGTPVTVS SASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLP PCREEMTKNQVSLWCLVKGFYPSDIAVEWESNGQPENNYKTTPPVLDSDGSFFLYSKLT VDKSRWQQGNVFSCSVMHEALHNHYTQKSLSLSPGK | 706 |
| CEA01 LC | DIQLTQSPSSLSASVGDRVTITCKASQDVGTSVAWYQQKPGKAPKLLIYWTSTRHTGVP SRFSGSGSGTDFTFTISSLQPEDIATYYCQQYSLYRSFGQGTKVEIKRTVAAPSVFIFP PSDEQLKSGTASVVCLLNNFYPREAKVQWKVDNALQSGNSQESVTEQDSKDSTYSLSST LTLSKADYEKHKVYACEVTHQGLSSPVTKSFNRGECGGGGSGGGGSGGGGSEPKSSDKT HTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKG QPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 707 |
| CEA02 HC | EVQLVESGGGLVQPGRSLRLSCAASGFTVSSYWMHWVRQAPGKCLEWVGFILNKANGGT TEYAASVKGRFTISRDDSKNTLYLQMNSLRAEDTAVYYCARDRGLRFYFDYWGQGTTVT VSSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYT LPPCREEMTKNQVSLWCLVKGFYPSDIAVEWESNGQPENNYKTTPPVLDSDGSFFLYSK LTVDKSRWQQGNVFSCSVMHEALHNHYTQKSLSLSPGK | 708 |
| CEA02 LC | QAVLTQPASLSASPGASASLTCTLRRGINVGAYSIYWYQQKPGSPPQYLLRYKSDSDKQ QGSGVSSRFSASKDASANAGILLISGLQSEDEADYYCMIWHSGASAVFGGGTKLTVLGQ PKAAPSVTLFPPSSEELQANKATLVCLISDFYPGAVTVAWKADSSPVKAGVETTTPSKQ SNNKYAASSYLSLTPEQWKSHRSYSCQVTHEGSTVEKTVAPTECSGGGGSGGGGSGGGG SEPKSSDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPI EKTISKAKGQPREPQVYTLPPCREEMTKNQVSLWCLVKGFYPSDIAVEWESNGQPENNY KTTPPVLDSDGSFFLYSKLTVDKSRWQQGNVFSCSVMHEALHNHYTQKSLSLSPGK | 709 |
| CEA03 HC | EVQLQQSGPGLVRPSQTLSLTCTASGFNIKDNYMHWVRQPPGRCLEWIGWIDPENGDTE YAPKFRGRVTMLADTSKNQFSLRLSSVTAADTAVYYCHVLIYAGYLAMDYWGQGTLVTV SSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPE LLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTL | 710 |
| | PPCREEMTKNQVSLWCLVKGFYPSDIAVEWESNGQPENNYKTTPPVLDSDGSFFLYSKL TVDKSRWQQGNVFSCSVMHEALHNHYTQKSLSLSPGK | |
| CEA03 LC | DIQMTQSPSSLSASVGDRVTITCSASSSVTYMHWYQQKPGKAPKLWIYSTSNLASGVPS RFSGSGSGTDYTFTISSLQPEDIATYYCQQRSTYPLTFGCGTKLEIKRTVAAPSVFIFP PSDEQLKSGTASVVCLLNNFYPREAKVQWKVDNALQSGNSQESVTEQDSKDSTYSLSST LTLSKADYEKHKVYACEVTHQGLSSPVTKSFNRGECGGGGSGGGGSGGGGSEPKSSDKT HTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKG QPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 711 |
| CEA04 HC | EVQLVESGGGLVQPGGSLRLSCAASGFNIKDTYMHWVRQAPGKCLEWVARIDPANGNSK YADSVKGRFTISADTSKNTAYLQMNSLRAEDTAVYYCAPFGYYVSDYAMAYWGQGTLVT VSSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYT LPPCREEMTKNQVSLWCLVKGFYPSDIAVEWESNGQPENNYKTTPPVLDSDGSFFLYSK LTVDKSRWQQGNVFSCSVMHEALHNHYTQKSLSLSPGK | 712 |
| CEA04 LC | DIQLTQSPSSLSASVGDRVTITCRAGESVDIFGVGFLHWYQQKPGKAPKLLIYRASNLE SGVPSRFSGSGSRTDFTLTISSLQPEDFATYYCQQTNEDPYTFGCGTKVEIKRTVAAPS VFIFPPSDEQLKSGTASVVCLLNNFYPREAKVQWKVDNALQSGNSQESVTEQDSKDSTY SLSSTLTLSKADYEKHKVYACEVTHQGLSSPVTKSFNRGECGGGGSGGGGSGGGGSEPK SSDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTI SKAKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | 713 |

Table 75 below shows the heavy chain and light chain nucleotide sequences of the engineered antibodies targeting CEACAM5.

**[Table 75]**

| **Name** | **Sequence** | **SEQ ID NO** |
|---|---|---|
| CEA01 HC | GAAGTGCAACTCGTAGAATCCGGCGGGGGCGTCGTACAGCCCGGCCGTTCTTTGAGACT TAGCTGTAGTGCTTCTGGGTTTGACTTCACTACATACTGGATGTCATGGGTAAGACAGG CACCTGGCAAGTGCCTTGAATGGATCGGTGAAATCCATCCCGACAGCTCCACAATCAAC TACGCCCCAAGTTTGAAAGACCGGTTCACCATATCTCGTGACAACGCCAAGAATACATT GTTCCTTCAGATGGATAGTCTTCGTCCAGAGGATACTGGGGTATATTTTTGTGCCAGCT TGTATTTTGGCTTCCCCTGGTTCGCTTATTGGGGCCAAGGTACACCCGTCACTGTCTCT TCTGCTAGCACCAAAGGACCTAGTGTTTTTCCTCTTGCCCCTTCCTCAAAGTCTACCTC TGGGGGGACAGCCGCTCTGGGCTGCCTGGTCAAGGATTATTTCCCAGAGCCTGTCACTG TCAGTTGGAACTCTGGAGCCTTGACTTCTGGTGTTCATACATTTCCTGCTGTCCTTCAA AGCAGCGGCTTGTACTCATTGTCTTCTGTTGTGACAGTACCCTCAAGCAGCCTCGGCAC TCAGACATACATCTGCAATGTCAACCACAAACCCTCAAATACAAAGGTAGATAAAAAAG TCGAACCAAAGTCTTGTGACAAAACTCACACGTGCCCACCGTGCCCAGCACCTGAACTC CTGGGGGGACCGTCAGTCTTCCTCTTCCCCCCAAAACCCAAGGACACCCTCATGATCTC CCGGACCCCTGAGGTCACATGCGTGGTGGTGGACGTGAGCCACGAAGACCCTGAGGTCA AGTTCAACTGGTACGTGGACGGCGTGGAGGTGCATAATGCCAAGACAAAGCCGCGGGAG GAGCAGTACAACAGCACGTACCGTGTGGTCAGCGTCCTCACCGTCCTGCACCAGGACTG GCTGAATGGCAAGGAGTACAAGTGCAAGGTCTCCAACAAAGCCCTCCCAGCCCCCATCG AGAAAACCATCTCCAAAGCCAAAGGGCAGCCCCGAGAACCACAGGTGTACACCCTGCCC CCATGCCGGGAGGAGATGACCAAGAACCAGGTCAGCCTGTGGTGCCTGGTCAAAGGCTT CTATCCCAGCGACATCGCCGTGGAGTGGGAGAGCAATGGGCAGCCGGAGAACAACTACA AGACCACGCCTCCCGTGCTGGACTCCGACGGCTCCTTCTTCCTCTACAGCAAGCTCACC GTGGACAAGAGCAGGTGGCAGCAGGGGAACGTCTTCTCATGCTCCGTGATGCATGAGGC TCTGCACAACCACTACACGCAGAAGAGCCTCTCCCTGTCTCCGGGTAAA | 714 |
| CEA01 LC | GACATCCAACTTACCCAGTCACCCTCATCTCTTAGTGCCTCTGTAGGGGACCGAGTTAC TATTACATGTAAAGCCAGTCAAGATGTTGGCACCTCAGTAGCATGGTATCAACAAAAGC CTGGTAAAGCCCCAAAACTGCTGATCTATTGGACAAGCACACGACATACAGGAGTGCCA AGTCGCTTCAGCGGTTCAGGTTCAGGCACAGATTTTACATTCACTATATCAAGCCTGCA ACCCGAGGACATTGCCACATATTACTGCCAGCAATATAGTCTGTATCGTAGCTTCGGAT GTGGCACCAAGGTTGAAATTAAGCGTACGGTGGCAGCTCCCAGCGTTTTTATCTTTCCC CCATCCGACGAGCAGCTCAAGAGTGGCACTGCCTCTGTAGTTTGTTTGCTGAATAACTT CTATCCACGTGAAGCAAAAGTACAGTGGAAGGTCGATAATGCCCTTCAGAGCGGTAACA GCCAAGAAAGTGTTACCGAGCAAGATTCCAAAGATTCCACTTACAGTCTGTCCAGCACA TTGACACTGAGTAAGGCTGATTACGAAAAACACAAGGTGTACGCATGCGAGGTGACACA CCAAGGTCTTTCATCTCCTGTAACTAAGAGCTTTAACCGGGGAGAATGTGGTGGTGGGG GCAGCGGGGGCGGAGGTAGTGGAGGCGGCGGTAGTGAACCAAAGAGTAGTGACAAAACT CACACGTGCCCACCGTGCCCAGCACCTGAACTCCTGGGGGGACCGTCAGTCTTCCTCTT CCCCCCAAAACCCAAGGACACCCTCATGATCTCCCGGACCCCTGAGGTCACATGCGTGG TGGTGGACGTGAGCCACGAAGACCCTGAGGTCAAGTTCAACTGGTACGTGGACGGCGTG | 716 |
| | GAGGTGCATAATGCCAAGACAAAGCCGCGGGAGGAGCAGTACAACAGCACGTACCGTGT GGTCAGCGTCCTCACCGTCCTGCACCAGGACTGGCTGAATGGCAAGGAGTACAAGTGCA AGGTCTCCAACAAAGCCCTCCCAGCCCCCATCGAGAAAACCATCTCCAAAGCCAAAGGG CAGCCCCGAGAACCACAGGTGTACACCCTGCCCCCATGCCGGGAGGAGATGACCAAGAA CCAGGTCAGCCTGTGGTGCCTGGTCAAAGGCTTCTATCCCAGCGACATCGCCGTGGAGT GGGAGAGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAGCAGGG GAACGTCTTCTCATGCTCCGTGATGCATGAGGCTCTGCACAACCACTACACGCAGAAGA GCCTCTCCCTGTCTCCGGGTAAA | |
| CEA02 HC | GAAGTTCAACTGGTGGAGTCTGGCGGCGGACTTGTCCAGCCAGGGCGAAGCCTGCGTCT CTCATGCGCTGCCTCCGGTTTCACTGTTTCTTCATACTGGATGCACTGGGTAAGACAGG CTCCTGGCAAGTGTCTTGAATGGGTGGGCTTCATTTTGAACAAGGCAAACGGCGGGACT ACCGAATACGCTGCCAGCGTTAAGGGTCGATTCACCATCTCAAGGGATGATTCTAAAAA CACATTGTACCTTCAGATGAACTCTCTGAGGGCCGAGGACACAGCAGTCTACTATTGCG CTAGAGATAGGGGGCTTAGATTCTATTTTGACTACTGGGGGCAGGGAACAACTGTCACC GTTTCCAGTGCTAGCACCAAAGGACCTAGTGTTTTTCCTCTTGCCCCTTCCTCAAAGTC TACCTCTGGGGGGACAGCCGCTCTGGGCTGCCTGGTCAAGGATTATTTCCCAGAGCCTG TCACTGTCAGTTGGAACTCTGGAGCCTTGACTTCTGGTGTTCATACATTTCCTGCTGTC CTTCAAAGCAGCGGCTTGTACTCATTGTCTTCTGTTGTGACAGTACCCTCAAGCAGCCT CGGCACTCAGACATACATCTGCAATGTCAACCACAAACCCTCAAATACAAAGGTAGATA AAAAAGTCGAACCAAAGTCTTGTGACAAAACTCACACGTGCCCACCGTGCCCAGCACCT GAACTCCTGGGGGGACCGTCAGTCTTCCTCTTCCCCCCAAAACCCAAGGACACCCTCAT GATCTCCCGGACCCCTGAGGTCACATGCGTGGTGGTGGACGTGAGCCACGAAGACCCTG AGGTCAAGTTCAACTGGTACGTGGACGGCGTGGAGGTGCATAATGCCAAGACAAAGCCG CGGGAGGAGCAGTACAACAGCACGTACCGTGTGGTCAGCGTCCTCACCGTCCTGCACCA GGACTGGCTGAATGGCAAGGAGTACAAGTGCAAGGTCTCCAACAAAGCCCTCCCAGCCC CCATCGAGAAAACCATCTCCAAAGCCAAAGGGCAGCCCCGAGAACCACAGGTGTACACC CTGCCCCCATGCCGGGAGGAGATGACCAAGAACCAGGTCAGCCTGTGGTGCCTGGTCAA AGGCTTCTATCCCAGCGACATCGCCGTGGAGTGGGAGAGCAATGGGCAGCCGGAGAACA ACTACAAGACCACGCCTCCCGTGCTGGACTCCGACGGCTCCTTCTTCCTCTACAGCAAG CTCACCGTGGACAAGAGCAGGTGGCAGCAGGGGAACGTCTTCTCATGCTCCGTGATGCA TGAGGCTCTGCACAACCACTACACGCAGAAGAGCCTCTCCCTGTCTCCGGGTAAA | 716 |
| CEA02 LC | CAGGCAGTGCTTACTCAGCCTGCTTCACTCTCTGCCTCACCAGGAGCATCTGCAAGCCT CACCTGCACATTGCGTCGAGGTATAAACGTAGGCGCTTACTCAATTTACTGGTACCAGC AGAAACCTGGGAGCCCACCTCAATACCTCCTCCGATACAAGAGCGATTCTGATAAGCAA CAGGGCAGTGGTGTATCCAGCAGATTTTCCGCCAGCAAGGATGCAAGCGCTAATGCAGG TATTCTTCTCATTTCAGGCTTGCAAAGCGAGGACGAAGCAGACTACTACTGCATGATTT GGCACTCCGGTGCCTCCGCAGTTTTTGGCTGCGGAACAAAGCTTACAGTCCTTGGTCAG CCCAAGGCTGCCCCCTCGGTCACTCTGTTCCCGCCCTCCTCTGAGGAGCTTCAAGCCAA CAAGGCCACACTGGTGTGTCTCATAAGTGACTTCTACCCGGGAGCCGTGACAGTGGCCT | 717 |
| | GGAAGGCAGATAGCAGCCCCGTCAAGGCGGGAGTGGAGACCACCACCCCTCCAAACAA AGCAACAACAAGTACGCGGCCAGCAGCTATCTGAGCCTGACGCCTGAGCAGTGGAAGTC CCACAGAAGCTACAGCTGCCAGGTCACGCATGAAGGGAGCACCGTGGAGAAGACAGTGG CCCCTACAGAATGTTCAGGTGGTGGGGGCAGCGGGGGCGGAGGTAGTGGAGGCGGCGGT AGTGAACCAAAGAGTAGTGACAAAACTCACACGTGCCCACCGTGCCCAGCACCTGAACT CCTGGGGGGACCGTCAGTCTTCCTCTTCCCCCCAAAACCCAAGGACACCCTCATGATCT CCCGGACCCCTGAGGTCACATGCGTGGTGGTGGACGTGAGCCACGAAGACCCTGAGGTC AAGTTCAACTGGTACGTGGACGGCGTGGAGGTGCATAATGCCAAGACAAAGCCGCGGGA GGAGCAGTACAACAGCACGTACCGTGTGGTCAGCGTCCTCACCGTCCTGCACCAGGACT GGCTGAATGGCAAGGAGTACAAGTGCAAGGTCTCCAACAAAGCCCTCCCAGCCCCCATC GAGAAAACCATCTCCAAAGCCAAAGGGCAGCCCCGAGAACCACAGGTGTACACCCTGCC CCCATGCCGGGAGGAGATGACCAAGAACCAGGTCAGCCTGTGGTGCCTGGTCAAAGGCT TCTATCCCAGCGACATCGCCGTGGAGTGGGAGAGCAATGGGCAGCCGGAGAACAACTAC AAGACCACGCCTCCCGTGCTGGACTCCGACGGCTCCTTCTTCCTCTACAGCAAGCTCAC CGTGGACAAGAGCAGGTGGCAGCAGGGGAACGTCTTCTCATGCTCCGTGATGCATGAGG CTCTGCACAACCACTACACGCAGAAGAGCCTCTCCCTGTCTCCGGGTAAA | |
| CEA03 HC | GAGGTACAACTGCAGCAAAGCGGTCCAGGCCTTGTTCGGCCTAGCCAGACTCTGTCACT GACCTGTACCGCAAGCGGTTTCAATATCAAGGACAACTATATGCACTGGGTCCGCCAGC CTCCAGGACGCTGTTTGGAGTGGATAGGATGGATAGACCCAGAAAATGGGGATACCGAA TACGCTCCAAAATTTCGTGGACGAGTTACCATGCTGGCCGATACATCCAAAAACCAGTT TTCTCTGAGGCTCTCCAGCGTGACAGCCGCAGATACCGCCGTATATTACTGTCATGTGT TGATCTATGCCGGGTATCTTGCAATGGATTACTGGGGACAGGGCACTCTCGTAACAGTC TCTTCAGCTAGCACCAAAGGACCTAGTGTTTTTCCTCTTGCCCCTTCCTCAAAGTCTAC CTCTGGGGGGACAGCCGCTCTGGGCTGCCTGGTCAAGGATTATTTCCCAGAGCCTGTCA CTGTCAGTTGGAACTCTGGAGCCTTGACTTCTGGTGTTCATACATTTCCTGCTGTCCTT CAAAGCAGCGGCTTGTACTCATTGTCTTCTGTTGTGACAGTACCCTCAAGCAGCCTCGG CACTCAGACATACATCTGCAATGTCAACCACAAACCCTCAAATACAAAGGTAGATAAAA AAGTCGAACCAAAGTCTTGTGACAAAACTCACACGTGCCCACCGTGCCCAGCACCTGAA CTCCTGGGGGGACCGTCAGTCTTCCTCTTCCCCCCAAAACCCAAGGACACCCTCATGAT CTCCCGGACCCCTGAGGTCACATGCGTGGTGGTGGACGTGAGCCACGAAGACCCTGAGG TCAAGTTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCACCAGGA CTGGCTGAATGGCAAGGAGTACAAGTGCAAGGTCTCCAACAAAGCCCTCCCAGCCCCCA TCGAGAAAACCATCTCCAAAGCCAAAGGGCAGCCCCGAGAACCACAGGTGTACACCCTG CCCCCATGCCGGGAGGAGATGACCAAGAACCAGGTCAGCCTGTGGTGCCTGGTCAAAGG CTTCTATCCCAGCGACATCGCCGTGGAGTGGGAGAGCAATGGGCAGCCGGAGAACAACT ACAAGACCACGCCTCCCGTGCTGGACTCCGACGGCTCCTTCTTCCTCTACAGCAAGCTC ACCGTGGACAAGAGCAGGTGGCAGCAGGGGAACGTCTTCTCATGCTCCGTGATGCATGA GGCTCTGCACAACCACTACACGCAGAAGAGCCTCTCCCTGTCTCCGGGTAAA | 718 |
| CEA03 LC | GACATACAAATGACCCAAAGCCCAAGCTCATTGAGCGCCTCCGTAGGAGATCGCGTAAC | 719 |
| | AATAACCTGCAGCGCCAGCAGTTCAGTAACTTATATGCACTGGTATCAGCAGAAGCCAG GTAAGGCTCCCAAACTGTGGATATATAGCACCAGCAACCTGGCATCTGGTGTACCCTCT CGATTTAGTGGCAGTGGTTCTGGAACAGACTATACCTTCACTATATCTTCTCTTCAGCC TGAGGATATAGCCACTTACTATTGCCAGCAACGTTCCACTTACCCCCTGACTTTTGGGT GTGGTACAAAGTTGGAAATAAAGCGTACGGTGGCAGCTCCCAGCGTTTTTATCTTTCCC CCATCCGACGAGCAGCTCAAGAGTGGCACTGCCTCTGTAGTTTGTTTGCTGAATAACTT CTATCCACGTGAAGCAAAAGTACAGTGGAAGGTCGATAATGCCCTTCAGAGCGGTAACA GCCAAGAAAGTGTTACCGAGCAAGATTCCAAAGATTCCACTTACAGTCTGTCCAGCACA TTGACACTGAGTAAGGCTGATTACGAAAAACACAAGGTGTACGCATGCGAGGTGACACA CCAAGGTCTTTCATCTCCTGTAACTAAGAGCTTTAACCGGGGAGAATGTGGTGGTGGGG GCAGCGGGGGCGGAGGTAGTGGAGGCGGCGGTAGTGAACCAAAGAGTAGTGACAAAACT CACACGTGCCCACCGTGCCCAGCACCTGAACTCCTGGGGGGACCGTCAGTCTTCCTCTT CCCCCCAAAACCCAAGGACACCCTCATGATCTCCCGGACCCCTGAGGTCACATGCGTGG TGGTGGACGTGAGCCACGAAGACCCTGAGGTCAAGTTCAACTGGTACGTGGACGGCGTG GAGGTGCATAATGCCAAGACAAAGCCGCGGGAGGAGCAGTACAACAGCACGTACCGTGT GGTCAGCGTCCTCACCGTCCTGCACCAGGACTGGCTGAATGGCAAGGAGTACAAGTGCA AGGTCTCCAACAAAGCCCTCCCAGCCCCCATCGAGAAAACCATCTCCAAAGCCAAAGGG CAGCCCCGAGAACCACAGGTGTACACCCTGCCCCCATGCCGGGAGGAGATGACCAAGAA CCAGGTCAGCCTGTGGTGCCTGGTCAAAGGCTTCTATCCCAGCGACATCGCCGTGGAGT GGGAGAGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAGCAGGG GAACGTCTTCTCATGCTCCGTGATGCATGAGGCTCTGCACAACCACTACACGCAGAAGA GCCTCTCCCTGTCTCCGGGTAAA | |
| CEA04 HC | GAAGTGCAGTTGGTGGAGTCTGGGGGCGGGTTGGTCCAACCAGGAGGCTCTCTGCGACT CTCTTGCGCAGCATCAGGCTTCAACATAAAGGACACATATATGCACTGGGTTCGGCAGG CTCCCGGAAAATGTCTTGAATGGGTCGCCCGAATTGATCCTGCAAATGGGAACAGTAAA TACGCAGATTCAGTTAAAGGCCGCTTCACTATCAGCGCAGATACATCCAAAAACACCGC ATACCTTCAGATGAACTCACTCCGTGCAGAAGACACTGCAGTCTACTATTGCGCTCCCT TCGGTTACTACGTCTCTGACTATGCAATGGCTTACTGGGGCCAAGGAACCTTGGTGACT GTATCTTCTGCTAGCACCAAAGGACCTAGTGTTTTTCCTCTTGCCCCTTCCTCAAAGTC TACCTCTGGGGGGACAGCCGCTCTGGGCTGCCTGGTCAAGGATTATTTCCCAGAGCCTG TCACTGTCAGTTGGAACTCTGGAGCCTTGACTTCTGGTGTTCATACATTTCCTGCTGTC CTTCAAAGCAGCGGCTTGTACTCATTGTCTTCTGTTGTGACAGTACCCTCAAGCAGCCT CGGCACTCAGACATACATCTGCAATGTCAACCACAAACCCTCAAATACAAAGGTAGATA AAAAAGTCGAACCAAAGTCTTGTGACAAAACTCACACGTGCCCACCGTGCCCAGCACCT GAACTCCTGGGGGGACCGTCAGTCTTCCTCTTCCCCCCAAAACCCAAGGACACCCTCAT GATCTCCCGGACCCCTGAGGTCACATGCGTGGTGGTGGACGTGAGCCACGAAGACCCTG AGGTCAAGTTCAACTGGTACGTGGACGGCGTGGAGGTGCATAATGCCAAGACAAAGCCG CGGGAGGAGCAGTACAACAGCACGTACCGTGTGGTCAGCGTCCTCACCGTCCTGCACCA GGACTGGCTGAATGGCAAGGAGTACAAGTGCAAGGTCTCCAACAAAGCCCTCCCAGCCC | 720 |
| | CCATCGAGAAAACCATCTCCAAAGCCAAAGGGCAGCCCCGAGAACCACAGGTGTACACC CTGCCCCCATGCCGGGAGGAGATGACCAAGAACCAGGTCAGCCTGTGGTGCCTGGTCAA AGGCTTCTATCCCAGCGACATCGCCGTGGAGTGGGAGAGCAATGGGCAGCCGGAGAACA ACTACAAGACCACGCCTCCCGTGCTGGACTCCGACGGCTCCTTCTTCCTCTACAGCAAG CTCACCGTGGACAAGAGCAGGTGGCAGCAGGGGAACGTCTTCTCATGCTCCGTGATGCA TGAGGCTCTGCACAACCACTACACGCAGAAGAGCCTCTCCCTGTCTCCGGGTAAA | |
| CEA04 LC | GATATACAGCTTACACAATCACCCTCCTCCCTGTCTGCCTCCGTAGGAGATAGAGTAAC AATCACATGCAGAGCTGGCGAGAGTGTTGATATATTCGGTGTTGGATTTTTGCACTGGT ACCAACAGAAACCAGGGAAAGCACCTAAGCTCTTGATTTATAGAGCTTCTAACCTTGAG AGCGGGGTGCCTAGTAGGTTTTCTGGGTCAGGAAGTCGGACCGATTTTACTCTCACAAT TTCATCCCTTCAGCCCGAAGACTTTGCAACCTACTACTGTCAGCAGACAAACGAAGACC CCTATACATTCGGATGTGGTACAAAGGTGGAGATTAAACGTACGGTGGCAGCTCCCAGC GTTTTTATCTTTCCCCCATCCGACGAGCAGCTCAAGAGTGGCACTGCCTCTGTAGTTTG TTTGCTGAATAACTTCTATCCACGTGAAGCAAAAGTACAGTGGAAGGTCGATAATGCCC TTCAGAGCGGTAACAGCCAAGAAAGTGTTACCGAGCAAGATTCCAAAGATTCCACTTAC AGTCTGTCCAGCACATTGACACTGAGTAAGGCTGATTACGAAAAACACAAGGTGTACGC ATGCGAGGTGACACACCAAGGTCTTTCATCTCCTGTAACTAAGAGCTTTAACCGGGGAG AATGTGGTGGTGGGGGCAGCGGGGGCGGAGGTAGTGGAGGCGGCGGTAGTGAACCAAAG AGTAGTGACAAAACTCACACGTGCCCACCGTGCCCAGCACCTGAACTCCTGGGGGGACC GTCAGTCTTCCTCTTCCCCCCAAAACCCAAGGACACCCTCATGATCTCCCGGACCCCTG AGGTCACATGCGTGGTGGTGGACGTGAGCCACGAAGACCCTGAGGTCAAGTTCAACTGG TACGTGGACGGCGTGGAGGTGCATAATGCCAAGACAAAGCCGCGGGAGGAGCAGTACAA CAGCACGTACCGTGTGGTCAGCGTCCTCACCGTCCTGCACCAGGACTGGCTGAATGGCA AGGAGTACAAGTGCAAGGTCTCCAACAAAGCCCTCCCAGCCCCCATCGAGAAAACCATC TCCAAAGCCAAAGGGCAGCCCCGAGAACCACAGGTGTACACCCTGCCCCCATGCCGGGA GGAGATGACCAAGAACCAGGTCAGCCTGTGGTGCCTGGTCAAAGGCTTCTATCCCAGCG ACATCGCCGTGGAGTGGGAGAGCAATGGGCAGCCGGAGAACAACTACAAGACCACGCCT CCCGTGCTGGACTCCGACGGCTCCTTCTTCCTCTACAGCAAGCTCACCGTGGACAAGAG CAGGTGGCAGCAGGGGAACGTCTTCTCATGCTCCGTGATGCATGAGGCTCTGCACAACC ACTACACGCAGAAGAGCCTCTCCCTGTCTCCGGGTAAA | 721 |

Table 76 below shows the polypeptide sequences of the heavy chain variable region (VH) and light chain variable region (VL) of the engineered antibodies targeting CEACAM5. Table 77 below shows the nucleotide sequences of the heavy chain variable region (VH) and light chain variable region (VL) of the engineered antibodies targeting CEACAM5.

**[Table 76]**

| Name | | Sequence | SEQ ID NO |
|---|---|---|---|
| CEA01 | VH | EVQLVESGGGVVQPGRSLRLSCSASGFDFTTYWMSWVRQAPGKCLE WIGEIHPDSSTINYAPSLKDRFTISRDNAKNTLFLQMDSLRPEDTGVYF CASLYFGFPWFAYWGQGTPVTVSS | 819 |
| | VL | DIQLTQSPSSLSASVGDRVTITCKASQDVGTSVAWYQQKPGKAPKLLI YWTSTRHTGVPSRFSGSGSGTDFTFTISSLQPEDIATYYCQQYSLYRSF GCGTKVEIK | 820 |
| CEA02 | VH | EVQLVESGGGLVQPGRSLRLSCAASGFTVSSYWMHWVRQAPGKCLE WVGFILNKANGGTTEYAASVKGRFTISRDDSKNTLYLQMNSLRAEDT AVYYCARDRGLRFYFDYWGQGTTVTVSS | 821 |
| | VL | QAVLTQPASLSASPGASASLTCTLRRGINVGAYSIYWYQQKPGSPPQY LLRYKSDSDKQQGSGVSSRFSASKDASANAGILLISGLQSEDEADYYC MIWHSGASAVFGCGTKLTVL | 822 |
| CEA03 | VH | EVQLQQSGPGLVRPSQTLSLTCTASGFNIKDNYMHWVRQPPGRCLEW IGWIDPENGDTEYAPKFRGRVTMLADTSKNQFSLRLSSVTAADTAVY YCHVLIYAGYLAMDYWGQGTLVTVSS | 823 |
| | VL | DIQMTQSPSSLSASVGDRVTITCSASSSVTYMHWYQQKPGKAPKLWI YSTSNLASGVPSRFSGSGSGTDYTFTISSLQPEDIATYYCQQRSTYPLTF GCGTKLEIK | 824 |
| CEA04 | VH | EVQLVESGGGLVQPGGSLRLSCAASGFNIKDTYMHWVRQAPGKCLE WVARIDPANGNSKYADSVKGRFTISADTSKNTAYLQMNSLRAEDTA VYYCAPFGYYVSDYAMAYWGQGTLVTVSS | 825 |
| | VL | DIQLTQSPSSLSASVGDRVTITCRAGESVDIFGVGFLHWYQQKPGKAP KLLIYRASNLESGVPSRFSGSGSRTDFTLTISSLQPEDFATYYCQQTNE DPYTFGCGTKVEIK | 826 |

**[Table 77]**

| Name | | Sequence | SEQ ID NO |
|---|---|---|---|
| CEA01 | VH | GAAGTGCAACTCGTAGAATCCGGCGGGGGCGTCGTACAGCCCGGC CGTTCTTTGAGACTTAGCTGTAGTGCTTCTGGGTTTGACTTCACTAC ATACTGGATGTCATGGGTAAGACAGGCACCTGGCAAGTGCCTTGA ATGGATCGGTGAAATCCATCCCGACAGCTCCACAATCAACTACGC CCCAAGTTTGAAAGACCGGTTCACCATATCTCGTGACAACGCCAA GAATACATTGTTCCTTCAGATGGATAGTCTTCGTCCAGAGGATACT GGGGTATATTTTTGTGCCAGCTTGTATTTTGGCTTCCCCTGGTTCGC TTATTGGGGCCAAGGTACACCCGTCACTGTCTCTTCT | 827 |
| | VL | GACATCCAACTTACCCAGTCACCCTCATCTCTTAGTGCCTCTGTAG GGGACCGAGTTACTATTACATGTAAAGCCAGTCAAGATGTTGGCA CCTCAGTAGCATGGTATCAACAAAAGCCTGGTAAAGCCCCAAAAC TGCTGATCTATTGGACAAGCACACGACATACAGGAGTGCCAAGTC GCTTCAGCGGTTCAGGTTCAGGCACAGATTTTACATTCACTATATC AAGCCTGCAACCCGAGGACATTGCCACATATTACTGCCAGCAATA TAGTCTGTATCGTAGCTTCGGATGTGGCACCAAGGTTGAAATTAAG | 722 |
| CEA02 | VH | GAAGTTCAACTGGTGGAGTCTGGCGGCGGACTTGTCCAGCCAGGG CGAAGCCTGCGTCTCTCATGCGCTGCCTCCGGTTTCACTGTTTCTTC ATACTGGATGCACTGGGTAAGACAGGCTCCTGGCAAGTGTCTTGA ATGGGTGGGCTTCATTTTGAACAAGGCAAACGGCGGGACTACCGA ATACGCTGCCAGCGTTAAGGGTCGATTCACCATCTCAAGGGATGAT TCTAAAAACACATTGTACCTTCAGATGAACTCTCTGAGGGCCGAGG ACACAGCAGTCTACTATTGCGCTAGAGATAGGGGGCTTAGATTCTA TTTTGACTACTGGGGGCAGGGAACAACTGTCACCGTTTCCAGT | 723 |
| | VL | CAGGCAGTGCTTACTCAGCCTGCTTCACTCTCTGCCTCACCAGGAG | 724 |
| | | CATCTGCAAGCCTCACCTGCACATTGCGTCGAGGTATAAACGTAGG CGCTTACTCAATTTACTGGTACCAGCAGAAACCTGGGAGCCCACCT CAATACCTCCTCCGATACAAGAGCGATTCTGATAAGCAACAGGGC AGTGGTGTATCCAGCAGATTTTCCGCCAGCAAGGATGCAAGCGCT AATGCAGGTATTCTTCTCATTTCAGGCTTGCAAAGCGAGGACGAAG CAGACTACTACTGCATGATTTGGCACTCCGGTGCCTCCGCAGTTTT TGGCTGCGGAACAAAGCTTACAGTCCTT | |
| CEA03 | VH | GAGGTACAACTGCAGCAAAGCGGTCCAGGCCTTGTTCGGCCTAGC CAGACTCTGTCACTGACCTGTACCGCAAGCGGTTTCAATATCAAGG ACAACTATATGCACTGGGTCCGCCAGCCTCCAGGACGCTGTTTGGA GTGGATAGGATGGATAGACCCAGAAAATGGGGATACCGAATACGC TCCAAAATTTCGTGGACGAGTTACCATGCTGGCCGATACATCCAAA AACCAGTTTTCTCTGAGGCTCTCCAGCGTGACAGCCGCAGATACCG CCGTATATTACTGTCATGTGTTGATCTATGCCGGGTATCTTGCAAT GGATTACTGGGGACAGGGCACTCTCGTAACAGTCTCTTCA | 725 |
| | VL | GACATACAAATGACCCAAAGCCCAAGCTCATTGAGCGCCTCCGTA GGAGATCGCGTAACAATAACCTGCAGCGCCAGCAGTTCAGTAACT TATATGCACTGGTATCAGCAGAAGCCAGGTAAGGCTCCCAAACTG TGGATATATAGCACCAGCAACCTGGCATCTGGTGTACCCTCTCGAT TTAGTGGCAGTGGTTCTGGAACAGACTATACCTTCACTATATCTTC TCTTCAGCCTGAGGATATAGCCACTTACTATTGCCAGCAACGTTCC ACTTACCCCCTGACTTTTGGGTGTGGTACAAAGTTGGAAATAAAG | 726 |
| CEA04 | VH | GAAGTGCAGTTGGTGGAGTCTGGGGGCGGGTTGGTCCAACCAGGA GGCTCTCTGCGACTCTCTTGCGCAGCATCAGGCTTCAACATAAAGG ACACATATATGCACTGGGTTCGGCAGGCTCCCGGAAAATGTCTTGA ATGGGTCGCCCGAATTGATCCTGCAAATGGGAACAGTAAATACGC AGATTCAGTTAAAGGCCGCTTCACTATCAGCGCAGATACATCCAA AAACACCGCATACCTTCAGATGAACTCACTCCGTGCAGAAGACAC TGCAGTCTACTATTGCGCTCCCTTCGGTTACTACGTCTCTGACTATG CAATGGCTTACTGGGGCCAAGGAACCTTGGTGACTGTATCTTCT | 727 |
| | VL | GATATACAGCTTACACAATCACCCTCCTCCCTGTCTGCCTCCGTAG GAGATAGAGTAACAATCACATGCAGAGCTGGCGAGAGTGTTGATA TATTCGGTGTTGGATTTTTGCACTGGTACCAACAGAAACCAGGGAA AGCACCTAAGCTCTTGATTTATAGAGCTTCTAACCTTGAGAGCGGG GTGCCTAGTAGGTTTTCTGGGTCAGGAAGTCGGACCGATTTTACTC TCACAATTTCATCCCTTCAGCCCGAAGACTTTGCAACCTACTACTG TCAGCAGACAAACGAAGACCCCTATACATTCGGATGTGGTACAAA GGTGGAGATTAAA | 728 |

Table 78 below shows the heavy chain and light chain CDR sequences of the engineered antibodies targeting CEACAM5.

**[Table 78]**

| Name | CDR | Sequence | SEQ ID NO |
|---|---|---|---|
| CEA01 | CDR-H1 | TYWMS | 729 |
| | CDR-H2 | EIHPDSSTINYAPSLKD | 730 |
| | CDR-H3 | LYFGFPWFAY | 731 |
| | CDR-L1 | KASQDVGTSVA | 732 |
| | CDR-L2 | WTSTRHT | 733 |
| | CDR-L3 | QQYSLYRS | 734 |
| CEA02 | CDR-H1 | SYWMH | 735 |
| | CDR-H2 | FILNKANGGTTEYAASVKG | 736 |
| | CDR-H3 | DRGLRFYFDY | 737 |
| | CDR-L1 | TLRRGINVGAYSIY | 738 |
| | CDR-L2 | SDKQQGS | 739 |
| | CDR-L3 | MIWHSGASAV | 740 |
| CEA03 | CDR-H1 | DNYMH | 741 |
| | CDR-H2 | WIDPENGDTEYAPKFRG | 742 |
| | CDR-H3 | LIYAGYLAMDY | 743 |
| | CDR-L1 | SASSSVTYMH | 744 |
| | CDR-L2 | STSNLAS | 745 |
| | CDR-L3 | QQRSTYPLT | 746 |
| CEA04 | CDR-H1 | DTYMH | 747 |
| | CDR-H2 | RIDPANGNSKYADSVKG | 748 |
| | CDR-H3 | FGYYVSDYAMAY | 749 |
| | CDR-L1 | RAGESVDIFGVGFLH | 750 |
| | CDR-L2 | RASNLES | 751 |
| | CDR-L3 | QQTNEDPYT | 752 |

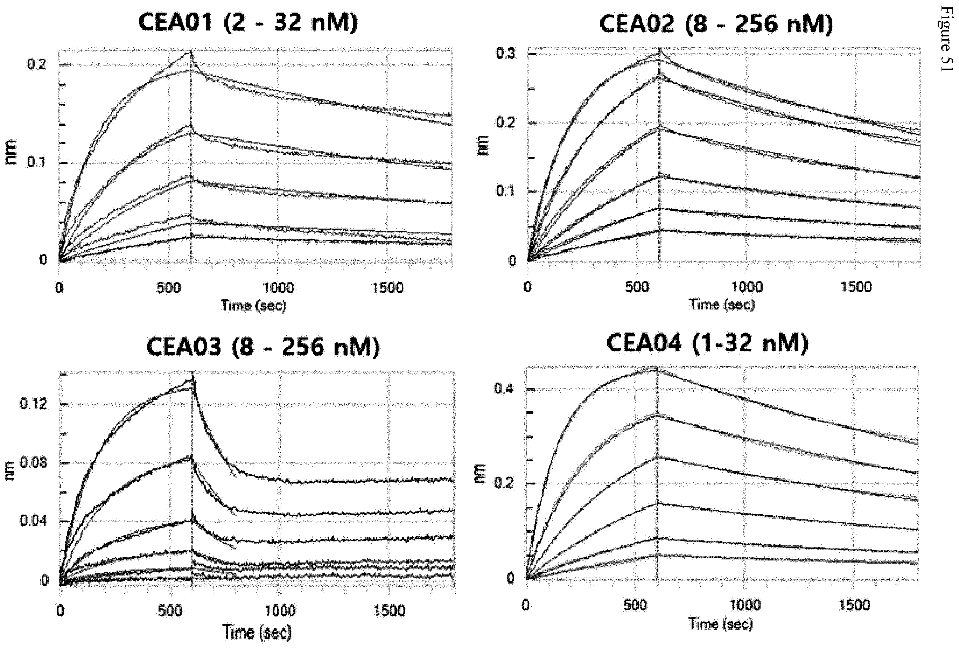

In order to analyze the binding constants, the human CEACAM5 recombinant protein (Sino Biologicals, 11077-H08H) was loaded onto the Anti-Penta-HIS (HIS1K) biosensor (Sartorius, 18-5120). Then, the antibodies were added in a binding reaction (600 seconds) and a dissociation reaction (1,200 seconds) at various concentrations, and their affinities for human CEACAM5 were calculated (Figure 51, Table 79). Table 79 below illustrates the binding constants of the engineered antibodies targeting human CEACAM5.

**[Table 79]**

| Antibodies | K_{D} (nM) | Kₐ (1/Ms) | K_{d} (1/s) |
|---|---|---|---|
| CEA01 | 1.5740 | 1.76E+05 | 2.78E-04 |
| CEA02 | 19.2395 | 2.03E+04 | 3.90E-04 |
| CEA03 | 274.7780 | 1.13E+04 | 3.09E-03 |
| CEA04 | 1.7856 | 2.05E+05 | 3.66E-04 |

### Example 23. Design, preparation and analysis of antibody structure targeting TROP2, mesothelin or LIV-1

The variant light chain and heavy chain polypeptide sequences of the antibody T01 that specifically binds to the TROP2 protein are shown in Table 80. For T01, expression vectors consisting of the sequences corresponding to Fc-Hole (SEQ ID NO: 7), T01 HC (SEQ ID NO: 753), and T01 LC (SEQ ID NO: 754) were co-transfected into EXPICHO-S^{™} (Gibco, A29127) (Figure 41a, Table 80), and purification and analysis were performed in the same manner as described in Example 1. The variant light chain and heavy chain polypeptide sequences of the antibody MSM01 that specifically binds to the mesothelin protein are shown in Table 80. For MSM01, expression vectors consisting of the sequences corresponding to Fc-Hole (SEQ ID NO: 7), MSM01 HC (SEQ ID NO: 755), and MSM01 LC (SEQ ID NO: 756) were co-transfected into EXPICHO-S^{™} (Gibco, A29127) (Figure 41a, Table 80), and purification and analysis were performed in the same manner as described in Example 1. The variant light chain and heavy chain polypeptide sequences of the antibody LIM01 that specifically binds to the LIV-1 protein are shown in Table 80. For LIM01, expression vectors consisting of the sequences corresponding to Fc-Hole (SEQ ID NO: 7), LIM01 HC (SEQ ID NO: 757), and LIM01 LC (SEQ ID NO: 758) were co-transfected into EXPICHO-S^{™} (Gibco, A29127) (Figure 41a, Table 80), and purification and analysis were performed in the same manner as described in Example 1.

**[Table 80]**

| Target Antigen | Name | Sequence | SEQ ID NO |
|---|---|---|---|
| Human TROP2 | T01 HC | QVQLQQSGSELKKPGASVKVSCKASGYTFTNYGMNWVKQAPGQC LKWMGWINTYTGEPTYTDDFKGRFAFSLDTSVSTAYLQISSLKAD DTAVYFCARGGFGSSYWYFDVWGQGSLVTVSSASTKGPSVFPLAP SSKSTSGGTAALGCLVKDYFPEPVTVSWNSGALTSGVHTFPAVLQS SGLYSLSSVVTVPSSSLGTQTYICNVNHKPSNTKVDKKVEPKSCDK THTCPPCPAPELLGGPSVFLFPPKPKDTLMISRTPEVTCVVVDVSHE DPEVKFNWYVDGVEVHNAKTKPREEQYNSTYRVVSVLTVLHQD WLNGKEYKCKVSNKALPAPIEKTISKAKGQPREPQVYTLPPCREEM TKNQVSLWCLVKGFYPSDIAVEWESNGQPENNYKTTPPVLDSDGS FFLYSKLTVDKSRWQQGNVFSCSVMHEALHNHYTQKSLSLSPGK | 753 |
| | T01 LC | DIQLTQSPSSLSASVGDRVSITCKASQDVSIAVAWYQQKPGKAPKL LIYSASYRYTGVPDRFSGSGSGTDFTLTISSLQPEDFAVYYCQQHYI TPLTFGCGTKVEIKRTVAAPSVFIFPPSDEQLKSGTASVVCLLNNFY PREAKVQWKVDNALQSGNSQESVTEQDSKDSTYSLSSTLTLSKAD YEKHKVYACEVTHQGLSSPVTKSFNRGECGGGGSGGGGSGGGGS EPKSSDKTHTCPPCPAPELLGGPSVFLFPPKPKDTLMISRTPEVTCV VVDVSHEDPEVKFNWYVDGVEVHNAKTKPREEQYNSTYRVVSVL TVLHQDWLNGKEYKCKVSNKALPAPIEKTISKAKGQPREPQVYTL PPCREEMTKNQVSLWCLVKGFYPSDIAVEWESNGQPENNYKTTPP VLDSDGSFFLYSKLTVDKSRWQQGNVFSCSVMHEALHNHYTQKS LSLSPGK | 754 |
| Human Mesothelin | MSM01 HC | QVQLQQSGPELEKPGASVKISCKASGYSFTGYTMNWVKQSHGKCL EWIGLITPYNGASSYNQKFRGKATLTVDKSSSTAYMDLLSLTSEDS AVYFCARGGYDGRGFDYWGQGTTVTVSSASTKGPSVFPLAPSSKS TSGGTAALGCLVKDYFPEPVTVSWNSGALTSGVHTFPAVLQSSGL YSLSSVVTVPSSSLGTQTYICNVNHKPSNTKVDKKVEPKSCDKTHT | 755 |
| | | CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPCREEMTKN QVSLWCLVKGFYPSDIAVEWESNGQPENNYKTTPPVLDSDGSFFL YSKLTVDKSRWQQGNVFSCSVMHEALHNHYTQKSLSLSPGK | |
| | MSM01 LC | DIELTQSPAIMSASPGEKVTMTCSASSSVSYMHWYQQKSGTSPKR WIYDTSKLASGVPGRFSGSGSGNSYSLTISSVEAEDDATYYCQQWS KHPLTFGCGTKLEIKRTVAAPSVFIFPPSDEQLKSGTASVVCLLNNF YPREAKVQWKVDNALQSGNSQESVTEQDSKDSTYSLSSTLTLSKA DYEKHKVYACEVTHQGLSSPVTKSFNRGECGGGGSGGGGSGGGG SEPKSSDKTHTCPPCPAPELLGGPSVFLFPPKPKDTLMISRTPEVTCV VVDVSHEDPEVKFNWYVDGVEVHNAKTKPREEQYNSTYRVVSVL TVLHQDWLNGKEYKCKVSNKALPAPIEKTISKAKGQPREPQVYTL PPCREEMTKNQVSLWCLVKGFYPSDIAVEWESNGQPENNYKTTPP VLDSDGSFFLYSKLTVDKSRWQQGNVFSCSVMHEALHNHYTQKS LSLSPGK | 756 |
| Human LIV-1 | LIM01 HC | EIQLQQSGPELMKPGASVKISCKASTYSFTRYFMHWVKQSHGECLE WIGYIDPFNGGTGYNQKFKGKATLTVDKSSSTAYMHLSSLTSEDSA VYYCVTYGSDYFDYWGQGTTLTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLMISRTPEVTCVVVDVSHEDPEVKFN WYVDGVEVHNAKTKPREEQYNSTYRVVSVLTVLHQDWLNGKEY KCKVSNKALPAPIEKTISKAKGQPREPQVYTLPPCREEMTKNQVSL WCLVKGFYPSDIAVEWESNGQPENNYKTTPPVLDSDGSFFLYSKLT VDKSRWQQGNVFSCSVMHEALHNHYTQKSLSLSPGK | 757 |
| | LIM01 LC | DIVMTQPQKFMSTSVGDRVSVTCKASQNVETDVVWYQQKPGQPP KALIYSASYRHSGVPDRFTGSGSGTNFTLTISTVQSEDLAEYFCQQY NNYPFTFGCGTKLEIKRTVAAPSVFIFPPSDEQLKSGTASVVCLLNN FYPREAKVQWKVDNALQSGNSQESVTEQDSKDSTYSLSSTLTLSK ADYEKHKVYACEVTHQGLSSPVTKSFNRGECGGGGSGGGGSGGG GSEPKSSDKTHTCPPCPAPELLGGPSVFLFPPKPKDTLMISRTPEVTC VVVDVSHEDPEVKFNWYVDGVEVHNAKTKPREEQYNSTYRVVSV LTVLHQDWLNGKEYKCKVSNKALPAPIEKTISKAKGQPREPQVYT LPPCREEMTKNQVSLWCLVKGFYPSDIAVEWESNGQPENNYKTTP PVLDSDGSFFLYSKLTVDKSRWQQGNVFSCSVMHEALHNHYTQKS LSLSPGK | 758 |

Table 81 below shows the heavy chain and light chain nucleotide sequences of the engineered antibodies targeting TROP2, mesothelin, or LIV-1.

**[Table 81]**

| **Target Ag** | **Name** | **Sequence** | **SEQ ID NO** |
|---|---|---|---|
| Human TROP2 | T01 HC | CAGGTGCAGCTCCAGCAGTCTGGTTCCGAGCTGAAGAAACCTGGGGCTTCAGTCAAAGTC TCTTGCAAGGCTTCAGGTTACACTTTCACCAATTATGGTATGAACTGGGTCAAGCAAGCT CCTGGTCAGTGTTTGAAGTGGATGGGGTGGATAAACACATATACTGGCGAACCTACATAC ACCGACGACTTCAAGGGACGCTTCGCCTTCTCTCTTGACACAAGTGTCTCAACAGCATAT CTCCAAATCAGTAGCCTTAAGGCCGACGACACAGCAGTTTATTTTTGCGCTAGGGGTGGA TTCGGATCTTCTTACTGGTATTTCGATGTCTGGGGACAAGGAAGTCTGGTCACAGTTTCC AGCGCTAGCACCAAAGGACCTAGTGTTTTTCCTCTTGCCCCTTCCTCAAAGTCTACCTCT GGGGGGACAGCCGCTCTGGGCTGCCTGGTCAAGGATTATTTCCCAGAGCCTGTCACTGTC AGTTGGAACTCTGGAGCCTTGACTTCTGGTGTTCATACATTTCCTGCTGTCCTTCAAAGC AGCGGCTTGTACTCATTGTCTTCTGTTGTGACAGTACCCTCAAGCAGCCTCGGCACTCAG ACATACATCTGCAATGTCAACCACAAACCCTCAAATACAAAGGTAGATAAAAAAGTCGAA CCAAAGTCTTGTGACAAAACTCACACGTGCCCACCGTGCCCAGCACCTGAACTCCTGGGG GGACCGTCAGTCTTCCTCTTCCCCCCAAAACCCAAGGACACCCTCATGATCTCCCGGACC CCTGAGGTCACATGCGTGGTGGTGGACGTGAGCCACGAAGACCCTGAGGTCAAGTTCAAC TGGTACGTGGACGGCGTGGAGGTGCATAATGCCAAGACAAAGCCGCGGGAGGAGCAGTAC AACAGCACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAAACCATC TCCAAAGCCAAAGGGCAGCCCCGAGAACCACAGGTGTACACCCTGCCCCCATGCCGGGAG GAGATGACCAAGAACCAGGTCAGCCTGTGGTGCCTGGTCAAAGGCTTCTATCCCAGCGAC ATCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAGG TGGCAGCAGGGGAACGTCTTCTCATGCTCCGTGATGCATGAGGCTCTGCACAACCACTAC ACGCAGAAGAGCCTCTCCCTGTCTCCGGGTAAA | 759 |
| | T01 LC | GACATACAACTCACACAATCTCCCTCTTCTTTGTCAGCTTCCGTTGGGGACAGGGTGTCA ATTACTTGCAAAGCCTCTCAAGATGTTTCTATAGCTGTAGCCTGGTATCAACAGAAACCC GGAAAAGCTCCCAAGTTGTTGATTTATAGTGCTAGTTATAGGTACACTGGCGTGCCAGAT AGATTCAGTGGTAGCGGTTCTGGGACCGACTTTACCTTGACTATTTCTTCCCTGCAACCT GAGGATTTTGCCGTTTACTATTGCCAACAACATTATATTACTCCCCTTACTTTTGGGTGT GGGACCAAGGTAGAAATCAAGCGTACGGTGGCAGCTCCCAGCGTTTTTATCTTTCCCCCA TCCGACGAGCAGCTCAAGAGTGGCACTGCCTCTGTAGTTTGTTTGCTGAATAACTTCTAT CCACGTGAAGCAAAAGTACAGTGGAAGGTCGATAATGCCCTTCAGAGCGGTAACAGCCAA GAAAGTGTTACCGAGCAAGATTCCAAAGATTCCACTTACAGTCTGTCCAGCACATTGACA CTGAGTAAGGCTGATTACGAAAAACACAAGGTGTACGCATGCGAGGTGACACACCAAGGT CTTTCATCTCCTGTAACTAAGAGCTTTAACCGGGGAGAATGTGGTGGTGGGGGCAGCGGG GGCGGAGGTAGTGGAGGCGGCGGTAGTGAACCAAAGAGTAGTGACAAAACTCACACGTGC CCACCGTGCCCAGCACCTGAACTCCTGGGGGGACCGTCAGTCTTCCTCTTCCCCCCAAAA CCCAAGGACACCCTCATGATCTCCCGGACCCCTGAGGTCACATGCGTGGTGGTGGACGTG AGCCACGAAGACCCTGAGGTCAAGTTCAACTGGTACGTGGACGGCGTGGAGGTGCATAAT | 760 |
| | | GCCAAGACAAAGCCGCGGGAGGAGCAGTACAACAGCACGTACCGTGTGGTCAGCGTCCTC ACCGTCCTGCACCAGGACTGGCTGAATGGCAAGGAGTACAAGTGCAAGGTCTCCAACAAA GCCCTCCCAGCCCCCATCGAGAAAACCATCTCCAAAGCCAAAGGGCAGCCCCGAGAACCA CAGGTGTACACCCTGCCCCCATGCCGGGAGGAGATGACCAAGAACCAGGTCAGCCTGTGG TGCCTGGTCAAAGGCTTCTATCCCAGCGACATCGCCGTGGAGTGGGAGAGCAATGGGCAG CCGGAGAACAACTACAAGACCACGCCTCCCGTGCTGGACTCCGACGGCTCCTTCTTCCTC TACAGCAAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCTCCGGGT AAA | |
| Human Mesothelin | MSM01 HC | CAGGTTCAATTGCAGCAGTCTGGACCAGAATTGGAGAAGCCTGGCGCAAGCGTCAAAATA TCTTGCAAGGCTTCAGGTTACAGTTTCACCGGATACACTATGAACTGGGTCAAGCAAAGC CACGGCAAGTGTCTTGAATGGATAGGATTGATTACTCCATACAATGGCGCTTCATCATAC AATCAGAAGTTTAGGGGCAAGGCAACTTTGACCGTGGATAAGTCATCATCTACCGCATAT ATGGACCTCTTGAGCCTCACAAGTGAAGACTCAGCTGTTTACTTTTGTGCCAGAGGAGGG TATGATGGGCGAGGATTCGACTATTGGGGTCAAGGAACCACCGTGACAGTAAGCTCTGCT AGCACCAAAGGACCTAGTGTTTTTCCTCTTGCCCCTTCCTCAAAGTCTACCTCTGGGGGG ACAGCCGCTCTGGGCTGCCTGGTCAAGGATTATTTCCCAGAGCCTGTCACTGTCAGTTGG AACTCTGGAGCCTTGACTTCTGGTGTTCATACATTTCCTGCTGTCCTTCAAAGCAGCGGC TTGTACTCATTGTCTTCTGTTGTGACAGTACCCTCAAGCAGCCTCGGCACTCAGACATAC ATCTGCAATGTCAACCACAAACCCTCAAATACAAAGGTAGATAAAAAAGTCGAACCAAAG TCTTGTGACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACCCCTGAG GTCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAGC ACGTACCGTGTGGTCAGCGTCCTCACCGTCCTGCACCAGGACTGGCTGAATGGCAAGGAG TACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCATGCCGGGAGGAGATG ACCAAGAACCAGGTCAGCCTGTGGTGCCTGGTCAAAGGCTTCTATCCCAGCGACATCGCC GTGGAGTGGGAGAGCAATGGGCAGCCGGAGAACAACTACAAGACCACGCCTCCCGTGCTG GACTCCGACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTACACGCAG AAGAGCCTCTCCCTGTCTCCGGGTAAA | 761 |
| | MSM01 LC | GACATAGAGTTGACTCAATCTCCAGCAATCATGTCAGCCTCACCCGGAGAAAAGGTCACC ATGACTTGTTCTGCAAGTTCCAGCGTTTCTTATATGCATTGGTACCAGCAGAAGTCAGGG ACTAGCCCTAAGAGATGGATTTACGATACCTCCAAACTGGCCTCCGGGGTGCCAGGCCGG TTTAGTGGCAGTGGAAGCGGTAACAGCTACTCTTTGACCATATCTAGCGTGGAAGCAGAG GACGACGCTACTTATTACTGTCAACAGTGGTCTAAGCACCCACTGACCTTCGGCTGTGGT ACAAAGCTCGAAATAAAACGTACGGTGGCAGCTCCCAGCGTTTTTATCTTTCCCCCATCC GACGAGCAGCTCAAGAGTGGCACTGCCTCTGTAGTTTGTTTGCTGAATAACTTCTATCCA CGTGAAGCAAAAGTACAGTGGAAGGTCGATAATGCCCTTCAGAGCGGTAACAGCCAAGAA | 762 |
| | | AGTGTTACCGAGCAAGATTCCAAAGATTCCACTTACAGTCTGTCCAGCACATTGACACTG AGTAAGGCTGATTACGAAAAACACAAGGTGTACGCATGCGAGGTGACACACCAAGGTCTT TCATCTCCTGTAACTAAGAGCTTTAACCGGGGAGAATGTGGTGGTGGGGGCAGCGGGGGC GGAGGTAGTGGAGGCGGCGGTAGTGAACCAAAGAGTAGTGACAAAACTCACACGTGCCCA CCGTGCCCAGCACCTGAACTCCTGGGGGGACCGTCAGTCTTCCTCTTCCCCCCAAAACCC AAGGACACCCTCATGATCTCCCGGACCCCTGAGGTCACATGCGTGGTGGTGGACGTGAGC CACGAAGACCCTGAGGTCAAGTTCAACTGGTACGTGGACGGCGTGGAGGTGCATAATGCC AAGACAAAGCCGCGGGAGGAGCAGTACAACAGCACGTACCGTGTGGTCAGCGTCCTCACC GTCCTGCACCAGGACTGGCTGAATGGCAAGGAGTACAAGTGCAAGGTCTCCAACAAAGCC CTCCCAGCCCCCATCGAGAAAACCATCTCCAAAGCCAAAGGGCAGCCCCGAGAACCACAG GTGTACACCCTGCCCCCATGCCGGGAGGAGATGACCAAGAACCAGGTCAGCCTGTGGTGC CTGGTCAAAGGCTTCTATCCCAGCGACATCGCCGTGGAGTGGGAGAGCAATGGGCAGCCG GAGAACAACTACAAGACCACGCCTCCCGTGCTGGACTCCGACGGCTCCTTCTTCCTCTAC AGCAAGCTCACCGTGGACAAGAGCAGGTGGCAGCAGGGGAACGTCTTCTCATGCTCCGTG ATGCATGAGGCTCTGCACAACCACTACACGCAGAAGAGCCTCTCCCTGTCTCCGGGTAAA | |
| Human LIV-1 | LIMO1 HC | GAGATACAGTTGCAGCAAAGCGGACCCGAGCTTATGAAACCAGGAGCTAGTGTGAAAATT AGCTGCAAGGCTAGCACCTACTCTTTTACTCGCTATTTTATGCATTGGGTTAAACAGTCA CATGGTGAGTGTTTGGAATGGATCGGGTACATTGATCCCTTTAATGGAGGGACTGGCTAC AACCAGAAGTTTAAAGGAAAAGCCACTCTCACTGTTGACAAAAGTAGTAGTACAGCATAT ATGCACCTCAGTTCCCTTACCAGTGAAGATAGCGCAGTTTACTATTGTGTCACTTACGGA TCAGACTACTTCGACTATTGGGGACAGGGTACAACCCTTACAGTCTCCAGTGCTAGCACC AAAGGACCTAGTGTTTTTCCTCTTGCCCCTTCCTCAAAGTCTACCTCTGGGGGGACAGCC GCTCTGGGCTGCCTGGTCAAGGATTATTTCCCAGAGCCTGTCACTGTCAGTTGGAACTCT GGAGCCTTGACTTCTGGTGTTCATACATTTCCTGCTGTCCTTCAAAGCAGCGGCTTGTAC TCATTGTCTTCTGTTGTGACAGTACCCTCAAGCAGCCTCGGCACTCAGACATACATCTGC AATGTCAACCACAAACCCTCAAATACAAAGGTAGATAAAAAAGTCGAACCAAAGTCTTGT GACAAAACTCACACGTGCCCACCGTGCCCAGCACCTGAACTCCTGGGGGGACCGTCAGTC TTCCTCTTCCCCCCAAAACCCAAGGACACCCTCATGATCTCCCGGACCCCTGAGGTCACA TGCGTGGTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGCACGTAC CGTGTGGTCAGCGTCCTCACCGTCCTGCACCAGGACTGGCTGAATGGCAAGGAGTACAAG TGCAAGGTCTCCAACAAAGCCCTCCCAGCCCCCATCGAGAAAACCATCTCCAAAGCCAAA GGGCAGCCCCGAGAACCACAGGTGTACACCCTGCCCCCATGCCGGGAGGAGATGACCAAG AACCAGGTCAGCCTGTGGTGCCTGGTCAAAGGCTTCTATCCCAGCGACATCGCCGTGGAG TGGGAGAGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAGCAGGGG AACGTCTTCTCATGCTCCGTGATGCATGAGGCTCTGCACAACCACTACACGCAGAAGAGC CTCTCCCTGTCTCCGGGTAAA | 763 |
| | LIMO1 LC | GACATAGTGATGACCCAACCACAAAAGTTTATGTCAACCTCCGTTGGAGATCGTGTTTCT GTCACTTGTAAGGCATCACAGAATGTAGAGACAGACGTAGTCTGGTATCAGCAGAAACCT | 764 |
| | | GGTCAGCCACCTAAAGCACTCATCTACAGTGCAAGTTACCGACATTCTGGCGTACCCGAC AGATTTACTGGGTCTGGTTCTGGTACAAATTTCACTCTCACCATCTCAACCGTCCAATCA GAAGACTTGGCCGAGTATTTTTGCCAGCAATATAACAATTATCCCTTTACATTCGGATGT GGGACCAAACTCGAAATCAAACGTACGGTGGCAGCTCCCAGCGTTTTTATCTTTCCCCCA TCCGACGAGCAGCTCAAGAGTGGCACTGCCTCTGTAGTTTGTTTGCTGAATAACTTCTAT CCACGTGAAGCAAAAGTACAGTGGAAGGTCGATAATGCCCTTCAGAGCGGTAACAGCCAA GAAAGTGTTACCGAGCAAGATTCCAAAGATTCCACTTACAGTCTGTCCAGCACATTGACA CTGAGTAAGGCTGATTACGAAAAACACAAGGTGTACGCATGCGAGGTGACACACCAAGGT CTTTCATCTCCTGTAACTAAGAGCTTTAACCGGGGAGAATGTGGTGGTGGGGGCAGCGGG GGCGGAGGTAGTGGAGGCGGCGGTAGTGAACCAAAGAGTAGTGACAAAACTCACACGTGC CCACCGTGCCCAGCACCTGAACTCCTGGGGGGACCGTCAGTCTTCCTCTTCCCCCCAAAA CCCAAGGACACCCTCATGATCTCCCGGACCCCTGAGGTCACATGCGTGGTGGTGGACGTG AGCCACGAAGACCCTGAGGTCAAGTTCAACTGGTACGTGGACGGCGTGGAGGTGCATAAT GCCAAGACAAAGCCGCGGGAGGAGCAGTACAACAGCACGTACCGTGTGGTCAGCGTCCTC ACCGTCCTGCACCAGGACTGGCTGAATGGCAAGGAGTACAAGTGCAAGGTCTCCAACAAA GCCCTCCCAGCCCCCATCGAGAAAACCATCTCCAAAGCCAAAGGGCAGCCCCGAGAACCA CAGGTGTACACCCTGCCCCCATGCCGGGAGGAGATGACCAAGAACCAGGTCAGCCTGTGG TGCCTGGTCAAAGGCTTCTATCCCAGCGACATCGCCGTGGAGTGGGAGAGCAATGGGCAG CCGGAGAACAACTACAAGACCACGCCTCCCGTGCTGGACTCCGACGGCTCCTTCTTCCTC TACAGCAAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCTCCGGGT AAA | |

Table 82 below shows the polypeptide sequences of the heavy chain variable region (VH) and light chain variable region (VL) of the engineered antibodies targeting TROP2, mesothelin, or LIV-1. Table 83 below shows the nucleotide sequences of the heavy chain variable region (VH) and light chain variable region (VL) of the engineered antibodies targeting TROP2, mesothelin, or LIV-1.

**[Table 82]**

| Antigen | Name | | Sequence | SEQ ID NO |
|---|---|---|---|---|
| Human TROP2 | T01 | VH | QVQLQQSGSELKKPGASVKVSCKASGYTFTNYGMNWVKQA PGQCLKWMGWINTYTGEPTYTDDFKGRFAFSLDTSVSTAYL QISSLKADDTAVYFCARGGFGSSYWYFDVWGQGSLVTVSS | 765 |
| | | VL | DIQLTQSPSSLSASVGDRVSITCKASQDVSIAVAWYQQKPGK APKLLIYSASYRYTGVPDRFSGSGSGTDFTLTISSLQPEDFAV YYCQQHYITPLTFGCGTKVEIK | 766 |
| Human Mesothe lin | MSM01 | VH | QVQLQQSGPELEKPGASVKISCKASGYSFTGYTMNWVKQSH GKCLEWIGLITPYNGASSYNQKFRGKATLTVDKSSSTAYMDL LSLTSEDSAVYFCARGGYDGRGFDYWGQGTTVTVSS | 767 |
| | | VL | DIELTQSPAIMSASPGEKVTMTCSASSSVSYMHWYQQKSGTS PKRWIYDTSKLASGVPGRFSGSGSGNSYSLTISSVEAEDDATY YCQQWSKHPLTFGCGTKLEIK | 768 |
| Human LIV-1 | LIM01 | VH | EIQLQQSGPELMKPGASVKISCKASTYSFTRYFMHWVKQSHG ECLEWIGYIDPFNGGTGYNQKFKGKATLTVDKSSSTAYMHL | 769 |
| | | | SSLTSEDSAVYYCVTYGSDYFDYWGQGTTLTVSS | |
| | | VL | DIVMTQPQKFMSTSVGDRVSVTCKASQNVETDVVWYQQKP GQPPKALIYSASYRHSGVPDRFTGSGSGTNFTLTISTVQSEDL AEYFCQQYNNYPFTFGCGTKLEIK | 770 |

**[Table 83]**

| Antigen | Name | | Sequence | SEQ ID NO |
|---|---|---|---|---|
| Human TROP2 | T01 | VH | CAGGTGCAGCTCCAGCAGTCTGGTTCCGAGCTGAAGAAAC CTGGGGCTTCAGTCAAAGTCTCTTGCAAGGCTTCAGGTTA CACTTTCACCAATTATGGTATGAACTGGGTCAAGCAAGCT CCTGGTCAGTGTTTGAAGTGGATGGGGTGGATAAACACAT ATACTGGCGAACCTACATACACCGACGACTTCAAGGGACG CTTCGCCTTCTCTCTTGACACAAGTGTCTCAACAGCATATC TCCAAATCAGTAGCCTTAAGGCCGACGACACAGCAGTTTA TTTTTGCGCTAGGGGTGGATTCGGATCTTCTTACTGGTATT TCGATGTCTGGGGACAAGGAAGTCTGGTCACAGTTTCCAG C | 771 |
| | | VL | GACATACAACTCACACAATCTCCCTCTTCTTTGTCAGCTTC CGTTGGGGACAGGGTGTCAATTACTTGCAAAGCCTCTCAA GATGTTTCTATAGCTGTAGCCTGGTATCAACAGAAACCCG GAAAAGCTCCCAAGTTGTTGATTTATAGTGCTAGTTATAG GTACACTGGCGTGCCAGATAGATTCAGTGGTAGCGGTTCT GGGACCGACTTTACCTTGACTATTTCTTCCCTGCAACCTGA GGATTTTGCCGTTTACTATTGCCAACAACATTATATTACTC CCCTTACTTTTGGGTGTGGGACCAAGGTAGAAATCAAG | 772 |
| Human Mesothe lin | MSM01 | VH | CAGGTTCAATTGCAGCAGTCTGGACCAGAATTGGAGAAGC CTGGCGCAAGCGTCAAAATATCTTGCAAGGCTTCAGGTTA CAGTTTCACCGGATACACTATGAACTGGGTCAAGCAAAGC CACGGCAAGTGTCTTGAATGGATAGGATTGATTACTCCAT ACAATGGCGCTTCATCATACAATCAGAAGTTTAGGGGCAA GGCAACTTTGACCGTGGATAAGTCATCATCTACCGCATAT ATGGACCTCTTGAGCCTCACAAGTGAAGACTCAGCTGTTT ACTTTTGTGCCAGAGGAGGGTATGATGGGCGAGGATTCGA CTATTGGGGTCAAGGAACCACCGTGACAGTAAGCTCT | 773 |
| | | VL | GACATAGAGTTGACTCAATCTCCAGCAATCATGTCAGCCT CACCCGGAGAAAAGGTCACCATGACTTGTTCTGCAAGTTC CAGCGTTTCTTATATGCATTGGTACCAGCAGAAGTCAGGG ACTAGCCCTAAGAGATGGATTTACGATACCTCCAAACTGG CCTCCGGGGTGCCAGGCCGGTTTAGTGGCAGTGGAAGCGG TAACAGCTACTCTTTGACCATATCTAGCGTGGAAGCAGAG GACGACGCTACTTATTACTGTCAACAGTGGTCTAAGCACC CACTGACCTTCGGCTGTGGTACAAAGCTCGAAATAAAA | 774 |
| Human LIV-1 | LIM01 | VH | GAGATACAGTTGCAGCAAAGCGGACCCGAGCTTATGAAA CCAGGAGCTAGTGTGAAAATTAGCTGCAAGGCTAGCACCT ACTCTTTTACTCGCTATTTTATGCATTGGGTTAAACAGTCA CATGGTGAGTGTTTGGAATGGATCGGGTACATTGATCCCT | 775 |
| | | | TTAATGGAGGGACTGGCTACAACCAGAAGTTTAAAGGAA AAGCCACTCTCACTGTTGACAAAAGTAGTAGTACAGCATA TATGCACCTCAGTTCCCTTACCAGTGAAGATAGCGCAGTTT ACTATTGTGTCACTTACGGATCAGACTACTTCGACTATTGG GGACAGGGTACAACCCTTACAGTCTCCAGT | |
| | | VL | GACATAGTGATGACCCAACCACAAAAGTTTATGTCAACCT CCGTTGGAGATCGTGTTTCTGTCACTTGTAAGGCATCACAG AATGTAGAGACAGACGTAGTCTGGTATCAGCAGAAACCTG GTCAGCCACCTAAAGCACTCATCTACAGTGCAAGTTACCG ACATTCTGGCGTACCCGACAGATTTACTGGGTCTGGTTCTG GTACAAATTTCACTCTCACCATCTCAACCGTCCAATCAGA AGACTTGGCCGAGTATTTTTGCCAGCAATATAACAATTAT CCCTTTACATTCGGATGTGGGACCAAACTCGAAATCAAA | 776 |

Table 84 below shows the heavy chain and light chain CDR sequences of the engineered antibodies targeting TROP2, mesothelin, or LIV-1.

**[Table 84]**

| Antigen | Name | CDR | Sequence | SEQ ID NO |
|---|---|---|---|---|
| Human TROP2 | CEA01 | CDR-H1 | NYGMN | 260 |
| | | CDR-H2 | WINTYTGEPTYTDDFKG | 261 |
| | | CDR-H3 | GGFGSSYWYFDV | 262 |
| | | CDR-L1 | KASQDVSIAVA | 263 |
| | | CDR-L2 | SASYRYT | 264 |
| | | CDR-L3 | QQHYITPLT | 265 |
| Human Mesothelin | MSM01 | CDR-H1 | GYTMN | 777 |
| | | CDR-H2 | LITPYNGASSYNQKFRG | 778 |
| | | CDR-H3 | GGYDGRGFDY | 779 |
| | | CDR-L1 | SASSSVSYMH | 780 |
| | | CDR-L2 | DTSKLAS | 781 |
| | | CDR-L3 | QQWSKHPLT | 782 |
| Human LIV-1 | LIM01 | CDR-H1 | RYFMH | 783 |
| | | CDR-H2 | YIDPFNGGTGYNQKFKG | 784 |
| | | CDR-H3 | YGSDYFDY | 785 |
| | | CDR-L1 | KASQNVETDW | 786 |
| | | CDR-L2 | SASYRHS | 787 |
| | | CDR-L3 | QQYNNYP | 788 |

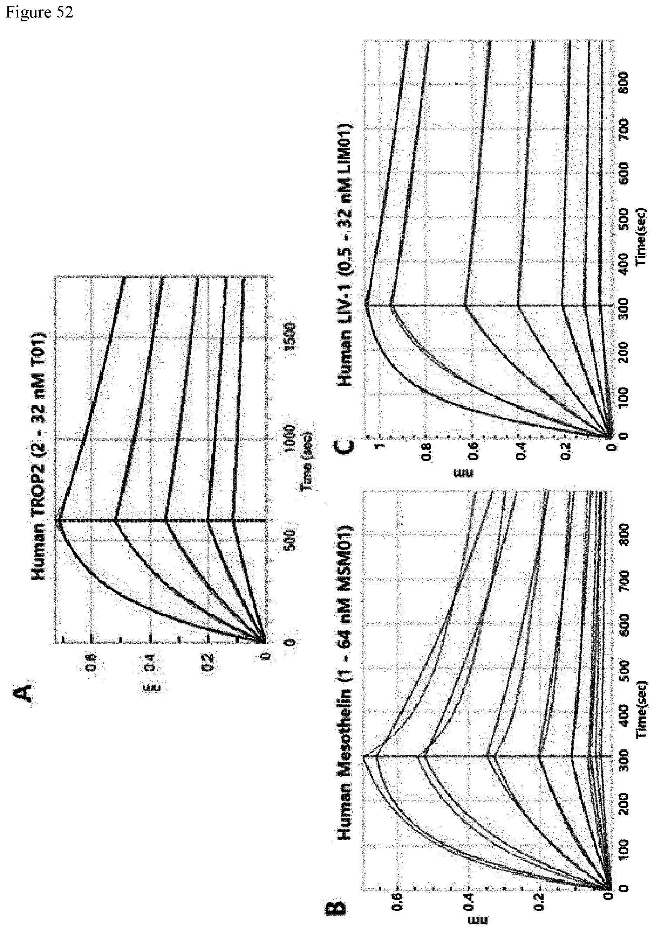

In order to analyze the binding constants, the human TROP2 recombinant protein (Sino Biologicals, 10428-H08H), the human mesothelin recombinant protein (Sino Biologicals, 13128-H08H) or the human LIV-1 recombinant protein (Aero biosystems, LV1-H5223) was loaded onto the Anti-Penta-HIS (HIS1K) biosensor (Sartorius, 18-5120). Then, the antibodies were added in a binding reactionand a dissociation reaction at various concentrations, and the affinities of each antibody for the human antigen was calculated (Figure 52, Table 85). Table 85 below illustrates the binding constants of the engineered antibodies targeting TROP2, mesothelin, or LIV-1.

**[Table 85]**

| Antigens | Antibodies | K_{D} (nM) | Kₐ (1/Ms) | K_{d} (1/s) |
|---|---|---|---|---|
| Human TROP2 | T01 | 1.2008 | 2.67E+05 | 3.21E-04 |
| Human Mesothelin | MSM01 | 7.1771 | 1.59E+05 | 1.14E-03 |
| Human LIV-1 | LIM01 | 0.8072 | 3.84E+05 | 3.10E-04 |

## Claims

1. A fusion protein comprising:
(a) an antigen-binding site consisting of a first polypeptide comprising at least one complementarity-determining region (CDR) sequence and a second polypeptide comprising at least one complementarity-determining region (CDR) sequence, wherein the first polypeptide and the second polypeptide form a dimer, and the antigen-binding site is capable of specifically binding to a target antigen,
(b) a first Fc domain or a variant thereof that is a dimer consisting of two polypeptide sequences, one of which is bound to the first polypeptide of the antigen-binding site, and
(c) a second Fc domain or a variant thereof that is a dimer consisting of two polypeptide sequences, one of which is bound to the second polypeptide of the antigen-binding site.

2. The fusion protein according to claim 1, wherein the first polypeptide of the antigen-binding site comprises CDR1, CDR2, and CDR3 of an antibody heavy chain, and the second polypeptide of the antigen-binding site comprises CDR1, CDR2, and CDR3 of an antibody light chain.

3. The fusion protein according to claim 2, wherein the first polypeptide of the antigen-binding site further comprises a CH1 region of an antibody heavy chain, and/or the second polypeptide of the antigen-binding site further comprises a constant region of an antibody light chain.

4. The fusion protein according to claim 1, wherein the antigen-binding site specifically binds to a protein expressed on the cell surface.

5. The fusion protein according to claim 1, wherein the antigen-binding site specifically binds to a cancer antigen.

6. The fusion protein according to claim 1, wherein the fusion protein induces improved antitumor activity compared to an IgG-based antibody of a conventional structure having the same antigen-binding site.

7. The fusion protein according to claim 1, wherein the antigen-binding site specifically binds to any one selected from the group consisting of PD-L1, EGFR, EGFRvIII, BCMA, CD22, CD25, CD30, CD33, CD37, CD38, CD52, CD56, CD123, c-Met, DLL3, DR4, DR5, GD2, nectin-4, RANKL, SLAMF7, Trop-2, LIV-1, claudin 18.2, IL13α2, CD3, HER2, HER3, FGFR2, FGFR3, GPC3, ROR1, Folα, CD20, CD19, CTLA-4, VEGFR, NCAM1, ICAM-1, ICAM-2, CEACAM5, CEACAM6, carcinoembryonic antigen (CEA), CA-125, alphafetoprotein (AFP), MUC-1, MUC-16, PSMA, PSCA, epithelial tumor antigen (ETA), melanoma-associated antigen (MAGE), immature laminin receptor, TAG-72, HPV E6/E7, BING-4, calcium-activated chloride channel 2, cyclin-B1, 9D7, Ep-CAM, EphA2, EphA3, mesothelin, SAP-1, survivin, and virus-derived antigens.

8. The fusion protein according to claim 1, wherein the first Fc domain and the second Fc domain are each a wild type Fc domain or an Fc domain variant.

9. The fusion protein according to claim 8, wherein the Fc region is an Fc region of IgG, IgA, IgE, IgD, or IgM or a variant thereof.

10. The fusion protein according to claim 8, wherein
the first Fc domain variant and the second Fc domain variant each independently comprise a knob variant or a hole variant that promotes the formation of an Fc heterodimer (heterodimeric Fc); and/or
the first Fc domain variant and the second Fc domain variant each independently comprise a variant that promotes the formation of a heterodimer by electrostatic steering mechanism.

11. The fusion protein according to claim 1, wherein the fusion protein comprises polypeptides of the following structural formulas (I), (II), (III), and (IV):
N'-X-(L1)n-A-C' (I);
N'-Y-(L2)m-B-C' (II);
N'-C-C' (III);
and
N'-D-C' (IV)
wherein, in the structural formulas (I), (II), (III), and (IV),
N' is the N-terminus of each polypeptide,
C' is the C-terminus of each polypeptide,
- refers to a linkage,
A, B, C, and D are monomeric polypeptide sequences of an Fc domain each comprising the CH2 and CH3 regions of an immunoglobulin, and optionally further comprising CH4 and/or a hinge sequence, wherein A forms a dimer with one of C or D to form the first Fc domain (b), and B forms a dimer with the remaining one of C or D to form the second Fc domain (c);
L1 and L2 are each peptide linker,
n and m are each independently 0 or 1,
X is a first polypeptide sequence of the antigen-binding site, which comprises heavy chain CDR1, CDR2, and CDR3 sequences of an antibody that specifically binds to a first antigen, or a heavy chain variable region of an antibody that specifically binds to a first antigen;
Y is a second polypeptide sequence of the antigen-binding site, which comprises light chain CDR1, CDR2, and CDR3 sequences of an antibody that specifically binds to a first antigen, or a light chain variable region of an antibody that specifically binds to a first antigen; and
X and Y pair with each other to form the antigen-binding site (a) that specifically binds to an antigen.

12. The fusion protein according to claim 11, wherein
X in the structural formula (I) further comprises a heavy chain CH1 region, and/or
Y in the structural formula (II) further comprises a light chain constant region.

13. The fusion protein according to claim 1, wherein the fusion protein comprises polypeptides of the following structural formulas (I'), (II'), (III), and (IV):
N'-VD1-(L3)p-X-(L1)n-A-C' (I');
N'-VD2-(L4)q-Y-(L2)m-B-C' (II');
N'-C-C' (III);
and
N'-D-C' (IV)
wherein, in the structural formulas (I'), (II'), (III), and (IV),
N' is the N-terminus of each polypeptide,
C' is the C-terminus of each polypeptide,
- refers to a linkage,
A, B, C, and D are monomeric polypeptide sequences of an Fc domain each comprising the CH2 and CH3 regions of an immunoglobulin, and optionally further comprising CH4 and/or a hinge sequence, wherein A forms a dimer with one of C or D to form the first Fc domain (b), and B forms a dimer with the remaining one of C or D to form the second Fc domain (c);
L1, L2, L3, and L4 are each peptide linker,
n, m, p, and q are each 0 or 1,
VD1 consists of a heavy chain or light chain variable region of an antibody that specifically binds to an antigen, or CDR1, CDR2, and CDR3 of an antibody heavy chain or light chain;
VD2 consists of a light chain or heavy chain variable region of an antibody that specifically binds to an antigen, or CDR1, CDR2, and CDR3 of an antibody heavy chain or light chain;
VD1 and VD2 pair with each other to form a second antibody variable region that specifically binds to a second antigen,
X comprises a heavy chain or light chain variable region of an antibody that specifically binds to an antigen, or CDR1, CDR2, and CDR3 of an antibody heavy chain or light chain;
Y comprises a light chain or heavy chain variable region of an antibody that specifically binds to an antigen, or CDR1, CDR2, and CDR3 of an antibody heavy chain or light chain; and
X and Y pair with each other to form a first antibody variable region that specifically binds to a first antigen, and
VD 1-(L3)p-X forms a first polypeptide sequence of the antigen-binding site (a), and VD2-(L4)q-Y forms a second polypeptide sequence of the antigen-binding site (a).

14. The fusion protein according to claim 13, wherein
the heavy chain variable region further comprises a heavy chain CH1 region, and
the light chain variable region further comprises a light chain constant region.

15. The fusion protein according to claim 11 or 13, wherein
the Fc domain monomer comprises a knob variant or a hole variant that promotes the formation of an Fc heterodimer (heterodimeric Fc); or
the Fc domain monomer comprises a variant that promotes the formation of a heterodimer by electrostatic steering mechanism.

16. The fusion protein according to claim 12 or 14, wherein the binding between X and Y is achieved
i) through a disulfide bond formed by Cys present in CH1 and a light chain constant region,
ii) through a disulfide bond formed by Cys present in a heavy chain variable region and a light chain variable region, or
iii) through a disulfide bond formed by Cys present in CH1 and a light chain constant region, and a disulfide bond formed by Cys present in a heavy chain variable region and a light chain variable region.

17. The fusion protein according to claim 14, wherein the binding between X and Y further comprises, in addition to a disulfide bond present between CH₁233 and CL214 based on Kabat numbering system,
i) a disulfide bond present between VH105 and VL43;
ii) a disulfide bond present between VH44 and VL100; or
iii) a disulfide bond present between CH₁122 and CL121.

18. A pharmaceutical composition for preventing or treating cancer, comprising the fusion protein according to any one of claims 1 to 17 as an active ingredient.

19. The pharmaceutical composition according to claim 18, wherein the cancer is any one selected from the group consisting of gastric cancer, liver cancer, lung cancer, large intestine cancer, breast cancer, prostate cancer, gallbladder cancer, bladder cancer, kidney cancer, esophageal cancer, skin cancer, rectal cancer, osteosarcoma, multiple myeloma, glioma, ovarian cancer, pancreatic cancer, cervical cancer, endometrial cancer, thyroid cancer, laryngeal cancer, testicular cancer, mesothelioma, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer, and lymphoma.

20. A transformed cell expressing the fusion protein according to any one of claims 1 to 17.

21. A method for treating or preventing cancer, comprising administering the fusion protein according to claim 1 to a subject in need of cancer treatment or cancer prevention.

22. A use of the fusion protein according to claim 1 for the treatment of cancer.

23. A use of the fusion protein according to claim 1 for manufacture of a medicament for treating cancer.
